# EUROPEAN PATENT SPECIFICATION

(11) **EP 3 006 460 B1**
(45) Date of publication and mention of the grant of the patent: **26.08.2020**
(21) Application number: 14803694.0
(22) Date of filing: 19.05.2014
(51) Int. Cl.: C07K 14/745, A61K 38/36, A61P 31/04, C07K 14/485, A61K 38/18

(54) **HUMAN SOURCE EGF STRUCTURAL DOMAIN PROTEIN AND APPLICATION OF SAME**
EGF-STRUKTURDOMÄNENPROTEIN MENSCHLICHEN URSPRUNGS UND ANWENDUNG DAVON
DOMAINE STRUCTURAL PROTÉIQUE DE TYPE EGF D'ORIGINE HUMAINE ET SON UTILISATION

(30) Priority: 29.05.2013 CN 201310206726; 30.04.2014 CN 201410182727
(43) Date of publication of application: 13.04.2016
(73) Proprietor: CHENGDU SOURCEBIO LIMITED-LIABILITY COMPANY, Chengdu, Sichuan 610041 (CN)
(72) Inventor: SONG, Xu, Chengdu Sichuan 610065 (CN); LI, Ling, Chengdu Sichuan 610065 (CN); CHEN, JinWu, Chengdu Sichuan 610065 (CN); MA, Deng Jiao, Chengdu Sichuan 610065 (CN)
(74) Representative: Grünecker Patent- und Rechtsanwälte PartG mbB
(86) International application number: PCT/CN2014/077789
(87) International publication number: WO 2014/190860

(56) References cited:
- WO-A1-2010/151736
- WO-A2-02/102319
- WO-A2-2004/108763
- WO-A2-2009/137837
- WO-A2-2010/006136
- CN-A- 101 018 859
- DIMARCQ J-L ET AL: "Cysteine-rich antimicrobial peptides in invertebrates", BIOPOLYMERS, JOHN WILEY & SONS, INC, US, vol. 47, 1 January 1998 (1998-01-01), pages 465-477, XP002250231, ISSN: 0006-3525, DOI: 10.1002/(SICI)1097-0282(1998)47:6<465::AID -BIP5>3.0.CO;2-#
- TAILOR R ET AL: "A novel family of small cysteine-rich antimicrobial peptides from seed of Impatiens balsamina is derived from a single precursor protein", JOURNAL OF BIOLOGICAL CHEMISTRY, AMERICAN SOCIETY FOR BIOCHEMISTRY AND MOLECULAR BIOLOGY, US, vol. 272, no. 39, 26 September 1997 (1997-09-26), pages 24480-24487, XP002124729, ISSN: 0021-9258, DOI: 10.1074/JBC.272.39.24480
- DIAMOND G ET AL: "TRACHEAL ANTIMICROBIAL PEPTIDE, A CYSTEINE-RICH PEPTIDE FROM MAMMALIAN TRACHEAL MUCOSA: PEPTIDE ISOLATION AND CLONING OF A CDNA", PROCEEDINGS OF THE NATIONAL ACADEMY OF SCIENCES, NATIONAL ACADEMY OF SCIENCES, US, vol. 88, no. 9, 1 May 1991 (1991-05-01), pages 3952-3956, XP000203188, ISSN: 0027-8424, DOI: 10.1073/PNAS.88.9.3952
- ZHANG, YINGHONG ET AL.: 'Capability of the First Epidermal Growth Factor Domain of Rat Coagulation Factor ? in Vitro.' CHINESE JOURNAL OF THROMBOSIS AND HEMOSTASIS. vol. 12, no. 4, 31 August 2007, pages 148 - 151, XP008181346
- MEI H. ET AL.: 'Binding of EGF1 Domain Peptide in Coagulation Factor VII with Tissue Factor and Its Implications for the Triggering of Coagulation. J Huazhong Univ Sci Technol' MED SCI vol. 30, no. 1, 28 February 2010, pages 42 - 47, XP055296985 DOI: 10.1007/S11596-010-0108-2
- J. S. JU ET AL: "A Novel 40-kDa Protein Containing Six Repeats of an Epidermal Growth Factor-Like Domain Functions as a Pattern Recognition Protein for Lipopolysaccharide", THE JOURNAL OF IMMUNOLOGY, vol. 177, no. 3, 1 August 2006 (2006-08-01), pages 1838-1845, XP055530585, US ISSN: 0022-1767, DOI: 10.4049/jimmunol.177.3.1838

## Description

### TECHNICAL FIELD

The present invention belongs to biomedical field, and especially relates to human-originated EGF domain proteins and use thereof in the pharmaceutical industry.

### BACKGROUND

Gram-negative bacteria refer broadly to the bacteria which are stained in red in Gram's staining reaction, and they are different from the Gram-positive bacteria during the Gram's staining due to the difference in the structure of the cell wall (the positive bacteria being stained in violet). The Gram-negative bacteria are represented by *Escherichia coli,* as well as Proteus, *Shigella dysenteriae, Klebsiella pneumoniae,* Brucella bacilli, *Haemophilus influenzae, Haemophilus parainfluenzae, Moraxella catarrhalis,* Acinetobacter and so on. Pathogenicity of such bacteria is often associated with a particular component in their cell-walls, lipopolysaccharide (also known as endotoxin). In human's body, lipopolysaccharide will induce the body to produce a large amount of cytokines and to activate the immune system, eventually evoking innate immune responses against the pathogenic bacteria within the body. For instance, redness and swelling are the results of generation and release of a large amount of cytokines.

In addition to eliminating the focus of infection and a symptomatic and supportive treatment, antibiotics also need to be used during the treatment of a Gram-negative bacteria infection. At present, the antibiotics mainly used are aminoglycosides, beta-lactams, etc. These antibiotics have potent bactericidal effects on various Gram-negative bacteria, and moreover exhibit longer post-antibiotic effects on the common Gram-negative bacilli such as *Pseudomonas aeruginosa, Klebsiella pneumonia,* and *E. coli.* In addition to the antibiotics, the agents such as prostaglandin synthetase inhibitor, levamisole, and tuftsin are also useful in treatment of the Gram-negative bacteria infection. However, with the extensive application of antibiotics, the abuse of antibiotics in clinical medicine results in progression of the drug resistance of bacteria from single drug resistance into multi drug resistance, thus making many second-line antibiotics which would have been used effectively as alternatives ineffective. Secondly, the antibacterial drug may induce the generation of endotoxin while killing/inhibiting the bacteria, which increases the difficulty of treating the disease. Thus, while novel antibiotics with potent killing effect come out continuously and advanced supportive therapies occurs, treatment of the endotoxemia caused by Gram's negative bacteria infection is still a challenge in the clinical scenario, particularly with an unacceptable mortality rate of 20-30%. It has been reported that lipopolysaccharide is the major pathogenic factor which leads to a series of toxicity reaction occurring post the Gram-negative bacteria infection. Although no effect on the lipopolysaccharide free in the blood and a wide variety of detrimental cytokines produced by target cells which are persistently stimulated with the free lipopolysaccharide. Therefore, when choosing an antibacterial drug clinically, a comprehensive consideration should be taken for the result of drug sensitive test and the feature of inducing release of the endotoxin. Thirdly, since lipopolysaccharide is located on the surface of the cell wall of Gram-negative bacteria, many early types of antibiotics are unable to inhibit such bacteria effectively. Based on these reasons, novel fields are being explored actively in treating Gram-negative bacteria infection in the recent years.

Antimicrobial peptides (AMPs) refer to short peptides having antibacterial activities, most of which have thermal stability, high alkalincity and broad-spectrum anti-bacterial activities. Currently, about more than or equal to 2000 of AMPs have already been identified from various organisms. These AMPs are synthesized after induction, play an important key role in an organism's resistance against the invasion of a pathogen, and are considered an important defensive constituent for the non-specific immunological function in an organism. Accordingly, it becomes a hot spot and a challenge to find out novel APMs against Gram-negative bacteria.

The EGF domain (the EGF-like domain, epidermal growth factor-like domain) is protein domains which is relatively conserved in evolution and has been designated due to first identification of the domain in epidermal growth factor. The EGF domain usually comprises 30-50 amino acid residues and has been currently identified in many animal proteins. E.g. human coagulation factor VII (human factor VII, hF VII). hF VII is a naturally-occurring protein in the human's body with molecular weight of about 50 kD. The molecule thereof comprises four domains: a membrane-bound N-terminal γ-carboxyglutamic acid domain (Gla domain), two EGF domains (EGF1 and EGF2), and a C-terminal serine protease domain. The human coagulation factor VII-EGF1 domain (hF VII-EGF1) has a molecular weight of about 3.9 kD. In human origin, there are also many analogues with the sequences similar to that of hF VII-EGF1, such as human-originated coagulation factor VII-EGF2 protein (hF VII-EGF2), human-originated coagulation factor IX-EGF1 protein (hF IX-EGF1), human-originated coagulation factor IX-EGF2 protein (hF IX-EGF2), human-originated coagulation factor X-EGF1 protein (hF X-EGF1), human-originated coagulation factor X-EGF2 protein (hF X-EGF2), etc. Up to now, there is no relevant report on treatment of a bacteria infection with a human-originated EGF domain protein as the antibacterial agent and no report on preparation of a medicament for treating endotoxemia caused by a Gram-negative bacterium.

### SUMMARY OF THE INVENTION

### The invention provides the following embodiments 1-13:

1. A human-originated EGF domain protein for use in treating Gram-negative bacteria infection, the amino acid sequence thereof being described in any one of SEQ ID NO: 1-SEQ ID NO: 1498 in the sequence listing.
2. The human-originated EGF domain protein for use in treating Gram-negative bacteria infection according to embodiment 1, characterized in that the amino acid sequence of the protein is described in SEQ ID NO:1, SEQ ID NO:2, SEQ ID NO:3, SEQ ID NO:4, SEQ ID NO:5, SEQ ID NO:6, SEQ ID NO:108, SEQ ID NO:109, SEQ ID NO:1472, SEQ ID NO:1473, SEQ ID NO:1474, SEQ ID NO:1475, SEQ ID NO:1476, SEQ ID NO:1477, SEQ ID NO:1478 or SEQ ID NO:1479 in the sequence listing.
3. The human-originated EGF domain protein for use in treating Gram-negative bacteria infection according to embodiment 1 or embodiment 2, characterized in that the protein is expressed by a prokaryotic or eukaryotic recombinant plasmid or prepared by chemical synthesis.
4. The human-originated EGF domain protein for use in treating Gram-negative bacteria infection according to any one of embodiments 1 to 3, characterized in that the infection causes endotoxemia.
5. The human-originated EGF domain protein for use in treating Gram-negative bacteria infection according to any one of embodiments 1 to 4, characterized in that the Gram-negative bacteria is one or more selected from Escherichia coli DH*5α*, Escherichia coli BL21, Pseudomonas aeruginosa, Klebsiella pneumonia, Enterobacter cloacae, Aeromonas hydrophila, Citrobacter diversus, Moraxella catarrhalis, Proteus mirabilis, Proteus vulgaris and Serratia marcescens.
6. A pharmaceutical composition for use in treating Gram-negative bacteria infection, characterized in that the composition comprises the human-originated EGF domain protein of any one of embodiments 1-5 for use in treating Gram-negative bacteria infection.
7. The pharmaceutical composition for use in treating Gram-negative bacteria infection according to embodiment 6, characterized in that the infection causes endotoxemia.
8. The pharmaceutical composition for use in treating Gram-negative bacteria infection according to embodiment 6 or 7, characterized in that the Gram-negative bacteria is one or more selected from Escherichia coli *DHSα, Escherichia coli* BL21, Pseudomonas aeruginosa, Klebsiella pneumonia, Enterobacter cloacae, Aeromonas hydrophila, Citrobacter diversus, Moraxella catarrhalis, Proteus mirabilis, Proteus vulgaris and Serratia marcescens.
9. An in vitro method for hydrolyzing a lipopolysaccharide of Gram-negative bacteria, comprising the administration of a human-originated EGF domain protein, the amino acid sequence of which is described in any one of SEQ ID NO: 1-SEQ ID NO: 1498 in the sequence listing.
10. The in vitro method of embodiment 9, characterized in that the Gram-negative bacteria is one or more selected from Escherichia coli *DHSα, Escherichia coli* BL21, Pseudomonas aeruginosa, Klebsiella pneumonia, Enterobacter cloacae, Aeromonas hydrophila, Citrobacter diversus, Moraxella catarrhalis, Proteus mirabilis, Proteus vulgaris and Serratia marcescens.
11. The in vitro method of any one of embodiments 9-10, characterized in that the amino acid sequence of the protein is described in SEQ ID NO:1, SEQ ID NO:2, SEQ ID NO:3, SEQ ID NO:4, SEQ ID NO:5, SEQ ID NO:6, SEQ ID NO:108, SEQ ID NO:109, SEQ ID NO:1472, SEQ ID NO:1473, SEQ ID NO:1474, SEQ ID NO:1475, SEQ ID NO:1476, SEQ ID NO:1477, SEQ ID NO:1478 or SEQ ID NO:1479 in the sequence listing.
12. An in vitro method for hydrolyzing a lipopolysaccharide of Gram-negative bacteria, comprising the administration of a composition, wherein the composition comprises the human-originated EGF domain protein of any one of embodiments 9-11.
13. The in vitro method of embodiment 12, characterized in that the Gram-negative bacteria is one or more selected from Escherichia coli *DH5α*, *Escherichia* co*l*i BL21, Pseudomonas aeruginosa, Klebsiella pneumonia, Enterobacter cloacae, Aeromonas hydrophila, Citrobacter diversus, Moraxella catarrhalis, Proteus mirabilis, Proteus vulgaris and Serratia marcescens.

In the following, the invention is defined by the appended claims.

One of the objectives of the present invention is to provide artificially-prepared human-originated EGF domain proteins. Another objective of the present invention is to prove that the human-originated EGF domain proteins have an inhibitory effect on the Gram-negative bacteria and that the human-originated EGF domain proteins can hydrolyze and thus eliminate endotoxin, so as to develop a novel class of medicaments for use in treating Gram-negative bacteria infection and medicaments for treating endotoxemia caused by the Gram-negative bacteria.

The human-originated EGF domain protein set forth in the present invention has the amino acid sequence described in SEQ ID NO: 1 in the sequence listing, or is the protein having homology of over 50% to the amino acid sequence described in SEQ ID NO: 1. The protein having homology of over 50% to the amino acid sequence of the protein described in SEQ ID NO: 1 has any of the amino acid sequences described in SEQ ID NO: 2 to SEQ ID NO: 1498 in the sequence listing.

The human-originated EGF domain proteins set forth in the present invention are designated as shown in Table 1.

**Table 1. Designation of the human-originated EGF domain proteins set forth in the present invention**

| No. in the sequence listing | Designation | Code in the invention |
|---|---|---|
| SEQ ID NO: 1 | human coagulation factor VII-EGF1 domain protein (hF VII-EGF1 protein) | PRT1 |
| SEQ ID NO: 2 | human coagulation factor VII-EGF2 domain protein (hF VII-EGF2 protein) | PRT2 |
| SEQ ID NO: 3 | human coagulation factor IX-EGF1 domain protein (hF IX-EGF1 protein) | PRT3 |
| SEQ ID NO: 4 | human coagulation factor IX-EGF2 domain protein (hF IX-EGF2 protein) | PRT4 |
| SEQ ID NO: 5 | human coagulation factor X-EGF1 domain protein (hF X-EGF1 protein) | PRT5 |
| SEQ ID NO: 6 | human coagulation factor X-EGF2 domain protein (hF X-EGF2 protein) | PRT6 |
| SEQ ID NO: 7 | human ADAM metallopeptidase domain 22-EGF1 protein | PRT7 |
| SEQ ID NO: 8 | human ADAM metallopeptidase domain 30-EGF1 protein | PRT8 |
| SEQ ID NO: 9 | human ADAM metallopeptidase domain 33, isoform CRA_g-EGF1 protein | PRT9 |
| SEQ ID NO: 10 | human ADAM8 protein-EGF1 protein | PRT10 |
| SEQ ID NO: 11 | human ADAM9 protein-EGF1 protein | PRT11 |
| SEQ ID NO: 12 | human ADAM9 protein-EGF2 protein | PRT12 |
| SEQ ID NO: 13 | human Aggrecan core protein-EGF1 protein | PRT13 |
| SEQ ID NO: 14 | human Agrin-EGF1 protein | PRT14 |
| SEQ ID NO: 15 | human Agrin-EGF2 protein | PRT15 |
| SEQ ID NO: 16 | human Agrin-EGF3 protein | PRT16 |
| SEQ ID NO: 17 | human Agrin-EGF4 protein | PRT17 |
| SEQ ID NO: 18 | human Agrin-EGF5 protein | PRT18 |
| SEQ ID NO: 19 | human Agrin-EGF6 protein | PRT19 |
| SEQ ID NO: 20 | human All-trans retinoic acid-induced differentiation lactor-EGF1 protein | PRT20 |
| SEQ ID NO: 21 | human Alpha-tectorin-EGF1 protein | PRT21 |
| SEQ ID NO: 22 | human Alpha-tectorin-EGF2 protein | PRT22 |
| SEQ ID NO: 23 | human Amphiregulin-EGF1 protein | PRT23 |
| SEQ ID NO: 24 | human Anchor protein-EGF1 protein | PRT24 |
| SEQ ID NO: 25 | human Anchor protein-EGF2 protein | PRT25 |
| SEQ ID NO: 26 | human Anchor protein-EGF3 protein | PRT26 |
| SEQ ID NO: 27 | human Anchor protein-EGF4 protein | PRT27 |
| SEQ ID NO: 28 | human Anchor protein-EGF5 protein | PRT28 |
| SEQ ID NO: 29 | human Anchor protein-EGF6 protein | PRT29 |
| SEQ ID NO: 30 | human Anchor protein-EGF7 protein | PRT30 |
| SEQ ID NO: 31 | human Anchor protein-EGF8 protein | PRT31 |
| SEQ ID NO: 32 | human Angiopoietin-I receptor-EGF1 protein | PRT32 |
| SEQ ID NO: 33 | human Angiopoietin-I receptor-EGF2 protein | PRT33 |
| SEQ ID NO: 34 | human Angiopoictin-I rcceptor-EGF3 protein | PRT34 |
| SEQ ID NO: 35 | human Astrotactin-1-EGF1 protein | PRT35 |
| SEQ ID NO: 36 | human Astrotactin-1-EGF2) protein | PRT36 |
| SEQ ID NO: 37 | human Astrotactin-1-EGF3 protein | PRT37 |
| SEQ ID NO: 38 | human Astrotactin-2-EGF1 protein | PRT38 |
| SEQ ID NO: 39 | human Astrotactin-2-EGF2 protein | PRT39 |
| SEQ ID NO: 40 | human Astrotactin-2-EGF3 protein | PRT40 |
| SEQ ID NO: 41 | human Attractin-EGF1 protein | PRT41 |
| SEQ ID NO: 42 | human Attractin-EGF2 protein | PRT42 |
| SEQ ID NO: 43 | human Attractin-EGF3 protein | PRT43 |
| SEQ ID NO: 44 | human Attractin-like protein I-EGF1 protein | PRT44 |
| SEQ ID NO: 45 | human Attractin-like protein I-EGF2 protein | PRT45 |
| SEQ ID NO: 46 | human Attractin-like protein I-EGF3 protein | PRT46 |
| SEQ ID NO: 47 | human Attractin-like protein I-EGF4 protein | PRT47 |
| SEQ ID NO: 48 | human Basement membrane-specific heparan sulfate proteoglycan core protein-EGF1 protein | PRT48 |
| SEQ ID NO: 49 | human Basement membrane-specific heparan sulfate proteoglycan core protein-EGF2 protein | PRT49 |
| SEQ ID NO: 50 | human Basement membrane-specific heparan sulfate proteoglycan core protein-EGF9 protein | PRT50 |
| SEQ ID NO: 51 | human Basement membrane-specific heparan sulfate proteoglycan core protein-EGF10 protein | PRT51 |
| SEQ ID NO: 52 | human Basement membrane-specificheparan sulfate proteoglycan core protein-EGF3 protein | PRT52 |
| SEQ ID NO: 53 | human Basement membrane-specific heparan sulfate proteoglycan core protein-EGF4 protein | PRT53 |
| SEQ ID NO: 54 | human Basement membranc-specific heparan sulfate proteoglycan core protein-EGF5 protein | PRT54 |
| SEQ ID NO: 55 | human Basement membrane-specific heparan sulfate proteoglycan core protein-EGF6 protein | PRT55 |
| SEQ ID NO: 56 | human Basement membrane-specific heparan sulfate proteoglycan core protein-EGF7 protein | PRT56 |
| SEQ ID NO: 57 | human Basement membrane-specific heparan sulfate proteoglycan core protein-EGF8 protein | PRT57 |
| SEQ ID NO: 58 | human BMP/retinoic acid-inducible neural-specific protein 2-EGF1 protein | PRT58 |
| SEQ ID NO: 59 | human BMP/retinoic acid-inducible neural-specifie protein 3-EGF1 protein | PRT59 |
| SEQ ID NO: 60 | human Bone morphogenetic protein I-EGF1 protein | PRT60 |
| SEQ ID NO: 61 | human Bone morphogenetic protein I-EGF2 protein | PRT61 |
| SEQ ID NO: 62 | human Brevican core protein-EGF1 protein | PRT62 |
| SEQ ID NO: 63 | human Clq receptor protein-EGF1 protein | PRT63 |
| SEQ ID NO: 64 | human Clq receptor protein-EGF2 protein | PRT64 |
| SEQ ID NO: 65 | human C5R protein-EGF1 protein | PRT65 |
| SEQ ID NO: 66 | human Cadherin EGF LAG seven-pass G-type receptor 1-EGF1 protein | PRT66 |
| SEQ ID NO: 67 | human Cadherin EGF LAG seven-pass G-type receptor 1-EGF2 protein | PRT67 |
| SEQ ID NO: 68 | human Cadherin EGF LAG seven-pass G-type receptor 1-EGF3 protein | PRT68 |
| SEQ ID NO: 69 | human Cadherin EGF LAG seven-pass G-type receptor 1-EGF4 protein | PRT69 |
| SEQ ID NO: 70 | human Cadherin EGF LAG seven-pass G-type receptor I-EGF5 protein | PRT70 |
| SEQ ID NO: 71 | human Cadherin EGF LAG seven-pass G-type receptor 1-EGF6 protein | PRT71 |
| SEQ ID NO: 72 | human Cadherin EGF LAG seven-pass G-type receptor 1-EGF7 protein | PRT72 |
| SEQ ID NO: 73 | human Cadherin EGF LAG seven-pass G-type receptor 1-EGF8 protein | PRT73 |
| SEQ ID NO: 74 | human Cadherin EGF LAG seven-pass G-type receptor 1-EGF9 protein | PRT74 |
| SEQ ID NO: 75 | human Cadherin EGF LAG seven-pass G-type receptor | PRT75 |
| | 2-EGF1 protein | |
| SEQ ID NO: 76 | human Cadherin EGF LAG seven-pass G-type receptor 2-EGF2 protein | PRT76 |
| SEQ ID NO: 77 | human Cadherin EGF LAG seven-pass G-type receptor 2-EGF3 protein | PRT77 |
| SEQ ID NO: 78 | human Cadherin EGF LAG seven-pass G-type receptor 2-EGF4 protein | PRT78 |
| SEQ ID NO: 79 | human Cadherin EGF LAG seven-pass G-type receptor 2-EGF5 protein | PRT79 |
| SEQ ID NO: 80 | human Cadherin EGF LAG seven-pass G-type receptor 2-EGF6 protein | PRT80 |
| SEQ ID NO: 81 | human Cadherin EGF LAG seven-pass G-type receptor 2-EGF7 protein | PRT81 |
| SEQ ID NO: 82 | human Cadherin EGF LAG seven-pass G-type receptor 2-EGF8 protein | PRT82 |
| SEQ ID NO: 83 | human Cadherin EGF LAG seven-pass G-type receptor 3-EGF1 protein | PRT83 |
| SEQ ID NO: 84 | human Cadherin EGF LAG seven-pass G-type receptor 3-EGF2 protein | PRT84 |
| SEQ ID NO: 85 | human Cadherin EGF LAG seven-pass G-type receptor 3-EGF3 protein | PRT85 |
| SEQ ID NO: 86 | human Cadherin EGF LAG seven-pass G-type receptor 3-EGF4 protein | PRT86 |
| SEQ ID NO: 87 | human Cadherin EGF LAG seven-pass G-type receptor 3-EGF5 protein | PRT87 |
| SEQ ID NO: 88 | human Cadherin EGF LAG seven-pass G-type receptor 3-EGF6 protein | PRT88 |
| SEQ ID NO: 89 | human Cadherin EGF LAG seven-pass G-type receptor 3-EGF7 protein | PRT89 |
| SEQ ID NO: 90 | human Cadherin EGF LAG seven-pass G-type receptor 3-EGF8 protein | PRT90 |
| SEQ ID NO: 91 | human Cartilage acidic protein 1-EGF1 protein | PRT91 |
| SEQ ID NO: 92 | human Cartilage matrix protein-EGF1 protein | PRT92 |
| SEQ ID NO: 93 | human Cartilage oligomeric matrix protein-EGF1 protein | PRT93 |
| SEQ ID NO: 94 | human Cartilage oligomerie matrix protein-EGF2 protein | PRT94 |
| SEQ ID NO: 95 | human Cartilage oligomeric matrix protein-EGF3 protein | PRT95 |
| SEQ ID NO: 96 | human Cartilage oligomeric matrix protein-EGF4 protein | PRT96 |
| SEQ ID NO: 97 | human CD97 antigen-EGF1 protein | PRT97 |
| SEQ ID NO: 98 | human CD97 anligen-EGF2 protein | PRT98 |
| SEQ ID NO: 99 | human CD97 antigen-EGF3 protein | PRT99 |
| SEQ ID NO: 100 | human CD97 antigen-EGF4 protein | PRTI00 |
| SEQ ID NO: 101 | human CD97 antigen-EGF5 protein | PRT101 |
| SEQ ID NO: 102 | human Cell recognition protein CASPR4-EGF1 protein | PRT102 |
| SEQ ID NO: 103 | human Cell recognition protein CASPR4-EGF2 protein | PRT103 |
| SEQ ID NO: 104 | human Chondroitin sulfate proteoglycan 2 (Versican) variant-EGFI protein | PRT 104 |
| SEQ ID NO: 105 | human Chondroitin sulfate proteoglycan 2 (Versican) variant-EGF2 protein | PRT105 |
| SEQ ID NO: 106 | human Chondroitin sulfate proteoglycan 5-EGF1 protein | PRT106 |
| SEQ ID NO: 107 | human CNTNAP3 protein-EGF1 protein | PRT107 |
| SEQ ID NO: 108 | human Coagulation factor XII-EGF1 protein | PRT108 |
| SEQ ID NO: 109 | human Coagulation factor XII-EGF2 protein | PRT109 |
| SEQ ID NO: 110 | human Collagen and calcium-binding EGF domain-containing protein 1-EGF1 protein | PRT110 |
| SEQ ID NO: 111 | human Complement C1r subcomponent-EGF1 protein | PRT111 |
| SEQ ID NO: 112 | human Complement C1s subcomponent-EGF1 protein | PRT112 |
| SEQ ID NO: 113 | human Complement component 1, r subcomponent, isoform CRA_a-EGF1 protein | PRT113 |
| SEQ ID NO: 114 | human Complement component C1q receptor-EGF1 protein | PRT114 |
| SEQ ID NO: 115 | human Complement component C 1q receptor-EGF2 protein | PRT115 |
| SEQ ID NO: 116 | human Complement component C1q receptor-EGF3 protein | PRT116 |
| SEQ ID NO: 117 | human Complement component C1q receptor-EGF4 protein | PRT117 |
| SEQ ID NO: 118 | human Complement component C1q receptor-EGF5 protein | PRT118 |
| SEQ ID NO: 119 | human Complement component C6-EGF1 protein | PRT119 |
| SEQ ID NO: 120 | human Complement component C7-EGF1 protein | PRT 20 |
| SEQ ID NO: 121 | human Complement component C8 alpha chain-EGF1 protein | PRT121 |
| SEQ ID NO: 122 | human Complement component C8 beta chain-EGF1 protein | PRT122 |
| SEQ ID NO: 123 | human Complement component C9-EGF1 protein | PRT123 |
| SEQ ID NO: 124 | human Contactin-associated protein 1-EGF1 protein | PRT124 |
| SEQ ID NO: 125 | human Contactin-associated protein I-EGF2 protein | PRT 125 |
| SEQ ID NO: 126 | human Contactin-associated protein-like 2-EGF1 protein | PRT126 |
| SEQ ID NO: 127 | human Contactin-associated protein-like 2-EGF2 protein | PRT127 |
| SEQ ID NO: 128 | human Contactin-associated protein-like 3-EGF1 protein | PRT128 |
| SEQ ID NO: 129 | human Contactin-associated protein-like 3-EGF2 protein | PRT129 |
| SEQ ID NO: 130 | human Contactin-associated protein-like 3B-EGF1 protein | PRT130 |
| SEQ ID NO: 131 | human Contactin-associated protein-like 3B-EGF2 protein | PRT131 |
| SEQ ID NO: 132 | human Contactin-associated protein-like 4-EGF1 protein | PRT132 |
| SEQ ID NO: 133 | human Contactin-associated protein-like 4-EGF2 protein | PRT133 |
| SEQ ID NO: 134 | human Contactin-associated protein-like 5-EGF1 protein | PRT 134 |
| SEQ ID NO: 135 | human Contactin-associated protein-like 5-EGF2 protein | PRT 135 |
| SEQ ID NO: 136 | human CPXV021 protein-EGF1 protein | PRT136 |
| SEQ ID NO: 137 | human Crumbs homolog 1 isoform II variant-EGF1 protein | PRT137 |
| SEQ ID NO: 138 | human Crumbs homolog 1 isoform 11 variant-EGF2 protein | PRT138 |
| SEQ ID NO: 139 | human Cryptic family protein 1 B-EGF1 protein | PRT139 |
| SEQ ID NO: 140 | human Cryptic protein-EGF1 protein | PRT140 |
| SEQ ID NO: 141 | human CSPG3 variant protein-EGF1 protein | PRT141 |
| SEQ ID NO: 142 | human C-type lectin domain family 14 member A-EGF1 protein | PRT142 |
| SEQ ID NO: 143 | human C-type lectin domain family 18 member A-EGF1 protein | PRT143 |
| SEQ ID NO: 144 | human Cubilin-EGF1 protein | PRT 144 |
| SEQ ID NO: 145 | human Cubilin-EGF2 protein | PRT 145 |
| SEQ ID NO: 146 | human Cubilin-EGF3 protein | PRT146 |
| SEQ ID NO: 147 | human Cubilin-EGF4 protein | PRT147 |
| SEQ ID NO: 148 | human Cubilin-EGF5 protein | PRT148 |
| SEQ ID NO: 149 | human Cubilin-EGF6 protein | PRT149 |
| SEQ ID NO: 1 50 | human Cubilin-EGF7 protein | PRT150 |
| SEQ ID NO: 151 | human Cyritestin 2-EGF1 protein | PRT151 |
| SEQ ID NO: 152 | human Cysteine-rich with EGF-like domain protein 1-EGF1 protein | PRT152 |
| SEQ ID NO: 153 | human Cysteine-rich with EGF-like domain protein 2-EGF1 protein | PRT153 |
| SEQ ID NO: 154 | human Cysteine-rich with EGF-like domain protein 2-EGF2 protein | PRT154 |
| SEQ ID NO: 155 | human Delta and Notch-like epidermal growth factor-related-EGF1 protein | PRT 155 |
| SEQ ID NO: 156 | human Delta and Notch-like epidermal growth factor-related-EGF10 protein | PRT 156 |
| SEQ ID NO: 157 | human Delta and Notch-like epidermal growth factor-related-EGF2 protein | PRT157 |
| SEQ ID NO: 158 | human Delta and Notch-like epidermal growth lactor-related-EGF3 protein | PRT158 |
| SEQ ID NO: 159 | human Delta and Notch-like epidermal growth lactor-related-EGF4 protein | PRT159 |
| SEQ ID NO: 160 | human Delta and Notch-like epidermal growth | PRT160 |
| | lactor-related EGF5 protein | |
| SEQ ID NO: 161 | human Delta and Notch-like epidermal growth factor-related-EGF6 protein | PRT161 |
| SEQ ID NO: 162 | human Delta and Notch-like epidermal growth factor-related-EGF7 protein | PRT 162 |
| SEQ ID NO: 163 | human Delta and Notch-like epidermal growth factor-related-EGF8 protein | PRT163 |
| SEQ ID NO: 164 | human Delta and Notch-like epidermal growth factore-related-EGF9 protein | PRT164 |
| SEQ ID NO: 165 | human Delta-like 1-EGF1 protein | PRT165 |
| SEQ ID NO: 166 | human Delta-like 1-EGF2 protein | PRT 166 |
| SEQ ID NO: 167 | human Delta-like 1-EGF3 protein | PRT167 |
| SEQ ID NO: 168 | human Delta-like protein 1-EGF1 protein | PRT168 |
| SEQ ID NO: 169 | human Delta-like protein 1-EGF2 protein | PRT169 |
| SEQ ID NO: 170 | human Delta-like protein 1-EGF3 protein | PRT170 |
| SEQ ID NO: 171 | human Delta-like protein 1-EGF4 protein | PRT171 |
| SEQ ID NO: 172 | human Delta-like protein 1-EGF5 protein | PRT172 |
| SEQ ID NO: 173 | human Delta-like protein 1-EGF6 protein | PRT173 |
| SEQ ID NO: 174 | human Delta-like protein 1-EGF7 protein | PRT174 |
| SEQ ID NO: 175 | human Delta-like protein 1-EGF8 protein | PRT175 |
| SEQ ID NO: 176 | human Delta-like protein 3-EGF1 protein | PRT176 |
| SEQ ID NO: 177 | human Delta-like protein 3-EGF2 protein | PRT177 |
| SEQ ID NO: 178 | human Delta-like protein 3-EGF3 protein | PRT178 |
| SEQ ID NO: 179 | human Delta-like protein 3-EGF4 protein | PRT179 |
| SEQ ID NO: 180 | human Delta-like protein 3-EGF5 protein | PRT 180 |
| SEQ ID NO: 181 | human Delta-like protein 3-EGF6 protein | PRT181 |
| SEQ ID NO: 182 | human Delta-like protein 4-EGF1 protein | PRT182 |
| SEQ ID NO: 183 | human Delta-like protein 4-EGF2 protein | PRT183 |
| SEQ ID NO: 184 | human Delta-like protein 4-EGF3 protein | PRT184 |
| SEQ ID NO: 185 | human Delta-like protein 4-EGF4 protein | PRT185 |
| SEQ ID NO: 186 | human Delta-like protein 4-EGF5 protein | PRT 186 |
| SEQ ID NO: 187 | human Delta-like protein 4-EGF6 protein | PRT187 |
| SEQ ID NO: 188 | human Delta-like protein 4-EGF7 protein | PRT188 |
| SEQ ID NO: 189 | human Delta-like protein 4-EGF8 protein | PRT189 |
| SEQ ID NO: 190 | human DIET1-EGF1 protein | PRT190 |
| SEQ ID NO: 191 | human Diphtheria toxin receptor (Heparin-binding epidermal growth factor-like growth factor) variant-EGF1 protein | PRT191 |
| SEQ ID NO: 192 | human Disintegrin and metalloproteinase domain-containing protein 11-EGF1 protein | PRT 192 |
| SEQ ID NO: 193 | human Disintegrin and metalloproteinase domain-containing protein 12-EGF1 protein | PRT193 |
| SEQ ID NO: 194 | human Disintegrin and metalloproteinase domain-containing protein 15-EGF1 protein | PRT194 |
| SEQ ID NO: 195 | human Disintegrin and metalloproteinase domain-containing protein 18-EGF1 protein | PRT195 |
| SEQ ID NO: 196 | human Disintegrin and metalloproteinase domain-containing protein 19-EGF1 protein | PRT 196 |
| SEQ ID NO: 197 | human Disintegrin and metalloproteinase domain-containing protein 2-EGF1 protein | PRT 197 |
| SEQ ID NO: 198 | human Disintegrin and metalloproteinase domain-containing protein 20-EGF1 protein | PRT198 |
| SEQ ID NO: 199 | human Disintegrin and metalloproteinase domain-containing protein 21-EGF1 protein | PRT199 |
| SEQ ID NO: 200 | human Disintegrin and metalloproteinase domain-containing protein 22-EGF1 protein | PRT200 |
| SEQ ID NO: 201 | human Disintegrin and metalloproteinase domain-containing protein 23-EGF1 protein | PRT201 |
| SEQ ID NO: 202 | human Disintegrin and metalloproteinase domain-containing | PRT202 |
| | protein 28-EGF1 protein | |
| SEQ ID NO: 203 | human Disintegrin and metalloproteinase domain-containing protein 29-EGFI protein | PRT203 |
| SEQ ID NO: 204 | human Disintegrin and metalloproteinase domain-containing protein 30-EGF1 protein | PRT204 |
| SEQ ID NO: 205 | human Disintegrin and metalloproteinase domain-containing protein 32-EGFI protein | PRT205 |
| SEQ ID NO: 206 | human Disintegrin and metalloproteinase domain-containing protein 33-EGF1 protein | PRT206 |
| SEQ ID NO: 207 | human Disintegrin and metalloproteinase domain-containing protein 8-EGF1 protein | PRT207 |
| SEQ ID NO: 208 | human Disintegrin and metalloproteinase domain-containing protein 9-EGF1 protein | PRT208 |
| SEQ ID NO: 209 | human EFEMP2 protein-EGF1 protein | PRT209 |
| SEQ ID NO: 210 | human EFEMP2 protein-EGF2 protein | PRT210 |
| SEQ ID NO: 211 | human EFEMP2 protein-EGF3 protein | PRT211 |
| SEQ ID NO: 212 | human EFEMP2 protein-EGF4 protein | PRT212 |
| SEQ ID NO: 213 | human EGF, latrophilin and seven transmembrane domain-containing protein 1-EGF1 protein | PRT213 |
| SEQ ID NO: 214 | human EGF, latrophilin and seven transmembrane domain-containing protein 1-EGF2 protein | PRT214 |
| SEQ ID NO: 215 | human EGF-containing fibulin-like extracellular matrix protein 1-EGF1 protein | PRT215 |
| SEQ ID NO: 216 | human EGF-containing fibulin-like extracellular matrix protein 1-EGF2 protein | PRT216 |
| SEQ ID NO: 217 | human EGF-containing fibulin-like extracellular matrix protein 1-EGF3 protein | PRT217 |
| SEQ ID NO: 218 | human EGF-containing fibulin-like extracellular matrix protein I-EGF4 protein | PRT218 |
| SEQ ID NO: 219 | human EGF-containing fibulin-like extracellular matrix protein 1-EGF5 protein | PRT219 |
| SEQ ID NO: 220 | human EGF-containing fibulin-like extracellular matrix protein 1-EGF6 protein | PRT220 |
| SEQ ID NO: 22 1 | human EGF-containing fibulin-like extracellular matrix protein 2-EGF1 protein | PRT221 |
| SEQ ID NO: 222 | human EGF-containing fibulin-like extracellular matrix protein 2-EGF2 protein | PRT222 |
| SEQ ID NO: 223 | human EGF-containing fibulin-like extracellular matrix protein 2-EGF3 protein | PRT223 |
| SEQ ID NO: 224 | human EGF-containing fibulin-like extracellular matrix protein 2-EGF4 protein | PRT224 |
| SEQ ID NO: 225 | human EGF-containing fibutin-like extracellular matrix protein 2-EGF5 protein | PRT225 |
| SEQ ID NO: 226 | human EGF-containing fibulin-like extracellular matrix protein 2-EGF6 protein | PRT226 |
| SEQ ID NO: 227 | human EGF-like module-containing mucin-like hormone receptor-like 1-EGF1 protein | PRT227 |
| SEQ ID NO: 228 | human EGF-like module-containing mucin-like hormone receptor-like 1-EGF2 protein | PRT228 |
| SEQ ID NO: 229 | human EGF-like module-containing mucin-like hormone receptor-like 1-EGF3 protein | PRT229 |
| SEQ ID NO: 230 | human EGF-like module-containing mucin-likc hormone receptor-like I-EGF4 protein | PRT230 |
| SEQ ID NO: 231 | human EGF-like module-containing mucin-likc hormone receptor-like 1-EGF5 protein | PRT231 |
| SEQ ID NO: 232 | human EGF-like module-containing mucin-like hormone receptor-like 1-EGF6 protein | PRT232 |
| SEQ ID NO: 233 | human EGF-like module-containing mucin-like hormone | PRT233 |
| | receptor-like 2-EGF1 protein | |
| SEQ ID NO: 234 | human EGF-like module-containing mucin-likc hormone receptor-like 2-EGF2 protein | PRT234 |
| SEQ ID NO: 235 | human EGF-like module-containing mucin-like hormone receptor-like 2-EGF3 protein | PRT235 |
| SEQ ID NO: 236 | human EGF-like module-conlaining mucin-like hormone receptor-like 3-EGF1 protein | PRT236 |
| SEQ ID NO: 237 | human EGF-like module-containing mucin-like hormone receptor-like 3-EGF2 protein | PRT237 |
| SEQ ID NO: 238 | human EGF-like repeat and discoidin I-like domain-containing protein 3-EGFI protein | PRT238 |
| SEQ ID NO: 239 | human EGF-like repeat and discoidin I-like domain-containing protein 3-EGF2 protein | PRT239 |
| SEQ ID NO: 240 | human EGF-like repeat and discoidin I-like domain-containing protein 3-EGF3 protein | PRT240 |
| SEQ ID NO: 241 | human Endosialin-EGFI protein | PRT241 |
| SEQ ID NO: 242 | human Epidermal growth factor-like protein 6-EGF1 protein | PRT242 |
| SEQ ID NO: 243 | human Epidermal growth factor-like protein 6-EGF2 protein | PRT243 |
| SEQ ID NO: 244 | human Epidermal growth factor-like protein 6-EGF3 protein | PRT244 |
| SEQ ID NO: 245 | human Epidermal growth factor-like protein 6-EGF4 protein | PRT245 |
| SEQ ID NO: 246 | human Epidermal growth factor-like protein 6-EGF5 protein | PRT246 |
| SEQ ID NO: 247 | human Epidermal growth factor-like protein 7-EGFI protein | PRT247 |
| SEQ ID NO: 248 | human Epidermal growth factor-like protein 7-EGF2 protein | PRT248 |
| SEQ ID NO: 249 | human Epidermal growth factor-like protein 8-EGFI protein | PRT249 |
| SEQ ID NO: 250 | human Epidermal growth factor-like protein 8-EGF2 protein | PRT250 |
| SEQ ID NO: 251 | human Epigen-EGF1 protein | PRT251 |
| SEQ ID NO: 252 | human E-selectin-EGF1 protein | PRT252 |
| SEQ ID NO: 253 | human FAT2 protein-EGF1 protein | PRT253 |
| SEQ ID NO: 254 | human FAT2 protein-EGF2 protein | PRT254 |
| SEQ ID NO: 255 | human FBLN7 protein-EGF1 protein | PRT255 |
| SEQ ID NO: 256 | human FEX1-EGF1 protein | PRT256 |
| SEQ ID NO: 257 | human FEX1 -EGF10 protein | PRT257 |
| SEQ ID NO: 258 | human FEX1-EGF2 protein | PRT258 |
| SEQ ID NO: 259 | human FEX1-EGF3 protein | PRT259 |
| SEQ ID NO: 260 | human FEX I-EGF4 protein | PRT260 |
| SEQ ID NO: 261 | human FEX I-EGF5 protein | PRT261 |
| SEQ ID NO: 262 | human FEX I-EGF6 protein | PRT262 |
| SEQ ID NO: 263 | human FEX1-EGF7 protein | PRT263 |
| SEQ ID NO: 264 | human FEX1-EGF8 protein | PRT264 |
| SEQ ID NO: 265 | human FEX1-EGF9 protein | PRT265 |
| SEQ ID NO: 266 | human Fibrillin 15-EGF1 protein | PRT266 |
| SEQ ID NO: 267 | human Fibrillin 15-EGF2 protein | PRT267 |
| SEQ ID NO: 268 | human Fibrillin 15-EGF3 protein | PRT268 |
| SEQ ID NO: 269 | human Fibrillin 15-EGF4 protein | PRT269 |
| SEQ ID NO: 270 | human Fibrillin 15-EGF5 protein | PRT270 |
| SEQ ID NO: 271 | human Fibrillin 15-EGF6 protein | PRT271 |
| SEQ ID NO: 272 | human Fibrillin 15-EGF7 protein | PRT272 |
| SEQ ID NO: 273 | human Fibrillin 15-EGF8 protein | PRT273 |
| SEQ ID NO: 274 | human Fibrillin-1-EGF1 protein | PRT274 |
| SEQ ID NO: 275 | human Fibrillin-1-EGF10 protein | PRT275 |
| SEQ ID NO: 276 | human Fibrillin-1-EGF11 protein | PRT276 |
| SEQ ID NO: 277 | human Fibrillin-1-EGF12 protein | PRT277 |
| SEQ ID NO: 278 | human Fibrillin- 1-EGF13 protein | PRT278 |
| SEQ ID NO: 279 | human Fibrillin-1-EGF14 protein | PRT279 |
| SEQ ID NO: 280 | human Fibrillin-1-EGF15 protein | PRT280 |
| SEQ ID NO: 281 | human Fibrillin-1-EGF16 protein | PRT28 1 |
| SEQ ID NO: 282 | human Fibrillin-1-EGF17 protein | PRT282 |
| SEQ ID NO: 283 | human Fibrillin-1-EGF18 protein | PRT283 |
| SEQ ID NO: 284 | human Fibrillin-1-EGF19 protein | PRT284 |
| SEQ ID NO: 285 | human Fibrillin-1-EGF2 protein | PRT285 |
| SEQ ID NO: 286 | human Fibrillin-1-EGF20 protein | PRT286 |
| SEQ ID NO: 287 | human Fibrillin-1-EGF21 protein | PRT287 |
| SEQ ID NO: 288 | human Fibrillin-1-EGF22 protein | PRT288 |
| SEQ ID NO: 289 | human Fibrillin-1-EGF23 protein | PRT289 |
| SEQ ID NO: 290 | human Fibrillin-1-EGF24 protein | PRT290 |
| SEQ ID NO: 291 | human Fibrillin-1-EGF25 protein | PRT291 |
| SEQ ID NO: 292 | human Fibrillin-1-EGF26 protein | PRT292 |
| SEQ ID NO: 293 | human Fibrillin-1-EGF27 protein | PRT293 |
| SEQ ID NO: 294 | human Fibrillin-1-EGF28 protein | PRT294 |
| SEQ ID NO: 295 | human Fibrillin-1-EGF29 protein | PRT295 |
| SEQ ID NO: 296 | human Fibrillin-1-EGF3 protein | PRT296 |
| SEQ ID NO: 297 | human Fibrillin-1-EGF30 protein | PRT297 |
| SEQ ID NO: 298 | human Fibrillin-1-EGF31 protein | PRT298 |
| SEQ ID NO: 299 | human Fibrillin-1-EGF32 protein | PRT299 |
| SEQ ID NO: 300 | human Fibrillin-1-EGF33 protein | PRT300 |
| SEQ ID NO: 301 | human Fibrillin-1-EGF34 protein | PRT301 |
| SEQ ID NO: 302 | human Fibrillin-1-EGF35 protein | PRT302 |
| SEQ ID NO: 303 | human Fibrillin-1-EGF36 protein | PRT303 |
| SEQ ID NO: 304 | human Fibrillin-1-EGF37 protein | PRT304 |
| SEQ ID NO: 305 | human Fibrillin-1-EGF38 protein | PRT305 |
| SEQ ID NO: 306 | human Fibrillin-1-EGF39 protein | PRT306 |
| SEQ ID NO: 307 | human Fibrillin-1-EGF4 protein | PRT307 |
| SEQ ID NO: 308 | human Fibrillin-1-EGF40 protein | PRT308 |
| SEQ ID NO: 309 | human Fibrillin-1-EGF41 protein | PRT309 |
| SEQ ID NO: 310 | human Fibrillin-1-EGF42 protein | PRT310 |
| SEQ ID NO: 311 | human Fibrillin-1-EGF43 protein | PRT311 |
| SEQ ID NO: 312 | human Fibrillin-1-EGF44 protein | PRT312 |
| SEQ ID NO: 313 | human Fibrillin-1-EGF45 protein | PRT313 |
| SEQ ID NO: 314 | human Fibrillin-1-EGF46 protein | PRT314 |
| SEQ ID NO: 315 | human Fibrillin-1-EGF47 protein | PRT315 |
| SEQ ID NO: 316 | human Fibrillin-1-EGF5 protein | PRT316 |
| SEQ ID NO: 317 | human Fibrillin-1-EGF6 protein | PRT317 |
| SEQ ID NO: 318 | human Fibrillin-1-EGF7 protein | PRT318 |
| SEQ ID NO: 319 | human Fibrillin-1-EGF8 protein | PRT319 |
| SEQ ID NO: 320 | human Fibrillin-1-EGF9 protein | PRT320 |
| SEQ ID NO: 321 | human Fibrillin-2-EGF1 protein | PRT321 |
| SEQ ID NO: 322 | human Fibrillin-2-EGF10 protein | PRT322 |
| SEQ ID NO: 323 | human Fibrillin-2-EGF11 protein | PRT323 |
| SEQ ID NO: 324 | human Fibrillin-2-EGF12 protein | PRT324 |
| SEQ ID NO: 325 | human Fibrillin-2-EGF13 protein | PRT325 |
| SEQ ID NO: 326 | human Fibrillin-2-EGF14 protein | PRT326 |
| SEQ ID NO: 327 | human Fibrillin-2-EGF15 protein | PRT327 |
| SEQ ID NO: 328 | human Fibrillin-2-EGF16 protein | PRT328 |
| SEQ ID NO: 329 | human Fibrillin-2-EGF17 protein | PRT329 |
| SEQ ID NO: 330 | human Fibrillin-2-EGF18 protein | PRT330 |
| SEQ ID NO: 331 | human Fibrillin-2-EGF19 protein | PRT331 |
| SEQ ID NO: 332 | human Fibrillin-2-EGF2 protein | PRT332 |
| SEQ ID NO: 333 | human Fibrillin-2-EGF20 protein | PRT333 |
| SEQ ID NO: 334 | human Fibrillin-2-EGF21 protein | PRT334 |
| SEQ ID NO: 335 | human Fibrillin-2-EGF22 protein | PRT335 |
| SEQ ID NO: 336 | human Fibrillin-2-EGF23 protein | PRT336 |
| SEQ ID NO: 337 | human Fibrillin-2-EGF24 protein | PRT337 |
| SEQ ID NO: 338 | human Fibrillin-2-EGF25 protein | PRT338 |
| SEQ ID NO: 339 | human Fibrillin-2-EGF26 protein | PRT339 |
| SEQ ID NO: 340 | human Fibrillin-2-EGF27 protein | PRT340 |
| SEQ ID NO: 341 | human Fibrillin-2-EGF28 protein | PRT341 |
| SEQ ID NO: 342 | human Fibrillin-2-EGF29 protein | PRT342 |
| SEQ ID NO: 343 | human Fibrillin-2-EGF3 protein | PRT343 |
| SEQ ID NO: 344 | human Fibrillin-2-EGF30 protein | PRT344 |
| SEQ ID NO: 345 | human Fibrillin-2-EGF31 protein | PRT345 |
| SEQ ID NO: 346 | human Fibrillin-2-EGF32 protein | PRT346 |
| SEQ ID NO: 347 | human Fibrillin-2-EGF33 protein | PRT347 |
| SEQ ID NO: 348 | human Fibrillin-2-EGF34 protein | PRT348 |
| SEQ ID NO: 349 | human Fibrillin-2-EGF35 protein | PRT349 |
| SEQ ID NO: 350 | human Fibrillin-2-EGF36 protein | PRT350 |
| SEQ ID NO: 351 | human Fibrillin-2-EGF37 protein | PRT351 |
| SEQ ID NO: 352 | human Fibrillin-2-EGF38 protein | PRT352 |
| SEQ ID NO: 353 | human Fibrillin-2-EGF39 protein | PRT353 |
| SEQ ID NO: 354 | human Fibrillin-2-EGF4 protein | PRT354 |
| SEQ ID NO: 355 | human Fibrillin-2-EGF40 protein | PRT355 |
| SEQ ID NO: 356 | human Fibrillin-2-EGF41 protein | PRT356 |
| SEQ ID NO: 357 | human Fibrillin-2-EGF42 protein | PRT357 |
| SEQ ID NO: 358 | human Fibrillin-2-EGF43 protein | PRT358 |
| SEQ ID NO: 359 | human Fibrillin-2-EGF44 protein | PRT359 |
| SEQ ID NO: 360 | human Fibrillin-2-EGF45 protein | PRT360 |
| SEQ ID NO: 361 | human Fibrillin-2-EGF46 protein | PRT361 |
| SEQ ID NO: 362 | human Fibrillin-2-EGF47 protein | PRT362 |
| SEQ ID NO: 363 | human Fibrillin-2-EGF5 protein | PRT363 |
| SEQ ID NO: 364 | human Fibrillin-2-EGF6 protein | PRT364 |
| SEQ ID NO: 365 | human Fibrillin-2-EGF7 protein | PRT365 |
| SEQ ID NO: 366 | human Fibrillin-2-EGF8 protein | PRT366 |
| SEQ ID NO: 367 | human Fibrillin-2-EGF9 protein | PRT367 |
| SEQ ID NO: 368 | human Fibrillin-3-EGF1 protein | PRT368 |
| SEQ ID NO: 369 | human Fibrillin-3-EGF10 protein | PRT369 |
| SEQ ID NO: 370 | human Fibrillin-3-EGF11 protein | PRT370 |
| SEQ ID NO: 371 | human Fibrillin-3-EGF12 protein | PRT371 |
| SEQ ID NO: 372 | human Fibrillin-3-EGF13 protein | PRT372 |
| SEQ ID NO: 373 | human Fibrillin-3-EGF14 protein | PRT373 |
| SEQ ID NO: 374 | human Fibrillin-3-EGF15 protein | PRT374 |
| SEQ ID NO: 375 | human Fibrillin-3-EGF16 protein | PRT375 |
| SEQ ID NO: 376 | human Fibrillin-3-EGF17 protein | PRT376 |
| SEQ ID NO: 377 | human Fibrillin-3-EGF18 protein | PRT377 |
| SEQ ID NO: 378 | human Fibrillin-3-EGF19 protein | PRT378 |
| SEQ ID NO: 379 | human Fibrillin-3-EGF2 protein | PRT379 |
| SEQ ID NO: 380 | human Fibrillin-3-EGF20 protein | PRT380 |
| SEQ ID NO: 381 | human Fibrillin-3-EGF21 protein | PRT381 |
| SEQ ID NO: 382 | human Fibrillin-3-EGF22 protein | PRT382 |
| SEQ ID NO: 383 | human Fibrillin-3-EGF23 protein | PRT383 |
| SEQ ID NO: 384 | human Fibrillin-3-EGF24 protein | PRT384 |
| SEQ ID NO: 385 | human Fibrillin-3-EGF25 protein | PRT385 |
| SEQ ID NO: 386 | human Fibrillin-3-EGF26 protein | PRT386 |
| SEQ ID NO: 387 | human Fibrillin-3-EGF27 protein | PRT387 |
| SEQ ID NO: 388 | human Fibrillin-3-EGF28 protein | PRT388 |
| SEQ ID NO: 389 | human Fibrillin-3-EGF29 protein | PRT389 |
| SEQ ID NO: 390 | human Fibrillin-3-EGF3 protein | PRT390 |
| SEQ ID NO: 391 | human Fibrillin-3-EGF30 protein | PRT391 |
| SEQ ID NO: 392 | human Fibrillin-3-EGF31 protein | PRT392 |
| SEQ ID NO: 393 | human Fibrillin-3-EGF32 protein | PRT393 |
| SEQ ID NO: 394 | human Fibrillin-3-EGF33 protein | PRT394 |
| SEQ ID NO: 395 | human Fibrillin-3-EGF34 protein | PRT395 |
| SEQ ID NO: 396 | human Fibrillin-3-EGF35 protein | PRT396 |
| SEQ ID NO: 397 | human Fibrillin-3-EGF36 protein | PRT397 |
| SEQ ID NO: 398 | human Fibrillin-3-EGF37 protein | PRT398 |
| SEQ ID NO: 399 | human Fibrillin-3-EGF38 protein | PRT399 |
| SEQ ID NO: 400 | human Fibrillin-3-EGF39 protein | PRT400 |
| SEQ ID NO: 401 | human Fibrillin-3-EGF4 protein | PRT401 |
| SEQ ID NO: 402 | human Fibrillin-3-EGF40 protein | PRT402 |
| SEQ ID NO: 403 | human Fibrillin-3-EGF41 protein | PRT403 |
| SEQ ID NO: 404 | human Fibrillin-3-EGF42 protein | PRT404 |
| SEQ ID NO: 405 | human Fibrillin-3-EGF43 protein | PRT405 |
| SEQ ID NO: 406 | human Fibrillin-3-EGF44 protein | PRT406 |
| SEQ ID NO: 407 | human Fibrillin-3-EGF5 protein | PRT407 |
| SEQ ID NO: 408 | human Fibrillin-3-EGF6 protein | PRT408 |
| SEQ ID NO: 409 | human Fibrillin-3-EGF7 protein | PRT409 |
| SEQ ID NO: 410 | human Fibrillin-3-EGF8 protein | PRT410 |
| SEQ ID NO: 411 | human Fibrillin-3-EGF9 protein | PRT411 |
| SEQ ID NO: 412 | human Fibulin-1-EGF1 protein | PRT412 |
| SEQ ID NO: 413 | human Fibulin-1-EGF2 protein | PRT413 |
| SEQ ID NO: 414 | human Fibulin-1-EGF3 protein | PRT414 |
| SEQ ID NO: 415 | human Fibulin-1-EGF4 protein | PRT415 |
| SEQ ID NO: 416 | human Fibulin-1-EGF5 protein | PRT416 |
| SEQ ID NO: 417 | human Fibulin-1-EGF6 protein | PRT417 |
| SEQ ID NO: 418 | human Fibulin-1-EGF7 protein | PRT418 |
| SEQ ID NO: 419 | human Fibulin-1-EGF8 protein | PRT419 |
| SEQ ID NO: 420 | human Fibulin-1-EGF9 protein | PRT420 |
| SEQ ID NO: 421 | human Fibulin-2-EGF1 protein | PRT42 1 |
| SEQ ID NO: 422 | human Fibulin-2-EGF10 protein | PRT422 |
| SEQ ID NO: 423 | human Fibulin-2-EGF2 protein | PRT423 |
| SEQ ID NO: 424 | human Fibulin-2-EGF3 protein | PRT424 |
| SEQ ID NO: 425 | human Fibulin-2-EGF4 protein | PRT425 |
| SEQ ID NO: 426 | human Fibulin-2-EGF5 protein | PRT426 |
| SEQ ID NO: 427 | human Fibulin-2-EGF6 protein | PRT427 |
| SEQ ID NO: 428 | human Fibulin-2-EGF7 protein | PRT428 |
| SEQ ID NO: 429 | human Fibulin-2-EGF8 protein | PRT429 |
| SEQ ID NO: 430 | human Fibulin-2-EGF9 protein | PRT430 |
| SEQ ID NO: 431 | human Fibulin-5-EGF1 protein | PRT431 |
| SEQ ID NO: 432 | human Fibulin-5-EGF2 protein | PRT432 |
| SEQ ID NO: 433 | human Fibulin-5-EGF3 protein | PRT433 |
| SEQ ID NO: 434 | human Fibulin-5-EGF4 protein | PRT434 |
| SEQ ID NO: 435 | human Fibulin-5-EGF5 protein | PRT435 |
| SEQ ID NO: 436 | human Fibulin-5-EGF6 protein | PRT436 |
| SEQ ID NO: 437 | human Fibulin-6-EGF1 protein | PRT437 |
| SEQ ID NO: 438 | human Fibulin-6-EGF2 protein | PRT438 |
| SEQ ID NO: 439 | human Fibulin-6-EGF3 protein | PRT439 |
| SEQ ID NO: 440 | human Fibulin-6-EGF4 protein | PRT440 |
| SEQ ID NO: 441 | human Fibulin-6-EGF5 protein | PRT44 1 |
| SEQ ID NO: 442 | human Fibulin-6-EGF6 protein | PRT442 |
| SEQ ID NO: 443 | human Fibulin-6-EGF7 protein | PRT443 |
| SEQ ID NO: 444 | human Fibulin-7-EGF1 protein | PRT444 |
| SEQ ID NO: 445 | human Fibulin-7-EGF2 protein | PRT445 |
| SEQ ID NO: 446 | human Fibulin-7-EGF3 protein | PRT446 |
| SEQ ID NO: 447 | human Fibulin-like extracellular matrix protein-EGF1 protein | PRT447 |
| SEQ ID NO: 448 | human Fibulin-like extracellular matrix protein-EGF2 protein | PRT448 |
| SEQ ID NO: 449 | human Fibulin-like extracellular matrix protein-EGF3 protein | PRT449 |
| SEQ ID NO: 450 | human Glycoprotein receptor gp330/megalin-EGF1 protein | PRT450 |
| SEQ ID NO: 451 | human Glycoprotein receptor gp330/megalin-EGF2 protein | PRT451 |
| SEQ ID NO: 452 | human Glycoprotein receptor gp330/megalin-EGF3 protein | PRT452 |
| SEQ ID NO: 453 | human Glycoprotein receptor gp330/megalin-EGF4 protein | PRT453 |
| SEQ ID NO: 454 | human Growth arrest-specific protein 6-EGF1 protein | PRT454 |
| SEQ ID NO: 455 | human Growth arrest-specific protein 6-EGF2 protein | PRT455 |
| SEQ ID NO: 456 | human Growth arrest-specific protein 6-EGF3 protein | PRT456 |
| SEQ ID NO: 457 | human Growth arrest-specific protein 6-EGF4 protein | PRT457 |
| SEQ ID NO: 458 | human Hedgehog-interacting protein-EGF1 protein | PRT458 |
| SEQ ID NO: 459 | human Hedgehog-interacting protein-EGF2 protein | PRT459 |
| SEQ ID NO: 460 | human Hemicentin-2-EGF1 protein | PRT460 |
| SEQ ID NO: 461 | human Hemicentin-2-EGF2 protein | PRT461 |
| SEQ ID NO: 462 | human Hemicentin-2-EGF3 protein | PRT462 |
| SEQ ID NO: 463 | human Hemicentin-2-EGF4 protein | PRT463 |
| SEQ ID NO: 464 | human Hemicentin-2-EGF5 protein | PRT464 |
| SEQ ID NO: 465 | human Hepatocyte growth factor activator-EGF1 protein | PRT465 |
| SEQ ID NO: 466 | human Hepatocyte growth factor activator-EGF2 protein | PRT466 |
| SEQ ID NO: 467 | human HMGA2ex3/WIF1ex3 fusion protein-EGF1 protein | PRT467 |
| SEQ ID NO: 468 | human HMGA2ex3/WIF1ex3 fusion protein-EGF2 protein | PRT468 |
| SEQ ID NO: 469 | human HMGA2ex3/WIF1ex3 fusion protein-EGF3 protein | PRT469 |
| SEQ ID NO: 470 | human HMGA2ex3/WIF1ex3 fusion protein-EGF4 protein | PRT470 |
| SEQ ID NO: 471 | human HMGA2ex3fWIF1ex3 fusion protein-EGF5 protein | PRT471 |
| SEQ ID NO: 472 | human HSPG2 protein-EGF1 protein | PRT472 |
| SEQ ID NO: 473 | human HSPG2 protein-EGF2 protein | PRT473 |
| SEQ ID NO: 474 | human Hyaluronan-binding protein 2-EGF1 protein | PRT474 |
| SEQ ID NO: 475 | human Hyaluronan-binding protein 2-EGF2 protein | PRT475 |
| SEQ ID NO: 476 | human Hyaluronan-binding protein 2-EGF3 protein | PRT476 |
| SEQ ID NO: 477 | human Hyaluronidase-1-EGF1 protein | PRT477 |
| SEQ ID NO: 478 | human Hyaluronidase-2-EGF1 protein | PRT478 |
| SEQ ID NO: 479 | human Hyaluronidase-3-EGF1 protein | PRT479 |
| SEQ ID NO: 480 | human Hyaluronidase-4-EGF1 protein | PRT480 |
| SEQ ID NO: 481 | human Inactive serine protease PAMR1-EGF1 protein | PRT481 |
| SEQ ID NO: 482 | human Integrin beta-like protein 1-EGF1 protein | PRT482 |
| SEQ ID NO: 483 | human Integrin beta-like protein 1-EGF2 protein | PRT483 |
| SEQ ID NO: 484 | human Integrin beta-like protein 1-EGF3 protein | PRT484 |
| SEQ ID NO: 485 | human Interphotoreceptor matrix proteoglycan 1, isoform CRA_a-EGF1 protein | PRT485 |
| SEQ ID NO: 486 | human Interphotoreceptor matrix proteoglycan 2-EGF1 protein | PRT486 |
| SEQ ID NO: 487 | human Interphotoreceptor matrix proteoglycan 2-EGF2 protein | PRT487 |
| SEQ ID NO: 488 | human Intestinal mucin-EGF1 protein | PRT488 |
| SEQ ID NO: 489 | human Intestinal mucin-EGF2 protein | PRT489 |
| SEQ ID NO: 490 | human KIAA0921 splice variant 1-EGF1 protein | PRT490 |
| SEQ ID NO: 491 | human Lactadherin-EGFI protein | PRT491 |
| SEQ ID NO: 492 | human Laminin subunit alpha-1-EGF1 protein | PRT492 |
| SEQ ID NO: 493 | human Laminin subunit alpha-1-EGF10 protein | PRT493 |
| SEQ ID NO: 494 | human Laminin subunit alpha-1-EGF11 protein | PRT494 |
| SEQ ID NO: 495 | human Laminin subunit alpha-1-EGF12 protein | PRT495 |
| SEQ ID NO: 496 | human Laminin subunit alpha-1-EGF13 protein | PRT496 |
| SEQ ID NO: 497 | human Laminin subunit alpha-1-EGF14 protein | PRT497 |
| SEQ ID NO: 498 | human Laminin subunit alpha-1-EGF15 protein | PRT498 |
| SEQ ID NO: 499 | human Laminin subunit alpha-1-EGF2 protein | PRT499 |
| SEQ ID NO: 500 | human Laminin subunit alpha-1-EGF3 protein | PRT500 |
| SEQ ID NO: 501 | human Laminin subunit alpha-1-EGF4 protein | PRT501 |
| SEQ ID NO: 502 | human Laminin subunit alpha-I-EGF5 protein | PRT502 |
| SEQ ID NO: 503 | human Laminin subunit alpha-1-EGF6 protein | PRT503 |
| SEQ ID NO: 504 | human Laminin subunit alpha-1-EGF7 protein | PRT504 |
| SEQ ID NO: 505 | human Laminin subunit alpha-1-EGF8 protein | PRT505 |
| SEQ ID NO: 506 | human Laminin subunit alpha-1-EGF9 protein | PRT506 |
| SEQ ID NO: 507 | human Laminin subunit alpha-2-EGF1 protein | PRT507 |
| SEQ ID NO: 508 | human Laminin subunit alpha-2-EGF10 protein | PRT508 |
| SEQ ID NO: 509 | human Laminin subunit alpha-2-EGF11 protein | PRT509 |
| SEQ ID NO: 510 | human Laminin subunit alpha-2-EGF12 protein | PRT5 10 |
| SEQ ID NO: 511 | human Laminin subunit alpha-2-EGF13 protein | PRT511 |
| SEQ ID NO: 512 | human Laminin subunit alpha-2-EGF14 protein | PRT512 |
| SEQ ID NO: 513 | human Laminin subunit alpha-2-EGF15 protein | PRT513 |
| SEQ ID NO: 514 | human Laminin subunit alpha-2-EGF2 protein | PRT514 |
| SEQ ID NO: 515 | human Laminin subunit alpha-2-EGF3 protein | PRT515 |
| SEQ ID NO: 516 | human Laminin subunit alpha-2-EGF4 protein | PRT516 |
| SEQ ID NO: 517 | human Laminin subunit alpha-2-EGF5 protein | PRT517 |
| SEQ ID NO: 518 | human Laminin subunit alpha-2-EGF6 protein | PRT518 |
| SEQ ID NO: 519 | human Laminin subunit alpha-2-EGF7 protein | PRT519 |
| SEQ ID NO: 520 | human Laminin subunit alpha-2-EGF8 protein | PRT520 |
| SEQ ID NO: 521 | human Laminin subunit alpha-2-EGF9 protein | PRT521 |
| SEQ ID NO: 522 | human Laminin subunit alpha-3-EGF1 protein | PRT522 |
| SEQ ID NO: 523 | human Laminin subunit alpha-3-EGF10 protein | PRT523 |
| SEQ ID NO: 524 | human Laminin subunit alpha-3-EGF11 protein | PRT524 |
| SEQ ID NO: 525 | human Laminin subunit alpha-3-EGF12 protein | PRT525 |
| SEQ ID NO: 526 | human Laminin subunit alpha-3-EGF13 protein | PRT526 |
| SEQ ID NO: 527 | human Laminin subunit alpha-3-EGF14 protein | PRT527 |
| SEQ ID NO: 528 | human Laminin subunit alpha-3-EGF15 protein | PRT528 |
| SEQ ID NO: 529 | human Laminin subunit alpha-3-EGF2 protein | PRT529 |
| SEQ ID NO: 530 | human Laminin subunit alpha-3-EGF3 protein | PRT530 |
| SEQ ID NO: 531 | human Laminin subunit alpha-3-EGF4 protein | PRT531 |
| SEQ ID NO: 532 | human Laminin subunit alpha-3-EGF5 protein | PRT532 |
| SEQ ID NO: 533 | human Laminin subunit alpha-3-EGF6 protein | PRT533 |
| SEQ ID NO: 534 | human Laminin subunit alpha-3-EGF7 protein | PRT534 |
| SEQ ID NO: 535 | human Laminin subunit alpha-3-EGF8 protein | PRT535 |
| SEQ ID NO: 536 | human Laminin subunit alpha-3-EGF9 protein | PRT536 |
| SEQ ID NO: 537 | human Laminin subunit alpha-4-EGF1 protein | PRT537 |
| SEQ ID NO: 538 | human Laminin subunit alpha-4-EGF2 protein | PRT538 |
| SEQ ID NO: 539 | human Laminin subunit alpha-4-EGF3 protein | PRT539 |
| SEQ ID NO: 540 | human Laminin subunit alpha-5-EGFI protein | PRT540 |
| SEQ ID NO: 541 | human Laminin subunit alpha-5-EGF10 protein | PRT541 |
| SEQ ID NO: 542 | human Laminin subunit alpha-5-EGF11 protein | PRT542 |
| SEQ ID NO: 543 | human Laminin subunit alpha-5-EGF12 protein | PRT543 |
| SEQ ID NO: 544 | human Laminin subunit alpha-5-EGF13 protein | PRT544 |
| SEQ ID NO: 545 | human Laminin subunit alpha-5-EGF14 protein | PRT545 |
| SEQ ID NO: 546 | human Laminin subunit alpha-5-EGF15 protein | PRT546 |
| SEQ ID NO: 547 | human Laminin subunit alpha-5-EGF16 protein | PRT547 |
| SEQ ID NO: 548 | human Laminin subunit alpha-5-EGF17 protein | PRT548 |
| SEQ ID NO: 549 | human Laminin subunit alpha-5-EGF18 protein | PRT549 |
| SEQ ID NO: 550 | human Laminin subunit alpha-5-EGF19 protein | PRT550 |
| SEQ ID NO: 551 | human Laminin subunit alpha-5-EGF2 protein | PRT551 |
| SEQ ID NO: 552 | human Laminin subunit alpha-5-EGF20 protein | PRT552 |
| SEQ ID NO: 553 | human Laminin subunit alpha-5-EGF21 protein | PRT553 |
| SEQ ID NO: 554 | human Laminin subunit alpha-5-EGF3 protein | PRT554 |
| SEQ ID NO: 555 | human Laminin subunit alpha-5-EGF4 protein | PRT555 |
| SEQ ID NO: 556 | human Laminin subunit alpha-5-EGF5 protein | PRT556 |
| SEQ ID NO: 557 | human Laminin subunit alpha-5-EGF6 protein | PRT557 |
| SEQ ID NO: 558 | human Laminin subunit alpha-5-EGF7 protein | PRT558 |
| SEQ ID NO: 559 | human Laminin subunit alpha-5-EGF8 protein | PRT559 |
| SEQ ID NO: 560 | human Laminin subunit alpha-5-EGF9 protein | PRT560 |
| SEQ ID NO: 561 | human Laminin subunit beta-1-EGF1 protein | PRT561 |
| SEQ ID NO: 562 | human Laminin subunit beta-1-EGF10 protein | PRT562 |
| SEQ ID NO: 563 | human Laminin subunit beta-1-EGF11 protein | PRT563 |
| SEQ ID NO: 564 | human Laminin subunit bcta-1-EGF12 protein | PRT564 |
| SEQ ID NO: 565 | human Laminin subunit bcta-1-EGF13 protein | PRT565 |
| SEQ ID NO: 566 | human Laminin subunit beta-1-EGF2 protein | PRT566 |
| SEQ ID NO: 567 | human Laminin subunit beta-1-EGF3 protein | PRT567 |
| SEQ ID NO: 568 | human Laminin subunit beta-1-EGF4 protein | PRT568 |
| SEQ ID NO: 569 | human Laminin subunit bcta-1-EGF5 protein | PRT569 |
| SEQ ID NO: 570 | human Laminin subunit beta-1-EGF6 protein | PRT570 |
| SEQ ID NO: 571 | human Laminin subunit beta-1-EGF7 protein | PRT571 |
| SEQ ID NO: 572 | human Laminin subunit beta-1-EGF8 protein | PRT572 |
| SEQ ID NO: 573 | human Laminin subunit beta- 1-EGF9 protein | PRT573 |
| SEQ ID NO: 574 | human Laminin subunit beta-2-EGF1 protein | PRT574 |
| SEQ ID NO: 575 | human Laminin subunit beta-2-EGF10 protein | PRT575 |
| SEQ ID NO: 576 | human Laminin subunit beta-2-EGF11 protein | PRT576 |
| SEQ ID NO: 577 | human Laminin subunit beta-2-EGF12 protein | PRT577 |
| SEQ ID NO: 578 | human Laminin subunit beta-2-EGF13 protein | PRT578 |
| SEQ ID NO: 579 | human Laminin subunit beta-2-EGF2 protein | PRT579 |
| SEQ ID NO: 580 | human Laminin subunit beta-2-EGF3 protein | PRT580 |
| SEQ ID NO: 581 | human Laminin subunit beta-2-EGF4 protein | PRT581 |
| SEQ ID NO: 582 | human Laminin subunit beta-2-EGF5 protein | PRT582 |
| SEQ ID NO: 583 | human Laminin subunit beta-2-EGF6 protein | PRT583 |
| SEQ ID NO: 584 | human Laminin subunit beta-2-EGF7 protein | PRT584 |
| SEQ ID NO: 585 | human Laminin subunit beta-2-EGF8 protein | PRT585 |
| SEQ ID NO: 586 | human Laminin subunit bcta-2-EGF9 protein | PRT586 |
| SEQ ID NO: 587 | human Laminin subunit beta-3-EGFI protein | PRT587 |
| SEQ ID NO: 588 | human Laminin subunit beta-3-EGF2 protein | PRT588 |
| SEQ ID NO: 589 | human Laminin subunit beta-3-EGF3 protein | PRT589 |
| SEQ ID NO: 590 | human Laminin subunit beta-3-EGF4 protein | PRT590 |
| SEQ ID NO: 591 | human Laminin subunit beta-3-EGF5 protein | PRT591 |
| SEQ ID NO: 592 | human Laminin subunit beta-3-EGF6 protein | PRT592 |
| SEQ ID NO: 593 | human Laminin subunit beta4-EGF1 protein | PRT593 |
| SEQ ID NO: 594 | human Laminin subunit beta-4-EGF10 protein | PRT594 |
| SEQ ID NO: 595 | human Laminin subunit beta-4-EGF1 1 protein | PRT595 |
| SEQ ID NO: 596 | human Laminin subunit beta-4-EGF12 protein | PRT596 |
| SEQ ID NO: 597 | human Laminin subunit beta-4-EGF13 protein | PRT597 |
| SEQ ID NO: 598 | human Laminin subunit beta-4-EGF2 protein | PRT598 |
| SEQ ID NO: 599 | human Laminin subunit beta-4-EGF3 protein | PRT599 |
| SEQ ID NO: 600 | human Laminin subunit bcta-4-EGF4 protein | PRT600 |
| SEQ ID NO: 601 | human Laminin subunit bcta-4-EGF5 protein | PRT601 |
| SEQ ID NO: 602 | human Laminin subunit beta-4-EGF6 protein | PRT602 |
| SEQ ID NO: 603 | human Laminin subunit beta-4-EGF7 protein | PRT603 |
| SEQ ID NO: 604 | human Laminin subunit bcta-4-EGF8 protein | PRT604 |
| SEQ ID NO: 605 | human Laminin subunit beta-4-EGF9 protein | PRT605 |
| SEQ ID NO: 606 | human Laminin subunit gamma-1-EGF1 protein | PRT606 |
| SEQ ID NO: 607 | human Laminin subunit gamma-1-EGF10 protein | PRT607 |
| SEQ ID NO: 608 | human Laminin subunit gamma-1-EGF2 protein | PRT608 |
| SEQ ID NO: 609 | human Laminin subunit gamma-1-EGF3 protein | PRT609 |
| SEQ ID NO: 610 | human Laminin subunit gamma-1-EGF4 protein | PRT610 |
| SEQ ID NO: 611 | human Laminin subunit gamma-1-EGF5 protein | PRT611 |
| SEQ ID NO: 612 | human Laminin subunit gamma-1-EGF6 protein | PRT612 |
| SEQ ID NO: 613 | human Laminin subunit gamma-1-EGF7 protein | PRT613 |
| SEQ ID NO: 614 | human Laminin subunit gamma-1-EGF8 protein | PRT614 |
| SEQ ID NO: 615 | human Laminin subunit gamma-1-EGF9 protein | PRT615 |
| SEQ ID NO: 616 | human Laminin subunit gamma-2-EGF1 protein | PRT616 |
| SEQ ID NO: 617 | human Laminin subunit gamma-2-EGF2 protein | PRT617 |
| SEQ ID NO: 618 | human Laminin subunit gamma-2-EGF3 protein | PRT618 |
| SEQ ID NO: 619 | human Laminin subunit gamma-2-EGF4 protein | PRT619 |
| SEQ ID NO: 620 | human Laminin subunit gamma-2-EGF5 protein | PRT620 |
| SEQ ID NO: 621 | human Laminin subunit gamma-2-EGF6 protein | PRT621 |
| SEQ ID NO: 622 | human Laminin subunit gamma-2-EGF7 protein | PRT622 |
| SEQ ID NO: 623 | human Laminin subunit gamma-3-EGF1 protein | PRT623 |
| SEQ ID NO: 624 | human Laminin subunit gamma-3-EGF10 protein | PRT624 |
| SEQ ID NO: 625 | human Laminin subunit gamma-3-EGF2 protein | PRT625 |
| SEQ ID NO: 626 | human Laminin subunit gamma-3-EGF3 protein | PRT626 |
| SEQ ID NO: 627 | human Laminin subunit gamma-3-EGF4 protein | PRT627 |
| SEQ ID NO: 628 | human Laminin subunit gamma-3-EGF5 protein | PRT628 |
| SEQ ID NO: 629 | human Laminin subunit gamma-3-EGF6 protein | PRT629 |
| SEQ ID NO: 630 | human Laminin subunit gamma-3-EGF7 protein | PRT630 |
| SEQ ID NO: 631 | human Laminin subunit gamma-3-EGF8 protein | PRT631 |
| SEQ ID NO: 632 | human Laminin subunit gamma-3-EGF9 protein | PRT632 |
| SEQ ID NO: 633 | human Latent-transforming growth factor beta-binding protein 1-EGF1 protein | PRT633 |
| SEQ ID NO: 634 | human Latent-transforming growth factor beta-binding protein 1-EGF10 protein | PRT634 |
| SEQ ID NO: 635 | human Latent-transforming growth factor beta-binding protein 1-EGF11 protein | PRT635 |
| SEQ ID NO: 636 | human Latent-transforming growth factor beta-binding protein 1-EGF12 protein | PRT636 |
| SEQ ID NO: 637 | human Latent-transforming growth factor beta-binding protein 1-EGF13 protein | PRT637 |
| SEQ ID NO: 638 | human Latent-transforming growth factor beta-binding protein 1-EGF14 protein | PRT638 |
| SEQ ID NO: 639 | human Latent-transforming growth factor beta-binding protein 1-EGF15 protein | PRT639 |
| SEQ ID NO: 640 | human Latent-transforming growth factor beta-binding protein 1-EGF16 protein | PRT640 |
| SEQ ID NO: 641 | human Latent-transforming growth factor beta-binding protein 1-EGF17 protein | PRT641 |
| SEQ ID NO: 642 | human Latent-transforming growth factor beta-binding protein 1-EGF18 protein | PRT642 |
| SEQ ID NO: 643 | human Latent-transforming growth factor beta-binding protein I-EGF2 protein | PRT643 |
| SEQ ID NO: 644 | human Latent-transforming growth factor beta-binding protein 1-EGF3 protein | PRT644 |
| SEQ ID NO: 645 | human Latent-transforming growth factor beta-binding protein 1-EGF4 protein | PRT645 |
| SEQ ID NO: 646 | human Latent-transforming growth factor beta-binding protein 1-EGF5 protein | PRT646 |
| SEQ ID NO: 647 | human Latent-transforming growth factor beta-binding protein 1-EGF6 protein | PRT647 |
| SEQ ID NO: 648 | human Latent-transforming growth factor beta-binding protein 1-EGF7 protein | PRT648 |
| SEQ ID NO: 649 | human Latent-transforming growth factor beta-binding protein 1-EGF8 protein | PRT649 |
| SEQ ID NO: 650 | human Latent-transforming growth factor beta-binding protein 1-EGF9 protein | PRT650 |
| SEQ ID NO: 651 | human Latent-transforming growth factor beta-binding protein 2-EGF1 protein | PRT651 |
| SEQ ID NO: 652 | human Latent-transforming growth factor beta-binding protein 2-EGF10 protein | PRT652 |
| SEQ ID NO: 653 | human Latent-transforming growth factor beta-binding protein 2-EGF11 protein | PRT653 |
| SEQ ID NO: 654 | human Latent-transforming growth factor beta-binding protein 2-EGF12 protein | PRT654 |
| SEQ ID NO: 655 | human Latent-transforming growth factor beta-binding protein 2-EGF13 protein | PRT655 |
| SEQ ID NO: 656 | human Latent-transforming growth factor beta-binding protein 2-EGF14 protein | PRT656 |
| SEQ ID NO: 657 | human Latent-transforming growth factor beta-binding protein 2-EGF15 protein | PRT657 |
| SEQ ID NO: 658 | human Latent-transforming growth factor beta-binding | PRT658 |
| | protein 2-EGF16 protein | |
| SEQ ID NO: 659 | human Latent-transforming growth factor beta-binding protein 2-EGF17 protein | PRT659 |
| SEQ ID NO: 660 | human Latent-transforming growth factor beta-binding protein 2-EGF18 protein | PRT660 |
| SEQ ID NO: 661 | human Latent-transforming growth factor beta-binding protein 2-EGF19 protein | PRT661 |
| SEQ ID NO: 662 | human Latent-transforming growth factor beta-binding protein 2-EGF2 protein | PRT662 |
| SEQ ID NO: 663 | human Latent-transforming growth factor beta-binding protein 2-EGF20 protein | PRT663 |
| SEQ ID NO: 664 | human Latent-transforming growth factor beta-binding protein 2-EGF3 protein | PRT664 |
| SEQ ID NO: 665 | human Latent-transforming growth factor beta-binding protein 2-EGF4 protein | PRT665 |
| SEQ ID NO: 666 | human Latent-transforming growth factor beta-binding protein 2-EGF5 protein | PRT666 |
| SEQ ID NO: 667 | human Latent-transforming growth factor beta-binding protein 2-EGF6 protein | PRT667 |
| SEQ ID NO: 668 | human Latent-transforming growth factor beta-binding protein 2-EGF7 protein | PRT668 |
| SEQ ID NO: 669 | human Latent-transforming growth factor beta-binding protein 2-EGF8 protein | PRT669 |
| SEQ ID NO: 670 | human Latent-transforming growth factor beta-binding protein 2-EGF9 protein | PRT670 |
| SEQ ID NO: 671 | human Latent-transforming growth factor beta-binding protein 3-EGFI protein | PRT671 |
| SEQ ID NO: 672 | human Latent-transforming growth factor beta-binding protein 3-EGF10 protein | PRT672 |
| SEQ ID NO: 673 | human Latent-transforming growth factor beta-binding protein 3-EGF11 protein | PRT673 |
| SEQ ID NO: 674 | human Latent-transforming growth factor beta-binding protein 3-EGF12 protein | PRT674 |
| SEQ ID NO: 675 | human Latent-transforming growth factor beta-binding protein 3-EGF13 protein | PRT675 |
| SEQ ID NO: 676 | human Latent-transforming growth factor beta-binding protein 3-EGF2 protein | PRT676 |
| SEQ ID NO: 677 | human Latent-transforming growth factor beta-binding protein 3-EGF3 protein | PRT677 |
| SEQ ID NO: 678 | human Latent-transforming growth factor beta-binding protein 3-EGF4 protein | PRT678 |
| SEQ ID NO: 679 | human Latent-transforming growth factor beta-binding protein 3-EGF5 protein | PRT679 |
| SEQ ID NO: 680 | human Latent-transforming growth factor beta-binding protein 3-EGF6 protein | PRT680 |
| SEQ ID NO: 681 | human Latent-transforming growth factor beta-binding protein 3-EGF7 protein | PRT681 |
| SEQ ID NO: 682 | human Latent-transforming growth factor beta-binding protein 3-EGF8 protein | PRT682 |
| SEQ ID NO: 683 | human Latent-transforming growth factor beta-binding protein 3-EGF9 protein | PRT683 |
| SEQ ID NO: 684 | human Latent-transforming growth factor beta-binding protein 4-EGF1 protein | PRT684 |
| SEQ ID NO: 685 | human Latent-transforming growth factor beta-binding protein 4-EGF10 protein | PRT685 |
| SEQ ID NO: 686 | human Latent-transforming growth factor beta-binding protein 4-EGF11 protein | PRT686 |
| SEQ ID NO: 687 | human Latent-transforming growth factor beta-binding | PRT687 |
| | protein 4-EGF12 protein | |
| SEQ ID NO: 688 | human Latent-transforming growth factor beta-binding protein 4-EGF13 protein | PRT688 |
| SEQ ID NO: 689 | human Latent-transforming growth factor beta-binding protein 4-EGF14 protein | PRT689 |
| SEQ ID NO: 690 | human Latent-transforming growth factor beta-binding protein 4-EGF15 protein | PRT690 |
| SEQ ID NO: 691 | human Latent-transforming growth factor beta-binding protein 4-EGF16 protein | PRT691 |
| SEQ ID NO: 692 | human Latent-transforming growth factor beta-binding protein 4-EGF2 protein | PRT692 |
| SEQ ID NO: 693 | human Latent-transforming growth factor beta-binding protein 4-EGF3 protein | PRT693 |
| SEQ ID NO: 694 | human Latent-transforming growth factor beta-binding protein 4-EGF4 protein | PRT694 |
| SEQ ID NO: 695 | human Latent-transforming growth factor beta-binding protein 4-EGF5 protein | PRT695 |
| SEQ ID NO: 696 | human Latent-transforming growth factor beta-binding protein 4-EGF6 protein | PRT696 |
| SEQ ID NO: 697 | human Latent-transforming growth factor beta-binding protein 4-EGF7 protein | PRT697 |
| SEQ ID NO: 698 | human Latent-transforming growth factor beta-binding protein 4-EGF8 protein | PRT698 |
| SEQ ID NO: 699 | human Latent-transforming growth factor beta-binding protein 4-EGF9 protein | PRT699 |
| SEQ ID NO: 700 | human Low-density lipoprotein rercptor-EGF1 protein | PRT700 |
| SEQ ID NO: 701 | human Low-density lipoprotein receptor-EGF2 protein | PRT701 |
| SEQ ID NO: 702 | human Low-density lipoprotein receptor-EGF3 protein | PRT702 |
| SEQ ID NO: 703 | human Low-density lipoprotein receptor-related protein | PRT703 |
| | 1-EGF1 protein | |
| SEQ ID NO: 704 | human Low-density lipoprotein receptor-related protein 1B-EGF1 protein | PRT704 |
| SEQ ID NO: 705 | human Low-density lipoprotein receptor-related protein 1B-EGF10 protein | PRT705 |
| SEQ ID NO: 706 | human Low-density lipoprotein receptor-related protein 1 B-EGF1 1 protein | PRT706 |
| SEQ ID NO: 707 | human Low-density lipoprotein receptor-related protein 1 B-EGF12 protein | PRT707 |
| SEQ ID NO: 708 | human Low-density lipoprotein receptor-related protein 1 B-EGF13 protein | PRT708 |
| SEQ ID NO: 709 | human Low-density lipoprotein receptor-related protein 1B-EGF14 protein | PRT709 |
| SEQ ID NO: 710 | human Low-density lipoprotein receptor-related protein 1 B-EGF2 protein | PRT710 |
| SEQ ID NO: 711 | human Low-density lipoprotein receptor-related protein 1B-EGF3 protein | PRT711 |
| SEQ ID NO: 712 | human Low-density lipoprotein receptor-related protein 1 B-EGF4 protein | PRT712 |
| SEQ ID NO: 713 | human Low-density lipoprotein receptor-related protein 1 B-EGF5 protein | PRT713 |
| SEQ ID NO: 714 | human Low-density lipoprotein receptor-related protein 1 B-EGF6 protein | PRT714 |
| SEQ ID NO: 715 | human Low-density lipoprotein receptor-related protein 1 B-EGF7 protein | PRT715 |
| SEQ ID NO: 716 | human Low-density lipoprotein receptor-related protein 1B-EGF8 protein | PRT716 |
| SEQ ID NO: 717 | human Low-density lipoprotein receptor-related protein 1 B-EGF9 protein | PRT717 |
| SEQ ID NO: 718 | human Low-density lipoprotein receptor-related protein 2-EGF1 protein | PRT718 |
| SEQ ID NO: 719 | human Low-density lipoprotein receptor-related protein 2-EGF10 protein | PRT719 |
| SEQ ID NO: 720 | human Low-density lipoprotein receptor-related protein 2-EGF1 1 protein | PRT720 |
| SEQ ID NO: 721 | human Low-density lipoprotein receptor-related protein 2-EGF12 protein | PRT721 |
| SEQ ID NO: 722 | human Low-density lipoprotein receptor-relalcd protein 2-EGF13 protein | PRT722 |
| SEQ ID NO: 723 | human Low-density lipoprotein receptor-related protein 2-EGF14 protein | PRT723 |
| SEQ ID NO: 724 | human Low-density lipoprotein receptor-related protein 2-EGF15 protein | PRT724 |
| SEQ ID NO: 725 | human Low-density lipoprotein receptor-related protein 2-EGF16 protein | PRT725 |
| SEQ ID NO: 726 | human Low-density lipoprotein receptor-related protein 2-EGF17 protein | PRT726 |
| SEQ ID NO: 727 | human Low-density lipoprotein receptor-related protein 2-EGF2 protein | PRT727 |
| SEQ ID NO: 728 | human Low-density lipoprotein receptor-related protein 2-EGF3 protein | PRT728 |
| SEQ ID NO: 729 | human Low-density lipoprotein receptor-related protein 2-EGF4 protein | PRT729 |
| SEQ ID NO: 730 | human Low-density lipoprotein receptor-related protein 2-EGF5 protein | PRT730 |
| SEQ ID NO: 731 | human Low-density lipoprotein receptor-related protein 2-EGF6 protein | PRT731 |
| SEQ ID NO: 732 | human Low-density lipoprotein receptor-related protein | PRT732 |
| | 2-EGF7 protein | |
| SEQ ID NO: 733 | human Low-density lipoprotein receptor-related protein 2-EGF8 protein | PRT733 |
| SEQ ID NO: 734 | human Low-density lipoprotein receptor-related protein 2-EGF9 protein | PRT734 |
| SEQ ID NO: 735 | human Low-density lipoprotein receptor-related protein 4-EGF1 protein | PRT735 |
| SEQ ID NO: 736 | human Low-density lipoprotein receptor-related protein 4-EGF2 protein | PRT736 |
| SEQ ID NO: 737 | human Low-density lipoprotein receptor-related protein 4-EGF3 protein | PRT737 |
| SEQ ID NO: 738 | human Low-density lipoprotein receptor-related protein 5-EGF1 protein | PRT738 |
| SEQ ID NO: 739 | human Low-density lipoprotein receptor-related protein 5-EGF2 protein | PRT739 |
| SEQ ID NO: 740 | human Low-density lipoprotein receptor-related protein 5-EGF3 protein | PRT740 |
| SEQ ID NO: 741 | human Low-density lipoprotein receptor-related protein 5-EGF4 protein | PRT741 |
| SEQ ID NO: 742 | human Low-density lipoprotein receptor-related protein 6-EGF1 protein | PRT742 |
| SEQ ID NO: 743 | human Low-density lipoprotein receptor-related protein 6-EGF2 protein | PRT743 |
| SEQ ID NO: 744 | human Low-density lipoprotein receptor-related protein 6-EGF3 protein | PRT744 |
| SEQ ID NO: 745 | human Low-density lipoprotein receptor-related protein 6-EGF4 protein | PRT745 |
| SEQ ID NO: 746 | human Low-density lipoprotein receptor-related protein 8-EGF1 protein | PRT746 |
| SEQ ID NO: 747 | human Low-density lipoprotein receptor-related protein 8-EGF2 protein | PRT747 |
| SEQ ID NO: 748 | human L-selectin-EGF1 protein | PRT748 |
| SEQ ID NO: 749 | human Mannan-binding lectin serine protease 1-EGP1 protein | PRT749 |
| SEQ ID NO: 750 | human Mannan-hinding lectin serine protease 2-EGF1 protein | PRT750 |
| SEQ ID NO: 751 | human MATN1 protein-EGF1 protein | PRT751 |
| SEQ ID NO: 752 | human Matrilin-2-EGF1 protein | PRT752 |
| SEQ ID NO: 753 | human Matrilin-2-EGF10 protein | PRT753 |
| SEQ ID NO: 754 | human Matrilin-2-EGF2 protein | PRT754 |
| SEQ ID NO: 755 | human Matrilin-2-EGF3 protein | PRT755 |
| SEQ ID NO: 756 | human Matrilin-2-EGF4 protein | PRT756 |
| SEQ ID NO: 757 | human Matrilin-2-EGF5 protein | PRT757 |
| SEQ ID NO: 758 | human Matrilin-2-EGF6 protein | PRT758 |
| SEQ ID NO: 759 | human Matrilin-2-EGF7 protein | PRT759 |
| SEQ ID NO: 760 | human Matrilin-2-EGF8 protein | PRT760 |
| SEQ ID NO: 761 | human Matrilin-2-EGF9 protein | PRT761 |
| SEQ ID NO: 762 | human Matrilin-3-EGF1 protein | PRT762 |
| SEQ ID NO: 763 | human Matrilin-3-EGF2 protein | PRT763 |
| SEQ ID NO: 764 | human Matrilin-3-EGF3 protein | PRT764 |
| SEQ ID NO: 765 | human Matrilin-3-EGF4 protein | PRT765 |
| SEQ ID NO: 766 | human Matrilin-4-EGF1 protein | PRT766 |
| SEQ ID NO: 767 | human Matrilin-4-EGF2 protein | PRT767 |
| SEQ ID NO: 768 | human Matrilin-4-EGF3 protein | PRT768 |
| SEQ ID NO: 769 | human Matrilin-4-EGF4 protein | PRT769 |
| SEQ ID NO: 770 | human Meprin A subunit alpha-EGFI protein | PRT770 |
| SEQ ID NO: 771 | human Meprin A subunit beta-EGFI protein | PRT771 |
| SEQ ID NO: 772 | human Mucin-12-EGF1 protein | PRT772 |
| SEQ ID NO: 773 | human Mucin-13-EGF1 protein | PRT773 |
| SEQ ID NO: 774 | human Mucin-13-EGF2 protein | PRT774 |
| SEQ ID NO: 775 | human Mucin-13-EGF3 protein | PRT775 |
| SEQ ID NO: 776 | human Mucin-17-EGF1 protein | PRT776 |
| SEQ ID NO: 777 | human Mucin-3A-EGF1 protein | PRT777 |
| SEQ ID NO: 778 | human Mucin-4-EGF1 protein | PRT778 |
| SEQ ID NO: 779 | human Mucin-4-EGF2 protein | PRT779 |
| SEQ ID NO: 780 | human Multimerin-1-EGF1 protein | PRT780 |
| SEQ ID NO: 781 | human Multiple epidermal growth factor-like domains protein 10-EGF1 protein | PRT781 |
| SEQ ID NO: 782 | human Multiple epidermal growth factor-like domains protein 10-EGF10 protein | PRT782 |
| SEQ ID NO: 783 | human Multiple epidermal growth factor-like domains protein 10-EGF11 protein | PRT783 |
| SEQ ID NO: 784 | human Multiple epidermal growth factor-like domains protein 10-EGF12 protein | PRT784 |
| SEQ ID NO: 785 | human Multiple epidermal growth factor-like domains protein 10-EGF13 protein | PRT785 |
| SEQ ID NO: 786 | human Multiple epidermal growth factor-like domains protein 10-EGF14 protein | PRT786 |
| SEQ ID NO: 787 | human Multiple epidermal growth factor-like domains protein 10-EGF15 protein | PRT787 |
| SEQ ID NO: 788 | human Multiple epidermal growth factor-like domains protein 10-EGF2 protein | PRT788 |
| SEQ ID NO: 789 | human Multiple epidermal growth factor-like domains protein 10-EGF3 protein | PRT789 |
| SEQ ID NO: 790 | human Multiple epidermal growth factor-like domains protein 10-EGF4 protein | PRT790 |
| SEQ ID NO: 791 | human Multiple epidermal growth factor-like domains | PRT791 |
| | protein 10-EGF5 protein | |
| SEQ ID NO: 792 | human Multiple epidermal growth factor-like domains protein 10-EGF6 protein | PRT792 |
| SEQ ID NO: 793 | human Multiple epidermal growth factor-like domains protein 10-EGF7 protein | PRT793 |
| SEQ ID NO: 794 | human Multiple epidermal growth factor-like domains protein 10-EGF8 protein | PRT794 |
| SEQ ID NO: 795 | human Multiple epidermal growth factor-like domains protein 10-EGF9 protein | PRT795 |
| SEQ ID NO: 796 | human Multiple epidermal growth factor-like domains protein 11-EGF1 protein | PRT796 |
| SEQ ID NO: 797 | human Multiple epidermal growth factor-like domains protein 11-EGF10 protein | PRT797 |
| SEQ ID NO: 798 | human Multiple epidermal growth factor-like domains protein 11-EGF11 protein | PRT798 |
| SEQ ID NO: 799 | human Multiple epidermal growth factor-like domains protein 11-EGF12 protein | PRT799 |
| SEQ ID NO: 800 | human Multiple epidermal growth factor-like domains protein 11-EGF13 protein | PRT800 |
| SEQ ID NO: 801 | human Multiple epidermal growth factor-like domains protein 11-EGF14 protein | PRT801 |
| SEQ ID NO: 802 | human Multiple epidermal growth factor-like domains protein 11-EGF2 protein | PRT802 |
| SEQ ID NO: 803 | human Multiple epidermal growth factor-like domains protein 11-EGF3 protein | PRT803 |
| SEQ ID NO: 804 | human Multiple epidermal growth factor-like domains protein 1 1-EGF4 protein | PRT804 |
| SEQ ID NO: 805 | human Multiple epidermal growth factor-like domains protein 1 1-EGF5 protein | PRT805 |
| SEQ ID NO: 806 | human Multiple epidermal growth factor-like domains protein 11-EGF6 protein | PRT806 |
| SEQ ID NO: 807 | human Multiple epidermal growth factor-like domains protein 11-EGF7 protein | PRT807 |
| SEQ ID NO: 808 | human Multiple epidermal growth factor-like domains protein 1 1-EGF8 protein | PRT808 |
| SEQ ID NO: 809 | human Multiple epidermal growth factor-like domains protein 1 1-EGF9 protein | PRT809 |
| SEQ ID NO: 810 | human Multiple epidermal growth factor-like domains protein 6-EGF1 protein | PRT810 |
| SEQ ID NO: 811 | human Multiple epidermal growth factor-like domains protein 6-EGF10 protein | PRT811 |
| SEQ ID NO: 812 | human Multiple epidermal growth factor-like domains protein 6-EGF1 1 protein | PRT812 |
| SEQ ID NO: 813 | human Multiple epidermal growth factor-like domains protein 6-EGF12 protein | PRT813 |
| SEQ ID NO: 814 | human Multiple epidermal growth factor-like domains protein 6-EGF13 protein | PRT814 |
| SEQ ID NO: 815 | human Multiple epidermal growth factor-like domains protein 6-EGF14 protein | PRT815 |
| SEQ ID NO: 816 | human Multiple epidermal growth factor-like domains protein 6-EGF15 protein | PRT816 |
| SEQ ID NO: 817 | human Multiple epidermal growth factor-like domains protein 6-EGF16 protein | PRT817 |
| SEQ ID NO: 818 | human Multiple epidermal growth factor-like domains 818 protein 6-EGF17 protein | PRT818 |
| SEQ ID NO: 819 | human Multiple epidermal growth factor-like domains protein 6-EGF18 protein | PRT819 |
| SEQ ID NO: 820 | human Multiple epidermal growth factor-like domains | PRT820 |
| | protein 6-EGF19 protein | |
| SEQ ID NO: 821 | human Multiple epidermal growth factor-like domains protein 6-EGF2 protein | PRT821 |
| SEQ ID NO: 822 | human Multiple epidermal growth factor-like domains protein 6-EGF20 protein | PRT822 |
| SEQ ID NO: 823 | human Multiple epidermal growth factor-like domains protein 6-EGF21 protein | PRT823 |
| SEQ ID NO: 824 | human Multiple epidermal growth factor-like domains protein 6-EGF22 protein | PRT824 |
| SEQ ID NO: 825 | human Multiple epidermal growth factor-like domains protein 6-EGF23 protein | PRT825 |
| SEQ ID NO: 826 | human Multiple epidermal growth factor-like domains protein 6-EGF24 protein | PRT826 |
| SEQ ID NO: 827 | human Multiple epidermal growth factor-like domains protein 6-EGF25 protein | PRT827 |
| SEQ ID NO: 828 | human Multiple epidermal growth factor-like domains protein 6-EGF26 protein | PRT828 |
| SEQ ID NO: 829 | human Multiple epidermal growth factor-like domains protein 6-EGF27 protein | PRT829 |
| SEQ ID NO: 830 | human Multiple epidermal growth factor-like domains protein 6-EGF3 protein | PRT830 |
| SEQ ID NO: 831 | human Multiple epidermal growth factor-like domains protein 6-EGF4 protein | PRT831 |
| SEQ ID NO: 832 | human Multiple epidermal growth factor-like domains protein 6-EGF5 protein | PRT832 |
| SEQ ID NO: 833 | human Multiple epidermal growth factor-likc domains protein 6-EGF6 protein | PRT833 |
| SEQ ID NO: 834 | human Multiple epidermal growth factor-like domains protein 6-EGF7 protein | PRT834 |
| SEQ ID NO: 835 | human Multiple epidermal growth factor-like domains protein 6-EGF8 protein | PRT835 |
| SEQ ID NO: 836 | human Multiple epidermal growth factor-like domains protein 6-EGF9 protein | PRT836 |
| SEQ ID NO: 837 | human Multiple epidermal growth factor-like domains protein 8-EGF1 protein | PRT837 |
| SEQ ID NO: 838 | human Multiple epidermal growth factor-like domains protein 8-EGF2 protein | PRT838 |
| SEQ ID NO: 839 | human Multiple epidermal growth factor-like domains protein 8-EGF3 protein | PRT839 |
| SEQ ID NO: 840 | human Multiple epidermal growth factor-like domains protein 8-EGF4 protein | PRT840 |
| SEQ ID NO: 841 | human Multiple epidermal growth factor-like domains protein 8-EGF5 protein | PRT841 |
| SEQ ID NO: 842 | human Multiple epidermal growth factor-like domains protein 8-EGF6 protein | PRT842 |
| SEQ ID NO: 843 | human Multiple epidermal growth factor-like domains protein 8-EGF7 protein | PRT843 |
| SEQ ID NO: 844 | human Multiple epidermal growth factor-like domains protein 8-EGF8 protein | PRT844 |
| SEQ ID NO: 845 | human Multiple epidermal growth factor-like domains protein 8-EGF9 protein | PRT845 |
| SEQ ID NO: 846 | human Multiple epidermal growth factor-like domains protein 9-EGFI protein | PRT846 |
| SEQ ID NO: 847 | human Multiple epidermal growth factor-like domains protein 9-EGF2 protein | PRT847 |
| SEQ ID NO: 848 | human Multiple epidermal growth factor-like domains protein 9-EGF3 protein | PRT848 |
| SEQ ID NO: 849 | human Multiple epidermal growth factor-like domains | PRT849 |
| | protein 9-EGF4 protein | |
| SEQ ID NO: 850 | human Multiple epidermal growth factor-like domains protein 9-EGF5 protein | PRT850 |
| SEQ ID NO: 851 | human N-acetylglucosamine-1-phosphodiester alpha-N-acetylglucosaminidase-EGF1 protein | PRT851 |
| SEQ ID NO: 852 | human Nephronectin-EGF1 protein | PRT852 |
| SEQ ID NO: 853 | human Nephronectin-EGF2 protein | PRT853 |
| SEQ ID NO: 854 | human Nephronectin-EGF3 protein | PRT854 |
| SEQ ID NO: 855 | human Nephronectin-EGF4 protein | PRT855 |
| SEQ ID NO: 856 | human Nephronectin-EGF5 protein | PRT856 |
| SEQ ID NO: 857 | human Netrin-1-EGF1 protein | PRT857 |
| SEQ ID NO: 858 | human Netrin-1-EGF2 protein | PRT858 |
| SEQ ID NO: 859 | human Netrin-1-EGF3 protein | PRT859 |
| SEQ ID NO: 860 | human Netrin-3-EGF1 protein | PRT860 |
| SEQ ID NO: 861 | human Netrin-3-EGF2 protein | PRT861 |
| SEQ ID NO: 862 | human Netrin-3-EGF3 protein | PRT862 |
| SEQ ID NO: 863 | human Netrin-4-EGF1 protein | PRT863 |
| SEQ ID NO: 864 | human Netrin-4-EGF2 protein | PRT864 |
| SEQ ID NO: 865 | human Netrin-4-EGF3 protein | PRT865 |
| SEQ ID NO: 866 | human Netrin-5-EGF1 protein | PRT866 |
| SEQ ID NO: 867 | human Netrin-5-EGF2 protein | PRT867 |
| SEQ ID NO: 868 | human Netrin-5-EGF3 protein | PRT868 |
| SEQ ID NO: 869 | human Netrin-G1-EGF1 protein | PRT869 |
| SEQ ID NO: 870 | human Netrin-G 1-EGF2 protein | PRT870 |
| SEQ ID NO: 871 | human Netrin-G1-EGF3 protein | PRT871 |
| SEQ ID NO: 872 | human Netrin-G2-EGF1 protein | PRT872 |
| SEQ ID NO: 873 | human Netrin-G2-EGF2 protein | PRT873 |
| SEQ ID NO: 874 | human Netrin-G2-EGF3 protein | PRT874 |
| SEQ ID NO: 875 | human Neurexin-1-EGF1 protein | PRT875 |
| SEQ ID NO: 876 | human Neurexin-1-EGF2 protein | PRT876 |
| SEQ ID NO: 877 | human Neurexin-1-EGF3 protein | PRT877 |
| SEQ ID NO: 878 | human Neurexin-2-EGF1 protein | PRT878 |
| SEQ ID NO: 879 | human Neurexin-2-EGF2 protein | PRT879 |
| SEQ ID NO: 880 | human Neurexin-2-EGF3 protein | PRT880 |
| SEQ ID NO: 881 | human Neurexin-3-EGF1 protein | PRT881 |
| SEQ ID NO: 882 | human Neurexin-3-EGF2 protein | PRT882 |
| SEQ ID NO: 883 | human Neurexin-3-EGF3 protein | PRT883 |
| SEQ ID NO: 884 | human Neurocan core protein-EGF1 protein | PRT884 |
| SEQ ID NO: 885 | human Neurocan core protein-EGF2 protein | PRT885 |
| SEQ ID NO: 886 | human Neurogenic locus notch homolog protein 1-EGF1 protein | PRT886 |
| SEQ ID NO: 887 | human Neurogenic locus notch homolog protein 1-EGF10 protein | PRT887 |
| SEQ ID NO: 888 | human Neurogenic locus notch homolog protein 1-EGF11 protein | PRT888 |
| SEQ ID NO: 889 | human Neurogenic locus notch homolog protein 1-EGF12 protein | PRT889 |
| SEQ ID NO: 890 | human Neurogenic locus notch homolog protein 1-EGF13 protein | PRT890 |
| SEQ ID NO: 891 | human Neurogenic locus notch homolog protein 1-EGF14 protein | PRT891 |
| SEQ ID NO: 892 | human Neurogenic locus notch homolog protein 1-EGF15 protein | PRT892 |
| SEQ ID NO: 893 | human Neurogenic locus notch homolog protein 1-EGF16 protein | PRT893 |
| SEQ ID NO: 894 | human Neurogenic locus notch homolog protein 1-EGF17 protein | PRT894 |
| SEQ ID NO: 895 | human Neurogenic locus notch homolog protein 1-EGF18 | PRT895 |
| | protein | |
| SEQ ID NO: 896 | human Neurogenic locus notch homolog protein 1-EGF19 protein | PRT896 |
| SEQ ID NO: 897 | human Neurogenic locus notch homolog protein 1-EGF2 protein | PRT897 |
| SEQ ID NO: 898 | human Neurogenic locus notch homolog protein 1-EGF20 protein | PRT898 |
| SEQ ID NO: 899 | human Neurogenic locus notch homolog protein 1-EGF21 protein | PRT899 |
| SEQ ID NO: 900 | human Neurogenic locus notch homolog protein 1-EGF22 protein | PRT900 |
| SEQ ID NO: 901 | human Neurogenic locus notch homolog protein 1-EGF23 protein | PRT901 |
| SEQ ID NO: 902 | human Neurogenic locus notch homolog protein 1-EGF24 protein | PRT902 |
| SEQ ID NO: 903 | human Neurogenic locus notch homolog protein 1-EGF25 protein | PRT903 |
| SEQ ID NO: 904 | human Neurogenic locus notch homolog protein 1-EGF26 protein | PRT904 |
| SEQ ID NO: 905 | human Neurogenic locus notch homolog protein 1-EGF27 protein | PRT905 |
| SEQ ID NO: 906 | human Neurogenic locus notch homolog protein 1-EGF28 protein | PRT906 |
| SEQ ID NO: 907 | human Neurogenic locus notch homolog protein 1-EGF29 protein | PRT907 |
| SEQ ID NO: 908 | human Neurogenic locus notch homolog protein 1-EGF3 protein | PRT908 |
| SEQ ID NO: 909 | human Neurogenic locus notch homolog protein 1-EGF30 protein | PRT909 |
| SEQ ID NO: 910 | human Neurogenic locus notch homolog protein 1-EGF31 protein | PRT910 |
| SEQ ID NO: 911 | human Neurogenic locus notch homolog protein 1-EGF32 protein | PRT911 |
| SEQ ID NO: 912 | human Neurogenic locus notch homolog protein 1-EGF33 protein | PRT912 |
| SEQ ID NO: 913 | human Neurogenic locus notch homolog protein 1-EGF34 protein | PRT913 |
| SEQ ID NO: 914 | human Neurogenic locus notch homolog protein 1-EGF35 protein | PRT914 |
| SEQ ID NO: 915 | human Neurogenic locus notch homolog protein 1-EGF36 protein | PRT915 |
| SEQ ID NO: 916 | human Neurogenic locus notch homolog protein I-EGF4 protein | PRT916 |
| SEQ ID NO: 917 | human Neurogenic locus notch homolog protein 1-EGF5 protein | PRT917 |
| SEQ ID NO: 918 | human Neurogenic locus notch homolog protein I-EGF6 protein | PRT918 |
| SEQ ID NO: 919 | human Neurogenic locus notch homolog protein 1-EGF7 protein | PRT919 |
| SEQ ID NO: 920 | human Neurogenic locus notch homolog protein 1-EGF8 protein | PRT920 |
| SEQ ID NO: 921 | human Neurogenic locus notch homolog protein 1-EGF9 protein | PRT921 |
| SEQ ID NO: 922 | human Neurogenic locus notch homolog protein 2-EGFI protein | PRT922 |
| SEQ ID NO: 923 | human Neurogenic locus notch homolog protein 2-EGF10 protein | PRT923 |
| SEQ ID NO: 924 | human Neurogenic locus notch homolog protein 2-EGF11 | PRT924 |
| | protein | |
| SEQ ID NO: 925 | human Neurogenic locus notch homolog protein 2-EGF12 protein | PRT925 |
| SEQ ID NO: 926 | human Neurogenic locus notch homolog protein 2-EGF13 protein | PRT926 |
| SEQ ID NO: 927 | human Neurogenic locus notch homolog protein 2-EGF14 protein | PRT927 |
| SEQ ID NO: 928 | human Neurogenic locus notch homolog protein 2-EGF15 protein | PRT928 |
| SEQ ID NO: 929 | human Neurogenic locus notch homolog protein 2-EGF16 protein | PRT929 |
| SEQ ID NO: 930 | human Neurogenic locus notch homolog protein 2-EGF17 protein | PRT930 |
| SEQ ID NO: 931 | human Neurogenic locus notch homolog protein 2-EGF18 protein | PRT931 |
| SEQ ID NO: 932 | human Neurogenic locus notch homolog protein 2-EGF19 protein | PRT932 |
| SEQ ID NO: 933 | human Neurogenic locus notch homolog protein 2-EGF2 protein | PRT933 |
| SEQ ID NO: 934 | human Neurogenic locus notch homolog protein 2-EGF20 protein | PRT934 |
| SEQ ID NO: 935 | human Neurogenic locus notch homolog protein 2-EGF21 protein | PRT935 |
| SEQ ID NO: 936 | human Neurogenic locus notch homolog protein 2-EGF22 protein | PRT936 |
| SEQ ID NO: 937 | human Neurogenic locus notch homolog protein 2-EGF23 protein | PRT937 |
| SEQ ID NO: 938 | human Neurogenic locus notch homolog protein 2-EGF24 protein | PRT938 |
| SEQ ID NO: 939 | human Neurogenic locus notch homolog protein 2-EGF25 protein | PRT939 |
| SEQ ID NO: 940 | human Neurogenic locus notch homolog protein 2-EGF26 protein | PRT940 |
| SEQ ID NO: 941 | human Neurogenic locus notch homolog protein 2-EGF27 protein | PRT941 |
| SEQ ID NO: 942 | human Neurogenic locus notch homolog protein 2-EGF28 protein | PRT942 |
| SEQ ID NO: 943 | human Neurogenic locus notch homolog protein 2-EGF29 protein | PRT943 |
| SEQ ID NO: 944 | human Neurogenic locus notch homolog protein 2-EGF3 protein | PRT944 |
| SEQ ID NO: 945 | human Neurogenic locus notch homolog protein 2-EGF30 protein | PRT945 |
| SEQ ID NO: 946 | human Neurogenic locus notch homolog protein 2-EGF31 protein | PRT946 |
| SEQ ID NO: 947 | human Neurogenic locus notch homolog protein 2-EGF32 protein | PRT947 |
| SEQ ID NO: 948 | human Neurogenic locus notch homolog protein 2-EGF33 protein | PRT948 |
| SEQ ID NO: 949 | human Neurogenic locus notch homolog protein 2-EGF34 protein | PRT949 |
| SEQ ID NO: 950 | human Neurogenic locus notch homolog protein 2-EGF35 protein | PRT950 |
| SEQ ID NO: 951 | human Neurogenic locus notch homolog protein 2-EGF4 protein | PRT951 |
| SEQ ID NO: 952 | human Neurogenic locus notch homolog protein 2-EGF5 protein | PRT952 |
| SEQ ID NO: 953 | human Neurogenic locus notch homolog protein 2-EGF6 | PRT953 |
| | protein | |
| SEQ ID NO: 954 | human Neurogenic locus notch homolog protein 2-EGF7 protein | PRT954 |
| SEQ ID NO: 955 | human Neurogenic locus notch homolog protein 2-EGF8 protein | PRT955 |
| SEQ ID NO: 956 | human Neurogenic locus notch homolog protein 2-EGF9 protein | PRT956 |
| SEQ ID NO: 957 | human Neurogenic locus notch homolog protein 3-EGFI protein | PRT957 |
| SEQ ID NO: 958 | human Neurogenic locus notch homolog protein 3-EGF10 protein | PRT958 |
| SEQ ID NO: 959 | human Neurogenic locus notch homolog protein 3-EGF11 protein | PRT959 |
| SEQ ID NO: 960 | human Neurogenic locus notch homolog protein 3-EGF12 protein | PRT960 |
| SEQ ID NO: 961 | human Neurogenic locus notch homolog protein 3-EGF13 protein | PRT961 |
| SEQ ID NO: 962 | human Neurogenic locus notch homolog protein 3-EGF14 protein | PRT962 |
| SEQ ID NO: 963 | human Neurogenic locus notch homolog protein 3-EGF15 protein | PRT963 |
| SEQ ID NO: 964 | human Neurogenic locus notch homolog protein 3-EGF16 protein | PRT964 |
| SEQ ID NO: 965 | human Neurogenic locus notch homolog protein 3-EGF17 protein | PRT965 |
| SEQ ID NO: 966 | human Neurogenic locus notch homolog protein 3-EGF18 protein | PRT966 |
| SEQ ID NO: 967 | human Neurogenic locus notch homolog protein 3-EGF19 protein | PRT967 |
| SEQ ID NO: 968 | human Neurogenic locus notch homolog protein 3-EGF2 protein | PRT968 |
| SEQ ID NO: 969 | human Neurogenic locus notch homolog protein 3-EGF20 protein | PRT969 |
| SEQ ID NO: 970 | human Neurogenic locus notch homolog protein 3-EGF21 protein | PRT970 |
| SEQ ID NO: 971 | human Neurogenic locus notch homolog protein 3-EGF22 protein | PRT971 |
| SEQ ID NO: 972 | human Neurogenic locus notch homolog protein 3-EGF23 protein | PRT972 |
| SEQ ID NO: 973 | human Neurogenic locus notch homolog protein 3-EGF24 protein | PRT973 |
| SEQ ID NO: 974 | human Neurogenic locus notch homolog protein 3-EGF25 protein | PRT974 |
| SEQ ID NO: 975 | human Neurogenic locus notch homolog protein 3-EGF26 protein | PRT975 |
| SEQ ID NO: 976 | human Neurogenic locus notch homolog protein 3-EGF27 protein | PRT976 |
| SEQ ID NO: 977 | human Neurogenic locus notch homolog protein 3-EGF28 protein | PRT977 |
| SEQ ID NO: 978 | human Neurogenic locus notch homolog protein 3-EGF29 protein | PRT978 |
| SEQ ID NO: 979 | human Neurogenic locus notch homolog protein 3-EGF3 protein | PRT979 |
| SEQ ID NO: 980 | human Neurogenic locus notch homolog protein 3-EGF30 protein | PRT980 |
| SEQ ID NO: 981 | human Neurogenic locus notch homolog protein 3-EGF31 protein | PRT981 |
| SEQ ID NO: 982 | human Neurogenic locus notch homolog protein 3-EGF32 | PRT982 |
| | protein | |
| SEQ ID NO: 983 | human Neurogenic locus notch homolog protein 3-EGF33 protein | PRT983 |
| SEQ ID NO: 984 | human Neurogenic locus notch homolog protein 3-EGF34 protein | PRT984 |
| SEQ ID NO: 985 | human Neurogenic locus notch homolog protein 3-EGF4 protein | PRT985 |
| SEQ ID NO: 986 | human Neurogenic locus notch homolog protein 3-EGF5 protein | PRT986 |
| SEQ ID NO: 987 | human Neurogenic locus notch homolog protein 3-EGF6 protein | PRT987 |
| SEQ ID NO: 988 | human Neurogenic locus notch homolog protein 3-EGF7 988 protein | PRT988 |
| SEQ ID NO: 989 | human Neurogenic locus notch homolog protein 3-EGF8 protein | PRT989 |
| SEQ ID NO: 990 | human Neurogenic locus notch homolog protein 3-EGF9 protein | PRT990 |
| SEQ ID NO: 991 | human Neurogenic locus notch homolog protein 4-EGFI protein | PRT991 |
| SEQ ID NO: 992 | human Neurogenic locus notch homolog protein 4-EGF10 protein | PRT992 |
| SEQ ID NO: 993 | human Neurogenic locus notch homolog protein 4-EGF11 protein | PRT993 |
| SEQ ID NO: 994 | human Neurogenic locus notch homolog protein 4-EGF12 protein | PRT994 |
| SEQ ID NO: 995 | human Neurogenic locus notch homolog protein 4-EGF13 protein | PRT995 |
| SEQ ID NO: 996 | human Neurogenic locus notch homolog protein 4-EGF14 protein | PRT996 |
| SEQ ID NO: 997 | human Neurogenic locus notch homolog protein 4-EGF15 protein | PRT997 |
| SEQ ID NO: 998 | human Neurogenic locus notch homolog protein 4-EGF16 protein | PRT998 |
| SEQ ID NO: 999 | human Neurogenic locus notch homolog protein 4-EGF17 protein | PRT999 |
| SEQ ID NO: 1000 | human Neurogenic locus notch homolog protein 4-EGF18 protein | PRT1000 |
| SEQ ID NO: 1001 | human Neurogenic locus notch homolog protein 4-EGF19 protein | PRT1001 |
| SEQ ID NO: 1002 | human Neurogenic locus notch homolog protein 4-EGF2 protein | PRT1002 |
| SEQ ID NO: 1003 | human Neurogenic locus notch homolog protein 4-EGF20 protein | PRT 1003 |
| SEQ ID NO: 1004 | human Neurogenic locus notch homolog protein 4-EGF21 protein | PRT 1004 |
| SEQ ID NO: 1005 | human Neurogenic locus notch homolog protein 4-EGF22 protein | PRT 1005 |
| SEQ ID NO: 1006 | human Neurogenic locus notch homolog protein 4-EGF23 protein | PRT1006 |
| SEQ ID NO: 1007 | human Neurogenic locus notch homolog protein 4-EGF24 protein | PRT 1007 |
| SEQ ID NO: 1008 | human Neurogenic locus notch homolog protein 4-EGF25 protein | PRT1008 |
| SEQ ID NO: 1009 | human Neurogenic locus notch homolog protein 4-EGF26 protein | PRT1009 |
| SEQ ID NO: 1010 | human Neurogenic locus notch homolog protein 4-EGF27 protein | PRT1010 |
| SEQ ID NO: 1011 | human Neurogenic locus notch homolog protein 4-EGF28 | PRT1011 |
| | protein | |
| SEQ ID NO: 1012 | human Neurogenic locus notch homolog protein 4-EGF29 protein | PRT1012 |
| SEQ ID NO: 1013 | human Neurogenic locus notch homolog protein 4-EGF3 protein | PRT1013 |
| SEQ ID NO: 1014 | human Neurogenic locus notch homolog protein 4-EGF4 protein | PRT1014 |
| SEQ ID NO: 1015 | human Neurogenic locus notch homolog protein 4-EGF5 protein | PRT1015 |
| SEQ ID NO: 1016 | human Neurogenic locus notch homolog protein 4-EGF6 protein | PRT1016 |
| SEQ ID NO: 1017 | human Neurogenic locus notch homolog protein 4-EGF7 protein | PRT1017 |
| SEQ ID NO: 1018 | human Neurogenic locus notch homolog protein 4-EGF8 protein | PRT1018 |
| SEQ ID NO: 1019 | human Neurogenic locus notch homolog protein 4-EGF9 protein | PRT1019 |
| SEQ ID NO: 1020 | human Nidogen-1-EGF1 protein | PRT020 |
| SEQ ID NO: 1021 | human Nidogen-1-EGF2 protein | PRT1021 |
| SEQ ID NO: 1022 | human Nidogen-1-EGF3 protein | PRT1022 |
| SEQ ID NO: 1023 | human Nidogen-1-EGF4 protein | PRT1023 |
| SEQ ID NO: 1024 | human Nidogen-1-EGF5 protein | PRT1024 |
| SEQ ID NO: 1025 | human Nidogen-1-EGF6 protein | PRT1025 |
| SEQ ID NO: 1026 | human Nidogen-2-EGF1 protein | PRT1026 |
| SEQ ID NO: 1027 | human Nidogen-2-EGF2 protein | PRT1027 |
| SEQ ID NO: 1028 | human Nidogen-2-EGF3 protein | PRT1028 |
| SEQ ID NO: 1029 | human Nidogen-2-EGF4 protein | PRT1029 |
| SEQ ID NO: 1030 | human Nidogen-2-EGF5 protein | PRT1030 |
| SEQ ID NO: 1031 | human Notch homolog 2 N-terminal-like protein-EGF1 | PRT1031 |
| | protein | |
| SEQ ID NO: 1032 | human Notch homolog 2 N-terminal-like protein-EGF2 protein | PRT1032 |
| SEQ ID NO: 1033 | human Notch homolog 2 N-terminal-like protein-EGF3 protein | PRT1033 |
| SEQ ID NO: 1034 | human Oncoprotein-induced transcript 3 protein-EGFI protein | PRT1034 |
| SEQ ID NO: 1035 | human Otogelin-EGF1 protein | PRT 1035 |
| SEQ ID NO: 1036 | human Pancreatic secretory granule membrane major glycoprotein GP2-EGFI protein | PRT1036 |
| SEQ ID NO: 1037 | human Perforin-1-EGF1 protein | PRT1037 |
| SEQ ID NO: 1038 | human Pikachurin-EGF1 protein | PRT1038 |
| SEQ ID NO: 1039 | human Pikachurin-EGF2 protein | PRT 1039 |
| SEQ ID NO: 1040 | human Pikachurin-EGF3 protein | PRT1040 |
| SEQ ID NO: 1041 | human Platelet endothelial aggregation receptor 1-EGF1 protein | PRT1041 |
| SEQ ID NO: 1042 | human Platelet endothelial aggregation receptor 1-EGF2 protein | PRT 1042 |
| SEQ ID NO: 1043 | human Platelet endothelial aggregation receptor 1-EGF3 protein | PRT 1043 |
| SEQ ID NO: 1044 | human Platelet endothelial aggregation receptor 1-EGF4 protein | PRT1044 |
| SEQ ID NO: 1045 | human Platelet endothelial aggregation receptor I-EGF5 protein | PRT1045 |
| SEQ ID NO: 1046 | human Platelet endothelial aggregation receptor I-EGF6 protein | PRT1046 |
| SEQ ID NO: 1047 | human Platelet endothelial aggregation receptor I-EGF7 protein | PRT 1047 |
| SEQ ID NO: 1048 | human Platelet endothelial aggregation receptor 1-EGF8 | PRT1048 |
| | protein | |
| SEQ ID NO: 1049 | human Platelet endothelial aggregation receptor 1-EGF9 protein | PRT1049 |
| SEQ ID NO: 1050 | human Probetacellulin-EGF1 protein | PRT1050 |
| SEQ ID NO: 1051 | human Pro-epidermal growth factor-EGF1 protein | PRTI051 |
| SEQ ID NO: 1052 | human Pro-epidermal growth factor-EGF2 protein | PRT1052 |
| SEQ ID NO: 1053 | human Pro-epidermal growth factor-EGF3 protein | PRT1053 |
| SEQ ID NO: 1054 | human Pro-epidermal growth factor-EGF4 protein | PRT1054 |
| SEQ ID NO: 1055 | human Pro-epidermal growth factor-EGF5 protein | PRT1055 |
| SEQ ID NO: 1056 | human Pro-epidermal growth factor-EGF6 protein | PRT1056 |
| SEQ ID NO: 1057 | human Pro-epidermal growth factor-EGF7 protein | PRT1057 |
| SEQ ID NO: 1058 | human Pro-epidermal growth factor-EGF8 protein | PRT1058 |
| SEQ ID NO: 1059 | human Pro-epidermal growth factor-EGF9 protein | PRT1059 |
| SEQ ID NO: 1060 | human Proepiregulin-EGF1 protein | PRT1060 |
| SEQ ID NO: 1061 | human Proheparin-binding EGF-like growth factor-EGF1 protein | PRT1061 |
| SEQ ID NO: 1062 | human Prolow-density lipoprotein receptor-related protein I-EGF1 protein | PRT062 |
| SEQ ID NO: 1063 | human Prolow-density lipoprotein receptor-related protein I-EGF10 protein | PRT063 |
| SEQ ID NO: 1064 | human Prolow-density lipoprotein receptor-related protein 1-EGF11 protein | PRT 1064 |
| SEQ ID NO: 1065 | human Prolow-density lipoprotein receptor-related protein 1-EGF12 protein | PRT1 065 |
| SEQ ID NO: 1066 | human Prolow-density lipoprotein receptor-related protein 1-EGF13 protein | PRT1 066 |
| SEQ ID NO: 1067 | human Prolow-density lipoprotein receptor-related protein 1-EGF14 protein | PRT1067 |
| SEQ ID NO: 1068 | human Prolow-density lipoprotein receptor-related protein | PRT1068 |
| | 1-EGF15 protein | |
| SEQ ID NO: 1069 | human Prolow-density lipoprotein receptor-related protein 1-EGF16 protein | PRT1069 |
| SEQ ID NO: 1070 | human Prolow-density lipoprotein receptor-related protein 1-EGF17 protein | PRT1070 |
| SEQ ID NO: 1071 | human Prolow-density lipoprotein receptor-related protein 1-EGF18 protein | PRT1071 |
| SEQ ID NO: 1072 | human Prolow-density lipoprotein receptor-related protein 1-EGF19 protein | PRT1072 |
| SEQ ID NO: 1073 | human Prolow-density lipoprotein receptor-related protein I-EGF2 protein | PRT1073 |
| SEQ ID NO: 1074 | human Prolow-density lipoprotein receptor-related protein I-EGF20 protein | PRT1074 |
| SEQ ID NO: 1075 | human Prolow-dcnsity lipoprotein receptor-related protein 1-EGF21 protein | PRT1075 |
| SEQ ID NO: 1076 | human Prolow-density lipoprotein receptor-related protein 1-EGF22 protein | PRT1076 |
| SEQ ID NO: 1077 | human Prolow-density lipoprotein receptor-related protein 1-EGF3 protein | PRT1077 |
| SEQ ID NO: 1078 | human Prolow-density lipoprotein receptor-related protein 1-EGF4 protein | PRT1078 |
| SEQ ID NO: 1079 | human Prolow-density lipoprotein receptor-related protein I-EGF5 protein | PRT 1079 |
| SEQ ID NO: 1080 | human Prolow-density lipoprotein receptor-related protein 1-EGF6 protein | PRT1080 |
| SEQ ID NO: 1081 | human Prolow-density lipoprotein receptor-related protein I-EGF7 protein | PRT1081 |
| SEQ ID NO: 1082 | human Prolow-density lipoprotein receptor-related protein I-EGF8 protein | PRT1082 |
| SEQ ID NO: 1083 | human Prolow-density lipoprotein receptor-related protein 1-EGF9 protein | PRT1083 |
| SEQ ID NO: 1084 | human Pro-neuregulin-1, membrane-bound isoform-EGF1 protein | PRT1084 |
| SEQ ID NO: 1085 | human Pro-neuregulin-2, membrane-bound isoform-EGF1 protein | PRT1085 |
| SEQ ID NO: 1086 | human Pro-neuregulin-3, membrane-bound isoform-EGF1 protein | PRT1086 |
| SEQ ID NO: 1087 | human Pro-neuregulin-4, membrane-bound isoform-EGFI protein | PRT1087 |
| SEQ ID NO: 1088 | human Prostaglandin G/H synthase 1-EGF1 protein | PRT1088 |
| SEQ ID NO: 1089 | human Prostaglandin G/H synthase 2-EGF1 protein | PRT1089 |
| SEQ ID NO: 1090 | human Protein crumbs homolog I-EGF10 protein | PRT1090 |
| SEQ ID NO: 1091 | human Protein crumbs homolog I-EGF11 protein | PRT1091 |
| SEQ ID NO: 1092 | human Protein crumbs homolog 1-EGF12 protein | PRT1092 |
| SEQ ID NO: 1093 | human Protein crumbs homolog 1-EGF13 protein | PRT1093 |
| SEQ ID NO: 1094 | human Protein crumbs homolog 1-EGF14 protein | PRT 1094 |
| SEQ ID NO: 1095 | human Protein crumbs homolog 1-EGF15 protein | PRT1095 |
| SEQ ID NO: 1096 | human Protein crumbs homolog 1-EGF16 protein | PRT1096 |
| SEQ ID NO: 1097 | human Protein crumbs homolog 1-EGF17 protein | PRT1097 |
| SEQ ID NO: 1098 | human Protein crumbs homolog 1-EGF18 protein | PRT1098 |
| SEQ ID NO: 1099 | human Protein crumbs homolog 1-EGF19 protein | PRT1099 |
| SEQ ID NO: 1100 | human Protein crumbs homolog 1-EGF20 protein | PRT1100 |
| SEQ ID NO: 1101 | human Protein crumbs homolog 1-EGF2 protein | PRT1101 |
| SEQ ID NO: 1102 | human Protein crumbs homolog 1-EGF3 protein | PRT1102 |
| SEQ ID NO: 1103 | human Protein crumbs homolog I-EGF4 protein | PRT1103 |
| SEQ ID NO: 1104 | human Protein crumbs homolog I-EGF5 protein | PRT1104 |
| SEQ ID NO: 1105 | human Protein crumbs homolog 1-EGF6 protein | PRT1105 |
| SEQ ID NO: 1106 | human Protein crumbs homolog I-EGF7 protein | PRT1106 |
| SEQ ID NO: 1107 | human Protein crumbs homolog 1-EGF8 protein | PRT1107 |
| SEQ ID NO: 1108 | human Protein crumbs homolog 1-EGF9 protein | PRT1108 |
| SEQ ID NO: 1109 | human Protein crumbs homolog 2-EGF1 protein | PRT1109 |
| SEQ ID NO: 1110 | human Protein crumbs homolog 2-EGF10 protein | PRT1110 |
| SEQ ID NO: 1111 | human Protein crumbs homolog 2-EGF11 protein | PRT1111 |
| SEQ ID NO: 1112 | human Protein crumbs homolog 2-EGF12 protein | PRT1112 |
| SEQ ID NO: 1113 | human Protein crumbs homolog 2-EGF13 protein | PRT1113 |
| SEQ ID NO: 1114 | human Protein crumbs homolog 2-EGF14 protein | PRT1114 |
| SEQ ID NO: 11 15 | human Protein crumbs homolog 2-EGF15 protein | PRT1115 |
| SEQ ID NO: 1116 | human Protein crumbs homolog 2-EGF2 protein | PRT1116 |
| SEQ ID NO: 1117 | human Protein crumbs homolog 2-EGF3 protein | PRT1117 |
| SEQ ID NO: 1118 | human Protein crumbs homolog 2-EGF4 protein | PRT1118 |
| SEQ ID NO: 1119 | human Protein crumbs homolog 2-EGF5 protein | PRT1119 |
| SEQ ID NO: 1120 | human Protein crumbs homolog 2-EGF6 protein | PRT1120 |
| SEQ ID NO: 1121 | human Protein crumbs homolog 2-EGF7 protein | PRT1121 |
| SEQ ID NO: 1122 | human Protein crumbs homolog 2-EGF8 protein | PRT1122 |
| SEQ ID NO: 1123 | human Protein crumbs homolog 2-EGF9 protein | PRT1123 |
| SEQ ID NO: 1124 | human Protein delta homolog 1-EGF1 protein | PRT1124 |
| SEQ ID NO: 1125 | human Protein delta homolog I-EGF2 protein | PRT1125 |
| SEQ ID NO: 1126 | human Protein delta homolog I-EGF3 protein | PRT 1126 |
| SEQ ID NO: 1127 | human Protein delta homolog I-EGF4 protein | PRT1127 |
| SEQ ID NO: 1128 | human Protein delta homolog I-EGF5 protein | PRT1128 |
| SEQ ID NO: 1129 | human Protein delta homolog I-EGF6 protein | PRT1129 |
| SEQ ID NO: 1130 | human Protein delta homolog 2-EGF1 protein | PRT1130 |
| SEQ ID NO: 1131 | human Protein delta homolog 2-EGF2 protein | PRT1131 |
| SEQ ID NO: 1132 | human Protein delta homolog 2-EGF3 protein | PRT1132 |
| SEQ ID NO: 1133 | human Protein delta homolog 2-EGF4 protein | PRT1133 |
| SEQ ID NO: 1134 | human Protein delta homolog 2-EGF5 protein | PRT1134 |
| SEQ ID NO: 1135 | human Protein delta homolog 2-EGF6 protein | PRT1135 |
| SEQ ID NO: 1136 | human Protein eyes shut homolog-EGF1 protein | PRT1136 |
| SEQ ID NO: 1137 | human Protein eyes shut homolog-EGF10 protein | PRT1137 |
| SEQ ID NO: 1138 | human Protein eyes shut homolog-EGF11 protein | PRT1138 |
| SEQ ID NO: 1139 | human Protein eyes shut homolog-EGF12 protein | PRT1139 |
| SEQ ID NO: 1140 | human Protein eyes shut homolog-EGF13 protein | PRT1140 |
| SEQ ID NO: 1141 | human Protein eyes shut homolog-EGF14 protein | PRT1141 |
| SEQ ID NO: 1142 | human Protein eyes shut homolog-EGF15 protein | PRT1142 |
| SEQ ID NO: 1143 | human Protein eyes shut homolog-EGF16 protein | PRT1143 |
| SEQ ID NO: 1144 | human Protein eyes shut homolog-EGF17 protein | PRT1144 |
| SEQ ID NO: 1145 | human Protein eyes shut homolog-EGF18 protein | PRT1145 |
| SEQ ID NO: 1146 | human Protein eyes shut homolog-EGF19 protein | PRT1146 |
| SEQ ID NO: 1147 | human Protein eyes shut homolog-EGF2 protein | PRT1147 |
| SEQ ID NO: 1148 | human Protein eyes shut homolog-EGF20 protein | PRT1148 |
| SEQ ID NO: 1149 | human Protein eyes shut homolog-EGF21 protein | PRT1149 |
| SEQ ID NO: 1150 | human Protein eyes shut homolog-EGF22 protein | PRT1150 |
| SEQ ID NO: 1151 | human Protein eyes shut homolog-EGF23 protein | PRT1151 |
| SEQ ID NO: 1152 | human Protein eyes shut homolog-EGF24 protein | PRT1152 |
| SEQ ID NO: 1153 | human Protein eyes shut homolog-EGF25 protein | PRT1153 |
| SEQ ID NO: 1154 | human Protein eyes shut homolog-EGF26 protein | PRT1154 |
| SEQ ID NO: 1155 | human Protein eyes shut homolog-EGF27 protein | PRT1155 |
| SEQ ID NO: 1156 | human Protein eyes shut homolog-EGF3 protein | PRT1156 |
| SEQ ID NO: 1157 | human Protein eyes shut homolog-EGF4 protein | PRT1157 |
| SEQ ID NO: 1158 | human Protein eyes shut homolog-EGF5 protein | PRT1158 |
| SEQ ID NO: 1159 | human Protein eyes shut homolog-EGF6 protein | PRT1159 |
| SEQ ID NO: 1160 | human Protein eyes shut homolog-EGF7 protein | PRT1160 |
| SEQ ID NO: 1161 | human Protein eyes shut homolog-EGF8 protein | PRT161 |
| SEQ ID NO: 1162 | human Protein eyes shut homolog-EGF9 protein | PRT1162 |
| SEQ ID NO: 1163 | human Protein HEG homolog 1-EGF1 protein | PRT1163 |
| SEQ ID NO: 1164 | human Protein HEG homolog 1-EGF2 protein | PRT1164 |
| SEQ ID NO: 1165 | human Protein jagged-1-EGF1 protein | PRT1165 |
| SEQ ID NO: 1166 | human Protein jagged-1-EGF10 protein | PRT1166 |
| SEQ ID NO: 1167 | human Protein jagged-1-EGF1 1 protein | PRT 1167 |
| SEQ ID NO: 1168 | human Protein jagged-1-EGF12 protein | PRT1168 |
| SEQ ID NO: 1169 | human Protein jagged-1-EGF13 protein | PRT1169 |
| SEQ ID NO: 1170 | human Protein jagged-1-EGF14 protein | PRT1170 |
| SEQ ID NO: 1171 | human Protein jagged-1-EGF15 protein | PRT1171 |
| SEQ ID NO: 1172 | human Protein jagged-1-EGF16 protein | PRT1172 |
| SEQ ID NO: 1173 | human Protein jagged-1-EGF2 protein | PRT1173 |
| SEQ ID NO: 1174 | human Protein jagged-1-EGF3 protein | PRT1174 |
| SEQ ID NO: 1175 | human Protein jagged-1-EGF4 protein | PRT1175 |
| SEQ ID NO: 1176 | human Protein jagged-1-EGF5 protein | PRT1176 |
| SEQ ID NO: 1177 | human Protein jagged-1-EGF6 protein | PRT1177 |
| SEQ ID NO: 1178 | human Protein jagged-1-EGF7 protein | PRT 1178 |
| SEQ ID NO: 1179 | human Protein jagged-1-EGF8 protein | PRT1179 |
| SEQ ID NO: 1180 | human Protein jagged-1-EGF9 protein | PRT1180 |
| SEQ ID NO: 1181 | human Protein jagged-2-EGF1 protein | PRT1181 |
| SEQ ID NO: 1182 | human Protein jagged-2-EGF10 protein | PRT1182 |
| SEQ ID NO: 1183 | human Protein jagged-2-EGF11 protein | PRT1183 |
| SEQ ID NO: 1184 | human Protein jagged-2-EGF12 protein | PRT1184 |
| SEQ ID NO: 1185 | human Protein jagged-2-EGF13 protein | PRT1185 |
| SEQ ID NO: 1186 | human Protein jagged-2-EGF14 protein | PRT1186 |
| SEQ ID NO: 1187 | human Protein jagged-2-EGF15 protein | PRT1187 |
| SEQ ID NO: 1188 | human Protein jagged-2-EGF16 protein | PRT1188 |
| SEQ ID NO: 1189 | human Protein jagged-2-EGF2 protein | PRT1189 |
| SEQ ID NO: 1190 | human Protein jagged-2-EGF3 protein | PRT 1190 |
| SEQ ID NO: 1191 | human Protein jagged-2-EGF4 protein | PRT1191 |
| SEQ ID NO: 1192 | human Protein jagged-2-EGF5 protein | PRT1192 |
| SEQ ID NO: 1193 | human Protein jagged-2-EGF6 protein | PRT 1193 |
| SEQ ID NO: 1194 | human Protein jagged-2-EGF7 protein | PRT1194 |
| SEQ ID NO: 1195 | human Protein jagged-2-EGF8 protein | PRT1195 |
| SEQ ID NO: 1196 | human Protein jagged-2-EGF9 protein | PRT1196 |
| SEQ ID NO: 1197 | human Protein kinase C-binding protein NELL1-EGF1 protein | PRT1197 |
| SEQ ID NO: 1198 | human Protein kinase C-binding protein NELL1-EGF2 protein | PRT1198 |
| SEQ ID NO: 1199 | human Protein kinase C-binding protein NELL1-EGF3 protein | PRT1199 |
| SEQ ID NO: 1200 | human Protein kinase C-binding protein NELL1-EGF4 protein | PRT1200 |
| SEQ ID NO: 1201 | human Protein kinase C-binding protein NELL1-EGF5 protein | PRT1201 |
| SEQ ID NO: 1202 | human Protein kinase C-binding protein NELL2-EGF1 protein | PRT1202 |
| SEQ ID NO: 1203 | human Protein kinase C-binding protein NELL2-EGF2 protein | PRT 1203 |
| SEQ ID NO: 1204 | human Protein kinase C-binding protein NELL2-EGF3 protein | PRT 1204 |
| SEQ ID NO: 1205 | human Protein kinase C-binding protein NELL2-EGF4 protein | PRT 1205 |
| SEQ ID NO: 1206 | human Protein kinase C-binding protein NELL2-EGF5 protein | PRT1206 |
| SEQ ID NO: 1207 | human Protein kinase C-binding protein NELL2-EGF6 protein | PRT1207 |
| SEQ ID NO: 1208 | human Protocadherin Fat 1-EGF1 protein | PRT1208 |
| SEQ ID NO: 1209 | human Protocadherin Fat 1-EGF2 protein | PRT1209 |
| SEQ ID NO: 1210 | human Protocadherin Fat 1-EGF3 protein | PRT1210 |
| SEQ ID NO: 1211 | human Protocadherin Fat 1-EGF4 protein | PRT1211 |
| SEQ ID NO: 1212 | human Protocadherin Fat 1-EGF5 protein | PRT1212 |
| SEQ ID NO: 1213 | human Protocadherin Fat 2-EGF1 protein | PRT1213 |
| SEQ ID NO: 1214 | human Protocadherin Fat 2-EGF2 protein | PRT1214 |
| SEQ ID NO: 1215 | human Protocadherin Fat 3-EGF1 protein | PRT1215 |
| SEQ ID NO: 1216 | human Protocadherin Fat 3-EGF2 protein | PRT1216 |
| SEQ ID NO: 1217 | human Protocadherin Fat 3-EGF3 protein | PRT1217 |
| SEQ ID NO: 1218 | human Protocadherin Fat 3-EGF4 protein | PRT1218 |
| SEQ ID NO: 1219 | human Protocadherin Fat 4-EGF1 protein | PRT1219 |
| SEQ ID NO: 1220 | human Protocadherin Fat 4-EGF2 protein | PRT1220 |
| SEQ ID NO: 1221 | human Protocadherin Fat 4-EGF3 protein | PRT1221 |
| SEQ ID NO: 1222 | human Protocadherin Fat 4-EGF4 protein | PRT1222 |
| SEQ ID NO: 1223 | human Protocadherin Fat 4-EGF5 protein | PRT1223 |
| SEQ ID NO: 1224 | human Protocadherin Fat 4-EGF6 protein | PRT1224 |
| SEQ ID NO: 1225 | human Protransforming growth factor alpha-EGF1 protein | PRT1225 |
| SEQ ID NO: 1226 | human P-selectin-EGF1 protein | PRT 1226 |
| SEQ ID NO: 1227 | human Putative disintegrin and metalloproteinase domain-containing protein 5-EGF1 protein | PRT 1227 |
| SEQ ID NO: 1228 | human Putative EGF-like module-containing mucin-like hormone receptor-like 4-EGF1 protein | PRT1228 |
| SEQ ID NO: 1229 | human Putative EGF-like module-containing mucin-like hormone receptor-like 4-EGF2 protein | PRT 1229 |
| SEQ ID NO: 1230 | human Putative teratocarcinoma-derived growth factor 3-EGF1 protein | PRT1230 |
| SEQ ID NO: 1231 | human Reelin-EGF1 protein | PRT1231 |
| SEQ ID NO: 1232 | human Reelin-EGF2 protein | PRT1232 |
| SEQ ID NO: 1233 | human Reelin-EGF3 protein | PRT1233 |
| SEQ ID NO: 1234 | human Reelin-EGF4 protein | PRT1234 |
| SEQ ID NO: 1235 | human Reelin-EGF5 protein | PRT1235 |
| SEQ ID NO: 1236 | human Reelin-EGF6 protein | PRT 1236 |
| SEQ ID NO: 1237 | human Reelin-EGF7 protein | PRT1237 |
| SEQ ID NO: 1238 | human Reelin-EGF8 protein | PRT1238 |
| SEQ ID NO: 1239 | human Scavenger receptor class F member 1-EGF1 protein | PRT 1239 |
| SEQ ID NO: 1240 | human Scavenger receptor class F member 1-EGF2 protein | PRT1240 |
| SEQ ID NO: 1241 | human Scavenger receptor class F member 1-EGF3 protein | PRT1241 |
| SEQ ID NO: 1242 | human Scavenger receptor class F member 1-EGF4 protein | PRT1242 |
| SEQ ID NO: 1243 | human Scavenger receptor class F member 1-EGF5 protein | PRT1243 |
| SEQ ID NO: 1244 | human Scavenger receptor class F member 1-EGF6 protein | PRT1244 |
| SEQ ID NO: 1245 | human Scavenger receptor class F member 2-EGF1 protein | PRT1245 |
| SEQ ID NO: 1246 | human Scavenger receptor class F member 2-EGF2 protein | PRT1246 |
| SEQ ID NO: 1247 | human Scavenger receptor class F member 2-EGF3 protein | PRT1247 |
| SEQ ID NO: 1248 | human Scavenger receptor class F member 2-EGF4 protein | PRT1248 |
| SEQ ID NO: 1249 | human Scavenger receptor class F member 2-EGF5 protein | PRT1249 |
| SEQ ID NO: 1250 | human Scavenger receptor class F member 2-EGF6 protein | PRT1250 |
| SEQ ID NO: 1251 | human Scavenger receptor class F member 2-EGF7 protein | PRT1251 |
| SEQ ID NO: 1252 | human SCO-spondin-EGF1 protein | PRT1252 |
| SEQ ID NO: 1253 | human SCO-spondin-EGF2 protein | PRT1253 |
| SEQ ID NO: 1254 | human Signal peptide, CUB and EGF-like domain-containing protein 1-EGF1 protein | PRT1254 |
| SEQ ID NO: 1255 | human Signal peptide, CUB and EGF-like domain-containing protein 1-EGF2 protein | PRT 1255 |
| SEQ ID NO: 1256 | human Signal peptide, CUB and EGF-like domain-containing protein 1-EGF3 protein | PRT1256 |
| SEQ ID NO: 1257 | human Signal peptide, CUB and EGF-like domain-containing protein 1-EGF4 protein | PRT1257 |
| SEQ ID NO: 1258 | human Signal peptide, CUB and EGF-like domain-containing protein 1-EGF5 protein | PRT1258 |
| SEQ ID NO: 1259 | human Signal peptide, CUB and EGF-like domain-containing protein 1-EGF6 protein | PRT1259 |
| SEQ ID NO: 1260 | human Signal peptide, CUB and EGF-like domain-containing protein 1-EGF7 protein | PRT1260 |
| SEQ ID NO: 1261 | human Signal peptide, CUB and EGF-like domain-containing protein 1-EGF8 protein | PRT1261 |
| SEQ ID NO: 1262 | human Signal peptide, CUB and EGF-like domain-containing protein 1-EGF9 protein | PRT1262 |
| SEQ ID NO: 1263 | human Signal peptide, CUB and EGF-like domain-containing protein 2-EGF1 protein | PRT 1263 |
| SEQ ID NO: 1264 | human Signal peptide, CUB and EGF-like domain-containing protein 2-EGF2 protein | PRT1264 |
| SEQ ID NO: 1265 | human Signal peptide, CUB and EGF-like domain-containing protein 2-EGF3 protein | PRT1265 |
| SEQ ID NO: 1266 | human Signal peptide, CUB and EGF-like domain-containing protein 2-EGF4 protein | PRT1266 |
| SEQ ID NO: 1267 | human Signal peptide, CUB and EGF-like domain-containing protein 2-EGF5 protein | PRT1267 |
| SEQ ID NO: 1268 | human Signal peptide, CUB and EGF-like domain-containing protein 2-EGF6 protein | PRT1268 |
| SEQ ID NO: 1269 | human Signal peptide, CUB and EGF-like domain-containing protein 2-EGF7 protein | PRT1269 |
| SEQ ID NO: 1270 | human Signal peptide, CUB and EGF-like domain-containing protein 2-EGF8 protein | PRT1270 |
| SEQ ID NO: 1271 | human Signal peptide, CUB and EGF-like domain-containing protein 2-EGF9 protein | PRT1271 |
| SEQ ID NO: 1272 | human Signal peptide, CUB and EGF-like domain-containing protein 3-EGF1 protein | PRT1272 |
| SEQ ID NO: 1273 | human Signal peptide, CUB and EGF-like domain-containing protein 3-EGF2 protein | PRT1273 |
| SEQ ID NO: 1274 | human Signal peptide, CUB and EGF-like | PRT1274 |
| | domain-containing protein 3-EGF3 protein | |
| SEQ ID NO: 1275 | human Signal peptide, CUB and EGF-like domain-containing protein 3-EGF4 protein | PRT1275 |
| SEQ ID NO: 1276 | human Signal peptide, CUB and EGF-like domain-containing protein 3-EGF5 protein | PRT1276 |
| SEQ ID NO: 1277 | human Signal peptide, CUB and EGF-like domain-containing protein 3-EGF6 protein | PRT1277 |
| SEQ ID NO: 1278 | human Signal peptide, CUB and EGF-like domain-containing protein 3-EGF7 protein | PRT1278 |
| SEQ ID NO: 1279 | human Signal peptide, CUB and EGF-like domain-containing protein 3-EGF8 protein | PRT1279 |
| SEQ ID NO: 1280 | human Signal peptide, CUB and EGF-like domain-containing protein 3-EGF9 protein | PRT1280 |
| SEQ ID NO: 1281 | human Slit homolog 1 protein-EGF1 protein | PRT1281 |
| SEQ ID NO: 1282 | human Slit homolog 1 protein-EGF2 protein | PRT1282 |
| SEQ ID NO: 1283 | human Slit homolog 1 protein-EGF3 protein | PRT1283 |
| SEQ ID NO: 1284 | human Slit homolog 1 protein-EGF4 protein | PRT1284 |
| SEQ ID NO: 1285 | human Slit homolog 1 protein-EGF5 protein | PRT 1285 |
| SEQ ID NO: 1286 | human Slit homolog 1 protein-EGF6 protein | PRT1286 |
| SEQ ID NO: 1287 | human Slit homolog 1 protein-EGF7 protein | PRT1287 |
| SEQ ID NO: 1288 | human Slit homolog 1 protein-EGF8 protein | PRT1288 |
| SEQ ID NO: 1289 | human Slit homolog 1 protein-EGF9 protein | PRT1289 |
| SEQ ID NO: 1290 | human Slit homolog 2 protein-EGF1 protein | PRT1290 |
| SEQ ID NO: 1291 | human Slit homolog 2 protein-EGF2 protein | PRT1291 |
| SEQ ID NO: 1292 | human Slit homolog 2 protein-EGF3 protein | PRT1292 |
| SEQ ID NO: 1293 | human Slit homolog 2 protein-EGF4 protein | PRT 1293 |
| SEQ ID NO: 1294 | human Slit homolog 2 protein-EGF5 protein | PRT1294 |
| SEQ ID NO: 1295 | human Slit homolog 2 protein-EGF6 protein | PRT1295 |
| SEQ ID NO: 1296 | human Slit homolog 2 protein-EGF7 protein | PRT 1296 |
| SEQ ID NO: 1297 | human Slit homolog 3 protein-EGF1 protein | PRT1297 |
| SEQ ID NO: 1298 | human Slit homolog 3 protein-EGF2 protein | PRT1298 |
| SEQ ID NO: 1299 | human Slit homolog 3 protein-EGF3 protein | PRT 1299 |
| SEQ ID NO: 1300 | human Slit homolog 3 protein-EGF4 protein | PRT1300 |
| SEQ ID NO: 1301 | human Slit homolog 3 protein-EGF5 protein | PRT1301 |
| SEQ ID NO: 1302 | human Slit homolog 3 proiein-EGF6 protein | PRT1302 |
| SEQ ID NO: 1303 | human Slit homolog 3 protein-EGF7 protein | PRT 1303 |
| SEQ ID NO: 1304 | human Slit homolog 3 protein-EGF8 protein | PRT 1304 |
| SEQ ID NO: 1305 | human Slit homolog 3 protein-EGF9 protein | PRT1305 |
| SEQ ID NO: 1306 | human Sortilin-related receptor-EGF1 protein | PRT1306 |
| SEQ ID NO: 1307 | human Stabilin-1-EGF1 protein | PRT 1307 |
| SEQ ID NO: 1308 | human Stabilin-1-EGF10 protein | PRT1308 |
| SEQ ID NO: 1309 | human Stabilin-1-EGF11 protein | PRT 1309 |
| SEQ ID NO: 1310 | human Stabilin-1-EGF12 protein | PRT1310 |
| SEQ ID NO: 1311 | human Stabilin-1-EGF13 protein | PRT1311 |
| SEQ ID NO: 1312 | human Stabilin-1-EGF14 protein | PRT1312 |
| SEQ ID NO: 1313 | human Stabilin-1-EGF15 protein | PRT1313 |
| SEQ ID NO: 1314 | human Stabilin-1-EGF16 protein | PRT1314 |
| SEQ ID NO: 1315 | human Stabilin-1-EGF2 protein | PRT1315 |
| SEQ ID NO: 1316 | human Stabilin-1-EGF3 protein | PRT1316 |
| SEQ ID NO: 1317 | human Stabilin-1-EGF4 protein | PRT1317 |
| SEQ ID NO: 1318 | human Stabilin-1-EGF5 protein | PRT1318 |
| SEQ ID NO: 1319 | human Stabilin-1-EGF6 protein | PRT1319 |
| SEQ ID NO: 1320 | human Stabilin-1-EGF7 protein | PRT1320 |
| SEQ ID NO: 1321 | human Stabilin-1-EGF8 protein | PRT1321 |
| SEQ ID NO: 1322 | human Stabilin-1-EGF9 protein | PRT1322 |
| SEQ ID NO: 1323 | human Stabilin-2-EGF1 protein | PRT1323 |
| SEQ ID NO: 1324 | human Stabilin-2-EGF10 protein | PRT 1324 |
| SEQ ID NO: 1325 | human Stabilin-2-EGF11 protein | PRT 1325 |
| SEQ ID NO: 1326 | human Stabilin-2-EGF12 protein | PRT1326 |
| SEQ ID NO: 1327 | human Stabilin-2-EGF13 protein | PRT1327 |
| SEQ ID NO: 1328 | human Stabilin-2-EGF14 protein | PRT1328 |
| SEQ ID NO: 1329 | human Stabilin-2-EGF15 protein | PRT1329 |
| SEQ ID NO: 1330 | human Stabilin-2-EGF16 protein | PRT1330 |
| SEQ ID NO: 1331 | human Stabilin-2-EGF17 protein | PRT1331 |
| SEQ ID NO: 1332 | human Stabilin-2-EGF2 protein | PRT1332 |
| SEQ ID NO: 1333 | human Stabilin-2-EGF3 protein | PRT1333 |
| SEQ ID NO: 1334 | human Stabilin-2-EGF4 protein | PRT1334 |
| SEQ ID NO: 1335 | human Stabilin-2-EGF5 protein | PRT1335 |
| SEQ ID NO: 1336 | human Stabilin-2-EGF6 protein | PRT1336 |
| SEQ ID NO: 1337 | human Stabilin-2-EGF7 protein | PRT1337 |
| SEQ ID NO: 1338 | human Stabilin-2-EGF8 protein | PRT1338 |
| SEQ ID NO: 1339 | human Stabilin-2-EGF9 protein | PRT1339 |
| SEQ ID NO: 1340 | human Sushi domain-containing protein 1-EGF1 protein | PRT1340 |
| SEQ ID NO: 1341 | human Sushi domain-containing protein 1-EGF2 protein | PRT1341 |
| SEQ ID NO: 1342 | human Sushi domain-containing protein 1-EGF3 protein | PRT 1342 |
| SEQ ID NO: 1343 | human Sushi, von Willebrand factor type A, EGF and pentraxin domain-containing protein 1-EGF1 protein | PRT1343 |
| SEQ ID NO: 1344 | human Sushi, von Willebrand factor type A, EGF and pentraxin domain-containing protein 1-EGF2 protein | PRT1344 |
| SEQ ID NO: 1345 | human Sushi, von Willebrand factor type A, EGF and pentraxin domain-containing protein I-EGF3 protein | PRT1345 |
| SEQ ID NO: 1346 | human Sushi, von Willebrand factor type A, EGF and pentraxin domain-containing protein 1-EGF4 protein | PRT1346 |
| SEQ ID NO: 1347 | human Sushi, von Willebrand factor type A, EGF and pentraxin domain-containing protein 1-EGF5 protein | PRT1347 |
| SEQ ID NO: 1348 | human Sushi, von Willebrand factor type A, EGF and pentraxin domain-containing protein 1-EGF6 protein | PRT1348 |
| SEQ ID NO: 1349 | human Sushi, von Willebrand factor type A, EGF and pentraxin domain-containing protein 1-EGF7 protein | PRT1349 |
| SEQ ill NO: 1350 | human Sushi, von Willebrand factor type A, EGF and pentraxin domain-containing protein 1-EGF8 protein | PRT 1350 |
| SEQ ID NO: 1351 | human Sushi, von Willebrand factor type A, EGF and pentraxin domain-containing protein 1-EGF9 protein | PRT1351 |
| SEQ ID NO: 1352 | human Tenascin-EGF1 protein | PRT1352 |
| SEQ ID NO: 1353 | human Tenascin-EGF10 protein | PRT1353 |
| SEQ ID NO: 1354 | human Tenascin-EGF11 protein | PRT1354 |
| SEQ ID NO: 1355 | human Tenascin-EGF12 protein | PRT1355 |
| SEQ ID NO: 1356 | human Tenascin-EGF13 protein | PRT1356 |
| SEQ ID NO: 1357 | human Tenascin-EGF14 protein | PRT1357 |
| SEQ ID NO: 1358 | human Tenascin-EGF2 protein | PRT1358 |
| SEQ ID NO: 1359 | human Tenascin-EGF3 protein | PRT1359 |
| SEQ ID NO: 1360 | human Tenascin-EGF4 protein | PRT1360 |
| SEQ ID NO: 1361 | human Tenascin-EGF5 protein | PRT1361 |
| SEQ ID NO: 1362 | human Tenascin-EGF6 protein | PRT1362 |
| SEQ ID NO: 1363 | human Tenascin-EGF7 protein | PRT1363 |
| SEQ ID NO: 1364 | human Tenascin-EGF8 protein | PRT1364 |
| SEQ ID NO: 1365 | human Tenascin-EGF9 protein | PRT1365 |
| SEQ ID NO: 1366 | human Tenascin-N-EGF1 protein | PRT1366 |
| SEQ ID NO: 1367 | human Tenascin-N-EGF2 protein | PRT1367 |
| SEQ ID NO: 1368 | human Tenascin-N-EGF3 protein | PRT 1368 |
| SEQ ID NO: 1369 | human Tenascin-X-EGF1 protein | PRT 1369 |
| SEQ ID NO: 1370 | human Tenascin-X-EGF1 0 protein | PRT1370 |
| SEQ ID NO: 1371 | human Tenascin-X-EGF1 1 protein | PRT1371 |
| SEQ ID NO: 1372 | human Tenascin-X-EGF12 protein | PRT1372 |
| SEQ ID NO: 1373 | human Tenascin-X-EGF13 protein | PRT1373 |
| SEQ ID NO: 1374 | human Tenascin-X-EGF14 protein | PRT1374 |
| SEQ ID NO: 1375 | human Tenascin-X-EGF 15 protein | PRT1375 |
| SEQ ID NO: 1376 | human Tenascin-X-EGF 16 protein | PRT1376 |
| SEQ ID NO: 1377 | human Tenascin-X-EGF17 protein | PRT1377 |
| SEQ ID NO: 1378 | human Tenascin-X-EGF18 protein | PRT1378 |
| SEQ ID NO: 1379 | human Tenascin-X-EGF2 protein | PRT1379 |
| SEQ ID NO: 1380 | human Tenascin-X-EGF3 protein | PRT 1380 |
| SEQ ID NO: 1381 | human Tenascin-X-EGF4 protein | PRT1381 |
| SEQ ID NO: 1382 | human Tenascin-X-EGF5 protein | PRT1382 |
| SEQ ID NO: 1383 | human Tenascin-X-EGF6 protein | PRT1383 |
| SEQ ID NO: 1384 | human Tenascin-X-EGF7 protein | PRT1384 |
| SEQ ID NO: 1385 | human Tenascin-X-EGF8 protein | PRT1385 |
| SEQ ID NO: 1386 | human Tenascin-X-EGF9 protein | PRT1386 |
| SEQ ID NO: 1387 | human Teneurin-1-EGF1 protein | PRT1387 |
| SEQ ID NO: 1388 | human Teneurin-1-EGF2 protein | PRT1388 |
| SEQ ID NO: 1389 | human Teneurin-1-EGF3 protein | PRT1389 |
| SEQ ID NO: 1390 | human Teneurin-1-EGF4 protein | PRT 1390 |
| SEQ ID NO: 1391 | human Teneurin-1-EGF5 protein | PRT1391 |
| SEQ ID NO: 1392 | human Teneurin-1-EGF6 protein | PRT1392 |
| SEQ ID NO: 1393 | human Teneurin-1-EGF7 protein | PRT1393 |
| SEQ ID NO: 1394 | human Teneurin-1-EGF8 protein | PRT1394 |
| SEQ ID NO: 1395 | human Teneurin-2-EGF1 protein | PRT1395 |
| SEQ ID NO: 1396 | human Teneurin-2-EGF2 protein | PRT1396 |
| SEQ ID NO: 1397 | human Teneurin-2-EGF3 protein | PRT1397 |
| SEQ ID NO: 1398 | human Teneurin-2-EGF4 protein | PRT1398 |
| SEQ ID NO: 1399 | human Teneurin-2-EGF5 protein | PRT 1399 |
| SEQ ID NO: 1400 | human Teneurin-2-EGF6 protein | PRT 1400 |
| SEQ ID NO: 1401 | human Teneurin-2-EGF7 protein | PRT1401 |
| SEQ ID NO: 1402 | human Teneurin-2-EGF8 protein | PRT1402 |
| SEQ ID NO: 1403 | human Tcneurin-3-EGF1 protein | PRT1403 |
| SEQ ID NO: 1404 | human Teneurin-3-EGF2 protein | PRT 1404 |
| SEQ ID NO: 1405 | human Teneurin-3-EGF3 protein | PRT1405 |
| SEQ ID NO: 1406 | human Teneurin-3-EGF4 protein | PRT 1406 |
| SEQ ID NO: 1407 | human Teneurin-3-EGF5 protein | PRT1407 |
| SEQ ID NO: 1408 | human Teneurin-3-EGF6 protein | PRT1408 |
| SEQ ID NO: 1409 | human Teneurin-3-EGF7 protein | PRT1409 |
| SEQ ID NO: 1410 | human Tencurin-3-EGF8 protein | PRT1410 |
| SEQ ID NO: 1411 | human Teneurin-4-EGF1 protein | PRT1411 |
| SEQ ID NO: 1412 | human Teneurin-4-EGF2 protein | PRT1412 |
| SEQ ID NO: 1413 | human Tencurin-4-EGF3 protein | PRT1413 |
| SEQ ID NO: 1414 | human Teneurin-4-EGF4 protein | PRT1414 |
| SEQ ID NO: 1415 | human Teneurin-4-EGF5 protein | PRT1415 |
| SEQ ID NO: 1416 | human Teneurin-4-EGF6 protein | PRT1416 |
| SEQ ID NO: 1417 | human Teneurin-4-EGF7 protein | PRT1417 |
| SEQ ID NO: 1418 | human Teneurin-4-EGF8 protein | PRT1418 |
| SEQ ID NO: 1419 | human Teratocarcinoma-derived growth factor 1-EGF1 protein | PRT1419 |
| SEQ ID NO: 1420 | human Thrombomodulin-EGF1 protein | PRT1420 |
| SEQ ID NO: 1421 | human Thrombomodulin-EGF2 protein | PRT1421 |
| SEQ ID NO: 1422 | human Thrombomodulin-EGF3 protein | PRT1422 |
| SEQ ID NO: 1423 | human Thrombomodulin-EGF4 protein | PRT1423 |
| SEQ ID NO: 1424 | human Thrombomodulin-EGF5 protein | PRT1424 |
| SEQ ID NO: 1425 | human Thrombomodulin-EGF6 protein | PRT1425 |
| SEQ ID NO: 1426 | human Thrombospondin-1-EGF1 protein | PRT1426 |
| SEQ ID NO: 1427 | human Thrombospondin-1-EGF2 protein | PRT1427 |
| SEQ ID NO: 1428 | human Thrombospondin-2-EGF1 protein | PRT1428 |
| SEQ ID NO: 1429 | human Thrombospondin-2-EGF2 protein | PRT1429 |
| SEQ ID NO: 1430 | human Thrombospondin-3-EGF1 protein | PRT1430 |
| SEQ ID NO: 1431 | human Thrombospondin-3-EGF2 protein | PRT1431 |
| SEQ ID NO: 1432 | human Thrombospondin-3-EGF3 protein | PRT1432 |
| SEQ ID NO: 1433 | human Thrombospondin-4-EGF1 protein | PRT1433 |
| SEQ ID NO: 1434 | human Thrombospondin-4-EGF2 protein | PRT1434 |
| SEQ ID NO: 1435 | human Thrombospondin-4-EGF3 protein | PRT1435 |
| SEQ ID NO: 1436 | human Thrombospondin-4-EGF4 protein | PRT1436 |
| SEQ ID NO: 1437 | human Thyroid peroxidase-EGFI protein | PRT1437 |
| SEQ ID NO: 1438 | human Tissue-type plasminogen activator-EGF1 protein | PRT1438 |
| SEQ ID NO: 1439 | human Tolloid-like protein 1-EGF1 protein | PRT1439 |
| SEQ ID NO: 1440 | human Tolloid-like protein 1-EGF2 protein | PRT1440 |
| SEQ ID NO: 1441 | human Tolloid-like protein 2-EGFI protein | PRT1441 |
| SEQ ID NO: 1442 | human Tolloid-like protein 2-EGF2 protein | PRT1442 |
| SEQ ID NO: 1443 | human Tomoregulin-1-EGF1 protein | PRT 1443 |
| SEQ ID NO: 1444 | human Tomoregulin-2-EGF1 protein | PRT 1444 |
| SEQ ID NO: 1445 | human Transmembrane protein 8A-EGF1 protein | PRT1445 |
| SEQ ID NO: 1446 | human Transmembrane protein 8B-EGF1 protein | PRT1446 |
| SEQ ID NO: 1447 | human Tyrosine-protein kinase receptor Tie-1-EGF1 protein | PRT1447 |
| SEQ ID NO: 1448 | human Tyrosine-protein kinase receptor Tie-1-EGF2 protein | PRT1448 |
| SEQ ID NO: 1449 | human Tyrosine-protein kinase receptor Tic-1-EGF3 protein | PRT1449 |
| SEQ ID NO: 1450 | human Urokinase-type plasminogen activator-EGFI protein | PRT1450 |
| SEQ ID NO: 1451 | human Uromodulin-EGF1 protein | PRT1451 |
| SEQ ID NO: 1452 | human Uromodulin-EGF2 protein | PRT1452 |
| SEQ ID NO: 1453 | human Uromodutin-EGF3 protein | PRT1453 |
| SEQ ID NO: 1454 | human Uromodulin-like 1-EGF1 protein | PRT1454 |
| SEQ ID NO: 1455 | human Uromodulin-like 1-EGF2 protein | PRT1455 |
| SEQ ID NO: 1456 | human Uromodulin-like 1-EGF3 protein | PRT1456 |
| SEQ ID NO: 1457 | human Usherin-EGF1 protein | PRT1457 |
| SEQ ID NO: 1458 | human Usherin-EGF10 protein | PRT1458 |
| SEQ ID NO: 1459 | human Usherin-EGF2 protein | PRT1459 |
| SEQ ID NO: 1460 | human Usherin-EGF3 protein | PRT1460 |
| SEQ ID NO: 1461 | human Usherin-EGF4 protein | PRT1461 |
| SEQ ID NO: 1462 | human Usherin-EGF5 protein | PRT1462 |
| SEQ ID NO: 1463 | human Usherin-EGF6 protein | PRT 1463 |
| SEQ ID NO: 1464 | human Usherin-EGF7 protein | PRT1464 |
| SEQ ID NO: 1465 | human Ushcrin-EGF8 protein | PRT1465 |
| SEQ ID NO: 1466 | human Ushcrin-EGF9 protein | PRT1466 |
| SEQ ID NO: 1467 | human Vasorin-EGF1 protein | PRT1467 |
| SEQ ID NO: 1468 | human Versican core protein-EGF1 protein | PRT 1468 |
| SEQ ID NO: 1469 | human Very low-density lipoprotein receptor-EGF1 protein | PRT1469 |
| SEQ ID NO: 1470 | human Very low-density lipoprotein receptor-EGF2 protein | PRT1470 |
| SEQ ID NO: 1471 | human Very low-density lipoprotein receptor-EGF3 protein | PRT1471 |
| SEQ ID NO: 1472 | human Vitamin K-dependent protein C-EGF1 protein | PRT1472 |
| SEQ ID NO: 1473 | human Vitamin K-dependent protein C-EGF2 protein | PRT1473 |
| SEQ ID NO: 1474 | human Vitamin K-dependent protein S-EGF1 protein | PRT1474 |
| SEQ ID NO: 1475 | human Vitamin K-dependent protein S-EGF2 protein | PRT1475 |
| SEQ ID NO: 1476 | human Vitamin K-dependent protein S-EGF3 protein | PRT1476 |
| SEQ ID NO: 1477 | human Vitamin K-dependent protein S-EGF4 protein | PRT1477 |
| SEQ ID NO: 1478 | human Vitamin K-dependent protein Z-EGF1 protein | PRT1478 |
| SEQ ID NO: 1479 | human Vitamin K-dependent protein Z-EGF2 protein | PRT1479 |
| SEQ ID NO: 1480 | human von Willebrand factor A domain-containing protein 2-EGF1 protein | PRT1480 |
| SEQ ID NO: 1481 | human von Willebrand factor A domain-containing protein 2-EGF2 protein | PRT1481 |
| SEQ ID NO: 1482 | human von Willebrand factor C and EGF domain-containing protein-EGF1 protein | PRT1482 |
| SEQ ID NO: 1483 | human von Willebrand factor C and EGF domain-containing protein-EGF2 protein | PRT1483 |
| SEQ ID NO: 1484 | human von Willebrand factor C and EGF domain-containing protein-EGF3 protein | PRT1484 |
| SEQ ID NO: 1485 | human von Willebrand factor C and EGF domain-containing protein-EGF4 protein | PRT1485 |
| SEQ ID NO: 1486 | human von Willebrand factor D and EGF domain-containing protein-EGF1 protein | PRT1486 |
| SEQ ID NO: 1487 | human von Willebrand factor D and EGF domain-containing protein-EGF2 protein | PRT1487 |
| SEQ ID NO: 1488 | human von Willebrand factor D and EGF domain-containing protein-EGF3 protein | PRT1488 |
| SEQ ID NO: 1489 | human von Willebrand factor D and EGF domain-containing protein-EGF4 protein | PRT1489 |
| SEQ ID NO: 1490 | human von Willebrand factor D and EGF domain-containing protein-EGF5 protein | PRT1490 |
| SEQ ID NO: 1491 | human von Willebrand factor D and EGF domain-containing protein-EGF6 protein | PRT1491 |
| SEQ ID NO: 1492 | human von Willebrand factor D and EGF domain-containing protein-EGF7 protein | PRT1492 |
| SEQ ID NO: 1493 | human Wnt inhibitory factor 1-EGF1 protein | PRT1493 |
| SEQ ID NO: 1494 | human Wnt inhibitory factor I-EGF2 protein | PRT1494 |
| SEQ ID NO: 1495 | human Wnt inhibitory factor 1-EGF3 protein | PRT1495 |
| SEQ ID NO: 1496 | human Wnt inhibitory factor 1-EGF4 protein | PRT1496 |
| SEQ ID NO: 1497 | human Wnt inhibitory factor 1-EGF5 protein | PRT1497 |
| SEQ ID NO: 1498 | human Zonadhesin-EGF1 protein | PRT1498 |

The human-originated EGF domain proteins mentioned above are expressed from a prokaryotic or eukaryotic recombinant plasmid or prepared by chemical synthesis.

The steps used in the present invention are as follows:
(1) obtain a gene encoding the human-originated EGF domain protein hF VII-EGF1 or a gene having homology of more than 50% thereto, as well as a gene coding for a fluorescent protein by PCR amplification or artificial synthesis; and obtain a fusion gene encoding for the RFP fusion protein, RFP-hF VII-EGF1 human-originated EGF domain protein, or a gene for a fusion protein having homology of more than 50% to the aforementioned fusion protein through overlapping PCR amplification reaction;
(2) insert the individual gene fragments mentioned above encoding for the recombinant fusion proteins respectively into a prokaryotic or eukaryotic vector to construct the recombinant prokaryotic or eukaryotic plasmid, followed by transformation of the resultant recombinant plasmid into competent bacteria to be cultured, and then screen the recombinant plasmid comprising the correctly inserted fragments by sequencing;
(3) transform the recombinant prokaryotic plasmids with the correct sequencing result into an engineering bacterium to be expressed; isolate and purify the expressed protein to obtain the fusion protein RFP-hF VII-EGF1 or the protein having homology of more than 50% to the fusion protein; or transfect the resultant recombinant eukaryotic plasmids with the correct sequencing result into mammalian cells to be cultured, establish a stable cell line for eukaryotic expression, isolate and purify the expressed protein to obtain the fusion protein RFP-hF VII-EGF1 or the protein having homology of more than 50% to the fusion protein;
(4) release the recombination protein hF VII-EGF1 or the protein having homology of more than 50% thereto by enzymatic digestion with rTEV enzyme, and purify the protein:
(5) analyze the antibacterial activity of the recombination protein hF VII-EGF1 or the protein having homology of more than 50% thereto;
(6) analyze the hydrolysis of the lipopolysaccharide by the recombination protein hF VII-EGF1 or the protein having homology of more than 50% thereto by silver staining;
where the prokaryotic vector is the pET plasmid system which includes the prokaryotic expression plasmids such as pET-14b, pET-19b, pET-21a (+), pET-28a (+), pET-42a (+), etc: and the eukaryotic vector is pcDNA3.1 (+), pcDNA3.1 (-), etc.

Chemical synthesis of the human-originated EGF domain proteins is usually outsourced to professional companies.

It has been demonstrated experimentally that: 1498 of the human-originated EGF domain proteins (PRT1 to PRT1498 in Table 1) set forth in the present invention such as hF VII-EGF1 protein are able to hydrolyze the lipopolysaccharide of Gram-negative bacteria (also known as endotoxin, a major component in the outer-membrane of a Gram-negative bacterium) and disrupt the stability of cellular structure, thereby exerting the bactericidal action. Meanwhile, the human-originated EGF domain proteins such as hF VII-EGF1, hF VII-EGF2, hF IX-EGF1, hF IX-EGF2, hF X-EGF1, and hF X-EGF2, have very strong binding to the tissue factor. When the body is injured, the tissue factor will be exposed at the wound in a large amount, which allows such EGF domain-containing proteins to aggregate accordingly at the wound, exerting a targeted bacteriostatic effect. Assays on the antibacterial activity demonstrate that 1498 of the human-originated EGF domain proteins (PRT1 to PRT1498 in Table 1) such as hFVII-EGF1 protein can inhibit the Gram-negative bacteria significantly. The minimum inhibitory concentrations (MICs) of the abovementioned recombinant proteins for *E. coli* DH5α, *E. coli* BL21, *Pseudomonas aeruginosa, Klebsiella pneumonia, Enterobacter cloacae, Aeromonas hydrophila, Citrobacter diversus, Moraxella catarrhalis, Proteus mirabilis, Proteus vulgaris, Serratia marcescens* are shown in Tables 2 and 3 in the Examples below.

1498 of the human-originated EGF domain proteins (PRT1 to PRT1498 in Table 1) set forth in the present invention such as hF VII-EGF1 protein can hydrolyze the lipopolysaccharide of Gram-negative bacteria (also known as endotoxin, seen in Example 17) and lipopolysaccharide is the major pathogenic factor causing endotoxemia. Therefore, 1498 of the human-originated EGF domain proteins (PRT1 to PRT1498 in Table 1) set forth in the present invention such as hF VII-EGF1 protein can be used in preparing a medicament for treating endotoxemia caused by the Gram-negative bacteria.

The present invention has the following advantageous effects:
1. 1498 of the artificially-prepared human-originated EGF domain proteins (PRT1 to PRT1498 in Table 1) provided in the present invention such as hF VII-EGF1 protein are able to hydrolyze the outer membrane of the Gram-negative bacteria and has been shown experimentally to have significant inhibitory effects on the Gram-negative bacteria, thereby providing a novel class of therapeutic drugs for use in treating diseases caused by the Gram-negative bacteria infection.
2. 1498 of the artificially-prepared human-originated EGF domain proteins (PRT1 to PRT1498 in Table 1) provided in the present invention such as hF VII-EGF1 protein are able to hydrolyze the lipopolysaccharide (also known as endotoxin) of the Gram-negative bacteria, thereby providing a novel class of therapeutic drugs for use in treating endotoxemia caused by the Gram-negative bacteria.
3. Six human-originated EGF domain proteins provided in the present invention which are artificially prepared, *i.e.* hF VII-EGF1, hF VII-EGF2, hF IX-EGF1, hF IX-EGF2, hF X-EGF1, and hF X-EGF2, can exhibit an excellent targeted bactericidal effect on Gram-negative bacteria at the wound site by targeted localization at the wound site via binding to tissue factor, and be prospective to be used in preparation of a novel targeted antibacterial drug.
4. 1498 of the human-originated EGF domain proteins (PRT1 to PRT1498 in Table 1) such as hF VII-EGF1 protein are natural and intrinsic components in the human body, and use of the EGF domain proteins as the bacteriostatic domain in the present invention can reduce the immunogenicity of the medicament effectively.
5. Since 1498 of the recombinant human-originated EGF domain proteins (PRT1 to PRT1498 in Table 1) such as hF VII-EGF1 protein could be expressed properly in cells of *E. coli* directly through the genetic engineering techniques, the production cost is lower, suitable for industrial production.

### Brief Description of Accompanying Drawings

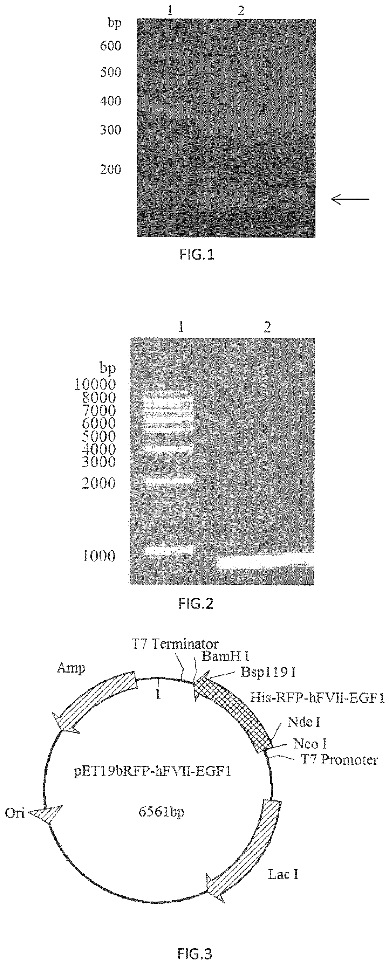

Fig. 1 is the electrophoregram of PCR amplification of the gene sequence encoding the recombinant protein hF VII-EGF1 in Example 1, wherein Lane 1 is the DNA molecular weight marker (Marker 1, purchased from TIANGEN BIOTECH Co. Ltd.) and Lane 2 is PCR amplification of the sequence encoding hF VII-EGF1. The arrowhead is directed toward the amplified fragment of interest.
Fig. 2 is the electrophoregram of PCR amplification of the gene sequence encoding the fusion protein RFP-hF VII-EGF1 in Example 1, wherein Lane 1 is the DNA molecular weight marker (1 kb Marker, purchased from TIANGEN BIOTECH Co. Ltd.) and Lane 2 is PCR amplification of the sequence encoding RFP-hF VII-EGF1.
Fig. 3 is the schematic representation of the recombinant prokaryotic plasmid pET19bRFP-hF VII-EGF1 in Example 2, wherein the counterclockwise sequence is the forward gene fragment and the clockwise one is the reverse gene fragment.

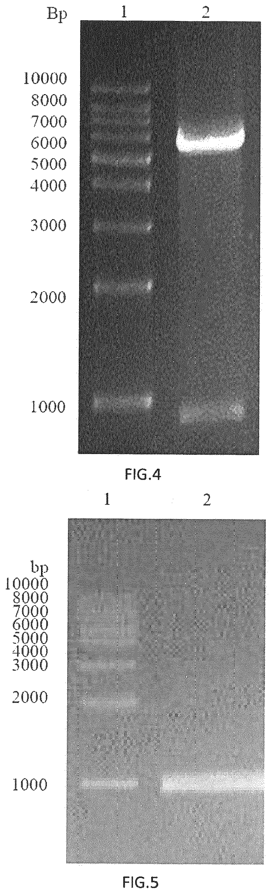

Fig.4 is the electrophoregram for identification of the restriction-endonuclease digested fragments of the recombinant plasmid pET19bRFP-hF VII-EGF1 in Example 2, wherein Lane 1 is the DNA molecular weight marker (1 kb Marker, purchased from TIANGEN BIOTECH Co. Ltd.) and Lane 2 is the pET19b vector fragment and the DNA fragment encoding the recombinant protein RFP-hF VII-EGF1 which are obtained after restriction-endonuclease double digestion of the recombinant plasmid pET19bRFP-hF VII-EGF1.
Fig. 5 is the electrophoregram of PCR amplification of the gene sequence encoding the fusion protein RFP-hF VII-EGF2 in Example 3, wherein Lane 1 is the DNA molecular weight marker (1 kb Marker, purchased from TIANGEN BIOTECH Co. Ltd.) and Lane 2 is PCR amplification of the sequence encoding RFP-hF VII-EGF2.

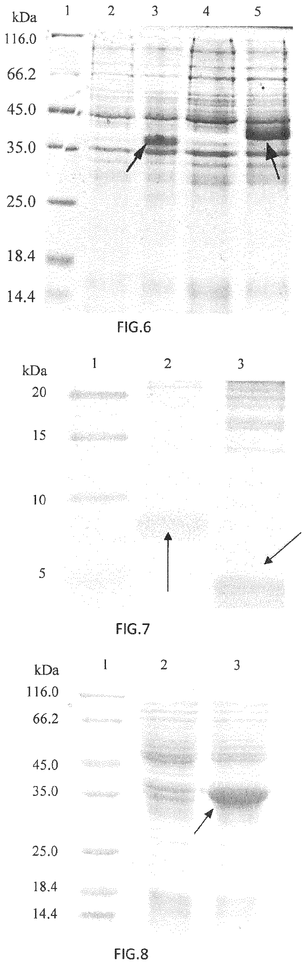

Fig. 6 is the SDS-PAGE analysis of inducible expression of the fusion proteins RFP-hF VII-EGF1 and RFP-hF VII-EGF2 from the recombinant plasmids pET19bRFP-hF VII-EGF1 and pET19bRFP-FVII-EGF2 in *E. coli* in Examples 5 and 8, wherein Lane 1 is the protein molecular weight marker (Unst. Protein Marker, purchased from Thermo Scientific); Lane 2 is the total protein of the E. coli without expressing the fusion protein RFP-hF VII-EGF1 before induction; Lane 3 is the total protein of the E. coli after expressing the fusion protein RFP-hF VII-EGF1 by induction; Lane 4 is the total protein of the E. coli without expressing the fusion protein RFP-hF VII-EGF2 before induction; and Lane 5 is the total protein of the E. coli after expressing the fusion protein RFP-hF VII-EGF2 by induction. The arrowheads indicate the expressed fusion proteins.
Fig. 7 is picture of Tricien gel analysis for identifying the recombination proteins hF VII-EGF1 and hF VII-EGF2 obtained after enzymatic digestion of the fusion proteins RFP-hF VII-EGF1 and RFP-hF VII-EGF2 with rTEV in Examples 7 and 8, in which Lane 1 is the lower molecular weight protein marker (PageRuler Unstained Low Range Protein Ladder, purchased from Thermo Scientific); Lane 2 is the recombination protein hF VII-EGF2 obtained after rTEV enzymatic digestion; Lane 3 is the recombination protein hF VII-EGFI obtained after rTEV enzymatic digestion; and the arrowheads indicate the proteins of interest after enzymatic digestion.
Fig. 8 is the SDS-PAGE analysis of inducible expression of the fusion protein RFP-hF IX-EGF1 from the recombinant plasmid pET21ahF IX-EGF1-RFP in *E. coli* in Example 8, wherein Lane 1 is the protein molecular weight marker (Unst. Protein Marker, purchased from Thermo Scientific); Lane 2 is the total protein of the E. coli without expressing the fusion protein RFP-hF IX-EGF1 before induction; and Lane 3 is the total protein of the E. coli after expressing the fusion protein ***RFP-hF IX-EGF1*** by induction. The arrowhead indicates the expressed fusion protein.

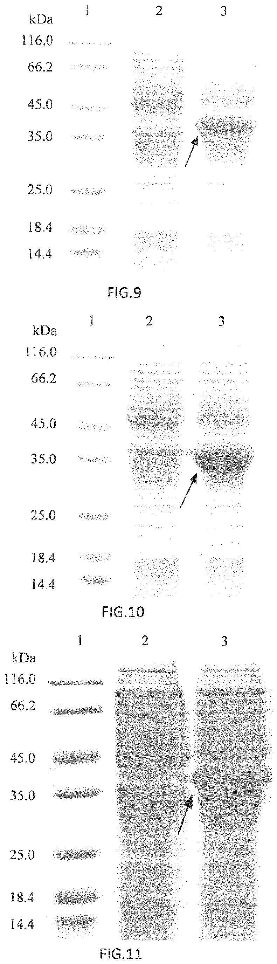

Fig. 9 is the SDS-PAGE analysis of inducible expression of the fusion protein RFP-hF IX-EGF2 from the recombinant plasmid pET21ahF IX-EGF2-RFP in *E*. *coli* in Example 8, wherein Lane 1 is the protein molecular weight marker (Unst. Protein Marker, purchased from Thermo Scientific): Lane 2 is the total protein of the E. coli without expressing the fusion protein RFP-hF IX-EGF2 before induction; and Lane 3 is the total protein of the E. coli after expressing the fusion protein RFP-hF IX-EGF2 by induction. The arrowhead indicates the expressed fusion protein.
Fig. 10 is the SDS-PAGE analysis of inducible expression of the fusion protein RFP-hF X-EGF1 from the recombinant plasmid pET21ahF X-EGF1-RFP in *E*. *coli* in Example 8, wherein Lane 1 is the protein molecular weight marker (Unst. Protein Marker, purchased from Thermo Scientific); Lane 2 is the total protein of the E. coli without expressing the fusion protein RFP-hF X-EGF1 before induction; and Lane 3 is the total protein of the E. coli after expressing the fusion protein RFP-hF X-EGF1 by induction. The arrowhead indicates the expressed fusion protein.
Fig. 11 is the SDS-PAGE analysis of inducible expression of the fusion protein RFP-hF X-EGF2 from the recombinant plasmid pET21ahF X-EGF2-RFP in *E. coli* in Example 8, wherein Lane 1 is the protein molecular weight marker (Unst. Protein Marker, purchased from Thermo Scientific); Lane 2 is the total protein of the E. coli without expressing the fusion protein RFP-hF X-EGF2 before induction; and Lane 3 is the total protein of the E. coli after expressing the fusion protein RFP-hF X-EGF2 by induction. The arrowhead indicates the expressed fusion protein.

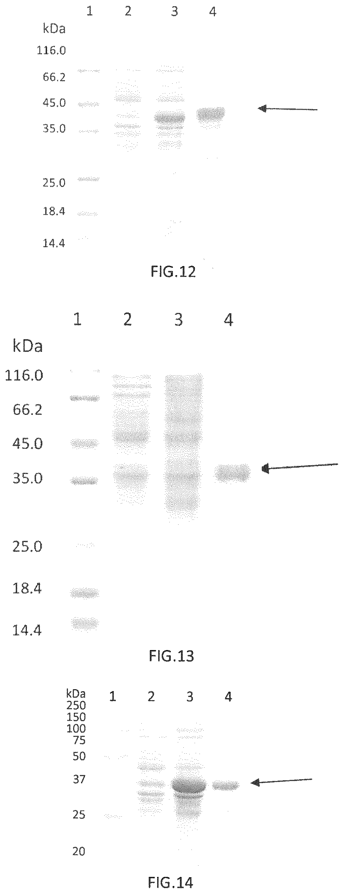

Fig. 12 is the SDS-PAGE analysis of inducible expression of the fusion protein RFP-Delta and Notch-like epidermal growth factor-related-EGF1 from the recombinant plasmid pET21ahDelta and Notch-like epidermal growth factor-related-EGF1-RFP in *E. coli* in Example 8, wherein Lane 1 is the protein molecular weight marker (Unst. Protein Marker, purchased from Thermo Scientific); Lane 2 is the total protein of the E. coli without expressing the fusion protein RFP-Delta and Notch-like epidermal growth factor-related-EGF1 before induction; and Lane 3 is the total protein of the E. coli after expressing the fusion protein RFP-Delta and Notch-like epidermal growth factor-related-EGF1 by induction: and Lane 4 is the purified fusion protein RFP-Delta and Notch-like epidermal growth factor-related-EGF1. The arrowhead indicates the expressed fusion protein.
Fig. 13 is the SDS-PAGE analysis of inducible expression of the fusion protein RFP-Zonadhesin-EGF1 from the recombinant plasmid pET21ahZonadhesin-EGF1-RFP in *E. coli* in Example 8, wherein Lane 1 is the protein molecular weight marker (Unst. Protein Marker, purchased from Thermo Scientific); Lane 2 is the total protein of the E. coli without expressing the fusion protein RFP-Zonadhesin-EGF1 before induction; and Lane 3 is the total protein of the E. coli after expressing the fusion protein RFP-Zonadhesin-EGF1 by induction; and Lane 4 is the purified fusion protein RFP-Zonadhesin-EGF1. The arrowhead indicates the expressed fusion protein.
Fig. 14 is the SDS-PAGE analysis of inducible expression of the fusion protein RFP-EGF, latrophilin and seven transmembrane domain-containing protein 1-EGF2 from the recombinant plasmid pET21ahEGF-latrophilin and seven transmembrane domain-containing protein 1-EGF2-RFP in *E. coli* in Example 8, wherein Lane 1 is the protein molecular weight marker (Precision Plus Protein Standards, purchased from BIO-RAD); Lane 2 is the total protein of the E. coli without expressing the fusion protein RFP-EGF, latrophilin and seven transmembrane domain-containing protein 1-EGF2 before induction; and Lane 3 is the total protein of the E. coli after expressing the fusion protein RFP-EGF, latrophilin and seven transmembrane domain-containing protein 1-EGF2 by induction; and Lane 4 is the purified fusion protein RFP-EGF, latrophilin and seven transmembrane domain-containing protein 1-EGF2. The arrowhead indicates the expressed fusion protein.

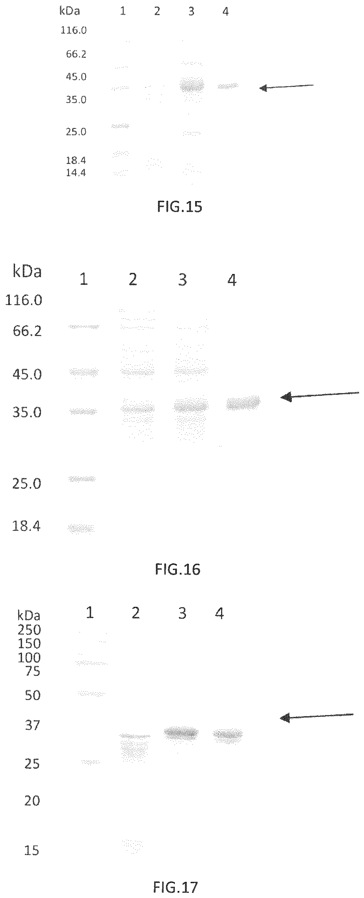

Fig. 15 is the SDS-PAGE analysis of inducible expression of the fusion protein RFP-Prostaglandin G/H synthase 1-EGF1 from the recombinant plasmid pET21ahProstaglandin G/H synthase 1-EGF1-RFP in *E*. *coli* in Example 8, wherein Lane 1 is the protein molecular weight marker (Unst. Protein Marker, purchased from Thermo Scientific); Lane 2 is the total protein of the E. coli without expressing the fusion protein RFP-Prostaglandin G/H synthase 1-EGF1 before induction; and Lane 3 is the total protein of the E. coli after expressing the fusion protein RFP-Prostaglandin G/H synthase 1-EGF1 by induction; and Lane 4 is the purified fusion protein RFP-Prostaglandin G/H synthase 1-EGF1. The arrowhead indicates the expressed fusion protein.
Fig. 16 is the SDS-PAGE analysis of inducible expression of the fusion protein RFP-Neurexin-1-EGF1 from the recombinant plasmid pET21ahNeurexin-1-EGF1-RFP in *E*. *coli* in Example 8, wherein Lane I is the protein molecular weight marker (Unst. Protein Marker, purchased from Thermo Scientific); Lane 2 is the total protein of the E. coli without expressing the fusion protein RFP-Neurexin-1-EGF1 before induction; and Lane 3 is the total protein of the E. coli after expressing the fusion protein RFP-Neurexin-1-GF1 by induction; and Lane 4 is the purified fusion protein RFP-Neurexin-1-EGF1. The arrowhead indicates the expressed fusion protein.
Fig. 17 is the SDS-PAGE analysis of inducible expression of the fusion protein RFP-EGF-containing fibulin-like extracellular matrix protein 1-EGF1 from the recombinant plasmid pET21ahEGF-containing fibulin-like extracellular matrix protein 1-EGF1-RFP in *E*. *coli* in Example 8, wherein Lane 1 is the protein molecular weight marker (Precision Plus Protein Standards, purchased from BIO-RAD); Lane 2 is the total protein of the E. coli without expressing the fusion protein RFP-EGF-containing fibulin-like extracellular matrix protein 1-EGF1 before induction; and Lane 3 is the total protein of the E. coli after expressing the fusion protein RFP-EGF-containing fibulin-like extracellular matrix protein 1-EGF1 by induction; and Lane 4 is the purified fusion protein RFP-EGF-containing fibulin-like extracellular matrix protein 1-EGF1. The arrowhead indicates the expressed fusion protein.

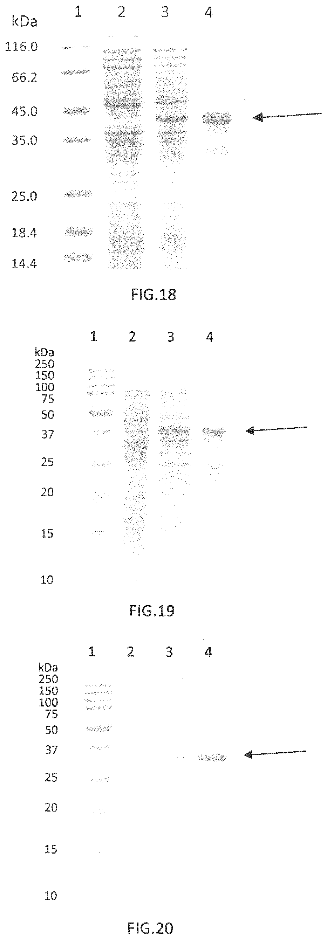

Fig. 18 is the SDS-PAGE analysis of inducible expression of the fusion protein RFP-Netrin-3-EGF1 from the recombinant plasmid pET21ahNetrin-3-EGF1-RFP in *E. coli* in Example 8, wherein Lane 1 is the protein molecular weight marker (Unst. Protein Marker, purchased from Thermo Scientific); Lane 2 is the total protein of the E. coli without expressing the fusion protein RFP-Netrin-3-EGF1 before induction; and Lane 3 is the total protein of the E. coli after expressing the fusion protein RFP-Netrin-3-EGF1 by induction; and Lane 4 is the purified fusion protein RFP-Netrin-3-EGF1. The arrowhead indicates the expressed fusion protein.
Fig. 19 is the SDS-PAGE analysis of inducible expression of the fusion protein RFP-Putative EGF-like module-containing mucin-like hormone receptor-like 4-EGF2 from the recombinant plasmid pET21ahPutative EGF-like module-containing mucin-like hormone receptor-like 4-EGF2-RFP in *E. coli* in Example 8, wherein Lane 1 is the protein molecular weight marker (Precision Plus Protein Standards, purchased from BIO-RAD); Lane 2 is the total protein of the E. coli without expressing the fusion protein RFP-Putative EGF-like module-containing mucin-like hormone receptor-like 4-EGF2 before induction; and Lane 3 is the total protein of the E. coli after expressing the fusion protein RFP-Putative EGF-like module-containing mucin-like hormone receptor-like 4-EGF2 by induction; and Lane 4 is the purified fusion protein RFP-Putative EGF-like module-containing mucin-like hormone receptor-like 4-EGF2. The arrowhead indicates the expressed fusion protein.
Fig. 20 is the SDS-PAGE analysis of inducible expression of the fusion protein RFP-Protransforming growth factor alpha-EGF1 from the recombinant plasmid pET21ahProtransforming growth factor alpha-EGF1-RFP in *E. coli* in Example 8, wherein Lane 1 is the protein molecular weight marker (Precision Plus Protein Standards, purchased from BIO-RAD); Lane 2 is the total protein of the E. coli without expressing the fusion protein RFP-Protransforming growth factor alpha-EGF1 before induction; and Lane 3 is the total protein of the E. coli after expressing the fusion protein RFP-Protransforming growth factor alpha-EGF1 by induction; and Lane 4 is the purified fusion protein RFP-Protransforming growth factor alpha-EGF1. The arrowhead indicates the expressed fusion protein.

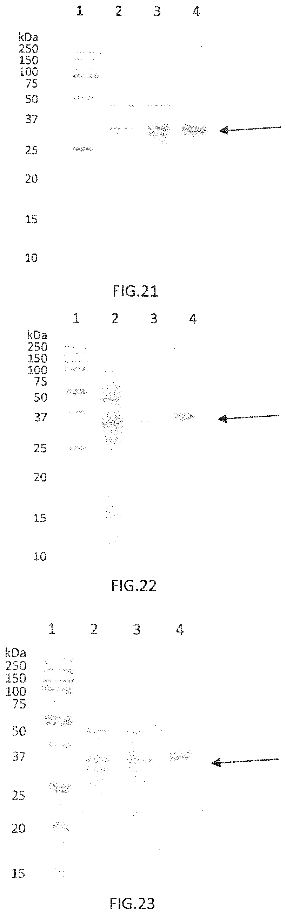

Fig. 21 is the SDS-PAGE analysis of inducible expression of the fusion protein RFP-Delta-like protein 4-EGF2 from the recombinant plasmid pET21ahDelta-like protein 4-EGF2-RFP in *E. coli* in Example 8, wherein Lane 1 is the protein molecular weight marker (Precision Plus Protein Standards, purchased from BIO-RAD); Lane 2 is the total protein of the E. coli without expressing the fusion protein RFP-Delta-like protein 4-EGF2 before induction; and Lane 3 is the total protein of the E. coli after expressing the fusion protein RFP-Delta-like protein 4-EGF2 by induction; and Lane 4 is the purified fusion protein RFP-Delta-like protein 4-EGF2. The arrowhead indicates the expressed fusion protein.
Fig. 22 is the SDS-PAGE analysis of inducible expression of the fusion protein RFP-Nidogen-1-EGF1 from the recombinant plasmid pET21ahNidogen-1-EGF1-RFP in *E. coli* in Example 8, wherein Lane 1 is the protein molecular weight marker (Precision Plus Protein Standards, purchased from BIO-RAD); Lane 2 is the total protein of the E. coli without expressing the fusion protein RFP-Nidogen-1-EGF1 before induction; and Lane 3 is the total protein of the E. coli after expressing the fusion protein RFP-Nidogen-1-EGF1 by induction: and Lane 4 is the purified fusion protein RFP-Nidogen-1-EGF1. The arrowhead indicates the expressed fusion protein.
Fig. 23 is the SDS-PAGE analysis of inducible expression of the fusion protein RFP-Uromodulin-like 1-EGF3 from the recombinant plasmid pET21ahNidogen-1-EGF1-RFP in E. *coli* in Example 8, wherein Lane I is the protein molecular weight marker (Precision Plus Protein Standards, purchased from BIO-RAD); Lane 2 is the total protein of the E. coli without expressing the fusion protein RFP-Uromodulin-like 1-EGF3 before induction; and Lane 3 is the total protein of the E. coli after expressing the fusion protein RFP-Uromodulin-like 1-EGF3 by induction; and Lane 4 is the purified fusion protein RFP-Uromodulin-like 1-EGF3. The arrowhead indicates the expressed fusion protein.

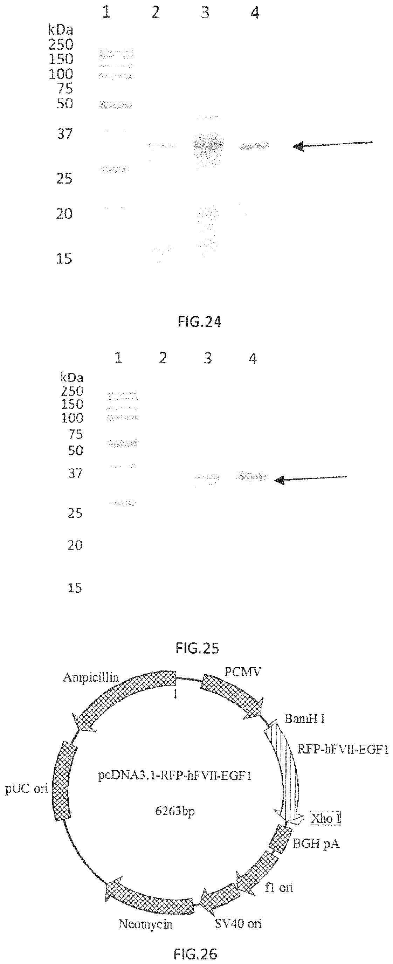

Fig. 24 is the SDS-PAGE analysis of inducible expression of the fusion protein RFP-Sushi, von Willebrand factor type A-EGF and pentraxin domain-containing protein 1-EGF2 from the recombinant plasmid pET21ahSushi, von Willebrand factor type A-EGF and pentraxin domain-containing protein 1-EGF2-RFP in *E. coli* in Example 8, wherein Lane 1 is the protein molecular weight marker (Precision Plus Protein Standards, purchased from BIO-RAD); Lane 2 is the total protein of the E. coli without expressing the fusion protein RFP-Sushi, von Willebrand factor type A-EGF and pentraxin domain-containing protein I-EGF2 before induction; and Lane 3 is the total protein of the E. coli after expressing the fusion protein RFP-Sushi, von Willebrand factor type A-EGF and pentraxin domain-containing protein I-EGF2 by induction; and Lane 4 is the purified fusion protein RFP-Sushi, von Willebrand factor type A-EGF and pentraxin domain-containing protein I-EGF2. The arrowhead indicates the expressed fusion protein.
Fig. 25 is the SDS-PAGE analysis of inducible expression of the fusion protein RFP-Protocadherin Fat 2-EGF1 from the recombinant plasmid pET21ahProlocadherin Fat 2-EGF1-RFP in *E. coli* in Example 8, wherein Lane I is the protein molecular weight marker (Precision Plus Protein Standards, purchased from BIO-RAD); Lane 2 is the total protein of the E. coli without expressing the fusion protein RFP-Protocadherin Fat 2-EGFI before induction; and Lane 3 is the total protein of the E. coli after expressing the fusion protein RFP-Protocadherin Fat 2-EGFI by induction; and Lane 4 is the purified fusion protein RFP-Protocadherin Fat 2-EGFI. The arrowhead indicates the expressed fusion protein.
Fig. 26 is the schematic representation of the recombinant eukaryotic plasmid pcDNA3.1-RFP-hF VII-EGF1 in Example 9, wherein the counterclockwise sequence is the forward gene fragment and the clockwise one is the reverse gene fragment.

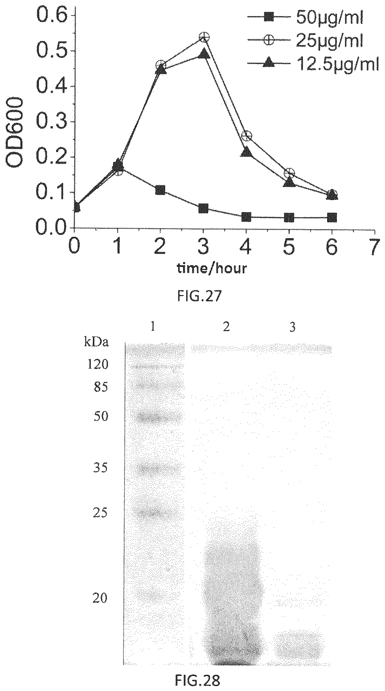

Fig. 27 is the growth curve of *E. coli* DH5α treated with the recombinant protein hF VII-EGFI in Example 14.
Fig. 28 is a silver staining image for hydrolysis of *E. coli* EH100 LPS by the recombinant protein hF VII-EGF1 in Example 17, in which Lane 1 is the prestained protein marker; Lane 2 is the *E. coli* EH100 LPS untreated; and Lane 3 is the *E. coli* EH100 LPS treated with hF VII-EGF1.

### DETAILED DESCRIPTION

The present invention will be further illustrated below in connection with the Examples. In the Examples described hereinafter, where the particular experiment conditions are not otherwise noted, they will follow the conventional conditions well known to the person of skill in the art, such as the conditions and experimental steps described in Molecular Cloning: A Laboratory Manual, Sambrook et al (Ed.), New York, Cold Spring Harbor Laboratory Press, 1989, An Conventional Manual for Laboratory Animals (National Center for Standard Laboratory Animals, November, 2004), and a Manual of Basic Technique, 5th Edition (John Wiley & Sons, Inc., 2005), or follow the conditions and experimental steps recommended by the manufacture. The particular experimental methods in the Examples are exemplified by the protein hF VII-EGF1, and the experimental methods for other human-originated EGF domain proteins (PRT2 to PRT1498 in Table 1) are performed with reference to those for the protein hF VII-EGFI. Nucleotide sequences for the expressed proteins of interest can be obtained from the databases on the web (http://www.uniprot.org/ or http://www.ncbi.nlm.nih.gov/).

### Example 1: Acquisition of the gene for the fusion protein RFP-hF VII-EGF1 with a red fluorescent protein (RFP) tag

### 1. The primers for PCR amplification as shown below were synthesized (by Invitrogen Biological Technology Co. Ltd.):

Primer 1:
   5'-TAACATATGGTGAGCAAGGGCGAGGAGGATA-3' SEQ ID NO: 1499
   Ndc I
Primer 2:
   5'-GAAAACCTGTACTTCCAGGGTCAATTCGAAGATGGGGACCAGTGTGCCTC-3'
   The enzymatic cleavage site for Bsp119I SEQ ID NO: 1500
   the recombinant protease
   (rTEV)
Primer 3:
   5'-CCTGGAAGTACAGGTTTTCCTTGTACAGCTCGTC-3' SEQ ID NO: 1501
Primer 4:
   5'-TATGGATCCTTACTCACAGTTCCGGCCCTCGAA-3' SEQ ID NO: 1502
   BamH I

### 2. Obtaining the coding sequences for hF VII-EGF1 and RFP by PCR amplification

### (1) Obtaining the DNA fragment encoding for hF VII- EGFI

The PCR amplification was performed with Primers 2 and 4 using a plasmid comprising the hF VII CDS region (FulenGen Co. Ltd., Guangzhou) as the template. A BamH I restriction site introduced into the sequence of Primer 4, and an enzymatic cleavage site for the recombinant tobacco mosaic virus proteinase (rTEV) and a restriction site for Bsp 119 I were introduced into the sequence of Primer 2, in order to facilitate replacement with the expressible sequences for expressing other human-originated EGF domain proteins (PRT2 to PRT1498 in Table I). The PCR reaction system (50 µL) was as follows:

| | |
|---|---|
| Deionized water: | 31.5 µL |
| 5 x Reaction butter: | 10 µL |
| dNTP Mix: | 4 µL |
| Primer 2 (10mM): | 1 µL |
| Primer 4 (10mM): | 1 µL |
| Plasmid comprising the hF VII CDS region (100 ng/µL) | 2 µL |
| PrimcStar DNA polymerase: | 0.5 µL |

The reaction condition for the PCR amplification was set with reference to the instruction of PrimeStar DNA polymerase (purchased from Takara): pre-denaturation at 98°C for 2 min, 30 cycles of denaturation at 98°C for 10 sec, annealing at 55°C for 15 sec, and elongation at 72°C for 30 sec, followed by elongation at 72°C for 4 min.

The amplified products were analyzed with 2% agarose gel electrophoresis, the DNA fragments comprising the coding sequence for hF VII- EGF1 was obtained (see Fig. 1), and the fragments of interest was recovered according to the instruction of a gel recovery kit (purchased from Omega).

### (2) Obtaining the DNA fragment encoding for the protein tag RFP

The PCR amplification was performed with Primers I and 3 using a plasmid comprising the coding sequence of the gene for the RFP protein (purchased from Takara) as the template. An Nde I restriction site was introduced into the sequence of Primer I. The reaction system was set up with reference to that for obtaining the DNA fragment encoding for hF VII- EGF1.

The reaction condition for the PCR amplification was set up with reference to the instruction of PrimeStar DNA polymerase: pre-denaturation at 98°C for 2 min, 30 cycles of denaturation at 98°C for 10 sec, annealing at 55°C for 15 sec, and elongation at 72°C for 1 min, followed by elongation at 72°C for 4 min.

The amplified products were analyzed with 2% agarose gel electrophoresis, the DNA fragment comprising the coding sequence for RFP was obtained, and the fragment of interest was recovered according to the instruction of a gel recovery kit (purchased from Omega).

### 3. Amplification of the gene encoding for the fusion protein RFP-hF VII-EGF1 by overlapping PCR.

A overlapping PCR amplification was performed with Primers 1 and 4 using the DNA fragment obtained from the two PCR amplifications mentioned above as templates, yielding a fusion gene fragment RFP-hF VII-EGF1 which comprises a coding sequence for the RFP-TEV enzymatic cleavage site and the human coagulation factor VII- EGF1 protein. The PCR reaction system (50 µL) was as follows:

| | |
|---|---|
| Deionized water: | 31.5 µL |
| 5 x Reaction buffer: | 10 µL |
| dNTP Mix: | 4 µL |
| Primer 1: | 1 µL |
| Primer 4: | 1 µL |
| the hF VII-EGF1-encoding DNA fragment obtained by the first PCR amplification (100 ng/(µL): | 1 µL |
| the RFP-encoding DNA fragment obtained by the second PCR amplification (100 ng/µL): | 1 µL |
| PrimeStar DNA polymerase: | 0.5 µL |

The reaction condition for the PCR amplification was set up with reference to the instruction of PrimeStar DNA polymerase: pre-denaturation at 98°C for 2 min, 30 cycles of denaturation at 98°C for 10 sec, annealing at 55°C for 15 sec, and elongation at 72°C for 1.5 min, followed by elongation at 72°C for 4 min.

The amplified products were analyzed with 2% agarose gel electrophoresis, the fragment comprising the sequence encoding for RFP-hF VII-EGF1 was obtained (see Fig. 2), and the fragment of interest was recovered according to the instruction of a gel recovery kit (purchased from Omega).

### Example 2: Construction of the recombinant prokaryotic plasmid for expressing the fusion protein RFP-hF VII-EGF1

### 1. Pre-treatment of the RFP-hF VII-EGF1 gene and the prokaryotic expression vector pET19b

The RFP-hF VII-EGF1 gene fragment obtained in Example 1 and the prokaryotic expression vector pET19b (the product from Novagen, the plasmid had been modified to carry a gene encoding for a red fluorescent protein) were individually double digested with the restriction endonucleases Nde I and BamH I (both purchased from TaKaRa or Fermentas) at 37°C overnight, and the reaction systems were established as follows:

| Reagent | *RFP-hF VII-EGF1* (µL) | pET19b (µL) |
|---|---|---|
| DNA | 20 | 20 |
| *Nde I* | 1 | 1 |
| *BamH I*/*Xho I* | 1 | 1 |
| 10 x Buffer | 6 | 6 |
| Double distilled water | 2 | 2 |
| Total volume | 30 | 30 |

After completion of the double digestion, the reaction products were analyzed with 1% agarose gel electrophoresis and the fragments of interest were recovered under the ultraviolet lamp of a gel imaging system according to the instruction of a gel recovery kit (purchased from Omega).

### 2. Ligation of the RFP-hF VII-EGFI gene to the prokaryotic expression vector pET19b and transformation

(1) The gene fragment RFP-hF VII-EGF1 and the prokaryotic vector fragment obtained in the above steps were quantified approximately and then subjected to ligation according to the ligation reaction principle where a ratio of the molecule number of the gene fragment to the molecule number of the prokaryotic vector is 3:1. The ligation reaction system was set up as follows:

| Reagent | Volume (µL) |
|---|---|
| pET19b DNA | 1.5 |
| *RFP-hF VII-EGF1* | 7 |
| T4 ligase | 0.5 |
| 10 × buffer | 1 |
| Total volume | 10 |

(2)The entire ligation reaction was proceeded at 16°C overnight treated with T4 DNA ligase (purchased from Fermentas). The gene fragment RFP-hF VII-EGF1 amplified above was inserted into the prokaryotic expression vector pET19b and the construction of the recombinant prokaryotic plasmid was schematically shown in Fig 3.
(3) The ligation product was transformed into the competent cells (prepared according to Molecular Cloning: a Laboratory Manual) of *E. coli* Top10 (purchased from Invitrogen company). The main steps of the procedure for transforming the ligation product were as follows:
1) 5 µL of the ligation product was pipetted into 100 µL of the competent cells of *E. coli* Top10 placed in a 1.5 mL Eppendorf tube, and the negative control is 100 µL of the competent cells of *E*. *coli* Top10 placed in a 1.5 mL Eppendorf tube;
2) the tubes were placed on ice for 30 min;
3) the competent cells were shocked in a metal bath at 42°C for 90 s;
4) the tubes were transferred rapidly into an ice-bath to be cooled for 2 min;
5) 900 µL of SOC medium (10g peptone, 5 g yeast extract, 0.5 g NaCl, 0.186 g KCl, 0.95 g MgCl₂, dissolved with ddH₂O and made up to a volume of 980 mL, autoclaved, cooled to room temperature and then 20 mL of sterile 20% aqueous glucose solution was added) was added, and placed on ice;
6) the tubes were incubated at 37°C for I h with shaking at 180 rpm;
7) the bacterial suspension was centrifuged at 4000 x g for 3 min, and the excessive medium was removed except 200 µL supernatant was kept. 200 µL of the remaining supernatant was used to resuspend the bacteria;
8) the resuspended transformants were plated uniformly onto the solid LB medium containing 0.1 g/mL of ampicillin and incubated in a constant-temperature incubator at 3T°C overnight.

### 3. Identification of the recombinant plasmid

The single clone of the transformed *E. coli* Top 10 mentioned above was picked, cultured in a small quantity and subjected to plasmid extraction with a plasmid extraction kit (purchased from OMEGA), followed by double digestion set forth in the steps of the present Example step I. The result of the double digested product resolved by gel-electrophoresis is shown in Fig. 4. The sizes of the two bands produced by the enzymatic digestion are consistent with those of the expected products, demonstrating the success of construction of the plasmid of interest.

The plasmids which had been identified by double digestion to be correct ones were send to Beijing Genomics Institute for sequencing of the inserted fusion gene fragment. Finally, the recombinant prokaryotic plasmid comprising the RFP-hF VII-EGF1 gene with the correct sequencing result was designated as pET19bRFP-hF VII-EGF1.

### Example 3: Obtaining the fusion genes for the RFP fusion proteins of other human-originated EGF domain proteins (PRT2 to PRT1498 in Table I) with a red fluorescent protein (RFP) tag

Since all of the expressed sequences of the human-originated EGF domain proteins (PRT1 to PRT1498 in Table 1) set forth in the present invention have been published, the expressible nucleotide sequences can be obtained directly by artificial synthesis. In this Example, synthesis of the gene fragments was outsourced respectively to the commercial companies such as Beijing Genomics Institute, GENEWIZ (Beijing) Co. Ltd., etc. When synthesized, a Bspl 19 I restriction site was introduced upstream of all these gene fragments, to facilitated construction of prokaryotic expression plasmids. Electrophoresis of an overlapping PCR amplification of the gene sequence encoding for the fusion protein RFP-hF VII-EGF2 is shown in Fig. 5.

### Example 4: Construction of the prokaryotic recombinant plasmids for expression of the RFP fusion proteins of other human-originated EGF domain proteins (PRT2 to PRT1498 in Table 1)

### 1. Pre-treatment of the genes and the prokaryotic expression vector pET19b

The gene fragments for other human-originated EGF domain proteins and the recombinant plasmid pET19bRFP-hF VII-EGF1 constructed in Example 2 were double digested with the restriction endonucleases Bspl 19 1 and BamH I (both purchased from TaKaRa or Fermentas) at 37°C overnight.

### 2. Other steps for ligation of the expression genes to the plasmid and identification were performed with reference to Example 2.

### Example 5: Expression from the recombinant plasmid pET19bRFP-hF VII-EGF1

1. The recombinant plasmid pET19bRFP-hF VII-EGF1 constructed in Example 2 was transformed into *E. coli* BL21(DE3) (purchased from Invitrogen); the single clone was picked into the LB medium containing 0.1 g/mL ampicillin and incubated at 37° C with shaking at 185 rpm to OD₆₀₀ of about 0.6, and 1 mL of the bacterial suspension was taken and frozen at -20 °C. Then, IPTG (isopropyl-β-D-thiogalactoside) was added to the remaining bacterial suspension at a final concentration of 0.8 mM. The incubation proceeded for 6h and 1mL of the bacterial suspension was taken and frozen at -20 °C.
2. The above-mentioned samples before and after IPTG induction were assayed by SDS-PAGE electrophoresis with 5% of the stacking gel and 12 % of the separating gel, in order to analyze expression of the protein of interest (see Fig. 6). The result indicated that the protein was expressed normally.

### Example 6: purification of the fusion protein RFP-hF VII-EGF1 by affinity chromatography

1. The bacterium RFP-hF VII-EGF1-pET19bBL21-(DE3) was cultured on a large scale according to the method in Example 5, induced by addition of IPTG, and cultured overnight at 18°C, with shaking at 175 rpm.
2. The above-mentioned bacterial suspension was centrifuged at 4000 x g for 3 min to collect the bacteria. The bacterial pellet was washed by resuspension in the EW buffer (20 mM sodium phosphate, 500 mM NaCl, I mM DTT, 0.1 mM PMSF, pH 7.4). The bacterial suspension was centrifuged at 4000 x g for 3 min, and the supernatant was discarded. Again, the bacterial pellet was resuspended with 10 mL of the EW buffer.
3. The bacteria were disrupted by sonication (power: 300 W; duration for sonication: 10 s; interval: 10 s; total time for sonication: 3 min; performed in ice-bath). After completion of the sonication, the bacterial suspension was centrifuged at 48,400 x g at 4°C for 1 h. The supernatant was collected and placed on ice.
4. The chromatographic column was preloaded according to the instruction of Co²⁺ purification system (Talon Metal Affinity Resin, Clontech company product), and the sample was loaded. The column was washed twice with 10 mL EW buffer and the protein impurities were washed out with 10 mM imidazole solution. Finally, the protein was eluted with 100 mM imidazole.
5. The cluent comprising the proteins of interest was ultrafiltrated with Tris-CI buffer (pH 7.4), for buffer exchange and concentration. After ultrafiltration, the high concentration of the fusion protein RFP-hF VII-EGF1 can be obtained. It was shown by protein quantitation that about 15 mg of the proteins of interest with more than 95% of purity can be obtained per litre of the expressing bacterium suspension.

### Example 7: Enzymatic cleavage of the fusion protein RFP-hF VII-EGF1 and purification of the recombination protein hF VII-EGF1

1. The Reasin Ni column (Ni column purchased from GE) was treated as follows: 200 µL of the Ni column mix was pipetted into a 1.5 mL EP tube (AxyGEN) and centrifuged at 700 × g for 2 min, the preservative solution for the Rcasin Ni column (20% ethanol) was discarded, then, the Ni column was washed twice with 1 mL deionised water, followed by equilibration of the Ni column with 1 mL binding buffer (0.5 M NaCl, 20 mM phosphate buffer, pH 7.4).
2. 450 µL of 3 µg/µL fusion proteins were mixed individually with the treated Ni column to homogeneity after equilibration of the Ni column, co-incubated at 4C for 1 h on a vertical rotary mixer.
3. After completion of incubation, the mixture was centrifuged at 4°C at 700 × g for 3 min. After completion of centrifugation, Ni beads obtained by centrifugation were washed twice with 500 µL of binding buffer. Ni beads were collected after completion of washing.
4. The beads were resuspended with 423 µL of binding buffer. After resuspending and mixing well, the resultant mixture was subjected to cleavage at 4°C with the rTEV proteinase (purchased from Shanghai Sangon). The mixture was placed on a vertical rotary mixer overnight with the time of period limited to 10 h. After completion of the enzymatic cleavage, the mixture was centrifuged at 700 x g at 4°C for 5 min. After completion of centrifugation, the supernatant was collected as the recombination protein hF VII-EGF1. The picture of Tricien gel analysis for identifying the recombination protein hF VII-EGF1 obtained after enzymatic cleavage of the fusion protein RFP-hF VII-EGF1 with rTEV is seen in Fig. 7. The enzymatic cleavage system was set up as follows:

| | |
|---|---|
| 20 × TEV buffer | 22.5 µL |
| 0.1 M DTT | 1.5 µL |
| Protein beads | 420 µL |
| rTEV enzyme | 3 µL |

### Example 8: Prokaryotic expression of the other recombination human-originated EGF domain proteins (PRT2 to PRT1498 in Table 1) and purification of the same

The other human-originated EGF domain proteins (PRT2 to PRT1498 in Table 1) can be expressed prokaryotically and then purified with the same methods as those in Examples 5-7. Partial profiles are shown in Figs. 6 to 25.

### Example 9: Construction of the recombinant eukaryotic plasmid for expressing the fusion protein RFP-hF VII-EGF1

### 1. The primers for PCR amplification as shown below were synthesized (by Invitrogen Biological Technology Co. Ltd.):

### 2. PCR Amplification System

The PCR amplification was performed with Primers 1 and 2 using the plasmid pET19bRFP-hF VII-EGF1 constructed in Example 2 as the template. A BamH I restriction enzyme cutting site and a Kozak sequence were introduced into the sequence of Primer 1, and an Xho I restriction enzyme cutting site was introduced into the sequence of Primer 2. The PCR reaction was performed with reference to Example 1.

### 3. Construction of the recombinant plasmid for eukaryotic expression of the fusion protein RFP-hF VII-EGF1

The cloned gene fragment RFP-hF VII-EGFI and the eukaryotic expression vector pcDNA3.1 (+) (the product from Novagen) were double digested with the restriction endonucleases BamH I and Xho I (both purchased from TaKaRa or Fermentas), and the reaction systems were set up with reference to Example 2. The steps for ligating the gene fragment to the eukaryotic expression vector and for transforming and identifying the recombinant plasmid were performed with reference to Example 2.

Finally, the recombinant plasmid comprising the RFP- hF VII-EGF1 gene with the correct sequencing result was designated as pcDNA3.1-RFP-hF VII-EGFI. The plasmid map of the recombinant plasmid is seen in Fig. 26.

### Example 10: Construction of a stable cell line for expressing the fusion protein RFP-hF VII-EGF1

### 1. Cell preparation

DG44 cells (purchased from ATCC) were cultured in the α-MEM complete culture medium (containing L-glutamine, ribonucleic acid and deoxyribonucleic acid, purchased from Hiclony) supplemented with 10% FBS (fetal bovine serum, purchased from Hiclony). On the day before transfection, DG44 cells were seeded in a 6-well plate at 4×10⁵ cells/mL with 2 mL per well.

### 2. Cell transfection

Cells were transfected by using liposome method following the instruction of Lipofectamine™ 2000 kit (purchased from Invitrogen). The detailed steps are as follows:
1) The cell culture medium was replaced with serum-free α-MEM medium, 2 mL/well.
2) 4.0 µg of the recombinant eukaryotic expression plasmid pcDNA3.1-RFP-hF VII-EGFI constructed in Example 9 was mixed with 250 µL Opti-MEM, while 10 µL of Lipofectamine™ 2000 was mixed with 250 µL of Opti-MEM and incubated for 5 min.
3) The two solutions were mixed well and incubated for 20 min. The liposome complexes were added into the well with cells, gently mixed, placed into an incubator and cultured at 37°C.
4) After 5 h, the medium was replaced with the α-MEM complete culture medium supplemented with 10 % FBS.

### 3. Screening and expanding of the positive cell clones

After 24 h of transfection with the recombinant plasmid, the cells were transferred into 100 mm petri dishes at a ratio of 1:2000. G418 (purchased from Gibco) was added at a final concentration of 800 µg/mL for screening. Cells transfected with an empty vector or not transfected were used as controls. After 15 days, the single clones were picked into and cultured in 24-well plates, and sequentially transferred into 6-well plates and into 100 mm petri-dishes for expanding culture once cellular confluence was reached. When the cell confluency was 90%, cells were frozen at a rate of 1:4 in FBS with 10% DMSO (purchased from Gibco).

### 4. Identification of the positive cell clones

The picked positive cell clones were identified by the genomic PCR and RT-PCR. The fragment of interest could be amplified by both of the techniques, which demonstrated that the fusion gene has been integrated into the genome of the cell and a functional mRNA is transcribed. The stable cell line obtained was designated as DG44-RFP-hF VII-EGF1.

### Example 11: Collection of the eukaryotically expressed fusion protein RFP-hF VII-EGF1

1. The DG44-RFP-hF VII-EGF1 cells were cultured in the α-MEM complete culture medium containing 10% FBS supplemented with 400 µg/mL G418, and passaged into 10 plates.
2. When the cell confluency in each plate reached to more than 90%, the α-MEM complete culture medium containing 10% FBS was replaced with CHO serum-free medium, and supplemented with Vitamin K (Sigma) to a final concentration of 1 µg/mL.
3. After 3 days of cultivation, the cellular pellet was collected and disrupted. Then, the supernatant was obtained by centrifugation, purified by cobalt ion affinity chromatography and identified by SDS-PAGE plus Western Blot. The steps were referred to those in Example 6.

### Example 12: Enzymatic cleavage of the eukaryotically expressed fusion protein RFP-hF VII-EGF1 and recovery of hF VII-EGF1

The experimental method followed that in Example 7.

### Example 13: Eukaryotic expression of the other human-originated EGF domain proteins (PRT2 to PRT1498 in Table 1) and purification of the same

According to the methods of Examples 9-10, the eukaryotic expression plasmids for the RFP fusion proteins of other human-originated EGF domain proteins (PRT2 to PRT1498 in Table 1) were constructed and the cell lines stably expressing these fusion proteins were established.

According to the methods of Examples 11-12, the eukaryotically expressed fusion proteins were collected and subjected to enzymatic cleavage, and the other human-originated EGF domain proteins (PRT2 to PRT1498 in Table 1) were finally obtained.

### Example 14: Determination of the antibacterial activity of the recombinant protein hF VII-EGF1

1. *E. coli* DH5α was exemplified. *E. coli* DH5α was streaked and cultured on LB solid medium. Then, a typical clone was picked and inoculated into common LB liquid medium, cultured at 37°C with shaking at 185 rpm until the logarithmic growth phase was reached (OD₆₀₀ of about 0.5). The bacterial culture was centrifuged at 4000 x g for 3 min, the supernatant was discarded, and the thalli were resuspended with fresh LB medium.
2. The above-mentioned bacterial suspension was diluted and inoculated into a 96-well plate with the total volume of 100 µL per well and a bacterial count of 5 x 10⁵ CFU/mL.
3. The recombinant protein hF VII-EGF1 was added to the above-mentioned wells with the final protein concentration in the well of the highest protein concentration being 50 µg/mL. Basing on this final concentration, a two-fold serial dilution was performed till a protein concentration gradient consisting of 50, 25, and 12.5 µg/mL was set up. Three parallel wells were set up for each concentration in the gradient.
4. The above-mentioned 96-well plate was placed and cultured at 37°C in shaking incubator shaking at 175 rpm. Samples were taken every 30 min, the absorbance values were determined at 600 nm wavelength under a ultra violet spectrophotometer, and an inhibition curve was plotted. Judgement on MIC: After 8 h of cultivation, the growth was observed with naked eyes or judged under the ultra violet spectrophotometer at a wavelength of 600 nm. The minimal inhibitory concentration MIC is defined as the lowest concentration of the recombinant protein at which there is no growth of the bacteria.

The growth curve of *E*. *coli* DH5α treated with the recombinant protein hF VII-EGF1 is seen in Fig. 27. The curve indicates that the recombinant protein hF VII-EGFI exhibits a significant inhibitory effect on the growth of *E. coli* DH5α, with the MIC being 12.5 (µg/mL.

### Example 15: Determination of antibacterial activities of the other human-originated EGF domain proteins (PRT2 to PRT1498 in Table 1)

According to the experimental procedures in Example 14, the minimal inhibitory concentrations (MIC, µg/mL) of the other human-originated EGF domain proteins (PRT2 to PRT1498 in Table 1) for *E. coli* DH5α were determined. The results are shown in Table 2:

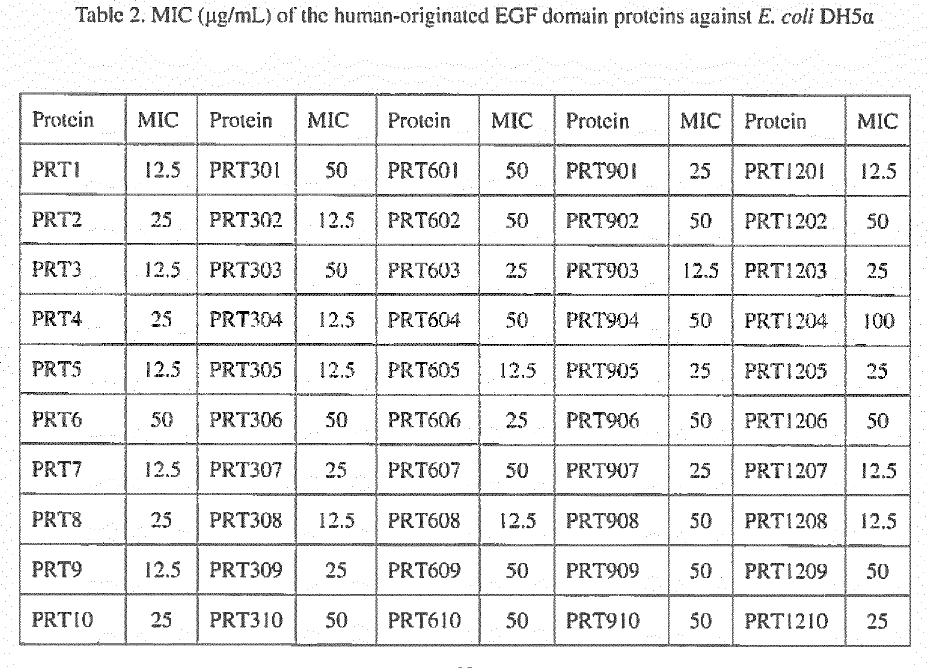

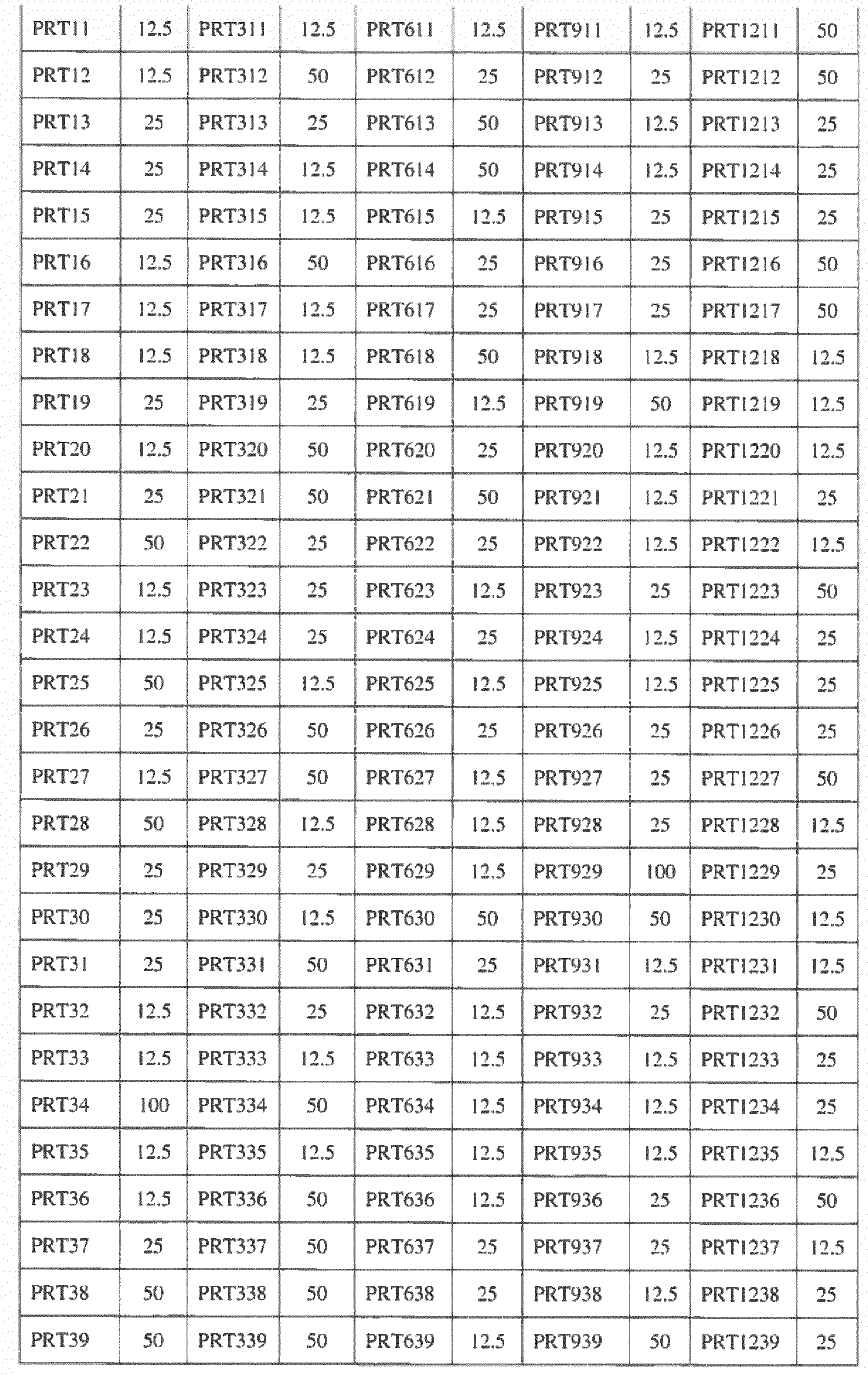

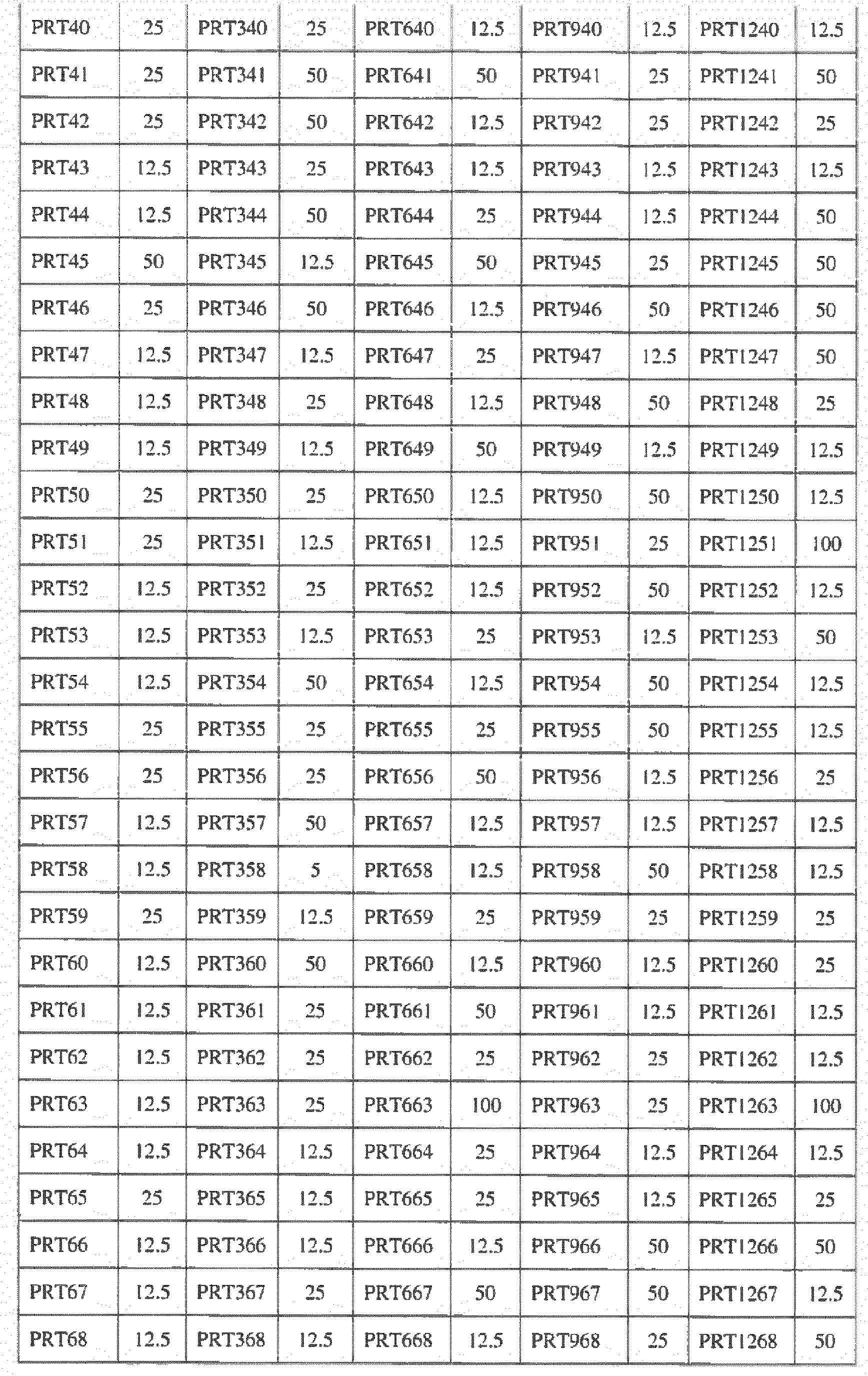

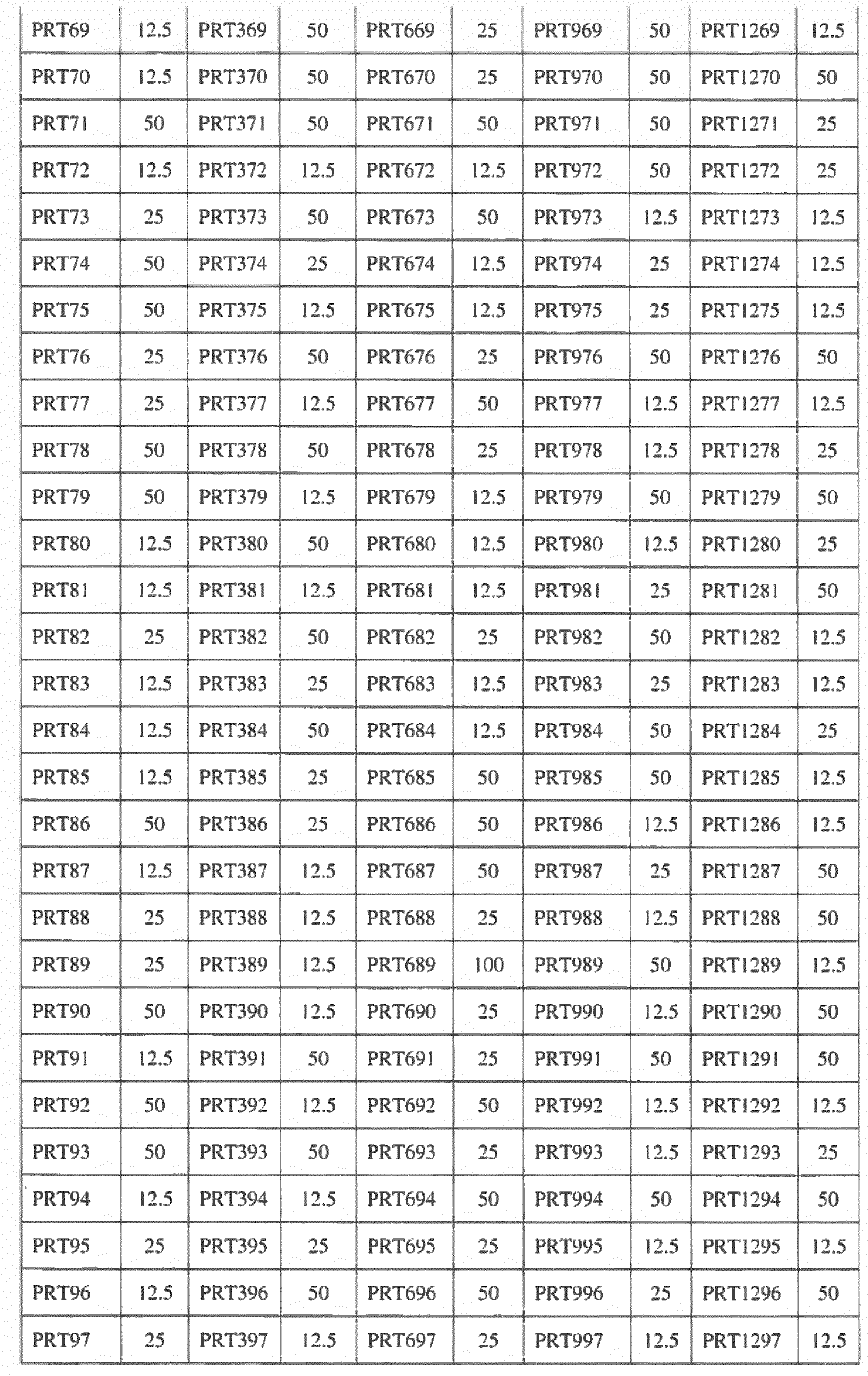

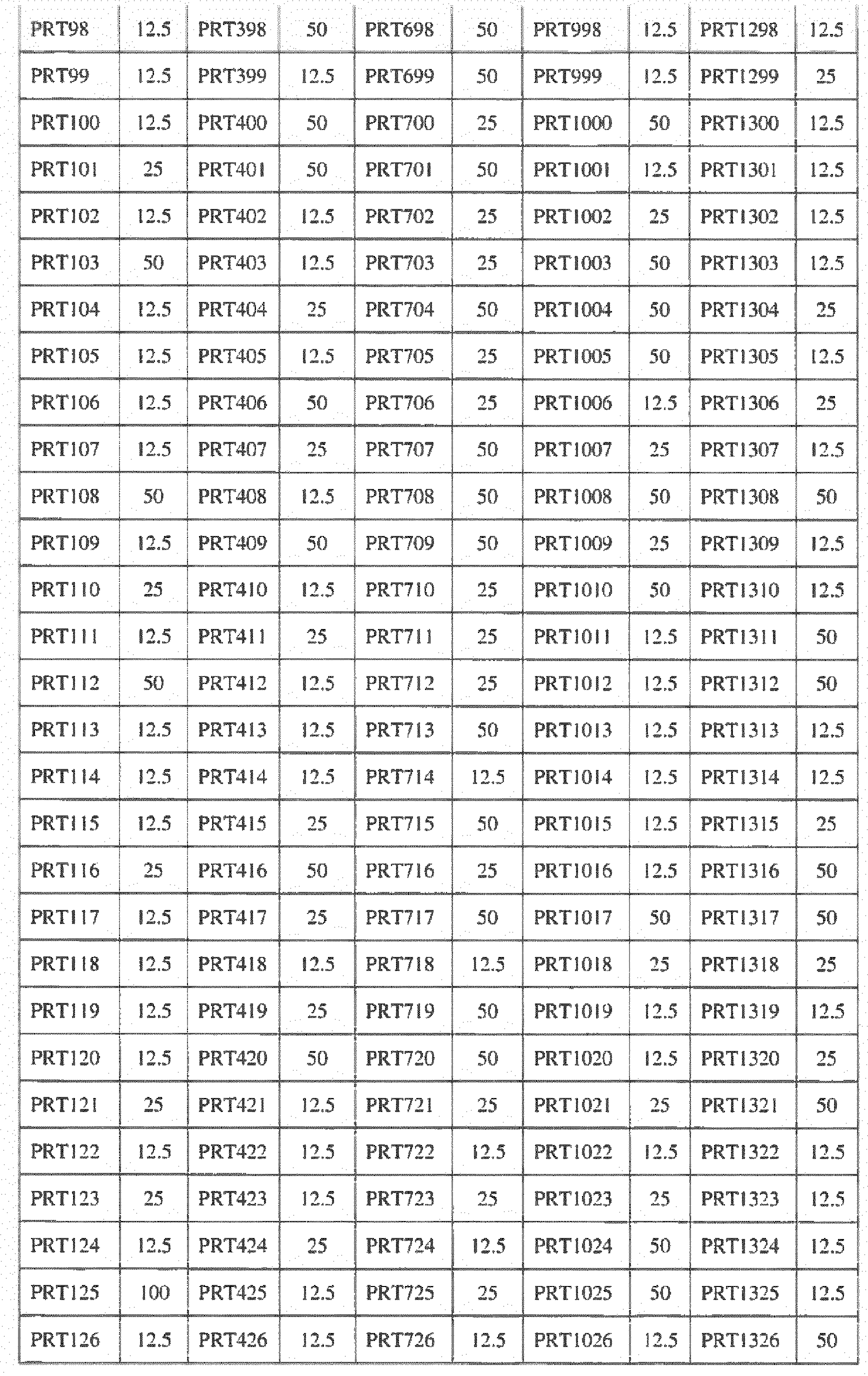

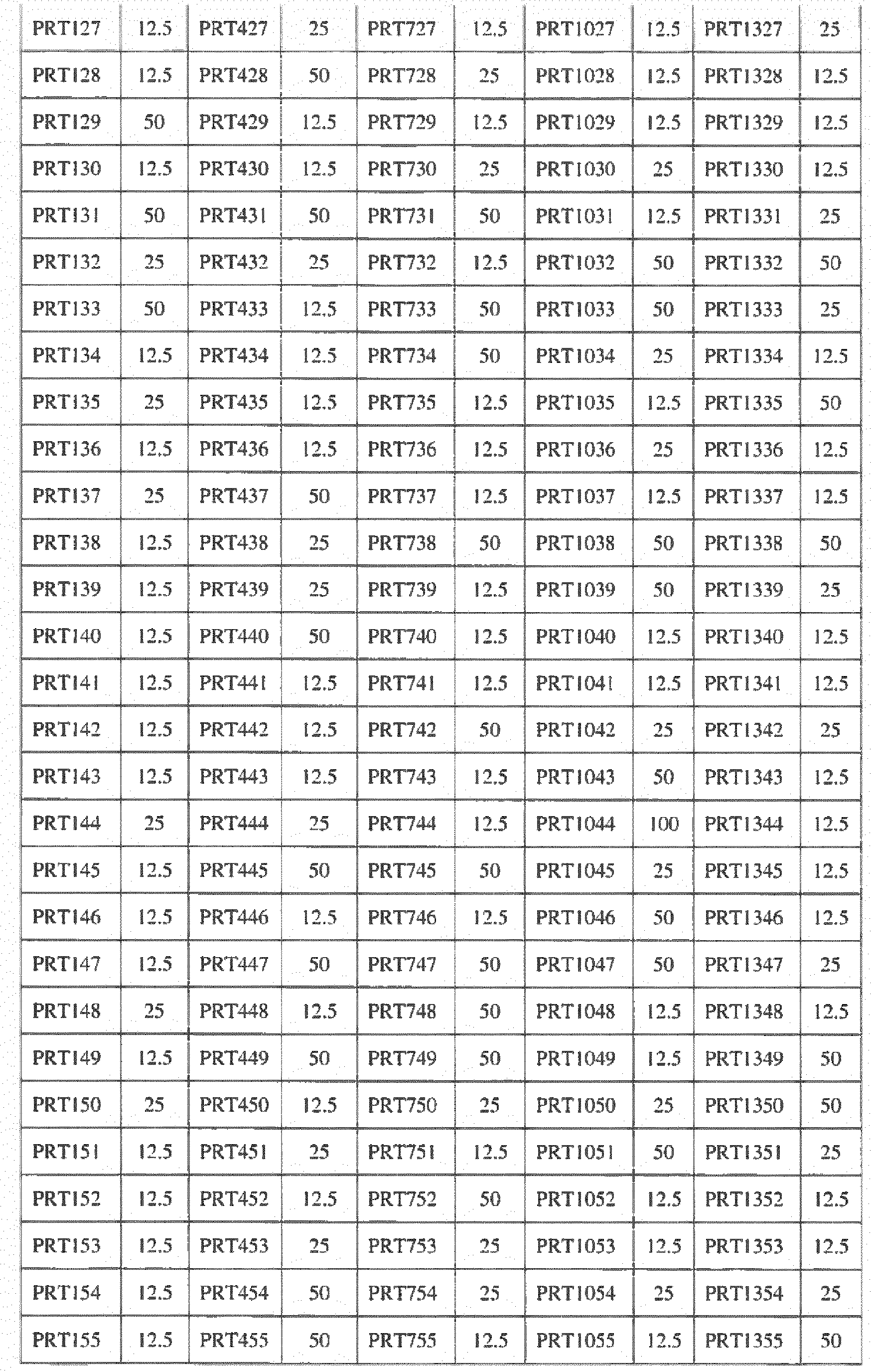

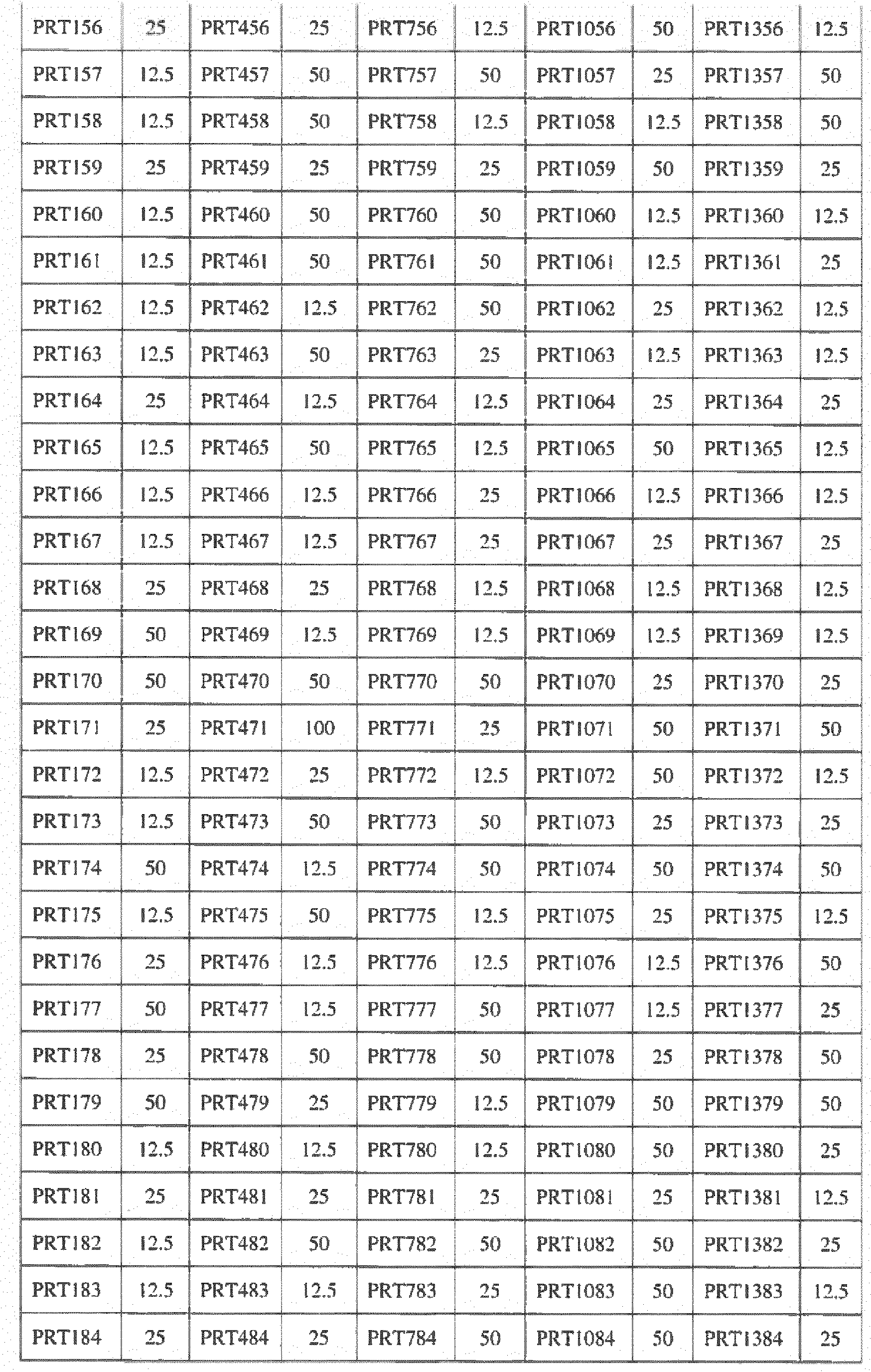

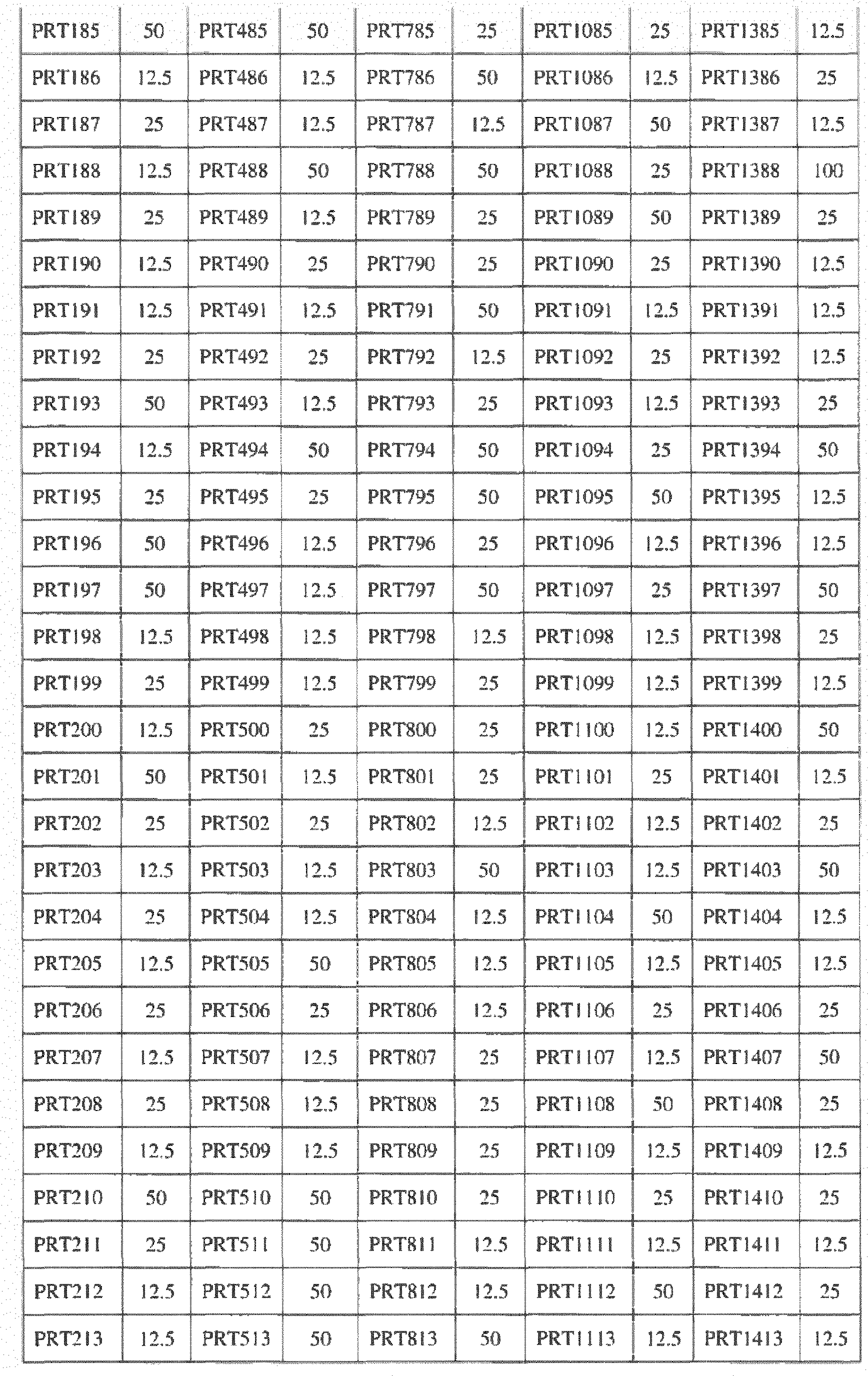

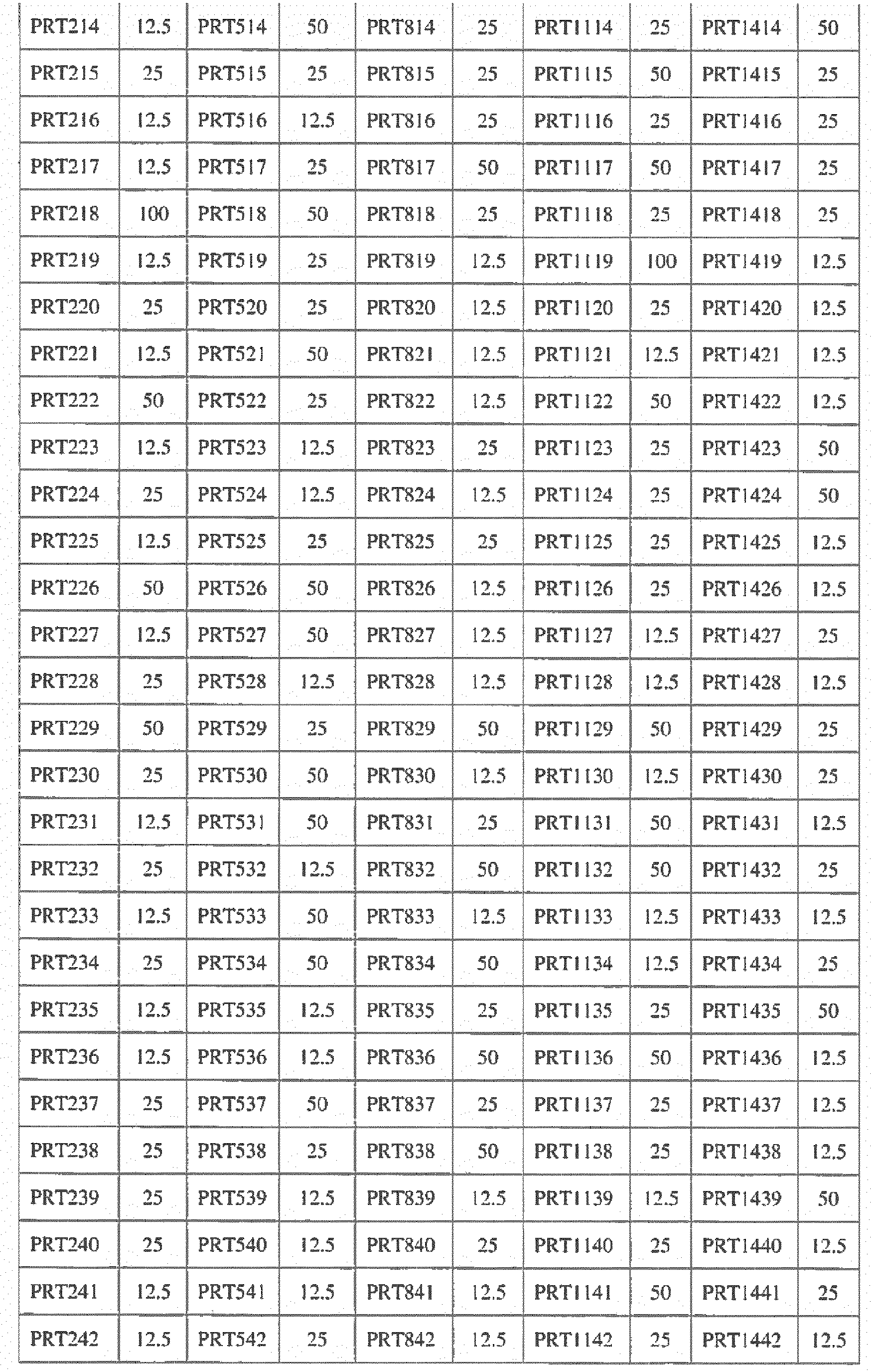

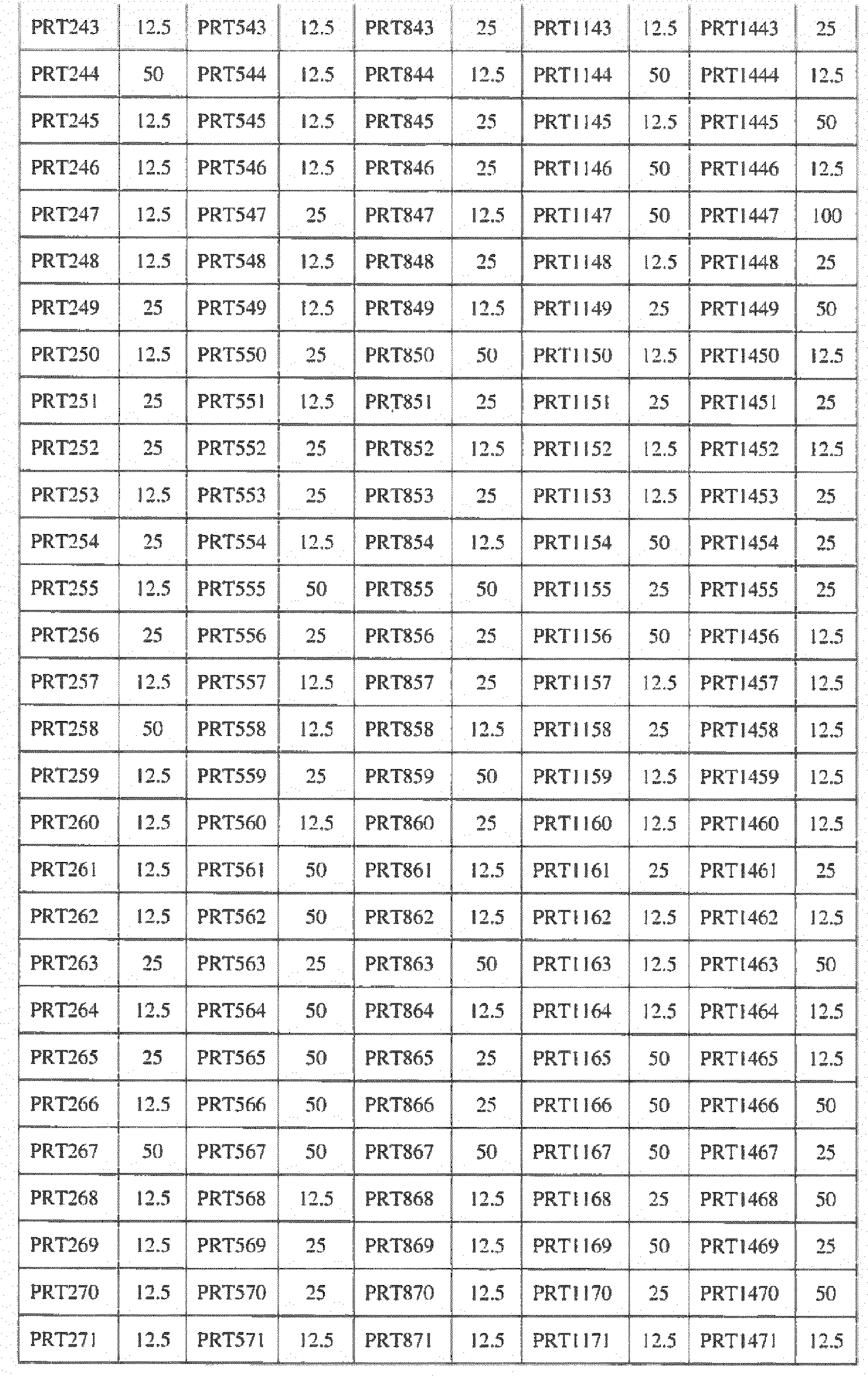

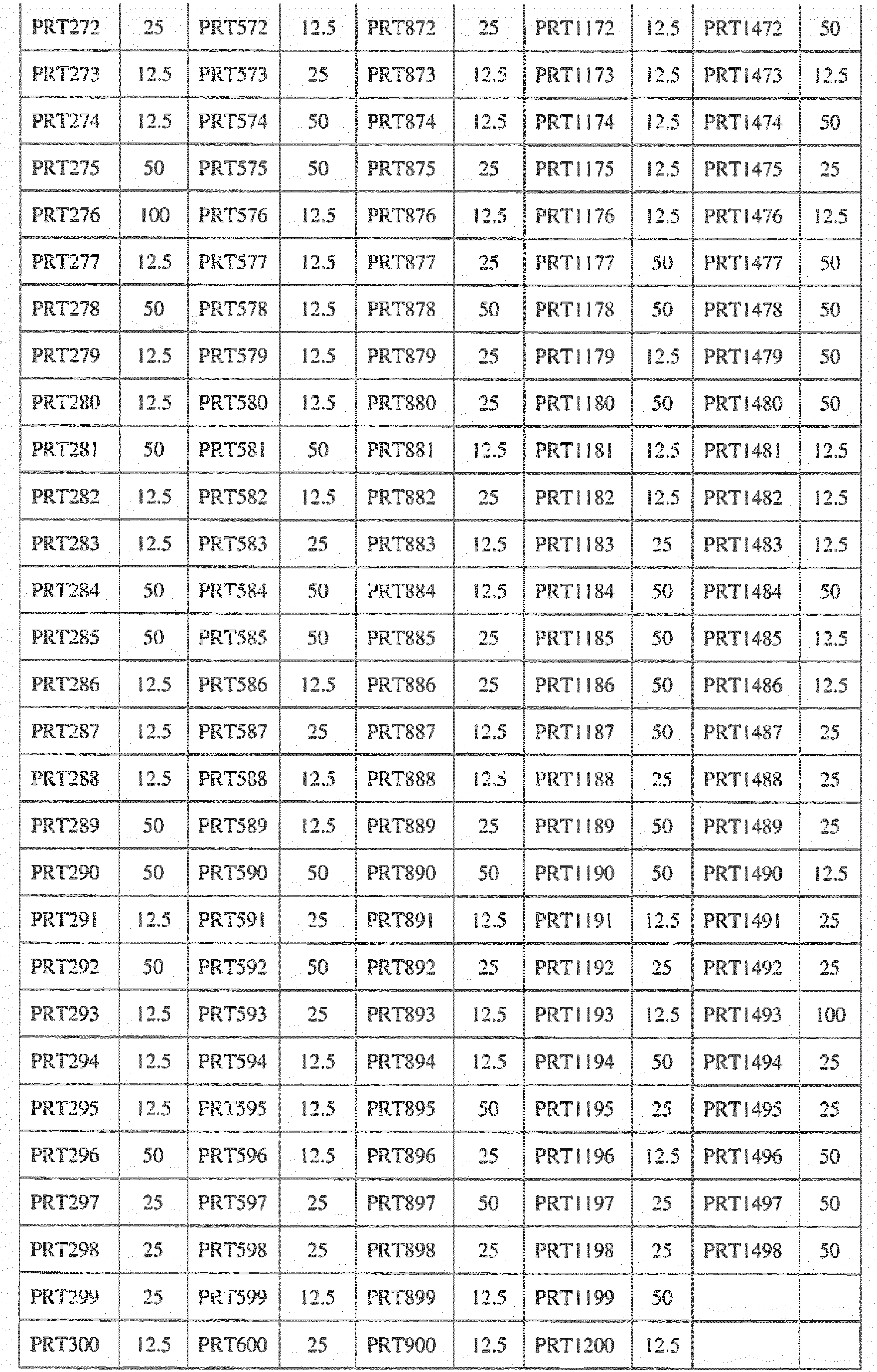

It can be known from the above table that: all of the human-originated EGF domain proteins (PRT1 to PRT1498 in Table I) have an inhibitory effect on *E. coli* DH5α.

### Example 16: Effect of the human-originated EGF domain proteins (PRT1 to PRT1498 in Table I) such as the recombination protein hF VII-EGF1, etc on the growth of various Gram-negative bacteria

1. Gram-negative bacteria, such as *E. coli* BL21, *Pseudomonas aeruginosa, Klebsiella pneumonia, Enterobacter cloacae, Aeromonas hydrophila, Citrobacter diversus, Moraxella catarrhalis, Proteus mirabilis, Proteus vulgaris, Serratia marcescens,* etc were streaked on solid LB medium and cultured respectively. Then, typical clones were picked, inoculated into common liquid LB medium respectively, and cultured at 37 °C with shaking at 185 rpm until the McFarland specific turbidity was 0.5. The bacterial culture of each of Gram-negative bacteria was centrifuged at 4000 x g for 3 min, the supernatant was discarded, and the thalli were resuspended with fresh LB medium.
2. The above-mentioned bacterial suspensions were diluted and inoculated into 96-well plates with the final concentration of the bacterial suspension being 5 x 10⁵ CFU/mL per well.
3. Each of the purified human-originated EGF domain proteins (PRT1 to PRT1498 in Table 1) was added to each of the above wells and subjected to two-fold serial dilution in concentration. Three parallel wells were set up for each concentration in the gradient.
4. The above-mentioned 96-well plates were placed and cultured at 37°C in shaking incubator shaking at 175 rpm. Judgement on MIC: After 10 h of cultivation, the growth was observed with naked eyes or judged under the ultra violet spectrophotometer at a wavelength of 600 nm. The minimal inhibitory concentration MIC is defined as the lowest concentration of the recombinant protein at which there is no growth of the bacteria. The results are shown in Table 3:

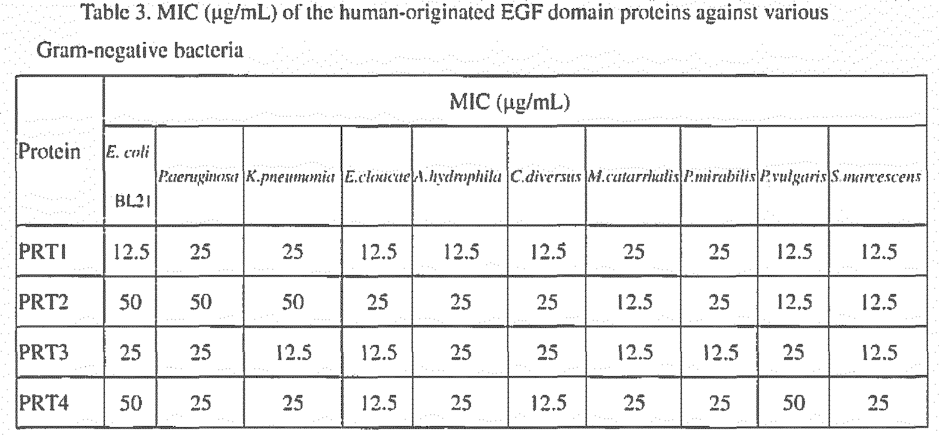

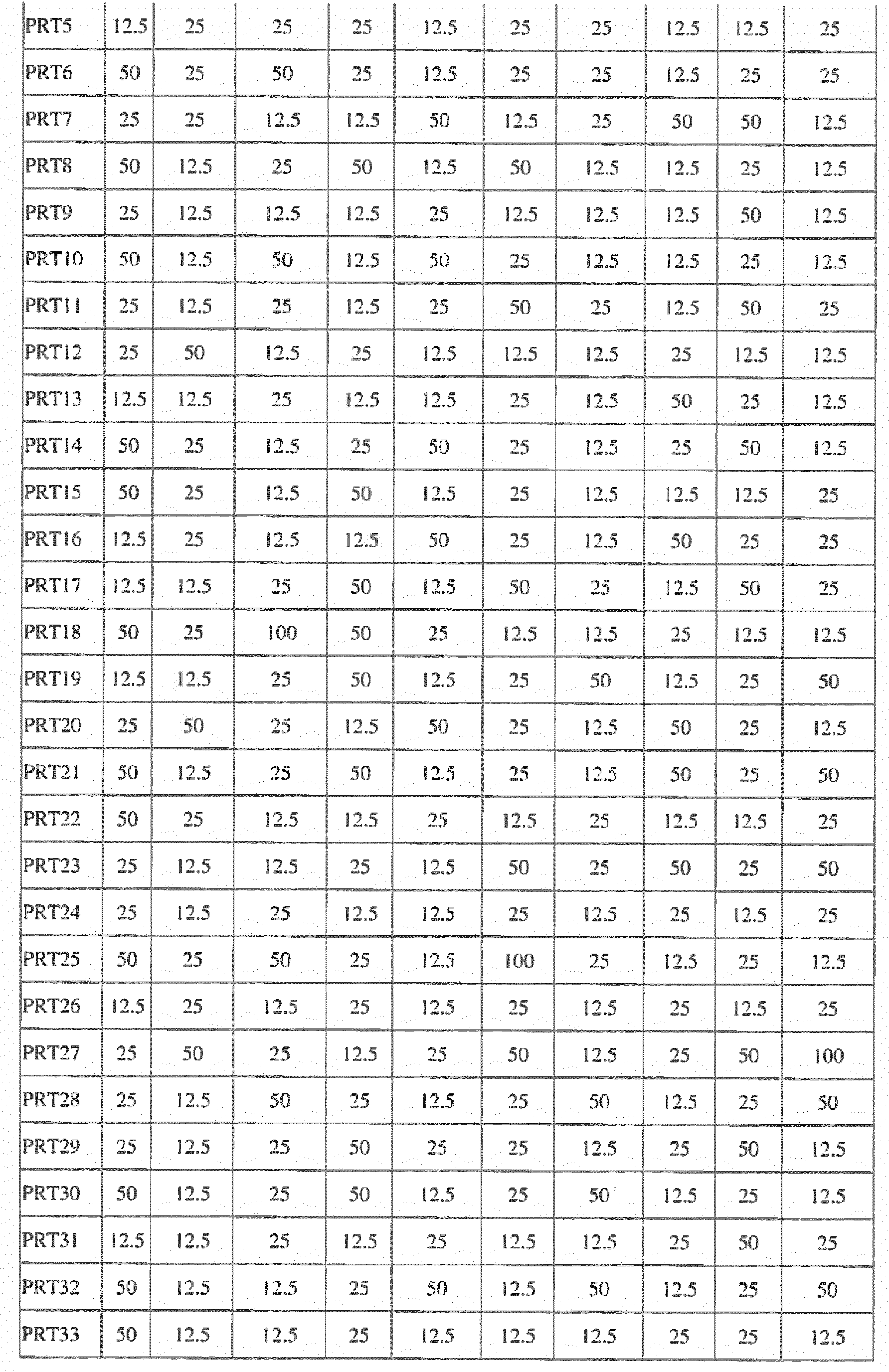

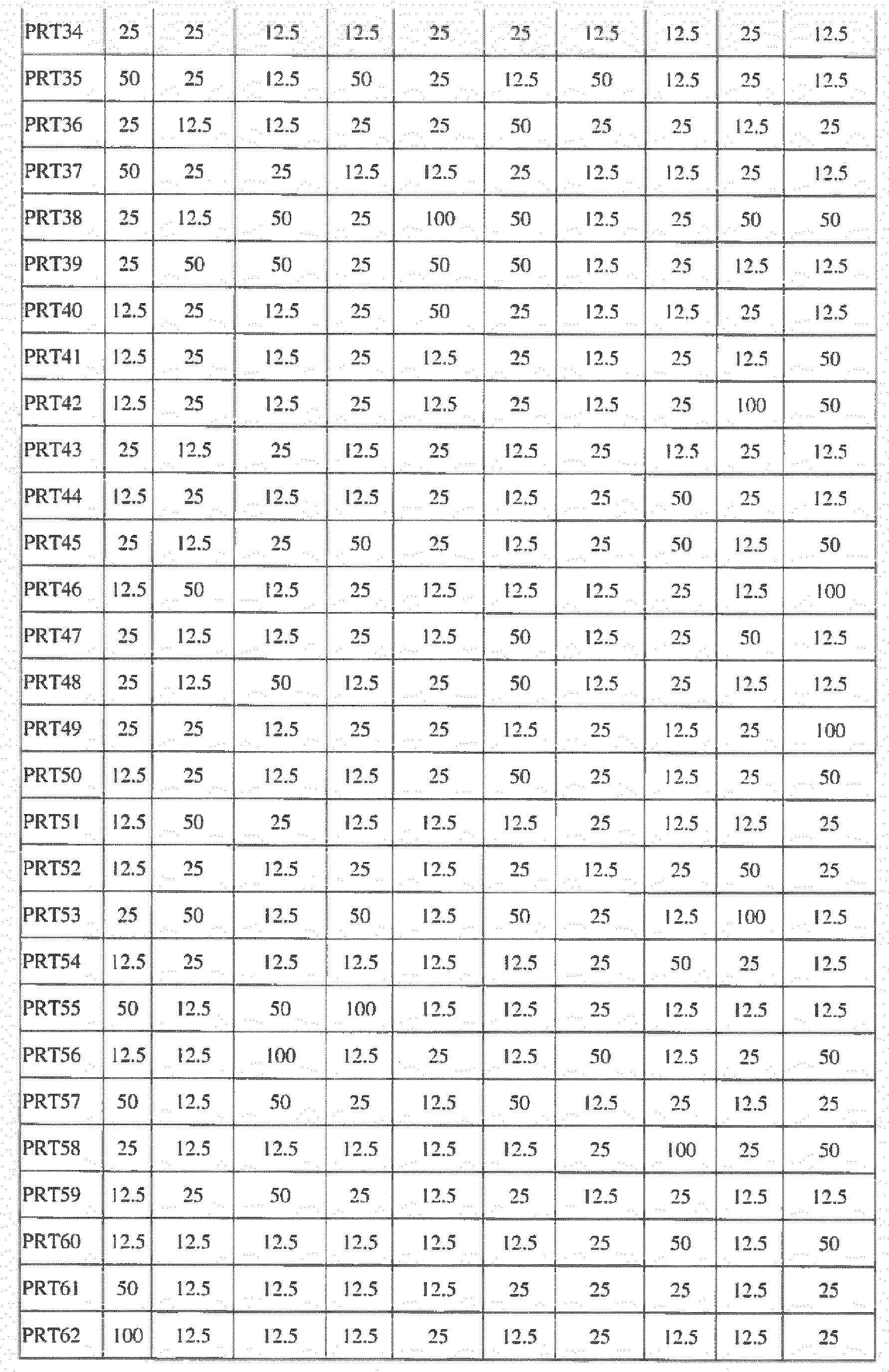

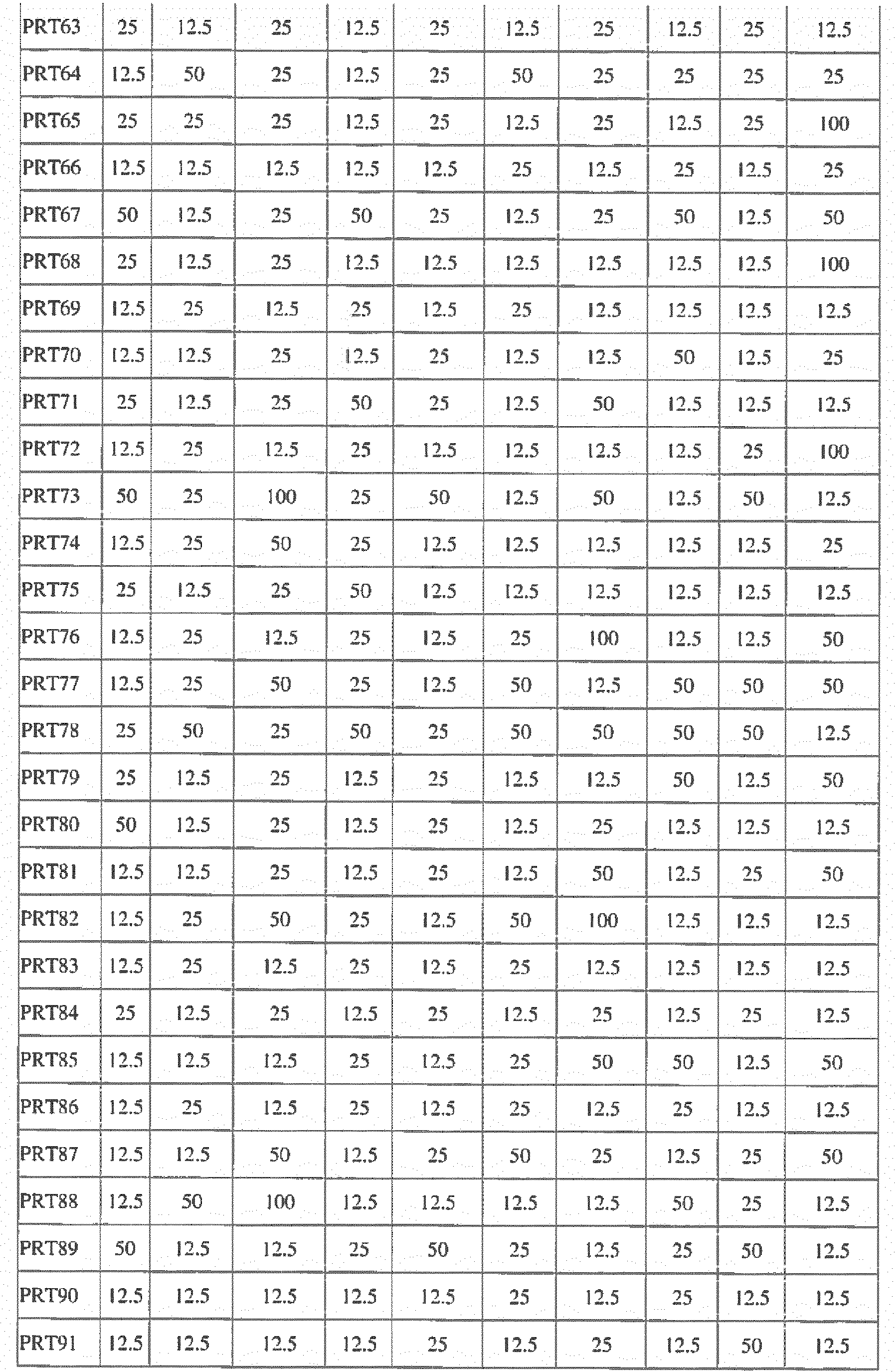

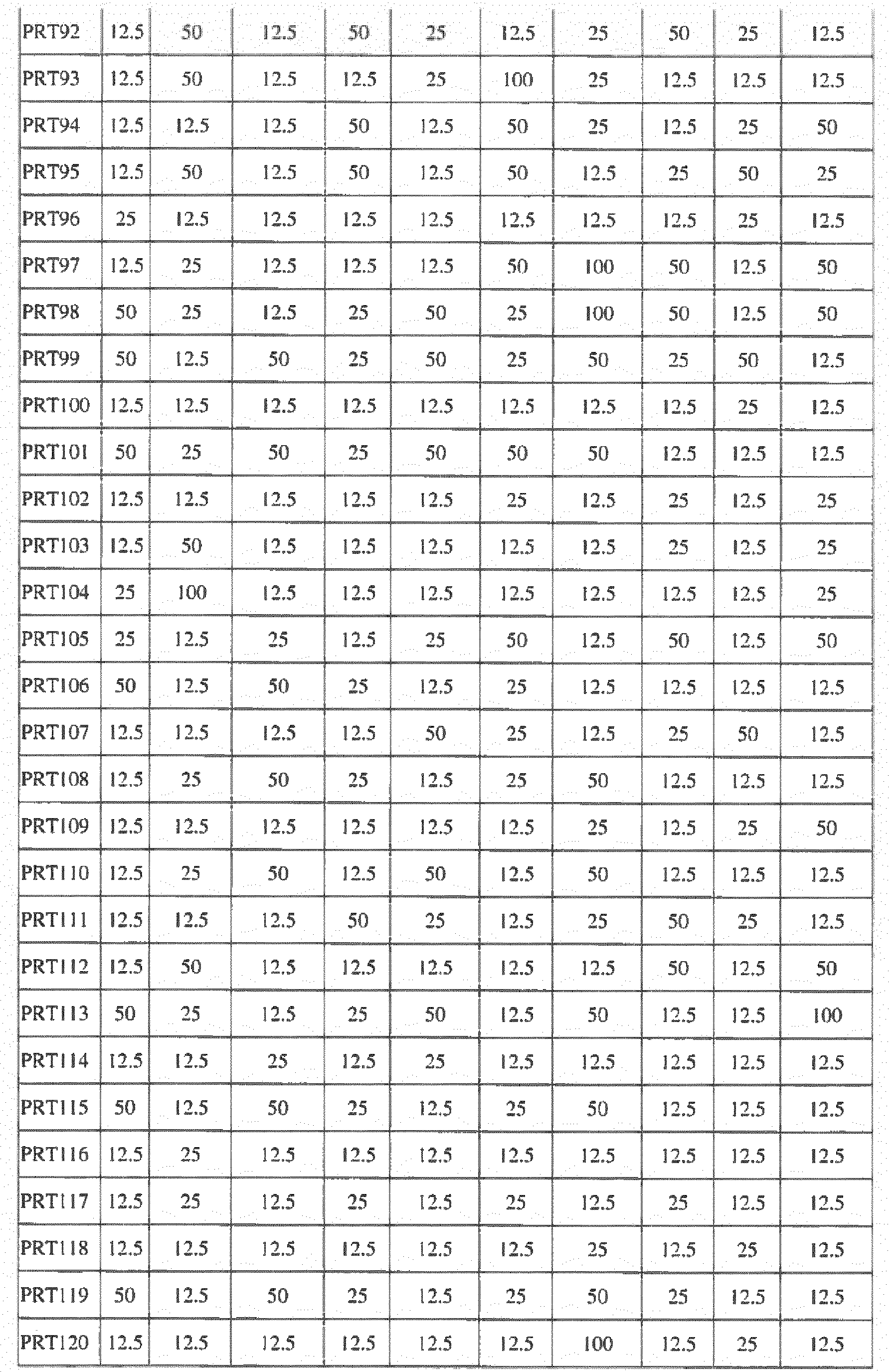

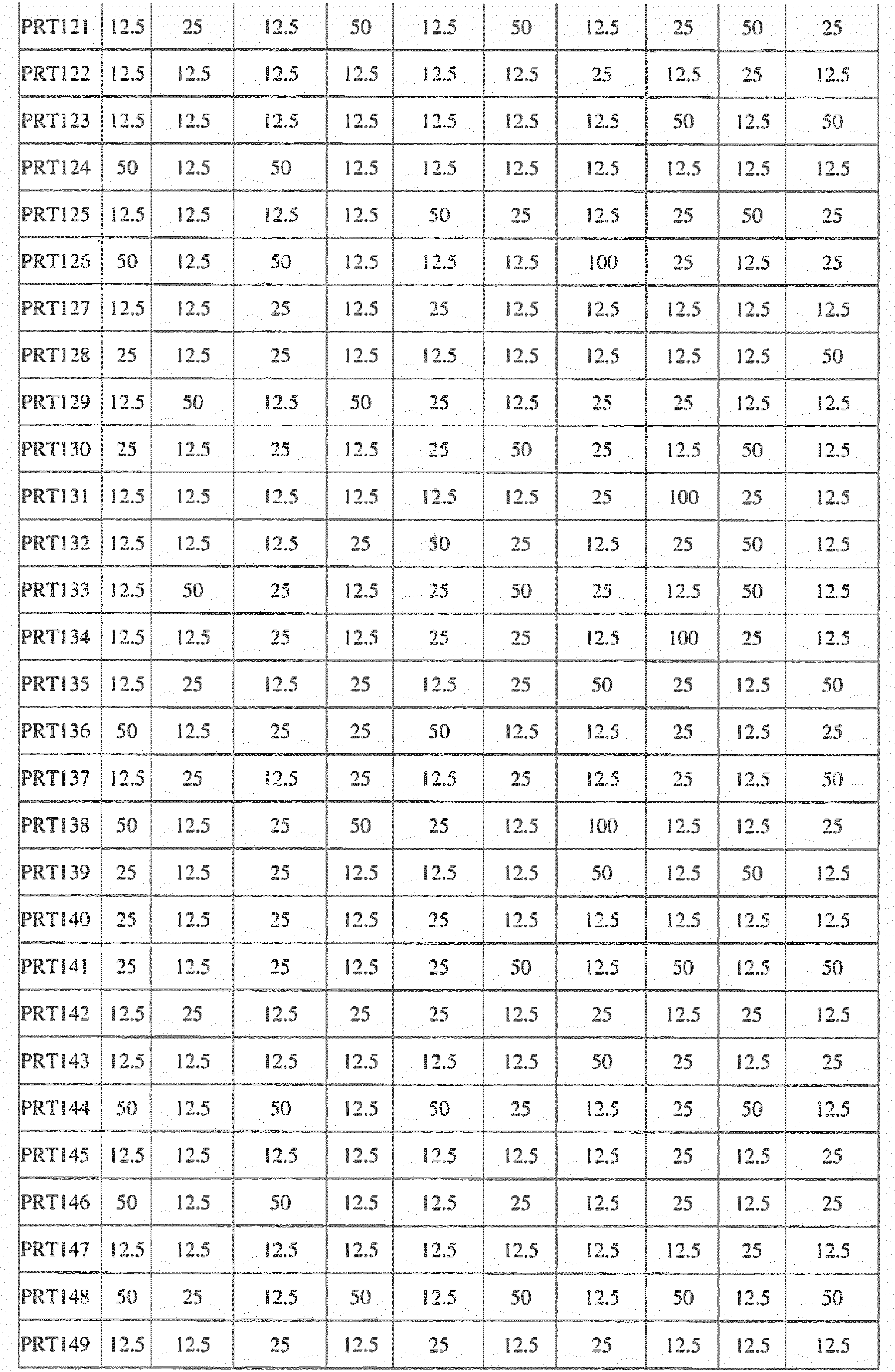

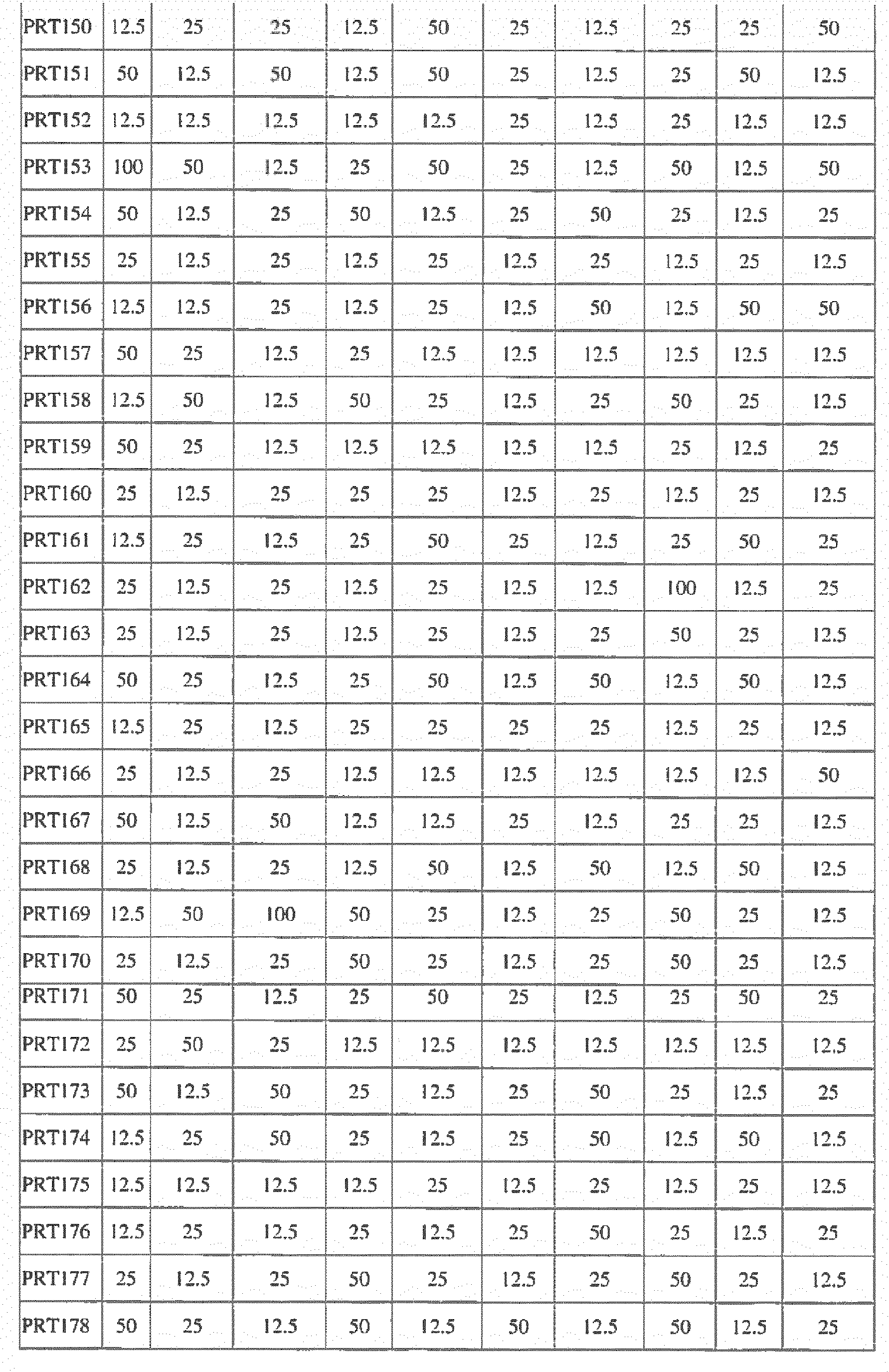

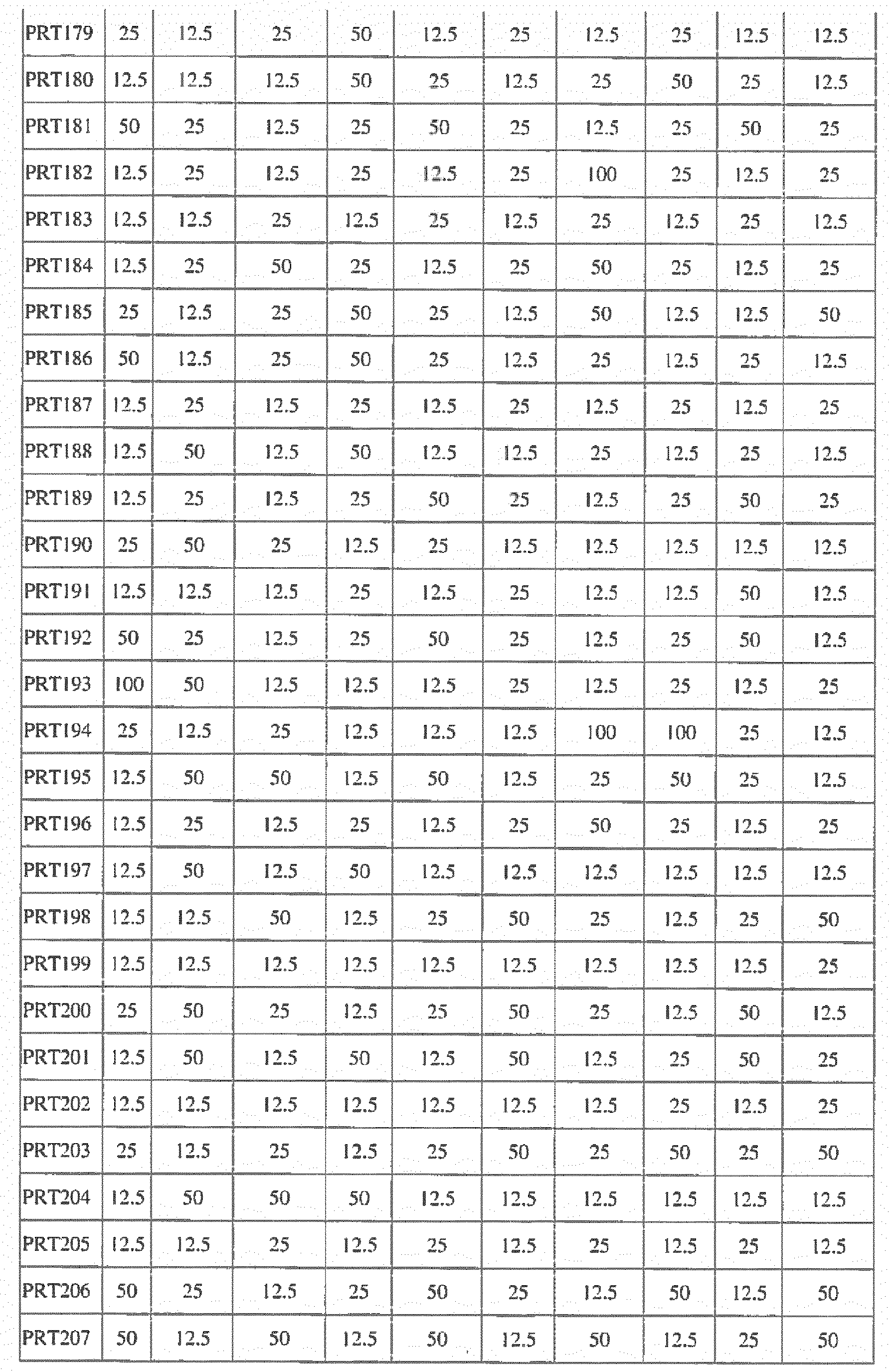

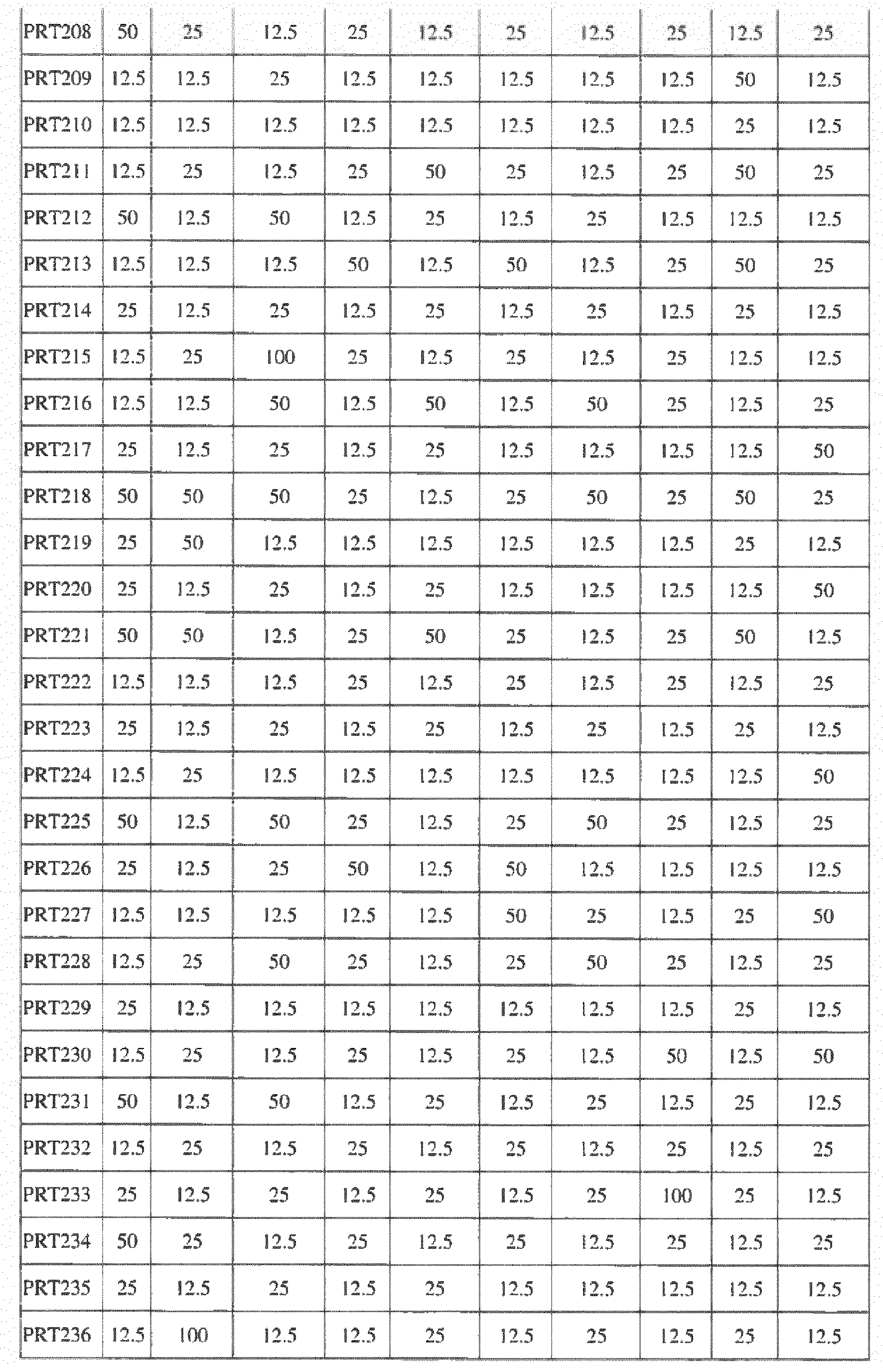

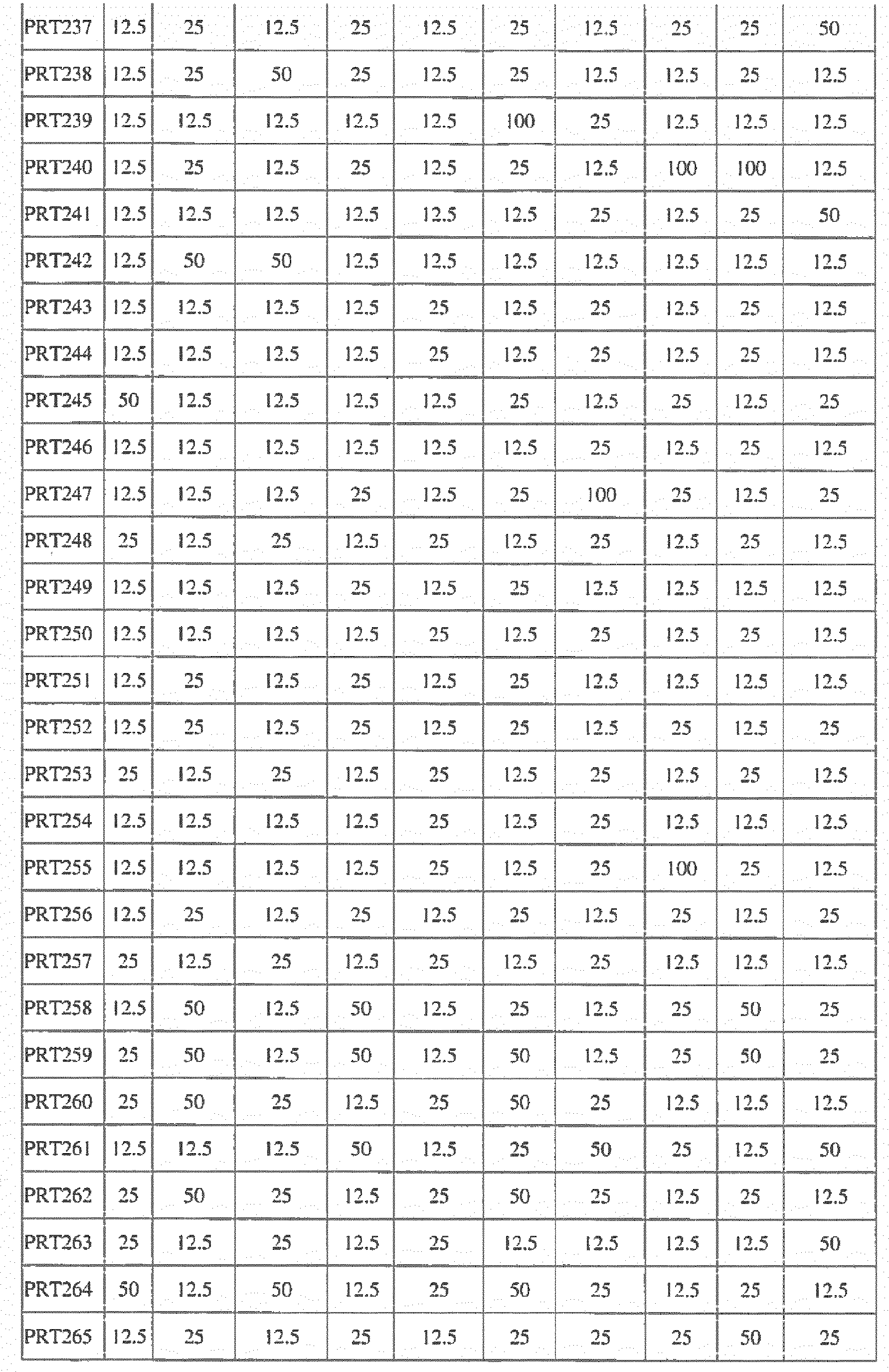

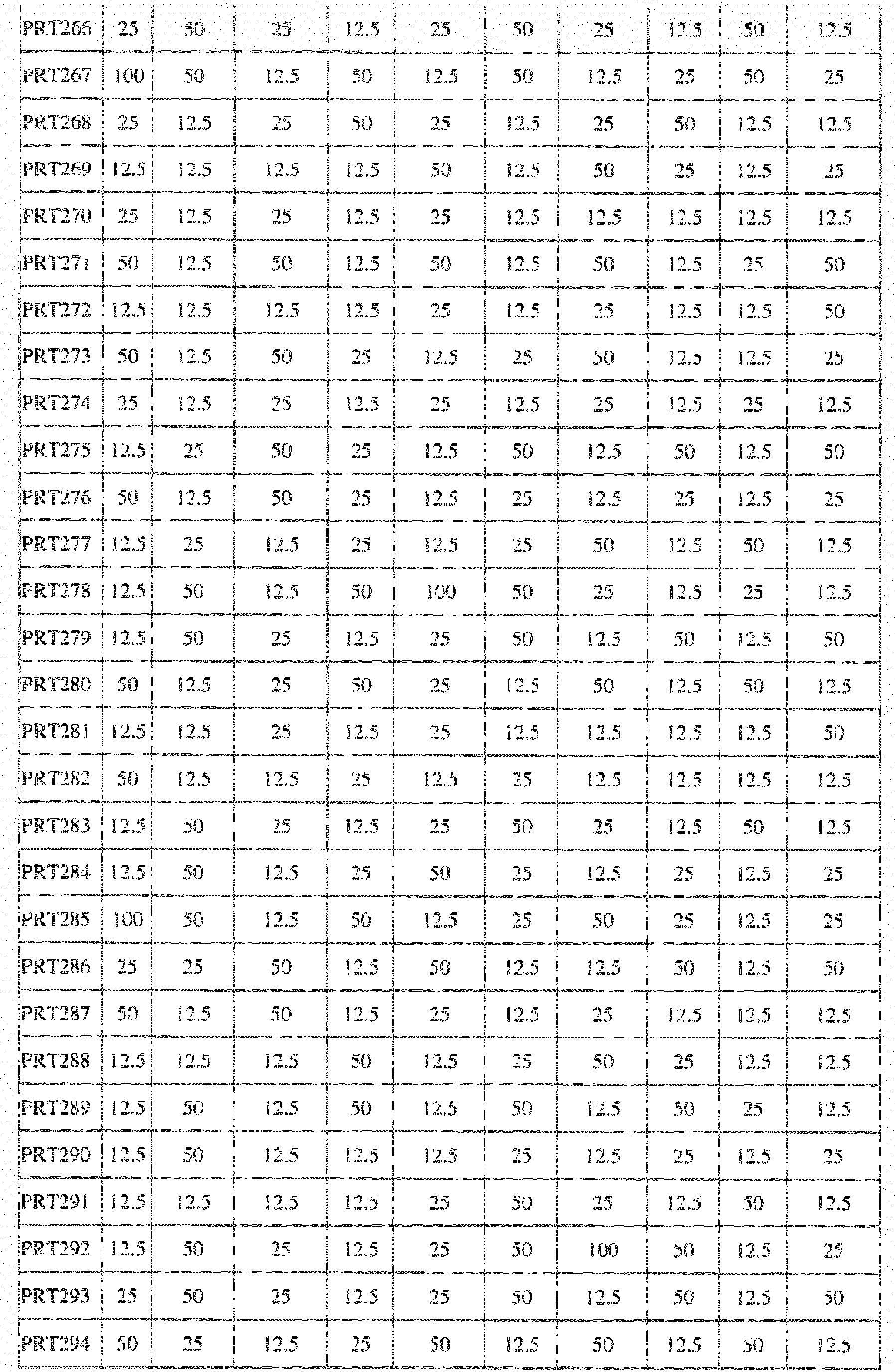

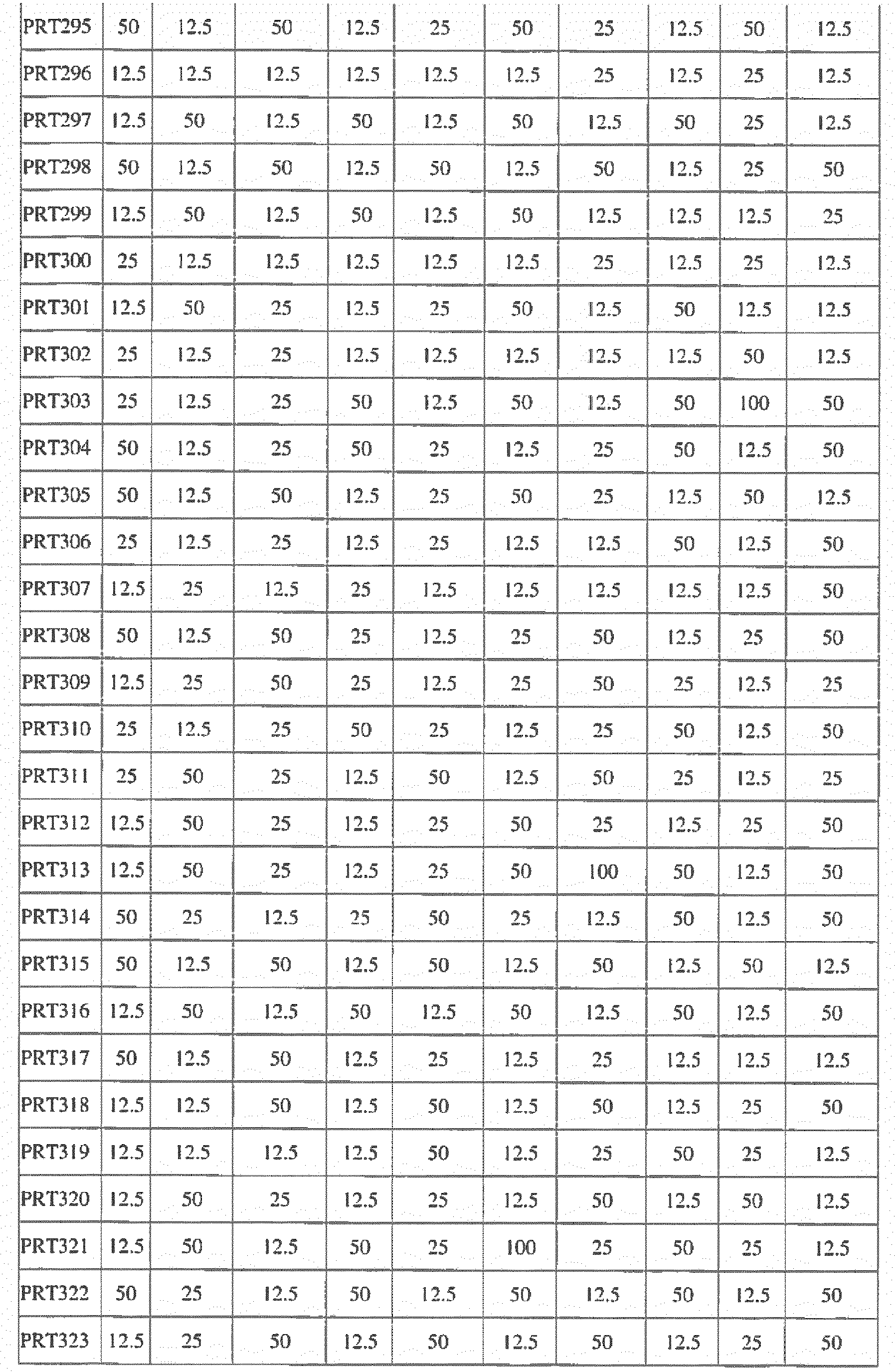

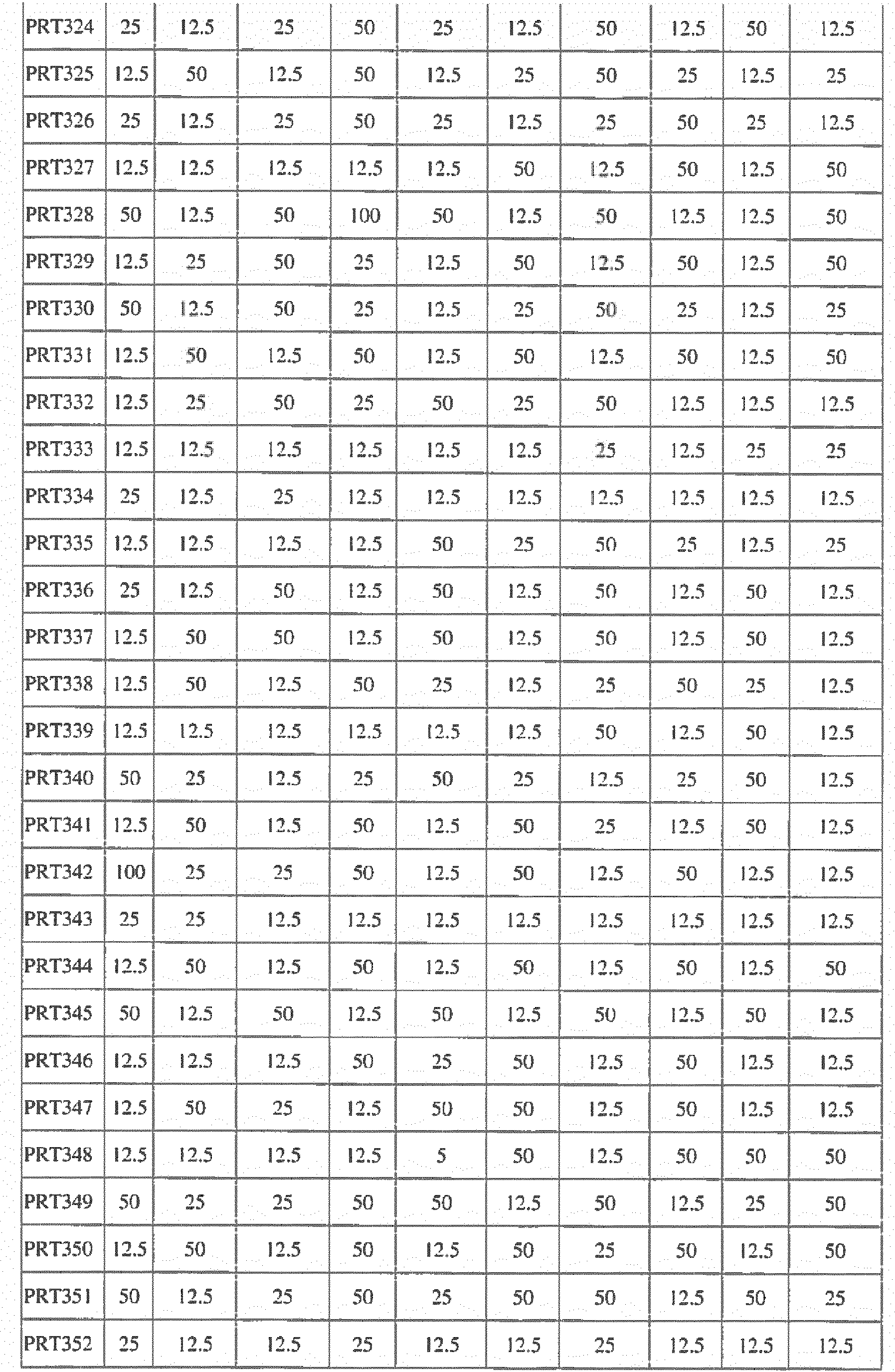

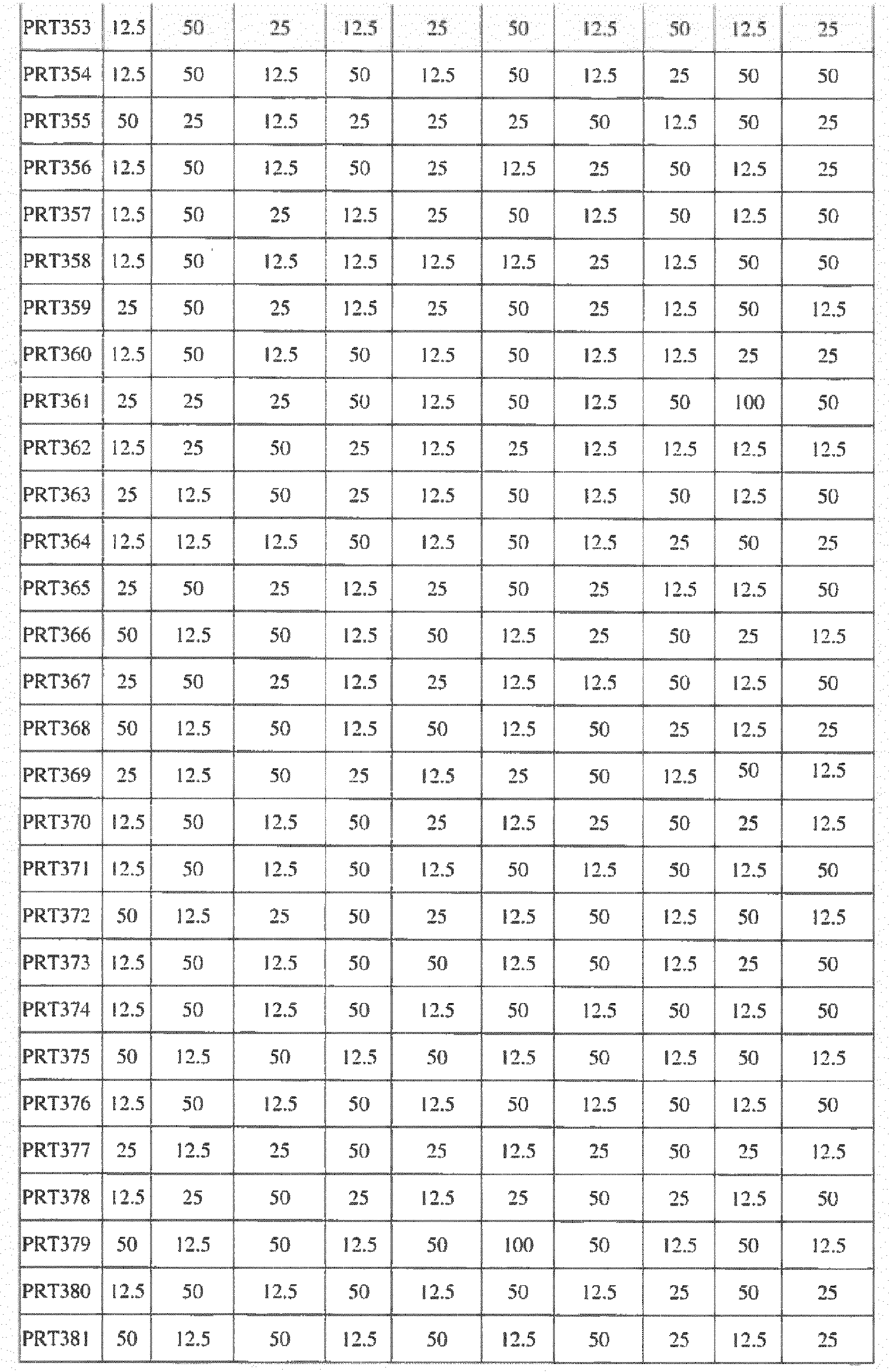

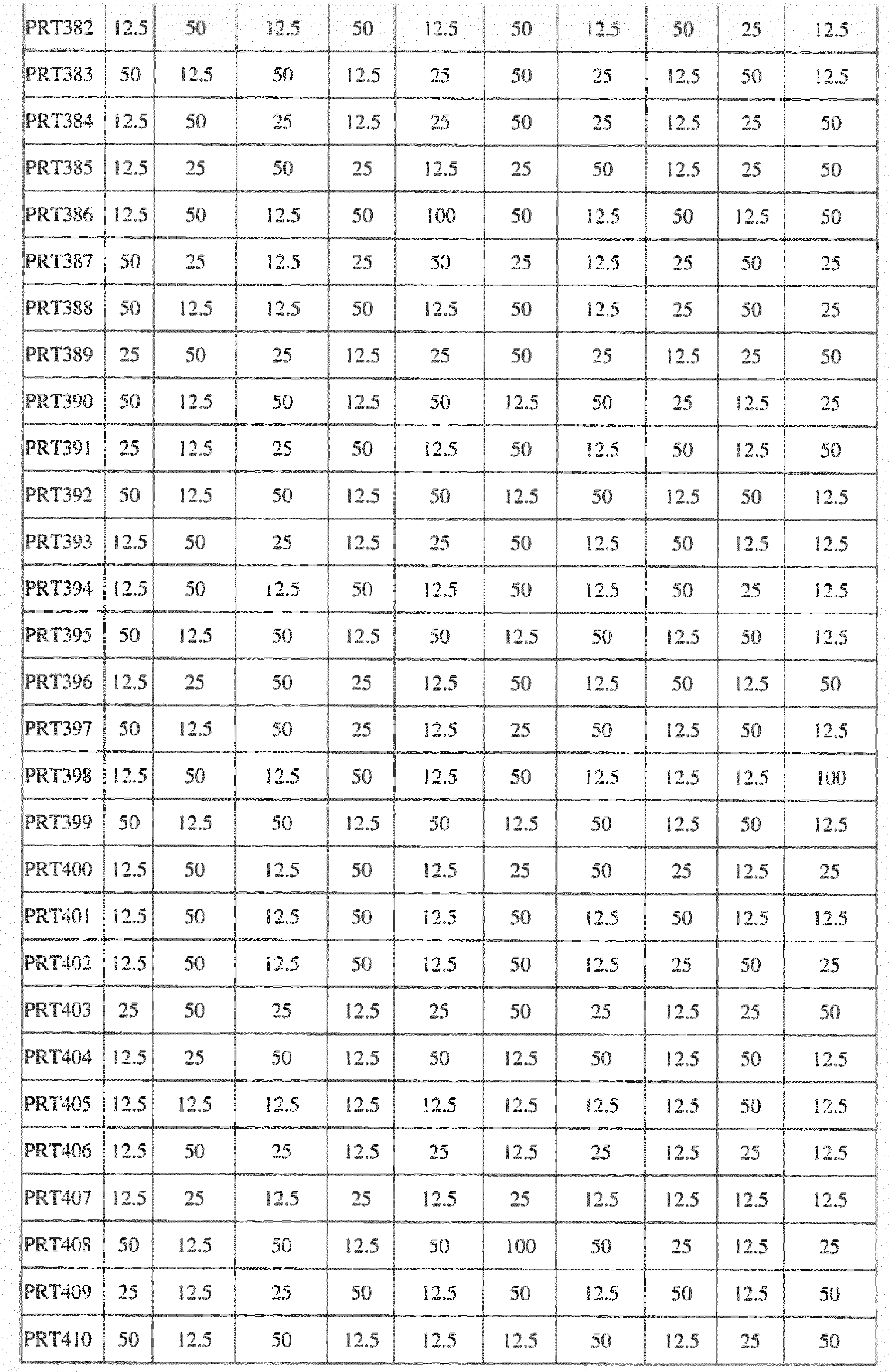

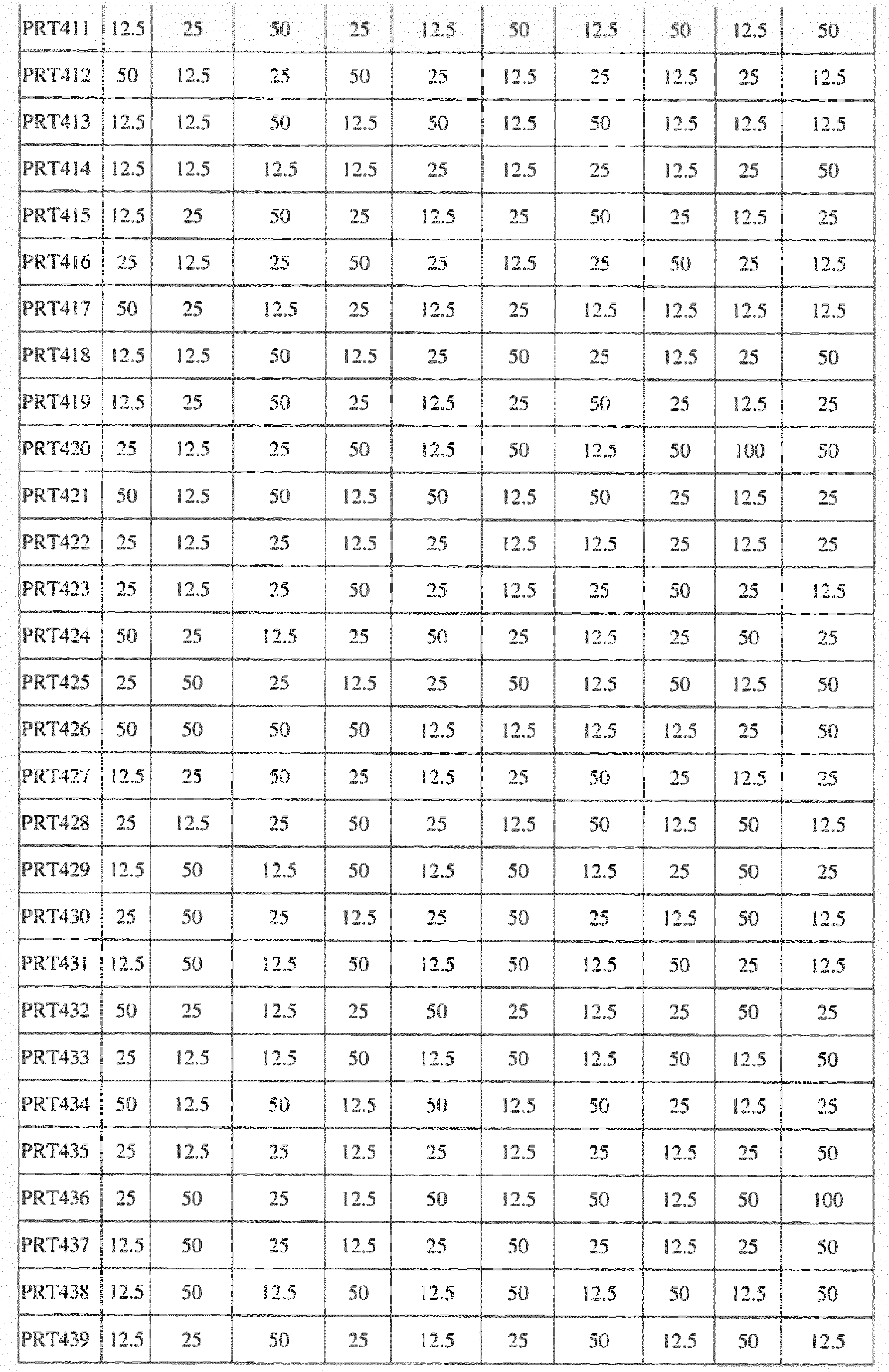

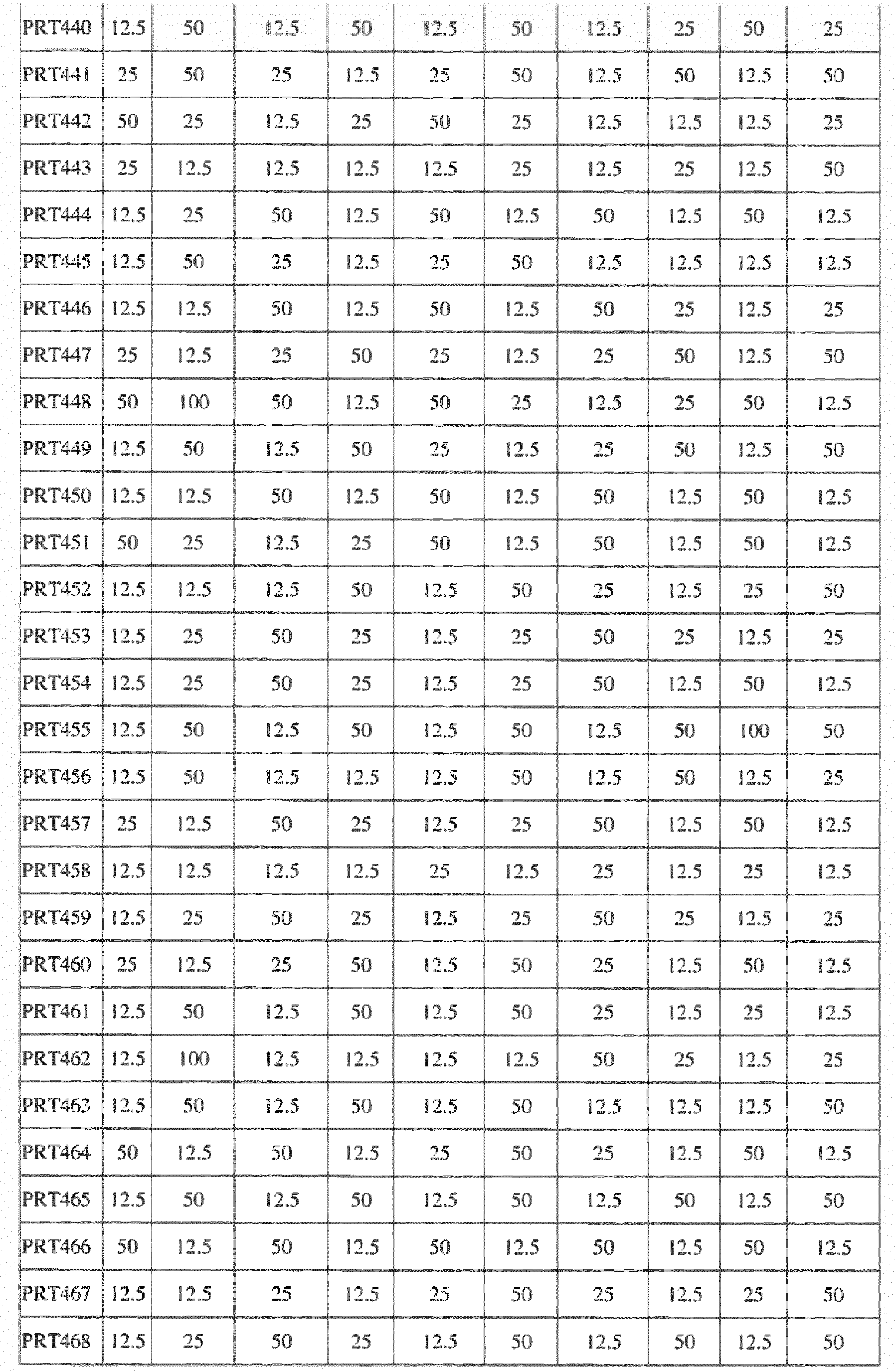

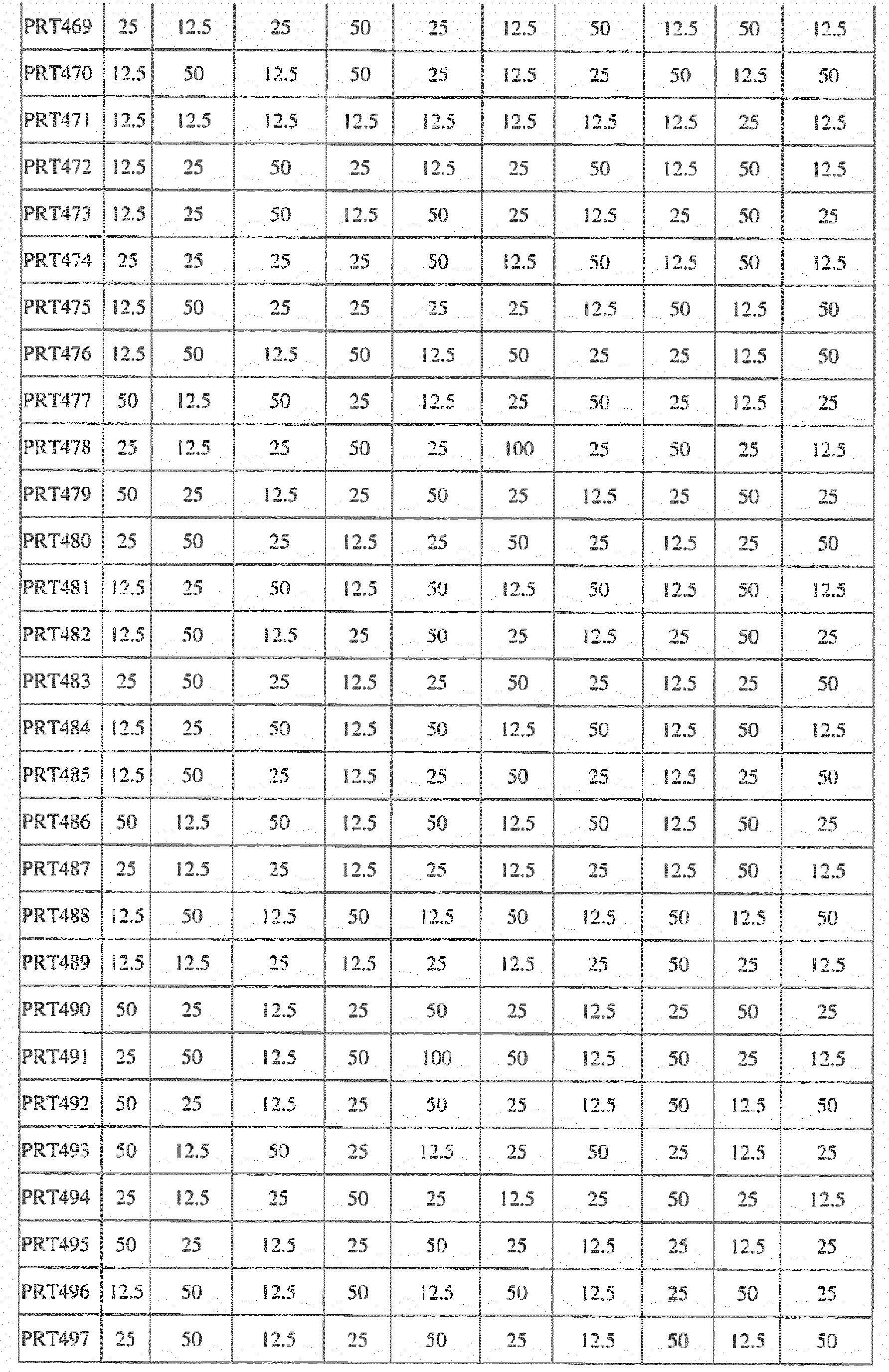

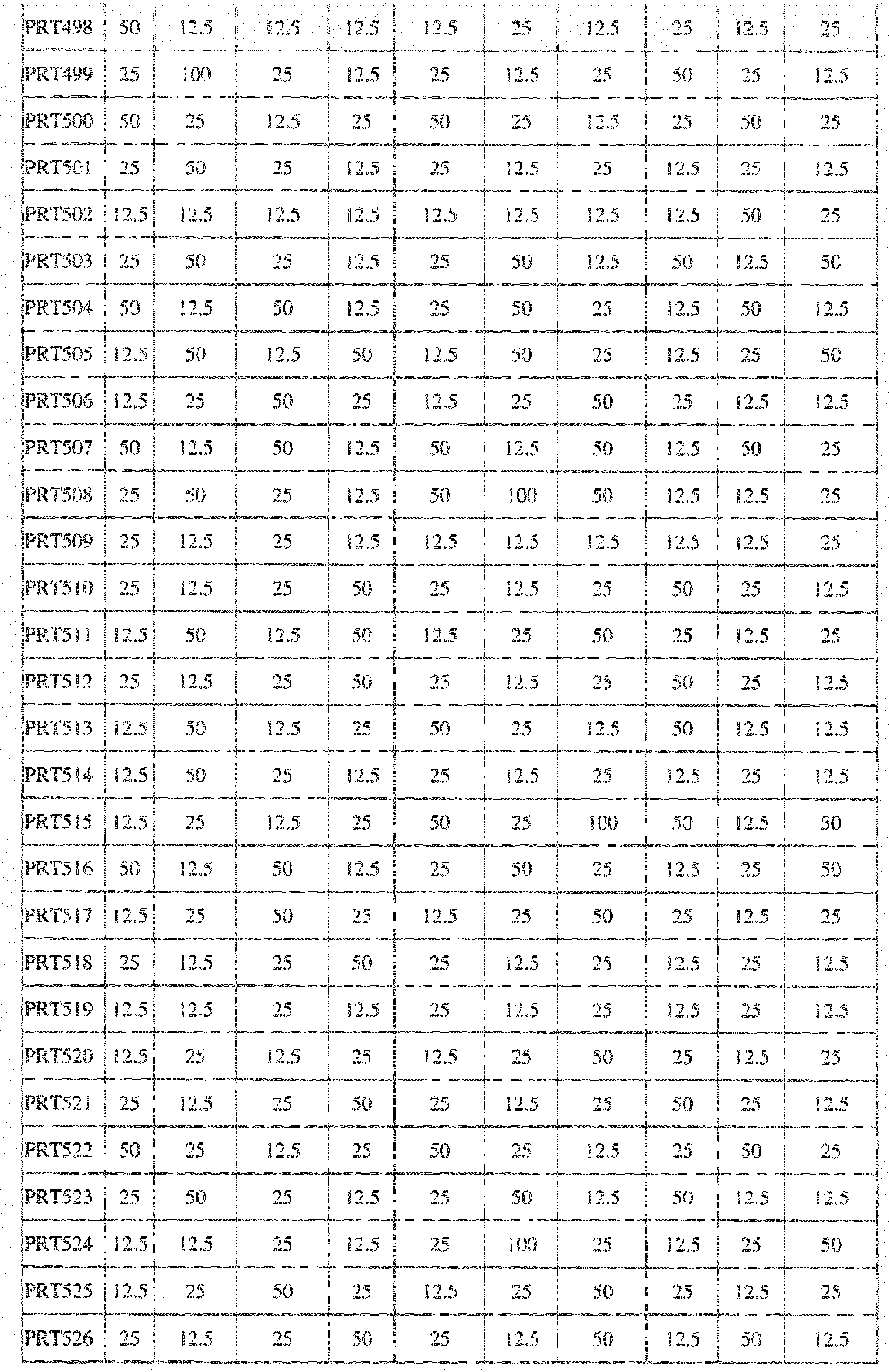

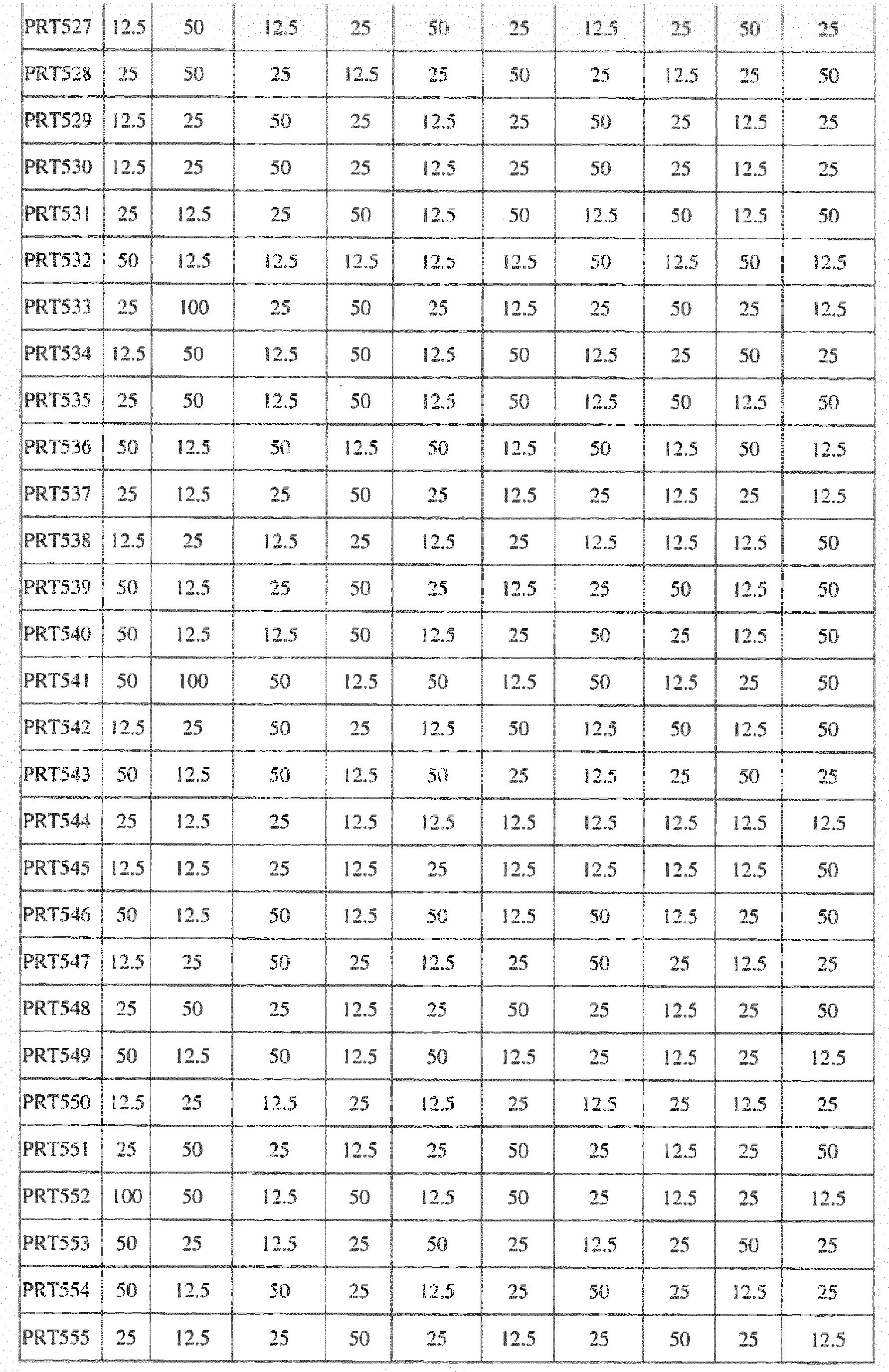

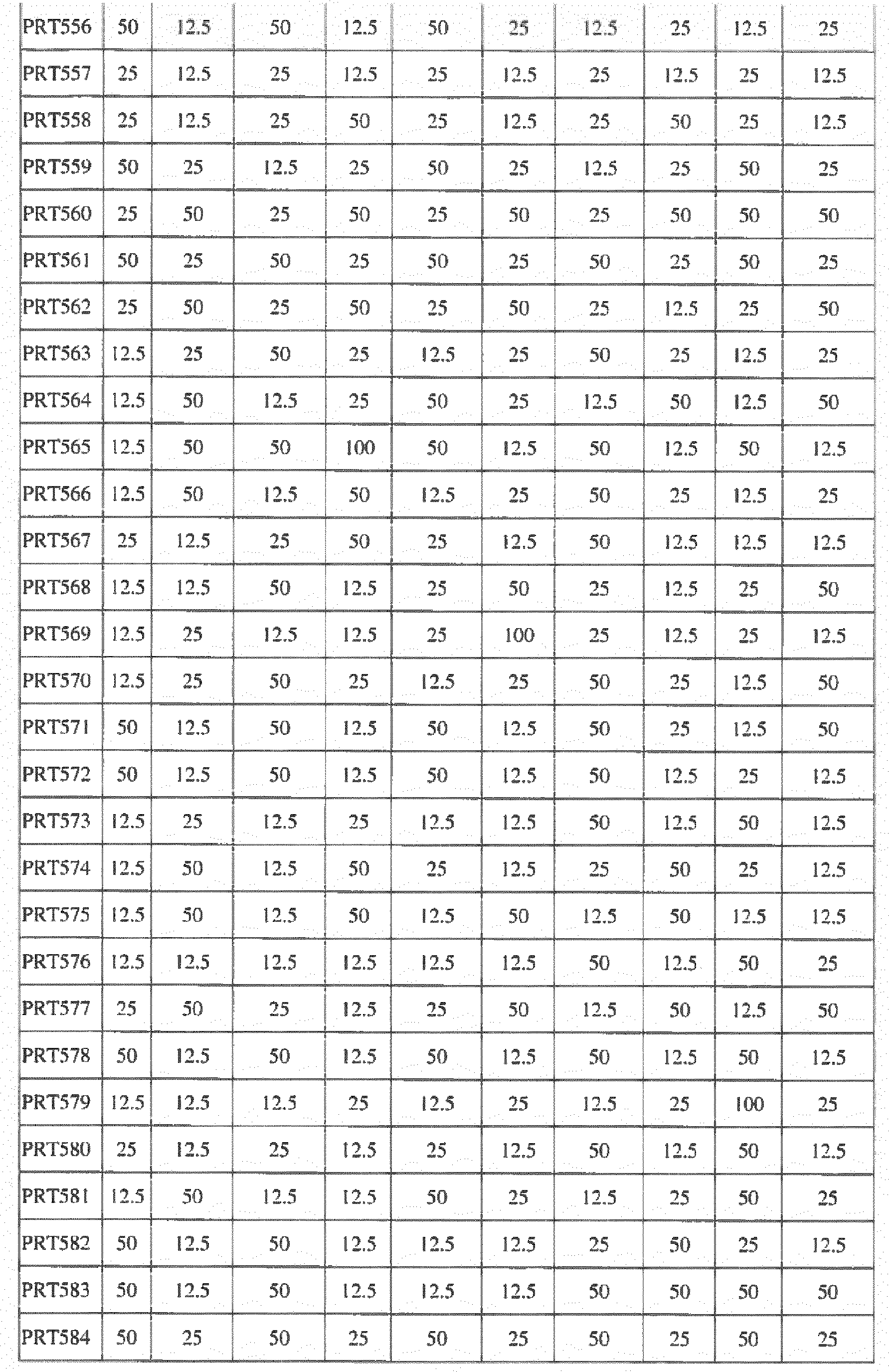

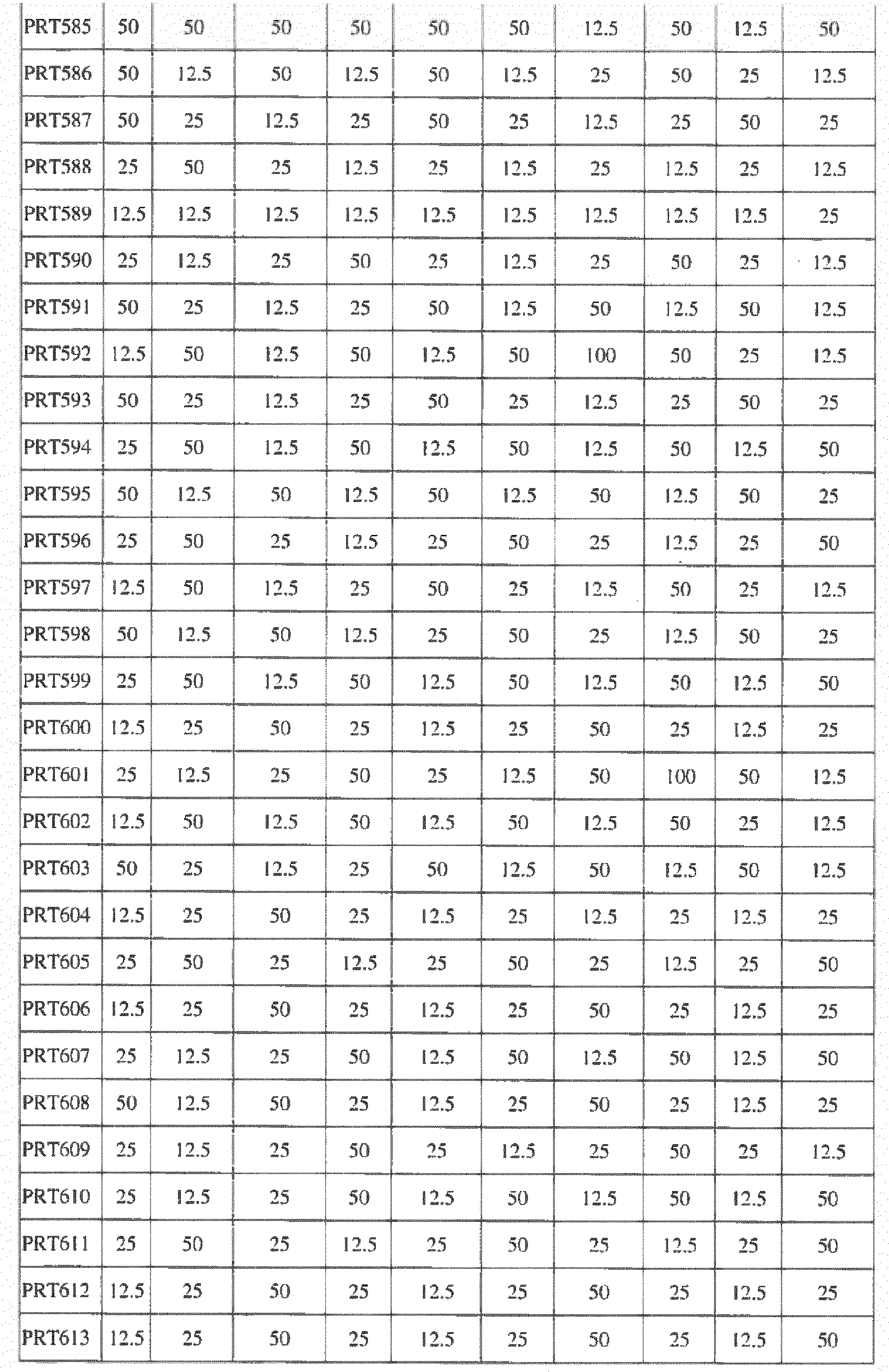

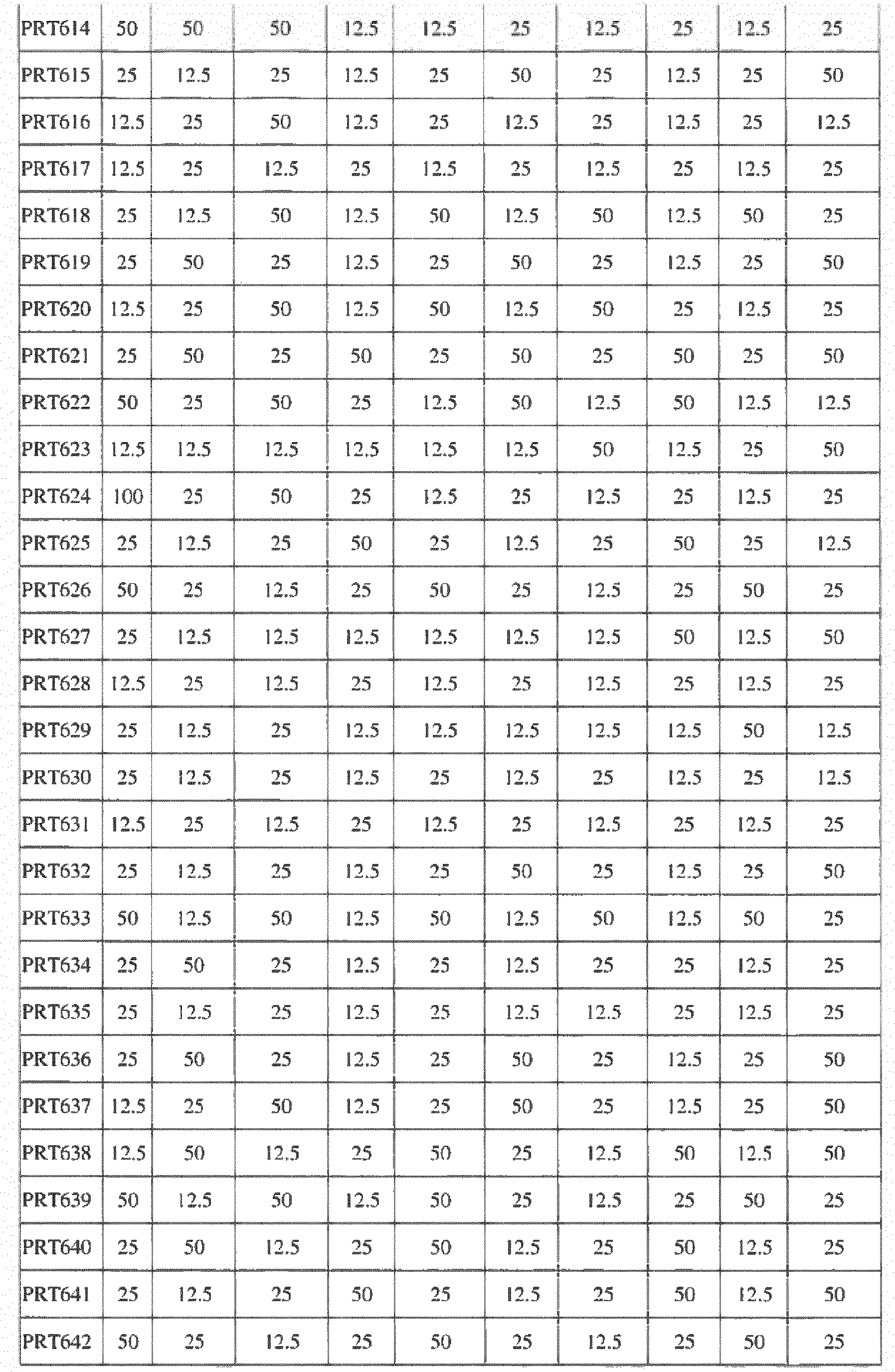

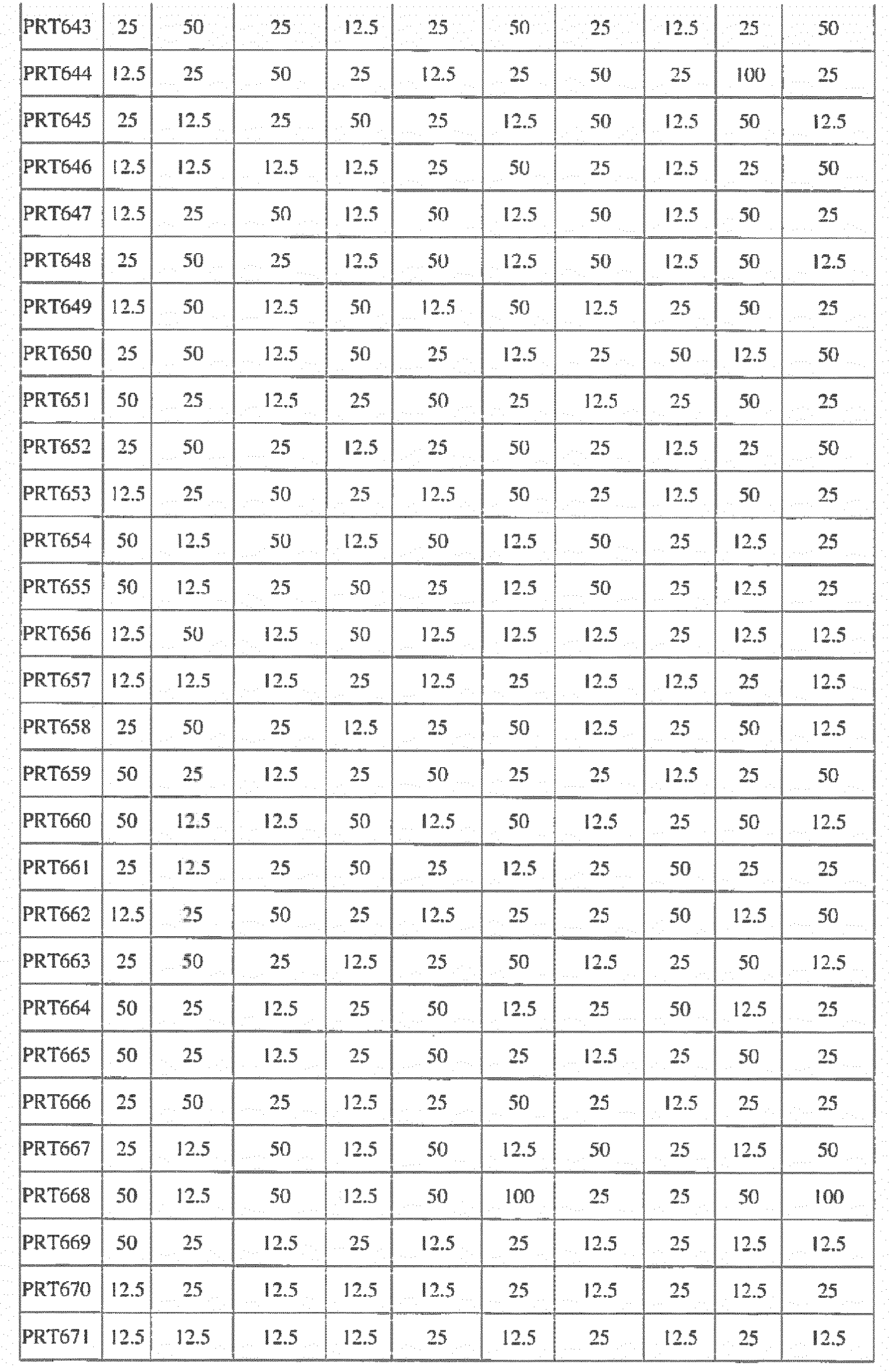

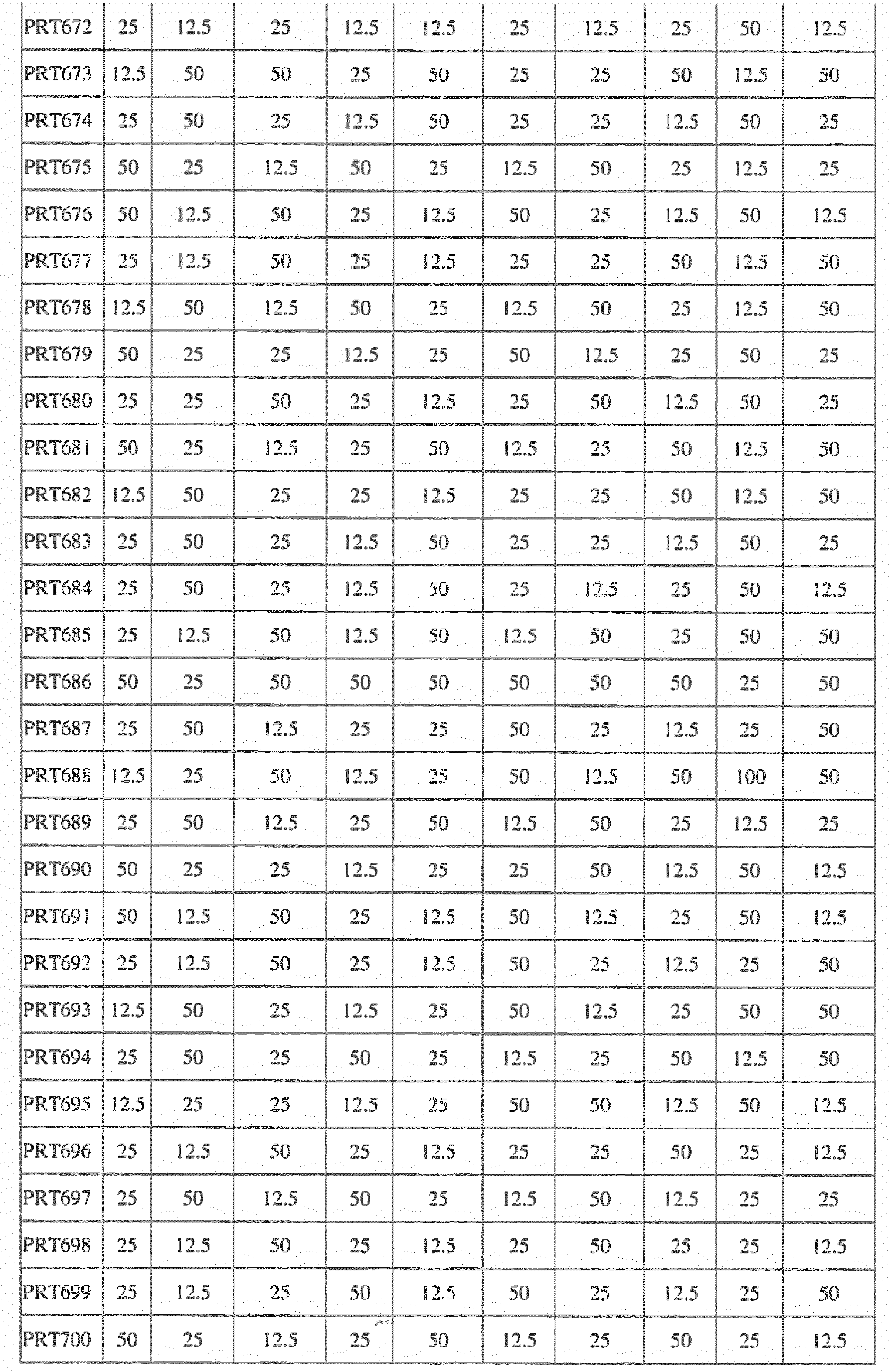

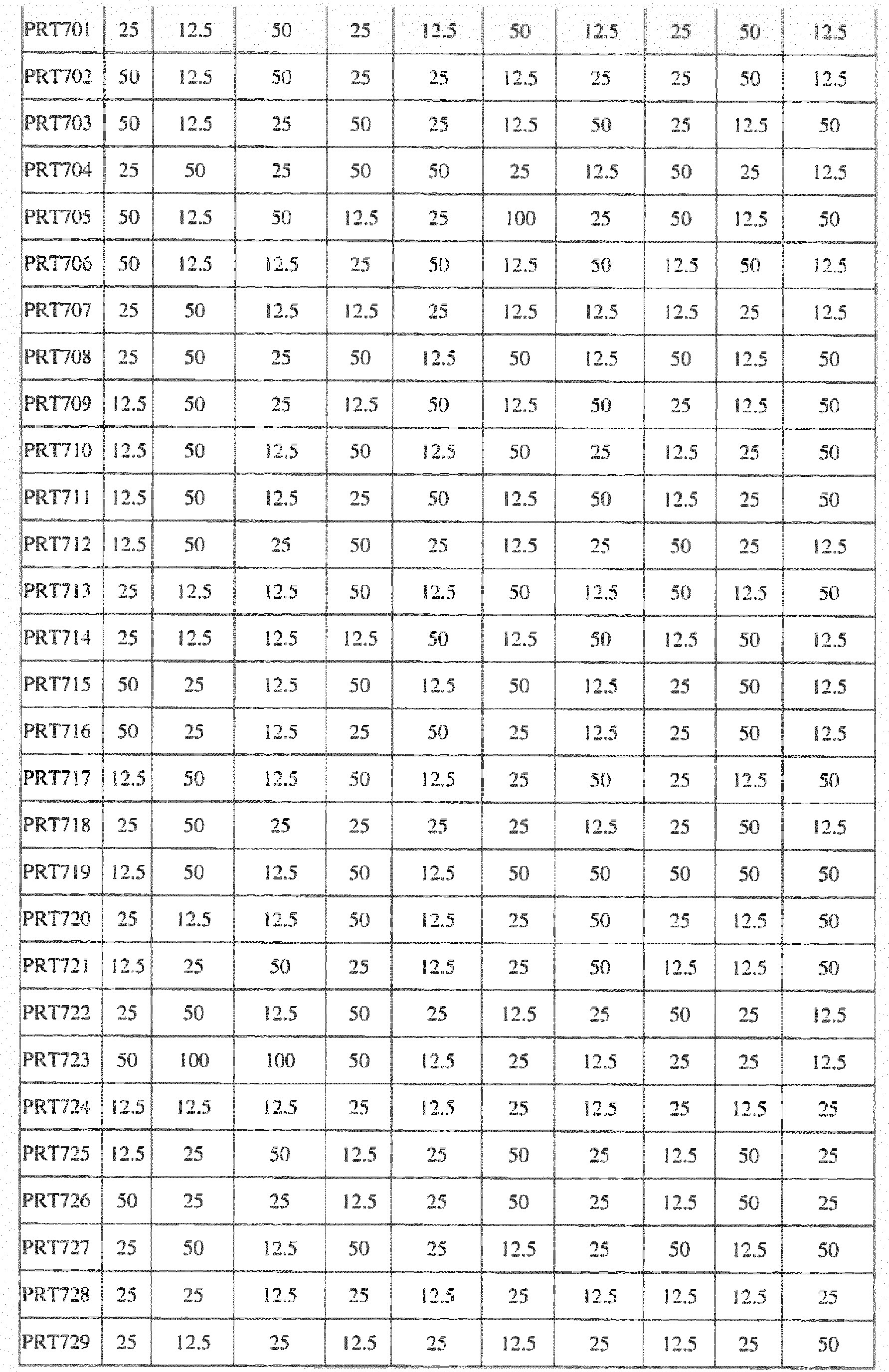

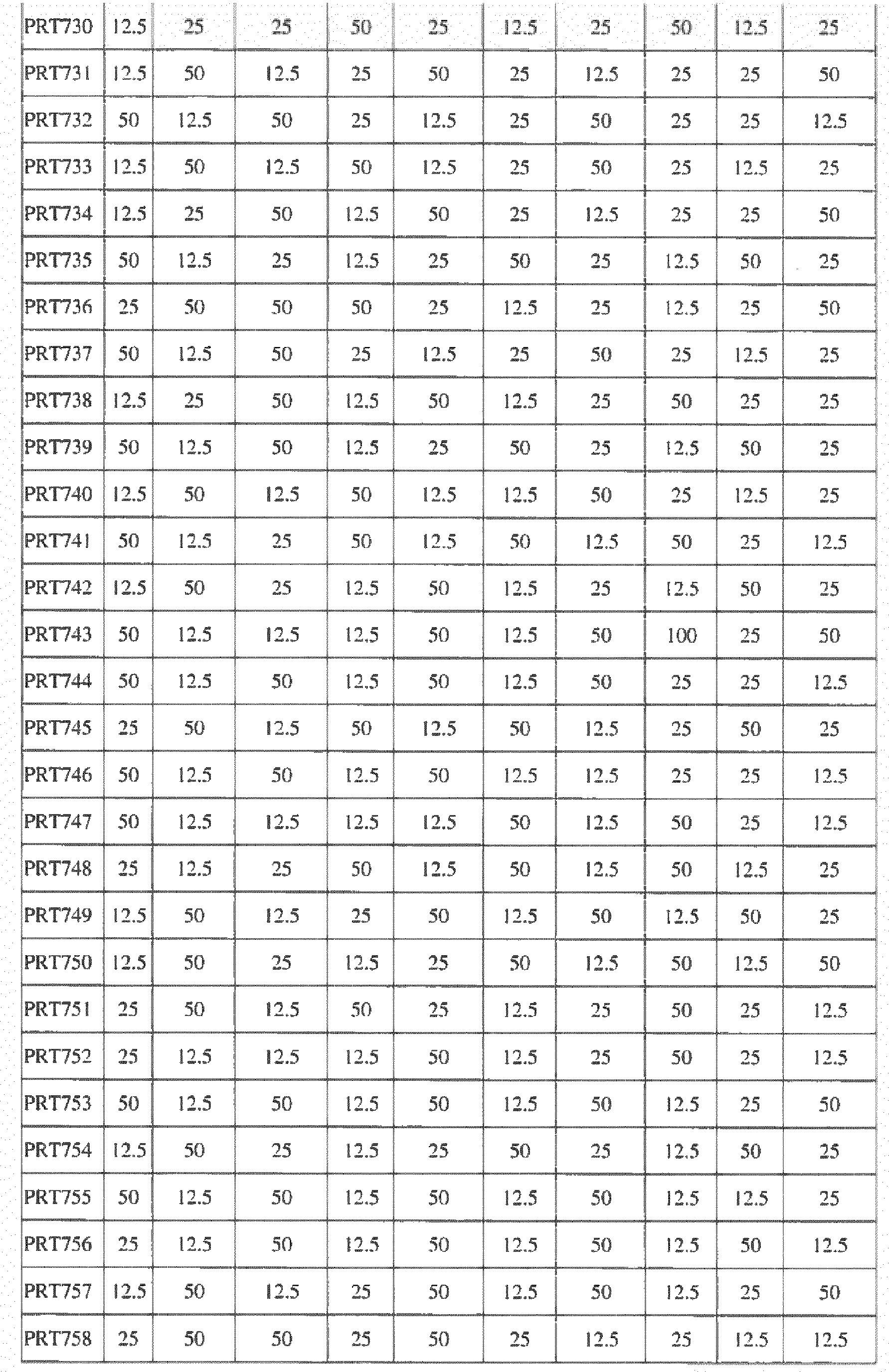

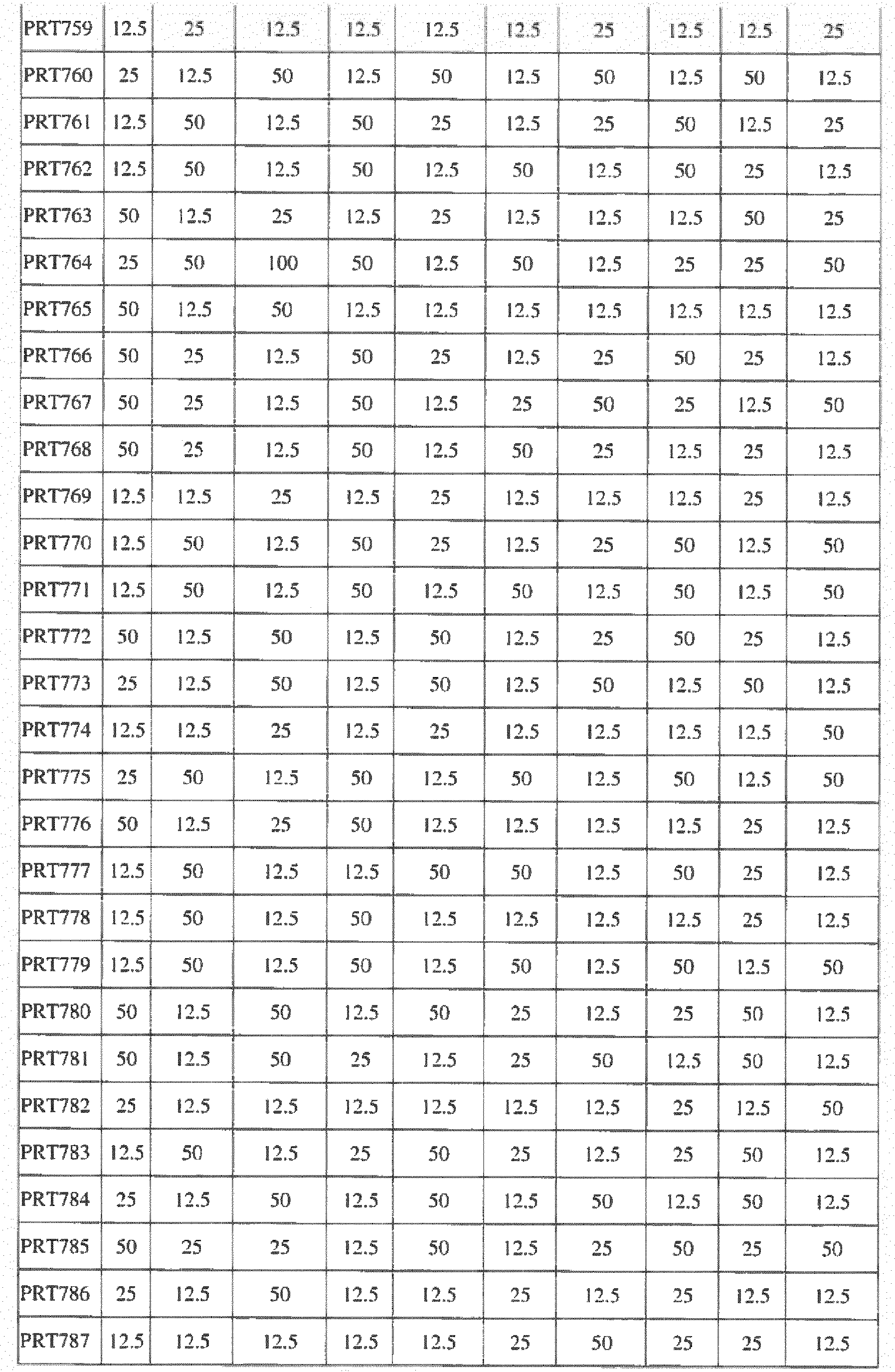

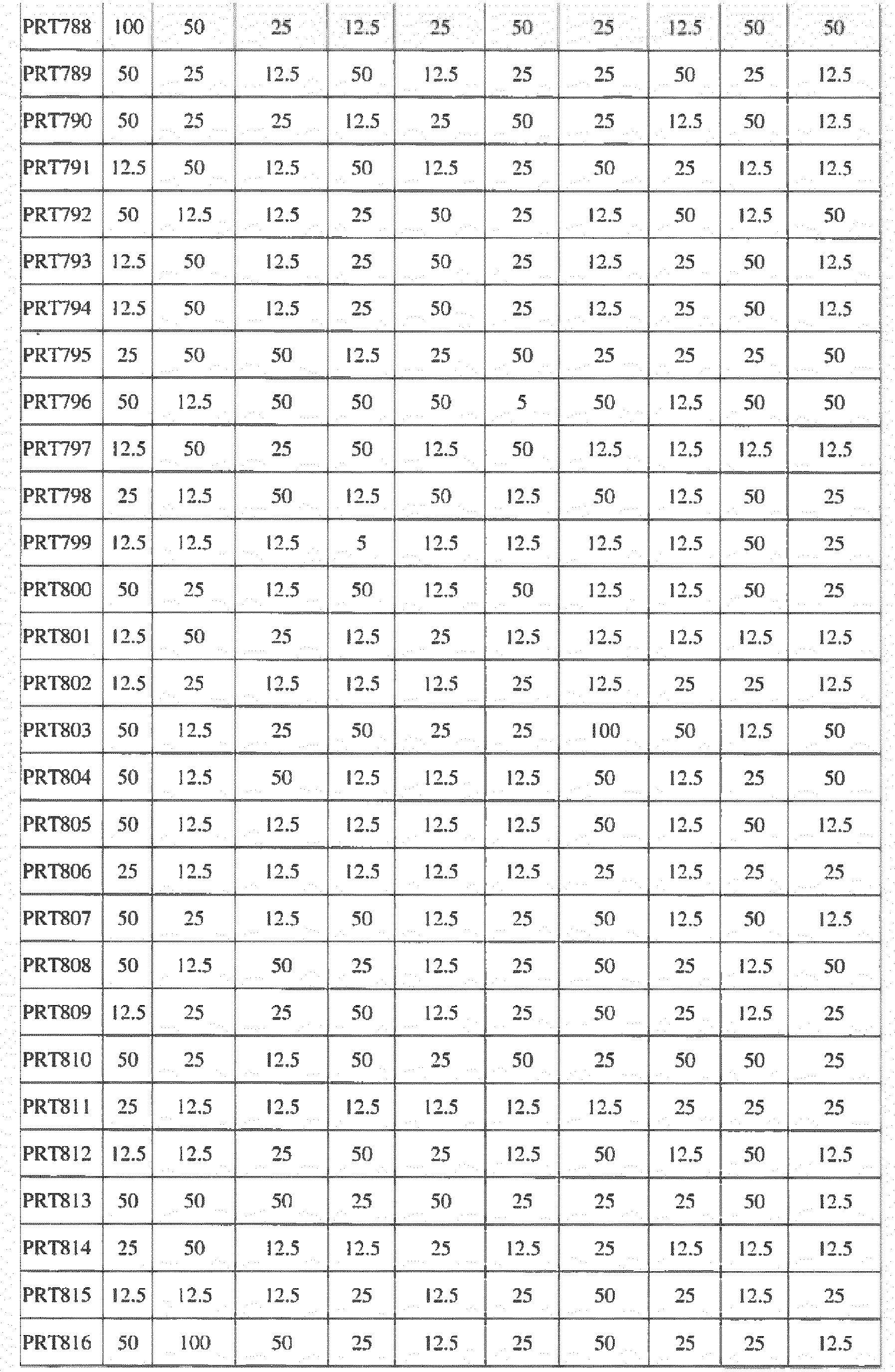

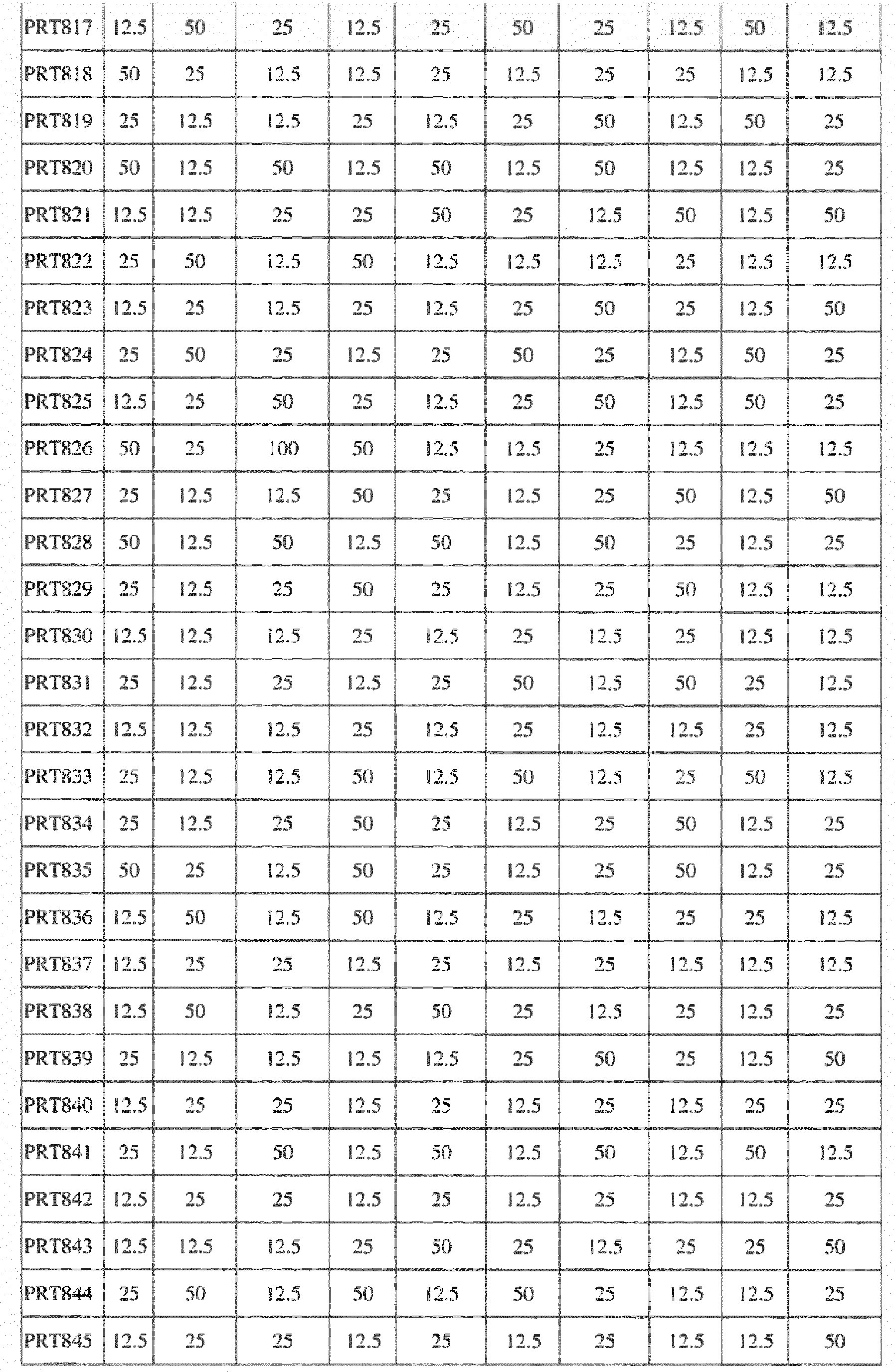

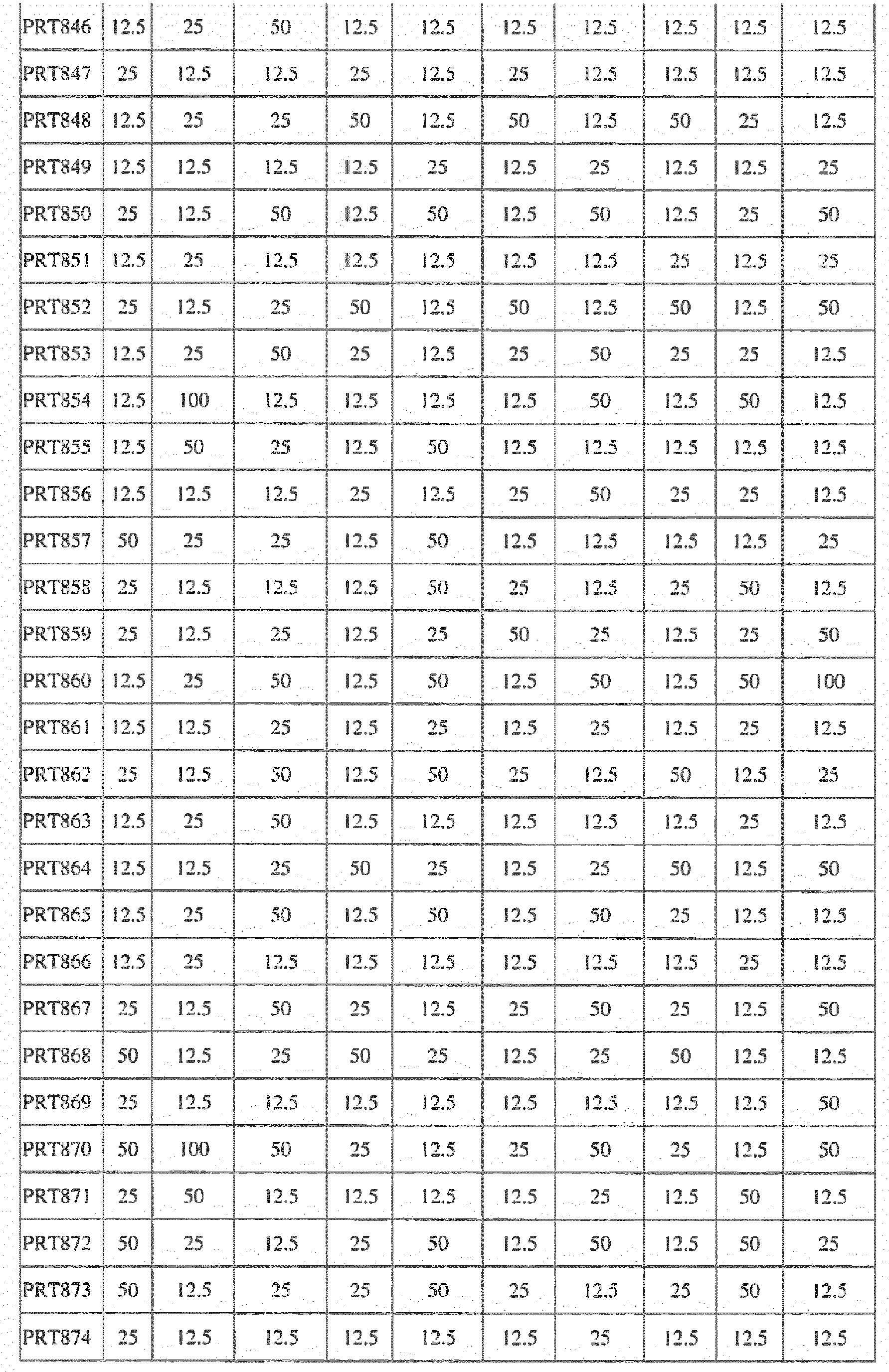

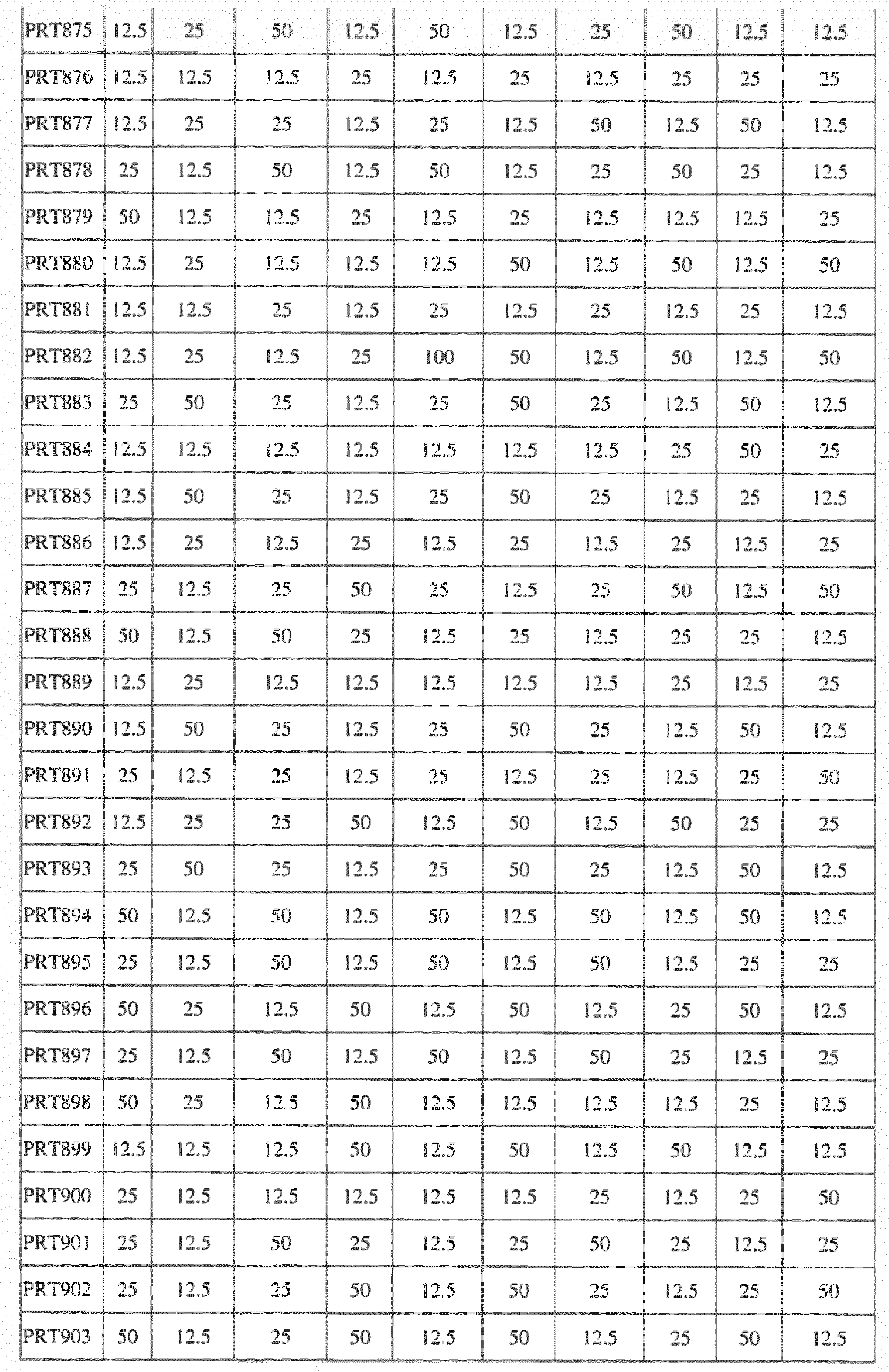

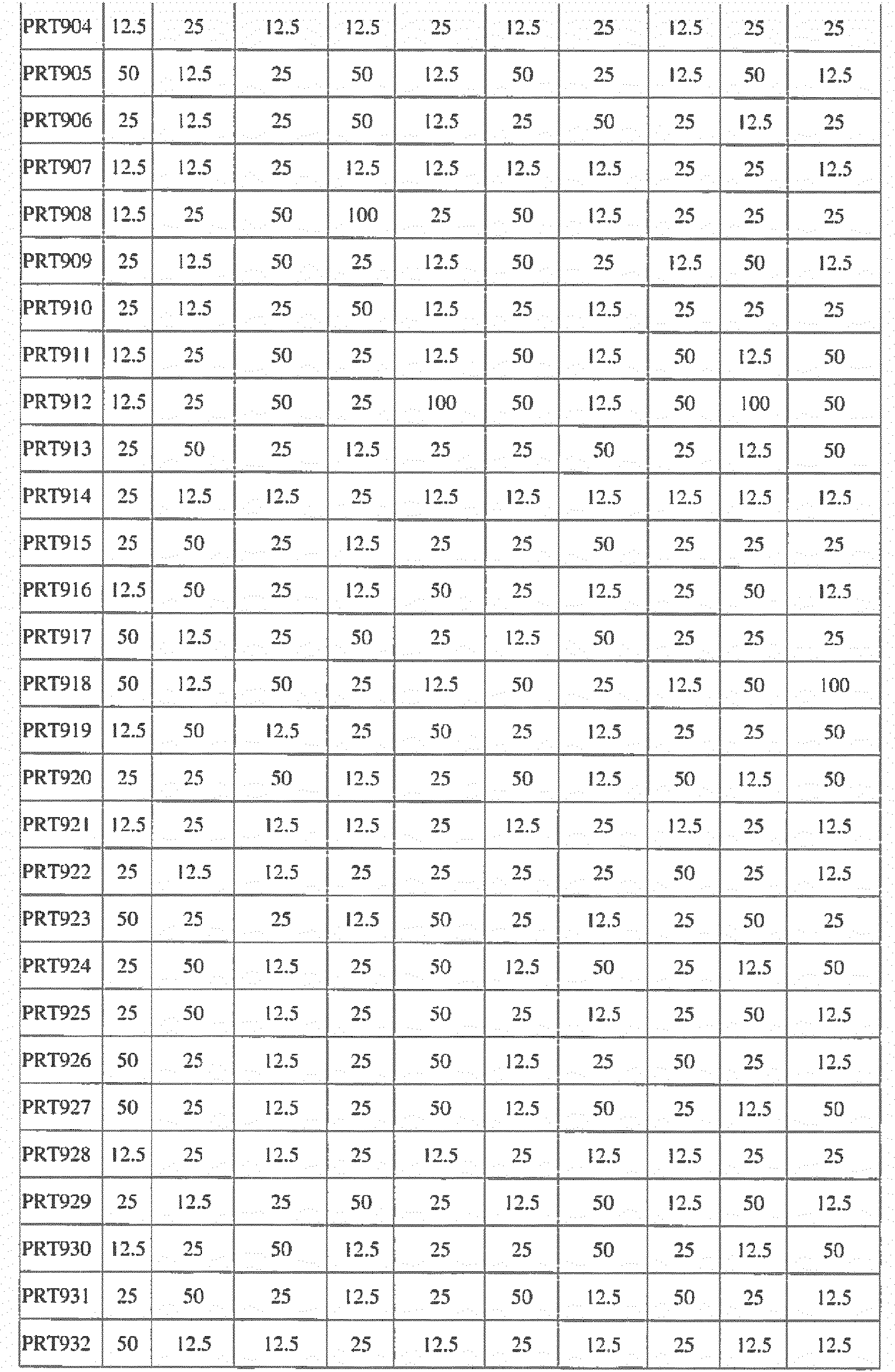

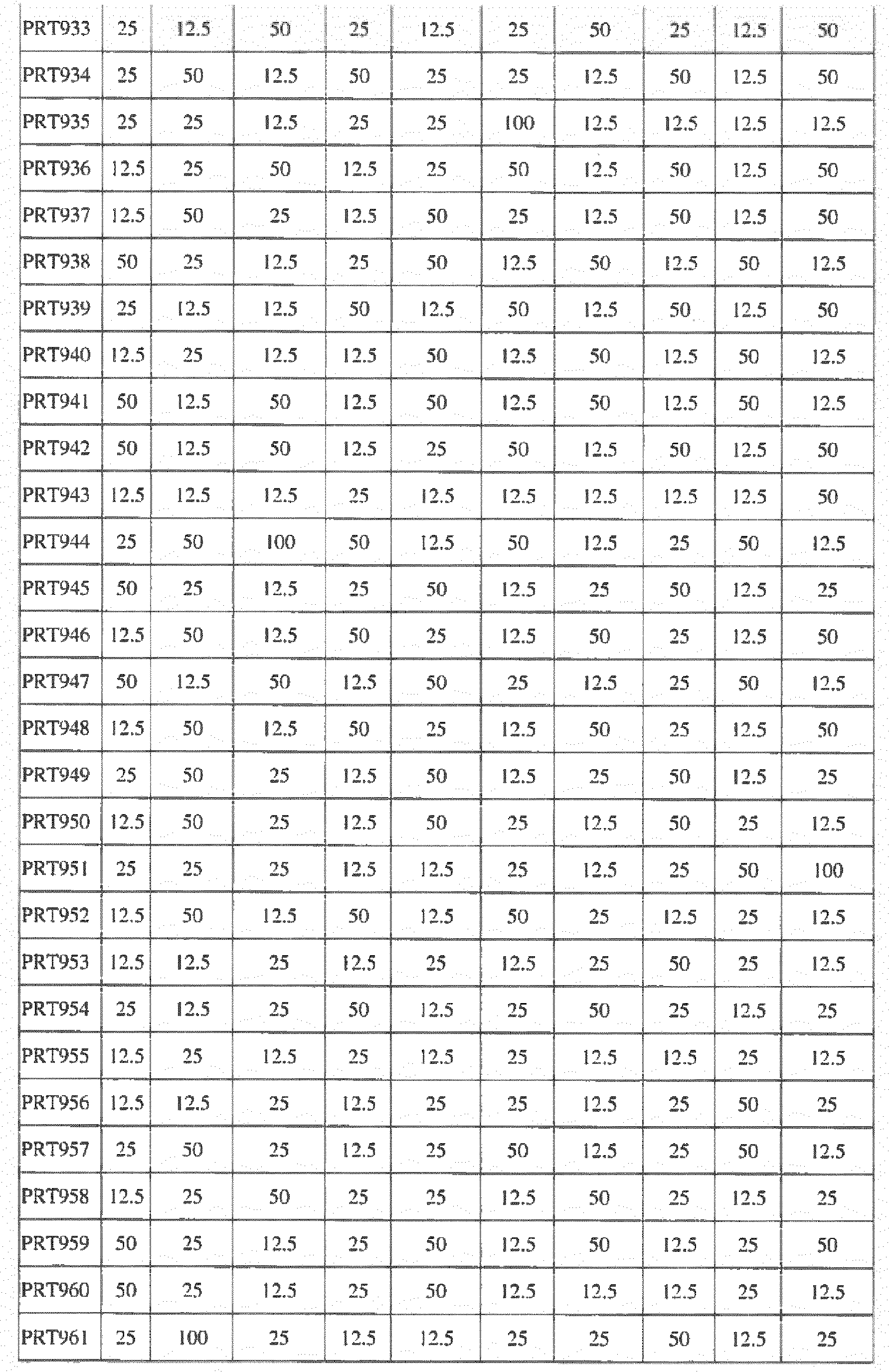

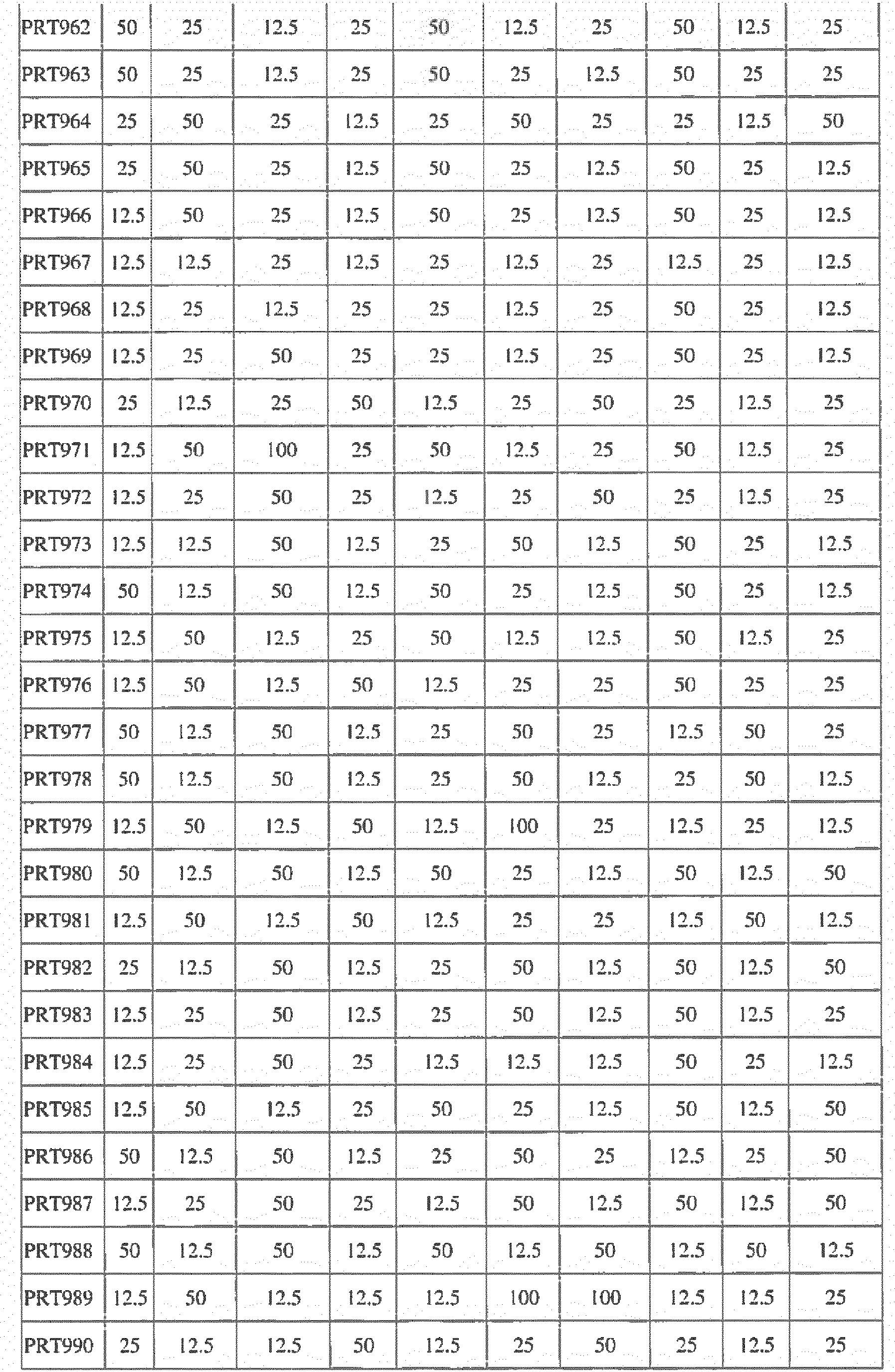

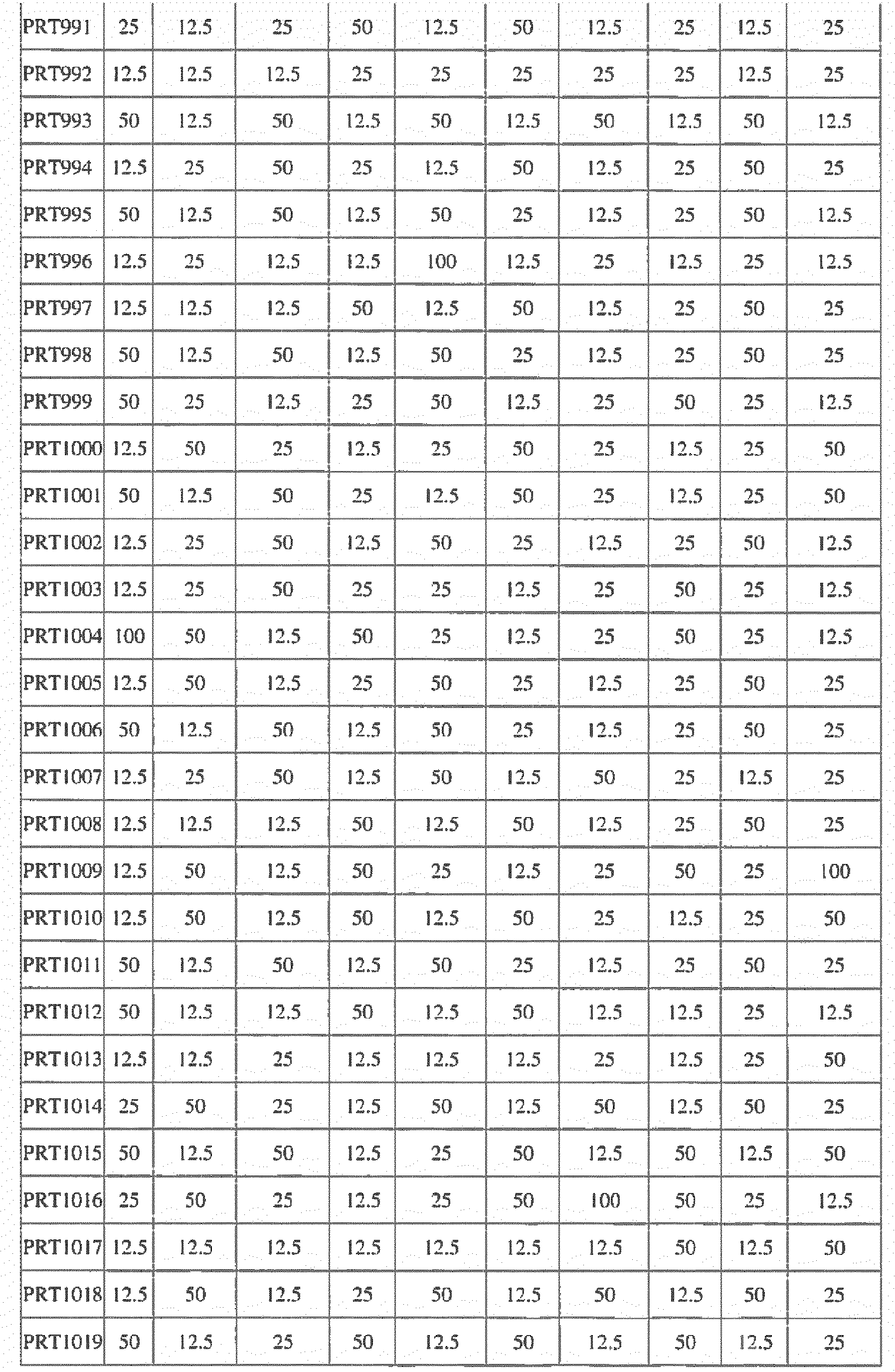

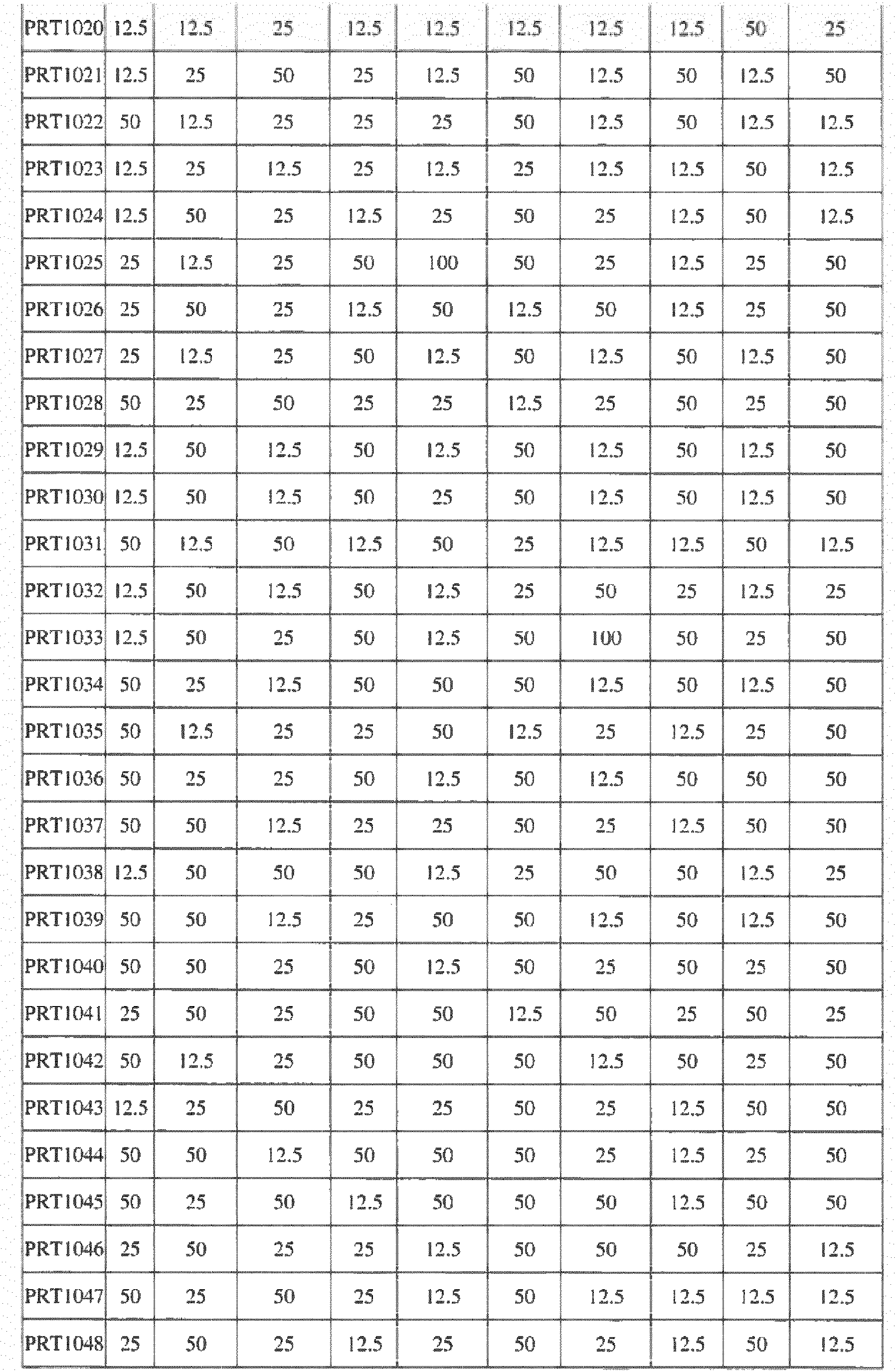

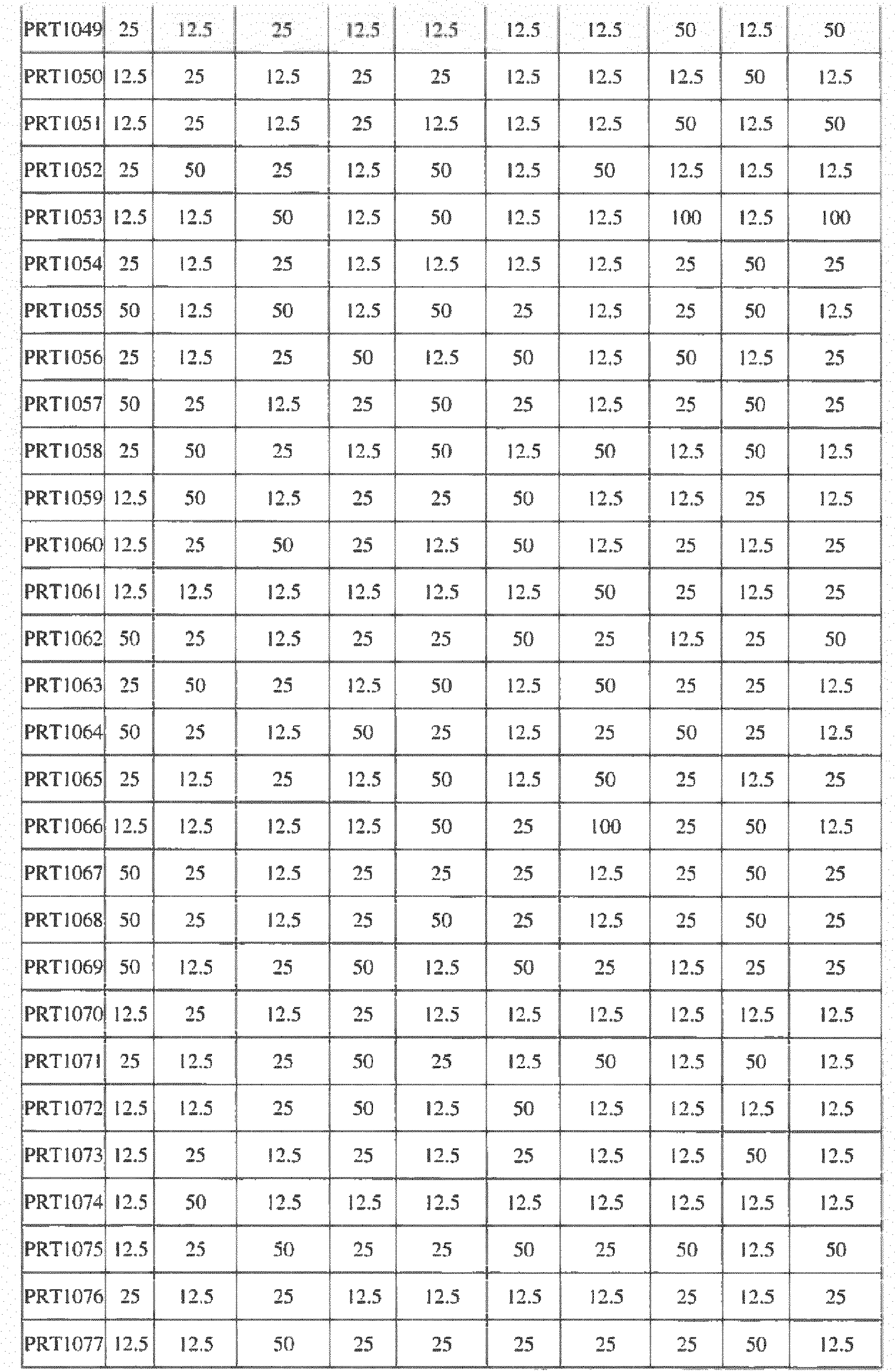

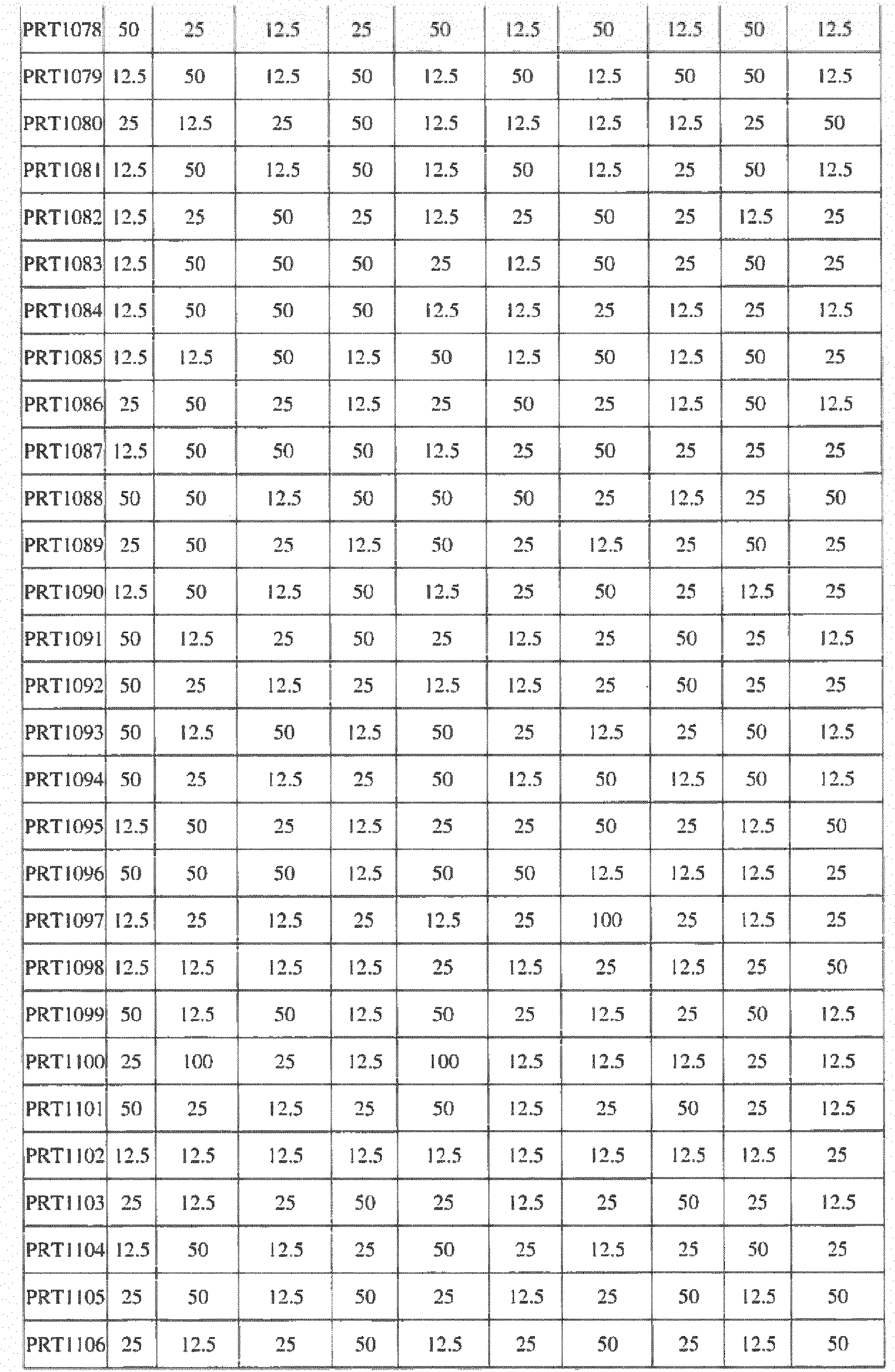

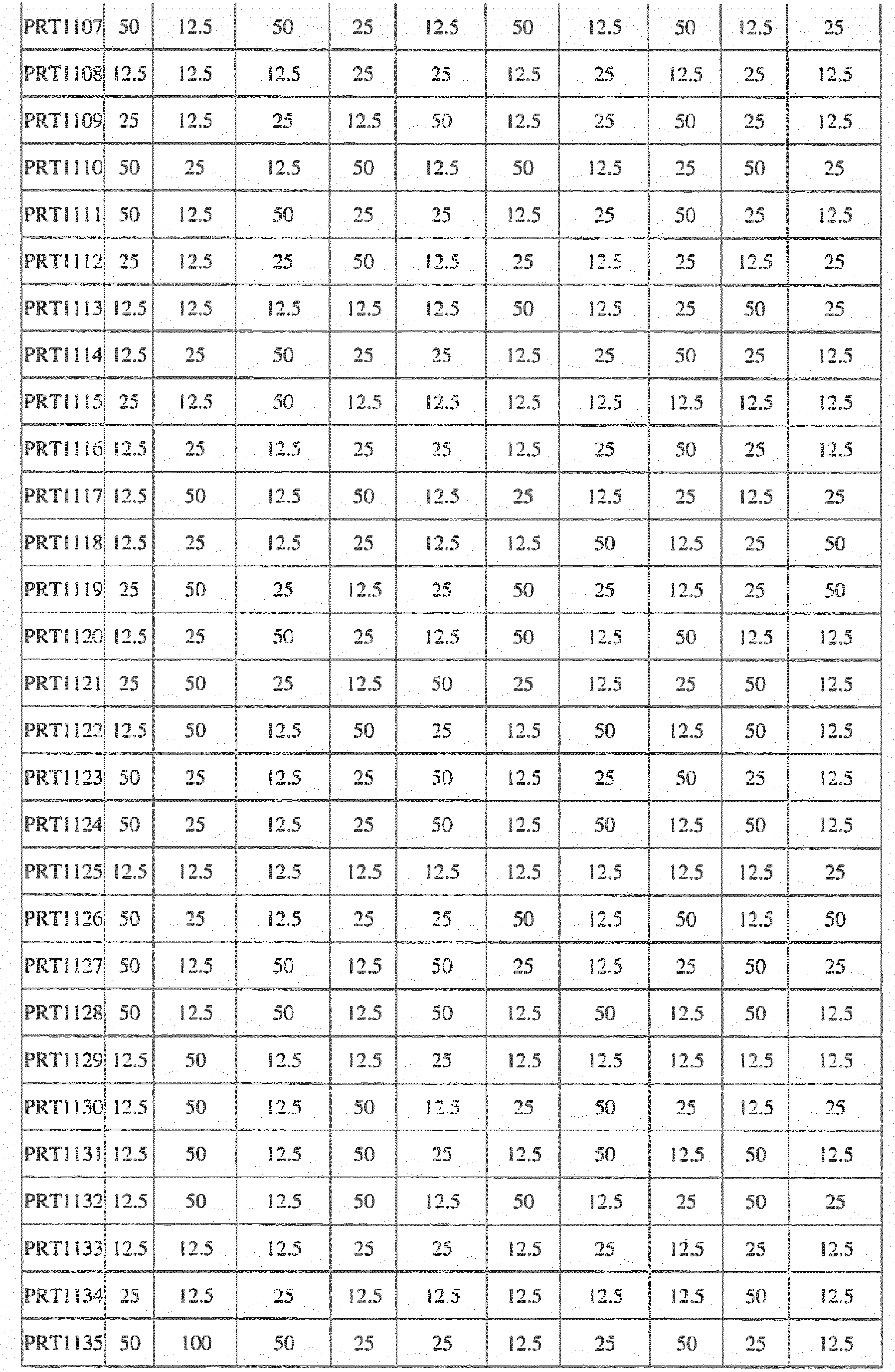

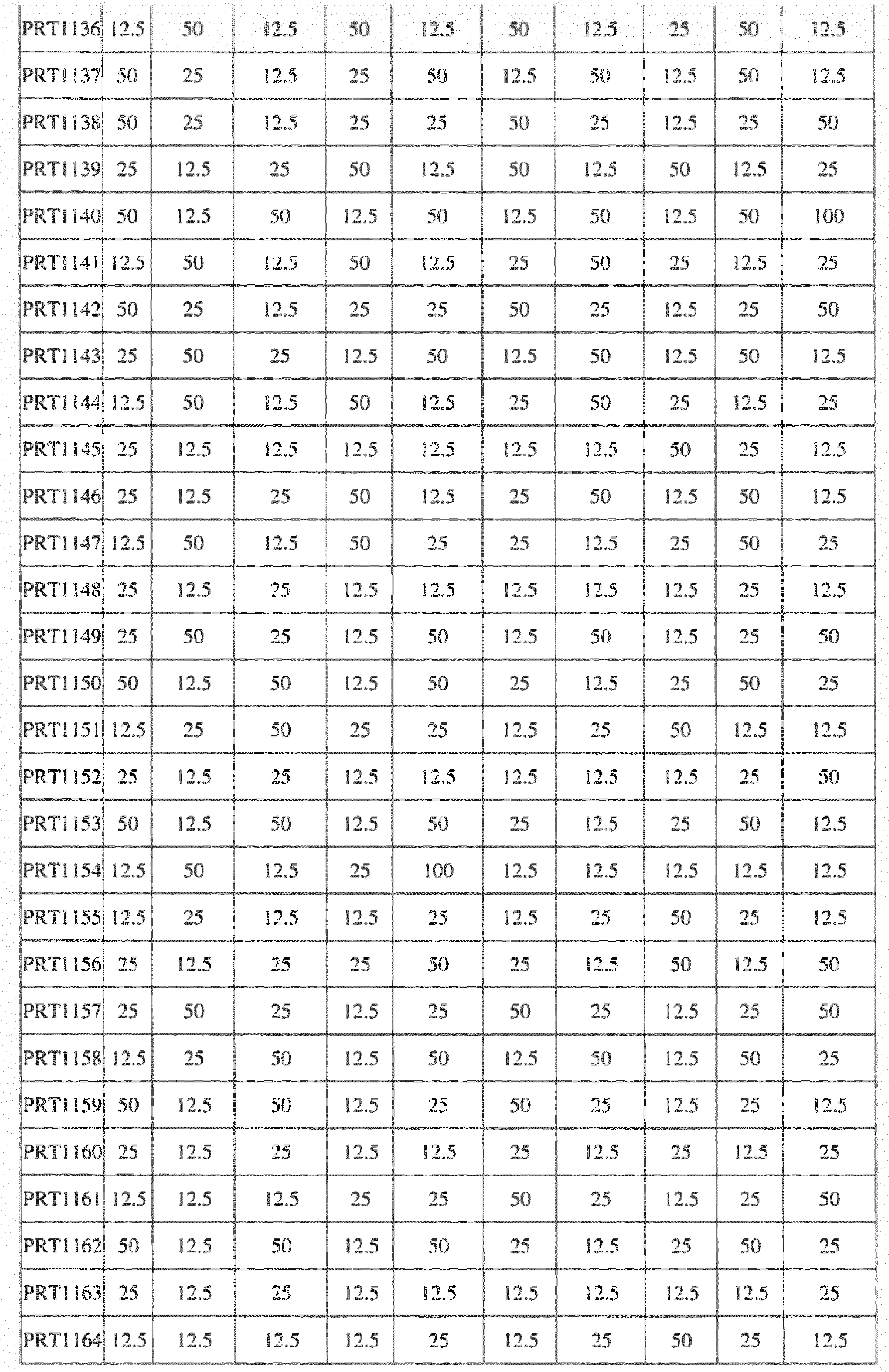

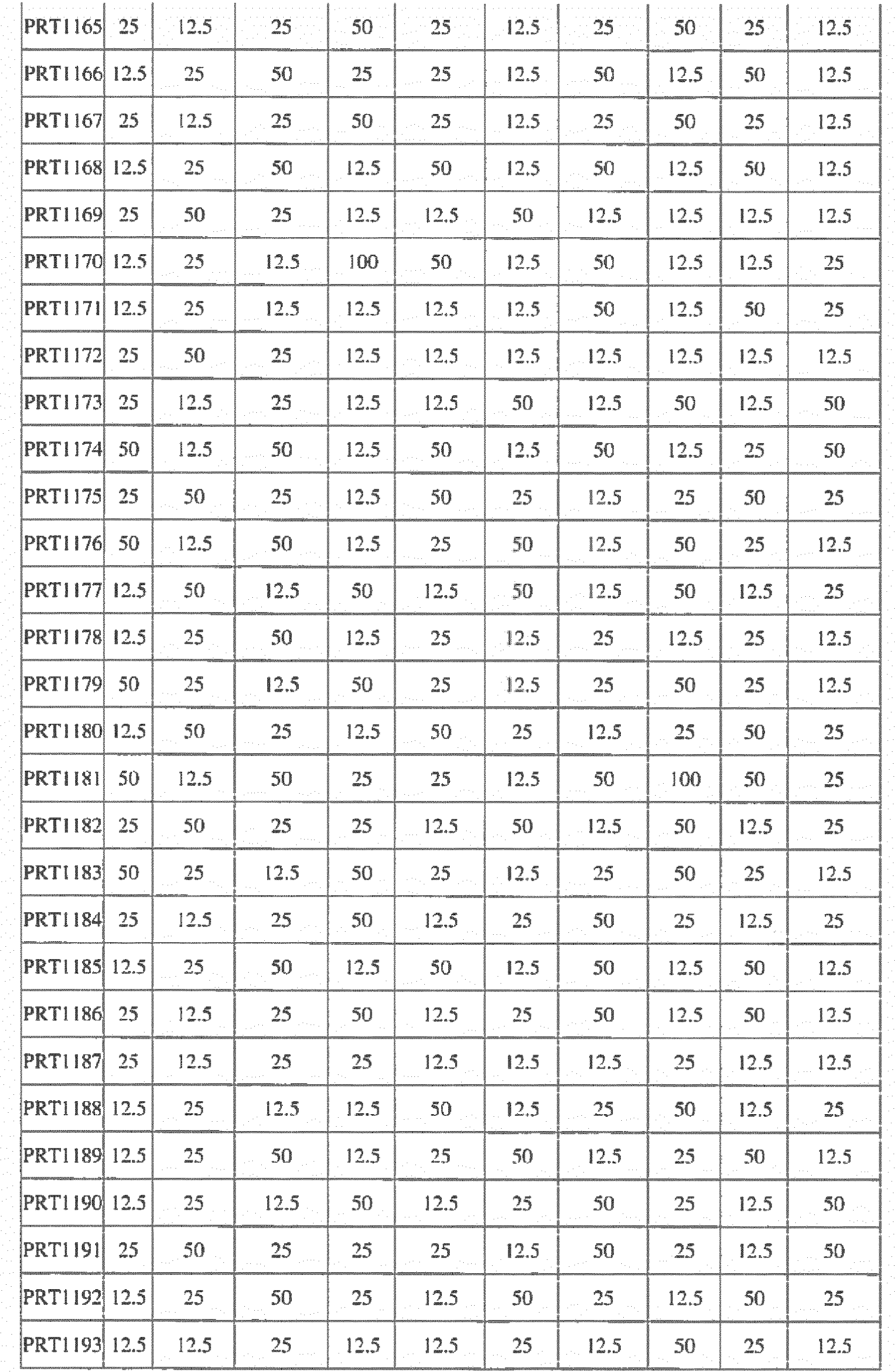

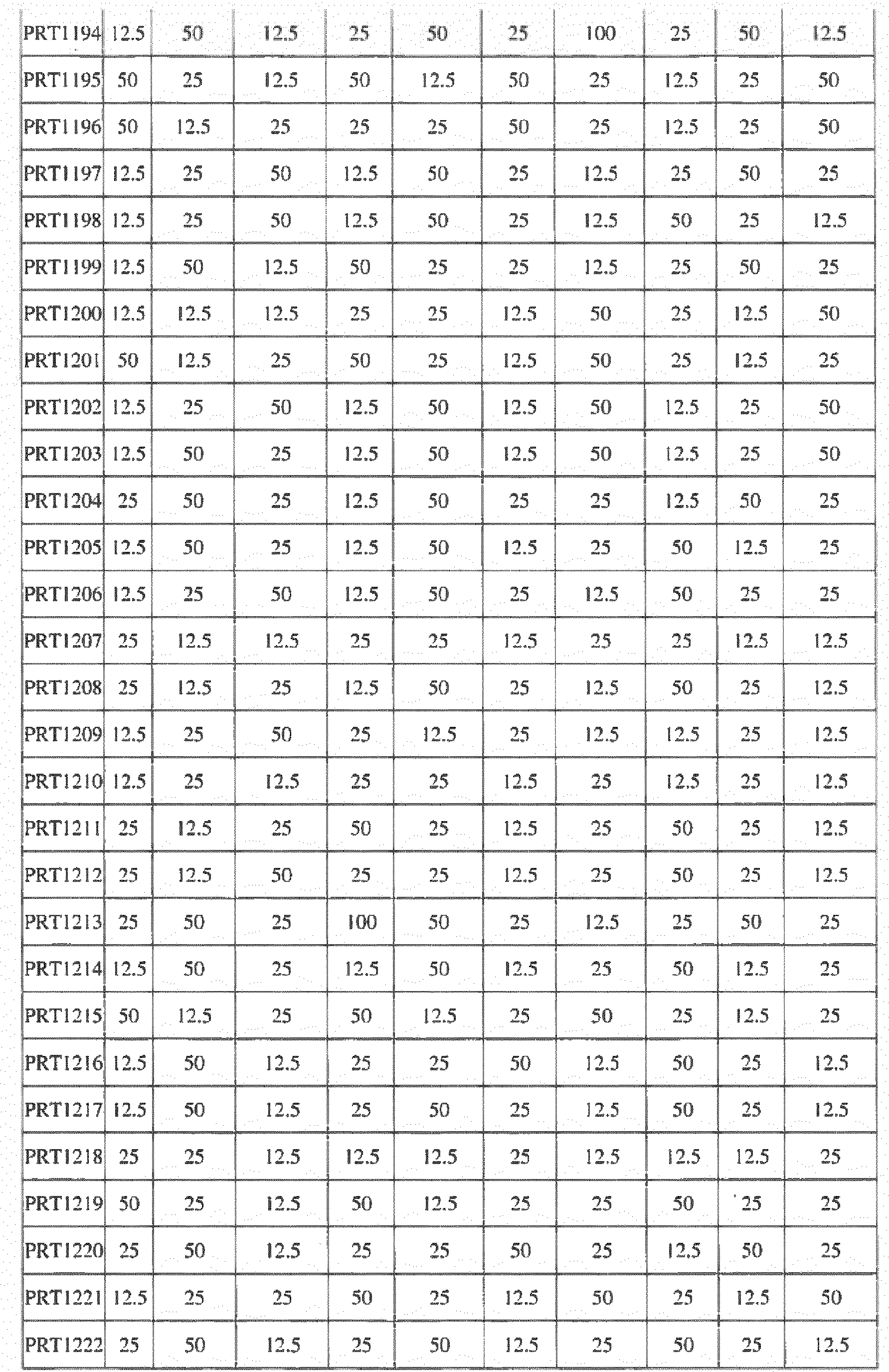

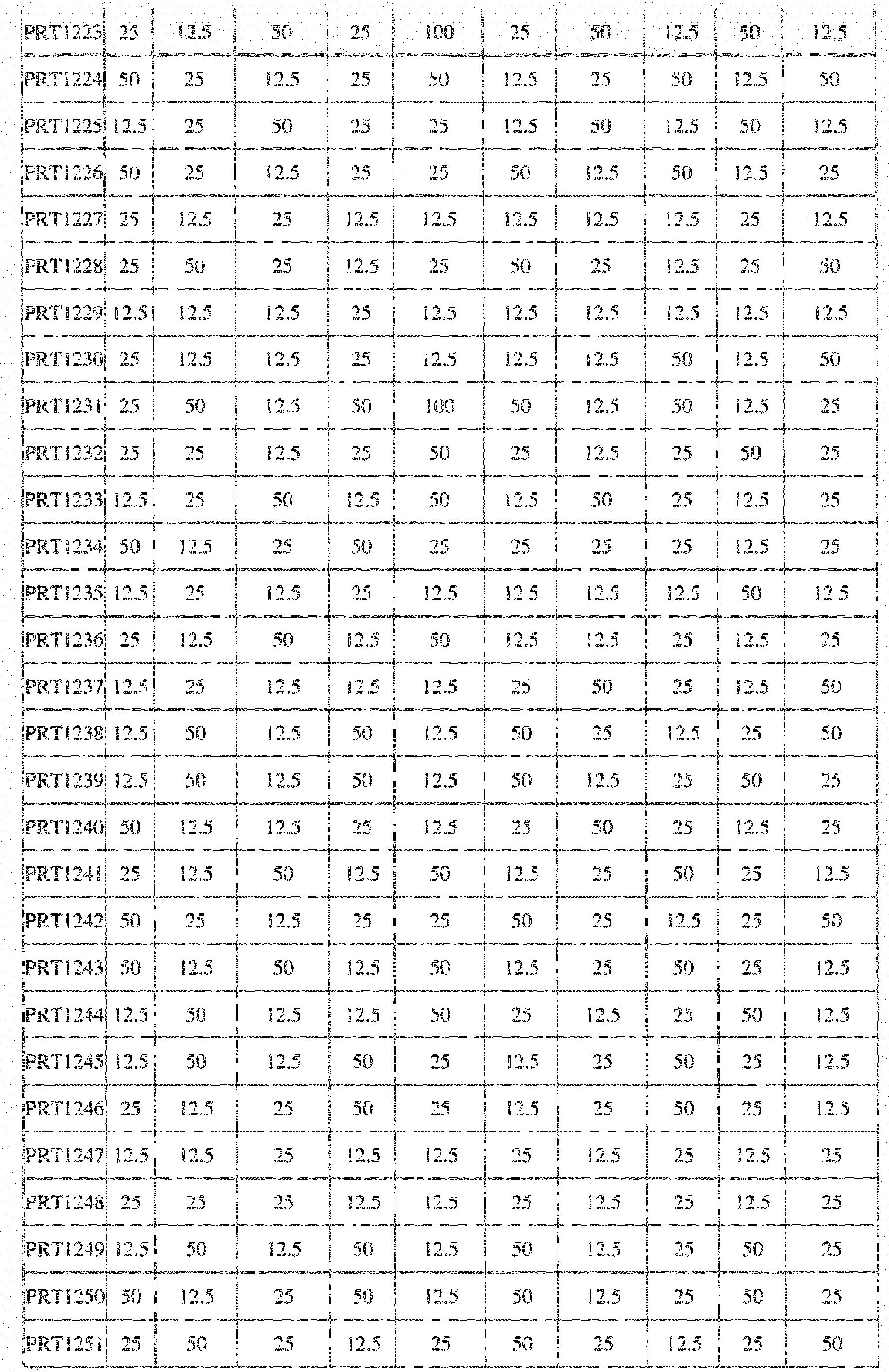

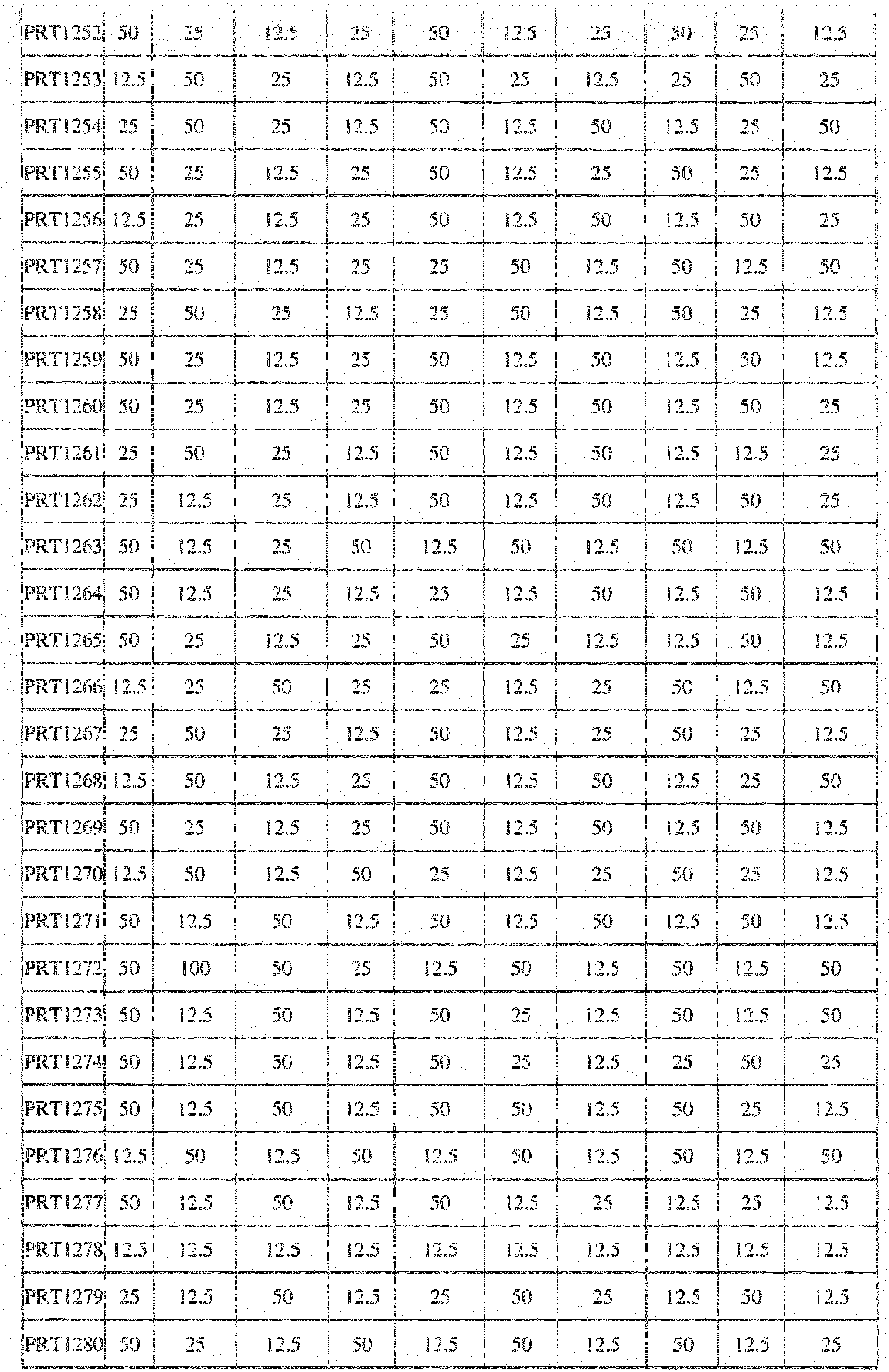

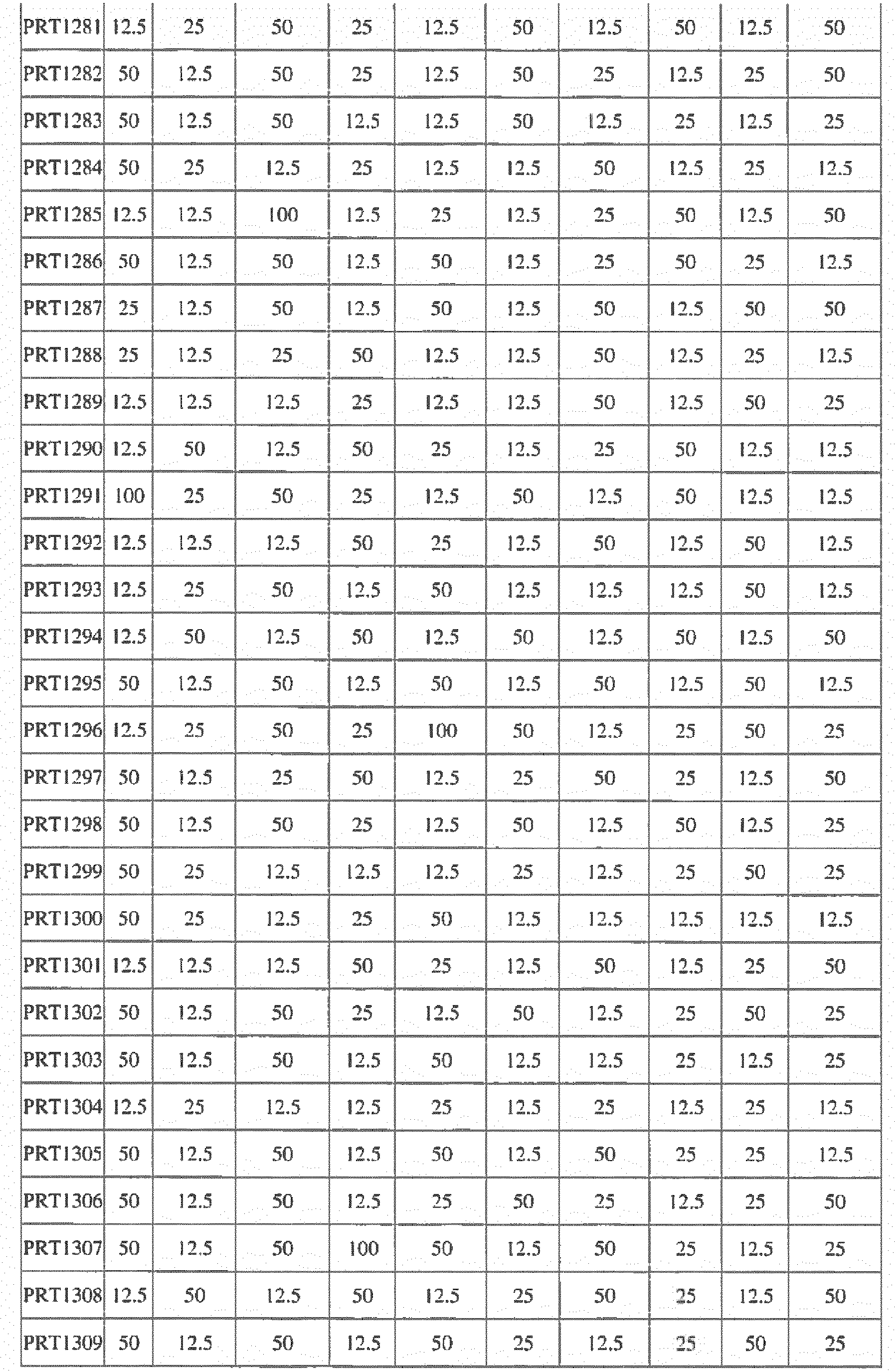

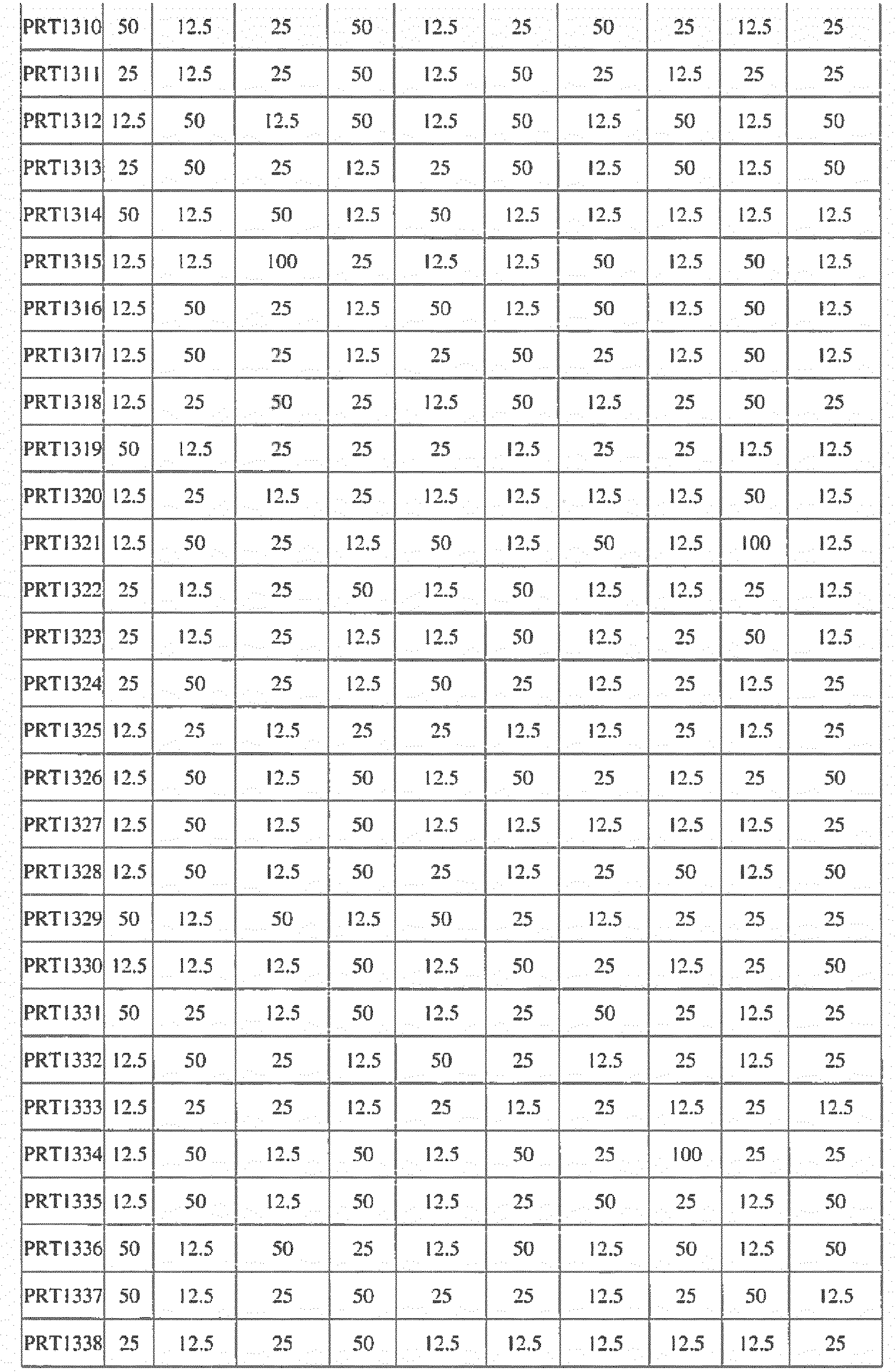

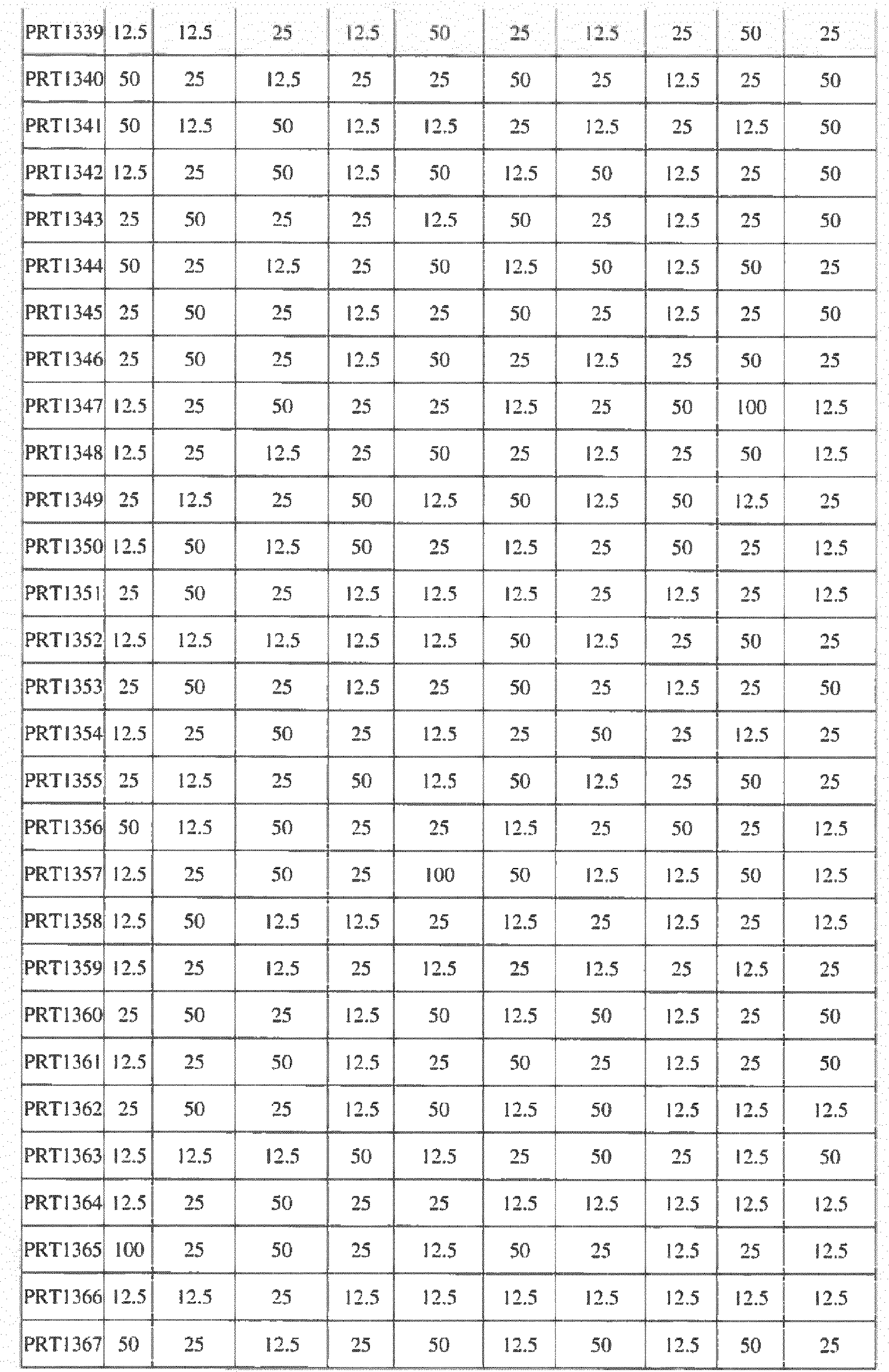

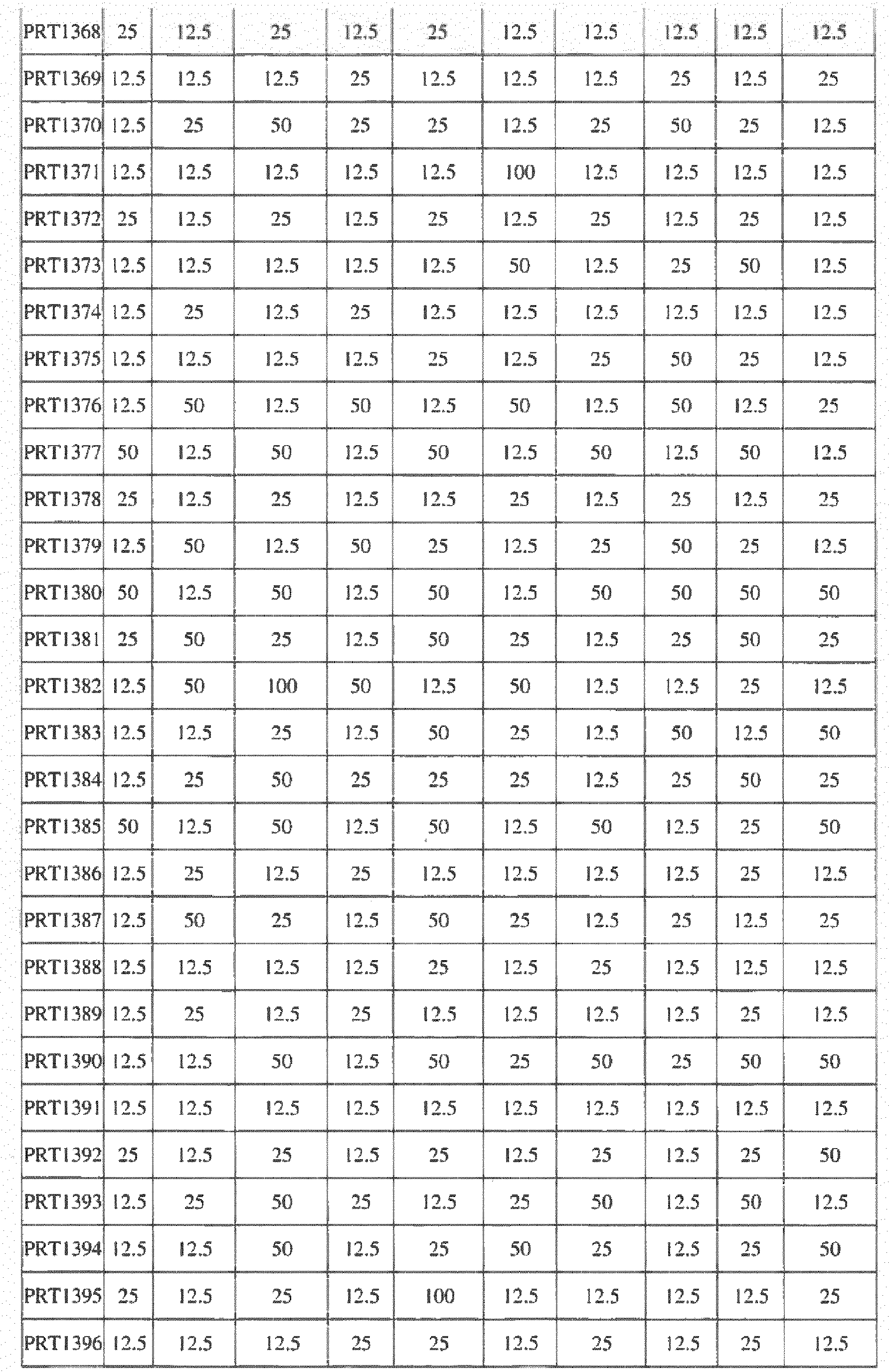

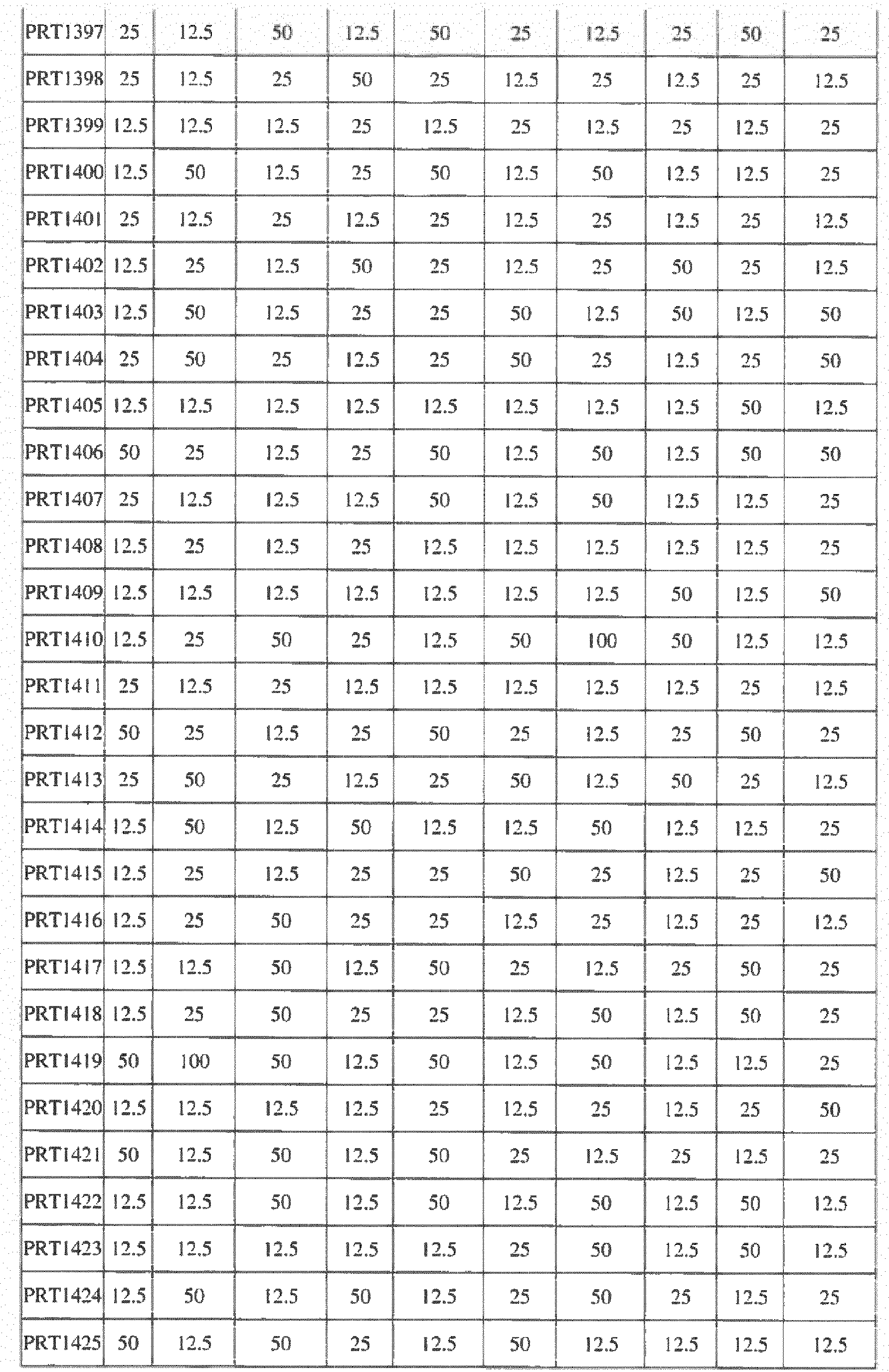

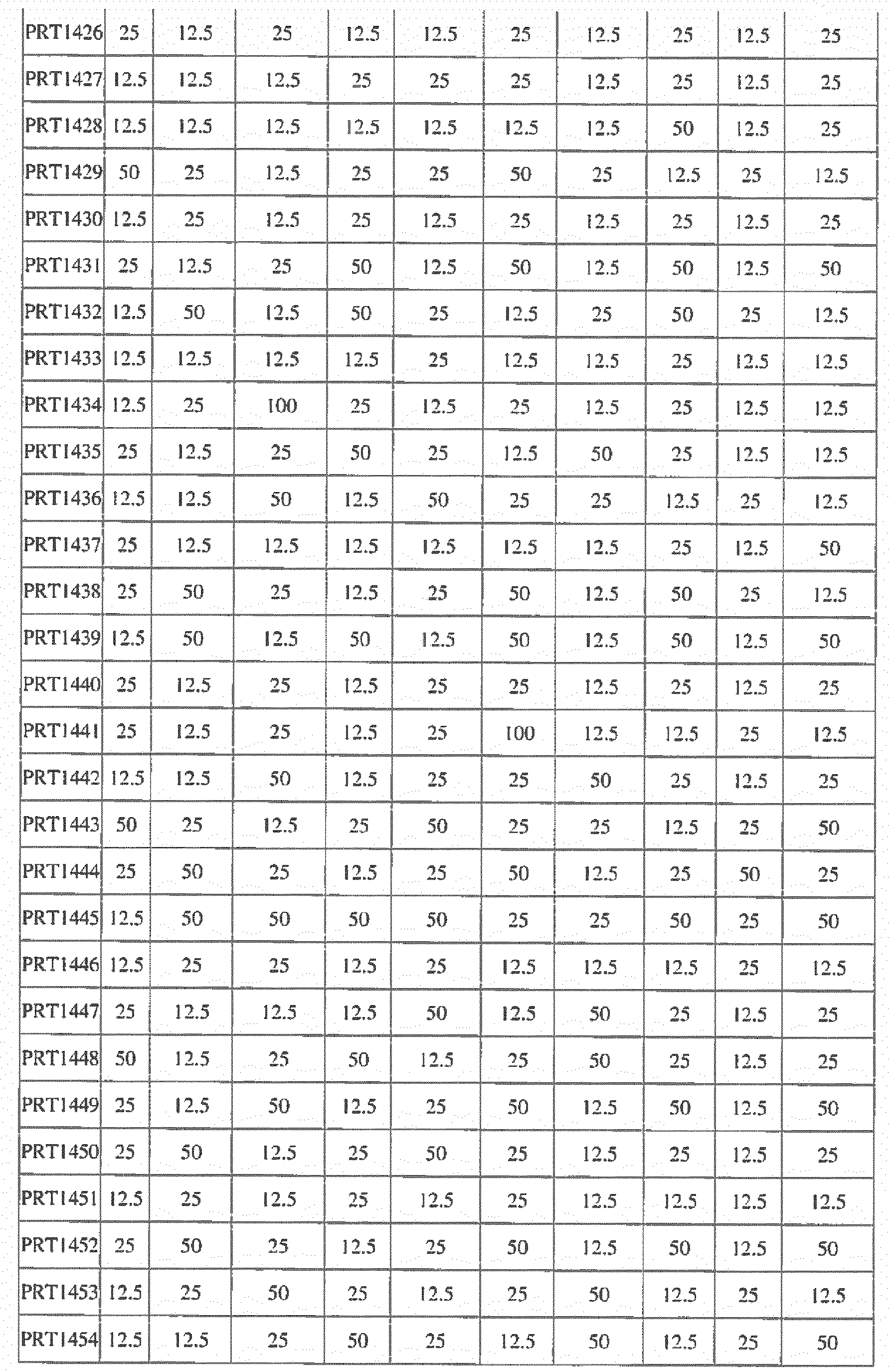

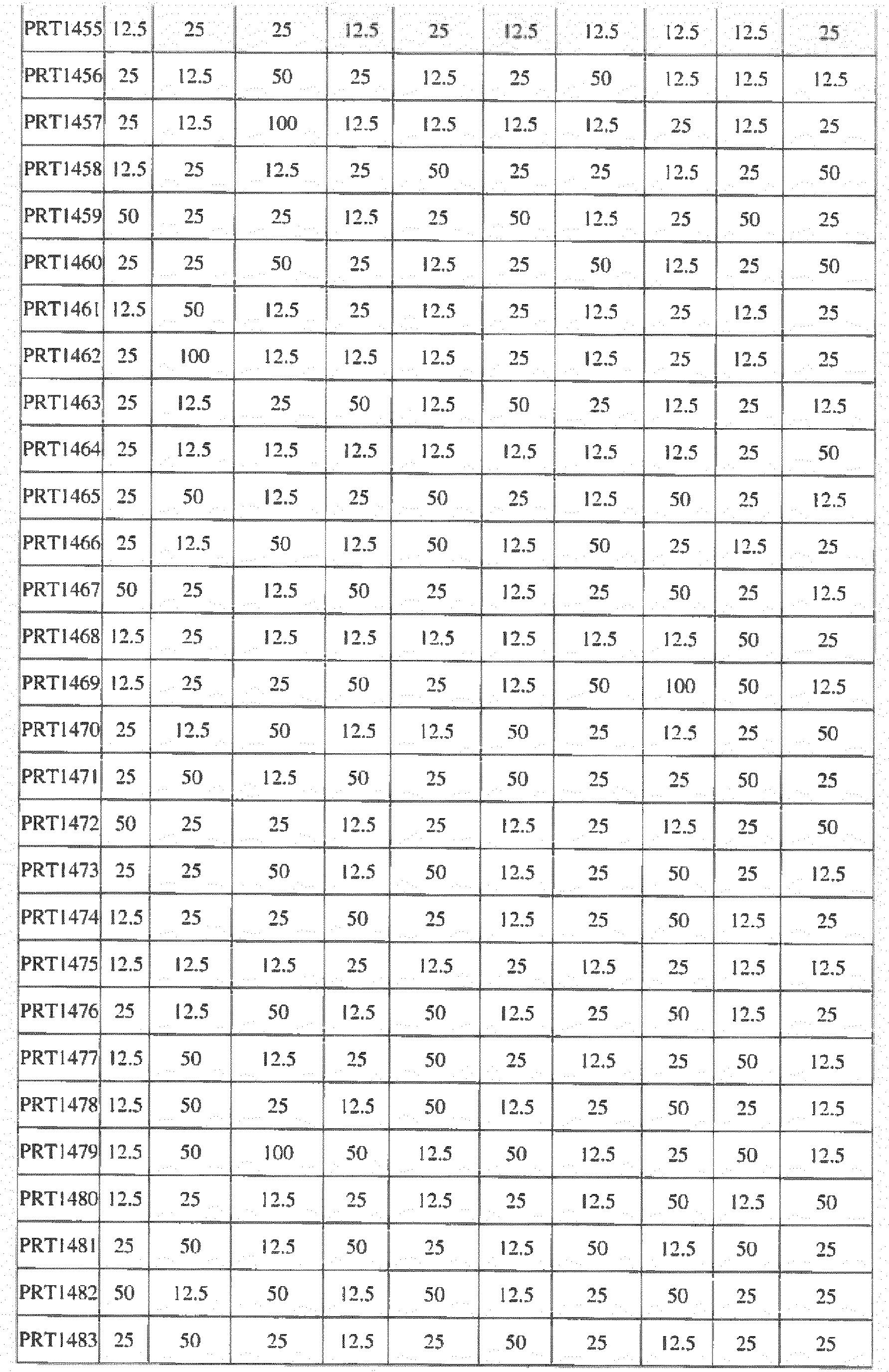

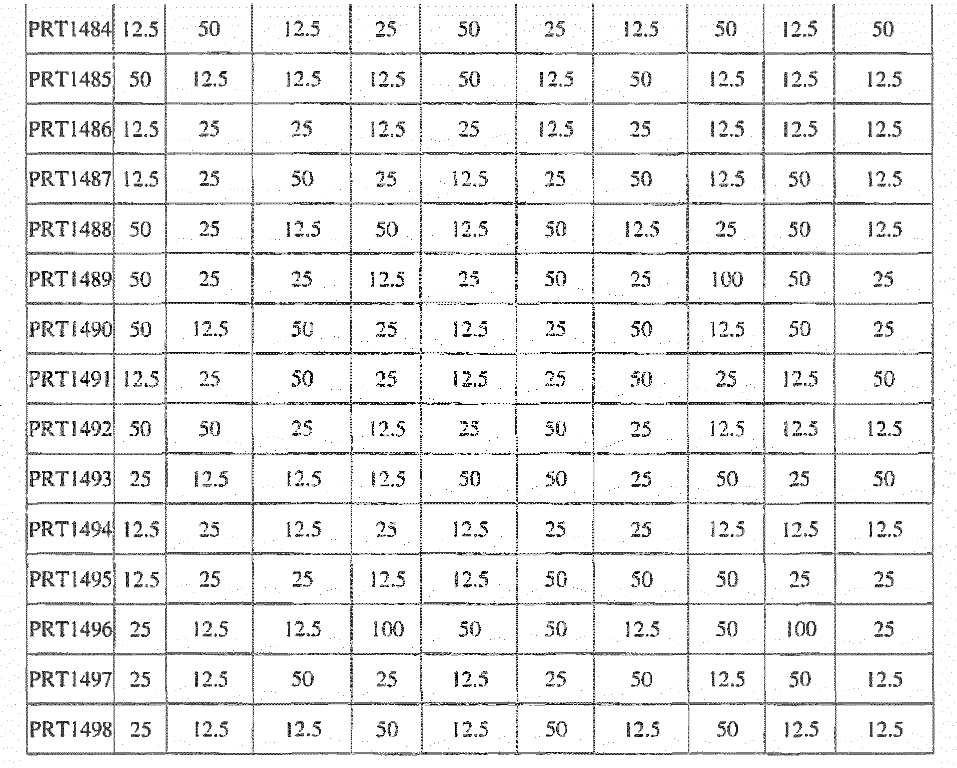

Conclusion: It can be known from the above table that: all of the recombinant human-originated EGF domain proteins (PRT1 to PRT1498 in Table I) set forth in the present invention exhibit inhibitory effects on various Gram-negative bacteria, such as, *E. coli, Pseudomonas aeruginosa, Klebsiella pneumonia, Enterobacter cloacae, Aeromonas hydrophila, Citrobacter diversus, Moraxella catarrhalis, Proteus mirabilis, Proteus vulgaris, Serratia marcescens,* etc_{.}

### Example 17: Silver staining detection of endotoxin (LPS) hydrolyzed by the human-originated EGF domain proteins (PRT1 to PRT1498 in Table I) such as the recombination protein hF VII-EGF1, etc.

1. The *E. coli* EH100 LPS (Sigma) samples were respectively incubated with the obtained human-originated EGF domain proteins (PRT1 to PRT1498 in Table I) such as the recombination protein hF VII-EGF1, etc.; and the control group was the LPS sample incubated with TBS buffer. The incubation lasted overnight. Then, the centrifugation was performed to collect the pellet.
2. The I × loading buffer for LPS (0.05 mol/L Tris-HCl, containing 10 g/L SDS, 100 g/L sucrose, 0.5% β-mercaptoethanol, 10 mg/L Bromophenol Blue, pH 6.8) was mixed with the sample of the treated pellet and incubated on a metal bath at 100°C for 5 min.
3. 15 % separating gel (containing 4 mol/L urea, Bio-Rad with a thickness of 1.0 mm, the detailed composition as follows: 1.15 mL of deionized water, 2.5 mL of 30% acrylamide, 1.3 mL of 1.5 mol/L Tris-HCl (pH 8.8, containing 10% SDS), 50 µL of 10% ammonium persulfate, 4 µL of TEMED) was mixed well and prepared. The top of the separating gel was overlaid with water.
4. 5% stacking gel was prepared as follows: 1.42 mL of deionized water, 0.33 mL of 30% acrylamide, 0.25 mL of 1 mol/L Tris-HCl (pH 6.8, containing 10% SDS), 20 µL of 10% ammonium persulfate, 2 µL of TEMED) were mixed well and poured. A ten-well comb was quickly inserted.
5. After the gel was prepared, the samples were loaded. Then, the gel was run at a voltage of 120 V to separate the samples. After running, the gel was taken to start silver staining. The gel was transferred into a tray washed thoroughly with deionized water, followed by washing the gel three times with deionized water.
6. After completion of the washing, the gel was fixed with 50 mL of fixation solution (30% alcohol, 10% glacial acetic acid, 7 g/L periodic acid) for oxidation at room temperature for 25 min. After completion of the fixation, the gel was washed three times each time with deionized water for 5 min.
7. After completion of the washing, the gel was stained in 100 mL of I g/L AgNO₃ at room temperature for 40 min. After completion of the staining, the gel was washed one time with ddH₂O.
8. The gel was transferred into 50 mL of 30 g/L Na₂CO₃ pre-chilled on ice, to which 0.02% formaldehyde was added just before visualization. When the band appeared or began to become dark, the reaction was stopped by adding 6 mL glacial acetic acid. The stopping solution was removed upon completion of the termination reaction and the gel was kept in deionized water.

The silver staining picture was exemplified by the protein hF VII-EGF1, as shown in Fig. 28. After treated with hF VH-EGF1, the LPS band is obviously decreased and becomes weak, indicating that the protein hF VII-EGF1 eliminates the majority of LPS. The silver staining pictures of PRT2 to PRT1498 in Table I are similar to that in Fig. 28. This indicated that the human-originated EGF domain proteins (PRT1 to PRT1498 in Table 1) described in the present invention can hydrolyze LPS and may be used in preparation of a medicament for treating endotoxemia caused by the Gram-negative bacteria.

## Claims

1. A human-originated EGF domain protein for use in treating Gram-negative bacteria infection, the amino acid sequence thereof being described in any one of SEQ ID NO: 1-SEQ ID NO: 1498 in the sequence listing.

2. The human-originated EGF domain protein for use in treating Gram-negative bacteria infection according to claim 1, **characterized in that** the amino acid sequence of the protein is described in SEQ ID NO:1, SEQ ID NO:2, SEQ ID NO:3, SEQ ID NO:4, SEQ ID NO:5, SEQ ID NO:6, SEQ ID NO:108, SEQ ID NO:109, SEQ ID NO:1472, SEQ ID NO:1473, SEQ ID NO:1474, SEQ ID NO:1475, SEQ ID NO:1476, SEQ ID NO:1477, SEQ ID NO:1478 or SEQ ID NO:1479 in the sequence listing.

3. The human-originated EGF domain protein for use in treating Gram-negative bacteria infection according to claim 1 or claim 2, **characterized in that** the protein is expressed by a prokaryotic or eukaryotic recombinant plasmid or prepared by chemical synthesis.

4. The human-originated EGF domain protein for use in treating Gram-negative bacteria infection according to any one of claims 1 to 3, **characterized in that** the infection causes endotoxemia.

5. The human-originated EGF domain protein for use in treating Gram-negative bacteria infection according to any one of claims 1 to 4, **characterized in that** the Gram-negative bacteria is one or more selected from *Escherichia coli DH5α, Escherichia coli BL21, Pseudomonas aeruginosa, Klebsiella pneumonia, Enterobacter cloacae, Aeromonas hydrophila, Citrobacter diversus, Moraxella catarrhalis, Proteus mirabilis, Proteus vulgaris* and *Serratia marcescens.*

6. A pharmaceutical composition for use in treating Gram-negative bacteria infection, **characterized in that** the composition comprises the human-originated EGF domain protein of any one of claims 1-5 for use in treating Gram-negative bacteria infection.

7. The pharmaceutical composition for use in treating Gram-negative bacteria infection according to claim 6, **characterized in that** the infection causes endotoxemia.

8. The pharmaceutical composition for use in treating Gram-negative bacteria infection according to claim 6 or 7, **characterized in that** the Gram-negative bacteria is one or more selected from *Escherichia coli DH5α, Escherichia coli BL21, Pseudomonas aeruginosa, Klebsiella pneumonia, Enterobacter cloacae, Aeromonas hydrophila, Citrobacter diversus, Moraxella catarrhalis, Proteus mirabilis, Proteus vulgaris* and *Serratia marcescens.*

9. An *in vitro* method for hydrolyzing a lipopolysaccharide of Gram-negative bacteria, comprising the administration of a human-originated EGF domain protein, the amino acid sequence of which is described in any one of SEQ ID NO: 1-SEQ ID NO: 1498 in the sequence listing.

10. The *in vitro* method of claim 9, **characterized in that** the Gram-negative bacteria is one or more selected from *Escherichia coli DH5α, Escherichia coli BL21, Pseudomonas aeruginosa, Klebsiella pneumonia, Enterobacter cloacae, Aeromonas hydrophila, Citrobacter diversus, Moraxella catarrhalis, Proteus mirabilis, Proteus vulgaris and Serratia marcescens.*

11. The *in vitro* method of any one of claims 9-10, **characterized in that** the amino acid sequence of the protein is described in SEQ ID NO:1, SEQ ID NO:2, SEQ ID NO:3, SEQ ID NO:4, SEQ ID NO:5, SEQ ID NO:6, SEQ ID NO:108, SEQ ID NO:109, SEQ ID NO:1472, SEQ ID NO:1473, SEQ ID NO:1474, SEQ ID NO:1475, SEQ ID NO:1476, SEQ ID NO:1477, SEQ ID NO:1478 or SEQ ID NO:1479 in the sequence listing.

12. An *in vitro* method for hydrolyzing a lipopolysaccharide of Gram-negative bacteria, comprising the administration of a composition, wherein the composition comprises the human-originated EGF domain protein of any one of claims 9-11.

13. The *in vitro* method of claim 12, **characterized in that** the Gram-negative bacteria is one or more selected from *Escherichia coli DH5α, Escherichia coli BL21, Pseudomonas aeruginosa, Klebsiella pneumonia, Enterobacter cloacae, Aeromonas hydrophila, Citrobacter diversus, Moraxella catarrhalis, Proteus mirabilis, Proteus vulgaris and Serratia marcescens.*

## Patentansprüche

1. Ein vom Menschen stammendes EGF-Domänen-Protein zur Verwendung bei der Behandlung einer Gram-negativen Bakterieninfektion, wobei die Aminosäuresequenz davon in einer der SEQ ID NR: 1-SEQ ID NR: 1498 im Sequenzprotokoll beschrieben ist.

2. Das vom Menschen stammende EGF-Domänen-Protein zur Verwendung bei der Behandlung einer Gram-negativen Bakterieninfektion nach Anspruch 1, **dadurch gekennzeichnet, dass** die Aminosäuresequenz des Proteins in SEQ ID NR:1, SEQ ID NR:2, SEQ ID NR:3, SEQ ID NR:4, SEQ ID NR:5, SEQ ID NR:6, SEQ ID NR:108, SEQ ID NR:109, SEQ ID NR:1472, SEQ ID NR:1473, SEQ ID NR:1474, SEQ ID NR:1475, SEQ ID NR:1476, SEQ ID NR:1477, SEQ ID NR:1478 oder SEQ ID NR:1479 im Sequenzprotokoll beschrieben ist.

3. Das vom Menschen stammende EGF-Domänen-Protein zur Verwendung bei der Behandlung einer Gram-negativen Bakterieninfektion nach Anspruch 1 oder Anspruch 2, **dadurch gekennzeichnet, dass** das Protein durch ein prokaryotisches oder eukaryotisches rekombinantes Plasmid exprimiert wird oder durch chemische Synthese hergestellt wird.

4. Das vom Menschen stammende EGF-Domänen-Protein zur Verwendung bei der Behandlung einer Gram-negativen Bakterieninfektion nach einem der Ansprüche 1 bis 3, **dadurch gekennzeichnet, dass** die Infektion Endotoxämie verursacht.

5. Das vom Menschen stammende EGF-Domänen-Protein zur Verwendung bei der Behandlung einer Gram-negativen Bakterieninfektion nach einem der Ansprüche 1 bis 4, **dadurch gekennzeichnet, dass** die Gram-negativen Bakterien ausgewählt sind aus einem oder mehreren von *Escherichia coli DH5α, Escherichia coli BL21, Pseudomonas aeruginosa, Klebsiella pneumonia, Enterobacter cloacae, Aeromonas hydrophila, Citrobacter diversus, Moraxella catarrhalis, Proteus mirabilis, Proteus vulgaris* und *Serratia marcescens.*

6. Eine pharmazeutische Zusammensetzung zur Verwendung bei der Behandlung einer Gram-negativen Bakterieninfektion, **dadurch gekennzeichnet, dass** die Zusammensetzung das vom Menschen stammende EGF-Domänen-Protein nach einem der Ansprüche 1-5 zur Verwendung bei der Behandlung einer Gram-negativen Bakterieninfektion umfasst.

7. Die pharmazeutische Zusammensetzung zur Verwendung bei der Behandlung einer Gram-negativen Bakterieninfektion nach Anspruch 6, **dadurch gekennzeichnet, dass** die Infektion Endotoxämie verursacht.

8. Die pharmazeutische Zusammensetzung zur Verwendung bei der Behandlung einer Gram-negativen Bakterieninfektion nach Anspruch 6 oder 7, **dadurch gekennzeichnet, dass** die Gram-negativen Bakterien ausgewählt sind aus einem oder mehreren von *Escherichia coli DH5α, Escherichia coli BL21, Pseudomonas aeruginosa, Klebsiella pneumonia, Enterobacter cloacae, Aeromonas hydrophila, Citrobacter diversus, Moraxella catarrhalis, Proteus mirabilis, Proteus vulgaris* und *Serratia marcescens.*

9. Ein *in vitro* -Verfahren zum Hydrolysieren eines Lipopolysaccharids von Gram-negativen Bakterien, umfassend die Verabreichung eines vom Menschen stammenden EGF-Domänen-Proteins, wobei die Aminosäuresequenz davon in einer der SEQ ID NR: 1-SEQ ID NR: 1498 im Sequenzprotokoll beschrieben ist.

10. Das *in vitro* -Verfahren nach Anspruch 9, **dadurch gekennzeichnet, dass** die Gram-negativen Bakterien ausgewählt sind aus einem oder mehreren von *Escherichia coli DH5α, Escherichia coli BL21, Pseudomonas aeruginosa, Klebsiella pneumonia, Enterobacter cloacae, Aeromonas hydrophila, Citrobacter diversus, Moraxella catarrhalis, Proteus mirabilis, Proteus vulgaris und Serratia marcescens.*

11. Das *in vitro* -Verfahren nach einem der Ansprüche 9-10, **dadurch gekennzeichnet, dass** die Aminosäuresequenz des Proteins in SEQ ID NR:1, SEQ ID NR:2, SEQ ID NR:3, SEQ ID NR:4, SEQ ID NR:5, SEQ ID NR:6, SEQ ID NR:108, SEQ ID NR:109, SEQ ID NR:1472, SEQ ID NR:1473, SEQ ID NR:1474, SEQ ID NR:1475, SEQ ID NR:1476, SEQ ID NR:1477, SEQ ID NR:1478 oder SEQ ID NR:1479 im Sequenzprotokoll beschrieben ist.

12. Ein *in vitro* -Verfahren zum Hydrolysieren eines Lipopolysaccharids von Gram-negativen Bakterien, umfassend die Verabreichung einer Zusammensetzung, wobei die Zusammensetzung das vom Menschen stammende EGF-Domänen-Protein nach einem der Ansprüche 9-11 umfasst.

13. Das *in vitro* -Verfahren nach Anspruch 12, **dadurch gekennzeichnet, dass** die Gram-negativen Bakterien ausgewählt sind aus einem oder mehreren von *Escherichia coli DH5α, Escherichia coli BL21, Pseudomonas aeruginosa, Klebsiella pneumonia, Enterobacter cloacae Aeromonas hydrophila, Citrobacter diversus, Moraxella catarrhalis, Proteus mirabilis, Proteus vulgaris und Serratia marcescens.*

## Revendications

1. Protéine à domaine EGF d'origine humaine destinée à être utilisée pour le traitement d'une infection à bactéries Gram-négatives, la séquence d'acides aminés de celle-ci étant décrite dans l'une quelconque de la SEQ ID N° : 1 à la SEQ ID N° : 1498 dans le listage de séquences.

2. Protéine à domaine EGF d'origine humaine destinée à être utilisée pour le traitement d'une infection à bactéries Gram-négatives selon la revendication 1, **caractérisée en ce que** la séquence d'acides aminés de la protéine est décrite dans la SEQ ID N° : 1, la SEQ ID N° : 2, la SEQ ID N° : 3, la SEQ ID N° : 4, la SEQ ID N° : 5, la SEQ ID N° : 6, la SEQ ID N° : 108, la SEQ ID N° : 109, la SEQ ID N° : 1472, la SEQ ID N° : 1473, la SEQ ID N° : 1474, la SEQ ID N° : 1475, la SEQ ID N° : 1476, la SEQ ID N° : 1477, la SEQ ID N° : 1478 ou la SEQ ID N° : 1479 dans le listage de séquences.

3. Protéine à domaine EGF d'origine humaine destinée à être utilisée pour le traitement d'une infection à bactéries Gram-négatives selon la revendication 1 ou la revendication 2, **caractérisée en ce que** la protéine est exprimée par un plasmide recombinant procaryote ou eucaryote ou préparée par synthèse chimique.

4. Protéine à domaine EGF d'origine humaine destinée à être utilisée pour le traitement d'une infection à bactéries Gram-négatives selon l'une quelconque des revendications 1 à 3, **caractérisée en ce que** l'infection entraîne une endotoxémie.

5. Protéine à domaine EGF d'origine humaine destinée à être utilisée pour le traitement d'une infection à bactéries Gram-négatives selon l'une quelconque des revendications 1 à 4, **caractérisée en ce que** les bactéries Gram-négatives sont l'une ou plusieurs choisies parmi *Escherichia coli DH5α, Escherichia coli BL21, Pseudomonas aeruginosa, Klebsiella pneumonia, Enterobacter cloacae, Aeromonas hydrophila, Citrobacter diversus, Moraxella catarrhalis, Proteus mirabilis, Proteus vulgaris* et *Serratia marcescens.*

6. Composition pharmaceutique destinée à être utilisée pour le traitement d'une infection à bactéries Gram-négatives, **caractérisée en ce que** la composition comprend la protéine à domaine EGF d'origine humaine selon l'une quelconque des revendications 1 à 5 destinée à être utilisée pour le traitement d'une infection à bactéries Gram-négatives.

7. Composition pharmaceutique destinée à être utilisée pour le traitement d'une infection à bactéries Gram-négatives selon la revendication 6, **caractérisée en ce que** l'infection entraîne une endotoxémie.

8. Composition pharmaceutique destinée à être utilisée pour le traitement d'une infection à bactéries Gram-négatives selon la revendication 6 ou 7, **caractérisée en ce que** les bactéries Gram-négatives sont l'une ou plusieurs choisies parmi *Escherichia coli DH5α, Escherichia coli BL21, Pseudomonas aeruginosa, Klebsiella pneumonia, Enterobacter cloacae, Aeromonas hydrophila, Citrobacter diversus, Moraxella catarrhalis, Proteus mirabilis, Proteus vulgaris* et *Serratia marcescens.*

9. Procédé *in vitro* d'hydrolyse d'un lipopolysaccharide de bactéries Gram-négatives, comprenant l'administration d'une protéine à domaine EGF d'origine humaine, dont la séquence d'acides aminés est décrite dans l'une quelconque de la SEQ ID N° : 1 à la SEQ ID N° : 1498 dans le listage de séquences.

10. Procédé *in vitro* selon la revendication 9, **caractérisé en ce que** les bactéries Gram-négatives sont l'une ou plusieurs choisies parmi *Escherichia coli DH5α, Escherichia coli BL21, Pseudomonas aeruginosa, Klebsiella pneumonia, Enterobacter cloacae, Aeromonas hydrophila, Citrobacter diversus, Moraxella catarrhalis, Proteus mirabilis, Proteus vulgaris* and *Serratia marcescens.*

11. Procédé *in vitro* selon l'une quelconque des revendications 9 à 10, **caractérisé en ce que** la séquence d'acides aminés de la protéine est décrite dans la SEQ ID N° : 1, la SEQ ID N° : 2, la SEQ ID N° : 3, la SEQ ID N° : 4, la SEQ ID N° : 5, la SEQ ID N° : 6, la SEQ ID N° : 108, la SEQ ID N° : 109, la SEQ ID N° : 1472, la SEQ ID N° : 1473, la SEQ ID N° : 1474, la SEQ ID N° : 1475, la SEQ ID N° : 1476, la SEQ ID N° : 1477, la SEQ ID N° : 1478 ou la SEQ ID N° : 1479 dans le listage de séquences.

12. Procédé *in vitro* d'hydrolyse d'un lipopolysaccharide de bactéries Gram-négatives, comprenant l'administration d'une composition, la composition comprenant la protéine à domaine EGF d'origine humaine selon l'une quelconque des revendications 9 à 11.

13. Procédé *in vitro* selon la revendication 12, **caractérisé en ce que** les bactéries Gram-négatives sont l'une ou plusieurs choisies parmi *Escherichia coli DH5α, Escherichia coli BL21, Pseudomonas aeruginosa, Klebsiella pneumonia, Enterobacter cloacae, Aeromonas hydrophila, Citrobacter diversus, Moraxella catarrhalis, Proteus mirabilis, Proteus vulgaris et Serratia marcescens.*


### SEQUENCE LISTING

<110> SICHUAN UNIVERSITY
<120> HUMAN-ORIGINATED EGF DOMAIN PROTEINS AND USE OF THE SAME
<160> 1498
<170> PatentIn version 3.3
<210> 1
   <211> 37
   <212> PRT
   <213> Homo sapiens
<400> 1
   Asp Gly Asp Gln Cys Ala Ser Ser Pro Cys Gln Asn Gly Gly Ser Cys
   1               5                   10                  15
   Lys Asp Gln Leu Gln Ser Tyr Ile Cys Phe Cys Leu Pro Ala Phe Glu
               20                  25                  30
   Gly Arg Asn Cys Glu
           35
<210> 2
   <211> 42
   <212> PRT
   <213> Homo sapiens
<400> 2
   Asp Gln Leu Ile Cys Val Asn Glu Asn Gly Gly Cys Glu Gln Tyr Cys
   1               5                   10                  15
   Ser Asp His Thr Gly Thr Lys Arg Ser Cys Arg Cys His Glu Gly Tyr
               20                  25                  30
   Ser Leu Leu Ala Asp Gly Val Ser Cys Thr
           35                  40
<210> 3
   <211> 37
   <212> PRT
   <213> Homo sapiens
<400> 3
   Asp Gly Asp Gln Cys Glu Ser Asn Pro Cys Leu Asn Gly Gly Ser Cys
   1               5                   10                  15
   Lys Asp Asp Ile Asn Ser Tyr Glu Cys Trp Cys Pro Phe Gly Phe Glu
               20                  25                  30
   Gly Lys Asn Cys Glu
           35
<210> 4
   <211> 42
   <212> PRT
   <213> Homo sapiens
<400> 4
   Leu Asp Val Thr Cys Asn Ile Lys Asn Gly Arg Cys Glu Gln Phe Cys
   1               5                   10                  15
   Lys Asn Ser Ala Asp Asn Lys Val Val Cys Ser Cys Thr Glu Gly Tyr
               20                  25                  30
   Arg Leu Ala Glu Asn Gln Lys Ser Cys Glu
           35                  40
<210> 5
   <211> 37
   <212> PRT
   <213> Homo sapiens
<400> 5
   Asp Gly Asp Gln Cys Glu Thr Ser Pro Cys Gln Asn Gln Gly Lys Cys
   1               5                   10                  15
   Lys Asp Gly Leu Gly Glu Tyr Thr Cys Thr Cys Leu Glu Gly Phe Glu
               20                  25                  30
   Gly Lys Asn Cys Glu
           35
<210> 6
   <211> 41
   <212> PRT
   <213> Homo sapiens
<400> 6
   Thr Arg Lys Leu Cys Ser Leu Asp Asn Gly Asp Cys Asp Gln Phe Cys
   1               5                   10                  15
   His Glu Glu Gln Asn Ser Val Val Cys Ser Cys Ala Arg Gly Tyr Thr
               20                  25                  30
   Leu Ala Asp Asn Gly Lys Ala Cys Ile
           35                  40
<210> 7
   <211> 35
   <212> PRT
   <213> Homo sapiens
<400> 7
   Thr Cys Leu Ser Ser Lys Glu Gly Thr Ile Cys Ser Gly Asn Gly Val
   1               5                   10                  15
   Cys Ser Asn Glu Leu Lys Cys Val Cys Asn Arg His Trp Ile Gly Ser
               20                  25                  30
   Asp Cys Asn
           35
<210> 8
   <211> 34
   <212> PRT
   <213> Homo sapiens
<400> 8
   Leu Gln Phe Asp Cys Leu Pro Glu Lys Cys Asn Thr Arg Gly Val Cys
   1               5                   10                  15
   Asn Asn Arg Lys Asn Cys His Cys Met Tyr Gly Trp Ala Pro Pro Phe
               20                  25                  30
   Cys Glu
<210> 9
   <211> 30
   <212> PRT
   <213> Homo sapiens
<400> 9
   Arg Cys Leu Thr Ala Cys His Ser His Gly Val Cys Asn Ser Asn His
   1               5                   10                  15
   Asn Cys His Cys Ala Pro Gly Trp Ala Pro Pro Phe Cys Asp
               20                  25                  30
<210> 10
   <211> 30
   <212> PRT
   <213> Homo sapiens
<400> 10
   Asn Cys Ser Ala Gln Cys His Asn His Gly Val Cys Asn His Lys Gln
   1               5                   10                  15
   Glu Cys His Cys His Ala Gly Trp Ala Pro Pro His Cys Ala
               20                  25                  30
<210> 11
   <211> 32
   <212> PRT
   <213> Homo sapiens
<400> 11
   Asp Cys Asp Val Gln Lys Lys Cys His Gly His Gly Val Cys Asn Ser
   1               5                   10                  15
   Asn Lys Asn Cys His Cys Glu Asn Gly Trp Ala Pro Pro Asn Cys Glu
               20                  25                  30
<210> 12
   <211> 35
   <212> PRT
   <213> Homo sapiens
<400> 12
   Leu Asn Tyr Asp Cys Asp Val Gln Lys Lys Cys His Gly His Gly Val
   1               5                   10                  15
   Cys Asn Ser Asn Lys Asn Cys His Cys Glu Asn Gly Trp Ala Pro Pro
               20                  25                  30
   Asn Cys Glu
           35
<210> 13
   <211> 36
   <212> PRT
   <213> Homo sapiens
<400> 13
   Pro Ala Arg Ser Cys Ala Glu Glu Pro Cys Gly Ala Gly Thr Cys Lys
   1               5                   10                  15
   Glu Thr Glu Gly His Val Ile Cys Leu Cys Pro Pro Gly Tyr Thr Gly
               20                  25                  30
   Glu His Cys Asn
           35
<210> 14
   <211> 39
   <212> PRT
   <213> Homo sapiens
<400> 14
   Pro Pro Lys Pro Cys Asp Ser Gln Pro Cys Phe His Gly Gly Thr Cys
   1               5                   10                  15
   Gln Asp Trp Ala Leu Gly Gly Gly Phe Thr Cys Ser Cys Pro Ala Gly
               20                  25                  30
   Arg Gly Gly Ala Val Cys Glu
           35
<210> 15
   <211> 38
   <212> PRT
   <213> Homo sapiens
<400> 15
   Gly Asp His Pro Cys Leu Pro Asn Pro Cys His Gly Gly Ala Pro Cys
   1               5                   10                  15
   Gln Asn Leu Glu Ala Gly Arg Phe His Cys Gln Cys Pro Pro Gly Arg
               20                  25                  30
   Val Gly Pro Thr Cys Ala
           35
<210> 16
   <211> 38
   <212> PRT
   <213> Homo sapiens
<400> 16
   Glu Lys Ser Pro Cys Gln Pro Asn Pro Cys His Gly Ala Ala Pro Cys
   1               5                   10                  15
   Arg Val Leu Pro Glu Gly Gly Ala Gln Cys Glu Cys Pro Leu Gly Arg
               20                  25                  30
   Glu Gly Thr Phe Cys Gln
           35
<210> 17
   <211> 40
   <212> PRT
   <213> Homo sapiens
<400> 17
   Ala Gly His Pro Cys Thr Arg Ala Ser Gly His Pro Cys Leu Asn Gly
   1               5                   10                  15
   Ala Ser Cys Val Pro Arg Glu Ala Ala Tyr Val Cys Leu Cys Pro Gly
               20                  25                  30
   Gly Phe Ser Gly Pro His Cys Glu
           35                  40
<210> 18
   <211> 54
   <212> PRT
   <213> Homo sapiens
<400> 18
   Cys Gln Cys Asn Pro His Gly Ser Tyr Gly Gly Thr Cys Asp Pro Ala
   1               5                   10                  15
   Thr Gly Gln Cys Ser Cys Arg Pro Gly Val Gly Gly Leu Arg Cys Asp
               20                  25                  30
   Arg Cys Glu Pro Gly Phe Trp Asn Phe Arg Gly Ile Val Thr Asp Gly
           35                  40                  45
   Arg Ser Gly Cys Thr Pro
       50
<210> 19
   <211> 47
   <212> PRT
   <213> Homo sapiens
<400> 19
   Cys Ser Cys Asp Pro Gln Gly Ala Val Arg Asp Asp Cys Glu Gln Met
   1               5                   10                  15
   Thr Gly Leu Cys Ser Cys Lys Pro Gly Val Ala Gly Pro Lys Cys Gly
               20                  25                  30
   Gln Cys Pro Asp Gly Arg Ala Leu Gly Pro Ala Gly Cys Glu Ala
           35                  40                  45
<210> 20
   <211> 42
   <212> PRT
   <213> Homo sapiens
<400> 20
   Gln Lys Asn Leu Cys Asn Asn Thr Gly Asp Pro Glu Met Cys Pro Glu
   1               5                   10                  15
   Asn Gly Ser Cys Val Pro Asp Gly Pro Gly Leu Leu Gln Cys Val Cys
               20                  25                  30
   Ala Asp Gly Phe His Gly Tyr Lys Cys Met
           35                  40
<210> 21
   <211> 24
   <212> PRT
   <213> Homo sapiens
<400> 21
   Asn Cys Ala Thr Pro Cys Thr Glu Gly Cys Glu Cys Asn Gln Gly Phe
   1               5                   10                  15
   Val Leu Ser Thr Ser Gln Cys Val
               20
<210> 22
   <211> 36
   <212> PRT
   <213> Homo sapiens
<400> 22
   Trp Cys Glu Asp Asn Gly Gly Cys Glu Gln Ile Cys Thr Ser Arg Val
   1               5                   10                  15
   Asp Gly Pro Leu Cys Ser Cys Val Thr Gly Thr Leu Gln Glu Asp Gly
               20                  25                  30
   Lys Ser Cys Arg
           35
<210> 23
   <211> 41
   <212> PRT
   <213> Homo sapiens
<400> 23
   Lys Lys Asn Pro Cys Asn Ala Glu Phe Gln Asn Phe Cys Ile His Gly
   1               5                   10                  15
   Glu Cys Lys Tyr Ile Glu His Leu Glu Ala Val Thr Cys Lys Cys Gln
               20                  25                  30
   Gln Glu Tyr Phe Gly Glu Arg Cys Gly
           35                  40
<210> 24
   <211> 37
   <212> PRT
   <213> Homo sapiens
<400> 24
   Glu Leu Asp Leu Cys Tyr Ser Asn Pro Cys Arg Asn Gly Gly Ala Cys
   1               5                   10                  15
   Ala Arg Arg Glu Gly Gly Tyr Thr Cys Val Cys Arg Pro Arg Phe Thr
               20                  25                  30
   Gly Glu Asp Cys Glu
           35
<210> 25
   <211> 34
   <212> PRT
   <213> Homo sapiens
<400> 25
   Pro Gly Val Cys Arg Asn Gly Gly Thr Cys Thr Asp Ala Pro Asn Gly
   1               5                   10                  15
   Gly Phe Arg Cys Gln Cys Pro Ala Gly Gly Ala Phe Glu Gly Pro Arg
               20                  25                  30
   Cys Glu
<210> 26
   <211> 33
   <212> PRT
   <213> Homo sapiens
<400> 26
   Cys Asp Ser Gly Pro Cys Lys Asn Ser Gly Phe Cys Ser Glu Arg Trp
   1               5                   10                  15
   Gly Ser Phe Ser Cys Asp Cys Pro Val Gly Phe Gly Gly Lys Asp Cys
               20                  25                  30
   Gln
<210> 27
   <211> 35
   <212> PRT
   <213> Homo sapiens
<400> 27
   Asn Ala Cys Ala Ser Gly Pro Cys Pro Pro His Ala Asp Cys Arg Asp
   1               5                   10                  15
   Leu Trp Gln Thr Phe Ser Cys Thr Cys Gln Pro Gly Tyr Tyr Gly Pro
               20                  25                  30
   Gly Cys Val
           35
<210> 28
   <211> 39
   <212> PRT
   <213> Homo sapiens
<400> 28
   Val Asp Ala Cys Leu Leu Asn Pro Cys Gln Asn Gln Gly Ser Cys Arg
   1               5                   10                  15
   His Leu Pro Gly Ala Pro His Gly Tyr Thr Cys Asp Cys Val Gly Gly
               20                  25                  30
   Tyr Phe Gly His His Cys Glu
           35
<210> 29
   <211> 38
   <212> PRT
   <213> Homo sapiens
<400> 29
   Pro Cys Asn Cys Asp Val His Lys Gly Phe Asp Pro Asn Cys Asn Lys
   1               5                   10                  15
   Thr Asn Gly Gln Cys His Cys Lys Glu Phe His Tyr Arg Pro Arg Gly
               20                  25                  30
   Ser Asp Ser Cys Leu Pro
           35
<210> 30
   <211> 39
   <212> PRT
   <213> Homo sapiens
<400> 30
   Pro Cys Asp Cys Tyr Pro Val Gly Ser Thr Ser Arg Ser Cys Ala Pro
   1               5                   10                  15
   His Ser Gly Gln Cys Pro Cys Arg Pro Gly Ala Leu Gly Arg Gln Cys
               20                  25                  30
   Asn Ser Cys Asp Ser Pro Phe
           35
<210> 31
   <211> 46
   <212> PRT
   <213> Homo sapiens
<400> 31
   Cys Asp Cys Tyr Pro Val Gly Ser Thr Ser Arg Ser Cys Ala Pro His
   1 5 10 15
   Ser Gly Gln Cys Pro Cys Arg Pro Gly Ala Leu Gly Arg Gln Cys Asn
   20 25 30
   Ser Cys Asp Ser Pro Phe Ala Glu Val Thr Ala Ser Gly Cys
   35 40 45
<210> 32
   <211> 43
   <212> PRT
   <213> Homo sapiens
<400> 32
   Arg Cys Glu Ala Gln Lys Trp Gly Pro Glu Cys Asn His Leu Cys Thr
   1 5 10 15
   Ala Cys Met Asn Asn Gly Val Cys His Glu Asp Thr Gly Glu Cys Ile
   20 25 30
   Cys Pro Pro Gly Phe Met Gly Arg Thr Cys Glu
   35 40
<210> 33
   <211> 46
   <212> PRT
   <213> Homo sapiens
<400> 33
   Ala Cys Glu Leu His Thr Phe Gly Arg Thr Cys Lys Glu Arg Cys Ser
   1 5 10 15
   Gly Gln Glu Gly Cys Lys Ser Tyr Val Phe Cys Leu Pro Asp Pro Tyr
   20 25 30
   Gly Cys Ser Cys Ala Thr Gly Trp Lys Gly Leu Gln Cys Asn
   35 40 45
<210> 34
   <211> 41
   <212> PRT
   <213> Homo sapiens
<400> 34
   Ala Cys His Pro Gly Phe Tyr Gly Pro Asp Cys Lys Leu Arg Cys Ser
   1 5 10 15
   Cys Asn Asn Gly Glu Met Cys Asp Arg Phe Gln Gly Cys Leu Cys Ser
   20 25 30
   Pro Gly Trp Gln Gly Leu Gln Cys Glu
   35 40
<210> 35
   <211> 49
   <212> PRT
   <213> Homo sapiens
<400> 35
   Ala Met Asp Leu Cys Ala Arg Arg Leu Leu Asp Pro Cys Glu His Gln
   1               5                   10                  15
   Cys Asp Pro Glu Thr Gly Arg Arg Glu His Arg Ala Ala Gly Glu Cys
               20                  25                  30
   Leu Cys Tyr Glu Gly Tyr Met Lys Asp Pro Val His Lys His Leu Cys
           35                  40                  45
   Ile
<210> 36
   <211> 45
   <212> PRT
   <213> Homo sapiens
<400> 36
   Pro Val Arg Asp Cys Ser Lys Asp Asn Gly Gly Cys Ser Lys Asn Phe
   1               5                   10                  15
   Arg Cys Ile Ser Asp Arg Lys Leu Asp Ser Thr Gly Cys Val Cys Pro
               20                  25                  30
   Ser Gly Leu Ser Pro Met Lys Asp Ser Ser Gly Cys Tyr
           35                  40                  45
<210> 37
   <211> 53
   <212> PRT
   <213> Homo sapiens
<400> 37
   Ile Gly Val Asp Cys Ser Asp Gly Phe Asn Gly Gly Cys Glu Gln Leu
   1               5                   10                  15
   Cys Leu Gln Gln Met Ala Pro Phe Pro Asp Asp Pro Thr Leu Tyr Asn
               20                  25                  30
   Ile Leu Met Phe Cys Gly Cys Ile Glu Asp Tyr Lys Leu Gly Val Asp
           35                  40                  45
   Gly Arg Ser Cys Gln
       50
<210> 38
   <211> 41
   <212> PRT
   <213> Homo sapiens
<400> 38
   Val Arg Asp Leu Cys Gly Gln Arg Thr Thr Asp Ala Cys Glu Gln Leu
   1               5                   10                  15
   Cys Asp Pro Glu Thr Gly Glu Cys Ser Cys His Glu Gly Tyr Ala Pro
               20                  25                  30
   Asp Pro Val His Arg His Leu Cys Val
           35                  40
<210> 39
   <211> 45
   <212> PRT
   <213> Homo sapiens
<400> 39
   Pro Val Arg Asp Cys Ser Arg Asn Asn Gly Gly Cys Thr Arg Asn Phe
   1               5                   10                  15
   Lys Cys Val Ser Asp Arg Gln Val Asp Ser Ser Gly Cys Val Cys Pro
               20                  25                  30
   Glu Glu Leu Lys Pro Met Lys Asp Gly Ser Gly Cys Tyr
           35                  40                  45
<210> 40
   <211> 53
   <212> PRT
   <213> Homo sapiens
<400> 40
   Lys Gly Ile Asp Cys Ser Asp Gly Phe Asn Gly Gly Cys Glu Gln Leu
   1               5                   10                  15
   Cys Leu Gln Gln Thr Leu Pro Leu Pro Tyr Asp Ala Thr Ser Ser Thr
               20                  25                  30
   Ile Phe Met Phe Cys Gly Cys Val Glu Glu Tyr Lys Leu Ala Pro Asp
           35                  40                  45
   Gly Lys Ser Cys Leu
       50
<210> 41
   <211> 29
   <212> PRT
   <213> Homo sapiens
<400> 41
   Cys Asp Arg Pro Cys Val Asn Gly Gly Arg Cys Asn Pro Gly Thr Gly
   1               5                   10                  15
   Gln Cys Val Cys Pro Ala Gly Trp Val Gly Glu Gln Cys
               20                  25
<210> 42
   <211> 46
   <212> PRT
   <213> Homo sapiens
<400> 42
   Cys Gln Cys Asn Gly His Ser Lys Cys Ile Asn Gln Ser Ile Cys Glu
   1               5                   10                  15
   Lys Cys Glu Asn Leu Thr Thr Gly Lys His Cys Glu Thr Cys Ile Ser
               20                  25                  30
   Gly Phe Tyr Gly Asp Pro Thr Asn Gly Gly Lys Cys Gln Pro
           35                  40                  45
<210> 43
   <211> 49
   <212> PRT
   <213> Homo sapiens
<400> 43
   Cys Lys Cys Asn Gly His Ala Ser Leu Cys Asn Thr Asn Thr Gly Lys
   1               5                   10                  15
   Cys Phe Cys Thr Thr Lys Gly Val Lys Gly Asp Glu Cys Gln Leu Cys
               20                  25                  30
   Glu Val Glu Asn Arg Tyr Gln Gly Asn Pro Leu Arg Gly Thr Cys Tyr
           35                  40                  45
   Tyr
<210> 44
   <211> 39
   <212> PRT
   <213> Homo sapiens
<400> 44
   Leu Tyr Ala Gln Val Ser Gln Ser Lys Pro Cys Glu Arg Thr Gly Ser
   1               5                   10                  15
   Cys Phe Ser Gly Arg Cys Val Asn Ser Thr Cys Leu Cys Asp Pro Gly
               20                  25                  30
   Trp Val Gly Asp Gln Cys Gln
           35
<210> 45
   <211> 39
   <212> PRT
   <213> Homo sapiens
<400> 45
   Ser Ile Asn Ser Cys Pro Asn Asn Cys Ser Gly His Gly Lys Cys Thr
   1               5                   10                  15
   Thr Ser Val Ser Val Pro Ser Gln Val Tyr Cys Glu Cys Asp Lys Tyr
               20                  25                  30
   Trp Lys Gly Glu Ala Cys Asp
           35
<210> 46
   <211> 46
   <212> PRT
   <213> Homo sapiens
<400> 46
   Cys Gln Cys Asn Gly His Ser Thr Cys Ile Asn Asn Asn Val Cys Glu
   1               5                   10                  15
   Gln Cys Lys Asn Leu Thr Thr Gly Lys Gln Cys Gln Asp Cys Met Pro
               20                  25                  30
   Gly Tyr Tyr Gly Asp Pro Thr Asn Gly Gly Gln Cys Thr Ala
           35                  40                  45
<210> 47
   <211> 49
   <212> PRT
   <213> Homo sapiens
<400> 47
   Cys Thr Cys Ser Gly His Ala Asn Ile Cys His Leu His Thr Gly Lys
   1               5                   10                  15
   Cys Phe Cys Thr Thr Lys Gly Ile Lys Gly Asp Gln Cys Gln Leu Cys
               20                  25                  30
   Asp Ser Glu Asn Arg Tyr Val Gly Asn Pro Leu Arg Gly Thr Cys Tyr
           35                  40                  45
   Tyr
<210> 48
   <211> 38
   <212> PRT
   <213> Homo sapiens
<400> 48
   His Cys Pro Thr Cys Arg Asp Arg Pro Cys Gln Asn Gly Gly Gln Cys
   1               5                   10                  15
   His Asp Ser Glu Ser Ser Ser Tyr Val Cys Val Cys Pro Ala Gly Phe
               20                  25                  30
   Thr Gly Ser Arg Cys Glu
      35
<210> 49
   <211> 39
   <212> PRT
   <213> Homo sapiens
<400> 49
   Gln Ala Leu His Cys His Pro Glu Ala Cys Gly Pro Asp Ala Thr Cys
   1               5                   10                  15
   Val Asn Arg Pro Asp Gly Arg Gly Tyr Thr Cys Arg Cys His Leu Gly
               20                  25                  30
   Arg Ser Gly Leu Arg Cys Glu
           35
<210> 50
   <211> 50
   <212> PRT
   <213> Homo sapiens
<400> 50
   Cys Glu Cys Asn Gly His Ser Asp Leu Cys His Pro Glu Thr Gly Ala
   1               5                   10                  15
   Cys Ser Gln Cys Gln His Asn Ala Ala Gly Glu Phe Cys Glu Leu Cys
               20                  25                  30
   Ala Pro Gly Tyr Tyr Gly Asp Ala Thr Ala Gly Thr Pro Glu Asp Cys
           35                  40                  45
   Gln Pro
       50
<210> 51
   <211> 58
   <212> PRT
   <213> Homo sapiens
<400> 51
   Cys Ala Cys Pro Leu Thr Asn Pro Glu Asn Met Phe Ser Arg Thr Cys
   1               5                   10                  15
   Glu Ser Leu Gly Ala Gly Gly Tyr Arg Cys Thr Ala Cys Glu Pro Gly
               20                  25                  30
   Tyr Thr Gly Gln Tyr Cys Glu Gln Cys Gly Pro Gly Tyr Val Gly Asn
           35                  40                  45
   Pro Ser Val Gln Gly Gly Gln Cys Leu Pro
       50                  55
<210> 52
   <211> 50
   <212> PRT
   <213> Homo sapiens
<400> 52
   Cys Asn Cys Asn Gly His Ala Ser Ser Cys Asp Pro Val Tyr Gly His
   1               5                   10                  15
   Cys Leu Asn Cys Gln His Asn Thr Glu Gly Pro Gln Cys Asn Lys Cys
               20                  25                  30
   Lys Ala Gly Phe Phe Gly Asp Ala Met Lys Ala Thr Ala Thr Ser Cys
           35                  40                  45
   Arg Pro
       50
<210> 53
   <211> 58
   <212> PRT
   <213> Homo sapiens
<400> 53
   Cys Pro Cys Pro Tyr Ile Asp Ala Ser Arg Arg Phe Ser Asp Thr Cys
   1               5                   10                  15
   Phe Leu Asp Thr Asp Gly Gln Ala Thr Cys Asp Ala Cys Ala Pro Gly
               20                  25                  30
   Tyr Thr Gly Arg Arg Cys Glu Ser Cys Ala Pro Gly Tyr Glu Gly Asn
           35                  40                  45
   Pro Ile Gln Pro Gly Gly Lys Cys Arg Pro
       50                  55
<210> 54
   <211> 45
   <212> PRT
   <213> Homo sapiens
<400> 54
   Cys Asp Glu Arg Gly Ser Met Gly Thr Ser Gly Glu Ala Cys Arg Cys
   1               5                   10                  15
   Lys Asn Asn Val Val Gly Arg Leu Cys Asn Glu Cys Ala Asp Gly Ser
               20                  25                  30
   Phe His Leu Ser Thr Arg Asn Pro Asp Gly Cys Leu Lys
           35                  40                  45
<210> 55
   <211> 50
   <212> PRT
   <213> Homo sapiens
<400> 55
   Cys Ser Cys His Gly His Ser Glu Ala Cys Glu Pro Glu Thr Gly Ala
   1               5                   10                  15
   Cys Gln Gly Cys Gln His His Thr Glu Gly Pro Arg Cys Glu Gln Cys
               20                  25                  30
   Gln Pro Gly Tyr Tyr Gly Asp Ala Gln Arg Gly Thr Pro Gln Asp Cys
           35                  40                  45
   Gln Leu
       50
<210> 56
   <211> 57
   <212> PRT
   <213> Homo sapiens
<400> 56
   Cys Pro Cys Tyr Gly Asp Pro Ala Ala Gly Gln Ala Ala His Thr Cys
   1               5                   10                  15
   Phe Leu Asp Thr Asp Gly His Pro Thr Cys Asp Ala Cys Ser Pro Gly
               20                  25                  30
   His Ser Gly Arg His Cys Glu Arg Cys Ala Pro Gly Tyr Tyr Gly Asn
           35                  40                  45
   Pro Ser Gln Gly Gln Pro Cys Gln Arg
       50                  55
<210> 57
   <211> 50
   <212> PRT
   <213> Homo sapiens
<400> 57
   Cys Asn Cys Asp Pro Gln Gly Ser Val Ser Ser Gln Cys Asp Ala Ala
   1               5                   10                  15
   Gly Gln Cys Gln Cys Lys Ala Gln Val Glu Gly Leu Thr Cys Ser His
               20                  25                  30
   Cys Arg Pro His His Phe His Leu Ser Ala Ser Asn Pro Asp Gly Cys
           35                  40                  45
   Leu Pro
       50
<210> 58
   <211> 36
   <212> PRT
   <213> Homo sapiens
<400> 58
   Pro Cys Ala Leu Gly Glu Gly Pro Ala Cys Ala His Cys Ala Pro Asp
   1               5                   10                  15
   Asn Ser Thr Arg Cys Gly Ser Cys Asn Pro Gly Tyr Val Leu Ala Gln
               20                  25                  30
   Gly Leu Cys Arg
           35
<210> 59
   <211> 36
   <212> PRT
   <213> Homo sapiens
<400> 59
   Pro Cys Thr Val Gly Asp Ala Ser Ala Cys Leu Thr Cys Ala Pro Asp
   1               5                   10                  15
   Asn Arg Thr Arg Cys Gly Thr Cys Asn Thr Gly Tyr Met Leu Ser Gln
               20                  25                  30
   Gly Leu Cys Lys
           35
<210> 60
   <211> 42
   <212> PRT
   <213> Homo sapiens
<400> 60
   Glu Val Asp Glu Cys Ser Arg Pro Asn Arg Gly Gly Cys Glu Gln Arg
   1               5                   10                  15
   Cys Leu Asn Thr Leu Gly Ser Tyr Lys Cys Ser Cys Asp Pro Gly Tyr
               20                  25                  30
   Glu Leu Ala Pro Asp Lys Arg Arg Cys Glu
           35                  40
<210> 61
   <211> 40
   <212> PRT
   <213> Homo sapiens
<400> 61
   Lys Asp Glu Cys Ser Lys Asp Asn Gly Gly Cys Gln Gln Asp Cys Val
   1               5                   10                  15
   Asn Thr Phe Gly Ser Tyr Glu Cys Gln Cys Arg Ser Gly Phe Val Leu
               20                  25                  30
   His Asp Asn Lys His Asp Cys Lys
           35                  40
<210> 62
   <211> 37
   <212> PRT
   <213> Homo sapiens
<400> 62
   Ala Ser Gly Asp Cys Val Pro Ser Pro Cys His Asn Gly Gly Thr Cys
   1               5                   10                  15
   Leu Glu Glu Glu Glu Gly Val Arg Cys Leu Cys Leu Pro Gly Tyr Gly
               20                  25                  30
   Gly Asp Leu Cys Asp
           35
<210> 63
   <211> 39
   <212> PRT
   <213> Homo sapiens
<400> 63
   Gly Cys Asn Phe Asn Asn Gly Gly Cys His Gln Asp Cys Phe Glu Gly
   1               5                   10                  15
   Gly Asp Gly Ser Phe Leu Cys Gly Cys Arg Pro Gly Phe Arg Leu Leu
               20                  25                  30
   Asp Asp Leu Val Thr Cys Ala
           35
<210> 64
   <211> 40
   <212> PRT
   <213> Homo sapiens
<400> 64
   Pro Cys Ser Ser Ser Pro Cys Arg Gly Gly Ala Thr Cys Ala Leu Gly
   1               5                   10                  15
   Pro His Gly Lys Asn Tyr Thr Cys Arg Cys Pro Gln Gly Tyr Gln Leu
               20                  25                  30
   Asp Ser Ser Gln Leu Asp Cys Val
           35                  40
<210> 65
   <211> 38
   <212> PRT
   <213> Homo sapiens
<400> 65
   Leu Cys Gly Pro Glu Gly Asp Gly Tyr Cys Leu His Gly Asp Cys Ile
   1               5                   10                  15
   His Ala Arg Asp Ile Asp Gly Met Tyr Cys Arg Cys Ser His Gly Tyr
               20                  25                  30
   Thr Gly Ile Arg Cys Gln
           35
<210> 66
   <211> 59
   <212> PRT
   <213> Homo sapiens
<400> 66
   Asp Asp Asn Ile Cys Leu Arg Glu Pro Cys Glu Asn Tyr Met Lys Cys
   1               5                   10                  15
   Val Ser Val Leu Arg Phe Asp Ser Ser Ala Pro Phe Leu Ser Ser Thr
               20                  25                  30
   Thr Val Leu Phe Arg Pro Ile His Pro Ile Asn Gly Leu Arg Cys Arg
           35                  40                  45
   Cys Pro Pro Gly Phe Thr Gly Asp Tyr Cys Glu
       50                  55
<210> 67
   <211> 37
   <212> PRT
   <213> Homo sapiens
<400> 67
   Glu Ile Asp Leu Cys Tyr Ser Asp Pro Cys Gly Ala Asn Gly Arg Cys
   1               5                   10                  15
   Arg Ser Arg Glu Gly Gly Tyr Thr Cys Glu Cys Phe Glu Asp Phe Thr
               20                  25                  30
   Gly Glu His Cys Glu
           35
<210> 68
   <211> 39
   <212> PRT
   <213> Homo sapiens
<400> 68
   Arg Ser Gly Arg Cys Ala Asn Gly Val Cys Lys Asn Gly Gly Thr Cys
   1               5                   10                  15
   Val Asn Leu Leu Ile Gly Gly Phe His Cys Val Cys Pro Pro Gly Glu
               20                  25                  30
   Tyr Glu Arg Pro Tyr Cys Glu
           35
<210> 69
   <211> 37
   <212> PRT
   <213> Homo sapiens
<400> 69
   Arg Arg Asn Phe Cys Asp Gly Arg Arg Cys Gln Asn Gly Gly Thr Cys
   1               5                   10                  15
   Val Asn Arg Trp Asn Met Tyr Leu Cys Glu Cys Pro Leu Arg Phe Gly
               20                  25                  30
   Gly Lys Asn Cys Glu
           35
<210> 70
   <211> 36
   <212> PRT
   <213> Homo sapiens
<400> 70
   Val Asp Asp Pro Cys Thr Ser Ser Pro Cys Pro Pro Asn Ser Arg Cys
   1 5 10 15
   His Asp Ala Trp Glu Asp Tyr Ser Cys Val Cys Asp Lys Gly Tyr Leu
   20 25 30
   Gly Ile Asn Cys
   35
<210> 71
   <211> 39
   <212> PRT
   <213> Homo sapiens
<400> 71
   Val Asp Ala Cys His Leu Asn Pro Cys Glu Asn Met Gly Ala Cys Val
   1 5 10 15
   Arg Ser Pro Gly Ser Pro Gln Gly Tyr Val Cys Glu Cys Gly Pro Ser
   20 25 30
   His Tyr Gly Pro Tyr Cys Glu
   35
<210> 72
   <211> 33
   <212> PRT
   <213> Homo sapiens
<400> 72
   Asn Lys Leu Asp Leu Pro Cys Pro Arg Gly Trp Trp Gly Asn Pro Val
   1 5 10 15
   Cys Gly Pro Cys His Cys Ala Val Ser Lys Gly Phe Asp Pro Asp Cys
   20 25 30
   Asn
<210> 73
   <211> 36
   <212> PRT
   <213> Homo sapiens
<400> 73
   Thr Asn Gly Gln Cys Gln Cys Lys Glu Asn Tyr Tyr Lys Leu Leu Ala
   1 5 10 15
   Gln Asp Thr Cys Leu Pro Cys Asp Cys Phe Pro His Gly Ser His Ser
   20 25 30
   Arg Thr Cys Asp
   35
<210> 74
   <211> 48
   <212> PRT
   <213> Homo sapiens
<400> 74
   Cys Asp Cys Phe Pro His Gly Ser His Ser Arg Thr Cys Asp Met Ala
   1 5 10 15
   Thr Gly Gln Cys Ala Cys Lys Pro Gly Val Ile Gly Arg Gln Cys Asn
   20 25 30
   Arg Cys Asp Asn Pro Phe Ala Glu Val Thr Thr Leu Gly Cys Glu Val
   35 40 45
<210> 75
   <211> 59
   <212> PRT
   <213> Homo sapiens
<400> 75
   Asp Asp Asn Ile Cys Leu Arg Glu Pro Cys Glu Asn Tyr Met Arg Cys
   1 5 10 15
   Val Ser Val Leu Arg Phe Asp Ser Ser Ala Pro Phe Ile Ala Ser Ser
   20 25 30
   Ser Val Leu Phe Arg Pro Ile His Pro Val Gly Gly Leu Arg Cys Arg
   35 40 45
   Cys Pro Pro Gly Phe Thr Gly Asp Tyr Cys Glu
   50 55
<210> 76
   <211> 37
   <212> PRT
   <213> Homo sapiens
<400> 76
   Glu Val Asp Leu Cys Tyr Ser Arg Pro Cys Gly Pro His Gly Arg Cys
   1 5 10 15
   Arg Ser Arg Glu Gly Gly Tyr Thr Cys Leu Cys Arg Asp Gly Tyr Thr
   20 25 30
   Gly Glu His Cys Glu
   35
<210> 77
   <211> 39
   <212> PRT
   <213> Homo sapiens
<400> 77
   Arg Ser Gly Arg Cys Thr Pro Gly Val Cys Lys Asn Gly Gly Thr Cys
   1 5 10 15
   Val Asn Leu Leu Val Gly Gly Phe Lys Cys Asp Cys Pro Ser Gly Asp
   20 25 30
   Phe Glu Lys Pro Tyr Cys Gln
   35
<210> 78
   <211> 37
   <212> PRT
   <213> Homo sapiens
<400> 78
   Lys Lys Asn Val Cys Asp Ser Asn Thr Cys His Asn Gly Gly Thr Cys
   1               5                   10                  15
   Val Asn Gln Trp Asp Ala Phe Ser Cys Glu Cys Pro Leu Gly Phe Gly
               20                  25                  30
   Gly Lys Ser Cys Ala
           35
<210> 79
   <211> 36
   <212> PRT
   <213> Homo sapiens
<400> 79
   Leu Pro Asp Pro Cys Asp Ser Asn Pro Cys Pro Ala Asn Ser Tyr Cys
   1               5                   10                  15
   Ser Asn Asp Trp Asp Ser Tyr Ser Cys Ser Cys Asp Pro Gly Tyr Tyr
               20                  25                  30
   Gly Asp Asn Cys
           35
<210> 80
   <211> 39
   <212> PRT
   <213> Homo sapiens
<400> 80
   Thr Asn Val Cys Asp Leu Asn Pro Cys Glu His Gln Ser Val Cys Thr
   1               5                   10                  15
   Arg Lys Pro Ser Ala Pro His Gly Tyr Thr Cys Glu Cys Pro Pro Asn
               20                  25                  30
   Tyr Leu Gly Pro Tyr Cys Glu
           35
<210> 81
   <211> 40
   <212> PRT
   <213> Homo sapiens
<400> 81
   Thr Cys Gly Pro Cys Asn Cys Asp Val Ser Lys Gly Phe Asp Pro Asp
   1               5                   10                  15
   Cys Asn Lys Thr Ser Gly Glu Cys His Cys Lys Glu Asn His Tyr Arg
               20                  25                  30
   Pro Pro Gly Ser Pro Thr Cys Leu
           35                  40
<210> 82
   <211> 48
   <212> PRT
   <213> Homo sapiens
<400> 82
   Cys Asp Cys Tyr Pro Thr Gly Ser Leu Ser Arg Val Cys Asp Pro Glu
   1 5 10 15
   Asp Gly Gln Cys Pro Cys Lys Pro Gly Val Ile Gly Arg Gln Cys Asp
   20 25 30
   Arg Cys Asp Asn Pro Phe Ala Glu Val Thr Thr Asn Gly Cys Glu Val
   35 40 45
<210> 83
   <211> 59
   <212> PRT
   <213> Homo sapiens
<400> 83
   Asp Asp Asn Val Cys Leu Arg Glu Pro Cys Glu Asn Tyr Met Lys Cys
   1 5 10 15
   Val Ser Val Leu Arg Phe Asp Ser Ser Ala Pro Phe Leu Ala Ser Ala
   20 25 30
   Ser Thr Leu Phe Arg Pro Ile Gln Pro Ile Ala Gly Leu Arg Cys Arg
   35 40 45
   Cys Pro Pro Gly Phe Thr Gly Asp Phe Cys Glu
   50 55
<210> 84
   <211> 40
   <212> PRT
   <213> Homo sapiens
<400> 84
   Glu Ala Gly Arg Cys Val Pro Gly Val Cys Arg Asn Gly Gly Thr Cys
   1 5 10 15
   Thr Asp Ala Pro Asn Gly Gly Phe Arg Cys Gln Cys Pro Ala Gly Gly
   20 25 30
   Ala Phe Glu Gly Pro Arg Cys Glu
   35 40
<210> 85
   <211> 37
   <212> PRT
   <213> Homo sapiens
<400> 85
   Lys Leu His Phe Cys Asp Ser Gly Pro Cys Lys Asn Ser Gly Phe Cys
   1 5 10 15
   Ser Glu Arg Trp Gly Ser Phe Ser Cys Asp Cys Pro Val Gly Phe Gly
   20 25 30
   Gly Lys Asp Cys Gln
   35
<210> 86
   <211> 37
   <212> PRT
   <213> Homo sapiens
<400> 86
   Val Thr Asn Ala Cys Ala Ser Gly Pro Cys Pro Pro His Ala Asp Cys
   1               5                   10                  15
   Arg Asp Leu Trp Gln Thr Phe Ser Cys Thr Cys Gln Pro Gly Tyr Tyr
               20                  25                  30
   Gly Pro Gly Cys Val
           35
<210> 87
   <211> 38
   <212> PRT
   <213> Homo sapiens
<400> 87
   Asp Ala Cys Leu Leu Asn Pro Cys Gln Asn Gln Gly Ser Cys Arg His
   1               5                   10                  15
   Leu Pro Gly Ala Pro His Gly Tyr Thr Cys Asp Cys Val Gly Gly Tyr
               20                  25                  30
   Phe Gly His His Cys Glu
           35
<210> 88
   <211> 33
   <212> PRT
   <213> Homo sapiens
<400> 88
   His Arg Met Asp Gln Gln Cys Pro Arg Gly Trp Trp Gly Ser Pro Thr
   1               5                   10                  15
   Cys Gly Pro Cys Asn Cys Asp Val His Lys Gly Phe Asp Pro Asn Cys
               20                  25                  30
   Asn
<210> 89
   <211> 36
   <212> PRT
   <213> Homo sapiens
<400> 89
   Thr Asn Gly Gln Cys His Cys Lys Glu Phe His Tyr Arg Pro Arg Gly
   1               5                   10                  15
   Ser Asp Ser Cys Leu Pro Cys Asp Cys Tyr Pro Val Gly Ser Thr Ser
               20                  25                  30
   Arg Ser Cys Ala
           35
<210> 90
   <211> 48
   <212> PRT
   <213> Homo sapiens
<400> 90
   Cys Asp Cys Tyr Pro Val Gly Ser Thr Ser Arg Ser Cys Ala Pro His
   1               5                   10                  15
   Ser Gly Gln Cys Pro Cys Arg Pro Gly Ala Leu Gly Arg Gln Cys Asn
               20                  25                  30
   Ser Cys Asp Ser Pro Phe Ala Glu Val Thr Ala Ser Gly Cys Arg Val
           35                  40                  45
<210> 91
   <211> 47
   <212> PRT
   <213> Homo sapiens
<400> 91
   Asp Thr Asn Glu Cys Ile Gln Phe Pro Phe Val Cys Pro Arg Asp Lys
   1               5                   10                  15
   Pro Val Cys Val Asn Thr Tyr Gly Ser Tyr Arg Cys Arg Thr Asn Lys
               20                  25                  30
   Lys Cys Ser Arg Gly Tyr Glu Pro Asn Glu Asp Gly Thr Ala Cys
           35                  40                  45
<210> 92
   <211> 41
   <212> PRT
   <213> Homo sapiens
<400> 92
   Val Ser Asp Leu Cys Ala Thr Gly Asp His Asp Cys Glu Gln Val Cys
   1               5                   10                  15
   Ile Ser Ser Pro Gly Ser Tyr Thr Cys Ala Cys His Glu Gly Phe Thr
               20                  25                  30
   Leu Asn Ser Asp Gly Lys Thr Cys Asn
           35                  40
<210> 93
   <211> 40
   <212> PRT
   <213> Homo sapiens
<400> 93
   Pro Leu Leu His Cys Ala Pro Gly Phe Cys Phe Pro Gly Val Ala Cys
   1               5                   10                  15
   Ile Gln Thr Glu Ser Gly Ala Arg Cys Gly Pro Cys Pro Ala Gly Phe
               20                  25                  30
   Thr Gly Asn Gly Ser His Cys Thr
           35                  40
<210> 94
   <211> 53
   <212> PRT
   <213> Homo sapiens
<400> 94
   Asp Val Asn Glu Cys Asn Ala His Pro Cys Phe Pro Arg Val Arg Cys
   1               5                   10                  15
   Ile Asn Thr Ser Pro Gly Phe Arg Cys Glu Ala Cys Pro Pro Gly Tyr
               20                  25                  30
   Ser Gly Pro Thr His Gln Gly Val Gly Leu Ala Phe Ala Lys Ala Asn
           35                  40                  45
   Lys Gln Val Cys Thr
       50
<210> 95
   <211> 43
   <212> PRT
   <213> Homo sapiens
<400> 95
   Asp Ile Asn Glu Cys Glu Thr Gly Gln His Asn Cys Val Pro Asn Ser
   1               5                   10                  15
   Val Cys Ile Asn Thr Arg Gly Ser Phe Gln Cys Gly Pro Cys Gln Pro
               20                  25                  30
   Gly Phe Val Gly Asp Gln Ala Ser Gly Cys Gln
           35                  40
<210> 96
   <211> 43
   <212> PRT
   <213> Homo sapiens
<400> 96
   Ala Gln Arg Phe Cys Pro Asp Gly Ser Pro Ser Glu Cys His Glu His
   1               5                   10                  15
   Ala Asp Cys Val Leu Glu Arg Asp Gly Ser Arg Ser Cys Val Cys Ala
               20                  25                  30
   Val Gly Trp Ala Gly Asn Gly Ile Leu Cys Gly
           35                  40
<210> 97
   <211> 42
   <212> PRT
   <213> Homo sapiens
<400> 97
   Asp Ser Arg Gly Cys Ala Arg Trp Cys Pro Gln Asn Ser Ser Cys Val
   1               5                   10                  15
   Asn Ala Thr Ala Cys Arg Cys Asn Pro Gly Phe Ser Ser Phe Ser Glu
               20                  25                  30
   Ile Ile Thr Thr Pro Thr Glu Thr Cys Asp
           35                  40
<210> 98
   <211> 52
   <212> PRT
   <213> Homo sapiens
<400> 98
   Asp Ile Asn Glu Cys Ala Thr Pro Ser Lys Val Ser Cys Gly Lys Phe
   1               5                   10                  15
   Ser Asp Cys Trp Asn Thr Glu Gly Ser Tyr Asp Cys Val Cys Ser Pro
               20                  25                  30
   Gly Tyr Glu Pro Val Ser Gly Ala Lys Thr Phe Lys Asn Glu Ser Glu
           35                  40                  45
   Asn Thr Cys Gln
       50
<210> 99
   <211> 44
   <212> PRT
   <213> Homo sapiens
<400> 99
   Asp Val Asp Glu Cys Gln Gln Asn Pro Arg Leu Cys Lys Ser Tyr Gly
   1               5                   10                  15
   Thr Cys Val Asn Thr Leu Gly Ser Tyr Thr Cys Gln Cys Leu Pro Gly
               20                  25                  30
   Phe Lys Phe Ile Pro Glu Asp Pro Lys Val Cys Thr
           35                  40
<210> 100
   <211> 49
   <212> PRT
   <213> Homo sapiens
<400> 100
   Asp Val Asn Glu Cys Thr Ser Gly Gln Asn Pro Cys His Ser Ser Thr
   1               5                   10                  15
   His Cys Leu Asn Asn Val Gly Ser Tyr Gln Cys Arg Cys Arg Pro Gly
               20                  25                  30
   Trp Gln Pro Ile Pro Gly Ser Pro Asn Gly Pro Asn Asn Thr Val Cys
           35                  40                  45
   Glu
<210> 101
   <211> 49
   <212> PRT
   <213> Homo sapiens
<400> 101
   Asp Val Asp Glu Cys Ser Ser Gly Gln His Gln Cys Asp Ser Ser Thr
   1               5                   10                  15
   Val Cys Phe Asn Thr Val Gly Ser Tyr Ser Cys Arg Cys Arg Pro Gly
               20                  25                  30
   Trp Lys Pro Arg His Gly Ile Pro Asn Asn Gln Lys Asp Thr Val Cys
           35                  40                  45
   Glu
<210> 102
   <211> 35
   <212> PRT
   <213> Homo sapiens
<400> 102
   Asp Arg Cys Leu Pro Asn Tyr Cys Glu His Gly Gly Glu Cys Ser Gln
   1 5 10 15
   Ser Trp Ser Thr Phe His Cys Asn Cys Thr Asn Thr Gly Tyr Arg Gly
   20 25 30
   Ala Thr Cys
   35
<210> 103
   <211> 35
   <212> PRT
   <213> Homo sapiens
<400> 103
   Cys Ser Ser Tyr Gly Lys Leu Cys Arg Asn Gly Gly Lys Cys Arg Glu
   1 5 10 15
   Arg Pro Ile Gly Phe Phe Cys Asp Cys Thr Phe Ser Ala Tyr Thr Gly
   20 25 30
   Pro Phe Cys
   35
<210> 104
   <211> 35
   <212> PRT
   <213> Homo sapiens
<400> 104
   Asp Arg Cys Lys Met Asn Pro Cys Leu Asn Gly Gly Thr Cys Tyr Pro
   1 5 10 15
   Thr Glu Thr Ser Tyr Val Cys Thr Cys Val Pro Gly Tyr Ser Gly Asp
   20 25 30
   Gln Cys Glu
   35
<210> 105
   <211> 37
   <212> PRT
   <213> Homo sapiens
<400> 105
   Asp Phe Asp Glu Cys His Ser Asn Pro Cys Arg Asn Gly Ala Thr Cys
   1 5 10 15
   Val Asp Gly Phe Asn Thr Phe Arg Cys Leu Cys Leu Pro Ser Tyr Val
   20 25 30
   Gly Ala Leu Cys Glu
   35
<210> 106
   <211> 43
   <212> PRT
   <213> Homo sapiens
<400> 106
   Arg Ser Val Cys Asp Leu Phe Pro Ser Tyr Cys His Asn Gly Gly Gln
   1               5                   10                  15
   Cys Tyr Leu Val Glu Asn Ile Gly Ala Phe Cys Arg Cys Asn Thr Gln
               20                  25                  30
   Asp Tyr Ile Trp His Lys Gly Met Arg Cys Glu
           35                  40
<210> 107
   <211> 35
   <212> PRT
   <213> Homo sapiens
<400> 107
   Asp Arg Cys Leu Pro Ser Tyr Cys Glu His Gly Gly Glu Cys Ser Gln
   1               5                   10                  15
   Ser Trp Asp Thr Phe Ser Cys Asp Cys Leu Gly Thr Gly Tyr Thr Gly
               20                  25                  30
   Glu Thr Cys
           35
<210> 108
   <211> 38
   <212> PRT
   <213> Homo sapiens
<400> 108
   Val Lys Asp His Cys Ser Lys His Ser Pro Cys Gln Lys Gly Gly Thr
   1               5                   10                  15
   Cys Val Asn Met Pro Ser Gly Pro His Cys Leu Cys Pro Gln His Leu
               20                  25                  30
   Thr Gly Asn His Cys Gln
           35
<210> 109
   <211> 37
   <212> PRT
   <213> Homo sapiens
<400> 109
   Ala Ser Gln Ala Cys Arg Thr Asn Pro Cys Leu His Gly Gly Arg Cys
   1               5                   10                  15
   Leu Glu Val Glu Gly His Arg Leu Cys His Cys Pro Val Gly Tyr Thr
               20                  25                  30
   Gly Ala Phe Cys Asp
           35
<210> 110
   <211> 42
   <212> PRT
   <213> Homo sapiens
<400> 110
   Asp Ile Asp Glu Cys Ala Ser Ser Asn Gly Thr Leu Cys Ala His Ile
   1               5                   10                  15
   Cys Ile Asn Thr Leu Gly Ser Tyr Arg Cys Glu Cys Arg Glu Gly Tyr
               20                  25                  30
   Ile Arg Glu Asp Asp Gly Lys Thr Cys Thr
           35                  40
<210> 111
   <211> 49
   <212> PRT
   <213> Homo sapiens
<400> 111
   Asp Leu Asp Glu Cys Ala Ser Arg Ser Lys Ser Gly Glu Glu Asp Pro
   1               5                   10                  15
   Gln Pro Gln Cys Gln His Leu Cys His Asn Tyr Val Gly Gly Tyr Phe
               20                  25                  30
   Cys Ser Cys Arg Pro Gly Tyr Glu Leu Gln Glu Asp Thr His Ser Cys
           35                  40                  45
   Gln
<210> 112
   <211> 42
   <212> PRT
   <213> Homo sapiens
<400> 112
   Asp Ile Asn Glu Cys Thr Asp Phe Val Asp Val Pro Cys Ser His Phe
   1               5                   10                  15
   Cys Asn Asn Phe Ile Gly Gly Tyr Phe Cys Ser Cys Pro Pro Glu Tyr
               20                  25                  30
   Phe Leu His Asp Asp Met Lys Asn Cys Gly
           35                  40
<210> 113
   <211> 49
   <212> PRT
   <213> Homo sapiens
<400> 113
   Asp Leu Asp Glu Cys Ala Ser Arg Ser Lys Ser Gly Glu Glu Asp Pro
   1               5                   10                  15
   Gln Pro Gln Cys Gln His Leu Cys His Asn Tyr Val Gly Gly Tyr Phe
               20                  25                  30
   Cys Ser Cys Arg Pro Gly Tyr Glu Leu Gln Glu Asp Arg His Ser Cys
           35                  40                  45
   Gln
<210> 114
   <211> 42
   <212> PRT
   <213> Homo sapiens
<400> 114
   Pro Lys Tyr Gly Cys Asn Phe Asn Asn Gly Gly Cys His Gln Asp Cys
   1               5                   10                  15
   Phe Glu Gly Gly Asp Gly Ser Phe Leu Cys Gly Cys Arg Pro Gly Phe
               20                  25                  30
   Arg Leu Leu Asp Asp Leu Val Thr Cys Ala
           35                  40
<210> 115
   <211> 43
   <212> PRT
   <213> Homo sapiens
<400> 115
   Ser Arg Asn Pro Cys Ser Ser Ser Pro Cys Arg Gly Gly Ala Thr Cys
   1               5                   10                  15
   Val Leu Gly Pro His Gly Lys Asn Tyr Thr Cys Arg Cys Pro Gln Gly
               20                  25                  30
   Tyr Gln Leu Asp Ser Ser Gln Leu Asp Cys Val
           35                  40
<210> 116
   <211> 40
   <212> PRT
   <213> Homo sapiens
<400> 116
   Asp Val Asp Glu Cys Gln Asp Ser Pro Cys Ala Gln Glu Cys Val Asn
   1               5                   10                  15
   Thr Pro Gly Gly Phe Arg Cys Glu Cys Trp Val Gly Tyr Glu Pro Gly
               20                  25                  30
   Gly Pro Gly Glu Gly Ala Cys Gln
           35                  40
<210> 117
   <211> 42
   <212> PRT
   <213> Homo sapiens
<400> 117
   Asp Val Asp Glu Cys Ala Leu Gly Arg Ser Pro Cys Ala Gln Gly Cys
   1               5                   10                  15
   Thr Asn Thr Asp Gly Ser Phe His Cys Ser Cys Glu Glu Gly Tyr Val
               20                  25                  30
   Leu Ala Gly Glu Asp Gly Thr Gln Cys Gln
           35                  40
<210> 118
   <211> 42
   <212> PRT
   <213> Homo sapiens
<400> 118
   Asp Val Asp Glu Cys Val Gly Pro Gly Gly Pro Leu Cys Asp Ser Leu
   1               5                   10                  15
   Cys Phe Asn Thr Gln Gly Ser Phe His Cys Gly Cys Leu Pro Gly Trp
               20                  25                  30
   Val Leu Ala Pro Asn Gly Val Ser Cys Thr
           35                  40
<210> 119
   <211> 31
   <212> PRT
   <213> Homo sapiens
<400> 119
   Cys Ala Pro Cys Pro Asn Asn Gly Arg Pro Thr Leu Ser Gly Thr Glu
   1               5                   10                  15
   Cys Leu Cys Val Cys Gln Ser Gly Thr Tyr Gly Glu Asn Cys Glu
               20                  25                  30
<210> 120
   <211> 31
   <212> PRT
   <213> Homo sapiens
<400> 120
   Cys Arg Pro Cys Gln Asn Gly Gly Leu Ala Thr Val Glu Gly Thr His
   1               5                   10                  15
   Cys Leu Cys His Cys Lys Pro Tyr Thr Phe Gly Ala Ala Cys Glu
               20                  25                  30
<210> 121
   <211> 31
   <212> PRT
   <213> Homo sapiens
<400> 121
   Cys Gly Pro Cys Phe Asn Asn Gly Val Pro Ile Leu Glu Gly Thr Ser
   1               5                   10                  15
   Cys Arg Cys Gln Cys Arg Leu Gly Ser Leu Gly Ala Ala Cys Glu
               20                  25                  30
<210> 122
   <211> 31
   <212> PRT
   <213> Homo sapiens
<400> 122
   Cys Ala Pro Cys Gln Gly Asn Gly Val Pro Val Leu Lys Gly Ser Arg
   1               5                   10                  15
   Cys Asp Cys Ile Cys Pro Val Gly Ser Gln Gly Leu Ala Cys Glu
               20                  25                  30
<210> 123
   <211> 31
   <212> PRT
   <213> Homo sapiens
<400> 123
   Cys His Thr Cys Gln Asn Gly Gly Thr Val Ile Leu Met Asp Gly Lys
   1               5                   10                  15
   Cys Leu Cys Ala Cys Pro Phe Lys Phe Glu Gly Ile Ala Cys Glu
               20                  25                  30
<210> 124
   <211> 38
   <212> PRT
   <213> Homo sapiens
<400> 124
   Ile Thr Asp Arg Cys Ser Pro Asn Met Cys Glu His Asp Gly Arg Cys
   1               5                   10                  15
   Tyr Gln Ser Trp Asp Asp Phe Ile Cys Tyr Cys Glu Leu Thr Gly Tyr
                   20                  25                  30
   Lys Gly Glu Thr Cys His
           35
<210> 125
   <211> 40
   <212> PRT
   <213> Homo sapiens
<400> 125
   Cys Thr Gly His Cys Ala His Pro Arg Leu Pro Cys Phe His Gly Gly
   1               5                   10                  15
   Arg Cys Val Glu Arg Tyr Ser Tyr Tyr Thr Cys Asp Cys Asp Leu Thr
               20                  25                  30
   Ala Phe Asp Gly Pro Tyr Cys Asn
           35                  40
<210> 126
   <211> 38
   <212> PRT
   <213> Homo sapiens
<400> 126
   Ile Ile Asp Arg Cys Val Pro Asn His Cys Glu His Gly Gly Lys Cys
   1               5                   10                  15
   Ser Gln Thr Trp Asp Ser Phe Lys Cys Thr Cys Asp Glu Thr Gly Tyr
               20                  25                  30
   Ser Gly Ala Thr Cys His
           35
<210> 127
   <211> 40
   <212> PRT
   <213> Homo sapiens
<400> 127
   Cys Ser Gly His Cys Thr Ser Tyr Gly Thr Asn Cys Glu Asn Gly Gly
   1               5                   10                  15
   Lys Cys Leu Glu Arg Tyr His Gly Tyr Ser Cys Asp Cys Ser Asn Thr
               20                  25                  30
   Ala Tyr Asp Gly Thr Phe Cys Asn
           35                  40
<210> 128
   <211> 33
   <212> PRT
   <213> Homo sapiens
<400> 128
   Cys Leu Pro Ser Tyr Cys Glu His Gly Gly Glu Cys Ser Gln Ser Trp
   1               5                   10                  15
   Asp Thr Phe Ser Cys Asp Cys Leu Gly Thr Gly Tyr Thr Gly Glu Thr
               20                  25                  30
   Cys
<210> 129
   <211> 35
   <212> PRT
   <213> Homo sapiens
<400> 129
   Cys Ser Thr Tyr Gly His Leu Cys Arg Asn Gly Gly Arg Cys Arg Glu
   1               5                   10                  15
   Lys Arg Arg Gly Val Thr Cys Asp Cys Ala Phe Ser Ala Tyr Asp Gly
               20                  25                  30
   Pro Phe Cys
           35
<210> 130
   <211> 38
   <212> PRT
   <213> Homo sapiens
<400> 130
   Ile Thr Asp Arg Cys Leu Pro Ser Tyr Cys Glu His Gly Gly Glu Cys
   1               5                   10                  15
   Ser Gln Ser Trp Asp Thr Phe Ser Cys Asp Cys Leu Gly Thr Gly Tyr
               20                  25                  30
   Thr Gly Glu Thr Cys His
           35
<210> 131
   <211> 39
   <212> PRT
   <213> Homo sapiens
<400> 131
   Ala Gly His Cys Ser Thr Tyr Gly His Leu Cys Arg Asn Gly Gly Arg
   1 5 10 15
   Cys Arg Glu Lys Arg Arg Gly Val Thr Cys Asp Cys Ala Phe Ser Ala
   20 25 30
   Tyr Asp Gly Pro Phe Cys Ser
   35
<210> 132
   <211> 38
   <212> PRT
   <213> Homo sapiens
<400> 132
   Ile Ser Asp Arg Cys Leu Pro Asn Tyr Cys Glu His Gly Gly Glu Cys
   1 5 10 15
   Ser Gln Ser Trp Ser Thr Phe His Cys Asn Cys Thr Asn Thr Gly Tyr
   20 25 30
   Arg Gly Ala Thr Cys His
   35
<210> 133
   <211> 40
   <212> PRT
   <213> Homo sapiens
<400> 133
   Cys Arg Gly His Cys Ser Ser Tyr Gly Lys Leu Cys Arg Asn Gly Gly
   1 5 10 15
   Lys Cys Arg Glu Arg Pro Ile Gly Phe Phe Cys Asp Cys Thr Phe Ser
   20 25 30
   Ala Tyr Thr Gly Pro Phe Cys Ser
   35 40
<210> 134
   <211> 38
   <212> PRT
   <213> Homo sapiens
<400> 134
   Ile Lys Asp Arg Cys Leu Pro Asn Tyr Cys Glu His Gly Gly Ser Cys
   1 5 10 15
   Ser Gln Ser Trp Thr Thr Phe Tyr Cys Asn Cys Ser Asp Thr Ser Tyr
   20 25 30
   Thr Gly Ala Thr Cys His
   35
<210> 135
   <211> 39
   <212> PRT
   <213> Homo sapiens
<400> 135
   Pro Gly His Cys Ser Ser Tyr Gly Ser Ile Cys His Asn Gly Gly Lys
   1               5                   10                  15
   Cys Val Glu Lys His Asn Gly Tyr Leu Cys Asp Cys Thr Asn Ser Pro
               20                  25                  30
   Tyr Glu Gly Pro Phe Cys Lys
           35
<210> 136
   <211> 41
   <212> PRT
   <213> Homo sapiens
<400> 136
   Ala Ile Arg Leu Cys Gly Pro Glu Gly Asp Gly Tyr Cys Leu His Gly
   1               5                   10                  15
   Asp Cys Ile His Ala Arg Asp Ile Asp Gly Met Tyr Cys Arg Cys Tyr
               20                  25                  30
   His Gly Tyr Ile Gly Ile Arg Cys Gln
           35                  40
<210> 137
   <211> 32
   <212> PRT
   <213> Homo sapiens
<400> 137
   Asn Pro Cys Gln Gly Ser Ala Thr Cys Val Asn Thr Pro Gly Glu Arg
   1               5                   10                  15
   Ser Phe Leu Cys Lys Cys Pro Pro Gly Tyr Ser Gly Thr Ile Cys Glu
               20                  25                  30
<210> 138
   <211> 29
   <212> PRT
   <213> Homo sapiens
<400> 138
   Pro Cys His Asn Asn Ala Thr Cys Glu Asp Ser Val Asp Asn Tyr Thr
   1               5                   10                  15
   Cys His Cys Trp Pro Gly Tyr Thr Gly Ala Gln Cys Glu
               20                  25
<210> 139
   <211> 25
   <212> PRT
   <213> Homo sapiens
<400> 139
   Cys Arg Asn Gly Gly Thr Cys Val Leu Gly Ser Phe Cys Val Cys Pro
   1               5                   10                  15
   Ala His Phe Thr Gly Arg Tyr Cys Glu
               20                  25
<210> 140
   <211> 30
   <212> PRT
   <213> Homo sapiens
<400> 140
   Ala Arg Pro Arg Cys Cys Arg Asn Gly Gly Thr Cys Val Leu Gly Ser
   1               5                   10                  15
   Phe Cys Val Cys Pro Ala His Phe Thr Gly Arg Tyr Cys Glu
               20                  25                  30
<210> 141
   <211> 35
   <212> PRT
   <213> Homo sapiens
<400> 141
   Asp Pro Cys Glu Asn Asn Pro Cys Leu His Gly Gly Thr Cys Asn Ala
   1               5                   10                  15
   Asn Gly Thr Met Tyr Gly Cys Ser Cys Asp Gln Gly Phe Ala Gly Glu
               20                  25                  30
   Asn Cys Glu
           35
<210> 142
   <211> 43
   <212> PRT
   <213> Homo sapiens
<400> 142
   Pro Cys Pro Gly Arg Tyr Leu Arg Ala Gly Lys Cys Ala Glu Leu Pro
   1               5                   10                  15
   Asn Cys Leu Asp Asp Leu Gly Gly Phe Ala Cys Glu Cys Ala Thr Gly
               20                  25                  30
   Phe Glu Leu Gly Lys Asp Gly Arg Ser Cys Val
           35                  40
<210> 143
   <211> 34
   <212> PRT
   <213> Homo sapiens
<400> 143
   Pro Arg Asn Pro Cys Arg Met Ser Cys Gln Asn His Gly Arg Leu Asn
   1               5                   10                  15
   Ile Ser Thr Cys His Cys His Cys Pro Pro Gly Tyr Thr Gly Arg Tyr
               20                  25                  30
   Cys Gln
<210> 144
   <211> 37
   <212> PRT
   <213> Homo sapiens
<400> 144
   Asp Lys Lys Val Cys Ser Ser Asn Pro Cys Gln Asn Gly Gly Thr Cys
   1               5                   10                  15
   Leu Asn Leu His Asp Ser Phe Phe Cys Ile Cys Pro Pro Gln Trp Lys
                    20                  25                  30
   Gly Pro Leu Cys Ser
           35
<210> 145
   <211> 42
   <212> PRT
   <213> Homo sapiens
<400> 145
   Asp Val Asn Glu Cys Glu Ile Tyr Ser Gly Thr Pro Leu Ser Cys Gln
   1               5                   10                  15
   Asn Gly Gly Thr Cys Val Asn Thr Met Gly Ser Tyr Ser Cys His Cys
               20                  25                  30
   Pro Pro Glu Thr Tyr Gly Pro Gln Cys Ala
           35                  40
<210> 146
   <211> 42
   <212> PRT
   <213> Homo sapiens
<400> 146
   Asp Arg Asp Glu Cys Ser Phe Gln Pro Gly Pro Cys Ser Thr Leu Val
   1               5                   10                  15
   Gln Cys Phe Asn Thr Gln Gly Ser Phe Tyr Cys Gly Ala Cys Pro Thr
               20                  25                  30
   Gly Trp Gln Gly Asn Gly Tyr Ile Cys Glu
           35                  40
<210> 147
   <211> 44
   <212> PRT
   <213> Homo sapiens
<400> 147
   Asp Ile Asn Glu Cys Glu Ile Asn Asn Gly Gly Cys Ser Val Ala Pro
   1               5                   10                  15
   Pro Val Glu Cys Val Asn Thr Pro Gly Ser Ser His Cys Gln Ala Cys
               20                  25                  30
   Pro Pro Gly Tyr Gln Gly Asp Gly Arg Val Cys Thr
           35                  40
<210> 148
   <211> 37
   <212> PRT
   <213> Homo sapiens
<400> 148
   Leu Thr Asp Ile Cys Ser Val Ser Asn Gly Gly Cys His Pro Asp Ala
   1               5                   10                  15
   Ser Cys Ser Ser Thr Leu Gly Ser Leu Pro Leu Cys Thr Cys Leu Pro
               20                  25                  30
   Gly Tyr Thr Gly Asn
           35
<210> 149
   <211> 36
   <212> PRT
   <213> Homo sapiens
<400> 149
   Leu Ser Asn Ile Cys Leu Ser His Pro Cys Leu Asn Gly Gln Cys Ile
   1               5                   10                  15
   Asp Thr Val Ser Gly Tyr Phe Cys Lys Cys Asp Ser Gly Trp Thr Gly
               20                  25                  30
   Val Asn Cys Thr
           35
<210> 150
   <211> 37
   <212> PRT
   <213> Homo sapiens
<400> 150
   Asn Ile Asn Glu Cys Leu Ser Asn Pro Cys Leu Asn Gly Gly Thr Cys
   1               5                   10                  15
   Val Asp Gly Val Asp Ser Phe Ser Cys Glu Cys Thr Arg Leu Trp Thr
               20                  25                  30
   Gly Ala Leu Cys Gln
           35
<210> 151
   <211> 41
   <212> PRT
   <213> Homo sapiens
<400> 151
   Gly Cys Lys Tyr Leu Ser Asp Ile Asn Ile Thr Arg Tyr Asn Ile Asn
   1               5                   10                  15
   Cys Arg Glu Asn Arg Ala Cys Asn Glu Leu Phe His Cys Gln Cys Asp
               20                  25                  30
   Pro Gly Tyr Ala Pro Pro Asp Cys Glu
           35                  40
<210> 152
   <211> 41
   <212> PRT
   <213> Homo sapiens
<400> 152
   Leu Pro Cys Pro Gly Gly Thr Glu Arg Pro Cys Gly Gly Tyr Gly Gln
   1               5                   10                  15
   Cys Glu Gly Glu Gly Thr Arg Gly Gly Ser Gly His Cys Asp Cys Gln
               20                  25                  30
   Ala Gly Tyr Gly Gly Glu Ala Cys Gly
           35                  40
<210> 153
   <211> 43
   <212> PRT
   <213> Homo sapiens
<400> 153
   Asp Cys Leu Ala Cys Gln Gly Gly Ser Gln Arg Pro Cys Ser Gly Asn
   1               5                   10                  15
   Gly His Cys Ser Gly Asp Gly Ser Arg Gln Gly Asp Gly Ser Cys Arg
               20                  25                  30
   Cys His Met Gly Tyr Gln Gly Pro Leu Cys Thr
           35                  40
<210> 154
   <211> 42
   <212> PRT
   <213> Homo sapiens
<400> 154
   Asp Val Asp Glu Cys Ser Leu Ala Glu Lys Thr Cys Val Arg Lys Asn
   1               5                   10                  15
   Glu Asn Cys Tyr Asn Thr Pro Gly Ser Tyr Val Cys Val Cys Pro Asp
               20                  25                  30
   Gly Phe Glu Glu Thr Glu Asp Ala Cys Val
           35                  40
<210> 155
   <211> 49
   <212> PRT
   <213> Homo sapiens
<400> 155
   Ala Pro Gly Pro Cys Ala Ala Gln Pro Cys Arg Asn Gly Gly Val Cys
   1               5                   10                  15
   Thr Ser Arg Pro Glu Pro Asp Pro Gln His Pro Ala Pro Ala Gly Glu
               20                  25                  30
   Pro Gly Tyr Ser Cys Thr Cys Pro Ala Gly Ile Ser Gly Ala Asn Cys
           35                  40                  45
   Gln
<210> 156
   <211> 37
   <212> PRT
   <213> Homo sapiens
<400> 156
   Asp Ile Asn Glu Cys Asp Ser Asn Pro Cys His His Gly Gly Ser Cys
   1               5                   10                  15
   Leu Asp Gln Pro Asn Gly Tyr Asn Cys His Cys Pro His Gly Trp Val
               20                  25                  30
   Gly Ala Asn Cys Glu
           35
<210> 157
   <211> 40
   <212> PRT
   <213> Homo sapiens
<400> 157
   Val Ala Asp Pro Cys Ala Ser Asn Pro Cys His His Gly Asn Cys Ser
   1               5                   10                  15
   Ser Ser Ser Ser Ser Ser Ser Asp Gly Tyr Leu Cys Ile Cys Asn Glu
               20                  25                  30
   Gly Tyr Glu Gly Pro Asn Cys Glu
           35                  40
<210> 158
   <211> 40
   <212> PRT
   <213> Homo sapiens
<400> 158
   Pro Gly Glu Ser His Ala Asn Asp Leu Glu Cys Ser Gly Lys Gly Lys
   1               5                   10                  15
   Cys Thr Thr Lys Pro Ser Glu Ala Thr Phe Ser Cys Thr Cys Glu Glu
               20                  25                  30
   Gln Tyr Val Gly Thr Phe Cys Glu
           35                  40
<210> 159
   <211> 42
   <212> PRT
   <213> Homo sapiens
<400> 159
   Glu Tyr Asp Ala Cys Gln Arg Lys Pro Cys Gln Asn Asn Ala Ser Cys
   1               5                   10                  15
   Ile Asp Ala Asn Glu Lys Gln Asp Gly Ser Asn Phe Thr Cys Val Cys
               20                  25                  30
   Leu Pro Gly Tyr Thr Gly Glu Leu Cys Gln
           35                  40
<210> 160
   <211> 37
   <212> PRT
   <213> Homo sapiens
<400> 160
   Lys Ile Asp Tyr Cys Ile Leu Asp Pro Cys Arg Asn Gly Ala Thr Cys
   1 5 10 15
   Ile Ser Ser Leu Ser Gly Phe Thr Cys Gln Cys Pro Glu Gly Tyr Phe
   20 25 30
   Gly Ser Ala Cys Glu
   35
<210> 161
   <211> 37
   <212> PRT
   <213> Homo sapiens
<400> 161
   Lys Val Asp Pro Cys Ala Ser Ser Pro Cys Gln Asn Asn Gly Thr Cys
   1 5 10 15
   Tyr Val Asp Gly Val His Phe Thr Cys Asn Cys Ser Pro Gly Phe Thr
   20 25 30
   Gly Pro Thr Cys Ala
   35
<210> 162
   <211> 36
   <212> PRT
   <213> Homo sapiens
<400> 162
   Leu Ile Asp Phe Cys Ala Leu Ser Pro Cys Ala His Gly Thr Cys Arg
   1 5 10 15
   Ser Val Gly Thr Ser Tyr Lys Cys Leu Cys Asp Pro Gly Tyr His Gly
   20 25 30
   Leu Tyr Cys Glu
   35
<210> 163
   <211> 37
   <212> PRT
   <213> Homo sapiens
<400> 163
   Glu Tyr Asn Glu Cys Leu Ser Ala Pro Cys Leu Asn Ala Ala Thr Cys
   1 5 10 15
   Arg Asp Leu Val Asn Gly Tyr Glu Cys Val Cys Leu Ala Glu Tyr Lys
   20 25 30
   Gly Thr His Cys Glu
   35
<210> 164
   <211> 37
   <212> PRT
   <213> Homo sapiens
<400> 164
   Tyr Lys Asp Pro Cys Ala Asn Val Ser Cys Leu Asn Gly Ala Thr Cys
   1 5 10 15
   Asp Ser Asp Gly Leu Asn Gly Thr Cys Ile Cys Ala Pro Gly Phe Thr
   20 25 30
   Gly Glu Glu Cys Asp
   35
<210> 165
   <211> 38
   <212> PRT
   <213> Homo sapiens
<400> 165
   Asp Val Arg Ala Cys Ser Ser Ala Pro Cys Ala Asn Asn Gly Thr Cys
   1 5 10 15
   Val Ser Leu Asp Asp Gly Leu Tyr Glu Cys Ser Cys Ala Pro Gly Tyr
   20 25 30
   Ser Gly Lys Asp Cys Gln
   35
<210> 166
   <211> 35
   <212> PRT
   <213> Homo sapiens
<400> 166
   Asn Gly Ser Pro Cys Gln His Gly Gly Thr Cys Val Asp Asp Glu Gly
   1 5 10 15
   Arg Ala Ser His Ala Ser Cys Leu Cys Pro Pro Gly Phe Ser Gly Asn
   20 25 30
   Phe Cys Glu
   35
<210> 167
   <211> 31
   <212> PRT
   <213> Homo sapiens
<400> 167
   Thr Pro Asn Pro Cys Glu Asn Asp Gly Val Cys Thr Asp Ile Gly Gly
   1 5 10 15
   Asp Phe Arg Cys Arg Cys Pro Ala Gly Phe Ile Asp Lys Thr Cys
   20 25 30
<210> 168
   <211> 29
   <212> PRT
   <213> Homo sapiens
<400> 168
   Cys Leu Pro Gly Cys Asp Glu Gln His Gly Phe Cys Asp Lys Pro Gly
   1 5 10 15
   Glu Cys Lys Cys Arg Val Gly Trp Gln Gly Arg Tyr Cys
   20 25
<210> 169
   <211> 29
   <212> PRT
   <213> Homo sapiens
<400> 169
   Cys Ile Arg Tyr Pro Gly Cys Leu His Gly Thr Cys Gln Gln Pro Trp
   1               5                   10                  15
   Gln Cys Asn Cys Gln Glu Gly Trp Gly Gly Leu Phe Cys
               20                  25
<210> 170
   <211> 34
   <212> PRT
   <213> Homo sapiens
<400> 170
   Cys Thr His His Lys Pro Cys Lys Asn Gly Ala Thr Cys Thr Asn Thr
   1               5                   10                  15
   Gly Gln Gly Ser Tyr Thr Cys Ser Cys Arg Pro Gly Tyr Thr Gly Ala
               20                  25                  30
   Thr Cys
<210> 171
   <211> 32
   <212> PRT
   <213> Homo sapiens
<400> 171
   Cys Asp Pro Ser Pro Cys Lys Asn Gly Gly Ser Cys Thr Asp Leu Glu
   1               5                   10                  15
   Asn Ser Tyr Ser Cys Thr Cys Pro Pro Gly Phe Tyr Gly Lys Ile Cys
               20                  25                  30
<210> 172
   <211> 33
   <212> PRT
   <213> Homo sapiens
<400> 172
   Cys Ala Asp Gly Pro Cys Phe Asn Gly Gly Arg Cys Ser Asp Ser Pro
   1               5                   10                  15
   Asp Gly Gly Tyr Ser Cys Arg Cys Pro Val Gly Tyr Ser Gly Phe Asn
               20                  25                  30
   Cys
<210> 173
   <211> 32
   <212> PRT
   <213> Homo sapiens
<400> 173
   Cys Ser Ser Ser Pro Cys Ser Asn Gly Ala Lys Cys Val Asp Leu Gly
   1               5                   10                  15
   Asp Ala Tyr Leu Cys Arg Cys Gln Ala Gly Phe Ser Gly Arg His Cys
               20                  25                  30
<210> 174
   <211> 32
   <212> PRT
   <213> Homo sapiens
<400> 174
   Cys Ala Ser Ser Pro Cys Ala Asn Gly Gly Thr Cys Arg Asp Gly Val
   1               5                   10                  15
   Asn Asp Phe Ser Cys Thr Cys Pro Pro Gly Tyr Thr Gly Arg Asn Cys
               20                  25                  30
<210> 175
   <211> 32
   <212> PRT
   <213> Homo sapiens
<400> 175
   Cys Glu His Ala Pro Cys His Asn Gly Ala Thr Cys His Glu Arg Gly
   1               5                   10                  15
   His Arg Tyr Val Cys Glu Cys Ala Arg Gly Tyr Gly Gly Pro Asn Cys
               20                  25                  30
<210> 176
   <211> 34
   <212> PRT
   <213> Homo sapiens
<400> 176
   Ala Pro Leu Val Cys Arg Ala Gly Cys Ser Pro Glu His Gly Phe Cys
   1               5                   10                  15
   Glu Gln Pro Gly Glu Cys Arg Cys Leu Glu Gly Trp Thr Gly Pro Leu
               20                  25                  30
   Cys Thr
<210> 177
   <211> 37
   <212> PRT
   <213> Homo sapiens
<400> 177
   Gly Pro Gly Pro Cys Asp Gly Asn Pro Cys Ala Asn Gly Gly Ser Cys
   1               5                   10                  15
   Ser Glu Thr Pro Arg Ser Phe Glu Cys Thr Cys Pro Arg Gly Phe Tyr
               20                  25                  30
   Gly Leu Arg Cys Glu
           35
<210> 178
   <211> 40
   <212> PRT
   <213> Homo sapiens
<400> 178
   Ser Gly Val Thr Cys Ala Asp Gly Pro Cys Phe Asn Gly Gly Leu Cys
   1               5                   10                  15
   Val Gly Gly Ala Asp Pro Asp Ser Ala Tyr Ile Cys His Cys Pro Pro
               20                  25                  30
   Gly Phe Gln Gly Ser Asn Cys Glu
           35                  40
<210> 179
   <211> 37
   <212> PRT
   <213> Homo sapiens
<400> 179
   Arg Val Asp Arg Cys Ser Leu Gln Pro Cys Arg Asn Gly Gly Leu Cys
   1               5                   10                  15
   Leu Asp Leu Gly His Ala Leu Arg Cys Arg Cys Arg Ala Gly Phe Ala
               20                  25                  30
   Gly Pro Arg Cys Glu
           35
<210> 180
   <211> 37
   <212> PRT
   <213> Homo sapiens
<400> 180
   Asp Leu Asp Asp Cys Ala Gly Arg Ala Cys Ala Asn Gly Gly Thr Cys
   1               5                   10                  15
   Val Glu Gly Gly Gly Ala His Arg Cys Ser Cys Ala Leu Gly Phe Gly
               20                  25                  30
   Gly Arg Asp Cys Arg
           35
<210> 181
   <211> 37
   <212> PRT
   <213> Homo sapiens
<400> 181
   Arg Ala Asp Pro Cys Ala Ala Arg Pro Cys Ala His Gly Gly Arg Cys
   1               5                   10                  15
   Tyr Ala His Phe Ser Gly Leu Val Cys Ala Cys Ala Pro Gly Tyr Met
               20                  25                  30
   Gly Ala Arg Cys Glu
           35
<210> 182
   <211> 34
   <212> PRT
   <213> Homo sapiens
<400> 182
   Gln Gln Pro Ile Cys Leu Ser Gly Cys His Glu Gln Asn Gly Tyr Cys
   1               5                   10                  15
   Ser Lys Pro Ala Glu Cys Leu Cys Arg Pro Gly Trp Gln Gly Arg Leu
               20                  25                  30
   Cys Asn
<210> 183
   <211> 31
   <212> PRT
   <213> Homo sapiens
<400> 183
   Glu Cys Ile Pro His Asn Gly Cys Arg His Gly Thr Cys Ser Thr Pro
   1               5                   10                  15
   Trp Gln Cys Thr Cys Asp Glu Gly Trp Gly Gly Leu Phe Cys Asp
               20                  25                  30
<210> 184
   <211> 39
   <212> PRT
   <213> Homo sapiens
<400> 184
   Asp Leu Asn Tyr Cys Thr His His Ser Pro Cys Lys Asn Gly Ala Thr
   1               5                   10                  15
   Cys Ser Asn Ser Gly Gln Arg Ser Tyr Thr Cys Thr Cys Arg Pro Gly
               20                  25                  30
   Tyr Thr Gly Val Asp Cys Glu
           35
<210> 185
   <211> 37
   <212> PRT
   <213> Homo sapiens
<400> 185
   Glu Leu Ser Glu Cys Asp Ser Asn Pro Cys Arg Asn Gly Gly Ser Cys
   1               5                   10                  15
   Lys Asp Gln Glu Asp Gly Tyr His Cys Leu Cys Pro Pro Gly Tyr Tyr
               20                  25                  30
   Gly Leu His Cys Glu
           35
<210> 186
   <211> 39
   <212> PRT
   <213> Homo sapiens
<400> 186
   Ser Thr Leu Ser Cys Ala Asp Ser Pro Cys Phe Asn Gly Gly Ser Cys
   1               5                   10                  15
   Arg Glu Arg Asn Gln Gly Ala Asn Tyr Ala Cys Glu Cys Pro Pro Asn
               20                  25                  30
   Phe Thr Gly Ser Asn Cys Glu
           35
<210> 187
   <211> 37
   <212> PRT
   <213> Homo sapiens
<400> 187
   Lys Val Asp Arg Cys Thr Ser Asn Pro Cys Ala Asn Gly Gly Gln Cys
   1               5                   10                  15
   Leu Asn Arg Gly Pro Ser Arg Met Cys Arg Cys Arg Pro Gly Phe Thr
               20                  25                  30
   Gly Thr Tyr Cys Glu
           35
<210> 188
   <211> 37
   <212> PRT
   <213> Homo sapiens
<400> 188
   His Val Ser Asp Cys Ala Arg Asn Pro Cys Ala His Gly Gly Thr Cys
   1               5                   10                  15
   His Asp Leu Glu Asn Gly Leu Met Cys Thr Cys Pro Ala Gly Phe Ser
               20                  25                  30
   Gly Arg Arg Cys Glu
           35
<210> 189
   <211> 39
   <212> PRT
   <213> Homo sapiens
<400> 189
   Ser Ile Asp Ala Cys Ala Ser Ser Pro Cys Phe Asn Arg Ala Thr Cys
   1               5                   10                  15
   Tyr Thr Asp Leu Ser Thr Asp Thr Phe Val Cys Asn Cys Pro Tyr Gly
               20                  25                  30
   Phe Val Gly Ser Arg Cys Glu
           35
<210> 190
   <211> 33
   <212> PRT
   <213> Homo sapiens
<400> 190
   Glu Cys Pro Leu Asn Tyr Cys Arg Asn Gly Gly Thr Cys Val Val Glu
   1               5                   10                  15
   Lys Asn Gly Pro Met Cys Arg Cys Arg Gln Gly Trp Lys Gly Asn Arg
               20                  25                  30
   Cys
<210> 191
   <211> 38
   <212> PRT
   <213> Homo sapiens
<400> 191
   Pro Cys Leu Arg Lys Tyr Lys Asp Phe Cys Ile His Gly Glu Cys Lys
   1               5                   10                  15
   Tyr Val Lys Glu Leu Arg Ala Pro Ser Cys Ile Cys His Pro Gly Tyr
               20                  25                  30
   His Gly Glu Arg Cys His
           35
<210> 192
   <211> 33
   <212> PRT
   <213> Homo sapiens
<400> 192
   Cys Pro Gly Ser Gly Glu Arg Arg Ile Cys Ser His His Gly Val Cys
   1               5                   10                  15
   Ser Asn Glu Gly Lys Cys Ile Cys Gln Pro Asp Trp Thr Gly Lys Asp
               20                  25                  30
   Cys
<210> 193
   <211> 33
   <212> PRT
   <213> Homo sapiens
<400> 193
   Gly Val His Glu Cys Ala Met Gln Cys His Gly Arg Gly Val Cys Asn
   1               5                   10                  15
   Asn Arg Lys Asn Cys His Cys Glu Ala His Trp Ala Pro Pro Phe Cys
               20                  25                  30
   Asp
<210> 194
   <211> 29
   <212> PRT
   <213> Homo sapiens
<400> 194
   Cys Arg Ser Lys Cys His Gly His Gly Val Cys Asp Ser Asn Arg His
   1               5                   10                  15
   Cys Tyr Cys Glu Glu Gly Trp Ala Pro Pro Asp Cys Thr
               20                  25
<210> 195
   <211> 35
   <212> PRT
   <213> Homo sapiens
<400> 195
   Met Gly Tyr Asn Cys Asn Ala Thr Thr Lys Cys Lys Gly Lys Gly Ile
   1               5                   10                  15
   Cys Asn Asn Phe Gly Asn Cys Gln Cys Phe Pro Gly His Arg Pro Pro
               20                  25                  30
   Asp Cys Lys
           35
<210> 196
   <211> 33
   <212> PRT
   <213> Homo sapiens
<400> 196
   Glu Thr Glu Gly Cys Gly Lys Lys Cys Asn Gly His Gly Val Cys Asn
   1               5                   10                  15
   Asn Asn Gln Asn Cys His Cys Leu Pro Gly Trp Ala Pro Pro Phe Cys
               20                  25                  30
   Asn
<210> 197
   <211> 34
   <212> PRT
   <213> Homo sapiens
<400> 197
   Leu Gly Tyr Asp Cys Thr Thr Asp Lys Cys Asn Asp Arg Gly Val Cys
   1               5                   10                  15
   Asn Asn Lys Lys His Cys His Cys Ser Ala Ser Tyr Leu Pro Pro Asp
               20                  25                  30
   Cys Ser
<210> 198
   <211> 29
   <212> PRT
   <213> Homo sapiens
<400> 198
   Cys Gln Pro Lys Thr Cys Asn Met Arg Gly Ile Cys Asn Asn Lys Gln
   1               5                   10                  15
   His Cys His Cys Asn His Glu Trp Ala Pro Pro Tyr Cys
               20                  25
<210> 199
   <211> 30
   <212> PRT
   <213> Homo sapiens
<400> 199
   Cys Leu Pro Glu Thr Cys Asn Met Lys Gly Ile Cys Asn Asn Lys His
   1 5 10 15
   His Cys His Cys Gly Tyr Gly Trp Ser Pro Pro Tyr Cys Gln
   20 25 30
<210> 200
   <211> 38
   <212> PRT
   <213> Homo sapiens
<400> 200
   Asn Phe Ser Thr Cys Leu Ser Ser Lys Glu Gly Thr Ile Cys Ser Gly
   1 5 10 15
   Asn Gly Val Cys Ser Asn Glu Leu Lys Cys Val Cys Asn Arg His Trp
   20 25 30
   Ile Gly Ser Asp Cys Asn
   35
<210> 201
   <211> 38
   <212> PRT
   <213> Homo sapiens
<400> 201
   Asn Met Ser Ser Cys Pro Leu Asp Ser Lys Gly Lys Val Cys Ser Gly
   1 5 10 15
   His Gly Val Cys Ser Asn Glu Ala Thr Cys Ile Cys Asp Phe Thr Trp
   20 25 30
   Ala Gly Thr Asp Cys Ser
   35
<210> 202
   <211> 33
   <212> PRT
   <213> Homo sapiens
<400> 202
   Lys Ser Thr Asn Cys Ser Ser Lys Cys Lys Gly His Ala Val Cys Asp
   1 5 10 15
   His Glu Leu Gln Cys Gln Cys Glu Glu Gly Trp Ile Pro Pro Asp Cys
   20 25 30
   Asp
<210> 203
   <211> 30
   <212> PRT
   <213> Homo sapiens
<400> 203
   Cys Ser Pro Ala Phe Cys Asn Lys Arg Gly Ile Cys Asn Asn Lys His
   1 5 10 15
   His Cys His Cys Asn Tyr Leu Trp Asp Pro Pro Asn Cys Leu
   20 25 30
<210> 204
   <211> 35
   <212> PRT
   <213> Homo sapiens
<400> 204
   Leu Gln Phe Asp Cys Leu Pro Glu Lys Cys Asn Thr Arg Gly Val Cys
   1               5                   10                  15
   Asn Asn Arg Lys Asn Cys His Cys Met Tyr Gly Trp Ala Pro Pro Phe
               20                  25                  30
   Cys Glu Glu
           35
<210> 205
   <211> 33
   <212> PRT
   <213> Homo sapiens
<400> 205
   Ser Ala His Val Cys Ser Gln Gln Cys Ser Gly His Gly Val Cys Asp
   1               5                   10                  15
   Ser Arg Asn Lys Cys His Cys Ser Pro Gly Tyr Lys Pro Pro Asn Cys
               20                  25                  30
   Gln
<210> 206
   <211> 33
   <212> PRT
   <213> Homo sapiens
<400> 206
   Glu Leu Gln Arg Cys Leu Thr Ala Cys His Ser His Gly Val Cys Asn
   1               5                   10                  15
   Ser Asn His Asn Cys His Cys Ala Pro Gly Trp Ala Pro Pro Phe Cys
               20                  25                  30
   Asp
<210> 207
   <211> 33
   <212> PRT
   <213> Homo sapiens
<400> 207
   Arg Ser Ser Asn Cys Ser Ala Gln Cys His Asn His Gly Val Cys Asn
   1               5                   10                  15
   His Lys Gln Glu Cys His Cys His Ala Gly Trp Ala Pro Pro His Cys
               20                  25                  30
   Ala
<210> 208
   <211> 55
   <212> PRT
   <213> Homo sapiens
<400> 208
   Cys Asp Val Gln Lys Lys Cys His Gly His Gly Val Cys Asn Ser Asn
   1               5                   10                  15
   Lys Asn Cys His Cys Glu Asn Gly Trp Ala Pro Pro Asn Cys Glu Thr
               20                  25                  30
   Lys Gly Tyr Gly Gly Ser Val Asp Ser Gly Pro Thr Tyr Asn Glu Met
           35                  40                  45
   Asn Thr Ala Leu Arg Asp Gly
       50                  55
<210> 209
   <211> 34
   <212> PRT
   <213> Homo sapiens
<400> 209
   Asp Val Asp Glu Cys Ala Gln Ala Leu His Asp Cys Arg Pro Ser Gln
   1               5                   10                  15
   Asp Cys His Asn Leu Pro Gly Ser Tyr Gln Cys Thr Cys Pro Asp Gly
               20                  25                  30
   Tyr Arg
<210> 210
   <211> 31
   <212> PRT
   <213> Homo sapiens
<400> 210
   Asp Ile Asp Glu Cys Arg Tyr Arg Tyr Cys Gln His Arg Cys Val Asn
   1               5                   10                  15
   Leu Pro Gly Ser Phe Arg Cys Gln Cys Glu Pro Gly Phe Gln Leu
               20                  25                  30
<210> 211
   <211> 32
   <212> PRT
   <213> Homo sapiens
<400> 211
   Asp Ile Asp Glu Cys Ser Tyr Ser Ser Tyr Leu Cys Gln Tyr Arg Cys
   1               5                   10                  15
   Val Asn Glu Pro Gly Arg Phe Ser Cys His Cys Pro Gln Gly Tyr Gln
               20                  25                  30
<210> 212
   <211> 28
   <212> PRT
   <213> Homo sapiens
<400> 212
   Asp Ile Asp Glu Cys Glu Ser Gly Ala His Gln Cys Ser Glu Ala Gln
   1               5                   10                  15
   Thr Cys Val Asn Phe His Gly Gly Tyr Arg Cys Val
               20                  25
<210> 213
   <211> 38
   <212> PRT
   <213> Homo sapiens
<400> 213
   Gln Asn Cys Thr Lys Thr Pro Cys Leu Pro Asn Ala Lys Cys Glu Ile
   1               5                   10                  15
   Arg Asn Gly Ile Glu Ala Cys Tyr Cys Asn Met Gly Phe Ser Gly Asn
               20                  25                  30
   Gly Val Thr Ile Cys Glu
           35
<210> 214
   <211> 51
   <212> PRT
   <213> Homo sapiens
<400> 214
   Asp Asp Asn Glu Cys Gly Asn Leu Thr Gln Ser Cys Gly Glu Asn Ala
   1               5                   10                  15
   Asn Cys Thr Asn Thr Glu Gly Ser Tyr Tyr Cys Met Cys Val Pro Gly
               20                  25                  30
   Phe Arg Ser Ser Ser Asn Gln Asp Arg Phe Ile Thr Asn Asp Gly Thr
           35                  40                  45
   Val Cys Ile
       50
<210> 215
   <211> 46
   <212> PRT
   <213> Homo sapiens
<400> 215
   Tyr Thr Gln Cys Thr Asp Gly Tyr Glu Trp Asp Pro Val Arg Gln Gln
   1               5                   10                  15
   Cys Lys Asp Ile Asp Glu Cys Asp Ile Val Pro Asp Ala Cys Lys Gly
               20                  25                  30
   Gly Met Lys Cys Val Asn His Tyr Gly Gly Tyr Leu Cys Leu
           35                  40                  45
<210> 216
   <211> 41
   <212> PRT
   <213> Homo sapiens
<400> 216
   Asp Ile Asp Glu Cys Thr Ala Gly Thr His Asn Cys Arg Ala Asp Gln
   1 5 10 15
   Val Cys Ile Asn Leu Arg Gly Ser Phe Ala Cys Gln Cys Pro Pro Gly
   20 25 30
   Tyr Gln Lys Arg Gly Glu Gln Cys Val
   35 40
<210> 217
   <211> 40
   <212> PRT
   <213> Homo sapiens
<400> 217
   Asp Ile Asp Glu Cys Thr Ile Pro Pro Tyr Cys His Gln Arg Cys Val
   1 5 10 15
   Asn Thr Pro Gly Ser Phe Tyr Cys Gln Cys Ser Pro Gly Phe Gln Leu
   20 25 30
   Ala Ala Asn Asn Tyr Thr Cys Val
   35 40
<210> 218
   <211> 40
   <212> PRT
   <213> Homo sapiens
<400> 218
   Asp Ile Asn Glu Cys Asp Ala Ser Asn Gln Cys Ala Gln Gln Cys Tyr
   1 5 10 15
   Asn Ile Leu Gly Ser Phe Ile Cys Gln Cys Asn Gln Gly Tyr Glu Leu
   20 25 30
   Ser Ser Asp Arg Leu Asn Cys Glu
   35 40
<210> 219
   <211> 40
   <212> PRT
   <213> Homo sapiens
<400> 219
   Asp Ile Asp Glu Cys Arg Thr Ser Ser Tyr Leu Cys Gln Tyr Gln Cys
   1 5 10 15
   Val Asn Glu Pro Gly Lys Phe Ser Cys Met Cys Pro Gln Gly Tyr Gln
   20 25 30
   Val Val Arg Ser Arg Thr Cys Gln
   35 40
<210> 220
   <211> 45
   <212> PRT
   <213> Homo sapiens
<400> 220
   Asp Ile Asn Glu Cys Glu Thr Thr Asn Glu Cys Arg Glu Asp Glu Met
   1 5 10 15
   Cys Trp Asn Tyr His Gly Gly Phe Arg Cys Tyr Pro Arg Asn Pro Cys
   20 25 30
   Gln Asp Pro Tyr Ile Leu Thr Pro Glu Asn Arg Cys Val
   35 40 45
<210> 221
   <211> 46
   <212> PRT
   <213> Homo sapiens
<400> 221
   Tyr Thr Glu Cys Thr Asp Gly Tyr Glu Trp Asp Pro Asp Ser Gln His
   1 5 10 15
   Cys Arg Asp Val Asn Glu Cys Leu Thr Ile Pro Glu Ala Cys Lys Gly
   20 25 30
   Glu Met Lys Cys Ile Asn His Tyr Gly Gly Tyr Leu Cys Leu
   35 40 45
<210> 222
   <211> 41
   <212> PRT
   <213> Homo sapiens
<400> 222
   Asp Val Asp Glu Cys Ala Gln Ala Leu His Asp Cys Arg Pro Ser Gln
   1 5 10 15
   Asp Cys His Asn Leu Pro Gly Ser Tyr Gln Cys Thr Cys Pro Asp Gly
   20 25 30
   Tyr Arg Lys Ile Gly Pro Glu Cys Val
   35 40
<210> 223
   <211> 39
   <212> PRT
   <213> Homo sapiens
<400> 223
   Asp Ile Asp Glu Cys Arg Tyr Arg Tyr Cys Gln His Arg Cys Val Asn
   1 5 10 15
   Leu Pro Gly Ser Phe Arg Cys Gln Cys Glu Pro Gly Phe Gln Leu Gly
   20 25 30
   Pro Asn Asn Arg Ser Cys Val
   35
<210> 224
   <211> 40
   <212> PRT
   <213> Homo sapiens
<400> 224
   Asp Val Asn Glu Cys Asp Met Gly Ala Pro Cys Glu Gln Arg Cys Phe
   1               5                   10                  15
   Asn Ser Tyr Gly Thr Phe Leu Cys Arg Cys His Gln Gly Tyr Glu Leu
               20                  25                  30
   His Arg Asp Gly Phe Ser Cys Ser
           35                  40
<210> 225
   <211> 40
   <212> PRT
   <213> Homo sapiens
<400> 225
   Asp Ile Asp Glu Cys Ser Tyr Ser Ser Tyr Leu Cys Gln Tyr Arg Cys
   1               5                   10                  15
   Ile Asn Glu Pro Gly Arg Phe Ser Cys His Cys Pro Gln Gly Tyr Gln
               20                  25                  30
   Leu Leu Ala Thr Arg Leu Cys Gln
           35                  40
<210> 226
   <211> 46
   <212> PRT
   <213> Homo sapiens
<400> 226
   Asp Ile Asp Glu Cys Glu Ser Gly Ala His Gln Cys Ser Glu Ala Gln
   1               5                   10                  15
   Thr Cys Val Asn Phe His Gly Gly Tyr Arg Cys Val Asp Thr Asn Arg
               20                  25                  30
   Cys Val Glu Pro Tyr Ile Gln Val Ser Glu Asn Arg Cys Leu
           35                  40                  45
<210> 227
   <211> 49
   <212> PRT
   <213> Homo sapiens
<400> 227
   Lys Gly Asn Asn Cys Arg Asp Ser Thr Leu Cys Pro Ala Tyr Ala Thr
   1               5                   10                  15
   Cys Thr Asn Thr Val Asp Ser Tyr Tyr Cys Ala Cys Lys Gln Gly Phe
               20                  25                  30
   Leu Ser Ser Asn Gly Gln Asn His Phe Lys Asp Pro Gly Val Arg Cys
           35                  40                  45
   Lys
<210> 228
   <211> 52
   <212> PRT
   <213> Homo sapiens
<400> 228
   Asp Ile Asp Glu Cys Ser Gln Ser Pro Gln Pro Cys Gly Pro Asn Ser
   1               5                   10                  15
   Ser Cys Lys Asn Leu Ser Gly Arg Tyr Lys Cys Ser Cys Leu Asp Gly
               20                  25                  30
   Phe Ser Ser Pro Thr Gly Asn Asp Trp Val Pro Gly Lys Pro Gly Asn
           35                  40                  45
   Phe Ser Cys Thr
       50
<210> 229
   <211> 40
   <212> PRT
   <213> Homo sapiens
<400> 229
   Asp Ile Asn Glu Cys Leu Thr Ser Ser Val Cys Pro Glu His Ser Asp
   1               5                   10                  15
   Cys Val Asn Ser Met Gly Ser Tyr Ser Cys Ser Cys Gln Val Gly Phe
               20                  25                  30
   Ile Ser Arg Asn Ser Thr Cys Glu
           35                  40
<210> 230
   <211> 49
   <212> PRT
   <213> Homo sapiens
<400> 230
   Asp Val Asp Glu Cys Ala Asp Pro Arg Ala Cys Pro Glu His Ala Thr
   1               5                   10                  15
   Cys Asn Asn Thr Val Gly Asn Tyr Ser Cys Phe Cys Asn Pro Gly Phe
               20                  25                  30
   Glu Ser Ser Ser Gly His Leu Ser Phe Gln Gly Leu Lys Ala Ser Cys
           35                  40                  45
   Glu
<210> 231
   <211> 47
   <212> PRT
   <213> Homo sapiens
<400> 231
   Asp Ile Asp Glu Cys Thr Glu Met Cys Pro Ile Asn Ser Thr Cys Thr
   1               5                   10                  15
   Asn Thr Pro Gly Ser Tyr Phe Cys Thr Cys His Pro Gly Phe Ala Pro
               20                  25                  30
   Ser Asn Gly Gln Leu Asn Phe Thr Asp Gln Gly Val Glu Cys Arg
           35                  40                  45
<210> 232
   <211> 49
   <212> PRT
   <213> Homo sapiens
<400> 232
   Asp Ile Asp Glu Cys Arg Gln Asp Pro Ser Thr Cys Gly Pro Asn Ser
   1               5                   10                  15
   Ile Cys Thr Asn Ala Leu Gly Ser Tyr Ser Cys Gly Cys Ile Ala Gly
               20                  25                  30
   Phe His Pro Asn Pro Glu Gly Ser Gln Lys Asp Gly Asn Phe Ser Cys
           35                  40                  45
   Gln
<210> 233
   <211> 42
   <212> PRT
   <213> Homo sapiens
<400> 233
   Asp Ser Arg Gly Cys Ala Arg Trp Cys Pro Gln Asp Ser Ser Cys Val
   1               5                   10                  15
   Asn Ala Thr Ala Cys Arg Cys Asn Pro Gly Phe Ser Ser Phe Ser Glu
               20                  25                  30
   Ile Ile Thr Thr Pro Met Glu Thr Cys Asp
           35                  40
<210> 234
   <211> 52
   <212> PRT
   <213> Homo sapiens
<400> 234
   Asp Ile Asn Glu Cys Ala Thr Leu Ser Lys Val Ser Cys Gly Lys Phe
   1               5                   10                  15
   Ser Asp Cys Trp Asn Thr Glu Gly Ser Tyr Asp Cys Val Cys Ser Pro
               20                  25                  30
   Gly Tyr Glu Pro Val Ser Gly Ala Lys Thr Phe Lys Asn Glu Ser Glu
           35                  40                  45
   Asn Thr Cys Gln
       50
<210> 235
   <211> 44
   <212> PRT
   <213> Homo sapiens
<400> 235
   Asp Val Asp Glu Cys Gln Gln Asn Pro Arg Leu Cys Lys Ser Tyr Gly
   1               5                   10                  15
   Thr Cys Val Asn Thr Leu Gly Ser Tyr Thr Cys Gln Cys Leu Pro Gly
               20                  25                  30
   Phe Lys Leu Lys Pro Glu Asp Pro Lys Leu Cys Thr
           35                  40
<210> 236
   <211> 43
   <212> PRT
   <213> Homo sapiens
<400> 236
   Thr Lys Thr Ser Cys Ala Lys Cys Pro Pro Asn Ala Ser Cys Val Asn
   1               5                   10                  15
   Asn Thr His Cys Thr Cys Asn His Gly Tyr Thr Ser Gly Ser Gly Gln
               20                  25                  30
   Lys Leu Phe Thr Phe Pro Leu Glu Thr Cys Asn
           35                  40
<210> 237
   <211> 52
   <212> PRT
   <213> Homo sapiens
<400> 237
   Asp Ile Asn Glu Cys Thr Pro Pro Tyr Ser Val Tyr Cys Gly Phe Asn
   1               5                   10                  15
   Ala Val Cys Tyr Asn Val Glu Gly Ser Phe Tyr Cys Gln Cys Val Pro
               20                  25                  30
   Gly Tyr Arg Leu His Ser Gly Asn Glu Gln Phe Ser Asn Ser Asn Glu
                   35                  40                  45
   Asn Thr Cys Gln
       50
<210> 238
   <211> 37
   <212> PRT
   <213> Homo sapiens
<400> 238
   Asp Ile Cys Asp Pro Asn Pro Cys Glu Asn Gly Gly Ile Cys Leu Pro
   1               5                   10                  15
   Gly Leu Ala Asp Gly Ser Phe Ser Cys Glu Cys Pro Asp Gly Phe Thr
               20                  25                  30
   Asp Pro Asn Cys Ser
           35
<210> 239
   <211> 44
   <212> PRT
   <213> Homo sapiens
<400> 239
   Ser Ala Gly Pro Cys Thr Pro Asn Pro Cys His Asn Gly Gly Thr Cys
   1               5                   10                  15
   Glu Ile Ser Glu Ala Tyr Arg Gly Asp Thr Phe Ile Gly Tyr Val Cys
               20                  25                  30
   Lys Cys Pro Arg Gly Phe Asn Gly Ile His Cys Gln
           35                  40
<210> 240
   <211> 37
   <212> PRT
   <213> Homo sapiens
<400> 240
   Asn Ile Asn Glu Cys Glu Val Glu Pro Cys Lys Asn Gly Gly Ile Cys
   1               5                   10                  15
   Thr Asp Leu Val Ala Asn Tyr Ser Cys Glu Cys Pro Gly Glu Phe Met
               20                  25                  30
   Gly Arg Asn Cys Gln
           35
<210> 241
   <211> 40
   <212> PRT
   <213> Homo sapiens
<400> 241
   Asp Thr Asp Glu Cys Gln Ile Ala Gly Val Cys Gln Gln Met Cys Val
   1               5                   10                  15
   Asn Tyr Val Gly Gly Phe Glu Cys Tyr Cys Ser Glu Gly His Glu Leu
               20                  25                  30
   Glu Ala Asp Gly Ile Ser Cys Ser
           35                  40
<210> 242
   <211> 27
   <212> PRT
   <213> Homo sapiens
<400> 242
   Cys Glu Pro Gly Cys Lys Phe Gly Glu Cys Val Gly Pro Asn Lys Cys
     1               5                   10                  15
   Arg Cys Phe Pro Gly Tyr Thr Gly Lys Thr Cys
               20                  25
<210> 243
   <211> 40
   <212> PRT
   <213> Homo sapiens
<400> 243
   Asp Val Asn Glu Cys Gly Met Lys Pro Arg Pro Cys Gln His Arg Cys
   1               5                   10                  15
   Val Asn Thr His Gly Ser Tyr Lys Cys Phe Cys Leu Ser Gly His Met
               20                  25                  30
   Leu Met Pro Asp Ala Thr Cys Val
           35                  40
<210> 244
   <211> 37
   <212> PRT
   <213> Homo sapiens
<400> 244
   Thr Cys Ala Met Ile Asn Cys Gln Tyr Ser Cys Glu Asp Thr Glu Glu
   1 5 10 15
   Gly Pro Gln Cys Leu Cys Pro Ser Ser Gly Leu Arg Leu Ala Pro Asn
   20 25 30
   Gly Arg Asp Cys Leu
   35
<210> 245
   <211> 39
   <212> PRT
   <213> Homo sapiens
<400> 245
   Asp Ile Asp Glu Cys Ala Ser Gly Lys Val Ile Cys Pro Tyr Asn Arg
   1 5 10 15
   Arg Cys Val Asn Thr Phe Gly Ser Tyr Tyr Cys Lys Cys His Ile Gly
   20 25 30
   Phe Glu Leu Gln Tyr Ile Ser
   35
<210> 246
   <211> 41
   <212> PRT
   <213> Homo sapiens
<400> 246
   Asp Ile Asn Glu Cys Thr Met Asp Ser His Thr Cys Ser His His Ala
   1 5 10 15
   Asn Cys Phe Asn Thr Gln Gly Ser Phe Lys Cys Lys Cys Lys Gln Gly
   20 25 30
   Tyr Lys Gly Asn Gly Leu Arg Cys Ser
   35 40
<210> 247
   <211> 33
   <212> PRT
   <213> Homo sapiens
<400> 247
   Gly Ala Ala Ile Cys Gln Pro Pro Cys Arg Asn Gly Gly Ser Cys Val
   1 5 10 15
   Gln Pro Gly Arg Cys Arg Cys Pro Ala Gly Trp Arg Gly Asp Thr Cys
   20 25 30
   Gln
<210> 248
   <211> 41
   <212> PRT
   <213> Homo sapiens
<400> 248
   Asp Val Asp Glu Cys Ser Ala Arg Arg Gly Gly Cys Pro Gln Arg Cys
   1               5                   10                  15
   Val Asn Thr Ala Gly Ser Tyr Trp Cys Gln Cys Trp Glu Gly His Ser
               20                  25                  30
   Leu Ser Ala Asp Gly Thr Leu Cys Val
           35                  40
<210> 249
   <211> 32
   <212> PRT
   <213> Homo sapiens
<400> 249
   Glu Ala Ile Cys Ala Lys Pro Cys Leu Asn Gly Gly Val Cys Val Arg
   1               5                   10                  15
   Pro Asp Gln Cys Glu Cys Ala Pro Gly Trp Gly Gly Lys His Cys His
               20                  25                  30
<210> 250
   <211> 41
   <212> PRT
   <213> Homo sapiens
<400> 250
   Asp Val Asp Glu Cys Arg Thr Ser Ile Thr Leu Cys Ser His His Cys
   1               5                   10                  15
   Phe Asn Thr Ala Gly Ser Phe Thr Cys Gly Cys Pro His Asp Leu Val
               20                  25                  30
   Leu Gly Val Asp Gly Arg Thr Cys Met
           35                  40
<210> 251
   <211> 41
   <212> PRT
   <213> Homo sapiens
<400> 251
   Phe Ser His Leu Cys Leu Glu Asp His Asn Ser Tyr Cys Ile Asn Gly
   1               5                   10                  15
   Ala Cys Ala Phe His His Glu Leu Glu Lys Ala Ile Cys Arg Cys Phe
               20                  25                  30
   Thr Gly Tyr Thr Gly Glu Arg Cys Glu
           35                  40
<210> 252
   <211> 36
   <212> PRT
   <213> Homo sapiens
<400> 252
   Thr Ala Ala Cys Thr Asn Thr Ser Cys Ser Gly His Gly Glu Cys Val
   1               5                   10                  15
   Glu Thr Ile Asn Asn Tyr Thr Cys Lys Cys Asp Pro Gly Phe Ser Gly
               20                  25                  30
   Leu Lys Cys Glu
           35
<210> 253
   <211> 36
   <212> PRT
   <213> Homo sapiens
<400> 253
   Asp Tyr Cys Ser Gln Asn Thr Cys Leu Asn Gly Gly Lys Cys Ser Trp
   1               5                   10                  15
   Thr His Gly Ala Gly Tyr Val Cys Lys Cys Pro Pro Gln Phe Ser Gly
               20                  25                  30
   Lys His Cys Glu
           35
<210> 254
   <211> 29
   <212> PRT
   <213> Homo sapiens
<400> 254
   Pro Cys Leu Glu Gly Gly Thr Cys Ile Leu Ser Pro Lys Gly Ala Ser
   1               5                   10                  15
   Cys Asn Cys Pro His Pro Tyr Thr Gly Asp Arg Cys Glu
               20                  25
<210> 255
   <211> 35
   <212> PRT
   <213> Homo sapiens
<400> 255
   Asp Val Asp Glu Cys Val Gly Leu Gln Pro Val Cys Pro Gln Gly Thr
   1               5                   10                  15
   Thr Cys Ile Asn Thr Gly Gly Ser Phe Gln Cys Val Ser Pro Glu Cys
               20                  25                  30
   Pro Glu Gly
           35
<210> 256
   <211> 29
   <212> PRT
   <213> Homo sapiens
<400> 256
   Cys His Leu His Ala Arg Cys Val Ser Gln Glu Gly Val Ala Arg Cys
   1               5                   10                  15
   Arg Cys Leu Asp Gly Phe Glu Gly Asp Gly Phe Ser Cys
               20                  25
<210> 257
   <211> 38
   <212> PRT
   <213> Homo sapiens
<400> 257
   Cys Thr Asp Gly His Arg Gly Gly Cys Ser Glu His Ala Asn Cys Leu
   1               5                   10                  15
   Ser Thr Gly Leu Asn Thr Arg Arg Cys Glu Cys His Ala Gly Tyr Val
               20                  25                  30
   Gly Asp Gly Leu Gln Cys
           35
<210> 258
   <211> 38
   <212> PRT
   <213> Homo sapiens
<400> 258
   Cys Ser His Pro Asp Arg Gly Gly Cys Ser Glu Asn Ala Glu Cys Val
   1               5                   10                  15
   Pro Gly Ser Leu Gly Thr His His Cys Thr Cys His Lys Gly Trp Ser
               20                  25                  30
   Gly Asp Gly Arg Val Cys
           35
<210> 259
   <211> 32
   <212> PRT
   <213> Homo sapiens
<400> 259
   Gly Gly Cys His Thr Asp Ala Leu Cys Ser Tyr Val Gly Pro Gly Gln
   1               5                   10                  15
   Ser Arg Cys Thr Cys Lys Leu Gly Phe Ala Gly Asp Gly Tyr Gln Cys
               20                  25                  30
<210> 260
   <211> 37
   <212> PRT
   <213> Homo sapiens
<400> 260
   Cys Arg Ala Gly Asn Gly Gly Cys His Gly Leu Ala Thr Cys Arg Ala
   1               5                   10                  15
   Val Gly Gly Gly Gln Arg Val Cys Thr Cys Pro Pro Gly Phe Gly Gly
               20                  25                  30
   Asp Gly Phe Ser Cys
           35
<210> 261
   <211> 37
   <212> PRT
   <213> Homo sapiens
<400> 261
   Cys Ala His Gly His Gly Gly Cys Ser Pro His Ala Asn Cys Thr Lys
   1               5                   10                  15
   Val Ala Pro Gly Gln Arg Thr Cys Thr Cys Gln Asp Gly Tyr Met Gly
               20                  25                  30
   Asp Gly Glu Leu Cys
           35
<210> 262
   <211> 38
   <212> PRT
   <213> Homo sapiens
<400> 262
   Cys Leu Ile His His Gly Gly Cys His Ile His Ala Glu Cys Ile Pro
   1               5                   10                  15
   Thr Gly Pro Gln Gln Val Ser Cys Ser Cys Arg Glu Gly Tyr Ser Gly
               20                  25                  30
   Asp Gly Ile Arg Thr Cys
           35
<210> 263
   <211> 38
   <212> PRT
   <213> Homo sapiens
<400> 263
   Cys Ser Lys Asn Asn Gly Gly Cys Ser Pro Tyr Ala Thr Cys Lys Ser
   1               5                   10                  15
   Thr Gly Asp Gly Gln Arg Thr Cys Thr Cys Asp Thr Ala His Thr Val
               20                  25                  30
   Gly Asp Gly Leu Thr Cys
           35
<210> 264
   <211> 30
   <212> PRT
   <213> Homo sapiens
<400> 264
   Ser Gly Gln Cys Leu Cys Arg Ser Gly Phe Ala Gly Thr Ala Cys Glu
   1               5                   10                  15
   Leu Cys Ala Pro Gly Ala Phe Gly Pro His Cys Gln Ala Cys
               20                  25                  30
<210> 265
   <211> 36
   <212> PRT
   <213> Homo sapiens
<400> 265
   Cys Gln Asp Gly His Gly Gly Cys Ser Glu His Ala Asn Cys Ser Gln
   1               5                   10                  15
   Val Gly Thr Met Val Thr Cys Thr Cys Leu Pro Asp Tyr Glu Gly Asp
               20                  25                  30
   Gly Trp Ser Cys
           35
<210> 266
   <211> 33
   <212> PRT
   <213> Homo sapiens
<400> 266
   Asp Ile Asp Glu Cys Glu Val Phe Pro Gly Val Cys Lys Asn Gly Leu
   1               5                   10                  15
   Cys Val Asn Thr Arg Gly Ser Phe Lys Cys Gln Cys Pro Ser Gly Met
               20                  25                  30
   Thr
<210> 267
   <211> 32
   <212> PRT
   <213> Homo sapiens
<400> 267
   Asp Ile Asn Glu Cys Lys Met Ile Pro Ser Leu Cys Thr His Gly Lys
   1               5                   10                  15
   Cys Arg Asn Thr Ile Gly Ser Phe Lys Cys Arg Cys Asp Ser Gly Phe
               20                  25                  30
<210> 268
   <211> 24
   <212> PRT
   <213> Homo sapiens
<400> 268
   Ser Phe Lys Cys Arg Cys Asp Ser Gly Phe Ala Leu Asp Ser Glu Glu
   1               5                   10                  15
   Arg Asn Cys Thr Asp Ile Asp Glu
               20
<210> 269
   <211> 35
   <212> PRT
   <213> Homo sapiens
<400> 269
   Asp Ile Asp Glu Cys Arg Ile Ser Pro Asp Leu Cys Gly Arg Gly Gln
   1 5 10 15
   Cys Val Asn Thr Pro Gly Asp Phe Glu Cys Lys Cys Asp Glu Gly Tyr
   20 25 30
   Glu Ser Gly
   35
<210> 270
   <211> 34
   <212> PRT
   <213> Homo sapiens
<400> 270
   Asp Ile Asp Glu Cys Gln Arg Asp Pro Leu Leu Cys Arg Gly Gly Val
   1 5 10 15
   Cys His Asn Thr Glu Gly Ser Tyr Arg Cys Glu Cys Pro Pro Gly His
   20 25 30
   Gln Leu
<210> 271
   <211> 34
   <212> PRT
   <213> Homo sapiens
<400> 271
   Asp Ile Asn Glu Cys Glu Leu Ser Ala His Leu Cys Pro Asn Gly Arg
   1 5 10 15
   Cys Val Asn Leu Ile Gly Lys Tyr Gln Cys Ala Cys Asn Pro Gly Tyr
   20 25 30
   His Ser
<210> 272
   <211> 41
   <212> PRT
   <213> Homo sapiens
<400> 272
   Asp Ile Asp Glu Cys Glu Asp Asn Pro Asn Ile Cys Asp Gly Gly Gln
   1 5 10 15
   Cys Thr Asn Ile Pro Gly Glu Tyr Arg Cys Leu Cys Tyr Asp Gly Phe
   20 25 30
   Met Ala Ser Glu Asp Met Lys Thr Cys
   35 40
<210> 273
   <211> 33
   <212> PRT
   <213> Homo sapiens
<400> 273
   Asp Val Asn Glu Cys Asp Leu Asn Pro Asn Ile Cys Leu Ser Gly Thr
   1               5                   10                  15
   Cys Glu Asn Thr Lys Gly Ser Phe Ile Cys His Cys Asp Met Gly Tyr
               20                  25                  30
   Ser
<210> 274
   <211> 32
   <212> PRT
   <213> Homo sapiens
<400> 274
   Ile Val Pro Ile Cys Arg His Ser Cys Gly Asp Gly Phe Cys Ser Arg
   1               5                   10                  15
   Pro Asn Met Cys Thr Cys Pro Ser Gly Gln Ile Ala Pro Ser Cys Gly
               20                  25                  30
<210> 275
   <211> 41
   <212> PRT
   <213> Homo sapiens
<400> 275
   Asp Ile Asn Glu Cys Glu Thr Pro Gly Ile Cys Met Asn Gly Arg Cys
   1               5                   10                  15
   Val Asn Thr Asp Gly Ser Tyr Arg Cys Glu Cys Phe Pro Gly Leu Ala
               20                  25                  30
   Val Gly Leu Asp Gly Arg Val Cys Val
           35                  40
<210> 276
   <211> 42
   <212> PRT
   <213> Homo sapiens
<400> 276
   Asp Ile Asn Glu Cys Ala Leu Asp Pro Asp Ile Cys Pro Asn Gly Ile
   1               5                   10                  15
   Cys Glu Asn Leu Arg Gly Thr Tyr Lys Cys Ile Cys Asn Ser Gly Tyr
               20                  25                  30
   Glu Val Asp Ser Thr Gly Lys Asn Cys Val
           35                  40
<210> 277
   <211> 42
   <212> PRT
   <213> Homo sapiens
<400> 277
   Asp Ile Asn Glu Cys Val Leu Asn Ser Leu Leu Cys Asp Asn Gly Gln
   1               5                   10                  15
   Cys Arg Asn Thr Pro Gly Ser Phe Val Cys Thr Cys Pro Lys Gly Phe
               20                  25                  30
   Ile Tyr Lys Pro Asp Leu Lys Thr Cys Glu
   35 40
<210> 278
   <211> 40
   <212> PRT
   <213> Homo sapiens
<400> 278
   Asp Ile Asp Glu Cys Glu Ser Ser Pro Cys Ile Asn Gly Val Cys Lys
   1               5                   10                  15
   Asn Ser Pro Gly Ser Phe Ile Cys Glu Cys Ser Ser Glu Ser Thr Leu
               20                  25                  30
   Asp Pro Thr Lys Thr Ile Cys Ile
           35                  40
<210> 279
   <211> 42
   <212> PRT
   <213> Homo sapiens
<400> 279
   Asp Ile Asp Glu Cys Glu Val Phe Pro Gly Val Cys Lys Asn Gly Leu
   1               5                   10                  15
   Cys Val Asn Thr Arg Gly Ser Phe Lys Cys Gln Cys Pro Ser Gly Met
               20                  25                  30
   Thr Leu Asp Ala Thr Gly Arg Ile Cys Leu
           35                  40
<210> 280
   <211> 42
   <212> PRT
   <213> Homo sapiens
<400> 280
   Asp Ile Asn Glu Cys Lys Met Ile Pro Ser Leu Cys Thr His Gly Lys
   1               5                   10                  15
   Cys Arg Asn Thr Ile Gly Ser Phe Lys Cys Arg Cys Asp Ser Gly Phe
               20                  25                  30
   Ala Leu Asp Ser Glu Glu Arg Asn Cys Thr
           35                  40
<210> 281
   <211> 43
   <212> PRT
   <213> Homo sapiens
<400> 281
   Asp Ile Asp Glu Cys Arg Ile Ser Pro Asp Leu Cys Gly Arg Gly Gln
   1               5                   10                  15
   Cys Val Asn Thr Pro Gly Asp Phe Glu Cys Lys Cys Asp Glu Gly Tyr
               20                  25                  30
   Glu Ser Gly Phe Met Met Met Lys Asn Cys Met
           35                  40
<210> 282
   <211> 42
   <212> PRT
   <213> Homo sapiens
<400> 282
   Asp Ile Asp Glu Cys Gln Arg Asp Pro Leu Leu Cys Arg Gly Gly Val
   1               5                   10                  15
   Cys His Asn Thr Glu Gly Ser Tyr Arg Cys Glu Cys Pro Pro Gly His
                   20                  25                  30
   Gln Leu Ser Pro Asn Ile Ser Ala Cys Ile
           35                  40
<210> 283
   <211> 42
   <212> PRT
   <213> Homo sapiens
<400> 283
   Asp Ile Asn Glu Cys Glu Leu Ser Ala His Leu Cys Pro Asn Gly Arg
   1               5                   10                  15
   Cys Val Asn Leu Ile Gly Lys Tyr Gln Cys Ala Cys Asn Pro Gly Tyr
               20                  25                  30
   His Ser Thr Pro Asp Arg Leu Phe Cys Val
           35                  40
<210> 284
   <211> 41
   <212> PRT
   <213> Homo sapiens
<400> 284
   Asp Ile Asp Glu Cys Ser Ile Met Asn Gly Gly Cys Glu Thr Phe Cys
   1               5                   10                  15
   Thr Asn Ser Glu Gly Ser Tyr Glu Cys Ser Cys Gln Pro Gly Phe Ala
               20                  25                  30
   Leu Met Pro Asp Gln Arg Ser Cys Thr
           35                  40
<210> 285
   <211> 32
   <212> PRT
   <213> Homo sapiens
<400> 285
   Ser Ile Gln His Cys Asn Ile Arg Cys Met Asn Gly Gly Ser Cys Ser
   1               5                   10                  15
   Asp Asp His Cys Leu Cys Gln Lys Gly Tyr Ile Gly Thr His Cys Gly
               20                  25                  30
<210> 286
   <211> 42
   <212> PRT
   <213> Homo sapiens
<400> 286
   Asp Ile Asp Glu Cys Glu Asp Asn Pro Asn Ile Cys Asp Gly Gly Gln
   1               5                   10                  15
   Cys Thr Asn Ile Pro Gly Glu Tyr Arg Cys Leu Cys Tyr Asp Gly Phe
               20                  25                  30
   Met Ala Ser Glu Asp Met Lys Thr Cys Val
           35                  40
<210> 287
   <211> 42
   <212> PRT
   <213> Homo sapiens
<400> 287
   Asp Val Asn Glu Cys Asp Leu Asn Pro Asn Ile Cys Leu Ser Gly Thr
   1               5                   10                  15
   Cys Glu Asn Thr Lys Gly Ser Phe Ile Cys His Cys Asp Met Gly Tyr
                   20                  25                  30
   Ser Gly Lys Lys Gly Lys Thr Gly Cys Thr
           35                  40
<210> 288
   <211> 41
   <212> PRT
   <213> Homo sapiens
<400> 288
   Asp Ile Asn Glu Cys Glu Ile Gly Ala His Asn Cys Gly Lys His Ala
   1               5                   10                  15
   Val Cys Thr Asn Thr Ala Gly Ser Phe Lys Cys Ser Cys Ser Pro Gly
               20                  25                  30
   Trp Ile Gly Asp Gly Ile Lys Cys Thr
           35                  40
<210> 289
   <211> 41
   <212> PRT
   <213> Homo sapiens
<400> 289
   Asp Leu Asp Glu Cys Ser Asn Gly Thr His Met Cys Ser Gln His Ala
   1               5                   10                  15
   Asp Cys Lys Asn Thr Met Gly Ser Tyr Arg Cys Leu Cys Lys Glu Gly
               20                  25                  30
   Tyr Thr Gly Asp Gly Phe Thr Cys Thr
           35                  40
<210> 290
   <211> 42
   <212> PRT
   <213> Homo sapiens
<400> 290
   Asp Leu Asp Glu Cys Ser Glu Asn Leu Asn Leu Cys Gly Asn Gly Gln
   1               5                   10                  15
   Cys Leu Asn Ala Pro Gly Gly Tyr Arg Cys Glu Cys Asp Met Gly Phe
               20                  25                  30
   Val Pro Ser Ala Asp Gly Lys Ala Cys Glu
           35                  40
<210> 291
   <211> 41
   <212> PRT
   <213> Homo sapiens
<400> 291
   Asp Ile Asp Glu Cys Ser Leu Pro Asn Ile Cys Val Phe Gly Thr Cys
   1               5                   10                  15
   His Asn Leu Pro Gly Leu Phe Arg Cys Glu Cys Glu Ile Gly Tyr Glu
               20                  25                  30
   Leu Asp Arg Ser Gly Gly Asn Cys Thr
           35                  40
<210> 292
   <211> 41
   <212> PRT
   <213> Homo sapiens
<400> 292
   Asp Val Asn Glu Cys Leu Asp Pro Thr Thr Cys Ile Ser Gly Asn Cys
   1               5                   10                  15
   Val Asn Thr Pro Gly Ser Tyr Ile Cys Asp Cys Pro Pro Asp Phe Glu
               20                  25                  30
   Leu Asn Pro Thr Arg Val Gly Cys Val
           35                  40
<210> 293
   <211> 42
   <212> PRT
   <213> Homo sapiens
<400> 293
   Asp Ile Asp Glu Cys Gln Glu Leu Pro Gly Leu Cys Gln Gly Gly Lys
   1               5                   10                  15
   Cys Ile Asn Thr Phe Gly Ser Phe Gln Cys Arg Cys Pro Thr Gly Tyr
               20                  25                  30
   Tyr Leu Asn Glu Asp Thr Arg Val Cys Asp
           35                  40
<210> 294
   <211> 41
   <212> PRT
   <213> Homo sapiens
<400> 294
   Asp Val Asn Glu Cys Glu Thr Pro Gly Ile Cys Gly Pro Gly Thr Cys
   1               5                   10                  15
   Tyr Asn Thr Val Gly Asn Tyr Thr Cys Ile Cys Pro Pro Asp Tyr Met
               20                  25                  30
   Gln Val Asn Gly Gly Asn Asn Cys Met
           35                  40
<210> 295
   <211> 42
   <212> PRT
   <213> Homo sapiens
<400> 295
   Asp Ile Asp Glu Cys Arg Glu Ile Pro Gly Val Cys Glu Asn Gly Val
   1               5                   10                  15
   Cys Ile Asn Met Val Gly Ser Phe Arg Cys Glu Cys Pro Val Gly Phe
               20                  25                  30
   Phe Tyr Asn Asp Lys Leu Leu Val Cys Glu
           35                  40
<210> 296
   <211> 32
   <212> PRT
   <213> Homo sapiens
<400> 296
   Gln Pro Val Cys Glu Ser Gly Cys Leu Asn Gly Gly Arg Cys Val Ala
   1               5                   10                  15
   Pro Asn Arg Cys Ala Cys Thr Tyr Gly Phe Thr Gly Pro Gln Cys Glu
               20                  25                  30
<210> 297
   <211> 41
   <212> PRT
   <213> Homo sapiens
<400> 297
   Asp Ile Asp Glu Cys Gln Asn Gly Pro Val Cys Gln Arg Asn Ala Glu
   1               5                   10                  15
   Cys Ile Asn Thr Ala Gly Ser Tyr Arg Cys Asp Cys Lys Pro Gly Tyr
               20                  25                  30
   Arg Phe Thr Ser Thr Gly Gln Cys Asn
           35                  40
<210> 298
   <211> 42
   <212> PRT
   <213> Homo sapiens
<400> 298
   Asp Arg Asn Glu Cys Gln Glu Ile Pro Asn Ile Cys Ser His Gly Gln
   1               5                   10                  15
   Cys Ile Asp Thr Val Gly Ser Phe Tyr Cys Leu Cys His Thr Gly Phe
               20                  25                  30
   Lys Thr Asn Asp Asp Gln Thr Met Cys Leu
           35                  40
<210> 299
   <211> 39
   <212> PRT
   <213> Homo sapiens
<400> 299
   Asp Ile Asn Glu Cys Glu Arg Asp Ala Cys Gly Asn Gly Thr Cys Arg
   1               5                   10                  15
   Asn Thr Ile Gly Ser Phe Asn Cys Arg Cys Asn His Gly Phe Ile Leu
               20                  25                  30
   Ser His Asn Asn Asp Cys Ile
           35
<210> 300
   <211> 43
   <212> PRT
   <213> Homo sapiens
<400> 300
   Asp Val Asp Glu Cys Ala Ser Gly Asn Gly Asn Leu Cys Arg Asn Gly
   1               5                   10                  15
   Gln Cys Ile Asn Thr Val Gly Ser Phe Gln Cys Gln Cys Asn Glu Gly
               20                  25                  30
   Tyr Glu Val Ala Pro Asp Gly Arg Thr Cys Val
           35                  40
<210> 301
   <211> 40
   <212> PRT
   <213> Homo sapiens
<400> 301
   Asp Ile Asn Glu Cys Leu Leu Glu Pro Arg Lys Cys Ala Pro Gly Thr
   1               5                   10                  15
   Cys Gln Asn Leu Asp Gly Ser Tyr Arg Cys Ile Cys Pro Pro Gly Tyr
               20                  25                  30
   Ser Leu Gln Asn Glu Lys Cys Glu
           35                  40
<210> 302
   <211> 42
   <212> PRT
   <213> Homo sapiens
<400> 302
   Asp Ile Asp Glu Cys Val Glu Glu Pro Glu Ile Cys Ala Leu Gly Thr
   1               5                   10                  15
   Cys Ser Asn Thr Glu Gly Ser Phe Lys Cys Leu Cys Pro Glu Gly Phe
               20                  25                  30
   Ser Leu Ser Ser Ser Gly Arg Arg Cys Gln
           35                  40
<210> 303
   <211> 39
   <212> PRT
   <213> Homo sapiens
<400> 303
   Asp Met Asp Glu Cys Lys Glu Pro Asp Val Cys Lys His Gly Gln Cys
   1               5                   10                  15
   Ile Asn Thr Asp Gly Ser Tyr Arg Cys Glu Cys Pro Phe Gly Tyr Ile
               20                  25                  30
   Leu Ala Gly Asn Glu Cys Val
           35
<210> 304
   <211> 40
   <212> PRT
   <213> Homo sapiens
<400> 304
   Asp Thr Asp Glu Cys Ser Val Gly Asn Pro Cys Gly Asn Gly Thr Cys
   1               5                   10                  15
   Lys Asn Val Ile Gly Gly Phe Glu Cys Thr Cys Glu Glu Gly Phe Glu
               20                  25                  30
   Pro Gly Pro Met Met Thr Cys Glu
           35                  40
<210> 305
   <211> 41
   <212> PRT
   <213> Homo sapiens
<400> 305
   Asp Ile Asn Glu Cys Ala Gln Asn Pro Leu Leu Cys Ala Phe Arg Cys
   1               5                   10                  15
   Val Asn Thr Tyr Gly Ser Tyr Glu Cys Lys Cys Pro Val Gly Tyr Val
               20                  25                  30
   Leu Arg Glu Asp Arg Arg Met Cys Lys
           35                  40
<210> 306
   <211> 44
   <212> PRT
   <213> Homo sapiens
<400> 306
   Asp Glu Asp Glu Cys Glu Glu Gly Lys His Asp Cys Thr Glu Lys Gln
   1               5                   10                  15
   Met Glu Cys Lys Asn Leu Ile Gly Thr Tyr Met Cys Ile Cys Gly Pro
               20                  25                  30
   Gly Tyr Gln Arg Arg Pro Asp Gly Glu Gly Cys Val
           35                  40
<210> 307
   <211> 42
   <212> PRT
   <213> Homo sapiens
<400> 307
   Asp Val Asp Glu Cys Gln Ala Ile Pro Gly Leu Cys Gln Gly Gly Asn
   1               5                   10                  15
   Cys Ile Asn Thr Val Gly Ser Phe Glu Cys Lys Cys Pro Ala Gly His
               20                  25                  30
   Lys Leu Asn Glu Val Ser Gln Lys Cys Glu
           35                  40
<210> 308
   <211> 42
   <212> PRT
   <213> Homo sapiens
<400> 308
   Asp Glu Asn Glu Cys Gln Thr Lys Pro Gly Ile Cys Glu Asn Gly Arg
   1               5                   10                  15
   Cys Leu Asn Thr Arg Gly Ser Tyr Thr Cys Glu Cys Asn Asp Gly Phe
               20                  25                  30
   Thr Ala Ser Pro Asn Gln Asp Glu Cys Leu
           35                  40
<210> 309
   <211> 42
   <212> PRT
   <213> Homo sapiens
<400> 309
   Asp Ile Asp Glu Cys Lys Val Ile His Asp Val Cys Arg Asn Gly Glu
   1               5                   10                  15
   Cys Val Asn Asp Arg Gly Ser Tyr His Cys Ile Cys Lys Thr Gly Tyr
               20                  25                  30
   Thr Pro Asp Ile Thr Gly Thr Ser Cys Val
           35                  40
<210> 310
   <211> 41
   <212> PRT
   <213> Homo sapiens
<400> 310
   Asp Leu Asn Glu Cys Asn Gln Ala Pro Lys Pro Cys Asn Phe Ile Cys
   1               5                   10                  15
   Lys Asn Thr Glu Gly Ser Tyr Gln Cys Ser Cys Pro Lys Gly Tyr Ile
               20                  25                  30
   Leu Gln Glu Asp Gly Arg Ser Cys Lys
           35                  40
<210> 311
   <211> 39
   <212> PRT
   <213> Homo sapiens
<400> 311
   Asp Leu Asp Glu Cys Ala Thr Lys Gln His Asn Cys Gln Phe Leu Cys
   1               5                   10                  15
   Val Asn Thr Ile Gly Gly Phe Thr Cys Lys Cys Pro Pro Gly Phe Thr
               20                  25                  30
   Gln His His Thr Ser Cys Ile
           35
<210> 312
   <211> 43
   <212> PRT
   <213> Homo sapiens
<400> 312
   Asp Asn Asn Glu Cys Thr Ser Asp Ile Asn Leu Cys Gly Ser Lys Gly
   1               5                   10                  15
   Ile Cys Gln Asn Thr Pro Gly Ser Phe Thr Cys Glu Cys Gln Arg Gly
               20                  25                  30
   Phe Ser Leu Asp Gln Thr Gly Ser Ser Cys Glu
           35                  40
<210> 313
   <211> 40
   <212> PRT
   <213> Homo sapiens
<400> 313
   Asp Val Asp Glu Cys Glu Gly Asn His Arg Cys Gln His Gly Cys Gln
   1               5                   10                  15
   Asn Ile Ile Gly Gly Tyr Arg Cys Ser Cys Pro Gln Gly Tyr Leu Gln
               20                  25                  30
   His Tyr Gln Trp Asn Gln Cys Val
           35                  40
<210> 314
   <211> 41
   <212> PRT
   <213> Homo sapiens
<400> 314
   Asp Glu Asn Glu Cys Leu Ser Ala His Ile Cys Gly Gly Ala Ser Cys
   1 5 10 15
   His Asn Thr Leu Gly Ser Tyr Lys Cys Met Cys Pro Ala Gly Phe Gln
   20 25 30
   Tyr Glu Gln Phe Ser Gly Gly Cys Gln
   35 40
<210> 315
   <211> 40
   <212> PRT
   <213> Homo sapiens
<400> 315
   Asp Ile Asn Glu Cys Gly Ser Ala Gln Ala Pro Cys Ser Tyr Gly Cys
   1 5 10 15
   Ser Asn Thr Glu Gly Gly Tyr Leu Cys Gly Cys Pro Pro Gly Tyr Phe
   20 25 30
   Arg Ile Gly Gln Gly His Cys Val
   35 40
<210> 316
   <211> 42
   <212> PRT
   <213> Homo sapiens
<400> 316
   Asp Ile Asp Glu Cys Ser Thr Ile Pro Gly Ile Cys Glu Gly Gly Glu
   1 5 10 15
   Cys Thr Asn Thr Val Ser Ser Tyr Phe Cys Lys Cys Pro Pro Gly Phe
   20 25 30
   Tyr Thr Ser Pro Asp Gly Thr Arg Cys Ile
   35 40
<210> 317
   <211> 41
   <212> PRT
   <213> Homo sapiens
<400> 317
   Val Thr Asp Tyr Cys Gln Leu Val Arg Tyr Leu Cys Gln Asn Gly Arg
   1 5 10 15
   Cys Ile Pro Thr Pro Gly Ser Cys Arg Cys Glu Cys Asn Lys Gly Phe
   20 25 30
   Gln Leu Asp Leu Arg Gly Glu Cys Ile
   35 40
<210> 318
   <211> 40
   <212> PRT
   <213> Homo sapiens
<400> 318
   Asp Val Asp Glu Cys Glu Lys Asn Pro Cys Ala Gly Gly Glu Cys Ile
   1               5                   10                  15
   Asn Asn Gln Gly Ser Tyr Thr Cys Gln Cys Arg Ala Gly Tyr Gln Ser
               20                  25                  30
   Thr Leu Thr Arg Thr Glu Cys Arg
           35                  40
<210> 319
   <211> 42
   <212> PRT
   <213> Homo sapiens
<400> 319
   Asp Ile Asp Glu Cys Leu Gln Asn Gly Arg Ile Cys Asn Asn Gly Arg
   1               5                   10                  15
   Cys Ile Asn Thr Asp Gly Ser Phe His Cys Val Cys Asn Ala Gly Phe
               20                  25                  30
   His Val Thr Arg Asp Gly Lys Asn Cys Glu
           35                  40
<210> 320
   <211> 41
   <212> PRT
   <213> Homo sapiens
<400> 320
   Asp Met Asp Glu Cys Ser Ile Arg Asn Met Cys Leu Asn Gly Met Cys
   1               5                   10                  15
   Ile Asn Glu Asp Gly Ser Phe Lys Cys Ile Cys Lys Pro Gly Phe Gln
               20                  25                  30
   Leu Ala Ser Asp Gly Arg Tyr Cys Lys
           35                  40
<210> 321
   <211> 32
   <212> PRT
   <213> Homo sapiens
<400> 321
   Ile Val Pro Ile Cys Arg Asn Ser Cys Gly Asp Gly Phe Cys Ser Arg
   1               5                   10                  15
   Pro Asn Met Cys Thr Cys Ser Ser Gly Gln Ile Ser Ser Thr Cys Gly
               20                  25                  30
<210> 322
   <211> 41
   <212> PRT
   <213> Homo sapiens
<400> 322
   Asp Val Asp Glu Cys Gln Thr Pro Gly Ile Cys Met Asn Gly His Cys
   1               5                   10                  15
   Ile Asn Ser Glu Gly Ser Phe Arg Cys Asp Cys Pro Pro Gly Leu Ala
               20                  25                  30
   Val Gly Met Asp Gly Arg Val Cys Val
           35                  40
<210> 323
   <211> 42
   <212> PRT
   <213> Homo sapiens
<400> 323
   Asp Ile Asn Glu Cys Ala Leu Asp Pro Asp Ile Cys Ala Asn Gly Ile
   1               5                   10                  15
   Cys Glu Asn Leu Arg Gly Ser Tyr Arg Cys Asn Cys Asn Ser Gly Tyr
               20                  25                  30
   Glu Pro Asp Ala Ser Gly Arg Asn Cys Ile
           35                  40
<210> 324
   <211> 42
   <212> PRT
   <213> Homo sapiens
<400> 324
   Asp Ile Asp Glu Cys Leu Val Asn Arg Leu Leu Cys Asp Asn Gly Leu
   1               5                   10                  15
   Cys Arg Asn Thr Pro Gly Ser Tyr Ser Cys Thr Cys Pro Pro Gly Tyr
               20                  25                  30
   Val Phe Arg Thr Glu Thr Glu Thr Cys Glu
           35                  40
<210> 325
   <211> 40
   <212> PRT
   <213> Homo sapiens
<400> 325
   Asp Ile Asn Glu Cys Glu Ser Asn Pro Cys Val Asn Gly Ala Cys Arg
   1               5                   10                  15
   Asn Asn Leu Gly Ser Phe Asn Cys Glu Cys Ser Pro Gly Ser Lys Leu
               20                  25                  30
   Ser Ser Thr Gly Leu Ile Cys Ile
           35                  40
<210> 326
   <211> 42
   <212> PRT
   <213> Homo sapiens
<400> 326
   Asp Val Asn Glu Cys Glu Val Phe Pro Gly Val Cys Pro Asn Gly Arg
   1               5                   10                  15
   Cys Val Asn Ser Lys Gly Ser Phe His Cys Glu Cys Pro Glu Gly Leu
               20                  25                  30
   Thr Leu Asp Gly Thr Gly Arg Val Cys Leu
           35                  40
<210> 327
   <211> 42
   <212> PRT
   <213> Homo sapiens
<400> 327
   Asp Ile Asn Glu Cys Lys Ala Phe Pro Gly Met Cys Thr Tyr Gly Lys
   1               5                   10                  15
   Cys Arg Asn Thr Ile Gly Ser Phe Lys Cys Arg Cys Asn Ser Gly Phe
               20                  25                  30
   Ala Leu Asp Met Glu Glu Arg Asn Cys Thr
           35                  40
<210> 328
   <211> 43
   <212> PRT
   <213> Homo sapiens
<400> 328
   Asp Ile Asp Glu Cys Arg Ile Ser Pro Asp Leu Cys Gly Ser Gly Ile
   1               5                   10                  15
   Cys Val Asn Thr Pro Gly Ser Phe Glu Cys Glu Cys Phe Glu Gly Tyr
               20                  25                  30
   Glu Ser Gly Phe Met Met Met Lys Asn Cys Met
           35                  40
<210> 329
   <211> 42
   <212> PRT
   <213> Homo sapiens
<400> 329
   Asp Ile Asp Glu Cys Glu Arg Asn Pro Leu Leu Cys Arg Gly Gly Thr
   1               5                   10                  15
   Cys Val Asn Thr Glu Gly Ser Phe Gln Cys Asp Cys Pro Leu Gly His
               20                  25                  30
   Glu Leu Ser Pro Ser Arg Glu Asp Cys Val
           35                  40
<210> 330
   <211> 42
   <212> PRT
   <213> Homo sapiens
<400> 330
   Asp Ile Asn Glu Cys Ser Leu Ser Asp Asn Leu Cys Arg Asn Gly Lys
   1 5 10 15
   Cys Val Asn Met Ile Gly Thr Tyr Gln Cys Ser Cys Asn Pro Gly Tyr
   20 25 30
   Gln Ala Thr Pro Asp Arg Gln Gly Cys Thr
   35 40
<210> 331
   <211> 41
   <212> PRT
   <213> Homo sapiens
<400> 331
   Asp Ile Asp Glu Cys Met Ile Met Asn Gly Gly Cys Asp Thr Gln Cys
   1 5 10 15
   Thr Asn Ser Glu Gly Ser Tyr Glu Cys Ser Cys Ser Glu Gly Tyr Ala
   20 25 30
   Leu Met Pro Asp Gly Arg Ser Cys Ala
   35 40
<210> 332
   <211> 32
   <212> PRT
   <213> Homo sapiens
<400> 332
   Ser Ile Gln Gln Cys Ser Val Arg Cys Met Asn Gly Gly Thr Cys Ala
   1 5 10 15
   Asp Asp His Cys Gln Cys Gln Lys Gly Tyr Ile Gly Thr Tyr Cys Gly
   20 25 30
<210> 333
   <211> 42
   <212> PRT
   <213> Homo sapiens
<400> 333
   Asp Ile Asp Glu Cys Glu Asn Asn Pro Asp Ile Cys Asp Gly Gly Gln
   1 5 10 15
   Cys Thr Asn Ile Pro Gly Glu Tyr Arg Cys Leu Cys Tyr Asp Gly Phe
   20 25 30
   Met Ala Ser Met Asp Met Lys Thr Cys Ile
   35 40
<210> 334
   <211> 42
   <212> PRT
   <213> Homo sapiens
<400> 334
   Asp Val Asn Glu Cys Asp Leu Asn Ser Asn Ile Cys Met Phe Gly Glu
   1               5                   10                  15
   Cys Glu Asn Thr Lys Gly Ser Phe Ile Cys His Cys Gln Leu Gly Tyr
               20                  25                  30
   Ser Val Lys Lys Gly Thr Thr Gly Cys Thr
           35                  40
<210> 335
   <211> 41
   <212> PRT
   <213> Homo sapiens
<400> 335
   Asp Val Asp Glu Cys Glu Ile Gly Ala His Asn Cys Asp Met His Ala
   1               5                   10                  15
   Ser Cys Leu Asn Ile Pro Gly Ser Phe Lys Cys Ser Cys Arg Glu Gly
               20                  25                  30
   Trp Ile Gly Asn Gly Ile Lys Cys Ile
           35                  40
<210> 336
   <211> 41
   <212> PRT
   <213> Homo sapiens
<400> 336
   Asp Leu Asp Glu Cys Ser Asn Gly Thr His Gln Cys Ser Ile Asn Ala
   1               5                   10                  15
   Gln Cys Val Asn Thr Pro Gly Ser Tyr Arg Cys Ala Cys Ser Glu Gly
               20                  25                  30
   Phe Thr Gly Asp Gly Phe Thr Cys Ser
           35                  40
<210> 337
   <211> 42
   <212> PRT
   <213> Homo sapiens
<400> 337
   Asp Val Asp Glu Cys Ala Glu Asn Ile Asn Leu Cys Glu Asn Gly Gln
   1               5                   10                  15
   Cys Leu Asn Val Pro Gly Ala Tyr Arg Cys Glu Cys Glu Met Gly Phe
               20                  25                  30
   Thr Pro Ala Ser Asp Ser Arg Ser Cys Gln
           35                  40
<210> 338
   <211> 41
   <212> PRT
   <213> Homo sapiens
<400> 338
   Asp Ile Asp Glu Cys Ser Phe Gln Asn Ile Cys Val Phe Gly Thr Cys
   1               5                   10                  15
   Asn Asn Leu Pro Gly Met Phe His Cys Ile Cys Asp Asp Gly Tyr Glu
               20                  25                  30
   Leu Asp Arg Thr Gly Gly Asn Cys Thr
           35                  40
<210> 339
   <211> 41
   <212> PRT
   <213> Homo sapiens
<400> 339
   Asp Ile Asp Glu Cys Ala Asp Pro Ile Asn Cys Val Asn Gly Leu Cys
   1               5                   10                  15
   Val Asn Thr Pro Gly Arg Tyr Glu Cys Asn Cys Pro Pro Asp Phe Gln
               20                  25                  30
   Leu Asn Pro Thr Gly Val Gly Cys Val
           35                  40
<210> 340
   <211> 42
   <212> PRT
   <213> Homo sapiens
<400> 340
   Asp Ile Asp Glu Cys Gln Glu Leu Pro Gly Leu Cys Gln Gly Gly Asn
   1               5                   10                  15
   Cys Ile Asn Thr Phe Gly Ser Phe Gln Cys Glu Cys Pro Gln Gly Tyr
               20                  25                  30
   Tyr Leu Ser Glu Asp Thr Arg Ile Cys Glu
           35                  40
<210> 341
   <211> 42
   <212> PRT
   <213> Homo sapiens
<400> 341
   Asp Ile Asp Glu Cys Phe Ala His Pro Gly Val Cys Gly Pro Gly Thr
   1               5                   10                  15
   Cys Tyr Asn Thr Leu Gly Asn Tyr Thr Cys Ile Cys Pro Pro Glu Tyr
               20                  25                  30
   Met Gln Val Asn Gly Gly His Asn Cys Met
           35                  40
<210> 342
   <211> 42
   <212> PRT
   <213> Homo sapiens
<400> 342
   Asp Ile Asp Glu Cys Lys Glu Ile Pro Gly Ile Cys Ala Asn Gly Val
   1               5                   10                  15
   Cys Ile Asn Gln Ile Gly Ser Phe Arg Cys Glu Cys Pro Thr Gly Phe
               20                  25                  30
   Ser Tyr Asn Asp Leu Leu Leu Val Cys Glu
           35                  40
<210> 343
   <211> 33
   <212> PRT
   <213> Homo sapiens
<400> 343
   Gly Gln Pro Val Cys Glu Asn Gly Cys Gln Asn Gly Gly Arg Cys Ile
   1               5                   10                  15
   Gly Pro Asn Arg Cys Ala Cys Val Tyr Gly Phe Thr Gly Pro Gln Cys
               20                  25                  30
   Glu
<210> 344
   <211> 42
   <212> PRT
   <213> Homo sapiens
<400> 344
   Asp Ile Asp Glu Cys Ser Asn Gly Asp Asn Leu Cys Gln Arg Asn Ala
   1               5                   10                  15
   Asp Cys Ile Asn Ser Pro Gly Ser Tyr Arg Cys Glu Cys Ala Ala Gly
               20                  25                  30
   Phe Lys Leu Ser Pro Asn Gly Ala Cys Val
           35                  40
<210> 345
   <211> 42
   <212> PRT
   <213> Homo sapiens
<400> 345
   Asp Arg Asn Glu Cys Leu Glu Ile Pro Asn Val Cys Ser His Gly Leu
   1               5                   10                  15
   Cys Val Asp Leu Gln Gly Ser Tyr Gln Cys Ile Cys His Asn Gly Phe
               20                  25                  30
   Lys Ala Ser Gln Asp Gln Thr Met Cys Met
           35                  40
<210> 346
   <211> 39
   <212> PRT
   <213> Homo sapiens
<400> 346
   Asp Val Asp Glu Cys Glu Arg His Pro Cys Gly Asn Gly Thr Cys Lys
   1               5                   10                  15
   Asn Thr Val Gly Ser Tyr Asn Cys Leu Cys Tyr Pro Gly Phe Glu Leu
               20                  25                  30
   Thr His Asn Asn Asp Cys Leu
           35
<210> 347
   <211> 43
   <212> PRT
   <213> Homo sapiens
<400> 347
   Asp Ile Asp Glu Cys Ser Ser Phe Phe Gly Gln Val Cys Arg Asn Gly
   1               5                   10                  15
   Arg Cys Phe Asn Glu Ile Gly Ser Phe Lys Cys Leu Cys Asn Glu Gly
               20                  25                  30
   Tyr Glu Leu Thr Pro Asp Gly Lys Asn Cys Ile
           35                  40
<210> 348
   <211> 40
   <212> PRT
   <213> Homo sapiens
<400> 348
   Asp Thr Asn Glu Cys Val Ala Leu Pro Gly Ser Cys Ser Pro Gly Thr
   1               5                   10                  15
   Cys Gln Asn Leu Glu Gly Ser Phe Arg Cys Ile Cys Pro Pro Gly Tyr
               20                  25                  30
   Glu Val Lys Ser Glu Asn Cys Ile
           35                  40
<210> 349
   <211> 42
   <212> PRT
   <213> Homo sapiens
<400> 349
   Asp Ile Asn Glu Cys Asp Glu Asp Pro Asn Ile Cys Leu Phe Gly Ser
   1               5                   10                  15
   Cys Thr Asn Thr Pro Gly Gly Phe Gln Cys Leu Cys Pro Pro Gly Phe
               20                  25                  30
   Val Leu Ser Asp Asn Gly Arg Arg Cys Phe
           35                  40
<210> 350
   <211> 42
   <212> PRT
   <213> Homo sapiens
<400> 350
   Asp Val Asn Glu Cys Leu Glu Ser Pro Gly Ile Cys Ser Asn Gly Gln
   1 5 10 15
   Cys Ile Asn Thr Asp Gly Ser Phe Arg Cys Glu Cys Pro Met Gly Tyr
   20 25 30
   Asn Leu Asp Tyr Thr Gly Val Arg Cys Val
   35 40
<210> 351
   <211> 40
   <212> PRT
   <213> Homo sapiens
<400> 351
   Asp Thr Asp Glu Cys Ser Ile Gly Asn Pro Cys Gly Asn Gly Thr Cys
   1 5 10 15
   Thr Asn Val Ile Gly Ser Phe Glu Cys Asn Cys Asn Glu Gly Phe Glu
   20 25 30
   Pro Gly Pro Met Met Asn Cys Glu
   35 40
<210> 352
   <211> 41
   <212> PRT
   <213> Homo sapiens
<400> 352
   Asp Ile Asn Glu Cys Ala Gln Asn Pro Leu Leu Cys Ala Phe Arg Cys
   1 5 10 15
   Met Asn Thr Phe Gly Ser Tyr Glu Cys Thr Cys Pro Ile Gly Tyr Ala
   20 25 30
   Leu Arg Glu Asp Gln Lys Met Cys Lys
   35 40
<210> 353
   <211> 44
   <212> PRT
   <213> Homo sapiens
<400> 353
   Asp Leu Asp Glu Cys Ala Glu Gly Leu His Asp Cys Glu Ser Arg Gly
   1 5 10 15
   Met Met Cys Lys Asn Leu Ile Gly Thr Phe Met Cys Ile Cys Pro Pro
   20 25 30
   Gly Met Ala Arg Arg Pro Asp Gly Glu Gly Cys Val
   35 40
<210> 354
   <211> 42
   <212> PRT
   <213> Homo sapiens
<400> 354
   Asp Val Asp Glu Cys Gln Ala Ile Pro Gly Ile Cys Gln Gly Gly Asn
   1 5 10 15
   Cys Ile Asn Thr Val Gly Ser Phe Glu Cys Arg Cys Pro Ala Gly His
   20 25 30
   Lys Gln Ser Glu Thr Thr Gln Lys Cys Glu
   35 40
<210> 355
   <211> 42
   <212> PRT
   <213> Homo sapiens
<400> 355
   Asp Glu Asn Glu Cys Arg Thr Lys Pro Gly Ile Cys Glu Asn Gly Arg
   1 5 10 15
   Cys Val Asn Ile Ile Gly Ser Tyr Arg Cys Glu Cys Asn Glu Gly Phe
   20 25 30
   Gln Ser Ser Ser Ser Gly Thr Glu Cys Leu
   35 40
<210> 356
   <211> 42
   <212> PRT
   <213> Homo sapiens
<400> 356
   Asp Ile Asp Glu Cys Lys Val Met Pro Asn Leu Cys Thr Asn Gly Gln
   1 5 10 15
   Cys Ile Asn Thr Met Gly Ser Phe Arg Cys Phe Cys Lys Val Gly Tyr
   20 25 30
   Thr Thr Asp Ile Ser Gly Thr Ser Cys Ile
   35 40
<210> 357
   <211> 41
   <212> PRT
   <213> Homo sapiens
<400> 357
   Asp Leu Asp Glu Cys Ser Gln Ser Pro Lys Pro Cys Asn Tyr Ile Cys
   1 5 10 15
   Lys Asn Thr Glu Gly Ser Tyr Gln Cys Ser Cys Pro Arg Gly Tyr Val
   20 25 30
   Leu Gln Glu Asp Gly Lys Thr Cys Lys
   35 40
<210> 358
   <211> 38
   <212> PRT
   <213> Homo sapiens
<400> 358
   Asp Leu Asp Glu Cys Gln Thr Lys Gln His Asn Cys Gln Phe Leu Cys
   1               5                   10                  15
   Val Asn Thr Leu Gly Gly Phe Thr Cys Lys Cys Pro Pro Gly Phe Thr
               20                  25                  30
   Gln His His Thr Ala Cys
           35
<210> 359
   <211> 43
   <212> PRT
   <213> Homo sapiens
<400> 359
   Asp Asn Asn Glu Cys Gly Ser Gln Pro Ser Leu Cys Gly Ala Lys Gly
   1               5                   10                  15
   Ile Cys Gln Asn Thr Pro Gly Ser Phe Ser Cys Glu Cys Gln Arg Gly
               20                  25                  30
   Phe Ser Leu Asp Ala Thr Gly Leu Asn Cys Glu
           35                  40
<210> 360
   <211> 40
   <212> PRT
   <213> Homo sapiens
<400> 360
   Asp Val Asp Glu Cys Asp Gly Asn His Arg Cys Gln His Gly Cys Gln
   1               5                   10                  15
   Asn Ile Leu Gly Gly Tyr Arg Cys Gly Cys Pro Gln Gly Tyr Ile Gln
               20                  25                  30
   His Tyr Gln Trp Asn Gln Cys Val
   35 40
<210> 361
   <211> 41
   <212> PRT
   <213> Homo sapiens
<400> 361
   Asp Glu Asn Glu Cys Ser Asn Pro Asn Ala Cys Gly Ser Ala Ser Cys
   1               5                   10                  15
   Tyr Asn Thr Leu Gly Ser Tyr Lys Cys Ala Cys Pro Ser Gly Phe Ser
               20                  25                  30
   Phe Asp Gln Phe Ser Ser Ala Cys His
           35                  40
<210> 362
   <211> 40
   <212> PRT
   <213> Homo sapiens
<400> 362
   Asp Val Asn Glu Cys Ser Ser Ser Lys Asn Pro Cys Asn Tyr Gly Cys
   1 5 10 15
   Ser Asn Thr Glu Gly Gly Tyr Leu Cys Gly Cys Pro Pro Gly Tyr Tyr
   20 25 30
   Arg Val Gly Gln Gly His Cys Val
   35 40
<210> 363
   <211> 42
   <212> PRT
   <213> Homo sapiens
<400> 363
   Asp Ile Asp Glu Cys Ser Ile Ile Pro Gly Ile Cys Glu Thr Gly Glu
   1 5 10 15
   Cys Ser Asn Thr Val Gly Ser Tyr Phe Cys Val Cys Pro Arg Gly Tyr
   20 25 30
   Val Thr Ser Thr Asp Gly Ser Arg Cys Ile
   35 40
<210> 364
   <211> 41
   <212> PRT
   <213> Homo sapiens
<400> 364
   Thr Ile Asp Ile Cys Lys His His Ala Asn Leu Cys Leu Asn Gly Arg
   1 5 10 15
   Cys Ile Pro Thr Val Ser Ser Tyr Arg Cys Glu Cys Asn Met Gly Tyr
   20 25 30
   Lys Gln Asp Ala Asn Gly Asp Cys Ile
   35 40
<210> 365
   <211> 40
   <212> PRT
   <213> Homo sapiens
<400> 365
   Asp Val Asp Glu Cys Thr Ser Asn Pro Cys Thr Asn Gly Asp Cys Val
   1 5 10 15
   Asn Thr Pro Gly Ser Tyr Tyr Cys Lys Cys His Ala Gly Phe Gln Arg
   20 25 30
   Thr Pro Thr Lys Gln Ala Cys Ile
   35 40
<210> 366
   <211> 42
   <212> PRT
   <213> Homo sapiens
<400> 366
   Asp Ile Asp Glu Cys Ile Gln Asn Gly Val Leu Cys Lys Asn Gly Arg
   1               5                   10                  15
   Cys Val Asn Thr Asp Gly Ser Phe Gln Cys Ile Cys Asn Ala Gly Phe
               20                  25                  30
   Glu Leu Thr Thr Asp Gly Lys Asn Cys Val
           35                  40
<210> 367
   <211> 41
   <212> PRT
   <213> Homo sapiens
<400> 367
   Asp His Asp Glu Cys Thr Thr Thr Asn Met Cys Leu Asn Gly Met Cys
   1               5                   10                  15
   Ile Asn Glu Asp Gly Ser Phe Lys Cys Ile Cys Lys Pro Gly Phe Val
               20                  25                  30
   Leu Ala Pro Asn Gly Arg Tyr Cys Thr
           35                  40
<210> 368
   <211> 33
   <212> PRT
   <213> Homo sapiens
<400> 368
   Gly Gln Pro Ile Cys Asp Arg Gly Cys His Asn Gly Gly Arg Cys Ile
   1               5                   10                  15
   Gly Pro Asn Arg Cys Ala Cys Val Tyr Gly Phe Met Gly Pro Gln Cys
               20                  25                  30
   Glu
<210> 369
   <211> 40
   <212> PRT
   <213> Homo sapiens
<400> 369
   Asp Val Asp Glu Cys Leu Ser Ser Pro Cys Val Ser Gly Val Cys Arg
   1               5                   10                  15
   Asn Leu Ala Gly Ser Tyr Thr Cys Lys Cys Gly Pro Gly Ser Arg Leu
               20                  25                  30
   Asp Pro Ser Gly Thr Phe Cys Leu
           35                  40
<210> 370
   <211> 42
   <212> PRT
   <213> Homo sapiens
<400> 370
   Asp Val Asn Glu Cys Glu Ser Phe Pro Gly Val Cys Pro Asn Gly Arg
   1               5                   10                  15
   Cys Val Asn Thr Ala Gly Ser Phe Arg Cys Glu Cys Pro Glu Gly Leu
               20                  25                  30
   Met Leu Asp Ala Ser Gly Arg Leu Cys Val
           35                  40
<210> 371
   <211> 42
   <212> PRT
   <213> Homo sapiens
<400> 371
   Asp Val Asn Glu Cys Lys Val Phe Pro Gly Leu Cys Thr His Gly Thr
   1               5                   10                  15
   Cys Arg Asn Thr Val Gly Ser Phe His Cys Ala Cys Ala Gly Gly Phe
               20                  25                  30
   Ala Leu Asp Ala Gln Glu Arg Asn Cys Thr
           35                  40
<210> 372
   <211> 43
   <212> PRT
   <213> Homo sapiens
<400> 372
   Asp Ile Asp Glu Cys Arg Ile Ser Pro Asp Leu Cys Gly Gln Gly Thr
   1               5                   10                  15
   Cys Val Asn Thr Pro Gly Ser Phe Glu Cys Glu Cys Phe Pro Gly Tyr
               20                  25                  30
   Glu Ser Gly Phe Met Leu Met Lys Asn Cys Met
           35                  40
<210> 373
   <211> 42
   <212> PRT
   <213> Homo sapiens
<400> 373
   Asp Val Asp Glu Cys Ala Arg Asp Pro Leu Leu Cys Arg Gly Gly Thr
   1               5                   10                  15
   Cys Thr Asn Thr Asp Gly Ser Tyr Lys Cys Gln Cys Pro Pro Gly His
               20                  25                  30
   Glu Leu Thr Ala Lys Gly Thr Ala Cys Glu
           35                  40
<210> 374
   <211> 42
   <212> PRT
   <213> Homo sapiens
<400> 374
   Asp Ile Asp Glu Cys Ser Leu Ser Asp Gly Leu Cys Pro His Gly Gln
   1               5                   10                  15
   Cys Val Asn Val Ile Gly Ala Phe Gln Cys Ser Cys His Ala Gly Phe
               20                  25                  30
   Gln Ser Thr Pro Asp Arg Gln Gly Cys Val
           35                  40
<210> 375
   <211> 41
   <212> PRT
   <213> Homo sapiens
<400> 375
   Asp Ile Asn Glu Cys Arg Val Gln Asn Gly Gly Cys Asp Val His Cys
   1               5                   10                  15
   Ile Asn Thr Glu Gly Ser Tyr Arg Cys Ser Cys Gly Gln Gly Tyr Ser
               20                  25                  30
   Leu Met Pro Asp Gly Arg Ala Cys Ala
           35                  40
<210> 376
   <211> 42
   <212> PRT
   <213> Homo sapiens
<400> 376
   Asp Val Asp Glu Cys Glu Glu Asn Pro Arg Val Cys Asp Gln Gly His
   1               5                   10                  15
   Cys Thr Asn Met Pro Gly Gly His Arg Cys Leu Cys Tyr Asp Gly Phe
               20                  25                  30
   Met Ala Thr Pro Asp Met Arg Thr Cys Val
           35                  40
<210> 377
   <211> 42
   <212> PRT
   <213> Homo sapiens
<400> 377
   Asp Val Asp Glu Cys Asp Leu Asn Pro His Ile Cys Leu His Gly Asp
   1               5                   10                  15
   Cys Glu Asn Thr Lys Gly Ser Phe Val Cys His Cys Gln Leu Gly Tyr
               20                  25                  30
   Met Val Arg Lys Gly Ala Thr Gly Cys Ser
           35                  40
<210> 378
   <211> 41
   <212> PRT
   <213> Homo sapiens
<400> 378
   Asp Val Asp Glu Cys Glu Val Gly Gly His Asn Cys Asp Ser His Ala
   1               5                   10                  15
   Ser Cys Leu Asn Ile Pro Gly Ser Phe Ser Cys Arg Cys Leu Pro Gly
               20                  25                  30
   Trp Val Gly Asp Gly Phe Glu Cys His
           35                  40
<210> 379
   <211> 42
   <212> PRT
   <213> Homo sapiens
<400> 379
   Asp Val Asp Glu Cys Gln Ala Val Pro Gly Leu Cys Gln Gly Gly Ser
   1               5                   10                  15
   Cys Val Asn Met Val Gly Ser Phe His Cys Arg Cys Pro Val Gly His
               20                  25                  30
   Arg Leu Ser Asp Ser Ser Ala Ala Cys Glu
           35                  40
<210> 380
   <211> 41
   <212> PRT
   <213> Homo sapiens
<400> 380
   Asp Leu Asp Glu Cys Val Ser Gln Glu His Arg Cys Ser Pro Arg Gly
   1               5                   10                  15
   Asp Cys Leu Asn Val Pro Gly Ser Tyr Arg Cys Thr Cys Arg Gln Gly
               20                  25                  30
   Phe Ala Gly Asp Gly Phe Phe Cys Glu
           35                  40
<210> 381
   <211> 42
   <212> PRT
   <213> Homo sapiens
<400> 381
   Asp Arg Asp Glu Cys Ala Glu Asn Val Asp Leu Cys Asp Asn Gly Gln
   1               5                   10                  15
   Cys Leu Asn Ala Pro Gly Gly Tyr Arg Cys Glu Cys Glu Met Gly Phe
               20                  25                  30
   Asp Pro Thr Glu Asp His Arg Ala Cys Gln
           35                  40
<210> 382
   <211> 41
   <212> PRT
   <213> Homo sapiens
<400> 382
   Asp Val Asp Glu Cys Ala Gln Gly Asn Leu Cys Ala Phe Gly Ser Cys
   1               5                   10                  15
   Glu Asn Leu Pro Gly Met Phe Arg Cys Ile Cys Asn Gly Gly Tyr Glu
               20                  25                  30
   Leu Asp Arg Gly Gly Gly Asn Cys Thr
           35                  40
<210> 383
   <211> 41
   <212> PRT
   <213> Homo sapiens
<400> 383
   Asp Ile Asn Glu Cys Ala Asp Pro Val Asn Cys Ile Asn Gly Val Cys
   1               5                   10                  15
   Ile Asn Thr Pro Gly Ser Tyr Leu Cys Ser Cys Pro Gln Asp Phe Glu
               20                  25                  30
   Leu Asn Pro Ser Gly Val Gly Cys Val
           35                  40
<210> 384
   <211> 42
   <212> PRT
   <213> Homo sapiens
<400> 384
   Asp Ile Asp Glu Cys Gln Glu Leu Pro Gly Leu Cys Gln Gly Gly Asp
   1               5                   10                  15
   Cys Val Asn Thr Phe Gly Ser Phe Gln Cys Glu Cys Pro Pro Gly Tyr
               20                  25                  30
   His Leu Ser Glu His Thr Arg Ile Cys Glu
           35                  40
<210> 385
   <211> 42
   <212> PRT
   <213> Homo sapiens
<400> 385
   Asp Ile Asp Glu Cys Ser Thr His Ser Gly Ile Cys Gly Pro Gly Thr
   1               5                   10                  15
   Cys Tyr Asn Thr Leu Gly Asn Tyr Thr Cys Val Cys Pro Ala Glu Tyr
               20                  25                  30
   Leu Gln Val Asn Gly Gly Asn Asn Cys Met
           35                  40
<210> 386
   <211> 42
   <212> PRT
   <213> Homo sapiens
<400> 386
   Asp Ile Asp Glu Cys Gly Glu Ile Pro Ala Ile Cys Ala Asn Gly Ile
   1               5                   10                  15
   Cys Ile Asn Gln Ile Gly Ser Phe Arg Cys Glu Cys Pro Ala Gly Phe
               20                  25                  30
   Asn Tyr Asn Ser Ile Leu Leu Ala Cys Glu
           35                  40
<210> 387
   <211> 42
   <212> PRT
   <213> Homo sapiens
<400> 387
   Asp Val Asp Glu Cys Gly Ser Arg Glu Ser Pro Cys Gln Gln Asn Ala
   1               5                   10                  15
   Asp Cys Ile Asn Ile Pro Gly Ser Tyr Arg Cys Lys Cys Thr Arg Gly
               20                  25                  30
   Tyr Lys Leu Ser Pro Gly Gly Ala Cys Val
           35                  40
<210> 388
   <211> 42
   <212> PRT
   <213> Homo sapiens
<400> 388
   Gly Arg Asn Glu Cys Arg Glu Ile Pro Asn Val Cys Ser His Gly Asp
   1               5                   10                  15
   Cys Met Asp Thr Glu Gly Ser Tyr Met Cys Leu Cys His Arg Gly Phe
               20                  25                  30
   Gln Ala Ser Ala Asp Gln Thr Leu Cys Met
           35                  40
<210> 389
   <211> 39
   <212> PRT
   <213> Homo sapiens
<400> 389
   Asp Ile Asp Glu Cys Asp Arg Gln Pro Cys Gly Asn Gly Thr Cys Lys
   1               5                   10                  15
   Asn Ile Ile Gly Ser Tyr Asn Cys Leu Cys Phe Pro Gly Phe Val Val
               20                  25                  30
   Thr His Asn Gly Asp Cys Val
           35
<210> 390
   <211> 41
   <212> PRT
   <213> Homo sapiens
<400> 390
   Thr Ile Asp Ile Cys Arg His Phe Thr Asn Leu Cys Leu Asn Gly Arg
   1 5 10 15
   Cys Leu Pro Thr Pro Ser Ser Tyr Arg Cys Glu Cys Asn Val Gly Tyr
   20 25 30
   Thr Gln Asp Val Arg Gly Glu Cys Ile
   35 40
<210> 391
   <211> 43
   <212> PRT
   <213> Homo sapiens
<400> 391
   Asp Phe Asp Glu Cys Thr Thr Leu Val Gly Gln Val Cys Arg Phe Gly
   1 5 10 15
   His Cys Leu Asn Thr Ala Gly Ser Phe His Cys Leu Cys Gln Asp Gly
   20 25 30
   Phe Glu Leu Thr Ala Asp Gly Lys Asn Cys Val
   35 40
<210> 392
   <211> 40
   <212> PRT
   <213> Homo sapiens
<400> 392
   Asp Thr Asn Glu Cys Leu Ser Leu Ala Gly Thr Cys Leu Pro Gly Thr
   1 5 10 15
   Cys Gln Asn Leu Glu Gly Ser Phe Arg Cys Ile Cys Pro Pro Gly Phe
   20 25 30
   Gln Val Gln Ser Asp His Cys Ile
   35 40
<210> 393
   <211> 42
   <212> PRT
   <213> Homo sapiens
<400> 393
   Asp Ile Asp Glu Cys Ser Glu Glu Pro Asn Leu Cys Leu Phe Gly Thr
   1 5 10 15
   Cys Thr Asn Ser Pro Gly Ser Phe Gln Cys Leu Cys Pro Pro Gly Phe
   20 25 30
   Val Leu Ser Asp Asn Gly His Arg Cys Phe
   35 40
<210> 394
   <211> 42
   <212> PRT
   <213> Homo sapiens
<400> 394
   Asp Val Asn Glu Cys Ala Glu Asn Pro Gly Val Cys Thr Asn Gly Val
   1               5                   10                  15
   Cys Val Asn Thr Asp Gly Ser Phe Arg Cys Glu Cys Pro Phe Gly Tyr
               20                  25                  30
   Ser Leu Asp Phe Thr Gly Ile Asn Cys Val
           35                  40
<210> 395
   <211> 40
   <212> PRT
   <213> Homo sapiens
<400> 395
   Asp Thr Asp Glu Cys Ser Val Gly His Pro Cys Gly Gln Gly Thr Cys
   1               5                   10                  15
   Thr Asn Val Ile Gly Gly Phe Glu Cys Ala Cys Ala Asp Gly Phe Glu
               20                  25                  30
   Pro Gly Leu Met Met Thr Cys Glu
           35                  40
<210> 396
   <211> 41
   <212> PRT
   <213> Homo sapiens
<400> 396
   Asp Ile Asp Glu Cys Ser Leu Asn Pro Leu Leu Cys Ala Phe Arg Cys
   1               5                   10                  15
   His Asn Thr Glu Gly Ser Tyr Leu Cys Thr Cys Pro Ala Gly Tyr Thr
               20                  25                  30
   Leu Arg Glu Asp Gly Ala Met Cys Arg
           35                  40
<210> 397
   <211> 45
   <212> PRT
   <213> Homo sapiens
<400> 397
   Asp Val Asp Glu Cys Ala Asp Gly Gln Gln Asp Cys His Ala Arg Gly
   1               5                   10                  15
   Met Glu Cys Lys Asn Leu Ile Gly Thr Phe Ala Cys Val Cys Pro Pro
               20                  25                  30
   Gly Met Arg Pro Leu Pro Gly Ser Gly Glu Gly Cys Thr
           35                  40                  45
<210> 398
   <211> 42
   <212> PRT
   <213> Homo sapiens
<400> 398
   Asp Asp Asn Glu Cys His Ala Gln Pro Asp Leu Cys Val Asn Gly Arg
   1               5                   10                  15
   Cys Val Asn Thr Ala Gly Ser Phe Arg Cys Asp Cys Asp Glu Gly Phe
               20                  25                  30
   Gln Pro Ser Pro Thr Leu Thr Glu Cys His
           35                  40
<210> 399
   <211> 42
   <212> PRT
   <213> Homo sapiens
<400> 399
   Asp Val Asp Glu Cys Arg Met Leu Ala His Leu Cys Ala His Gly Glu
   1               5                   10                  15
   Cys Ile Asn Ser Leu Gly Ser Phe Arg Cys His Cys Gln Ala Gly Tyr
               20                  25                  30
   Thr Pro Asp Ala Thr Ala Thr Thr Cys Leu
           35                  40
<210> 400
   <211> 41
   <212> PRT
   <213> Homo sapiens
<400> 400
   Asp Met Asp Glu Cys Ser Gln Val Pro Lys Pro Cys Thr Phe Leu Cys
   1               5                   10                  15
   Lys Asn Thr Lys Gly Ser Phe Leu Cys Ser Cys Pro Arg Gly Tyr Leu
               20                  25                  30
   Leu Glu Glu Asp Gly Arg Thr Cys Lys
           35                  40
<210> 401
   <211> 40
   <212> PRT
   <213> Homo sapiens
<400> 401
   Asp Val Asp Glu Cys Thr Ser Ser Pro Cys His His Gly Asp Cys Val
   1               5                   10                  15
   Asn Ile Pro Gly Thr Tyr His Cys Arg Cys Tyr Pro Gly Phe Gln Ala
               20                  25                  30
   Thr Pro Thr Arg Gln Ala Cys Val
           35                  40
<210> 402
   <211> 39
   <212> PRT
   <213> Homo sapiens
<400> 402
   Asp Leu Asp Glu Cys Thr Ser Arg Gln His Asn Cys Gln Phe Leu Cys
   1               5                   10                  15
   Val Asn Thr Val Gly Ala Phe Thr Cys Arg Cys Pro Pro Gly Phe Thr
               20                  25                  30
   Gln His His Gln Ala Cys Phe
           35
<210> 403
   <211> 43
   <212> PRT
   <213> Homo sapiens
<400> 403
   Asp Asn Asp Glu Cys Ser Ala Gln Pro Gly Pro Cys Gly Ala His Gly
   1               5                   10                  15
   His Cys His Asn Thr Pro Gly Ser Phe Arg Cys Glu Cys His Gln Gly
               20                  25                  30
   Phe Thr Leu Val Ser Ser Gly His Gly Cys Glu
           35                  40
<210> 404
   <211> 40
   <212> PRT
   <213> Homo sapiens
<400> 404
   Asp Val Asn Glu Cys Asp Gly Pro His Arg Cys Gln His Gly Cys Gln
   1               5                   10                  15
   Asn Gln Leu Gly Gly Tyr Arg Cys Ser Cys Pro Gln Gly Phe Thr Gln
               20                  25                  30
   His Ser Gln Trp Ala Gln Cys Val
           35                  40
<210> 405
   <211> 42
   <212> PRT
   <213> Homo sapiens
<400> 405
   Asp Glu Asn Glu Cys Ala Leu Ser Pro Pro Thr Cys Gly Ser Ala Ser
   1               5                   10                  15
   Cys Arg Asn Thr Leu Gly Gly Phe Arg Cys Val Cys Pro Ser Gly Phe
               20                  25                  30
   Asp Phe Asp Gln Ala Leu Gly Gly Cys Gln
           35                  40
<210> 406
   <211> 40
   <212> PRT
   <213> Homo sapiens
<400> 406
   Glu Val Asp Glu Cys Ala Gly Arg Arg Gly Pro Cys Ser Tyr Ser Cys
   1               5                   10                  15
   Ala Asn Thr Pro Gly Gly Phe Leu Cys Gly Cys Pro Gln Gly Tyr Phe
               20                  25                  30
   Arg Ala Gly Gln Gly His Cys Val
           35                  40
<210> 407
   <211> 42
   <212> PRT
   <213> Homo sapiens
<400> 407
   Asp Val Asp Glu Cys Ile Val Ser Gly Gly Leu Cys His Leu Gly Arg
   1               5                   10                  15
   Cys Val Asn Thr Glu Gly Ser Phe Gln Cys Val Cys Asn Ala Gly Phe
               20                  25                  30
   Glu Leu Ser Pro Asp Gly Lys Asn Cys Val
           35                  40
<210> 408
   <211> 41
   <212> PRT
   <213> Homo sapiens
<400> 408
   Asp His Asn Glu Cys Ala Thr Ser Thr Met Cys Val Asn Gly Val Cys
   1               5                   10                  15
   Leu Asn Glu Asp Gly Ser Phe Ser Cys Leu Cys Lys Pro Gly Phe Leu
               20                  25                  30
   Leu Ala Pro Gly Gly His Tyr Cys Met
           35                  40
<210> 409
   <211> 41
   <212> PRT
   <213> Homo sapiens
<400> 409
   Asp Ile Asp Glu Cys Gln Thr Pro Gly Ile Cys Val Asn Gly His Cys
   1               5                   10                  15
   Thr Asn Thr Glu Gly Ser Phe Arg Cys Gln Cys Leu Gly Gly Leu Ala
               20                  25                  30
   Val Gly Thr Asp Gly Arg Val Cys Val
           35                  40
<210> 410
   <211> 42
   <212> PRT
   <213> Homo sapiens
<400> 410
   Asp Ile Asn Glu Cys Ala Leu Asp Pro Glu Val Cys Ala Asn Gly Val
   1 5 10 15
   Cys Glu Asn Leu Arg Gly Ser Tyr Arg Cys Val Cys Asn Leu Gly Tyr
   20 25 30
   Glu Ala Gly Ala Ser Gly Lys Asp Cys Thr
   35 40
<210> 411
   <211> 42
   <212> PRT
   <213> Homo sapiens
<400> 411
   Asp Val Asp Glu Cys Ala Leu Asn Ser Leu Leu Cys Asp Asn Gly Trp
   1 5 10 15
   Cys Gln Asn Ser Pro Gly Ser Tyr Ser Cys Ser Cys Pro Pro Gly Phe
   20 25 30
   His Phe Trp Gln Asp Thr Glu Ile Cys Lys
   35 40
<210> 412
   <211> 40
   <212> PRT
   <213> Homo sapiens
<400> 412
   Leu Asn Asp Arg Cys Arg Gly Gly Gly Pro Cys Lys Gln Gln Cys Arg
   1 5 10 15
   Asp Thr Gly Asp Glu Val Val Cys Ser Cys Phe Val Gly Tyr Gln Leu
   20 25 30
   Leu Ser Asp Gly Val Ser Cys Glu
   35 40
<210> 413
   <211> 46
   <212> PRT
   <213> Homo sapiens
<400> 413
   Asp Val Asn Glu Cys Ile Thr Gly Ser His Ser Cys Arg Leu Gly Glu
   1 5 10 15
   Ser Cys Ile Asn Thr Val Gly Ser Phe Arg Cys Gln Arg Asp Ser Ser
   20 25 30
   Cys Gly Thr Gly Tyr Glu Leu Thr Glu Asp Asn Ser Cys Lys
   35 40 45
<210> 414
   <211> 46
   <212> PRT
   <213> Homo sapiens
<400> 414
   Asp Ile Asp Glu Cys Glu Ser Gly Ile His Asn Cys Leu Pro Asp Phe
   1               5                   10                  15
   Ile Cys Gln Asn Thr Leu Gly Ser Phe Arg Cys Arg Pro Lys Leu Gln
               20                  25                  30
   Cys Lys Ser Gly Phe Ile Gln Asp Ala Leu Gly Asn Cys Ile
           35                  40                  45
<210> 415
   <211> 48
   <212> PRT
   <213> Homo sapiens
<400> 415
   Asp Ile Asn Glu Cys Leu Ser Ile Ser Ala Pro Cys Pro Ile Gly His
   1               5                   10                  15
   Thr Cys Ile Asn Thr Glu Gly Ser Tyr Thr Cys Gln Lys Asn Val Pro
               20                  25                  30
   Asn Cys Gly Arg Gly Tyr His Leu Asn Glu Glu Gly Thr Arg Cys Val
           35                  40                  45
<210> 416
   <211> 43
   <212> PRT
   <213> Homo sapiens
<400> 416
   Asp Val Asp Glu Cys Ala Pro Pro Ala Glu Pro Cys Gly Lys Gly His
   1               5                   10                  15
   Arg Cys Val Asn Ser Pro Gly Ser Phe Arg Cys Glu Cys Lys Thr Gly
               20                  25                  30
   Tyr Tyr Phe Asp Gly Ile Ser Arg Met Cys Val
           35                  40
<210> 417
   <211> 42
   <212> PRT
   <213> Homo sapiens
<400> 417
   Asp Val Asn Glu Cys Gln Arg Tyr Pro Gly Arg Leu Cys Gly His Lys
   1               5                   10                  15
   Cys Glu Asn Thr Leu Gly Ser Tyr Leu Cys Ser Cys Ser Val Gly Phe
               20                  25                  30
   Arg Leu Ser Val Asp Gly Arg Ser Cys Glu
           35                  40
<210> 418
   <211> 40
   <212> PRT
   <213> Homo sapiens
<400> 418
   Asp Ile Asn Glu Cys Ser Ser Ser Pro Cys Ser Gln Glu Cys Ala Asn
   1               5                   10                  15
   Val Tyr Gly Ser Tyr Gln Cys Tyr Cys Arg Arg Gly Tyr Gln Leu Ser
               20                  25                  30
   Asp Val Asp Gly Val Thr Cys Glu
           35                  40
<210> 419
   <211> 44
   <212> PRT
   <213> Homo sapiens
<400> 419
   Asp Ile Asp Glu Cys Ala Leu Pro Thr Gly Gly His Ile Cys Ser Tyr
   1               5                   10                  15
   Arg Cys Ile Asn Ile Pro Gly Ser Phe Gln Cys Ser Cys Pro Ser Ser
               20                  25                  30
   Gly Tyr Arg Leu Ala Pro Asn Gly Arg Asn Cys Gln
           35                  40
<210> 420
   <211> 54
   <212> PRT
   <213> Homo sapiens
<400> 420
   Asp Ile Asp Glu Cys Val Thr Gly Ile His Asn Cys Ser Ile Asn Glu
   1               5                   10                  15
   Thr Cys Phe Asn Ile Gln Gly Gly Phe Arg Cys Leu Ala Phe Glu Cys
               20                  25                  30
   Pro Glu Asn Tyr Arg Arg Ser Ala Ala Thr Leu Gln Gln Glu Lys Thr
           35                  40                  45
   Asp Thr Val Arg Cys Ile
       50
<210> 421
   <211> 42
   <212> PRT
   <213> Homo sapiens
<400> 421
   Asp Gln Asp Glu Cys Leu Leu Leu Pro Gly Glu Leu Cys Gln His Leu
   1               5                   10                  15
   Cys Ile Asn Thr Val Gly Ser Tyr His Cys Ala Cys Phe Pro Gly Phe
               20                  25                  30
   Ser Leu Gln Asp Asp Gly Arg Thr Cys Arg
           35                  40
<210> 422
   <211> 45
   <212> PRT
   <213> Homo sapiens
<400> 422
   Asp Val Asp Glu Cys Ala Leu Gly Thr His Asn Cys Ser Glu Ala Glu
   1               5                   10                  15
   Thr Cys His Asn Ile Gln Gly Ser Phe Arg Cys Leu Arg Phe Glu Cys
               20                  25                  30
   Pro Pro Asn Tyr Val Gln Val Ser Lys Thr Lys Cys Glu
           35                  40                  45
<210> 423
   <211> 40
   <212> PRT
   <213> Homo sapiens
<400> 423
   Gln Pro Asn Thr Cys Lys Asp Asn Gly Pro Cys Lys Gln Val Cys Ser
   1               5                   10                  15
   Thr Val Gly Gly Ser Ala Ile Cys Ser Cys Phe Pro Gly Tyr Ala Ile
                   20                  25                  30
   Met Ala Asp Gly Val Ser Cys Glu
           35                  40
<210> 424
   <211> 45
   <212> PRT
   <213> Homo sapiens
<400> 424
   Asp Ile Asn Glu Cys Val Thr Asp Leu His Thr Cys Ser Arg Gly Glu
   1               5                   10                  15
   His Cys Val Asn Thr Leu Gly Ser Phe His Cys Tyr Lys Ala Leu Thr
               20                  25                  30
   Cys Glu Pro Gly Tyr Ala Leu Lys Asp Gly Glu Cys Glu
           35                  40                  45
<210> 425
   <211> 46
   <212> PRT
   <213> Homo sapiens
<400> 425
   Asp Val Asp Glu Cys Ala Met Gly Thr His Thr Cys Gln Pro Gly Phe
   1               5                   10                  15
   Leu Cys Gln Asn Thr Lys Gly Ser Phe Tyr Cys Gln Ala Arg Gln Arg
               20                  25                  30
   Cys Met Asp Gly Phe Leu Gln Asp Pro Glu Gly Asn Cys Val
           35                  40                  45
<210> 426
   <211> 48
   <212> PRT
   <213> Homo sapiens
<400> 426
   Asp Ile Asn Glu Cys Thr Ser Leu Ser Glu Pro Cys Arg Pro Gly Phe
   1               5                   10                  15
   Ser Cys Ile Asn Thr Val Gly Ser Tyr Thr Cys Gln Arg Asn Pro Leu
               20                  25                  30
   Ile Cys Ala Arg Gly Tyr His Ala Ser Asp Asp Gly Thr Lys Cys Val
           35                  40                  45
<210> 427
   <211> 43
   <212> PRT
   <213> Homo sapiens
<400> 427
   Asp Val Asn Glu Cys Glu Thr Gly Val His Arg Cys Gly Glu Gly Gln
   1               5                   10                  15
   Val Cys His Asn Leu Pro Gly Ser Tyr Arg Cys Asp Cys Lys Ala Gly
               20                  25                  30
   Phe Gln Arg Asp Ala Phe Gly Arg Gly Cys Ile
           35                  40
<210> 428
   <211> 42
   <212> PRT
   <213> Homo sapiens
<400> 428
   Asp Val Asn Glu Cys Trp Ala Ser Pro Gly Arg Leu Cys Gln His Thr
   1               5                   10                  15
   Cys Glu Asn Thr Leu Gly Ser Tyr Arg Cys Ser Cys Ala Ser Gly Phe
               20                  25                  30
   Leu Leu Ala Ala Asp Gly Lys Arg Cys Glu
           35                  40
<210> 429
   <211> 39
   <212> PRT
   <213> Homo sapiens
<400> 429
   Asp Val Asn Glu Cys Glu Ala Gln Arg Cys Ser Gln Glu Cys Ala Asn
   1               5                   10                  15
   Ile Tyr Gly Ser Tyr Gln Cys Tyr Cys Arg Gln Gly Tyr Gln Leu Ala
               20                  25                  30
   Glu Asp Gly His Thr Cys Thr
           35
<210> 430
   <211> 43
   <212> PRT
   <213> Homo sapiens
<400> 430
   Asp Ile Asp Glu Cys Ala Gln Gly Ala Gly Ile Leu Cys Thr Phe Arg
   1               5                   10                  15
   Cys Leu Asn Val Pro Gly Ser Tyr Gln Cys Ala Cys Pro Glu Gln Gly
               20                  25                  30
   Tyr Thr Met Thr Ala Asn Gly Arg Ser Cys Lys
           35                  40
<210> 431
   <211> 41
   <212> PRT
   <213> Homo sapiens
<400> 431
   Asp Ile Asp Glu Cys Arg Thr Ile Pro Glu Ala Cys Arg Gly Asp Met
   1               5                   10                  15
   Met Cys Val Asn Gln Asn Gly Gly Tyr Leu Cys Ile Pro Arg Thr Asn
               20                  25                  30
   Pro Val Tyr Arg Gly Pro Tyr Ser Asn
           35                  40
<210> 432
   <211> 41
   <212> PRT
   <213> Homo sapiens
<400> 432
   Asp Val Asp Glu Cys Ala Thr Asp Ser His Gln Cys Asn Pro Thr Gln
   1               5                   10                  15
   Ile Cys Ile Asn Thr Glu Gly Gly Tyr Thr Cys Ser Cys Thr Asp Gly
               20                  25                  30
   Tyr Trp Leu Leu Glu Gly Gln Cys Leu
           35                  40
<210> 433
   <211> 39
   <212> PRT
   <213> Homo sapiens
<400> 433
   Asp Ile Asp Glu Cys Arg Tyr Gly Tyr Cys Gln Gln Leu Cys Ala Asn
   1               5                   10                  15
   Val Pro Gly Ser Tyr Ser Cys Thr Cys Asn Pro Gly Phe Thr Leu Asn
               20                  25                  30
   Glu Asp Gly Arg Ser Cys Gln
           35
<210> 434
   <211> 40
   <212> PRT
   <213> Homo sapiens
<400> 434
   Asp Val Asn Glu Cys Ala Thr Glu Asn Pro Cys Val Gln Thr Cys Val
   1               5                   10                  15
   Asn Thr Tyr Gly Ser Phe Ile Cys Arg Cys Asp Pro Gly Tyr Glu Leu
               20                  25                  30
   Glu Glu Asp Gly Val His Cys Ser
           35                  40
<210> 435
   <211> 41
   <212> PRT
   <213> Homo sapiens
<400> 435
   Asp Met Asp Glu Cys Ser Phe Ser Glu Phe Leu Cys Gln His Glu Cys
   1               5                   10                  15
   Val Asn Gln Pro Gly Thr Tyr Phe Cys Ser Cys Pro Pro Gly Tyr Ile
               20                  25                  30
   Leu Leu Asp Asp Asn Arg Ser Cys Gln
           35                  40
<210> 436
   <211> 46
   <212> PRT
   <213> Homo sapiens
<400> 436
   Asp Ile Asn Glu Cys Glu His Arg Asn His Thr Cys Asn Leu Gln Gln
   1               5                   10                  15
   Thr Cys Tyr Asn Leu Gln Gly Gly Phe Lys Cys Ile Asp Pro Ile Arg
               20                  25                  30
   Cys Glu Glu Pro Tyr Leu Arg Ile Ser Asp Asn Arg Cys Met
           35                  40                  45
<210> 437
   <211> 40
   <212> PRT
   <213> Homo sapiens
<400> 437
   Asp Glu Asp Glu Cys Ala Ala Gly Asn Pro Cys Ser His Ser Cys His
   1               5                   10                  15
   Asn Ala Met Gly Thr Tyr Tyr Cys Ser Cys Pro Lys Gly Leu Thr Ile
               20                  25                  30
   Ala Ala Asp Gly Arg Thr Cys Gln
           35                  40
<210> 438
   <211> 45
   <212> PRT
   <213> Homo sapiens
<400> 438
   Asp Ile Asp Glu Cys Ala Leu Gly Arg His Thr Cys His Ala Gly Gln
   1               5                   10                  15
   Asp Cys Asp Asn Thr Ile Gly Ser Tyr Arg Cys Val Val Arg Cys Gly
               20                  25                  30
   Ser Gly Phe Arg Arg Thr Ser Asp Gly Leu Ser Cys Gln
           35                  40                  45
<210> 439
   <211> 38
   <212> PRT
   <213> Homo sapiens
<400> 439
   Asp Ile Asn Glu Cys Gln Glu Ser Ser Pro Cys His Gln Arg Cys Phe
   1               5                   10                  15
   Asn Ala Ile Gly Ser Phe His Cys Gly Cys Glu Pro Gly Tyr Gln Leu
               20                  25                  30
   Lys Gly Arg Lys Cys Met
           35
<210> 440
   <211> 42
   <212> PRT
   <213> Homo sapiens
<400> 440
   Asp Val Asn Glu Cys Arg Gln Asn Val Cys Arg Pro Asp Gln His Cys
   1               5                   10                  15
   Lys Asn Thr Arg Gly Gly Tyr Lys Cys Ile Asp Leu Cys Pro Asn Gly
               20                  25                  30
   Met Thr Lys Ala Glu Asn Gly Thr Cys Ile
           35                  40
<210> 441
   <211> 36
   <212> PRT
   <213> Homo sapiens
<400> 441
   Asp Ile Asp Glu Cys Lys Asp Gly Thr His Gln Cys Arg Tyr Asn Gln
   1               5                   10                  15
   Ile Cys Glu Asn Thr Arg Gly Ser Tyr Arg Cys Val Cys Pro Arg Gly
               20                  25                  30
   Tyr Arg Ser Gln
           35
<210> 442
   <211> 41
   <212> PRT
   <213> Homo sapiens
<400> 442
   Asp Ile Asn Glu Cys Glu Gln Val Pro Lys Pro Cys Ala His Gln Cys
   1               5                   10                  15
   Ser Asn Thr Pro Gly Ser Phe Lys Cys Ile Cys Pro Pro Gly Gln His
               20                  25                  30
   Leu Leu Gly Asp Gly Lys Ser Cys Ala
           35                  40
<210> 443
   <211> 40
   <212> PRT
   <213> Homo sapiens
<400> 443
   Asp Ile Asp Glu Cys Glu Asn Thr Asp Ala Cys Gln His Glu Cys Lys
   1               5                   10                  15
   Asn Thr Phe Gly Ser Tyr Gln Cys Ile Cys Pro Pro Gly Tyr Gln Leu
               20                  25                  30
   Thr His Asn Gly Lys Thr Cys Gln
           35                  40
<210> 444
   <211> 37
   <212> PRT
   <213> Homo sapiens
<400> 444
   Gly Ile Ser Glu Cys Ser Ser Gln Pro Cys Gln Asn Gly Gly Thr Cys
   1               5                   10                  15
   Val Glu Gly Val Asn Gln Tyr Arg Cys Ile Cys Pro Pro Gly Arg Thr
               20                  25                  30
   Gly Asn Arg Cys Gln
           35
<210> 445
   <211> 46
   <212> PRT
   <213> Homo sapiens
<400> 445
   Asp Val Asn Glu Cys Glu Leu Tyr Gly Gln Glu Gly Arg Pro Arg Leu
   1               5                   10                  15
   Cys Met His Ala Cys Val Asn Thr Pro Gly Ser Tyr Arg Cys Thr Cys
               20                  25                  30
   Pro Gly Gly Tyr Arg Thr Leu Ala Asp Gly Lys Ser Cys Glu
           35                  40                  45
<210> 446
   <211> 50
   <212> PRT
   <213> Homo sapiens
<400> 446
   Asp Val Asp Glu Cys Val Gly Leu Gln Pro Val Cys Pro Gln Gly Thr
   1               5                   10                  15
   Thr Cys Ile Asn Thr Gly Gly Ser Phe Gln Cys Val Ser Pro Glu Cys
               20                  25                  30
   Pro Glu Gly Ser Gly Asn Val Ser Tyr Val Lys Thr Ser Pro Phe Gln
           35                  40                  45
   Cys Glu
       50
<210> 447
   <211> 39
   <212> PRT
   <213> Homo sapiens
<400> 447
   Asp Val Asp Glu Cys Ala Gln Ala Leu His Asp Arg Arg Pro Ser Gln
   1               5                   10                  15
   Asp Cys His Asn Leu Ser Gly Ser Tyr Gln Cys Thr Cys Pro Asp Gly
               20                  25                  30
   Tyr Arg Lys Ile Gly Pro Glu
           35
<210> 448
   <211> 24
   <212> PRT
   <213> Homo sapiens
<400> 448
   Ser Phe Arg Cys Gln Cys Glu Pro Gly Phe Gln Leu Gly Pro Asn Asn
   1               5                   10                  15
   Arg Ser Cys Val Asp Val Asn Glu
               20
<210> 449
   <211> 23
   <212> PRT
   <213> Homo sapiens
<400> 449
   Phe Leu Cys Arg Cys His Gln Gly Tyr Glu Leu His Arg Asp Gly Phe
   1               5                   10                  15
   Ser Cys Ser Asp Ile Asp Glu
               20
<210> 450
   <211> 22
   <212> PRT
   <213> Homo sapiens
<400> 450
   Lys Cys Leu Cys Pro Leu Gly Phe Leu Leu Ala Asn Asp Ser Lys Thr
   1 5 10 15
   Cys Glu Asp Ile Asp Glu
   20
<210> 451
   <211> 24
   <212> PRT
   <213> Homo sapiens
<400> 451
   Ser Phe Tyr Cys Ser Cys Arg Pro Gly Tyr Lys Leu Met Ser Asp Lys
   1 5 10 15
   Arg Thr Cys Val Asp Ile Asp Glu
   20
<210> 452
   <211> 40
   <212> PRT
   <213> Homo sapiens
<400> 452
   Asp Ile Asp Glu Cys Thr Glu Met Pro Phe Val Cys Ser Gln Lys Cys
   1 5 10 15
   Glu Asn Val Ile Gly Ser Tyr Ile Cys Lys Cys Ala Pro Gly Tyr Leu
   20 25 30
   Arg Glu Pro Asp Gly Lys Thr Cys
   35 40
<210> 453
   <211> 31
   <212> PRT
   <213> Homo sapiens
<400> 453
   Asp Ile Asn Glu Cys Glu Gln Phe Gly Thr Cys Pro Gln His Cys Arg
   1 5 10 15
   Asn Thr Lys Gly Ser Tyr Glu Cys Val Cys Ala Asp Gly Phe Thr
   20 25 30
<210> 454
   <211> 39
   <212> PRT
   <213> Homo sapiens
<400> 454
   Leu Pro Asp Gln Cys Thr Pro Asn Pro Cys Asp Arg Lys Gly Thr Gln
   1 5 10 15
   Ala Cys Gln Asp Leu Met Gly Asn Phe Phe Cys Leu Cys Lys Ala Gly
   20 25 30
   Trp Gly Gly Arg Leu Cys Asp
   35
<210> 455
   <211> 41
   <212> PRT
   <213> Homo sapiens
<400> 455
   Asp Val Asn Glu Cys Ser Gln Glu Asn Gly Gly Cys Leu Gln Ile Cys
   1               5                   10                  15
   His Asn Lys Pro Gly Ser Phe His Cys Ser Cys His Ser Gly Phe Glu
               20                  25                  30
   Leu Ser Ser Asp Gly Arg Thr Cys Gln
           35                  40
<210> 456
   <211> 41
   <212> PRT
   <213> Homo sapiens
<400> 456
   Asp Ile Asp Glu Cys Ala Asp Ser Glu Ala Cys Gly Glu Ala Arg Cys
   1               5                   10                  15
   Lys Asn Leu Pro Gly Ser Tyr Ser Cys Leu Cys Asp Glu Gly Phe Ala
               20                  25                  30
   Tyr Ser Ser Gln Glu Lys Ala Cys Arg
           35                  40
<210> 457
   <211> 41
   <212> PRT
   <213> Homo sapiens
<400> 457
   Asp Val Asp Glu Cys Leu Gln Gly Arg Cys Glu Gln Val Cys Val Asn
   1               5                   10                  15
   Ser Pro Gly Ser Tyr Thr Cys His Cys Asp Gly Arg Gly Gly Leu Lys
               20                  25                  30
   Leu Ser Gln Asp Met Asp Thr Cys Glu
           35                  40
<210> 458
   <211> 28
   <212> PRT
   <213> Homo sapiens
<400> 458
   Glu Cys Ser Arg Leu Cys Arg Asn Gly Tyr Cys Thr Pro Thr Gly Lys
   1               5                   10                  15
   Cys Cys Cys Ser Pro Gly Trp Glu Gly Asp Phe Cys
               20                  25
<210> 459
   <211> 33
   <212> PRT
   <213> Homo sapiens
<400> 459
   Arg Thr Ala Lys Cys Glu Pro Ala Cys Arg His Gly Gly Val Cys Val
   1 5 10 15
   Arg Pro Asn Lys Cys Leu Cys Lys Lys Gly Tyr Leu Gly Pro Gln Cys
   20 25 30
   Glu
<210> 460
   <211> 41
   <212> PRT
   <213> Homo sapiens
<400> 460
   Asp Arg Asp Glu Cys Ser Gly Gly Pro Ser Pro Cys Ser His Ala Cys
   1 5 10 15
   Leu Asn Ala Pro Gly Arg Phe Ser Cys Thr Cys Pro Thr Gly Phe Ala
   20 25 30
   Leu Ala Trp Asp Asp Arg Asn Cys Arg
   35 40
<210> 461
   <211> 44
   <212> PRT
   <213> Homo sapiens
<400> 461
   Asp Val Asp Glu Cys Ala Trp Asp Ala His Leu Cys Arg Glu Gly Gln
   1 5 10 15
   Arg Cys Val Asn Leu Leu Gly Ser Tyr Arg Cys Leu Pro Asp Cys Gly
   20 25 30
   Pro Gly Phe Arg Val Ala Asp Gly Ala Gly Cys Glu
   35 40
<210> 462
   <211> 39
   <212> PRT
   <213> Homo sapiens
<400> 462
   Asp Val Asp Glu Cys Leu Glu Gly Leu Asp Asp Cys His Tyr Asn Gln
   1 5 10 15
   Leu Cys Glu Asn Thr Pro Gly Gly His Arg Cys Ser Cys Pro Arg Gly
   20 25 30
   Tyr Arg Met Gln Gly Pro Ser
   35
<210> 463
   <211> 41
   <212> PRT
   <213> Homo sapiens
<400> 463
   Asp Val Asn Glu Cys Leu Gln Leu Pro Lys Ala Cys Ala Tyr Gln Cys
   1               5                   10                  15
   His Asn Leu Gln Gly Ser Tyr Arg Cys Leu Cys Pro Pro Gly Gln Thr
               20                  25                  30
   Leu Leu Arg Asp Gly Lys Ala Cys Thr
           35                  40
<210> 464
   <211> 40
   <212> PRT
   <213> Homo sapiens
<400> 464
   Asp Leu Asp Glu Cys Arg Val Arg Asn Leu Cys Gln His Ala Cys Arg
   1               5                   10                  15
   Asn Thr Glu Gly Ser Tyr Gln Cys Leu Cys Pro Ala Gly Tyr Arg Leu
               20                  25                  30
   Leu Pro Ser Gly Lys Asn Cys Gln
           35                  40
<210> 465
   <211> 39
   <212> PRT
   <213> Homo sapiens
<400> 465
   Ala Leu Asp Pro Cys Ala Ser Gly Pro Cys Leu Asn Gly Gly Ser Cys
   1               5                   10                  15
   Ser Asn Thr Gln Asp Pro Gln Ser Tyr His Cys Ser Cys Pro Arg Ala
               20                  25                  30
   Phe Thr Gly Lys Asp Cys Gly
           35
<210> 466
   <211> 39
   <212> PRT
   <213> Homo sapiens
<400> 466
   Arg His Thr Ala Cys Leu Ser Ser Pro Cys Leu Asn Gly Gly Thr Cys
   1               5                   10                  15
   His Leu Ile Val Ala Thr Gly Thr Thr Val Cys Ala Cys Pro Pro Gly
               20                  25                  30
   Phe Ala Gly Arg Leu Cys Asn
           35
<210> 467
   <211> 30
   <212> PRT
   <213> Homo sapiens
<400> 467
   Glu Cys Pro Gly Gly Cys Arg Asn Gly Gly Phe Cys Asn Glu Arg Arg
   1 5 10 15
   Ile Cys Glu Cys Pro Asp Gly Phe His Gly Pro His Cys Glu
   20 25 30
<210> 468
   <211> 30
   <212> PRT
   <213> Homo sapiens
<400> 468
   Leu Cys Thr Pro Arg Cys Met Asn Gly Gly Leu Cys Val Thr Pro Gly
   1 5 10 15
   Phe Cys Ile Cys Pro Pro Gly Phe Tyr Gly Val Asn Cys Asp
   20 25 30
<210> 469
   <211> 30
   <212> PRT
   <213> Homo sapiens
<400> 469
   Asn Cys Ser Thr Thr Cys Phe Asn Gly Gly Thr Cys Phe Tyr Pro Gly
   1 5 10 15
   Lys Cys Ile Cys Pro Pro Gly Leu Glu Gly Glu Gln Cys Glu
   20 25 30
<210> 470
   <211> 30
   <212> PRT
   <213> Homo sapiens
<400> 470
   Lys Cys Pro Gln Pro Cys Arg Asn Gly Gly Lys Cys Ile Gly Lys Ser
   1 5 10 15
   Lys Cys Lys Cys Ser Lys Gly Tyr Gln Gly Asp Leu Cys Ser
   20 25 30
<210> 471
   <211> 30
   <212> PRT
   <213> Homo sapiens
<400> 471
   Val Cys Glu Pro Gly Cys Gly Ala His Gly Thr Cys His Glu Pro Asn
   1 5 10 15
   Lys Cys Gln Cys Gln Glu Gly Trp His Gly Arg His Cys Asn
   20 25 30
<210> 472
   <211> 30
   <212> PRT
   <213> Homo sapiens
<400> 472
   Pro Cys Gln Asn Gly Gly Gln Cys His Asp Ser Glu Ser Ser Ser Tyr
   1               5                   10                  15
   Val Cys Val Cys Pro Ala Gly Phe Thr Gly Ser Arg Cys Glu
               20                  25                  30
<210> 473
   <211> 30
   <212> PRT
   <213> Homo sapiens
<400> 473
   Cys Gly Pro Asp Ala Thr Cys Val Asn Arg Pro Asp Gly Arg Gly Tyr
   1               5                   10                  15
   Thr Cys Arg Cys His Leu Gly Arg Ser Gly Leu Arg Cys Glu
               20                  25                  30
<210> 474
   <211> 37
   <212> PRT
   <213> Homo sapiens
<400> 474
   Gln Ala Asp Pro Cys Gln Pro Asn Pro Cys Glu His Gly Gly Asp Cys
   1               5                   10                  15
   Leu Val His Gly Ser Thr Phe Thr Cys Ser Cys Leu Ala Pro Phe Ser
               20                  25                  30
   Gly Asn Lys Cys Gln
           35
<210> 475
   <211> 38
   <212> PRT
   <213> Homo sapiens
<400> 475
   Val Gln Asn Thr Cys Lys Asp Asn Pro Cys Gly Arg Gly Gln Cys Leu
   1               5                   10                  15
   Ile Thr Gln Ser Pro Pro Tyr Tyr Arg Cys Val Cys Lys His Pro Tyr
               20                  25                  30
   Thr Gly Pro Ser Cys Ser
           35
<210> 476
   <211> 39
   <212> PRT
   <213> Homo sapiens
<400> 476
   Val Val Pro Val Cys Arg Pro Asn Pro Cys Gln Asn Gly Ala Thr Cys
   1               5                   10                  15
   Ser Arg His Lys Arg Arg Ser Lys Phe Thr Cys Ala Cys Pro Asp Gln
               20                  25                  30
   Phe Lys Gly Lys Phe Cys Glu
           35
<210> 477
   <211> 74
   <212> PRT
   <213> Homo sapiens
<400> 477
   Leu Cys Ser Gln Ala Leu Cys Ser Gly His Gly Arg Cys Val Arg Arg
   1               5                   10                  15
   Thr Ser His Pro Lys Ala Leu Leu Leu Leu Asn Pro Ala Ser Phe Ser
               20                  25                  30
   Ile Gln Leu Thr Pro Gly Gly Gly Pro Leu Ser Leu Arg Gly Ala Leu
           35                  40                  45
   Ser Leu Gly Asp Gln Ala Gln Met Ala Val Glu Phe Lys Cys Arg Cys
       50                  55                  60
   Tyr Pro Gly Trp Gln Ala Pro Trp Cys Glu
   65                  70
<210> 478
   <211> 60
   <212> PRT
   <213> Homo sapiens
<400> 478
   Ala Thr Gln Tyr Cys Ser Arg Ala Gln Cys His Gly His Gly Arg Cys
   1               5                   10                  15
   Val Arg Arg Asn Pro Ser Ala Ser Thr Phe Leu His Leu Ser Thr Asn
               20                  25                  30
   Ser Phe Arg Leu Val Pro Gly His Ala Pro Gly Glu Pro Gln Leu Arg
           35                  40                  45
   Pro Val Gly Glu Leu Ser Trp Ala Asp Ile Asp His
       50                  55                  60
<210> 479
   <211> 56
   <212> PRT
   <213> Homo sapiens
<400> 479
   Ala Ala Met Ala Cys Ser His Gln Arg Cys His Gly His Gly Arg Cys
   1               5                   10                  15
   Ala Arg Arg Asp Pro Gly Gln Met Glu Ala Phe Leu His Leu Trp Pro
               20                  25                  30
   Asp Gly Ser Leu Gly Asp Trp Lys Ser Phe Ser Cys His Cys Tyr Trp
           35                  40                  45
   Gly Trp Ala Gly Pro Thr Cys Gln
       50                  55
<210> 480
   <211> 73
   <212> PRT
   <213> Homo sapiens
<400> 480
   Val Cys Ser Leu His Leu Cys Arg Asn Asn Gly Arg Cys Ile Arg Lys
   1               5                   10                  15
   Met Trp Asn Ala Pro Ser Tyr Leu His Leu Asn Pro Ala Ser Tyr His
               20                  25                  30
   Ile Glu Ala Ser Glu Asp Gly Glu Phe Thr Val Lys Gly Lys Ala Ser
           35                  40                  45
   Asp Thr Asp Leu Ala Val Met Ala Asp Thr Phe Ser Cys His Cys Tyr
       50                  55                  60
   Gln Gly Tyr Glu Gly Ala Asp Cys Arg
   65                  70
<210> 481
   <211> 38
   <212> PRT
   <213> Homo sapiens
<400> 481
   Glu Ile Thr Ala Cys Ser Ser Ser Pro Cys Phe His Asp Gly Thr Cys
   1               5                   10                  15
   Val Leu Asp Lys Ala Gly Ser Tyr Lys Cys Ala Cys Leu Ala Gly Tyr
               20                  25                  30
   Thr Gly Gln Arg Cys Glu
           35
<210> 482
   <211> 34
   <212> PRT
   <213> Homo sapiens
<400> 482
   Glu Cys Ile Asp Asp Glu Thr Glu Glu Ile Cys Gly Gly His Gly Lys
   1               5                   10                  15
   Cys Tyr Cys Gly Asn Cys Tyr Cys Lys Ala Gly Trp His Gly Asp Lys
               20                  25                  30
   Cys Glu
<210> 483
   <211> 37
   <212> PRT
   <213> Homo sapiens
<400> 483
   Asp Cys Arg Ala Val Tyr Asp Arg Tyr Ser Asp Asp Phe Cys Ser Gly
   1               5                   10                  15
   His Gly Gln Cys Asn Cys Gly Arg Cys Asp Cys Lys Ala Gly Trp Tyr
               20                  25                  30
   Gly Lys Lys Cys Glu
           35
<210> 484
   <211> 33
   <212> PRT
   <213> Homo sapiens
<400> 484
   Arg Cys Glu Asp Leu Asp Gly Val Val Cys Gly Gly His Gly Thr Cys
   1               5                   10                  15
   Ser Cys Gly Arg Cys Val Cys Glu Arg Gly Trp Phe Gly Lys Leu Cys
               20                  25                  30
   Gln
<210> 485
   <211> 39
   <212> PRT
   <213> Homo sapiens
<400> 485
   Gln Ala Asp Pro Cys Lys Phe Leu Ala Cys Gly Glu Phe Ala Gln Cys
   1               5                   10                  15
   Val Lys Asn Glu Arg Thr Glu Glu Ala Glu Cys Arg Cys Lys Pro Gly
               20                  25                  30
   Tyr Asp Ser Gln Gly Ser Leu
           35
<210> 486
   <211> 42
   <212> PRT
   <213> Homo sapiens
<400> 486
   Glu Ala Asn Pro Cys Lys Phe Gln Ala Cys Asn Glu Phe Ser Glu Cys
   1               5                   10                  15
   Leu Val Asn Pro Trp Ser Gly Glu Ala Lys Cys Arg Cys Phe Pro Gly
               20                  25                  30
   Tyr Leu Ser Val Glu Glu Arg Pro Cys Gln
           35                  40
<210> 487
   <211> 42
   <212> PRT
   <213> Homo sapiens
<400> 487
   Ser Leu Cys Asp Leu Gln Pro Asp Phe Cys Leu Asn Asp Gly Lys Cys
   1               5                   10                  15
   Asp Ile Met Pro Gly His Gly Ala Ile Cys Arg Cys Arg Val Gly Glu
   20 25 30
   Asn Trp Trp Tyr Arg Gly Lys His Cys Glu
   35 40
<210> 488
   <211> 32
   <212> PRT
   <213> Homo sapiens
<400> 488
   Asp Arg Cys Gln Leu Gln Thr Arg Cys Gln Asn Gly Gly Gln Trp Asp
   1 5 10 15
   Gly Leu Lys Cys Gln Cys Pro Ser Thr Phe Tyr Gly Ser Ser Cys Glu
   20 25 30
<210> 489
   <211> 32
   <212> PRT
   <213> Homo sapiens
<400> 489
   Cys Asp Asn Gly Gly Thr Trp Glu Gln Gly Gln Cys Ala Cys Leu Pro
   1 5 10 15
   Gly Phe Ser Gly Asp Arg Cys Gln Leu Gln Thr Arg Cys Gln Asn Gly
   20 25 30
<210> 490
   <211> 34
   <212> PRT
   <213> Homo sapiens
<400> 490
   Cys Ala Ser Ala Pro Cys Arg Asn Gly Gly Val Cys Arg Glu Gly Trp
   1 5 10 15
   Asn Arg Phe Ile Cys Asp Cys Ile Gly Thr Gly Phe Leu Gly Arg Val
   20 25 30
   Cys Glu
<210> 491
   <211> 44
   <212> PRT
   <213> Homo sapiens
<400> 491
   Leu Asp Ile Cys Ser Lys Asn Pro Cys His Asn Gly Gly Leu Cys Glu
   1 5 10 15
   Glu Ile Ser Gln Glu Val Arg Gly Asp Val Phe Pro Ser Tyr Thr Cys
   20 25 30
   Thr Cys Leu Lys Gly Tyr Ala Gly Asn His Cys Glu
   35 40
<210> 492
   <211> 57
   <212> PRT
   <213> Homo sapiens
<400> 492
   Cys Ile Cys Tyr Gly His Ala Ser Ser Cys Pro Trp Asp Glu Thr Thr
   1               5                   10                  15
   Lys Lys Leu Gln Cys Gln Cys Glu His Asn Thr Cys Gly Glu Ser Cys
               20                  25                  30
   Asn Arg Cys Cys Pro Gly Tyr His Gln Gln Pro Trp Arg Pro Gly Thr
           35                  40                  45
   Val Ser Ser Gly Asn Thr Cys Glu Ala
       50                  55
<210> 493
   <211> 46
   <212> PRT
   <213> Homo sapiens
<400> 493
   Cys Asp Cys Pro His Thr Gln Asn Thr Cys Asp Pro Glu Thr Gly Glu
   1               5                   10                  15
   Cys Val Cys Pro Pro His Thr Gln Gly Val Lys Cys Glu Glu Cys Glu
               20                  25                  30
   Asp Gly His Trp Gly Tyr Asp Ala Glu Val Gly Cys Gln Ala
           35                  40                  45
<210> 494
   <211> 46
   <212> PRT
   <213> Homo sapiens
<400> 494
   Cys Asn Cys Ser Leu Val Gly Ser Thr His His Arg Cys Asp Val Val
   1               5                   10                  15
   Thr Gly His Cys Gln Cys Lys Ser Lys Phe Gly Gly Arg Ala Cys Asp
               20                  25                  30
   Gln Cys Ser Leu Gly Tyr Arg Asp Phe Pro Asp Cys Val Pro
           35                  40                  45
<210> 495
   <211> 60
   <212> PRT
   <213> Homo sapiens
<400> 495
   Cys Asp Cys Asp Leu Arg Gly Thr Ser Gly Asp Ala Cys Asn Leu Glu
   1               5                   10                  15
   Gln Gly Leu Cys Gly Cys Val Glu Glu Thr Gly Ala Cys Pro Cys Lys
               20                  25                  30
   Glu Asn Val Phe Gly Pro Gln Cys Asn Glu Cys Arg Glu Gly Thr Phe
           35                  40                  45
   Ala Leu Arg Ala Asp Asn Pro Leu Gly Cys Ser Pro
       50                  55                  60
<210> 496
   <211> 49
   <212> PRT
   <213> Homo sapiens
<400> 496
   Cys Ser Cys Asn Asn His Ser Asp Thr Cys Asp Pro Asn Thr Gly Lys
   1               5                   10                  15
   Cys Leu Asn Cys Gly Asp Asn Thr Ala Gly Asp His Cys Asp Val Cys
               20                  25                  30
   Thr Ser Gly Tyr Tyr Gly Lys Val Thr Gly Ser Ala Ser Asp Cys Ala
           35                  40                  45
   Leu
<210> 497
   <211> 57
   <212> PRT
   <213> Homo sapiens
<400> 497
   Cys Ala Cys Pro His Ser Pro Pro Ala Ser Phe Ser Pro Thr Cys Val
   1               5                   10                  15
   Leu Glu Gly Asp His Asp Phe Arg Cys Asp Ala Cys Leu Leu Gly Tyr
               20                  25                  30
   Glu Gly Lys His Cys Glu Arg Cys Ser Ser Ser Tyr Tyr Gly Asn Pro
           35                  40                  45
   Gln Thr Pro Gly Gly Ser Cys Gln Lys
       50                  55
<210> 498
   <211> 47
   <212> PRT
   <213> Homo sapiens
<400> 498
   Cys Asp Cys Asn Pro His Gly Ser Val His Gly Asp Cys Asp Arg Thr
   1               5                   10                  15
   Ser Gly Gln Cys Val Cys Arg Leu Gly Ala Ser Gly Leu Arg Cys Asp
               20                  25                  30
   Glu Cys Glu Pro Arg His Ile Leu Met Glu Thr Asp Cys Val Ser
           35                  40                  45
<210> 499
   <211> 70
   <212> PRT
   <213> Homo sapiens
<400> 499
   Cys Asn Cys His Asn Lys Ala Lys Asp Cys Tyr Tyr Asp Glu Ser Val
   1               5                   10                  15
   Ala Lys Gln Lys Lys Ser Leu Asn Thr Ala Gly Gln Phe Arg Gly Gly
               20                  25                  30
   Gly Val Cys Ile Asn Cys Leu Gln Asn Thr Met Gly Ile Asn Cys Glu
           35                  40                  45
   Thr Cys Ile Asp Gly Tyr Tyr Arg Pro His Lys Val Ser Pro Tyr Glu
       50                  55                  60
   Asp Glu Pro Cys Arg Pro
   65                  70
<210> 500
   <211> 57
   <212> PRT
   <213> Homo sapiens
<400> 500
   Cys Asn Cys Asp Pro Val Gly Ser Leu Ser Ser Val Cys Ile Lys Asp
   1               5                   10                  15
   Asp Leu His Ser Asp Leu His Asn Gly Lys Gln Pro Gly Gln Cys Pro
               20                  25                  30
   Cys Lys Glu Gly Tyr Thr Gly Glu Lys Cys Asp Arg Cys Gln Leu Gly
           35                  40                  45
   Tyr Lys Asp Tyr Pro Thr Cys Val Ser
       50                  55
<210> 501
   <211> 49
   <212> PRT
   <213> Homo sapiens
<400> 501
   Cys Gly Cys Asn Pro Val Gly Ser Ala Ser Asp Glu Pro Cys Thr Gly
   1               5                   10                  15
   Pro Cys Val Cys Lys Glu Asn Val Glu Gly Lys Ala Cys Asp Arg Cys
               20                  25                  30
   Lys Pro Gly Phe Tyr Asn Leu Lys Glu Lys Asn Pro Arg Gly Cys Ser
           35                  40                  45
   Glu
<210> 502
   <211> 49
   <212> PRT
   <213> Homo sapiens
<400> 502
   Cys Glu Cys His Gly His Ala Ala Glu Cys Asn Val His Gly Val Cys
   1               5                   10                  15
   Ile Ala Cys Ala His Asn Thr Thr Gly Val His Cys Glu Gln Cys Leu
               20                  25                  30
   Pro Gly Phe Tyr Gly Glu Pro Ser Arg Gly Thr Pro Gly Asp Cys Gln
           35                  40                  45
   Pro
<210> 503
   <211> 58
   <212> PRT
   <213> Homo sapiens
<400> 503
   Cys Ala Cys Pro Leu Thr Ile Ala Ser Asn Asn Phe Ser Pro Thr Cys
   1               5                   10                  15
   His Leu Asn Asp Gly Asp Glu Val Val Cys Asp Trp Cys Ala Pro Gly
               20                  25                  30
   Tyr Ser Gly Ala Trp Cys Glu Arg Cys Ala Asp Gly Tyr Tyr Gly Asn
           35                  40                  45
   Pro Thr Val Pro Gly Glu Ser Cys Val Pro
       50                  55
<210> 504
   <211> 53
   <212> PRT
   <213> Homo sapiens
<400> 504
   Cys Asp Cys Ser Gly Asn Val Asp Pro Ser Glu Ala Gly His Cys Asp
   1               5                   10                  15
   Ser Val Thr Gly Glu Cys Leu Lys Cys Leu Gly Asn Thr Asp Gly Ala
               20                  25                  30
   His Cys Glu Arg Cys Ala Asp Gly Phe Tyr Gly Asp Ala Val Thr Ala
           35                  40                  45
   Lys Asn Cys Arg Ala
       50
<210> 505
   <211> 49
   <212> PRT
   <213> Homo sapiens
<400> 505
   Cys Glu Cys His Val Lys Gly Ser His Ser Ala Val Cys His Leu Glu
   1               5                   10                  15
   Thr Gly Leu Cys Asp Cys Lys Pro Asn Val Thr Gly Gln Gln Cys Asp
               20                  25                  30
   Gln Cys Leu His Gly Tyr Tyr Gly Leu Asp Ser Gly His Gly Cys Arg
           35                  40                  45
   Pro
<210> 506
   <211> 47
   <212> PRT
   <213> Homo sapiens
<400> 506
   Cys Asn Cys Ser Val Ala Gly Ser Val Ser Asp Gly Cys Thr Asp Glu
   1               5                   10                  15
   Gly Gln Cys His Cys Val Pro Gly Val Ala Gly Lys Arg Cys Asp Arg
               20                  25                  30
   Cys Ala His Gly Phe Tyr Ala Tyr Gln Asp Gly Ser Cys Thr Pro
           35                  40                  45
<210> 507
   <211> 57
   <212> PRT
   <213> Homo sapiens
<400> 507
   Cys Ile Cys Tyr Gly His Ala Arg Ala Cys Pro Leu Asp Pro Ala Thr
   1               5                   10                  15
   Asn Lys Ser Arg Cys Glu Cys Glu His Asn Thr Cys Gly Asp Ser Cys
               20                  25                  30
   Asp Gln Cys Cys Pro Gly Phe His Gln Lys Pro Trp Arg Ala Gly Thr
           35                  40                  45
   Phe Leu Thr Lys Thr Glu Cys Glu Ala
       50                  55
<210> 508
   <211> 46
   <212> PRT
   <213> Homo sapiens
<400> 508
   Cys Glu Cys Ser His Leu Gly Asn Asn Cys Asp Pro Lys Thr Gly Arg
   1               5                   10                  15
   Cys Ile Cys Pro Pro Asn Thr Ile Gly Glu Lys Cys Ser Lys Cys Ala
               20                  25                  30
   Pro Asn Thr Trp Gly His Ser Ile Thr Thr Gly Cys Lys Ala
           35                  40                  45
<210> 509
   <211> 46
   <212> PRT
   <213> Homo sapiens
<400> 509
   Cys Asn Cys Ser Thr Val Gly Ser Leu Asp Phe Gln Cys Asn Val Asn
   1               5                   10                  15
   Thr Gly Gln Cys Asn Cys His Pro Lys Phe Ser Gly Ala Lys Cys Thr
               20                  25                  30
   Glu Cys Ser Arg Gly His Trp Asn Tyr Pro Arg Cys Asn Leu
           35                  40                  45
<210> 510
   <211> 60
   <212> PRT
   <213> Homo sapiens
<400> 510
   Cys Asp Cys Phe Leu Pro Gly Thr Asp Ala Thr Thr Cys Asp Ser Glu
   1               5                   10                  15
   Thr Lys Lys Cys Ser Cys Ser Asp Gln Thr Gly Gln Cys Thr Cys Lys
               20                  25                  30
   Val Asn Val Glu Gly Ile His Cys Asp Arg Cys Arg Pro Gly Lys Phe
           35                  40                  45
   Gly Leu Asp Ala Lys Asn Pro Leu Gly Cys Ser Ser
       50                  55                  60
<210> 511
   <211> 49
   <212> PRT
   <213> Homo sapiens
<400> 511
   Cys Gln Cys Asn Gly His Ser Ser Leu Cys Asp Pro Glu Thr Ser Ile
   1               5                   10                  15
   Cys Gln Asn Cys Gln His His Thr Ala Gly Asp Phe Cys Glu Arg Cys
               20                  25                  30
   Ala Leu Gly Tyr Tyr Gly Ile Val Lys Gly Leu Pro Asn Asp Cys Gln
           35                  40                  45
   Gln
<210> 512
   <211> 58
   <212> PRT
   <213> Homo sapiens
<400> 512
   Cys Ala Cys Pro Leu Ile Ser Ser Ser Asn Asn Phe Ser Pro Ser Cys
   1               5                   10                  15
   Val Ala Glu Gly Leu Asp Asp Tyr Arg Cys Thr Ala Cys Pro Arg Gly
               20                  25                  30
   Tyr Glu Gly Gln Tyr Cys Glu Arg Cys Ala Pro Gly Tyr Thr Gly Ser
           35                  40                  45
   Pro Gly Asn Pro Gly Gly Ser Cys Gln Glu
       50                  55
<210> 513
   <211> 47
   <212> PRT
   <213> Homo sapiens
<400> 513
   Cys Glu Cys Asp Pro Tyr Gly Ser Leu Pro Val Pro Cys Asp Pro Val
   1               5                   10                  15
   Thr Gly Phe Cys Thr Cys Arg Pro Gly Ala Thr Gly Arg Lys Cys Asp
               20                  25                  30
   Gly Cys Lys His Trp His Ala Arg Glu Gly Trp Glu Cys Val Phe
           35                  40                  45
<210> 514
   <211> 70
   <212> PRT
   <213> Homo sapiens
<400> 514
   Cys Asn Cys His Gly Lys Ala Glu Glu Cys Tyr Tyr Asp Glu Asn Val
   1               5                   10                  15
   Ala Arg Arg Asn Leu Ser Leu Asn Ile Arg Gly Lys Tyr Ile Gly Gly
               20                  25                  30
   Gly Val Cys Ile Asn Cys Thr Gln Asn Thr Ala Gly Ile Asn Cys Glu
           35                  40                  45
   Thr Cys Thr Asp Gly Phe Phe Arg Pro Lys Gly Val Ser Pro Asn Tyr
       50                  55                  60
   Pro Arg Pro Cys Gln Pro
   65                  70
<210> 515
   <211> 55
   <212> PRT
   <213> Homo sapiens
<400> 515
   Cys His Cys Asp Pro Ile Gly Ser Leu Asn Glu Val Cys Val Lys Asp
   1               5                   10                  15
   Glu Lys His Ala Arg Arg Gly Leu Ala Pro Gly Ser Cys His Cys Lys
               20                  25                  30
   Thr Gly Phe Gly Gly Val Ser Cys Asp Arg Cys Ala Arg Gly Tyr Thr
           35                  40                  45
   Gly Tyr Pro Asp Cys Lys Ala
       50                  55
<210> 516
   <211> 49
   <212> PRT
   <213> Homo sapiens
<400> 516
   Cys Asn Cys Ser Gly Leu Gly Ser Lys Asn Glu Asp Pro Cys Phe Gly
   1               5                   10                  15
   Pro Cys Ile Cys Lys Glu Asn Val Glu Gly Gly Asp Cys Ser Arg Cys
               20                  25                  30
   Lys Ser Gly Phe Phe Asn Leu Gln Glu Asp Asn Trp Lys Gly Cys Asp
           35                  40                  45
   Glu
<210> 517
   <211> 50
   <212> PRT
   <213> Homo sapiens
<400> 517
   Cys Gln Cys Phe Gly His Ala Glu Ser Cys Asp Asp Val Thr Gly Glu
   1               5                   10                  15
   Cys Leu Asn Cys Lys Asp His Thr Gly Gly Pro Tyr Cys Asp Lys Cys
               20                  25                  30
   Leu Pro Gly Phe Tyr Gly Glu Pro Thr Lys Gly Thr Ser Glu Asp Cys
           35                  40                  45
   Gln Pro
       50
<210> 518
   <211> 58
   <212> PRT
   <213> Homo sapiens
<400> 518
   Cys Ala Cys Pro Leu Asn Ile Pro Ser Asn Asn Phe Ser Pro Thr Cys
   1               5                   10                  15
   His Leu Asp Arg Ser Leu Gly Leu Ile Cys Asp Gly Cys Pro Val Gly
               20                  25                  30
   Tyr Thr Gly Pro Arg Cys Glu Arg Cys Ala Glu Gly Tyr Phe Gly Gln
           35                  40                  45
   Pro Ser Val Pro Gly Gly Ser Cys Gln Pro
       50                  55
<210> 519
   <211> 53
   <212> PRT
   <213> Homo sapiens
<400> 519
   Cys Gln Cys Asn Asp Asn Leu Asp Phe Ser Ile Pro Gly Ser Cys Asp
   1               5                   10                  15
   Ser Leu Ser Gly Ser Cys Leu Ile Cys Lys Pro Gly Thr Thr Gly Arg
               20                  25                  30
   Tyr Cys Glu Leu Cys Ala Asp Gly Tyr Phe Gly Asp Ala Val Asp Ala
           35                  40                  45
   Lys Asn Cys Gln Pro
       50
<210> 520
   <211> 49
   <212> PRT
   <213> Homo sapiens
<400> 520
   Cys Arg Cys Asn Ala Gly Gly Ser Phe Ser Glu Val Cys His Ser Gln
   1               5                   10                  15
   Thr Gly Gln Cys Glu Cys Arg Ala Asn Val Gln Gly Gln Arg Cys Asp
               20                  25                  30
   Lys Cys Lys Ala Gly Thr Phe Gly Leu Gln Ser Ala Arg Gly Cys Val
           35                  40                  45
   Pro
<210> 521
   <211> 47
   <212> PRT
   <213> Homo sapiens
<400> 521
   Cys Asn Cys Asn Ser Phe Gly Ser Lys Ser Phe Asp Cys Glu Glu Ser
   1               5                   10                  15
   Gly Gln Cys Trp Cys Gln Pro Gly Val Thr Gly Lys Lys Cys Asp Arg
               20                  25                  30
   Cys Ala His Gly Tyr Phe Asn Phe Gln Glu Gly Gly Cys Thr Ala
           35                  40                  45
<210> 522
   <211> 57
   <212> PRT
   <213> Homo sapiens
<400> 522
   Cys Val Cys Asn Gly His Ala Glu Val Cys Asn Ile Asn Asn Pro Glu
   1               5                   10                  15
   Lys Leu Phe Arg Cys Glu Cys Gln His His Thr Cys Gly Glu Thr Cys
               20                  25                  30
   Asp Arg Cys Cys Thr Gly Tyr Asn Gln Arg Arg Trp Arg Pro Ala Ala
           35                  40                  45
   Trp Glu Gln Ser His Glu Cys Glu Ala
       50                  55
<210> 523
   <211> 44
   <212> PRT
   <213> Homo sapiens
<400> 523
   Cys Ser Cys Gly Arg Arg Leu Cys Glu Glu Met Thr Gly Gln Cys Arg
   1               5                   10                  15
   Cys Pro Pro Arg Thr Val Arg Pro Gln Cys Glu Val Cys Glu Thr His
               20                  25                  30
   Ser Phe Ser Phe His Pro Met Ala Gly Cys Glu Gly
           35                  40
<210> 524
   <211> 49
   <212> PRT
   <213> Homo sapiens
<400> 524
   Cys Asn Cys Ser Arg Arg Gly Thr Ile Glu Ala Ala Met Pro Glu Cys
   1               5                   10                  15
   Asp Arg Asp Ser Gly Gln Cys Arg Cys Lys Pro Arg Ile Thr Gly Arg
               20                  25                  30
   Gln Cys Asp Arg Cys Ala Ser Gly Phe Tyr Arg Phe Pro Glu Cys Val
           35                  40                  45
   Pro
<210> 525
   <211> 51
   <212> PRT
   <213> Homo sapiens
<400> 525
   Cys Asn Cys Asn Arg Asp Gly Thr Glu Pro Gly Val Cys Asp Pro Gly
   1               5                   10                  15
   Thr Gly Ala Cys Leu Cys Lys Glu Asn Val Glu Gly Thr Glu Cys Asn
               20                  25                  30
   Val Cys Arg Glu Gly Ser Phe His Leu Asp Pro Ala Asn Leu Lys Gly
           35                  40                  45
   Cys Thr Ser
       50
<210> 526
   <211> 47
   <212> PRT
   <213> Homo sapiens
<400> 526
   Cys Asn Cys Asn Gly His Ser Asn Gln Cys Gln Asp Gly Ser Gly Ile
   1               5                   10                  15
   Cys Val Asn Cys Gln His Asn Thr Ala Gly Glu His Cys Glu Arg Cys
               20                  25                  30
   Gln Glu Gly Tyr Tyr Gly Asn Ala Val His Gly Ser Cys Arg Ala
           35                  40                  45
<210> 527
   <211> 53
   <212> PRT
   <213> Homo sapiens
<400> 527
   Cys Pro Cys Pro His Thr Asn Ser Phe Ala Thr Gly Cys Val Val Asn
   1               5                   10                  15
   Gly Gly Asp Val Arg Cys Ser Cys Lys Ala Gly Tyr Thr Gly Thr Gln
               20                  25                  30
   Cys Glu Arg Cys Ala Pro Gly Tyr Phe Gly Asn Pro Gln Lys Phe Gly
           35                  40                  45
   Gly Ser Cys Gln Pro
       50
<210> 528
   <211> 35
   <212> PRT
   <213> Homo sapiens
<400> 528
   Cys Ser Cys Asn Ser Asn Gly Gln Leu Gly Ser Cys His Pro Leu Thr
   1               5                   10                  15
   Gly Asp Cys Ile Asn Gln Glu Pro Lys Asp Ser Ser Pro Ala Glu Glu
               20                  25                  30
   Cys Asp Asp
           35
<210> 529
   <211> 70
   <212> PRT
   <213> Homo sapiens
<400> 529
   Cys Asn Cys His Gly His Ala Ser Asn Cys Tyr Tyr Asp Pro Asp Val
   1               5                   10                  15
   Glu Arg Gln Gln Ala Ser Leu Asn Thr Gln Gly Ile Tyr Ala Gly Gly
               20                  25                  30
   Gly Val Cys Ile Asn Cys Gln His Asn Thr Ala Gly Val Asn Cys Glu
           35                  40                  45
   Gln Cys Ala Lys Gly Tyr Tyr Arg Pro Tyr Gly Val Pro Val Asp Ala
       50                  55                  60
   Pro Asp Gly Cys Ile Pro
   65                  70
<210> 530
   <211> 44
   <212> PRT
   <213> Homo sapiens
<400> 530
   Cys Ser Cys Asp Pro Glu His Ala Asp Gly Cys Glu Gln Gly Ser Gly
   1               5                   10                  15
   Arg Cys His Cys Lys Pro Asn Phe His Gly Asp Asn Cys Glu Lys Cys
               20                  25                  30
   Ala Ile Gly Tyr Tyr Asn Phe Pro Phe Cys Leu Arg
           35                  40
<210> 531
   <211> 45
   <212> PRT
   <213> Homo sapiens
<400> 531
   Cys Asp Cys Asn Leu Glu Gly Val Leu Pro Glu Ile Cys Asp Ala His
   1               5                   10                  15
   Gly Arg Cys Leu Cys Arg Pro Gly Val Glu Gly Pro Arg Cys Asp Thr
                   20                  25                  30
   Cys Arg Ser Gly Phe Tyr Ser Phe Pro Ile Cys Gln Ala
           35                  40                  45
<210> 532
   <211> 53
   <212> PRT
   <213> Homo sapiens
<400> 532
   Cys Trp Cys Ser Ala Leu Gly Ser Tyr Gln Met Pro Cys Ser Ser Val
   1               5                   10                  15
   Thr Gly Gln Cys Glu Cys Arg Pro Gly Val Thr Gly Gln Arg Cys Asp
               20                  25                  30
   Arg Cys Leu Ser Gly Ala Tyr Asp Phe Pro His Cys Gln Gly Ser Ser
           35                  40                  45
   Ser Ala Cys Asp Pro
       50
<210> 533
   <211> 41
   <212> PRT
   <213> Homo sapiens
<400> 533
   Gly Thr Ile Asn Ser Asn Leu Gly Tyr Cys Gln Cys Lys Leu His Val
   1               5                   10                  15
   Glu Gly Pro Thr Cys Ser Arg Cys Lys Leu Leu Tyr Trp Asn Leu Asp
               20                  25                  30
   Lys Glu Asn Pro Ser Gly Cys Ser Glu
           35                  40
<210> 534
   <211> 53
   <212> PRT
   <213> Homo sapiens
<400> 534
   Cys Lys Cys His Lys Ala Gly Thr Val Ser Gly Thr Gly Glu Cys Arg
   1               5                   10                  15
   Gln Gly Asp Gly Asp Cys His Cys Lys Ser His Val Gly Gly Asp Ser
               20                  25                  30
   Cys Asp Thr Cys Glu Asp Gly Tyr Phe Ala Leu Glu Lys Ser Asn Tyr
           35                  40                  45
   Phe Gly Cys Gln Gly
       50
<210> 535
   <211> 45
   <212> PRT
   <213> Homo sapiens
<400> 535
   Cys Gln Cys Asp Ile Gly Gly Ala Leu Ser Ser Met Cys Ser Gly Pro
   1               5                   10                  15
   Ser Gly Val Cys Gln Cys Arg Glu His Val Val Gly Lys Val Cys Gln
               20                  25                  30
   Arg Pro Glu Asn Asn Tyr Tyr Phe Pro Asp Leu His His
           35                  40                  45
<210> 536
   <211> 46
   <212> PRT
   <213> Homo sapiens
<400> 536
   Cys Glu Cys His Pro Thr Gly Ala Thr Gly Pro His Cys Ser Pro Glu
   1               5                   10                  15
   Gly Gly Gln Cys Pro Cys Gln Pro Asn Val Ile Gly Arg Gln Cys Thr
               20                  25                  30
   Arg Cys Ala Thr Gly His Tyr Gly Phe Pro Arg Cys Lys Pro
           35                  40                  45
<210> 537
   <211> 50
   <212> PRT
   <213> Homo sapiens
<400> 537
   Cys Asp Cys Asn Gly Asn Ser Asn Glu Cys Leu Asp Gly Ser Gly Tyr
   1               5                   10                  15
   Cys Val His Cys Gln Arg Asn Thr Thr Gly Glu His Cys Glu Lys Cys
               20                  25                  30
   Leu Asp Gly Tyr Ile Gly Asp Ser Ile Arg Gly Ala Pro Gln Phe Cys
           35                  40                  45
   Gln Pro
       50
<210> 538
   <211> 55
   <212> PRT
   <213> Homo sapiens
<400> 538
   Cys Pro Cys Pro Leu Pro His Leu Ala Asn Phe Ala Glu Ser Cys Tyr
   1               5                   10                  15
   Arg Lys Asn Gly Ala Val Arg Cys Ile Cys Asn Glu Asn Tyr Ala Gly
               20                  25                  30
   Pro Asn Cys Glu Arg Cys Ala Pro Gly Tyr Tyr Gly Asn Pro Leu Leu
           35                  40                  45
   Ile Gly Ser Thr Cys Lys Lys
       50                  55
<210> 539
   <211> 54
   <212> PRT
   <213> Homo sapiens
<400> 539
   Cys Asp Cys Ser Gly Asn Ser Asp Pro Asn Leu Ile Phe Glu Asp Cys
   1               5                   10                  15
   Asp Glu Val Thr Gly Gln Cys Arg Asn Cys Leu Arg Asn Thr Thr Gly
               20                  25                  30
   Phe Lys Cys Glu Arg Cys Ala Pro Gly Tyr Tyr Gly Asp Ala Arg Ile
           35                  40                  45
   Ala Lys Asn Cys Ala Val
       50
<210> 540
   <211> 59
   <212> PRT
   <213> Homo sapiens
<400> 540
   Cys Val Cys His Gly His Ala Asp Ala Cys Asp Ala Lys Asp Pro Thr
   1               5                   10                  15
   Asp Pro Phe Arg Leu Gln Cys Thr Cys Gln His Asn Thr Cys Gly Gly
               20                  25                  30
   Thr Cys Asp Arg Cys Cys Pro Gly Phe Asn Gln Gln Pro Trp Lys Pro
           35                  40                  45
   Ala Thr Ala Asn Ser Ala Asn Glu Cys Gln Ser
       50                  55
<210> 541
   <211> 53
   <212> PRT
   <213> Homo sapiens
<400> 541
   Cys Ser Cys Asp Leu Arg Gly Thr Leu Gly Gly Val Ala Glu Cys Gln
   1               5                   10                  15
   Pro Gly Thr Gly Gln Cys Phe Cys Lys Pro His Val Cys Gly Gln Ala
               20                  25                  30
   Cys Ala Ser Cys Lys Asp Gly Phe Phe Gly Leu Asp Gln Ala Asp Tyr
           35                  40                  45
   Phe Gly Cys Arg Ser
       50
<210> 542
   <211> 22
   <212> PRT
   <213> Homo sapiens
<400> 542
   Cys Arg Cys Asp Ile Gly Gly Ala Leu Gly Gln Ser Cys Glu Pro Arg
   1               5                   10                  15
   Thr Gly Val Cys Arg Cys
               20
<210> 543
   <211> 46
   <212> PRT
   <213> Homo sapiens
<400> 543
   Cys Gly Cys His Glu Val Gly Ala Thr Gly Pro Thr Cys Glu Pro Phe
   1               5                   10                  15
   Gly Gly Gln Cys Pro Cys His Ala His Val Ile Gly Arg Asp Cys Ser
               20                  25                  30
   Arg Cys Ala Thr Gly Tyr Trp Gly Phe Pro Asn Cys Arg Pro
           35                  40                  45
<210> 544
   <211> 44
   <212> PRT
   <213> Homo sapiens
<400> 544
   Cys Asp Cys Gly Ala Arg Leu Cys Asp Glu Leu Thr Gly Gln Cys Ile
   1               5                   10                  15
   Cys Pro Pro Arg Thr Ile Pro Pro Asp Cys Leu Leu Cys Gln Pro Gln
               20                  25                  30
   Thr Phe Gly Cys His Pro Leu Val Gly Cys Glu Glu
           35                  40
<210> 545
   <211> 49
   <212> PRT
   <213> Homo sapiens
<400> 545
   Cys Asn Cys Ser Gly Pro Gly Ile Gln Glu Leu Thr Asp Pro Thr Cys
   1               5                   10                  15
   Asp Thr Asp Ser Gly Gln Cys Lys Cys Arg Pro Asn Val Thr Gly Arg
               20                  25                  30
   Arg Cys Asp Thr Cys Ser Pro Gly Phe His Gly Tyr Pro Arg Cys Arg
           35                  40                  45
   Pro
<210> 546
   <211> 51
   <212> PRT
   <213> Homo sapiens
<400> 546
   Cys Asp Cys His Glu Ala Gly Thr Ala Pro Gly Val Cys Asp Pro Leu
   1               5                   10                  15
   Thr Gly Gln Cys Tyr Cys Lys Glu Asn Val Gln Gly Pro Lys Cys Asp
               20                  25                  30
   Gln Cys Ser Leu Gly Thr Phe Ser Leu Asp Ala Ala Asn Pro Lys Gly
           35                  40                  45
   Cys Thr Arg
       50
<210> 547
   <211> 49
   <212> PRT
   <213> Homo sapiens
<400> 547
   Cys Gln Cys His Gly His Ser Asp Arg Cys Leu Pro Gly Ser Gly Val
   1               5                   10                  15
   Cys Val Asp Cys Gln His Asn Thr Glu Gly Ala His Cys Glu Arg Cys
               20                  25                  30
   Gln Ala Gly Phe Val Ser Ser Arg Asp Asp Pro Ser Ala Pro Cys Val
           35                  40                  45
   Ser
<210> 548
   <211> 56
   <212> PRT
   <213> Homo sapiens
<400> 548
   Cys Pro Cys Pro Leu Ser Val Pro Ser Asn Asn Phe Ala Glu Gly Cys
   1               5                   10                  15
   Val Leu Arg Gly Gly Arg Thr Gln Cys Leu Cys Lys Pro Gly Tyr Ala
               20                  25                  30
   Gly Ala Ser Cys Glu Arg Cys Ala Pro Gly Phe Phe Gly Asn Pro Leu
           35                  40                  45
   Val Leu Gly Ser Ser Cys Gln Pro
       50                  55
<210> 549
   <211> 54
   <212> PRT
   <213> Homo sapiens
<400> 549
   Cys Asp Cys Ser Gly Asn Gly Asp Pro Asn Leu Leu Phe Ser Asp Cys
   1               5                   10                  15
   Asp Pro Leu Thr Gly Ala Cys Arg Gly Cys Leu Arg His Thr Thr Gly
               20                  25                  30
   Pro Arg Cys Glu Ile Cys Ala Pro Gly Phe Tyr Gly Asn Ala Leu Leu
           35                  40                  45
   Pro Gly Asn Cys Thr Arg
       50
<210> 550
   <211> 47
   <212> PRT
   <213> Homo sapiens
<400> 550
   Cys Asp Cys Thr Pro Cys Gly Thr Glu Ala Cys Asp Pro His Ser Gly
   1               5                   10                  15
   His Cys Leu Cys Lys Ala Gly Val Thr Gly Arg Arg Cys Asp Arg Cys
               20                  25                  30
   Gln Glu Gly His Phe Gly Phe Asp Gly Cys Gly Gly Cys Arg Pro
           35                  40                  45
<210> 551
   <211> 70
   <212> PRT
   <213> Homo sapiens
<400> 551
   Cys Asn Cys Tyr Gly His Ala Thr Asp Cys Tyr Tyr Asp Pro Glu Val
   1               5                   10                  15
   Asp Arg Arg Arg Ala Ser Gln Ser Leu Asp Gly Thr Tyr Gln Gly Gly
               20                  25                  30
   Gly Val Cys Ile Asp Cys Gln His His Thr Thr Gly Val Asn Cys Glu
           35                  40                  45
   Arg Cys Leu Pro Gly Phe Tyr Arg Ser Pro Asn His Pro Leu Asp Ser
       50                  55                  60
   Pro His Val Cys Arg Arg
   65                  70
<210> 552
   <211> 47
   <212> PRT
   <213> Homo sapiens
<400> 552
   Cys Ala Cys Gly Pro Ala Ala Glu Gly Ser Glu Cys His Pro Gln Ser
   1               5                   10                  15
   Gly Gln Cys His Cys Arg Pro Gly Thr Met Gly Pro Gln Cys Arg Glu
               20                  25                  30
   Cys Ala Pro Gly Tyr Trp Gly Leu Pro Glu Gln Gly Cys Arg Arg
           35                  40                  45
<210> 553
   <211> 50
   <212> PRT
   <213> Homo sapiens
<400> 553
   Cys Gln Cys Pro Gly Gly Arg Cys Asp Pro His Thr Gly Arg Cys Asn
   1               5                   10                  15
   Cys Pro Pro Gly Leu Ser Gly Glu Arg Cys Asp Thr Cys Ser Gln Gln
               20                  25                  30
   His Gln Val Pro Val Pro Gly Gly Pro Val Gly His Ser Ile His Cys
           35                  40                  45
   Glu Val
       50
<210> 554
   <211> 46
   <212> PRT
   <213> Homo sapiens
<400> 554
   Cys Asn Cys Glu Ser Asp Phe Thr Asp Gly Thr Cys Glu Asp Leu Thr
   1               5                   10                  15
   Gly Arg Cys Tyr Cys Arg Pro Asn Phe Ser Gly Glu Arg Cys Asp Val
               20                  25                  30
   Cys Ala Glu Gly Phe Thr Gly Phe Pro Ser Cys Tyr Pro Thr
           35                  40                  45
<210> 555
   <211> 47
   <212> PRT
   <213> Homo sapiens
<400> 555
   Cys Asp Cys Ser Ala Ala Gly Thr Gln Gly Asn Ala Cys Arg Lys Asp
   1               5                   10                  15
   Pro Arg Val Gly Arg Cys Leu Cys Lys Pro Asn Phe Gln Gly Thr His
               20                  25                  30
   Cys Glu Leu Cys Ala Pro Gly Phe Tyr Gly Pro Gly Cys Gln Pro
           35                  40                  45
<210> 556
   <211> 46
   <212> PRT
   <213> Homo sapiens
<400> 556
   Cys Gln Cys Ser Ser Pro Gly Val Ala Asp Asp Arg Cys Asp Pro Asp
   1               5                   10                  15
   Thr Gly Gln Cys Arg Cys Arg Val Gly Phe Glu Gly Ala Thr Cys Asp
               20                  25                  30
   Arg Cys Ala Pro Gly Tyr Phe His Phe Pro Leu Cys Gln Leu
           35                  40                  45
<210> 557
   <211> 45
   <212> PRT
   <213> Homo sapiens
<400> 557
   Cys Gly Cys Ser Pro Ala Gly Thr Leu Pro Glu Gly Cys Asp Glu Ala
   1               5                   10                  15
   Gly Arg Cys Leu Cys Gln Pro Glu Phe Ala Gly Pro His Cys Asp Arg
               20                  25                  30
   Cys Arg Pro Gly Tyr His Gly Phe Pro Asn Cys Gln Ala
           35                  40                  45
<210> 558
   <211> 45
   <212> PRT
   <213> Homo sapiens
<400> 558
   Cys Thr Cys Asp Pro Arg Gly Ala Leu Asp Gln Leu Cys Gly Ala Gly
   1               5                   10                  15
   Gly Leu Cys Arg Cys Arg Pro Gly Tyr Thr Gly Thr Ala Cys Gln Glu
   20 25 30
   Cys Ser Pro Gly Phe His Gly Phe Pro Ser Cys Val Pro
   35 40 45
<210> 559
   <211> 46
   <212> PRT
   <213> Homo sapiens
<400> 559
   Cys His Cys Ser Ala Glu Gly Ser Leu His Ala Ala Cys Asp Pro Arg
   1 5 10 15
   Ser Gly Gln Cys Ser Cys Arg Pro Arg Val Thr Gly Leu Arg Cys Asp
   20 25 30
   Thr Cys Val Pro Gly Ala Tyr Asn Phe Pro Tyr Cys Glu Ala
   35 40 45
<210> 560
   <211> 53
   <212> PRT
   <213> Homo sapiens
<400> 560
   Gly Ser Cys His Pro Ala Gly Leu Ala Pro Val Asp Pro Ala Leu Pro
   1 5 10 15
   Glu Ala Gln Val Pro Cys Met Cys Arg Ala His Val Glu Gly Pro Ser
   20 25 30
   Cys Asp Arg Cys Lys Pro Gly Phe Trp Gly Leu Ser Pro Ser Asn Pro
   35 40 45
   Glu Gly Cys Thr Arg
   50
<210> 561
   <211> 64
   <212> PRT
   <213> Homo sapiens
<400> 561
   Cys Phe Cys Tyr Gly His Ala Ser Glu Cys Ala Pro Val Asp Gly Phe
   1 5 10 15
   Asn Glu Glu Val Glu Gly Met Val His Gly His Cys Met Cys Arg His
   20 25 30
   Asn Thr Lys Gly Leu Asn Cys Glu Leu Cys Met Asp Phe Tyr His Asp
   35 40 45
   Leu Pro Trp Arg Pro Ala Glu Gly Arg Asn Ser Asn Ala Cys Lys Lys
   50 55 60
<210> 562
   <211> 52
   <212> PRT
   <213> Homo sapiens
<400> 562
   Cys Gln Cys His Asn Asn Ile Asp Thr Thr Asp Pro Glu Ala Cys Asp
   1               5                   10                  15
   Lys Glu Thr Gly Arg Cys Leu Lys Cys Leu Tyr His Thr Glu Gly Glu
               20                  25                  30
   His Cys Gln Phe Cys Arg Phe Gly Tyr Tyr Gly Asp Ala Leu Gln Gln
           35                  40                  45
   Asp Cys Arg Lys
       50
<210> 563
   <211> 56
   <212> PRT
   <213> Homo sapiens
<400> 563
   Cys Val Cys Asn Tyr Leu Gly Thr Val Gln Glu His Cys Asn Gly Ser
   1               5                   10                  15
   Asp Cys Gln Cys Asp Lys Ala Thr Gly Gln Cys Leu Cys Leu Pro Asn
               20                  25                  30
   Val Ile Gly Gln Asn Cys Asp Arg Cys Ala Pro Asn Thr Trp Gln Leu
           35                  40                  45
   Ala Ser Gly Thr Gly Cys Asp Pro
       50                  55
<210> 564
   <211> 48
   <212> PRT
   <213> Homo sapiens
<400> 564
   Cys Asn Cys Asn Ala Ala His Ser Phe Gly Pro Ser Cys Asn Glu Phe
   1               5                   10                  15
   Thr Gly Gln Cys Gln Cys Met Pro Gly Phe Gly Gly Arg Thr Cys Ser
               20                  25                  30
   Glu Cys Gln Glu Leu Phe Trp Gly Asp Pro Asp Val Glu Cys Arg Ala
           35                  40                  45
<210> 565
   <211> 47
   <212> PRT
   <213> Homo sapiens
<400> 565
   Cys Asp Cys Asp Pro Arg Gly Ile Glu Thr Pro Gln Cys Asp Gln Ser
   1               5                   10                  15
   Thr Gly Gln Cys Val Cys Val Glu Gly Val Glu Gly Pro Arg Cys Asp
               20                  25                  30
   Lys Cys Thr Arg Gly Tyr Ser Gly Val Phe Pro Asp Cys Thr Pro
           35                  40                  45
<210> 566
   <211> 63
   <212> PRT
   <213> Homo sapiens
<400> 566
   Cys Asn Cys Asn Glu His Ser Ile Ser Cys His Phe Asp Met Ala Val
   1 5 10 15
   Tyr Leu Ala Thr Gly Asn Val Ser Gly Gly Val Cys Asp Asp Cys Gln
   20 25 30
   His Asn Thr Met Gly Arg Asn Cys Glu Gln Cys Lys Pro Phe Tyr Tyr
   35 40 45
   Gln His Pro Glu Arg Asp Ile Arg Asp Pro Asn Phe Cys Glu Arg
   50 55 60
<210> 567
   <211> 60
   <212> PRT
   <213> Homo sapiens
<400> 567
   Cys Thr Cys Asp Pro Ala Gly Ser Gln Asn Glu Gly Ile Cys Asp Ser
   1 5 10 15
   Tyr Thr Asp Phe Ser Thr Gly Leu Ile Ala Gly Gln Cys Arg Cys Lys
   20 25 30
   Leu Asn Val Glu Gly Glu His Cys Asp Val Cys Lys Glu Gly Phe Tyr
   35 40 45
   Asp Leu Ser Ser Glu Asp Pro Phe Gly Cys Lys Ser
   50 55 60
<210> 568
   <211> 52
   <212> PRT
   <213> Homo sapiens
<400> 568
   Cys Ala Cys Asn Pro Leu Gly Thr Ile Pro Gly Gly Asn Pro Cys Asp
   1 5 10 15
   Ser Glu Thr Gly His Cys Tyr Cys Lys Arg Leu Val Thr Gly Gln His
   20 25 30
   Cys Asp Gln Cys Leu Pro Glu His Trp Gly Leu Ser Asn Asp Leu Asp
   35 40 45
   Gly Cys Arg Pro
   50
<210> 569
   <211> 31
   <212> PRT
   <213> Homo sapiens
<400> 569
   Cys Asp Cys Asp Leu Gly Gly Ala Leu Asn Asn Ser Cys Phe Ala Glu
   1 5 10 15
   Ser Gly Gln Cys Ser Cys Arg Pro His Met Ile Gly Arg Gln Cys
   20 25 30
<210> 570
   <211> 48
   <212> PRT
   <213> Homo sapiens
<400> 570
   Cys Glu Cys Asp Pro Gln Gly Ser Leu Ser Ser Val Cys Asp Pro Asn
   1               5                   10                  15
   Gly Gly Gln Cys Gln Cys Arg Pro Asn Val Val Gly Arg Thr Cys Asn
               20                  25                  30
   Arg Cys Ala Pro Gly Thr Phe Gly Phe Gly Pro Ser Gly Cys Lys Pro
           35                  40                  45
<210> 571
   <211> 46
   <212> PRT
   <213> Homo sapiens
<400> 571
   Cys Glu Cys His Leu Gln Gly Ser Val Asn Ala Phe Cys Asn Pro Val
   1               5                   10                  15
   Thr Gly Gln Cys His Cys Phe Gln Gly Val Tyr Ala Arg Gln Cys Asp
               20                  25                  30
   Arg Cys Leu Pro Gly His Trp Gly Phe Pro Ser Cys Gln Pro
           35                  40                  45
<210> 572
   <211> 50
   <212> PRT
   <213> Homo sapiens
<400> 572
   Cys Gln Cys Asn Gly His Ala Asp Asp Cys Asp Pro Val Thr Gly Glu
   1               5                   10                  15
   Cys Leu Asn Cys Gln Asp Tyr Thr Met Gly His Asn Cys Glu Arg Cys
               20                  25                  30
   Leu Ala Gly Tyr Tyr Gly Asp Pro Ile Ile Gly Ser Gly Asp His Cys
           35                  40                  45
   Arg Pro
       50
<210> 573
   <211> 59
   <212> PRT
   <213> Homo sapiens
<400> 573
   Cys Pro Cys Pro Asp Gly Pro Asp Ser Gly Arg Gln Phe Ala Arg Ser
   1               5                   10                  15
   Cys Tyr Gln Asp Pro Val Thr Leu Gln Leu Ala Cys Val Cys Asp Pro
               20                  25                  30
   Gly Tyr Ile Gly Ser Arg Cys Asp Asp Cys Ala Ser Gly Tyr Phe Gly
           35                  40                  45
   Asn Pro Ser Glu Val Gly Gly Ser Cys Gln Pro
       50                  55
<210> 574
   <211> 64
   <212> PRT
   <213> Homo sapiens
<400> 574
   Cys Phe Cys Tyr Gly His Ala Ser Glu Cys Ala Pro Ala Pro Gly Ala
   1               5                   10                  15
   Pro Ala His Ala Glu Gly Met Val His Gly Ala Cys Ile Cys Lys His
               20                  25                  30
   Asn Thr Arg Gly Leu Asn Cys Glu Gln Cys Gln Asp Phe Tyr Arg Asp
           35                  40                  45
   Leu Pro Trp Arg Pro Ala Glu Asp Gly His Ser His Ala Cys Arg Lys
       50                  55                  60
<210> 575
   <211> 52
   <212> PRT
   <213> Homo sapiens
<400> 575
   Cys Glu Cys Ser Gly Asn Ile Asp Pro Met Asp Pro Asp Ala Cys Asp
   1               5                   10                  15
   Pro His Thr Gly Gln Cys Leu Arg Cys Leu His His Thr Glu Gly Pro
               20                  25                  30
   His Cys Ala His Cys Lys Pro Gly Phe His Gly Gln Ala Ala Arg Gln
           35                  40                  45
   Ser Cys His Arg
       50
<210> 576
   <211> 57
   <212> PRT
   <213> Homo sapiens
<400> 576
   Cys Thr Cys Asn Leu Leu Gly Thr Asn Pro Gln Gln Cys Pro Ser Pro
   1               5                   10                  15
   Asp Gln Cys His Cys Asp Pro Ser Ser Gly Gln Cys Pro Cys Leu Pro
               20                  25                  30
   Asn Val Gln Gly Pro Ser Cys Asp Arg Cys Ala Pro Asn Phe Trp Asn
           35                  40                  45
   Leu Thr Ser Gly His Gly Cys Gln Pro
       50                  55
<210> 577
   <211> 48
   <212> PRT
   <213> Homo sapiens
<400> 577
   Cys Ala Cys His Pro Ser Arg Ala Arg Gly Pro Thr Cys Asn Glu Phe
   1               5                   10                  15
   Thr Gly Gln Cys His Cys Arg Ala Gly Phe Gly Gly Arg Thr Cys Ser
               20                  25                  30
   Glu Cys Gln Glu Leu His Trp Gly Asp Pro Gly Leu Gln Cys His Ala
           35                  40                  45
<210> 578
   <211> 47
   <212> PRT
   <213> Homo sapiens
<400> 578
   Cys Asp Cys Asp Ser Arg Gly Ile Asp Thr Pro Gln Cys His Arg Phe
   1               5                   10                  15
   Thr Gly His Cys Ser Cys Arg Pro Gly Val Ser Gly Val Arg Cys Asp
               20                  25                  30
   Gln Cys Ala Arg Gly Phe Ser Gly Ile Phe Pro Ala Cys His Pro
           35                  40                  45
<210> 579
   <211> 63
   <212> PRT
   <213> Homo sapiens
<400> 579
   Cys Glu Cys His Gly His Thr His Ser Cys His Phe Asp Met Ala Val
   1               5                   10                  15
   Tyr Leu Ala Ser Gly Asn Val Ser Gly Gly Val Cys Asp Gly Cys Gln
               20                  25                  30
   His Asn Thr Ala Gly Arg His Cys Glu Leu Cys Arg Pro Phe Phe Tyr
           35                  40                  45
   Arg Asp Pro Thr Lys Asp Leu Arg Asp Pro Ala Val Cys Arg Ser
       50                  55                  60
<210> 580
   <211> 60
   <212> PRT
   <213> Homo sapiens
<400> 580
   Cys Asp Cys Asp Pro Met Gly Ser Gln Asp Gly Gly Arg Cys Asp Ser
   1               5                   10                  15
   His Asp Asp Pro Ala Leu Gly Leu Val Ser Gly Gln Cys Arg Cys Lys
               20                  25                  30
   Glu His Val Val Gly Thr Arg Cys Gln Gln Cys Arg Asp Gly Phe Phe
           35                  40                  45
   Gly Leu Ser Ile Ser Asp Arg Leu Gly Cys Arg Arg
                   50                  55                  60
<210> 581
   <211> 52
   <212> PRT
   <213> Homo sapiens
<400> 581
   Cys Gln Cys Asn Ala Arg Gly Thr Val Pro Gly Ser Thr Pro Cys Asp
   1               5                   10                  15
   Pro Asn Ser Gly Ser Cys Tyr Cys Lys Arg Leu Val Thr Gly Arg Gly
               20                  25                  30
   Cys Asp Arg Cys Leu Pro Gly His Trp Gly Leu Ser His Asp Leu Leu
           35                  40                  45
   Gly Cys Arg Pro
       50
<210> 582
   <211> 31
   <212> PRT
   <213> Homo sapiens
<400> 582
   Cys Asp Cys Asp Val Gly Gly Ala Leu Asp Pro Gln Cys Asp Glu Gly
   1               5                   10                  15
   Thr Gly Gln Cys His Cys Arg Gln His Met Val Gly Arg Arg Cys
               20                  25                  30
<210> 583
   <211> 48
   <212> PRT
   <213> Homo sapiens
<400> 583
   Cys Gln Cys Asn Pro Gln Gly Ser Leu Ser Ser Glu Cys Asn Pro His
   1               5                   10                  15
   Gly Gly Gln Cys Leu Cys Lys Pro Gly Val Val Gly Arg Arg Cys Asp
               20                  25                  30
   Leu Cys Ala Pro Gly Tyr Tyr Gly Phe Gly Pro Thr Gly Cys Gln Ala
           35                  40                  45
<210> 584
   <211> 46
   <212> PRT
   <213> Homo sapiens
<400> 584
   Cys Gln Cys Ser His Glu Gly Ala Leu Ser Ser Leu Cys Glu Lys Thr
   1               5                   10                  15
   Ser Gly Gln Cys Leu Cys Arg Thr Gly Ala Phe Gly Leu Arg Cys Asp
               20                  25                  30
   Arg Cys Gln Arg Gly Gln Trp Gly Phe Pro Ser Cys Arg Pro
           35                  40                  45
<210> 585
   <211> 50
   <212> PRT
   <213> Homo sapiens
<400> 585
   Cys Val Cys Asn Gly His Ala Asp Glu Cys Asn Thr His Thr Gly Ala
   1               5                   10                  15
   Cys Leu Gly Cys Arg Asp His Thr Gly Gly Glu His Cys Glu Arg Cys
               20                  25                  30
   Ile Ala Gly Phe His Gly Asp Pro Arg Leu Pro Tyr Gly Gly Gln Cys
           35                  40                  45
   Arg Pro
       50
<210> 586
   <211> 59
   <212> PRT
   <213> Homo sapiens
<400> 586
   Cys Pro Cys Pro Glu Gly Pro Gly Ser Gln Arg His Phe Ala Thr Ser
   1               5                   10                  15
   Cys His Gln Asp Glu Tyr Ser Gln Gln Ile Val Cys His Cys Arg Ala
               20                  25                  30
   Gly Tyr Thr Gly Leu Arg Cys Glu Ala Cys Ala Pro Gly His Phe Gly
           35                  40                  45
   Asp Pro Ser Arg Pro Gly Gly Arg Cys Gln Leu
       50                  55
<210> 587
   <211> 66
   <212> PRT
   <213> Homo sapiens
<400> 587
   Cys Phe Cys His Gly His Ala Asp Arg Cys Ala Pro Lys Pro Gly Ala
   1               5                   10                  15
   Ser Ala Gly Pro Ser Thr Ala Val Gln Val His Asp Val Cys Val Cys
               20                  25                  30
   Gln His Asn Thr Ala Gly Pro Asn Cys Glu Arg Cys Ala Pro Phe Tyr
           35                  40                  45
   Asn Asn Arg Pro Trp Arg Pro Ala Glu Gly Gln Asp Ala His Glu Cys
       50                  55                  60
   Gln Arg
   65
<210> 588
   <211> 63
   <212> PRT
   <213> Homo sapiens
<400> 588
   Cys Asp Cys Asn Gly His Ser Glu Thr Cys His Phe Asp Pro Ala Val
   1               5                   10                  15
   Phe Ala Ala Ser Gln Gly Ala Tyr Gly Gly Val Cys Asp Asn Cys Arg
               20                  25                  30
   Asp His Thr Glu Gly Lys Asn Cys Glu Arg Cys Gln Leu His Tyr Phe
           35                  40                  45
   Arg Asn Arg Arg Pro Gly Ala Ser Ile Gln Glu Thr Cys Ile Ser
       50                  55                  60
<210> 589
   <211> 52
   <212> PRT
   <213> Homo sapiens
<400> 589
   Cys Glu Cys Asp Pro Asp Gly Ala Val Pro Gly Ala Pro Cys Asp Pro
   1               5                   10                  15
   Val Thr Gly Gln Cys Val Cys Lys Glu His Val Gln Gly Glu Arg Cys
               20                  25                  30
   Asp Leu Cys Lys Pro Gly Phe Thr Gly Leu Thr Tyr Ala Asn Pro Gln
           35                  40                  45
   Gly Cys His Arg
       50
<210> 590
   <211> 50
   <212> PRT
   <213> Homo sapiens
<400> 590
   Cys Asp Cys Asn Ile Leu Gly Ser Arg Arg Asp Met Pro Cys Asp Glu
   1               5                   10                  15
   Glu Ser Gly Arg Cys Leu Cys Leu Pro Asn Val Val Gly Pro Lys Cys
               20                  25                  30
   Asp Gln Cys Ala Pro Tyr His Trp Lys Leu Ala Ser Gly Gln Gly Cys
           35                  40                  45
   Glu Pro
       50
<210> 591
   <211> 53
   <212> PRT
   <213> Homo sapiens
<400> 591
   Cys Ala Cys Asp Pro His Asn Ser Leu Ser Pro Gln Cys Asn Gln Phe
   1               5                   10                  15
   Thr Gly Gln Cys Pro Cys Arg Glu Gly Phe Gly Gly Leu Met Cys Ser
               20                  25                  30
   Ala Ala Ala Ile Arg Gln Cys Pro Asp Arg Thr Tyr Gly Asp Val Ala
           35                  40                  45
   Thr Gly Cys Arg Ala
       50
<210> 592
   <211> 47
   <212> PRT
   <213> Homo sapiens
<400> 592
   Cys Asp Cys Asp Phe Arg Gly Thr Glu Gly Pro Gly Cys Asp Lys Ala
   1               5                   10                  15
   Ser Gly Arg Cys Leu Cys Arg Pro Gly Leu Thr Gly Pro Arg Cys Asp
               20                  25                  30
   Gln Cys Gln Arg Gly Tyr Cys Asn Arg Tyr Pro Val Cys Val Ala
           35                  40                  45
<210> 593
   <211> 67
   <212> PRT
   <213> Homo sapiens
<400> 593
   Cys Phe Cys Asn Gly His Ala Ser Glu Cys Arg Pro Met Gln Lys Met
   1               5                   10                  15
   Arg Gly Asp Val Phe Ser Pro Pro Gly Met Val His Gly Gln Cys Val
               20                  25                  30
   Cys Gln His Asn Thr Asp Gly Pro Asn Cys Glu Arg Cys Lys Asp Phe
           35                  40                  45
   Phe Gln Asp Ala Pro Trp Arg Pro Ala Ala Asp Leu Gln Asp Asn Ala
       50                  55                  60
   Cys Arg Ser
   65
<210> 594
   <211> 52
   <212> PRT
   <213> Homo sapiens
<400> 594
   Cys Ala Cys Asn Asn Asn Ile Asp Val Thr Asp Pro Glu Ser Cys Ser
   1               5                   10                  15
   Arg Val Thr Gly Glu Cys Leu Arg Cys Leu His Asn Thr Gln Gly Ala
               20                  25                  30
   Asn Cys Gln Leu Cys Lys Pro Gly His Tyr Gly Ser Ala Leu Asn Gln
           35                  40                  45
   Thr Cys Arg Arg
       50
<210> 595
   <211> 58
   <212> PRT
   <213> Homo sapiens
<400> 595
   Cys Ser Cys His Ala Ser Gly Val Ser Pro Met Glu Cys Pro Pro Gly
   1               5                   10                  15
   Gly Gly Ala Cys Leu Cys Asp Pro Val Thr Gly Ala Cys Pro Cys Leu
               20                  25                  30
   Pro Asn Val Thr Gly Leu Ala Cys Asp Arg Cys Ala Asp Gly Tyr Trp
           35                  40                  45
   Asn Leu Val Pro Gly Arg Gly Cys Gln Ser
       50                  55
<210> 596
   <211> 48
   <212> PRT
   <213> Homo sapiens
<400> 596
   Cys Asp Cys Asp Pro Arg Thr Ser Gln Ser Ser His Cys Asp Gln Leu
   1               5                   10                  15
   Thr Gly Gln Cys Pro Cys Lys Leu Gly Tyr Gly Gly Lys Arg Cys Ser
               20                  25                  30
   Glu Cys Gln Glu Asn Tyr Tyr Gly Asp Pro Pro Gly Arg Cys Ile Pro
           35                  40                  45
<210> 597
   <211> 47
   <212> PRT
   <213> Homo sapiens
<400> 597
   Cys Asp Cys Asn Arg Ala Gly Thr Gln Lys Pro Ile Cys Asp Pro Asp
   1               5                   10                  15
   Thr Gly Met Cys Arg Cys Arg Glu Gly Val Ser Gly Gln Arg Cys Asp
               20                  25                  30
   Arg Cys Ala Arg Gly His Ser Gln Glu Phe Pro Thr Cys Leu Gln
           35                  40                  45
<210> 598
   <211> 63
   <212> PRT
   <213> Homo sapiens
<210> 599
   <211> 60
   <212> PRT
   <213> Homo sapiens
<400> 599
   Cys Glu Cys Asp Pro Asp Gly Thr Ile Ser Gly Gly Ile Cys Val Ser
   1               5                   10                  15
   His Ser Asp Pro Ala Leu Gly Ser Val Ala Gly Gln Cys Leu Cys Lys
               20                  25                  30
   Glu Asn Val Glu Gly Ala Lys Cys Asp Gln Cys Lys Pro Asn His Tyr
           35                  40                  45
   Gly Leu Ser Ala Thr Asp Pro Leu Gly Cys Gln Pro
       50                  55                  60
<210> 600
   <211> 51
   <212> PRT
   <213> Homo sapiens
<400> 600
   Cys Asp Cys Asn Pro Leu Gly Ser Leu Pro Phe Leu Thr Cys Asp Val
   1               5                   10                  15
   Asp Thr Gly Gln Cys Leu Cys Leu Ser Tyr Val Thr Gly Ala His Cys
               20                  25                  30
   Glu Glu Cys Thr Val Gly Tyr Trp Gly Leu Gly Asn His Leu His Gly
           35                  40                  45
   Cys Ser Pro
       50
<210> 601
   <211> 47
   <212> PRT
   <213> Homo sapiens
<400> 601
   Cys Asp Cys Asp Ile Gly Gly Ala Tyr Ser Asn Val Cys Ser Pro Lys
   1               5                   10                  15
   Asn Gly Gln Cys Glu Cys Arg Pro His Val Thr Gly Arg Ser Cys Ser
               20                  25                  30
   Glu Pro Ala Pro Gly Tyr Phe Phe Ala Pro Leu Asn Phe Tyr Leu
           35                  40                  45
<210> 602
   <211> 48
   <212> PRT
   <213> Homo sapiens
<400> 602
   Cys Lys Cys His Pro Gln Gly Ser Val Gly Ser Ser Cys Ser Arg Leu
   1               5                   10                  15
   Gly Gly Gln Cys Gln Cys Lys Pro Leu Val Val Gly Arg Cys Cys Asp
               20                  25                  30
   Arg Cys Ser Thr Gly Ser Tyr Asp Leu Gly His His Gly Cys His Pro
           35                  40                  45
<210> 603
   <211> 46
   <212> PRT
   <213> Homo sapiens
<400> 603
   Cys His Cys His Pro Gln Gly Ser Lys Asp Thr Val Cys Asp Gln Val
   1               5                   10                  15
   Thr Gly Gln Cys Pro Cys His Gly Glu Val Ser Gly Arg Arg Cys Asp
               20                  25                  30
   Arg Cys Leu Ala Gly Tyr Phe Gly Phe Pro Ser Cys His Pro
           35                  40                  45
<210> 604
   <211> 48
   <212> PRT
   <213> Homo sapiens
<400> 604
   Cys Pro Cys Asn Arg Phe Ala Glu Leu Cys Asp Pro Glu Thr Gly Ser
   1               5                   10                  15
   Cys Phe Asn Cys Gly Gly Phe Thr Thr Gly Arg Asn Cys Glu Arg Cys
               20                  25                  30
   Ile Asp Gly Tyr Tyr Gly Asn Pro Ser Ser Gly Gln Pro Cys Arg Pro
           35                  40                  45
<210> 605
   <211> 59
   <212> PRT
   <213> Homo sapiens
<400> 605
   Cys Leu Cys Pro Asp Asp Pro Ser Ser Asn Gln Tyr Phe Ala His Ser
   1               5                   10                  15
   Cys Tyr Gln Asn Leu Trp Ser Ser Asp Val Ile Cys Asn Cys Leu Gln
               20                  25                  30
   Gly Tyr Thr Gly Thr Gln Cys Gly Glu Cys Ser Thr Gly Phe Tyr Gly
           35                  40                  45
   Asn Pro Arg Ile Ser Gly Ala Pro Cys Gln Pro
       50                  55
<210> 606
   <211> 56
   <212> PRT
   <213> Homo sapiens
<400> 606
   Cys Lys Cys Asn Gly His Ala Ser Glu Cys Met Lys Asn Glu Phe Asp
   1               5                   10                  15
   Lys Leu Val Cys Asn Cys Lys His Asn Thr Tyr Gly Val Asp Cys Glu
               20                  25                  30
   Lys Cys Leu Pro Phe Phe Asn Asp Arg Pro Trp Arg Arg Ala Thr Ala
           35                  40                  45
   Glu Ser Ala Ser Glu Cys Leu Pro
       50                  55
<210> 607
   <211> 48
   <212> PRT
   <213> Homo sapiens
<400> 607
   Cys Asp Cys His Pro Glu Gly Ser Leu Ser Leu Gln Cys Lys Asp Asp
   1               5                   10                  15
   Gly Arg Cys Glu Cys Arg Glu Gly Phe Val Gly Asn Arg Cys Asp Gln
               20                  25                  30
   Cys Glu Glu Asn Tyr Phe Tyr Asn Arg Ser Trp Pro Gly Cys Gln Glu
           35                  40                  45
<210> 608
   <211> 56
   <212> PRT
   <213> Homo sapiens
<400> 608
   Cys Asp Cys Asn Gly Arg Ser Gln Glu Cys Tyr Phe Asp Pro Glu Leu
   1               5                   10                  15
   Tyr Arg Ser Thr Gly His Gly Gly His Cys Thr Asn Cys Gln Asp Asn
               20                  25                  30
   Thr Asp Gly Ala His Cys Glu Arg Cys Arg Glu Asn Phe Phe Arg Leu
           35                  40                  45
   Gly Asn Asn Glu Ala Cys Ser Ser
       50                  55
<210> 609
   <211> 47
   <212> PRT
   <213> Homo sapiens
<400> 609
   Cys His Cys Ser Pro Val Gly Ser Leu Ser Thr Gln Cys Asp Ser Tyr
   1               5                   10                  15
   Gly Arg Cys Ser Cys Lys Pro Gly Val Met Gly Asp Lys Cys Asp Arg
               20                  25                  30
   Cys Gln Pro Gly Phe His Ser Leu Thr Glu Ala Gly Cys Arg Pro
           35                  40                  45
<210> 610
   <211> 50
   <212> PRT
   <213> Homo sapiens
<400> 610
   Cys Ser Cys Asp Pro Ser Gly Ser Ile Asp Glu Cys Asn Ile Glu Thr
   1               5                   10                  15
   Gly Arg Cys Val Cys Lys Asp Asn Val Glu Gly Phe Asn Cys Glu Arg
               20                  25                  30
   Cys Lys Pro Gly Phe Phe Asn Leu Glu Ser Ser Asn Pro Arg Gly Cys
           35                  40                  45
   Thr Pro
       50
<210> 611
   <211> 49
   <212> PRT
   <213> Homo sapiens
<400> 611
   Cys Ala Cys Asn Gly His Ser Glu Thr Cys Asp Pro Glu Thr Gly Val
   1               5                   10                  15
   Cys Asn Cys Arg Asp Asn Thr Ala Gly Pro His Cys Glu Lys Cys Ser
               20                  25                  30
   Asp Gly Tyr Tyr Gly Asp Ser Thr Ala Gly Thr Ser Ser Asp Cys Gln
                   35                  40                  45
   Pro
<210> 612
   <211> 55
   <212> PRT
   <213> Homo sapiens
<400> 612
   Cys Pro Cys Pro Gly Gly Ser Ser Cys Ala Val Val Pro Lys Thr Lys
   1               5                   10                  15
   Glu Val Val Cys Thr Asn Cys Pro Thr Gly Thr Thr Gly Lys Arg Cys
               20                  25                  30
   Glu Leu Cys Asp Asp Gly Tyr Phe Gly Asp Pro Leu Gly Arg Asn Gly
           35                  40                  45
   Pro Val Arg Leu Cys Arg Leu
       50                  55
<210> 613
   <211> 56
   <212> PRT
   <213> Homo sapiens
<400> 613
   Cys Gln Cys Ser Asp Asn Ile Asp Pro Asn Ala Val Gly Asn Cys Asn
   1               5                   10                  15
   Arg Leu Thr Gly Glu Cys Leu Lys Cys Ile Tyr Asn Thr Ala Gly Phe
               20                  25                  30
   Tyr Cys Asp Arg Cys Lys Asp Gly Phe Phe Gly Asn Pro Leu Ala Pro
           35                  40                  45
   Asn Pro Ala Asp Lys Cys Lys Ala
       50                  55
<210> 614
   <211> 51
   <212> PRT
   <213> Homo sapiens
<400> 614
   Cys Asn Cys Asn Leu Tyr Gly Thr Met Lys Gln Gln Ser Ser Cys Asn
   1               5                   10                  15
   Pro Val Thr Gly Gln Cys Glu Cys Leu Pro His Val Thr Gly Gln Asp
               20                  25                  30
   Cys Gly Ala Cys Asp Pro Gly Phe Tyr Asn Leu Gln Ser Gly Gln Gly
           35                  40                  45
   Cys Glu Arg
       50
<210> 615
   <211> 48
   <212> PRT
   <213> Homo sapiens
<400> 615
   Cys Asp Cys His Ala Leu Gly Ser Thr Asn Gly Gln Cys Asp Ile Arg
   1               5                   10                  15
   Thr Gly Gln Cys Glu Cys Gln Pro Gly Ile Thr Gly Gln His Cys Glu
               20                  25                  30
   Arg Cys Glu Val Asn His Phe Gly Phe Gly Pro Glu Gly Cys Lys Pro
           35                  40                  45
<210> 616
   <211> 56
   <212> PRT
   <213> Homo sapiens
<400> 616
   Cys Asp Cys Asn Gly Lys Ser Arg Gln Cys Ile Phe Asp Arg Glu Leu
   1               5                   10                  15
   His Arg Gln Thr Gly Asn Gly Phe Arg Cys Leu Asn Cys Asn Asp Asn
               20                  25                  30
   Thr Asp Gly Ile His Cys Glu Lys Cys Lys Asn Gly Phe Tyr Arg His
           35                  40                  45
   Arg Glu Arg Asp Arg Cys Leu Pro
       50                  55
<210> 617
   <211> 47
   <212> PRT
   <213> Homo sapiens
<400> 617
   Cys Asn Cys Asn Ser Lys Gly Ser Leu Ser Ala Arg Cys Asp Asn Ser
   1               5                   10                  15
   Gly Arg Cys Ser Cys Lys Pro Gly Val Thr Gly Ala Arg Cys Asp Arg
               20                  25                  30
   Cys Leu Pro Gly Phe His Met Leu Thr Asp Ala Gly Cys Thr Gln
           35                  40                  45
<210> 618
   <211> 48
   <212> PRT
   <213> Homo sapiens
<400> 618
   Cys Asp Cys Asp Pro Ala Gly Ile Ala Gly Pro Cys Asp Ala Gly Arg
   1               5                   10                  15
   Cys Val Cys Lys Pro Ala Val Thr Gly Glu Arg Cys Asp Arg Cys Arg
               20                  25                  30
   Ser Gly Tyr Tyr Asn Leu Asp Gly Gly Asn Pro Glu Gly Cys Thr Gln
           35                  40                  45
<210> 619
   <211> 46
   <212> PRT
   <213> Homo sapiens
<400> 619
   Cys Asn Cys Gln Gly Gly Gly Ala Cys Asp Pro Asp Thr Gly Asp Cys
   1               5                   10                  15
   Tyr Ser Gly Asp Glu Asn Pro Asp Ile Glu Cys Ala Asp Cys Pro Ile
               20                  25                  30
   Gly Phe Tyr Asn Asp Pro His Asp Pro Arg Ser Cys Lys Pro
           35                  40                  45
<210> 620
   <211> 55
   <212> PRT
   <213> Homo sapiens
<400> 620
   Cys Pro Cys His Asn Gly Phe Ser Cys Ser Val Met Pro Glu Thr Glu
   1               5                   10                  15
   Glu Val Val Cys Asn Asn Cys Pro Pro Gly Val Thr Gly Ala Arg Cys
               20                  25                  30
   Glu Leu Cys Ala Asp Gly Tyr Phe Gly Asp Pro Phe Gly Glu His Gly
           35                  40                  45
   Pro Val Arg Pro Cys Gln Pro
       50                  55
<210> 621
   <211> 56
   <212> PRT
   <213> Homo sapiens
<400> 621
   Cys Gln Cys Asn Asn Asn Val Asp Pro Ser Ala Ser Gly Asn Cys Asp
   1               5                   10                  15
   Arg Leu Thr Gly Arg Cys Leu Lys Cys Ile His Asn Thr Ala Gly Ile
               20                  25                  30
   Tyr Cys Asp Gln Cys Lys Ala Gly Tyr Phe Gly Asp Pro Leu Ala Pro
           35                  40                  45
   Asn Pro Ala Asp Lys Cys Arg Ala
       50                  55
<210> 622
   <211> 30
   <212> PRT
   <213> Homo sapiens
<400> 622
   Cys Asn Cys Asn Pro Met Gly Ser Glu Pro Val Gly Cys Arg Ser Asp
   1               5                   10                  15
   Gly Thr Cys Val Cys Lys Pro Gly Phe Gly Gly Pro Asn Cys
               20                  25                  30
<210> 623
   <211> 56
   <212> PRT
   <213> Homo sapiens
<400> 623
   Cys Lys Cys Asn Gly His Ala Ser Glu Cys Gly Pro Asp Val Ala Gly
   1               5                   10                  15
   Gln Leu Ala Cys Arg Cys Gln His Asn Thr Thr Gly Thr Asp Cys Glu
               20                  25                  30
   Arg Cys Leu Pro Phe Phe Gln Asp Arg Pro Trp Ala Arg Gly Thr Ala
           35                  40                  45
   Glu Ala Ala His Glu Cys Leu Pro
       50                  55
<210> 624
   <211> 49
   <212> PRT
   <213> Homo sapiens
<400> 624
   Cys Arg Cys Ser Pro Leu Gly Ala Ala Ser Ala Gln Cys His Glu Asn
   1               5                   10                  15
   Gly Thr Cys Val Cys Arg Pro Gly Phe Glu Gly Tyr Lys Cys Asp Arg
               20                  25                  30
   Cys His Asp Asn Phe Phe Leu Thr Ala Asp Gly Thr His Cys Gln Gln
           35                  40                  45
   Cys
<210> 625
   <211> 56
   <212> PRT
   <213> Homo sapiens
<400> 625
   Cys Asn Cys Ser Gly Arg Ser Glu Glu Cys Thr Phe Asp Arg Glu Leu
   1               5                   10                  15
   Phe Arg Ser Thr Gly His Gly Gly Arg Cys His His Cys Arg Asp His
               20                  25                  30
   Thr Ala Gly Pro His Cys Glu Arg Cys Gln Glu Asn Phe Tyr His Trp
           35                  40                  45
   Asp Pro Arg Met Pro Cys Gln Pro
       50                  55
<210> 626
   <211> 47
   <212> PRT
   <213> Homo sapiens
<400> 626
   Cys Asp Cys Gln Ser Ala Gly Ser Leu His Leu Gln Cys Asp Asp Thr
   1               5                   10                  15
   Gly Thr Cys Ala Cys Lys Pro Thr Val Thr Gly Trp Lys Cys Asp Arg
               20                  25                  30
   Cys Leu Pro Gly Phe His Ser Leu Ser Glu Gly Gly Cys Arg Pro
           35                  40                  45
<210> 627
   <211> 50
   <212> PRT
   <213> Homo sapiens
<400> 627
   Cys Thr Cys Asn Pro Ala Gly Ser Leu Asp Thr Cys Asp Pro Arg Ser
   1               5                   10                  15
   Gly Arg Cys Pro Cys Lys Glu Asn Val Glu Gly Asn Leu Cys Asp Arg
               20                  25                  30
   Cys Arg Pro Gly Thr Phe Asn Leu Gln Pro His Asn Pro Ala Gly Cys
           35                  40                  45
   Ser Ser
       50
<210> 628
   <211> 48
   <212> PRT
   <213> Homo sapiens
<400> 628
   Cys Thr Cys Asn Gln His Gly Thr Cys Asp Pro Asn Thr Gly Ile Cys
   1               5                   10                  15
   Val Cys Ser His His Thr Glu Gly Pro Ser Cys Glu Arg Cys Leu Pro
               20                  25                  30
   Gly Phe Tyr Gly Asn Pro Phe Ala Gly Gln Ala Asp Asp Cys Gln Pro
           35                  40                  45
<210> 629
   <211> 55
   <212> PRT
   <213> Homo sapiens
<400> 629
   Cys Pro Cys Pro Gly Gln Ser Ala Cys Thr Thr Ile Pro Glu Ser Arg
   1               5                   10                  15
   Glu Val Val Cys Thr His Cys Pro Pro Gly Gln Arg Gly Arg Arg Cys
                   20                      25                      30
   Glu Val Cys Asp Asp Gly Phe Phe Gly Asp Pro Leu Gly Leu Phe Gly
               35                      40                      45
   His Pro Gln Pro Cys His Gln
           50                      55
<210> 630
   <211> 56
   <212> PRT
   <213> Homo sapiens
<400> 630
   Cys Gln Cys Ser Gly Asn Val Asp Pro Asn Ala Val Gly Asn Cys Asp
   1               5                   10                  15
   Pro Leu Ser Gly His Cys Leu Arg Cys Leu His Asn Thr Thr Gly Asp
               20                  25                  30
   His Cys Glu His Cys Gln Glu Gly Phe Tyr Gly Ser Ala Leu Ala Pro
           35                  40                  45
   Arg Pro Ala Asp Lys Cys Met Pro
       50                  55
<210> 631
   <211> 51
   <212> PRT
   <213> Homo sapiens
<400> 631
   Cys Ser Cys His Pro Gln Gly Ser Val Ser Glu Gln Met Pro Cys Asp
   1               5                   10                  15
   Pro Val Thr Gly Gln Cys Ser Cys Leu Pro His Val Thr Ala Arg Asp
               20                  25                  30
   Cys Ser Arg Cys Tyr Pro Gly Phe Phe Asp Leu Gln Pro Gly Arg Gly
           35                  40                  45
   Cys Arg Ser
       50
<210> 632
   <211> 48
   <212> PRT
   <213> Homo sapiens
<400> 632
   Cys Lys Cys His Pro Leu Gly Ser Gln Glu Asp Gln Cys His Pro Lys
   1               5                   10                  15
   Thr Gly Gln Cys Thr Cys Arg Pro Gly Val Thr Gly Gln Ala Cys Asp
               20                  25                  30
   Arg Cys Gln Leu Gly Phe Phe Gly Phe Ser Ile Lys Gly Cys Arg Ala
           35                  40                  45
<210> 633
   <211> 33
   <212> PRT
   <213> Homo sapiens
<400> 633
   Thr Lys Pro Ser Cys Val Pro Pro Cys Gln Asn Gly Gly Met Cys Leu
   1 5 10 15
   Arg Pro Gln Leu Cys Val Cys Lys Pro Gly Thr Lys Gly Lys Ala Cys
   20 25 30
   Glu
<210> 634
   <211> 41
   <212> PRT
   <213> Homo sapiens
<400> 634
   Asp Ile Asp Glu Cys Gln His Arg His Leu Cys Ala His Gly Gln Cys
   1 5 10 15
   Arg Asn Thr Glu Gly Ser Phe Gln Cys Val Cys Asp Gln Gly Tyr Arg
   20 25 30
   Ala Ser Gly Leu Gly Asp His Cys Glu
   35 40
<210> 635
   <211> 41
   <212> PRT
   <213> Homo sapiens
<400> 635
   Asp Ile Asn Glu Cys Leu Glu Asp Lys Ser Val Cys Gln Arg Gly Asp
   1 5 10 15
   Cys Ile Asn Thr Ala Gly Ser Tyr Asp Cys Thr Cys Pro Asp Gly Phe
   20 25 30
   Gln Leu Asp Asp Asn Lys Thr Cys Gln
   35 40
<210> 636
   <211> 42
   <212> PRT
   <213> Homo sapiens
<400> 636
   Asp Ile Asn Glu Cys Glu His Pro Gly Leu Cys Gly Pro Gln Gly Glu
   1 5 10 15
   Cys Leu Asn Thr Glu Gly Ser Phe His Cys Val Cys Gln Gln Gly Phe
   20 25 30
   Ser Ile Ser Ala Asp Gly Arg Thr Cys Glu
   35 40
<210> 637
   <211> 38
   <212> PRT
   <213> Homo sapiens
<400> 637
   Asp Ile Asp Glu Cys Val Asn Asn Thr Val Cys Asp Ser His Gly Phe
   1               5                   10                  15
   Cys Asp Asn Thr Ala Gly Ser Phe Arg Cys Leu Cys Tyr Gln Gly Phe
               20                  25                  30
   Gln Ala Pro Gln Asp Gly
           35
<210> 638
   <211> 43
   <212> PRT
   <213> Homo sapiens
<400> 638
   Asp Val Asn Glu Cys Glu Leu Leu Ser Gly Val Cys Gly Glu Ala Phe
   1               5                   10                  15
   Cys Glu Asn Val Glu Gly Ser Phe Leu Cys Val Cys Ala Asp Glu Asn
               20                  25                  30
   Gln Glu Tyr Ser Pro Met Thr Gly Gln Cys Arg
           35                  40
<210> 639
   <211> 43
   <212> PRT
   <213> Homo sapiens
<400> 639
   Asp Ala Asp Glu Cys Leu Leu Phe Gly Gln Glu Ile Cys Lys Asn Gly
   1               5                   10                  15
   Phe Cys Leu Asn Thr Arg Pro Gly Tyr Glu Cys Tyr Cys Lys Gln Gly
               20                  25                  30
   Thr Tyr Tyr Asp Pro Val Lys Leu Gln Cys Phe
           35                  40
<210> 640
   <211> 37
   <212> PRT
   <213> Homo sapiens
<400> 640
   Asp Met Asp Glu Cys Gln Asp Pro Ser Ser Cys Ile Asp Gly Gln Cys
   1               5                   10                  15
   Val Asn Thr Glu Gly Ser Tyr Asn Cys Phe Cys Thr His Pro Met Val
               20                  25                  30
   Leu Asp Ala Ser Glu
           35
<210> 641
   <211> 37
   <212> PRT
   <213> Homo sapiens
<400> 641
   Gln Ala Glu Glu Cys Gly Ile Leu Asn Gly Cys Glu Asn Gly Arg Cys
   1               5                   10                  15
   Val Arg Val Gln Glu Gly Tyr Thr Cys Asp Cys Phe Asp Gly Tyr His
               20                  25                  30
   Leu Asp Thr Ala Lys
           35
<210> 642
   <211> 45
   <212> PRT
   <213> Homo sapiens
<400> 642
   Asp Val Asn Glu Cys Asp Glu Leu Asn Asn Arg Met Ser Leu Cys Lys
   1               5                   10                  15
   Asn Ala Lys Cys Ile Asn Thr Asp Gly Ser Tyr Lys Cys Leu Cys Leu
               20                  25                  30
   Pro Gly Tyr Val Pro Ser Asp Lys Pro Asn Tyr Cys Thr
           35                  40                  45
<210> 643
   <211> 33
   <212> PRT
   <213> Homo sapiens
<400> 643
   Arg Val Val Ile Cys His Leu Pro Cys Met Asn Gly Gly Gln Cys Ser
   1               5                   10                  15
   Ser Arg Asp Lys Cys Gln Cys Pro Pro Asn Phe Thr Gly Lys Leu Cys
               20                  25                  30
   Gln
<210> 644
   <211> 38
   <212> PRT
   <213> Homo sapiens
<400> 644
   Asp Ile Asn Glu Cys Gln Leu Gln Gly Val Cys Pro Asn Gly Glu Cys
   1               5                   10                  15
   Leu Asn Thr Met Gly Ser Tyr Arg Cys Thr Cys Lys Ile Gly Phe Gly
               20                  25                  30
   Pro Asp Pro Thr Phe Ser
           35
<210> 645
   <211> 38
   <212> PRT
   <213> Homo sapiens
<400> 645
   Glu Ile Asn Glu Cys Thr Val Asn Pro Asp Ile Cys Gly Ala Gly His
   1               5                   10                  15
   Cys Ile Asn Leu Pro Val Arg Tyr Thr Cys Ile Cys Tyr Glu Gly Tyr
               20                  25                  30
   Arg Phe Ser Glu Gln Gln
           35
<210> 646
   <211> 42
   <212> PRT
   <213> Homo sapiens
<400> 646
   Asp Ile Asp Glu Cys Thr Gln Val Gln His Leu Cys Ser Gln Gly Arg
   1               5                   10                  15
   Cys Glu Asn Thr Glu Gly Ser Phe Leu Cys Ile Cys Pro Ala Gly Phe
               20                  25                  30
   Met Ala Ser Glu Glu Gly Thr Asn Cys Ile
           35                  40
<210> 647
   <211> 41
   <212> PRT
   <213> Homo sapiens
<400> 647
   Asp Val Asp Glu Cys Leu Arg Pro Asp Val Cys Gly Glu Gly His Cys
   1               5                   10                  15
   Val Asn Thr Val Gly Ala Phe Arg Cys Glu Tyr Cys Asp Ser Gly Tyr
               20                  25                  30
   Arg Met Thr Gln Arg Gly Arg Cys Glu
           35                  40
<210> 648
   <211> 40
   <212> PRT
   <213> Homo sapiens
<400> 648
   Asp Ile Asp Glu Cys Leu Asn Pro Ser Thr Cys Pro Asp Glu Gln Cys
   1               5                   10                  15
   Val Asn Ser Pro Gly Ser Tyr Gln Cys Val Pro Cys Thr Glu Gly Phe
               20                  25                  30
   Arg Gly Trp Asn Gly Gln Cys Leu
           35                  40
<210> 649
   <211> 41
   <212> PRT
   <213> Homo sapiens
<400> 649
   Asp Val Asp Glu Cys Leu Glu Pro Asn Val Cys Ala Asn Gly Asp Cys
   1               5                   10                  15
   Ser Asn Leu Glu Gly Ser Tyr Met Cys Ser Cys His Lys Gly Tyr Thr
               20                  25                  30
   Arg Thr Pro Asp His Lys His Cys Arg
           35                  40
<210> 650
   <211> 41
   <212> PRT
   <213> Homo sapiens
<400> 650
   Asp Ile Asp Glu Cys Gln Gln Gly Asn Leu Cys Val Asn Gly Gln Cys
   1               5                   10                  15
   Lys Asn Thr Glu Gly Ser Phe Arg Cys Thr Cys Gly Gln Gly Tyr Gln
               20                  25                  30
   Leu Ser Ala Ala Lys Asp Gln Cys Glu
           35                  40
<210> 651
   <211> 33
   <212> PRT
   <213> Homo sapiens
<400> 651
   Ile Lys Pro Val Cys Glu Pro Pro Cys Gln Asn Arg Gly Ser Cys Ser
   1               5                   10                  15
   Arg Pro Gln Leu Cys Val Cys Arg Ser Gly Phe Arg Gly Ala Arg Cys
               20                  25                  30
   Glu
<210> 652
   <211> 42
   <212> PRT
   <213> Homo sapiens
<400> 652
   Asp Leu Asp Glu Cys Ala Phe Pro Gly Val Cys Pro Ser Gly Val Cys
   1               5                   10                  15
   Thr Asn Thr Ala Gly Ser Phe Ser Cys Lys Asp Cys Asp Gly Gly Tyr
               20                  25                  30
   Arg Pro Ser Pro Leu Gly Asp Ser Cys Glu
           35                  40
<210> 653
   <211> 41
   <212> PRT
   <213> Homo sapiens
<400> 653
   Asp Val Asp Glu Cys Glu Asp Pro Gln Ser Ser Cys Leu Gly Gly Glu
   1               5                   10                  15
   Cys Lys Asn Thr Val Gly Ser Tyr Gln Cys Leu Cys Pro Gln Gly Phe
               20                  25                  30
   Gln Leu Ala Asn Gly Thr Val Cys Glu
           35                  40
<210> 654
   <211> 42
   <212> PRT
   <213> Homo sapiens
<400> 654
   Asp Val Asn Glu Cys Met Gly Glu Glu His Cys Ala Pro His Gly Glu
   1               5                   10                  15
   Cys Leu Asn Ser His Gly Ser Phe Phe Cys Leu Cys Ala Pro Gly Phe
               20                  25                  30
   Val Ser Ala Glu Gly Gly Thr Ser Cys Gln
           35                  40
<210> 655
   <211> 41
   <212> PRT
   <213> Homo sapiens
<400> 655
   Asp Val Asp Glu Cys Ala Thr Thr Asp Pro Cys Val Gly Gly His Cys
   1               5                   10                  15
   Val Asn Thr Glu Gly Ser Phe Asn Cys Leu Cys Glu Thr Gly Phe Gln
               20                  25                  30
   Pro Ser Pro Glu Ser Gly Glu Cys Val
           35                  40
<210> 656
   <211> 44
   <212> PRT
   <213> Homo sapiens
<400> 656
   Asp Ile Asp Glu Cys Glu Asp Tyr Gly Asp Pro Val Cys Gly Thr Trp
   1               5                   10                  15
   Lys Cys Glu Asn Ser Pro Gly Ser Tyr Arg Cys Val Leu Gly Cys Gln
               20                  25                  30
   Pro Gly Phe His Met Ala Pro Asn Gly Asp Cys Ile
           35                  40
<210> 657
   <211> 42
   <212> PRT
   <213> Homo sapiens
<400> 657
   Asp Ile Asp Glu Cys Ala Asn Asp Thr Met Cys Gly Ser His Gly Phe
   1               5                   10                  15
   Cys Asp Asn Thr Asp Gly Ser Phe Arg Cys Leu Cys Asp Gln Gly Phe
               20                  25                  30
   Glu Ile Ser Pro Ser Gly Trp Asp Cys Val
           35                  40
<210> 658
   <211> 43
   <212> PRT
   <213> Homo sapiens
<400> 658
   Asp Val Asn Glu Cys Glu Leu Met Leu Ala Val Cys Gly Ala Ala Leu
   1               5                   10                  15
   Cys Glu Asn Val Glu Gly Ser Phe Leu Cys Leu Cys Ala Ser Asp Leu
               20                  25                  30
   Glu Glu Tyr Asp Ala Gln Glu Gly His Cys Arg
           35                  40
<210> 659
   <211> 43
   <212> PRT
   <213> Homo sapiens
<400> 659
   Asp Ala Asp Glu Cys Val Ile Phe Gly Pro Gly Leu Cys Pro Asn Gly
   1               5                   10                  15
   Arg Cys Leu Asn Thr Val Pro Gly Tyr Val Cys Leu Cys Asn Pro Gly
               20                  25                  30
   Phe His Tyr Asp Ala Ser His Lys Lys Cys Glu
           35                  40
<210> 660
   <211> 40
   <212> PRT
   <213> Homo sapiens
<400> 660
   Asp His Asp Glu Cys Gln Asp Leu Ala Cys Glu Asn Gly Glu Cys Val
   1               5                   10                  15
   Asn Thr Glu Gly Ser Phe His Cys Phe Cys Ser Pro Pro Leu Thr Leu
               20                  25                  30
   Asp Leu Ser Gln Gln Arg Cys Met
           35                  40
<210> 661
   <211> 41
   <212> PRT
   <213> Homo sapiens
<400> 661
   Gln Ala Glu Glu Cys Gly Ile Leu Asn Gly Cys Glu Asn Gly Arg Cys
   1               5                   10                  15
   Val Arg Val Arg Glu Gly Tyr Thr Cys Asp Cys Phe Glu Gly Phe Gln
               20                  25                  30
   Leu Asp Ala Ala His Met Ala Cys Val
           35                  40
<210> 662
   <211> 33
   <212> PRT
   <213> Homo sapiens
<400> 662
   Arg Ile Tyr Phe Cys Gln Ile Pro Cys Leu Asn Gly Gly Arg Cys Ile
   1               5                   10                  15
   Gly Arg Asp Glu Cys Trp Cys Pro Ala Asn Ser Thr Gly Lys Phe Cys
               20                  25                  30
   His
<210> 663
   <211> 45
   <212> PRT
   <213> Homo sapiens
<400> 663
   Asp Val Asn Glu Cys Asp Asp Leu Asn Gly Pro Ala Val Leu Cys Val
   1               5                   10                  15
   His Gly Tyr Cys Glu Asn Thr Glu Gly Ser Tyr Arg Cys His Cys Ser
               20                  25                  30
   Pro Gly Tyr Val Ala Glu Ala Gly Pro Pro His Cys Thr
           35                  40                  45
<210> 664
   <211> 41
   <212> PRT
   <213> Homo sapiens
<400> 664
   Asp Ile Asn Glu Cys Leu Thr Leu Gly Leu Cys Lys Asp Ala Glu Cys
   1               5                   10                  15
   Val Asn Thr Arg Gly Ser Tyr Leu Cys Thr Cys Arg Pro Gly Leu Met
               20                  25                  30
   Leu Asp Pro Ser Arg Ser Arg Cys Val
           35                  40
<210> 665
   <211> 43
   <212> PRT
   <213> Homo sapiens
<400> 665
   Gly Ile Asp Arg Cys Ala Ala Gly Ala Thr Asn Val Cys Gly Pro Gly
   1               5                   10                  15
   Thr Cys Val Asn Leu Pro Asp Gly Tyr Arg Cys Val Cys Ser Pro Gly
               20                  25                  30
   Tyr Gln Leu His Pro Ser Gln Ala Tyr Cys Thr
           35                  40
<210> 666
   <211> 43
   <212> PRT
   <213> Homo sapiens
<400> 666
   Asp Asp Asn Glu Cys Leu Arg Asp Pro Cys Lys Gly Lys Gly Arg Cys
   1               5                   10                  15
   Ile Asn Arg Val Gly Ser Tyr Ser Cys Phe Cys Tyr Pro Gly Tyr Thr
               20                  25                  30
   Leu Ala Thr Ser Gly Ala Thr Gln Glu Cys Gln
           35                  40
<210> 667
   <211> 40
   <212> PRT
   <213> Homo sapiens
<400> 667
   Asp Ile Asn Glu Cys Glu Gln Pro Gly Val Cys Ser Gly Gly Gln Cys
   1               5                   10                  15
   Thr Asn Thr Glu Gly Ser Tyr His Cys Glu Cys Asp Gln Gly Tyr Ile
               20                  25                  30
   Met Val Arg Lys Gly His Cys Gln
           35                  40
<210> 668
   <211> 40
   <212> PRT
   <213> Homo sapiens
<400> 668
   Asp Ile Asn Glu Cys Arg His Pro Gly Thr Cys Pro Asp Gly Arg Cys
   1               5                   10                  15
   Val Asn Ser Pro Gly Ser Tyr Thr Cys Leu Ala Cys Glu Glu Gly Tyr
               20                  25                  30
   Arg Gly Gln Ser Gly Ser Cys Val
           35                  40
<210> 669
   <211> 41
   <212> PRT
   <213> Homo sapiens
<400> 669
   Asp Val Asn Glu Cys Leu Thr Pro Gly Val Cys Ala His Gly Lys Cys
   1               5                   10                  15
   Thr Asn Leu Glu Gly Ser Phe Arg Cys Ser Cys Glu Gln Gly Tyr Glu
               20                  25                  30
   Val Thr Ser Asp Glu Lys Gly Cys Gln
           35                  40
<210> 670
   <211> 42
   <212> PRT
   <213> Homo sapiens
<400> 670
   Asp Val Asp Glu Cys Ala Ser Arg Ala Ser Cys Pro Thr Gly Leu Cys
   1               5                   10                  15
   Leu Asn Thr Glu Gly Ser Phe Ala Cys Ser Ala Cys Glu Asn Gly Tyr
               20                  25                  30
   Trp Val Asn Glu Asp Gly Thr Ala Cys Glu
           35                  40
<210> 671
   <211> 33
   <212> PRT
   <213> Homo sapiens
<400> 671
   Arg Val Val Val Cys Pro Leu Pro Cys Met Asn Gly Gly Gln Cys Ser
   1               5                   10                  15
   Ser Arg Asn Gln Cys Leu Cys Pro Pro Asp Phe Thr Gly Arg Phe Cys
               20                  25                  30
   Gln
<210> 672
   <211> 43
   <212> PRT
   <213> Homo sapiens
<400> 672
   Asp Ile Asp Glu Cys Met Leu Phe Gly Ser Glu Ile Cys Lys Glu Gly
   1               5                   10                  15
   Lys Cys Val Asn Thr Gln Pro Gly Tyr Glu Cys Tyr Cys Lys Gln Gly
               20                  25                  30
   Phe Tyr Tyr Asp Gly Asn Leu Leu Glu Cys Val
           35                  40
<210> 673
   <211> 41
   <212> PRT
   <213> Homo sapiens
<400> 673
   Asp Val Asp Glu Cys Leu Asp Glu Ser Asn Cys Arg Asn Gly Val Cys
   1               5                   10                  15
   Glu Asn Thr Arg Gly Gly Tyr Arg Cys Ala Cys Thr Pro Pro Ala Glu
               20                  25                  30
   Tyr Ser Pro Ala Gln Arg Gln Cys Leu
           35                  40
<210> 674
   <211> 41
   <212> PRT
   <213> Homo sapiens
<400> 674
   Asp Val Asp Glu Cys Gln Asp Pro Ala Ala Cys Arg Pro Gly Arg Cys
   1               5                   10                  15
   Val Asn Leu Pro Gly Ser Tyr Arg Cys Glu Cys Arg Pro Pro Trp Val
               20                  25                  30
   Pro Gly Pro Ser Gly Arg Asp Cys Gln
           35                  40
<210> 675
   <211> 45
   <212> PRT
   <213> Homo sapiens
<400> 675
   Asp Ile Asp Glu Cys Arg Glu Leu Asn Gln Arg Gly Leu Leu Cys Lys
   1               5                   10                  15
   Ser Glu Arg Cys Val Asn Thr Ser Gly Ser Phe Arg Cys Val Cys Lys
               20                  25                  30
   Ala Gly Phe Ala Arg Ser Arg Pro His Gly Ala Cys Val
           35                  40                  45
<210> 676
   <211> 41
   <212> PRT
   <213> Homo sapiens
<400> 676
   Asp Ile Asn Glu Cys Ala Met Pro Gly Val Cys Arg His Gly Asp Cys
   1               5                   10                  15
   Leu Asn Asn Pro Gly Ser Tyr Arg Cys Val Cys Pro Pro Gly His Ser
               20                  25                  30
   Leu Gly Pro Ser Arg Thr Gln Cys Ile
           35                  40
<210> 677
   <211> 42
   <212> PRT
   <213> Homo sapiens
<400> 677
   Glu Thr Asp Glu Cys Arg Leu Asn Gln Asn Ile Cys Gly His Gly Glu
   1               5                   10                  15
   Cys Val Pro Gly Pro Pro Asp Tyr Ser Cys His Cys Asn Pro Gly Tyr
               20                  25                  30
   Arg Ser His Pro Gln His Arg Tyr Cys Val
           35                  40
<210> 678
   <211> 44
   <212> PRT
   <213> Homo sapiens
<400> 678
   Asp Val Asn Glu Cys Glu Ala Glu Pro Cys Gly Pro Gly Arg Gly Ile
   1               5                   10                  15
   Cys Met Asn Thr Gly Gly Ser Tyr Asn Cys His Cys Asn Arg Gly Tyr
               20                  25                  30
   Arg Leu His Val Gly Ala Gly Gly Arg Ser Cys Val
           35                  40
<210> 679
   <211> 43
   <212> PRT
   <213> Homo sapiens
<400> 679
   Asp Leu Asn Glu Cys Ala Lys Pro His Leu Cys Gly Asp Gly Gly Phe
   1               5                   10                  15
   Cys Ile Asn Phe Pro Gly His Tyr Lys Cys Asn Cys Tyr Pro Gly Tyr
               20                  25                  30
   Arg Leu Lys Ala Ser Arg Pro Pro Val Cys Glu
           35                  40
<210> 680
   <211> 41
   <212> PRT
   <213> Homo sapiens
<400> 680
   Asp Val Asn Glu Cys Ala Glu Gly Ser Pro Cys Ser Pro Gly Trp Cys
   1               5                   10                  15
   Glu Asn Leu Pro Gly Ser Phe Arg Cys Thr Cys Ala Gln Gly Tyr Ala
               20                  25                  30
   Pro Ala Pro Asp Gly Arg Ser Cys Leu
           35                  40
<210> 681
   <211> 41
   <212> PRT
   <213> Homo sapiens
<400> 681
   Asp Val Asp Glu Cys Glu Ala Gly Asp Val Cys Asp Asn Gly Ile Cys
   1               5                   10                  15
   Ser Asn Thr Pro Gly Ser Phe Gln Cys Gln Cys Leu Ser Gly Tyr His
               20                  25                  30
   Leu Ser Arg Asp Arg Ser His Cys Glu
           35                  40
<210> 682
   <211> 40
   <212> PRT
   <213> Homo sapiens
<400> 682
   Asp Ile Asp Glu Cys Asp Phe Pro Ala Ala Cys Ile Gly Gly Asp Cys
   1               5                   10                  15
   Ile Asn Thr Asn Gly Ser Tyr Arg Cys Leu Cys Pro Gln Gly His Arg
               20                  25                  30
   Leu Val Gly Gly Arg Lys Cys Gln
           35                  40
<210> 683
   <211> 43
   <212> PRT
   <213> Homo sapiens
<400> 683
   Asp Ile Asp Glu Cys Ser Gln Asp Pro Ser Leu Cys Leu Pro His Gly
   1               5                   10                  15
   Ala Cys Lys Asn Leu Gln Gly Ser Tyr Val Cys Val Cys Asp Glu Gly
               20                  25                  30
   Phe Thr Pro Thr Gln Asp Gln His Gly Cys Glu
           35                  40
<210> 684
   <211> 33
   <212> PRT
   <213> Homo sapiens
<400> 684
   Ala Pro Val Leu Cys Pro Leu Ile Cys His Asn Gly Gly Val Cys Val
   1               5                   10                  15
   Lys Pro Asp Arg Cys Leu Cys Pro Pro Asp Phe Ala Gly Lys Phe Cys
               20                  25                  30
   Gln
<210> 685
   <211> 42
   <212> PRT
   <213> Homo sapiens
<400> 685
   Asp Val Asn Glu Cys Leu Glu Gly Asp Phe Cys Phe Pro His Gly Glu
   1 5 10 15
   Cys Leu Asn Thr Asp Gly Ser Phe Ala Cys Thr Cys Ala Pro Gly Tyr
   20 25 30
   Arg Pro Gly Pro Arg Gly Ala Ser Cys Leu
   35 40
<210> 686
   <211> 41
   <212> PRT
   <213> Homo sapiens
<400> 686
   Asp Val Asp Glu Cys Ser Glu Glu Asp Leu Cys Gln Ser Gly Ile Cys
   1 5 10 15
   Thr Asn Thr Asp Gly Ser Phe Glu Cys Ile Cys Pro Pro Gly His Arg
   20 25 30
   Ala Gly Pro Asp Leu Ala Ser Cys Leu
   35 40
<210> 687
   <211> 42
   <212> PRT
   <213> Homo sapiens
<400> 687
   Asp Val Asp Glu Cys Arg Asn Arg Ser Phe Cys Gly Ala His Ala Val
   1 5 10 15
   Cys Gln Asn Leu Pro Gly Ser Phe Gln Cys Leu Cys Asp Gln Gly Tyr
   20 25 30
   Glu Gly Ala Arg Asp Gly Arg His Cys Val
   35 40
<210> 688
   <211> 43
   <212> PRT
   <213> Homo sapiens
<400> 688
   Asp Val Asp Glu Cys Gln Leu Phe Arg Asp Gln Val Cys Lys Ser Gly
   1 5 10 15
   Val Cys Val Asn Thr Ala Pro Gly Tyr Ser Cys Tyr Cys Ser Asn Gly
   20 25 30
   Tyr Tyr Tyr His Thr Gln Arg Leu Glu Cys Ile
   35 40
<210> 689
   <211> 42
   <212> PRT
   <213> Homo sapiens
<400> 689
   Asp Asn Asp Glu Cys Ala Asp Glu Glu Pro Ala Cys Glu Gly Gly Arg
   1               5                   10                  15
   Cys Val Asn Thr Val Gly Ser Tyr His Cys Thr Cys Glu Pro Pro Leu
               20                  25                  30
   Val Leu Asp Gly Ser Gln Arg Arg Cys Val
           35                  40
<210> 690
   <211> 41
   <212> PRT
   <213> Homo sapiens
<400> 690
   Glu Ala Glu Glu Cys Gly Ile Leu Asp Gly Cys Thr Asn Gly Arg Cys
   1               5                   10                  15
   Val Arg Val Pro Glu Gly Phe Thr Cys Arg Cys Phe Asp Gly Tyr Arg
               20                  25                  30
   Leu Asp Met Thr Arg Met Ala Cys Val
           35                  40
<210> 691
   <211> 45
   <212> PRT
   <213> Homo sapiens
<400> 691
   Asp Ile Asn Glu Cys Asp Glu Ala Glu Ala Ala Ser Pro Leu Cys Val
   1               5                   10                  15
   Asn Ala Arg Cys Leu Asn Thr Asp Gly Ser Phe Arg Cys Ile Cys Arg
               20                  25                  30
   Pro Gly Phe Ala Pro Thr His Gln Pro His His Cys Ala
           35                  40                  45
<210> 692
   <211> 41
   <212> PRT
   <213> Homo sapiens
<400> 692
   Asp Val Asp Glu Cys Ala Thr Gly Gly Arg Cys Gln His Gly Glu Cys
   1               5                   10                  15
   Ala Asn Thr Arg Gly Gly Tyr Thr Cys Val Cys Pro Asp Gly Phe Leu
               20                  25                  30
   Leu Asp Ser Ser Arg Ser Ser Cys Ile
           35                  40
<210> 693
   <211> 42
   <212> PRT
   <213> Homo sapiens
<400> 693
   Ser Ser Gly Met Cys Gln Arg Asn Pro Gln Val Cys Gly Pro Gly Arg
   1               5                   10                  15
   Cys Ile Ser Arg Pro Ser Gly Tyr Thr Cys Ala Cys Asp Ser Gly Phe
               20                  25                  30
   Arg Leu Ser Pro Gln Gly Thr Arg Cys Ile
           35                  40
<210> 694
   <211> 42
   <212> PRT
   <213> Homo sapiens
<400> 694
   Asp Val Asp Glu Cys Arg Arg Val Pro Pro Pro Cys Ala Pro Gly Arg
   1               5                   10                  15
   Cys Glu Asn Ser Pro Gly Ser Phe Arg Cys Val Cys Gly Pro Gly Phe
               20                  25                  30
   Arg Ala Gly Pro Arg Ala Ala Glu Cys Leu
           35                  40
<210> 695
   <211> 42
   <212> PRT
   <213> Homo sapiens
<400> 695
   Asp Val Asp Glu Cys His Arg Val Pro Pro Pro Cys Asp Leu Gly Arg
   1               5                   10                  15
   Cys Glu Asn Thr Pro Gly Ser Phe Leu Cys Val Cys Pro Ala Gly Tyr
               20                  25                  30
   Gln Ala Ala Pro His Gly Ala Ser Cys Gln
           35                  40
<210> 696
   <211> 38
   <212> PRT
   <213> Homo sapiens
<400> 696
   Asp Val Asp Glu Cys Thr Gln Ser Pro Gly Leu Cys Gly Arg Gly Ala
   1               5                   10                  15
   Cys Lys Asn Leu Pro Gly Ser Phe Arg Cys Val Cys Pro Ala Gly Phe
               20                  25                  30
   Arg Gly Ser Ala Cys Glu
           35
<210> 697
   <211> 42
   <212> PRT
   <213> Homo sapiens
<400> 697
   Asp Val Asp Glu Cys Ala Gln Glu Pro Pro Pro Cys Gly Pro Gly Arg
   1               5                   10                  15
   Cys Asp Asn Thr Ala Gly Ser Phe His Cys Ala Cys Pro Ala Gly Phe
               20                  25                  30
   Arg Ser Arg Gly Pro Gly Ala Pro Cys Gln
           35                  40
<210> 698
   <211> 42
   <212> PRT
   <213> Homo sapiens
<400> 698
   Asp Val Asp Glu Cys Ala Arg Ser Pro Pro Pro Cys Thr Tyr Gly Arg
   1               5                   10                  15
   Cys Glu Asn Thr Glu Gly Ser Phe Gln Cys Val Cys Pro Met Gly Phe
               20                  25                  30
   Gln Pro Asn Thr Ala Gly Ser Glu Cys Glu
           35                  40
<210> 699
   <211> 44
   <212> PRT
   <213> Homo sapiens
<400> 699
   Asp Val Asp Glu Cys Ser Ser Gly Ala Pro Pro Cys Gly Pro His Gly
   1               5                   10                  15
   His Cys Thr Asn Thr Glu Gly Ser Phe Arg Cys Ser Cys Ala Pro Gly
               20                  25                  30
   Tyr Arg Ala Pro Ser Gly Arg Pro Gly Pro Cys Ala
           35                  40
<210> 700
   <211> 40
   <212> PRT
   <213> Homo sapiens
<400> 700
   Gly Thr Asn Glu Cys Leu Asp Asn Asn Gly Gly Cys Ser His Val Cys
   1               5                   10                  15
   Asn Asp Leu Lys Ile Gly Tyr Glu Cys Leu Cys Pro Asp Gly Phe Gln
               20                  25                  30
   Leu Val Ala Gln Arg Arg Cys Glu
           35                  40
<210> 701
   <211> 40
   <212> PRT
   <213> Homo sapiens
<400> 701
   Asp Ile Asp Glu Cys Gln Asp Pro Asp Thr Cys Ser Gln Leu Cys Val
   1               5                   10                  15
   Asn Leu Glu Gly Gly Tyr Lys Cys Gln Cys Glu Glu Gly Phe Gln Leu
               20                  25                  30
   Asp Pro His Thr Lys Ala Cys Lys
           35                  40
<210> 702
   <211> 50
   <212> PRT
   <213> Homo sapiens
<400> 702
   Gly Val Asn Trp Cys Glu Arg Thr Thr Leu Ser Asn Gly Gly Cys Gln
   1               5                   10                  15
   Tyr Leu Cys Leu Pro Ala Pro Gln Ile Asn Pro His Ser Pro Lys Phe
               20                  25                  30
   Thr Cys Ala Cys Pro Asp Gly Met Leu Leu Ala Arg Asp Met Arg Ser
           35                  40                  45
   Cys Leu
       50
<210> 703
   <211> 36
   <212> PRT
   <213> Homo sapiens
<400> 703
   Asn Cys Ser Arg Leu Gly Cys Gln His His Cys Val Pro Thr Leu Asp
   1               5                   10                  15
   Gly Pro Thr Cys Tyr Cys Asn Ser Ser Phe Gln Leu Gln Ala Asp Gly
               20                  25                  30
   Lys Thr Cys Lys
           35
<210> 704
   <211> 39
   <212> PRT
   <213> Homo sapiens
<400> 704
   Leu Leu Ser Asn Cys Gln Gln Leu Asn Cys Gln Tyr Lys Cys Thr Met
   1               5                   10                  15
   Val Arg Asn Ser Thr Arg Cys Tyr Cys Glu Asp Gly Phe Glu Ile Thr
               20                  25                  30
   Glu Asp Gly Arg Ser Cys Lys
           35
<210> 705
   <211> 34
   <212> PRT
   <213> Homo sapiens
<400> 705
   Leu Asp Asp Ser Cys Lys Leu Thr Cys Glu Asn Gly Gly Arg Cys Ile
   1               5                   10                  15
   Leu Asn Glu Lys Gly Asp Leu Arg Cys His Cys Trp Pro Ser Tyr Ser
               20                  25                  30
   Gly Glu
<210> 706
   <211> 37
   <212> PRT
   <213> Homo sapiens
<400> 706
   Glu Val Asn His Cys Ser Asn Tyr Cys Gln Asn Gly Gly Thr Cys Val
   1               5                   10                  15
   Pro Ser Val Leu Gly Arg Pro Thr Cys Ser Cys Ala Leu Gly Phe Thr
                   20                  25                  30
   Gly Pro Asn Cys Gly
           35
<210> 707
   <211> 37
   <212> PRT
   <213> Homo sapiens
<400> 707
   Gly Lys Thr Val Cys Glu Asp Phe Cys Gln Asn Gly Gly Thr Cys Ile
   1               5                   10                  15
   Val Thr Ala Gly Asn Gln Pro Tyr Cys His Cys Gln Pro Glu Tyr Thr
               20                  25                  30
   Gly Asp Arg Cys Gln
           35
<210> 708
   <211> 37
   <212> PRT
   <213> Homo sapiens
<400> 708
   Gln Tyr Tyr Val Cys His His Tyr Cys Val Asn Ser Glu Ser Cys Thr
   1               5                   10                  15
   Ile Gly Asp Asp Gly Ser Val Glu Cys Val Cys Pro Thr Arg Tyr Glu
               20                  25                  30
   Gly Pro Lys Cys Glu
           35
<210> 709
   <211> 38
   <212> PRT
   <213> Homo sapiens
<400> 709
   Ser Cys Gln Leu Cys Asp Gly Tyr Cys Tyr Asn Gly Gly Thr Cys Gln
   1 5 10 15
   Leu Asp Pro Glu Thr Asn Val Pro Val Cys Leu Cys Ser Thr Asn Trp
   20 25 30
   Ser Gly Thr Gln Cys Glu
   35
<210> 710
   <211> 40
   <212> PRT
   <213> Homo sapiens
<400> 710
   Asp Gln Asp Glu Cys Ala Val Tyr Gly Thr Cys Ser Gln Thr Cys Arg
   1 5 10 15
   Asn Thr His Gly Ser Tyr Thr Cys Ser Cys Val Glu Gly Tyr Leu Met
   20 25 30
   Gln Pro Asp Asn Arg Ser Cys Lys
   35 40
<210> 711
   <211> 41
   <212> PRT
   <213> Homo sapiens
<400> 711
   Gly Asp Asn Met Cys Arg Val Asn Asn Gly Gly Cys Ser Thr Leu Cys
   1 5 10 15
   Leu Ala Ile Pro Gly Gly Arg Val Cys Ala Cys Ala Asp Asn Gln Leu
   20 25 30
   Leu Asp Glu Asn Gly Thr Thr Cys Thr
   35 40
<210> 712
   <211> 42
   <212> PRT
   <213> Homo sapiens
<400> 712
   Gly Ser Asn Ser Cys Gln Leu Asn Asn Gly Gly Cys Ser Gln Leu Cys
   1 5 10 15
   Leu Pro Thr Ser Glu Thr Thr Arg Thr Cys Met Cys Thr Val Gly Tyr
   20 25 30
   Tyr Leu Gln Lys Asn Arg Met Ser Cys Gln
   35 40
<210> 713
   <211> 41
   <212> PRT
   <213> Homo sapiens
<400> 713
   Gly Thr Asn Val Cys Ala Arg Asp Asn Gly Gly Cys Lys Gln Leu Cys
   1               5                   10                  15
   Leu Tyr Arg Gly Asn Ser Arg Arg Thr Cys Ala Cys Ala His Gly Tyr
               20                  25                  30
   Leu Ala Glu Asp Gly Val Thr Cys Leu
           35                  40
<210> 714
   <211> 42
   <212> PRT
   <213> Homo sapiens
<400> 714
   His Ile Asn Glu Cys Leu Ser Lys Lys Val Ser Gly Cys Ser Gln Asp
   1               5                   10                  15
   Cys Gln Asp Leu Pro Val Ser Tyr Lys Cys Lys Cys Trp Pro Gly Phe
               20                  25                  30
   Gln Leu Lys Asp Asp Gly Lys Thr Cys Val
           35                  40
<210> 715
   <211> 41
   <212> PRT
   <213> Homo sapiens
<400> 715
   Asp Ile Asp Glu Cys Ser Ser Gly Phe Pro Cys Ser Gln Gln Cys Ile
   1               5                   10                  15
   Asn Thr Tyr Gly Thr Tyr Lys Cys Leu Cys Thr Asp Gly Tyr Glu Ile
               20                  25                  30
   Gln Pro Asp Asn Pro Asn Gly Cys Lys
           35                  40
<210> 716
   <211> 43
   <212> PRT
   <213> Homo sapiens
<400> 716
   Thr Glu Tyr Thr Cys Glu Asp Asn Val Asn Pro Cys Gly Asp Asp Ala
   1               5                   10                  15
   Tyr Cys Asn Gln Ile Lys Thr Ser Val Phe Cys Arg Cys Lys Pro Gly
               20                  25                  30
   Phe Gln Arg Asn Met Lys Asn Arg Gln Cys Glu
           35                  40
<210> 717
   <211> 38
   <212> PRT
   <213> Homo sapiens
<400> 717
   Asp Leu Asn Glu Cys Leu Val Phe Gly Thr Cys Ser His Gln Cys Ile
   1               5                   10                  15
   Asn Val Glu Gly Ser Tyr Lys Cys Val Cys Asp Gln Asn Phe Gln Glu
               20                  25                  30
   Arg Asn Asn Thr Cys Ile
           35
<210> 718
   <211> 39
   <212> PRT
   <213> Homo sapiens
<400> 718
   Met Thr Leu Cys Ser Ala Leu Asn Cys Gln Tyr Gln Cys His Glu Thr
   1               5                   10                  15
   Pro Tyr Gly Gly Ala Cys Phe Cys Pro Pro Gly Tyr Ile Ile Asn His
               20                  25                  30
   Asn Asp Ser Arg Thr Cys Val
           35
<210> 719
   <211> 43
   <212> PRT
   <213> Homo sapiens
<400> 719
   Cys Asn Asn Pro Cys Glu Gln Phe Asn Gly Gly Cys Ser His Ile Cys
   1               5                   10                  15
   Ala Pro Gly Pro Asn Gly Ala Glu Cys Gln Cys Pro His Glu Gly Asn
               20                  25                  30
   Trp Tyr Leu Ala Asn Asn Arg Lys His Cys Ile
           35                  40
<210> 720
   <211> 41
   <212> PRT
   <213> Homo sapiens
<400> 720
   Ile Asn Glu Cys His Asp Pro Ser Ile Ser Gly Cys Asp His Asn Cys
   1               5                   10                  15
   Thr Asp Thr Leu Thr Ser Phe Tyr Cys Ser Cys Arg Pro Gly Tyr Lys
               20                  25                  30
   Leu Met Ser Asp Lys Arg Thr Cys Val
           35                  40
<210> 721
   <211> 41
   <212> PRT
   <213> Homo sapiens
<400> 721
   Asp Ile Asp Glu Cys Thr Glu Met Pro Phe Val Cys Ser Gln Lys Cys
   1               5                   10                  15
   Glu Asn Val Ile Gly Ser Tyr Ile Cys Lys Cys Ala Pro Gly Tyr Leu
               20                  25                  30
   Arg Glu Pro Asp Gly Lys Thr Cys Arg
           35                  40
<210> 722
   <211> 45
   <212> PRT
   <213> Homo sapiens
<400> 722
   Val Ser Asn Pro Cys Gly Thr Asn Asn Gly Gly Cys Ser His Leu Cys
   1               5                   10                  15
   Leu Ile Lys Pro Gly Gly Lys Gly Phe Thr Cys Glu Cys Pro Asp Asp
               20                  25                  30
   Phe Arg Thr Leu Gln Leu Ser Gly Ser Thr Tyr Cys Met
           35                  40                  45
<210> 723
   <211> 41
   <212> PRT
   <213> Homo sapiens
<400> 723
   Lys Glu Arg Thr Cys Ala Glu Asn Ile Cys Glu Gln Asn Cys Thr Gln
   1               5                   10                  15
   Leu Asn Glu Gly Gly Phe Ile Cys Ser Cys Thr Ala Gly Phe Glu Thr
               20                  25                  30
   Asn Val Phe Asp Arg Thr Ser Cys Leu
           35                  40
<210> 724
   <211> 42
   <212> PRT
   <213> Homo sapiens
<400> 724
   Asp Ile Asn Glu Cys Glu Gln Phe Gly Thr Cys Pro Gln His Cys Arg
   1               5                   10                  15
   Asn Thr Lys Gly Ser Tyr Glu Cys Val Cys Ala Asp Gly Phe Thr Ser
               20                  25                  30
   Met Ser Asp Arg Pro Gly Lys Arg Cys Ala
           35                  40
<210> 725
   <211> 39
   <212> PRT
   <213> Homo sapiens
<400> 725
   Val Pro Asn Leu Cys Lys Gln Ile Cys Ser His Leu Cys Leu Leu Arg
   1               5                   10                  15
   Pro Gly Gly Tyr Ser Cys Ala Cys Pro Gln Gly Ser Ser Phe Ile Glu
               20                  25                  30
   Gly Ser Thr Thr Glu Cys Asp
           35
<210> 726
   <211> 35
   <212> PRT
   <213> Homo sapiens
<400> 726
   Leu Pro Pro Pro Cys Arg Cys Met His Gly Gly Asn Cys Tyr Phe Asp
   1               5                   10                  15
   Glu Thr Asp Leu Pro Lys Cys Lys Cys Pro Ser Gly Tyr Thr Gly Lys
               20                  25                  30
   Tyr Cys Glu
           35
<210> 727
   <211> 39
   <212> PRT
   <213> Homo sapiens
<400> 727
   Glu Phe Asp Asp Cys Gln Ile Trp Gly Ile Cys Asp Gln Lys Cys Glu
   1               5                   10                  15
   Ser Arg Pro Gly Arg His Leu Cys His Cys Glu Glu Gly Tyr Ile Leu
               20                  25                  30
   Glu Arg Gly Gln Tyr Cys Lys
           35
<210> 728
   <211> 47
   <212> PRT
   <213> Homo sapiens
<400> 728
   Ala Thr Asn Pro Cys Lys Asp Asn Asn Gly Gly Cys Glu Gln Val Cys
   1               5                   10                  15
   Val Leu Ser His Arg Thr Asp Asn Asp Gly Leu Gly Phe Arg Cys Lys
               20                  25                  30
   Cys Thr Phe Gly Phe Gln Leu Asp Thr Asp Glu Arg His Cys Ile
           35                  40                  45
<210> 729
   <211> 45
   <212> PRT
   <213> Homo sapiens
<400> 729
   Gly Ser Asn Ala Cys Asn Gln Pro Thr His Pro Asn Gly Asp Cys Ser
   1               5                   10                  15
   His Phe Cys Phe Pro Val Pro Asn Phe Gln Arg Val Cys Gly Cys Pro
               20                  25                  30
   Tyr Gly Met Arg Leu Ala Ser Asn His Leu Thr Cys Glu
           35                  40                  45
<210> 730
   <211> 41
   <212> PRT
   <213> Homo sapiens
<400> 730
   Asn Gly Asn Ser Cys Ser Asp Phe Asn Gly Gly Cys Thr His Glu Cys
   1               5                   10                  15
   Val Gln Glu Pro Phe Gly Ala Lys Cys Leu Cys Pro Leu Gly Phe Leu
               20                  25                  30
   Leu Ala Asn Asp Ser Lys Thr Cys Glu
           35                  40
<210> 731
   <211> 40
   <212> PRT
   <213> Homo sapiens
<400> 731
   Asp Ile Asp Glu Cys Asp Ile Leu Gly Ser Cys Ser Gln His Cys Tyr
   1               5                   10                  15
   Asn Met Arg Gly Ser Phe Arg Cys Ser Cys Asp Thr Gly Tyr Met Leu
               20                  25                  30
   Glu Ser Asp Gly Arg Thr Cys Lys
           35                  40
<210> 732
   <211> 42
   <212> PRT
   <213> Homo sapiens
<400> 732
   Ser Val Asn Pro Cys Ala Phe Ser Arg Cys Ser His Leu Cys Leu Leu
   1               5                   10                  15
   Ser Ser Gln Gly Pro His Phe Tyr Ser Cys Val Cys Pro Ser Gly Trp
               20                  25                  30
   Ser Leu Ser Pro Asp Leu Leu Asn Cys Leu
           35                  40
<210> 733
   <211> 42
   <212> PRT
   <213> Homo sapiens
<400> 733
   Ser Ser Asn Gly Cys Ser Asn Asn Met Asn Ala Cys Gln Gln Ile Cys
   1 5 10 15
   Leu Pro Val Pro Gly Gly Leu Phe Ser Cys Ala Cys Ala Thr Gly Phe
   20 25 30
   Lys Leu Asn Pro Asp Asn Arg Ser Cys Ser
   35 40
<210> 734
   <211> 42
   <212> PRT
   <213> Homo sapiens
<400> 734
   Asn Asn Asn Pro Cys Leu Glu Asn Asn Gly Gly Cys Ser His Leu Cys
   1 5 10 15
   Phe Ala Leu Pro Gly Leu His Thr Pro Lys Cys Asp Cys Ala Phe Gly
   20 25 30
   Thr Leu Gln Ser Asp Gly Lys Asn Cys Ala
   35 40
<210> 735
   <211> 41
   <212> PRT
   <213> Homo sapiens
<400> 735
   Gly Glu Glu Asn Cys Asn Val Asn Asn Gly Gly Cys Ala Gln Lys Cys
   1 5 10 15
   Gln Met Val Arg Gly Ala Val Gln Cys Thr Cys His Thr Gly Tyr Arg
   20 25 30
   Leu Thr Glu Asp Gly His Thr Cys Gln
   35 40
<210> 736
   <211> 40
   <212> PRT
   <213> Homo sapiens
<400> 736
   Asp Val Asn Glu Cys Ala Glu Glu Gly Tyr Cys Ser Gln Gly Cys Thr
   1 5 10 15
   Asn Ser Glu Gly Ala Phe Gln Cys Trp Cys Glu Thr Gly Tyr Glu Leu
   20 25 30
   Arg Pro Asp Arg Arg Ser Cys Lys
   35 40
<210> 737
   <211> 40
   <212> PRT
   <213> Homo sapiens
<400> 737
   Gly Lys Asn Arg Cys Gly Asp Asn Asn Gly Gly Cys Thr His Leu Cys
   1               5                   10                  15
   Leu Pro Ser Gly Gln Asn Tyr Thr Cys Ala Cys Pro Thr Gly Phe Arg
               20                  25                  30
   Lys Ile Ser Ser His Ala Cys Ala
           35                  40
<210> 738
   <211> 43
   <212> PRT
   <213> Homo sapiens
<400> 738
   Phe His Thr Arg Cys Glu Glu Asp Asn Gly Gly Cys Ser His Leu Cys
   1               5                   10                  15
   Leu Leu Ser Pro Ser Glu Pro Phe Tyr Thr Cys Ala Cys Pro Thr Gly
               20                  25                  30
   Val Gln Leu Gln Asp Asn Gly Arg Thr Cys Lys
           35                  40
<210> 739
   <211> 41
   <212> PRT
   <213> Homo sapiens
<400> 739
   Gly Thr Asn Pro Cys Ala Asp Arg Asn Gly Gly Cys Ser His Leu Cys
   1               5                   10                  15
   Phe Phe Thr Pro His Ala Thr Arg Cys Gly Cys Pro Ile Gly Leu Glu
               20                  25                  30
   Leu Leu Ser Asp Met Lys Thr Cys Ile
           35                  40
<210> 740
   <211> 41
   <212> PRT
   <213> Homo sapiens
<400> 740
   Gly Leu Asn Asp Cys Met His Asn Asn Gly Gln Cys Gly Gln Leu Cys
   1               5                   10                  15
   Leu Ala Ile Pro Gly Gly His Arg Cys Gly Cys Ala Ser His Tyr Thr
               20                  25                  30
   Leu Asp Pro Ser Ser Arg Asn Cys Ser
           35                  40
<210> 741
   <211> 42
   <212> PRT
   <213> Homo sapiens
<400> 741
   Ser Ala His Pro Cys Ala Arg Asp Asn Gly Gly Cys Ser His Ile Cys
   1               5                   10                  15
   Ile Ala Lys Gly Asp Gly Thr Pro Arg Cys Ser Cys Pro Val His Leu
               20                  25                  30
   Val Leu Leu Gln Asn Leu Leu Thr Cys Gly
           35                  40
<210> 742
   <211> 43
   <212> PRT
   <213> Homo sapiens
<400> 742
   Ala Thr Asn Pro Cys Gly Ile Asp Asn Gly Gly Cys Ser His Leu Cys
   1               5                   10                  15
   Leu Met Ser Pro Val Lys Pro Phe Tyr Gln Cys Ala Cys Pro Thr Gly
               20                  25                  30
   Val Lys Leu Leu Glu Asn Gly Lys Thr Cys Lys
           35                  40
<210> 743
   <211> 41
   <212> PRT
   <213> Homo sapiens
<400> 743
   Gly Ser Asn Pro Cys Ala Glu Glu Asn Gly Gly Cys Ser His Leu Cys
   1               5                   10                  15
   Leu Tyr Arg Pro Gln Gly Leu Arg Cys Ala Cys Pro Ile Gly Phe Glu
               20                  25                  30
   Leu Ile Ser Asp Met Lys Thr Cys Ile
           35                  40
<210> 744
   <211> 42
   <212> PRT
   <213> Homo sapiens
<400> 744
   Gly Trp Asn Glu Cys Ala Ser Ser Asn Gly His Cys Ser His Leu Cys
   1               5                   10                  15
   Leu Ala Val Pro Val Gly Gly Phe Val Cys Gly Cys Pro Ala His Tyr
               20                  25                  30
   Ser Leu Asn Ala Asp Asn Arg Thr Cys Ser
           35                  40
<210> 745
   <211> 42
   <212> PRT
   <213> Homo sapiens
<400> 745
   Arg Gln His Pro Cys Ala Gln Asp Asn Gly Gly Cys Ser His Ile Cys
   1               5                   10                  15
   Leu Val Lys Gly Asp Gly Thr Thr Arg Cys Ser Cys Pro Met His Leu
               20                  25                  30
   Val Leu Leu Gln Asp Glu Leu Ser Cys Gly
           35                  40
<210> 746
   <211> 40
   <212> PRT
   <213> Homo sapiens
<400> 746
   Gly Leu Asn Glu Cys Leu His Asn Asn Gly Gly Cys Ser His Ile Cys
   1               5                   10                  15
   Thr Asp Leu Lys Ile Gly Phe Glu Cys Thr Cys Pro Ala Gly Phe Gln
               20                  25                  30
   Leu Leu Asp Gln Lys Thr Cys Gly
           35                  40
<210> 747
   <211> 40
   <212> PRT
   <213> Homo sapiens
<400> 747
   Asp Ile Asp Glu Cys Lys Asp Pro Asp Ala Cys Ser Gln Ile Cys Val
   1               5                   10                  15
   Asn Tyr Lys Gly Tyr Phe Lys Cys Glu Cys Tyr Pro Gly Tyr Glu Met
               20                  25                  30
   Asp Leu Leu Thr Lys Asn Cys Lys
           35                  40
<210> 748
   <211> 37
   <212> PRT
   <213> Homo sapiens
<400> 748
   Tyr Thr Ala Ser Cys Gln Pro Trp Ser Cys Ser Gly His Gly Glu Cys
   1               5                   10                  15
   Val Glu Ile Ile Asn Asn Tyr Thr Cys Asn Cys Asp Val Gly Tyr Tyr
               20                  25                  30
   Gly Pro Gln Cys Gln
           35
<210> 749
   <211> 44
   <212> PRT
   <213> Homo sapiens
<400> 749
   Asp Val Asp Glu Cys Lys Glu Arg Glu Asp Glu Glu Leu Ser Cys Asp
   1               5                   10                  15
   His Tyr Cys His Asn Tyr Ile Gly Gly Tyr Tyr Cys Ser Cys Arg Phe
               20                  25                  30
   Gly Tyr Ile Leu His Thr Asp Asn Arg Thr Cys Arg
           35                  40
<210> 750
   <211> 44
   <212> PRT
   <213> Homo sapiens
<210> 751
   <211> 38
   <212> PRT
   <213> Homo sapiens
<400> 751
   Leu Cys Ala Thr Gly Asp His Asp Cys Glu Gln Val Cys Ile Ser Ser
   1               5                   10                  15
   Pro Gly Ser Tyr Thr Cys Ala Cys His Glu Gly Phe Thr Leu Asn Ser
               20                  25                  30
   Asp Gly Lys Thr Cys Asn
           35
<210> 752
   <211> 41
   <212> PRT
   <213> Homo sapiens
<400> 752
   Thr Ala His Met Cys Ser Thr Leu Glu His Asn Cys Ala His Phe Cys
   1               5                   10                  15
   Ile Asn Ile Pro Gly Ser Tyr Val Cys Arg Cys Lys Gln Gly Tyr Ile
               20                  25                  30
   Leu Asn Ser Asp Gln Thr Thr Cys Arg
           35                  40
<210> 753
   <211> 41
   <212> PRT
   <213> Homo sapiens
<400> 753
   Arg Lys Asp Val Cys Lys Ser Thr His His Gly Cys Glu His Ile Cys
   1               5                   10                  15
   Val Asn Asn Gly Asn Ser Tyr Ile Cys Lys Cys Ser Glu Gly Phe Val
               20                  25                  30
   Leu Ala Glu Asp Gly Arg Arg Cys Lys
           35                  40
<210> 754
   <211> 41
   <212> PRT
   <213> Homo sapiens
<400> 754
   Ile Gln Asp Leu Cys Ala Met Glu Asp His Asn Cys Glu Gln Leu Cys
   1               5                   10                  15
   Val Asn Val Pro Gly Ser Phe Val Cys Gln Cys Tyr Ser Gly Tyr Ala
               20                  25                  30
   Leu Ala Glu Asp Gly Lys Arg Cys Val
           35                  40
<210> 755
   <211> 41
   <212> PRT
   <213> Homo sapiens
<400> 755
   Ala Val Asp Tyr Cys Ala Ser Glu Asn His Gly Cys Glu His Glu Cys
   1               5                   10                  15
   Val Asn Ala Asp Gly Ser Tyr Leu Cys Gln Cys His Glu Gly Phe Ala
               20                  25                  30
   Leu Asn Pro Asp Lys Lys Thr Cys Thr
           35                  40
<210> 756
   <211> 41
   <212> PRT
   <213> Homo sapiens
<400> 756
   Lys Ile Asp Tyr Cys Ala Ser Ser Asn His Gly Cys Gln His Glu Cys
   1               5                   10                  15
   Val Asn Thr Asp Asp Ser Tyr Ser Cys His Cys Leu Lys Gly Phe Thr
               20                  25                  30
   Leu Asn Pro Asp Lys Lys Thr Cys Arg
           35                  40
<210> 757
   <211> 41
   <212> PRT
   <213> Homo sapiens
<400> 757
   Arg Ile Asn Tyr Cys Ala Leu Asn Lys Pro Gly Cys Glu His Glu Cys
   1 5 10 15
   Val Asn Met Glu Glu Ser Tyr Tyr Cys Arg Cys His Arg Gly Tyr Thr
   20 25 30
   Leu Asp Pro Asn Gly Lys Thr Cys Ser
   35 40
<210> 758
   <211> 41
   <212> PRT
   <213> Homo sapiens
<400> 758
   Arg Val Asp His Cys Ala Gln Gln Asp His Gly Cys Glu Gln Leu Cys
   1 5 10 15
   Leu Asn Thr Glu Asp Ser Phe Val Cys Gln Cys Ser Glu Gly Phe Leu
   20 25 30
   Ile Asn Glu Asp Leu Lys Thr Cys Ser
   35 40
<210> 759
   <211> 41
   <212> PRT
   <213> Homo sapiens
<400> 759
   Arg Val Asp Tyr Cys Leu Leu Ser Asp His Gly Cys Glu Tyr Ser Cys
   1 5 10 15
   Val Asn Met Asp Arg Ser Phe Ala Cys Gln Cys Pro Glu Gly His Val
   20 25 30
   Leu Arg Ser Asp Gly Lys Thr Cys Ala
   35 40
<210> 760
   <211> 41
   <212> PRT
   <213> Homo sapiens
<400> 760
   Lys Leu Asp Ser Cys Ala Leu Gly Asp His Gly Cys Glu His Ser Cys
   1 5 10 15
   Val Ser Ser Glu Asp Ser Phe Val Cys Gln Cys Phe Glu Gly Tyr Ile
   20 25 30
   Leu Arg Glu Asp Gly Lys Thr Cys Arg
   35 40
<210> 761
   <211> 41
   <212> PRT
   <213> Homo sapiens
<400> 761
   Arg Lys Asp Val Cys Gln Ala Ile Asp His Gly Cys Glu His Ile Cys
   1               5                   10                  15
   Val Asn Ser Asp Asp Ser Tyr Thr Cys Glu Cys Leu Glu Gly Phe Arg
               20                  25                  30
   Leu Ala Glu Asp Gly Lys Arg Cys Arg
           35                  40
<210> 762
   <211> 42
   <212> PRT
   <213> Homo sapiens
<400> 762
   Ala Leu Asp Pro Cys Val Leu Gly Thr His Gln Cys Gln His Val Cys
   1               5                   10                  15
   Ile Ser Asp Gly Glu Gly Lys His His Cys Glu Cys Ser Gln Gly Tyr
               20                  25                  30
   Thr Leu Asn Ala Asp Lys Lys Thr Cys Ser
           35                  40
<210> 763
   <211> 42
   <212> PRT
   <213> Homo sapiens
<400> 763
   Ala Leu Asp Arg Cys Ala Leu Asn Thr His Gly Cys Glu His Ile Cys
   1               5                   10                  15
   Val Asn Asp Arg Ser Gly Ser Tyr His Cys Glu Cys Tyr Glu Gly Tyr
               20                  25                  30
   Thr Leu Asn Glu Asp Arg Lys Thr Cys Ser
           35                  40
<210> 764
   <211> 42
   <212> PRT
   <213> Homo sapiens
<400> 764
   Ala Gln Asp Lys Cys Ala Leu Gly Thr His Gly Cys Gln His Ile Cys
   1               5                   10                  15
   Val Asn Asp Arg Thr Gly Ser His His Cys Glu Cys Tyr Glu Gly Tyr
               20                  25                  30
   Thr Leu Asn Ala Asp Lys Lys Thr Cys Ser
                         35                  40
<210> 765
   <211> 42
   <212> PRT
   <213> Homo sapiens
<400> 765
   Val Arg Asp Lys Cys Ala Leu Gly Ser His Gly Cys Gln His Ile Cys
   1               5                   10                  15
   Val Ser Asp Gly Ala Ala Ser Tyr His Cys Asp Cys Tyr Pro Gly Tyr
               20                  25                  30
   Thr Leu Asn Glu Asp Lys Lys Thr Cys Ser
           35                  40
<210> 766
   <211> 41
   <212> PRT
   <213> Homo sapiens
<400> 766
   Gly Lys Asp Gln Cys Ala Glu Gly Gly His Gly Cys Gln His Gln Cys
   1               5                   10                  15
   Val Asn Ala Trp Ala Met Phe His Cys Thr Cys Asn Pro Gly Tyr Lys
               20                  25                  30
   Leu Ala Ala Asp Asn Lys Ser Cys Leu
           35                  40
<210> 767
   <211> 37
   <212> PRT
   <213> Homo sapiens
<400> 767
   Ala Ile Asp Leu Cys Ala Glu Gly Thr His Gly Cys Glu His His Cys
   1               5                   10                  15
   Val Asn Ser Pro Gly Ser Tyr Phe Cys His Cys Gln Val Gly Phe Val
               20                  25                  30
   Leu Gln Gln Asp Gln
           35
<210> 768
   <211> 41
   <212> PRT
   <213> Homo sapiens
<400> 768
   Ala Ile Asp Tyr Cys Ser Phe Gly Asn His Ser Cys Gln His Glu Cys
   1               5                   10                  15
   Val Ser Thr Pro Gly Gly Pro Arg Cys His Cys Arg Glu Gly His Asp
               20                  25                  30
   Leu Gln Pro Asp Gly Arg Ser Cys Gln
           35                  40
<210> 769
   <211> 36
   <212> PRT
   <213> Homo sapiens
<400> 769
   Cys Asn Gly Val Asp His Gly Cys Glu Phe Gln Cys Val Ser Glu Gly
   1 5 10 15
   Leu Ser Tyr Arg Cys Leu Cys Pro Glu Gly Arg Gln Leu Gln Ala Asp
   20 25 30
   Gly Lys Ser Cys
   35
<210> 770
   <211> 41
   <212> PRT
   <213> Homo sapiens
<400> 770
   Phe Arg Asp Pro Cys Asp Pro Asn Pro Cys Gln Asn Asp Gly Ile Cys
   1 5 10 15
   Val Asn Val Lys Gly Met Ala Ser Cys Arg Cys Ile Ser Gly His Ala
   20 25 30
   Phe Phe Tyr Thr Gly Glu Arg Cys Gln
   35 40
<210> 771
   <211> 41
   <212> PRT
   <213> Homo sapiens
<400> 771
   Val Gln Asp Leu Cys Ser Lys Thr Thr Cys Lys Asn Asp Gly Val Cys
   1 5 10 15
   Thr Val Arg Asp Gly Lys Ala Glu Cys Arg Cys Gln Ser Gly Glu Asp
   20 25 30
   Trp Trp Tyr Met Gly Glu Arg Cys Glu
   35 40
<210> 772
   <211> 39
   <212> PRT
   <213> Homo sapiens
<400> 772
   Thr Ile Val Ser Thr Glu Ser Leu Glu Thr Leu Ala Pro Gly Leu Cys
   1 5 10 15
   Gln Glu Gly Gln Ile Trp Asn Gly Lys Gln Cys Val Cys Pro Gln Gly
   20 25 30
   Tyr Val Gly Tyr Gln Cys Leu
   35
<210> 773
   <211> 39
   <212> PRT
   <213> Homo sapiens
<400> 773
   Pro Ser Asn Pro Cys Gln Asp Asp Pro Cys Ala Asp Asn Ser Leu Cys
   1               5                   10                  15
   Val Lys Leu His Asn Thr Ser Phe Cys Leu Cys Leu Glu Gly Tyr Tyr
               20                  25                  30
   Tyr Asn Ser Ser Thr Cys Lys
           35
<210> 774
   <211> 40
   <212> PRT
   <213> Homo sapiens
<400> 774
   Leu Thr Leu Arg Cys Asp Tyr Tyr Gly Cys Asn Gln Thr Ala Asp Asp
   1               5                   10                  15
   Cys Leu Asn Gly Leu Ala Cys Asp Cys Lys Ser Asp Leu Gln Arg Pro
               20                  25                  30
   Asn Pro Gln Ser Pro Phe Cys Val
           35                  40
<210> 775
   <211> 42
   <212> PRT
   <213> Homo sapiens
<400> 775
   Ser Ser Leu Lys Cys Pro Asp Ala Cys Asn Ala Gln His Lys Gln Cys
   1               5                   10                  15
   Leu Ile Lys Lys Ser Gly Gly Ala Pro Glu Cys Ala Cys Val Pro Gly
               20                  25                  30
   Tyr Gln Glu Asp Ala Asn Gly Asn Cys Gln
           35                  40
<210> 776
   <211> 40
   <212> PRT
   <213> Homo sapiens
<400> 776
   Arg Thr Thr Thr Cys Phe Gly Asp Gly Cys Gln Asn Thr Ala Ser Arg
   1               5                   10                  15
   Cys Lys Asn Gly Gly Thr Trp Asp Gly Leu Lys Cys Gln Cys Pro Asn
               20                  25                  30
   Leu Tyr Tyr Gly Glu Leu Cys Glu
           35                  40
<210> 777
   <211> 34
   <212> PRT
   <213> Homo sapiens
<400> 777
   Ser Gly Asp Arg Cys Gln Leu Gln Thr Arg Cys Gln Asn Gly Gly Gln
   1 5 10 15
   Trp Asp Gly Leu Lys Cys Gln Cys Pro Ser Thr Phe Tyr Gly Ser Ser
   20 25 30
   Cys Glu
<210> 778
   <211> 40
   <212> PRT
   <213> Homo sapiens
<400> 778
   Gln Asn Gln Ser Cys Pro Val Asn Tyr Cys Tyr Asn Gln Gly His Cys
   1 5 10 15
   Tyr Ile Ser Gln Thr Leu Gly Cys Gln Pro Met Cys Thr Cys Pro Pro
   20 25 30
   Ala Phe Thr Asp Ser Arg Cys Phe
   35 40
<210> 779
   <211> 40
   <212> PRT
   <213> Homo sapiens
<400> 779
   Val Ser Pro Cys Ser Arg Gly Tyr Cys Asp His Gly Gly Gln Cys Gln
   1 5 10 15
   His Leu Pro Ser Gly Pro Arg Cys Ser Cys Val Ser Phe Ser Ile Tyr
   20 25 30
   Thr Ala Trp Gly Glu His Cys Glu
   35 40
<210> 780
   <211> 37
   <212> PRT
   <213> Homo sapiens
<400> 780
   Glu Tyr Ser Ser Cys Ser Arg His Pro Cys Gln Asn Gly Gly Thr Cys
   1 5 10 15
   Ile Asn Gly Arg Thr Ser Phe Thr Cys Ala Cys Arg His Pro Phe Thr
   20 25 30
   Gly Asp Asn Cys Thr
   35
<210> 781
   <211> 31
   <212> PRT
   <213> Homo sapiens
<400> 781
   Val Pro His Cys Ala Asp Lys Cys Val His Gly Arg Cys Ile Ala Pro
   1               5                   10                  15
   Asn Thr Cys Gln Cys Glu Pro Gly Trp Gly Gly Thr Asn Cys Ser
               20                  25                  30
<210> 782
   <211> 31
   <212> PRT
   <213> Homo sapiens
<400> 782
   Ala Glu Arg Cys Asp Cys Ser His Ala Asp Gly Cys His Pro Thr Thr
   1               5                   10                  15
   Gly His Cys Arg Cys Leu Pro Gly Trp Ser Gly Val His Cys Asp
               20                  25                  30
<210> 783
   <211> 36
   <212> PRT
   <213> Homo sapiens
<400> 783
   Trp Gly Pro Asn Cys Ser Leu Pro Cys Tyr Cys Lys Asn Gly Ala Ser
   1               5                   10                  15
   Cys Ser Pro Asp Asp Gly Ile Cys Glu Cys Ala Pro Gly Phe Arg Gly
               20                  25                  30
   Thr Thr Cys Gln
           35
<210> 784
   <211> 36
   <212> PRT
   <213> Homo sapiens
<400> 784
   Phe Gly Lys Asn Cys Ala Gly Ile Cys Thr Cys Thr Asn Asn Gly Thr
   1               5                   10                  15
   Cys Asn Pro Ile Asp Arg Ser Cys Gln Cys Tyr Pro Gly Trp Ile Gly
               20                  25                  30
   Ser Asp Cys Ser
           35
<210> 785
   <211> 31
   <212> PRT
   <213> Homo sapiens
<400> 785
   Ile His Thr Cys Asn Cys His Asn Gly Ala Phe Cys Ser Ala Tyr Asp
   1               5                   10                  15
   Gly Glu Cys Lys Cys Thr Pro Gly Trp Thr Gly Leu Tyr Cys Thr
               20                  25                  30
<210> 786
   <211> 36
   <212> PRT
   <213> Homo sapiens
<400> 786
   Tyr Gly Lys Asp Cys Ala Leu Ile Cys Gln Cys Gln Asn Gly Ala Asp
   1               5                   10                  15
   Cys Asp His Ile Ser Gly Gln Cys Thr Cys Arg Thr Gly Phe Met Gly
               20                  25                  30
   Arg His Cys Glu
           35
<210> 787
   <211> 31
   <212> PRT
   <213> Homo sapiens
<400> 787
   Arg Gln Ile Cys Asp Cys Leu Asn Asn Ser Thr Cys Asp His Ile Thr
   1               5                   10                  15
   Gly Thr Cys Tyr Cys Ser Pro Gly Trp Lys Gly Ala Arg Cys Asp
               20                  25                  30
<210> 788
   <211> 36
   <212> PRT
   <213> Homo sapiens
<400> 788
   Trp Gly Pro His Cys Thr Ser Arg Cys Gln Cys Lys Asn Gly Ala Leu
   1               5                   10                  15
   Cys Asn Pro Ile Thr Gly Ala Cys His Cys Ala Ala Gly Phe Arg Gly
               20                  25                  30
   Trp Arg Cys Glu
           35
<210> 789
   <211> 36
   <212> PRT
   <213> Homo sapiens
<400> 789
   Tyr Gly Asn Asp Cys His Gln Arg Cys Gln Cys Gln Asn Gly Ala Thr
   1               5                   10                  15
   Cys Asp His Val Thr Gly Glu Cys Arg Cys Pro Pro Gly Tyr Thr Gly
               20                  25                  30
   Ala Phe Cys Glu
           35
<210> 790
   <211> 36
   <212> PRT
   <213> Homo sapiens
<400> 790
   His Gly Pro Gln Cys Glu Gln Arg Cys Pro Cys Gln Asn Gly Gly Val
   1               5                   10                  15
   Cys His His Val Thr Gly Glu Cys Ser Cys Pro Ser Gly Trp Met Gly
               20                  25                  30
   Thr Val Cys Gly
           35
<210> 791
   <211> 31
   <212> PRT
   <213> Homo sapiens
<400> 791
   Ser Gln Glu Cys Gln Cys His Asn Gly Gly Thr Cys Asp Ala Ala Thr
   1               5                   10                  15
   Gly Gln Cys His Cys Ser Pro Gly Tyr Thr Gly Glu Arg Cys Gln
               20                  25                  30
<210> 792
   <211> 36
   <212> PRT
   <213> Homo sapiens
<400> 792
   Tyr Gly Val Leu Cys Ala Glu Thr Cys Gln Cys Val Asn Gly Gly Lys
   1               5                   10                  15
   Cys Tyr His Val Ser Gly Ala Cys Leu Cys Glu Ala Gly Phe Ala Gly
               20                  25                  30
   Glu Arg Cys Glu
           35
<210> 793
   <211> 36
   <212> PRT
   <213> Homo sapiens
<400> 793
   Tyr Gly Glu Ala Cys Gln Gln Ile Cys Ser Cys Gln Asn Gly Ala Asp
   1               5                   10                  15
   Cys Asp Ser Val Thr Gly Lys Cys Thr Cys Ala Pro Gly Phe Lys Gly
               20                  25                  30
   Ile Asp Cys Ser
           35
<210> 794
   <211> 31
   <212> PRT
   <213> Homo sapiens
<400> 794
   Ser Ser Arg Cys Gly Cys Lys Asn Asp Ala Val Cys Ser Pro Val Asp
   1               5                   10                  15
   Gly Ser Cys Thr Cys Lys Ala Gly Trp His Gly Val Asp Cys Ser
                   20                  25                  30
<210> 795
   <211> 36
   <212> PRT
   <213> Homo sapiens
<400> 795
   Trp Gly Phe Gly Cys Asn Leu Thr Cys Gln Cys Leu Asn Gly Gly Ala
   1               5                   10                  15
   Cys Asn Thr Leu Asp Gly Thr Cys Thr Cys Ala Pro Gly Trp Arg Gly
               20                  25                  30
   Glu Lys Cys Glu
           35
<210> 796
   <211> 36
   <212> PRT
   <213> Homo sapiens
<400> 796
   Ser Gly Asp Phe Cys Ile Pro Leu Cys Thr Glu Glu Cys Val His Gly
   1               5                   10                  15
   Arg Cys Val Ser Pro Asp Thr Cys His Cys Glu Pro Gly Trp Gly Gly
               20                  25                  30
   Pro Asp Cys Ser
           35
<210> 797
   <211> 36
   <212> PRT
   <213> Homo sapiens
<400> 797
   Trp Gly Pro Asn Cys Ser Val Ser Cys Ser Cys Glu Asn Gly Gly Ser
   1               5                   10                  15
   Cys Ser Pro Glu Asp Gly Ser Cys Glu Cys Ala Pro Gly Phe Arg Gly
               20                  25                  30
   Pro Leu Cys Gln
           35
<210> 798
   <211> 36
   <212> PRT
   <213> Homo sapiens
<400> 798
   Phe Gly Gln Asp Cys Ala Gln Leu Cys Ser Cys Ala Asn Asn Gly Thr
   1               5                   10                  15
   Cys Ser Pro Ile Asp Gly Ser Cys Gln Cys Phe Pro Gly Trp Ile Gly
               20                  25                  30
   Lys Asp Cys Ser
           35
<210> 799
   <211> 31
   <212> PRT
   <213> Homo sapiens
<400> 799
   Phe His Ala Cys Ser Cys His Asn Gly Ala Ser Cys Ser Ala Glu Asp
   1               5                   10                  15
   Gly Ala Cys His Cys Thr Pro Gly Trp Thr Gly Leu Phe Cys Thr
              20                  25                  30
<210> 800
   <211> 31
   <212> PRT
   <213> Homo sapiens
<400> 800
   Gly Arg Val Cys Gln Cys Gln Asn Gly Ala Ser Cys Asp His Ile Ser
   1               5                   10                  15
   Gly Lys Cys Thr Cys Arg Thr Gly Phe Thr Gly Gln His Cys Glu
               20                  25                  30
<210> 801
   <211> 36
   <212> PRT
   <213> Homo sapiens
<400> 801
   Phe Gly Tyr Gly Cys Gln Gln Leu Cys Glu Cys Met Asn Asn Ser Thr
   1               5                   10                  15
   Cys Asp His Val Thr Gly Thr Cys Tyr Cys Ser Pro Gly Phe Lys Gly
               20                  25                  30
   Ile Arg Cys Asp
           35
<210> 802
   <211> 31
   <212> PRT
   <213> Homo sapiens
<400> 802
   Ser Asn Arg Cys Gln Cys Gln Asn Gly Ala Leu Cys Asn Pro Ile Thr
   1               5                   10                  15
   Gly Ala Cys Val Cys Ala Ala Gly Phe Arg Gly Trp Arg Cys Glu
               20                  25                  30
<210> 803
   <211> 36
   <212> PRT
   <213> Homo sapiens
<400> 803
   His Gly Lys Gly Cys Gln Leu Pro Cys Gln Cys Arg His Gly Ala Ser
   1               5                   10                  15
   Cys Asp Pro Arg Ala Gly Glu Cys Leu Cys Ala Pro Gly Tyr Thr Gly
               20                  25                  30
   Val Tyr Cys Glu
           35
<210> 804
   <211> 36
   <212> PRT
   <213> Homo sapiens
<400> 804
   His Gly Ala His Cys Glu Leu Arg Cys Pro Cys Gln Asn Gly Gly Thr
   1               5                   10                  15
   Cys His His Ile Thr Gly Glu Cys Ala Cys Pro Pro Gly Trp Thr Gly
               20                  25                  30
   Ala Val Cys Ala
           35
<210> 805
   <211> 36
   <212> PRT
   <213> Homo sapiens
<400> 805
   Phe Gly Gln Asn Cys Ser Gln Asp Cys Pro Cys His His Gly Gly Gln
   1               5                   10                  15
   Cys Asp His Val Thr Gly Gln Cys His Cys Thr Ala Gly Tyr Met Gly
               20                  25                  30
   Asp Arg Cys Gln
           35
<210> 806
   <211> 36
   <212> PRT
   <213> Homo sapiens
<400> 806
   Phe Gly Phe Gln Cys Ser Gln His Cys Asp Cys His Asn Gly Gly Gln
   1               5                   10                  15
   Cys Ser Pro Thr Thr Gly Ala Cys Glu Cys Glu Pro Gly Tyr Lys Gly
               20                  25                  30
   Pro Arg Cys Gln
           35
<210> 807
   <211> 36
   <212> PRT
   <213> Homo sapiens
<400> 807
   Tyr Gly Asp Gly Cys Gln Leu Pro Cys Thr Cys Gln Asn Gly Ala Asp
   1               5                   10                  15
   Cys His Ser Ile Thr Gly Gly Cys Thr Cys Ala Pro Gly Phe Met Gly
               20                  25                  30
   Glu Val Cys Ala
           35
<210> 808
   <211> 36
   <212> PRT
   <213> Homo sapiens
<400> 808
   Tyr Gly Pro Asn Cys Ser Ser Ile Cys Ser Cys Asn Asn Gly Gly Thr
   1               5                   10                  15
   Cys Ser Pro Val Asp Gly Ser Cys Thr Cys Lys Glu Gly Trp Gln Gly
               20                  25                  30
   Leu Asp Cys Thr
           35
<210> 809
   <211> 31
   <212> PRT
   <213> Homo sapiens
<400> 809
   Asn Glu Ser Cys Thr Cys Ala Asn Gly Ala Ala Cys Ser Pro Ile Asp
   1               5                   10                  15
   Gly Ser Cys Ser Cys Thr Pro Gly Trp Leu Gly Asp Thr Cys Glu
               20                  25                  30
<210> 810
   <211> 36
   <212> PRT
   <213> Homo sapiens
<400> 810
   Leu Ser Ala Glu Cys Ser Ala Ser Leu Cys Phe His Gly Gly Arg Cys
   1               5                   10                  15
   Val Pro Gly Ser Ala Gln Pro Cys His Cys Pro Pro Gly Phe Gln Gly
               20                  25                  30
   Pro Arg Cys Gln
           35
<210> 811
   <211> 36
   <212> PRT
   <213> Homo sapiens
<400> 811
   Phe Gly Lys Asn Cys Ser Phe Ser Cys Ser Cys Gln Asn Gly Gly Thr
   1               5                   10                  15
   Cys Asp Ser Val Thr Gly Ala Cys Arg Cys Pro Pro Gly Val Ser Gly
               20                  25                  30
   Thr Asn Cys Glu
           35
<210> 812
   <211> 36
   <212> PRT
   <213> Homo sapiens
<400> 812
   Tyr Gly Lys His Cys Arg Lys Lys Cys Asn Cys Ala Asn Arg Gly Arg
   1               5                   10                  15
   Cys His Arg Leu Tyr Gly Ala Cys Leu Cys Asp Pro Gly Leu Tyr Gly
               20                  25                  30
   Arg Phe Cys His
           35
<210> 813
   <211> 35
   <212> PRT
   <213> Homo sapiens
<400> 813
   Phe Gly Val Asn Cys Ser Ser Ser Cys Ser Cys Gly Gly Ala Pro Cys
   1               5                   10                  15
   His Gly Val Thr Gly Gln Cys Arg Cys Pro Pro Gly Arg Thr Gly Glu
               20                  25                  30
   Asp Cys Glu
           35
<210> 814
   <211> 32
   <212> PRT
   <213> Homo sapiens
<400> 814
   Gln Glu Ile Cys Pro Ala Cys Gln His Ala Ala Arg Cys Asp Pro Glu
   1               5                   10                  15
   Thr Gly Ala Cys Leu Cys Leu Pro Gly Phe Val Gly Ser Arg Cys Gln
               20                  25                  30
<210> 815
   <211> 36
   <212> PRT
   <213> Homo sapiens
<400> 815
   Tyr Gly Pro Ser Cys Gln Thr Arg Cys Ser Cys Ala Asn Asp Gly His
   1               5                   10                  15
   Cys His Pro Ala Thr Gly His Cys Ser Cys Ala Pro Gly Trp Thr Gly
               20                  25                  30
   Phe Ser Cys Gln
           35
<210> 816
   <211> 37
   <212> PRT
   <213> Homo sapiens
<400> 816
   Trp Gly Pro Asp Cys Ser His Pro Cys Asn Cys Ser Ala Gly His Gly
   1               5                   10                  15
   Ser Cys Asp Ala Ile Ser Gly Leu Cys Leu Cys Glu Ala Gly Tyr Val
               20                  25                  30
   Gly Pro Arg Cys Glu
           35
<210> 817
   <211> 36
   <212> PRT
   <213> Homo sapiens
<400> 817
   Phe Gly Pro Gly Cys Glu Gln Arg Cys Gln Cys Gln His Gly Ala Ala
   1               5                   10                  15
   Cys Asp His Val Ser Gly Ala Cys Thr Cys Pro Ala Gly Trp Arg Gly
               20                  25                  30
   Thr Phe Cys Glu
           35
<210> 818
   <211> 33
   <212> PRT
   <213> Homo sapiens
<400> 818
   Asp Cys Arg Ser Ala Cys Asn Cys Thr Ala Gly Ala Ala Cys Asp Ala
   1               5                   10                  15
   Val Asn Gly Ser Cys Leu Cys Pro Ala Gly Arg Arg Gly Pro Arg Cys
               20                  25                  30
   Ala
<210> 819
   <211> 36
   <212> PRT
   <213> Homo sapiens
<400> 819
   Tyr Gly His Asn Cys Ser Gln Ala Cys Ala Cys Phe Asn Gly Ala Ser
   1               5                   10                  15
   Cys Asp Pro Val His Gly Gln Cys His Cys Ala Pro Gly Trp Met Gly
               20                  25                  30
   Pro Ser Cys Leu
      35
<210> 820
   <211> 36
   <212> PRT
   <213> Homo sapiens
<400> 820
   Tyr Gly Asp Asn Cys Arg His Ser Cys Leu Cys Gln Asn Gly Gly Thr
   1               5                   10                  15
   Cys Asp Pro Val Ser Gly His Cys Ala Cys Pro Glu Gly Trp Ala Gly
               20                  25                  30
   Leu Ala Cys Glu
           35
<210> 821
   <211> 41
   <212> PRT
   <213> Homo sapiens
<400> 821
   Asp Val Asp Glu Cys Arg Thr His Asn Gly Gly Cys Gln His Arg Cys
   1               5                   10                  15
   Val Asn Thr Pro Gly Ser Tyr Leu Cys Glu Cys Lys Pro Gly Phe Arg
               20                  25                  30
   Leu His Thr Asp Ser Arg Thr Cys Leu
           35                  40
<210> 822
   <211> 36
   <212> PRT
   <213> Homo sapiens
<400> 822
   Val Arg Ala Gly Cys Arg His Ser Gly Gly Cys Leu Asn Gly Gly Leu
   1 5 10 15
   Cys Asp Pro His Thr Gly Arg Cys Leu Cys Pro Ala Gly Trp Thr Gly
   20 25 30
   Asp Lys Cys Gln
   35
<210> 823
   <211> 36
   <212> PRT
   <213> Homo sapiens
<400> 823
   Phe Gly Glu Ala Cys Ala Gln Arg Cys Ser Cys Pro Pro Gly Ala Ala
   1 5 10 15
   Cys His His Val Thr Gly Ala Cys Arg Cys Pro Pro Gly Phe Thr Gly
   20 25 30
   Ser Gly Cys Glu
   35
<210> 824
   <211> 36
   <212> PRT
   <213> Homo sapiens
<400> 824
   Tyr Gly Pro Gly Cys Glu Gln Leu Cys Gly Cys Leu Asn Gly Gly Ser
   1 5 10 15
   Cys Asp Ala Ala Thr Gly Ala Cys Arg Cys Pro Thr Gly Phe Leu Gly
   20 25 30
   Thr Asp Cys Asn
   35
<210> 825
   <211> 36
   <212> PRT
   <213> Homo sapiens
<400> 825
   Phe Gly Pro Asn Cys Thr His Val Cys Gly Cys Gly Gln Gly Ala Ala
   1 5 10 15
   Cys Asp Pro Val Thr Gly Thr Cys Leu Cys Pro Pro Gly Arg Ala Gly
   20 25 30
   Val Arg Cys Glu
   35
<210> 826
   <211> 36
   <212> PRT
   <213> Homo sapiens
<400> 826
   Phe Gly Val Gly Cys Glu His Thr Cys Ser Cys Arg Asn Gly Gly Leu
   1 5 10 15
   Cys His Ala Ser Asn Gly Ser Cys Ser Cys Gly Leu Gly Trp Thr Gly
   20 25 30
   Arg His Cys Glu
   35
<210> 827
   <211> 31
   <212> PRT
   <213> Homo sapiens
<400> 827
   His Leu Glu Cys Ser Cys His Asn Asn Ser Thr Cys Glu Pro Ala Thr
   1 5 10 15
   Gly Thr Cys Arg Cys Gly Pro Gly Phe Tyr Gly Gln Ala Cys Glu
   20 25 30
<210> 828
   <211> 36
   <212> PRT
   <213> Homo sapiens
<400> 828
   His Gly Ala Gly Cys Gln Gly Leu Cys Trp Cys Gln His Gly Ala Pro
   1 5 10 15
   Cys Asp Pro Ile Ser Gly Arg Cys Leu Cys Pro Ala Gly Phe His Gly
   20 25 30
   His Phe Cys Glu
   35
<210> 829
   <211> 36
   <212> PRT
   <213> Homo sapiens
<400> 829
   Phe Gly Pro Ser Cys Thr Leu His Cys Asp Cys Gly Gly Gly Ala Asp
   1 5 10 15
   Cys Asp Pro Val Ser Gly Gln Cys His Cys Val Asp Gly Tyr Met Gly
   20 25 30
   Pro Thr Cys Arg
   35
<210> 830
   <211> 37
   <212> PRT
   <213> Homo sapiens
<400> 830
   Cys Ala Leu Gly Asn Gly Gly Cys Gln His His Cys Val Gln Leu Thr
   1 5 10 15
   Ile Thr Arg His Arg Cys Gln Cys Arg Pro Gly Phe Gln Leu Gln Glu
   20 25 30
   Asp Gly Arg His Cys
   35
<210> 831
   <211> 47
   <212> PRT
   <213> Homo sapiens
<400> 831
   Asp Gly Arg His Cys Val Arg Arg Ser Pro Cys Ala Asn Arg Asn Gly
   1 5 10 15
   Ser Cys Met His Arg Cys Gln Val Val Arg Gly Leu Ala Arg Cys Glu
   20 25 30
   Cys His Val Gly Tyr Gln Leu Ala Ala Asp Gly Lys Ala Cys Glu
   35 40 45
<210> 832
   <211> 41
   <212> PRT
   <213> Homo sapiens
<400> 832
   Asp Val Asp Glu Cys Ala Ala Gly Leu Ala Gln Cys Ala His Gly Cys
   1 5 10 15
   Leu Asn Thr Gln Gly Ser Phe Lys Cys Val Cys His Ala Gly Tyr Glu
   20 25 30
   Leu Gly Ala Asp Gly Arg Gln Cys Tyr
   35 40
<210> 833
   <211> 36
   <212> PRT
   <213> Homo sapiens
<400> 833
   Cys Glu Ala Asn Asn Gly Gly Cys Ser His Gly Cys Ser His Thr Ser
   1 5 10 15
   Ala Gly Pro Leu Cys Thr Cys Pro Arg Gly Tyr Glu Leu Asp Thr Asp
   20 25 30
   Gln Arg Thr Cys
   35
<210> 834
   <211> 37
   <212> PRT
   <213> Homo sapiens
<400> 834
   Asp Cys Ala Asp Ser Pro Cys Cys Gln Gln Val Cys Thr Asn Asn Pro
   1               5                   10                  15
   Gly Gly Tyr Glu Cys Gly Cys Tyr Ala Gly Tyr Arg Leu Ser Ala Asp
               20                  25                  30
   Gly Cys Gly Cys Glu
           35
<210> 835
   <211> 41
   <212> PRT
   <213> Homo sapiens
<400> 835
   Asp Val Asp Glu Cys Ala Ser Ser Arg Gly Gly Cys Glu His His Cys
   1               5                   10                  15
   Thr Asn Leu Ala Gly Ser Phe Gln Cys Ser Cys Glu Ala Gly Tyr Arg
               20                  25                  30
   Leu His Glu Asp Arg Arg Gly Cys Ser
           35                  40
<210> 836
   <211> 37
   <212> PRT
   <213> Homo sapiens
<400> 836
   Phe Gly His Asp Cys Ser Leu Thr Cys Asp Asp Cys Arg Asn Gly Gly
   1               5                   10                  15
   Thr Cys Leu Leu Gly Leu Asp Gly Cys Asp Cys Pro Glu Gly Trp Thr
               20                  25                  30
   Gly Leu Ile Cys Asn
           35
<210> 837
   <211> 31
   <212> PRT
   <213> Homo sapiens
<400> 837
   Arg Phe Ser Leu Cys Pro Gly Gly Cys Gln Ser His Gly Gln Cys Gln
   1               5                   10                  15
   Pro Pro Gly Val Cys Ala Cys Glu Pro Gly Trp Gly Gly Pro Asp
               20                  25                  30
<210> 838
   <211> 34
   <212> PRT
   <213> Homo sapiens
<400> 838
   Gly Leu Gln Glu Cys Ser Ala Tyr Cys Gly Ser His Gly Thr Cys Ala
   1               5                   10                  15
   Ser Pro Leu Gly Pro Cys Arg Cys Glu Pro Gly Phe Leu Gly Arg Ala
               20                  25                  30
   Cys Asp
<210> 839
   <211> 42
   <212> PRT
   <213> Homo sapiens
<400> 839
   Asp Val Asp Glu Cys Arg Leu Gly Leu Ala Arg Cys His Pro Arg Ala
   1               5                   10                  15
   Thr Cys Leu Asn Thr Pro Leu Ser Tyr Glu Cys His Cys Gln Arg Gly
               20                  25                  30
   Tyr Gln Gly Asp Gly Ile Ser His Cys Asn
           35                  40
<210> 840
   <211> 43
   <212> PRT
   <213> Homo sapiens
<400> 840
   Gly Ser Ala Arg Cys Gly Ser Gly Gly Pro Gly Ser Cys Pro Val Pro
   1               5                   10                  15
   Gln Glu Cys Val Pro Gln Asp Gly Ala Ala Gly Ala Gly Leu Cys Arg
               20                  25                  30
   Cys Pro Gln Gly Trp Ala Gly Pro His Cys Arg
           35                  40
<210> 841
   <211> 39
   <212> PRT
   <213> Homo sapiens
<400> 841
   Pro Glu Asp Glu Cys Ala Asn Gly His His Asp Cys Asn Glu Thr Gln
   1               5                   10                  15
   Asn Cys His Asp Gln Pro His Gly Tyr Glu Cys Ser Cys Lys Thr Gly
               20                  25                  30
   Tyr Thr Met Asp Asn Met Thr
           35
<210> 842
   <211> 48
   <212> PRT
   <213> Homo sapiens
<400> 842
   Cys Gly Cys Ser Phe His Ser His Cys Arg Lys Arg Gly Pro Gly Phe
   1               5                   10                  15
   Cys Asp Glu Cys Gln Asp Trp Thr Trp Gly Glu His Cys Glu Arg Cys
               20                  25                  30
   Arg Pro Gly Ser Phe Gly Asn Ala Thr Gly Ser Arg Gly Cys Arg Pro
           35                  40                  45
<210> 843
   <211> 51
   <212> PRT
   <213> Homo sapiens
<400> 843
   Cys Gln Cys Asn Gly His Gly Asp Pro Arg Arg Gly His Cys Asp Asn
   1               5                   10                  15
   Leu Ser Gly Leu Cys Phe Cys Gln Asp His Thr Glu Gly Ala His Cys
               20                  25                  30
   Gln Leu Cys Ser Pro Gly Tyr Tyr Gly Asp Pro Arg Ala Gly Gly Ser
           35                  40                  45
   Cys Phe Arg
       50
<210> 844
   <211> 49
   <212> PRT
   <213> Homo sapiens
<400> 844
   Cys Arg Cys Asn Arg His Ser Glu Cys Ala Gly Val Gly Ala Arg Asp
   1               5                   10                  15
   His Cys Leu Leu Cys Arg Asn His Thr Lys Gly Ser His Cys Glu Gln
               20                  25                  30
   Cys Leu Pro Leu Phe Val Gly Ser Ala Val Gly Gly Gly Thr Cys Arg
           35                  40                  45
   Pro
<210> 845
   <211> 64
   <212> PRT
   <213> Homo sapiens
<400> 845
   Cys Gln Cys Asn Gly His Ala Asp Thr Cys Asn Glu Gln Asp Gly Thr
   1               5                   10                  15
   Gly Cys Pro Cys Gln Asn Asn Thr Glu Thr Gly Thr Cys Gln Gly Ser
               20                  25                  30
   Ser Pro Ser Asp Arg Arg Asp Cys Tyr Lys Tyr Gln Cys Ala Lys Cys
           35                  40                  45
   Arg Glu Ser Phe His Gly Ser Pro Leu Gly Gly Gln Gln Cys Tyr Arg
       50                  55                  60
<210> 846
   <211> 50
   <212> PRT
   <213> Homo sapiens
<400> 846
   Cys Asn Cys Ser Val Val Gly Ser Leu Asn Val Asn Arg Cys Asn Gln
   1               5                   10                  15
   Thr Thr Gly Gln Cys Glu Cys Arg Pro Gly Tyr Gln Gly Leu His Cys
               20                  25                  30
   Glu Thr Cys Lys Glu Gly Phe Tyr Leu Asn Tyr Thr Ser Gly Leu Cys
           35                  40                  45
   Gln Pro
       50
<210> 847
   <211> 47
   <212> PRT
   <213> Homo sapiens
<400> 847
   Cys Asp Cys Ser Pro His Gly Ala Leu Ser Ile Pro Cys Asn Ser Ser
   1               5                   10                  15
   Gly Lys Cys Gln Cys Lys Val Gly Val Ile Gly Ser Ile Cys Asp Arg
               20                  25                  30
   Cys Gln Asp Gly Tyr Tyr Gly Phe Ser Lys Asn Gly Cys Leu Pro
           35                  40                  45
<210> 848
   <211> 48
   <212> PRT
   <213> Homo sapiens
<400> 848
   Cys Gln Cys Asn Asn Arg Ser Ala Ser Cys Asp Ala Leu Thr Gly Ala
   1               5                   10                  15
   Cys Leu Asn Cys Gln Glu Asn Ser Lys Gly Asn His Cys Glu Glu Cys
               20                  25                  30
   Lys Glu Gly Phe Tyr Gln Ser Pro Asp Ala Thr Lys Glu Cys Leu Arg
           35                  40                  45
<210> 849
   <211> 51
   <212> PRT
   <213> Homo sapiens
<400> 849
   Cys Pro Cys Ser Ala Val Thr Ser Thr Gly Ser Cys Ser Ile Lys Ser
   1               5                   10                  15
   Ser Glu Leu Glu Pro Glu Cys Asp Gln Cys Lys Asp Gly Tyr Ile Gly
               20                  25                  30
   Pro Asn Cys Asn Lys Cys Glu Asn Gly Tyr Tyr Asn Phe Asp Ser Ile
           35                  40                  45
   Cys Arg Lys
       50
<210> 850
   <211> 52
   <212> PRT
   <213> Homo sapiens
<400> 850
   Cys Gln Cys His Gly His Val Asp Pro Val Lys Thr Pro Lys Ile Cys
   1               5                   10                  15
   Lys Pro Glu Ser Gly Glu Cys Ile Asn Cys Leu His Asn Thr Thr Gly
               20                  25                  30
   Phe Trp Cys Glu Asn Cys Leu Glu Gly Tyr Val His Asp Leu Glu Gly
           35                  40                  45
   Asn Cys Ile Lys
       50
<210> 851
   <211> 33
   <212> PRT
   <213> Homo sapiens
<400> 851
   Asp Glu Leu Asp Cys Gly Pro Ser Asn Cys Ser Gln His Gly Leu Cys
   1               5                   10                  15
   Thr Glu Thr Gly Cys Arg Cys Asp Ala Gly Trp Thr Gly Ser Asn Cys
               20                  25                  30
   Ser
<210> 852
   <211> 36
   <212> PRT
   <213> Homo sapiens
<400> 852
   Ser Trp Gly Gln Cys Gln Pro Val Cys Gln Pro Arg Cys Lys His Gly
   1               5                   10                  15
   Glu Cys Ile Gly Pro Asn Lys Cys Lys Cys His Pro Gly Tyr Ala Gly
               20                  25                  30
   Lys Thr Cys Asn
           35
<210> 853
   <211> 40
   <212> PRT
   <213> Homo sapiens
<400> 853
   Asp Leu Asn Glu Cys Gly Leu Lys Pro Arg Pro Cys Lys His Arg Cys
   1               5                   10                  15
   Met Asn Thr Tyr Gly Ser Tyr Lys Cys Tyr Cys Leu Asn Gly Tyr Met
               20                  25                  30
   Leu Met Pro Asp Gly Ser Cys Ser
           35                  40
<210> 854
   <211> 37
   <212> PRT
   <213> Homo sapiens
<400> 854
   Thr Cys Ser Met Ala Asn Cys Gln Tyr Gly Cys Asp Val Val Lys Gly
   1 5 10 15
   Gln Ile Arg Cys Gln Cys Pro Ser Pro Gly Leu Gln Leu Ala Pro Asp
   20 25 30
   Gly Arg Thr Cys Val
   35
<210> 855
   <211> 45
   <212> PRT
   <213> Homo sapiens
<400> 855
   Asp Val Asp Glu Cys Ala Thr Gly Arg Ala Ser Cys Pro Arg Phe Arg
   1 5 10 15
   Gln Cys Val Asn Thr Phe Gly Ser Tyr Ile Cys Lys Cys His Lys Gly
   20 25 30
   Phe Asp Leu Met Tyr Ile Gly Gly Lys Tyr Gln Cys His
   35 40 45
<210> 856
   <211> 41
   <212> PRT
   <213> Homo sapiens
<400> 856
   Asp Ile Asp Glu Cys Ser Leu Gly Gln Tyr Gln Cys Ser Ser Phe Ala
   1 5 10 15
   Arg Cys Tyr Asn Ile Arg Gly Ser Tyr Lys Cys Lys Cys Lys Glu Gly
   20 25 30
   Tyr Gln Gly Asp Gly Leu Thr Cys Val
   35 40
<210> 857
   <211> 56
   <212> PRT
   <213> Homo sapiens
<400> 857
   Cys Lys Cys Asn Gly His Ala Ala Arg Cys Val Arg Asp Arg Asp Asp
   1 5 10 15
   Ser Leu Val Cys Asp Cys Arg His Asn Thr Ala Gly Pro Glu Cys Asp
   20 25 30
   Arg Cys Lys Pro Phe His Tyr Asp Arg Pro Trp Gln Arg Ala Thr Ala
   35 40 45
   Arg Glu Ala Asn Glu Cys Val Ala
   50 55
<210> 858
   <211> 63
   <212> PRT
   <213> Homo sapiens
<400> 858
   Cys Asn Cys Asn Leu His Ala Arg Arg Cys Arg Phe Asn Met Glu Leu
   1               5                   10                  15
   Tyr Lys Leu Ser Gly Arg Lys Ser Gly Gly Val Cys Leu Asn Cys Arg
               20                  25                  30
   His Asn Thr Ala Gly Arg His Cys His Tyr Cys Lys Glu Gly Tyr Tyr
           35                  40                  45
   Arg Asp Met Gly Lys Pro Ile Thr His Arg Lys Ala Cys Lys Ala
       50                  55                  60
<210> 859
   <211> 50
   <212> PRT
   <213> Homo sapiens
<400> 859
   Cys Asp Cys His Pro Val Gly Ala Ala Gly Lys Thr Cys Asn Gln Thr
   1               5                   10                  15
   Thr Gly Gln Cys Pro Cys Lys Asp Gly Val Thr Gly Ile Thr Cys Asn
               20                  25                  30
   Arg Cys Ala Lys Gly Tyr Gln Gln Ser Arg Ser Pro Ile Ala Pro Cys
           35                  40                  45
   Ile Lys
       50
<210> 860
   <211> 54
   <212> PRT
   <213> Homo sapiens
<400> 860
   Cys Lys Cys Asn Gly His Ala Ser Arg Cys Leu Leu Asp Thr Gln Gly
   1               5                   10                  15
   His Leu Ile Cys Asp Cys Arg His Gly Thr Glu Gly Pro Asp Cys Gly
               20                  25                  30
   Arg Cys Lys Pro Phe Tyr Cys Asp Arg Pro Trp Gln Arg Ala Thr Ala
           35                  40                  45
   Arg Glu Ser His Ala Cys
       50
<210> 861
   <211> 61
   <212> PRT
   <213> Homo sapiens
<400> 861
   Cys Ser Cys Asn Gly His Ala Arg Arg Cys Arg Phe Asn Met Glu Leu
   1               5                   10                  15
   Tyr Arg Leu Ser Gly Arg Arg Ser Gly Gly Val Cys Leu Asn Cys Arg
               20                  25                  30
   His Asn Thr Ala Gly Arg His Cys His Tyr Cys Arg Glu Gly Phe Tyr
           35                  40                  45
   Arg Asp Pro Gly Arg Ala Leu Ser Asp Arg Arg Ala Cys
       50                  55                  60
<210> 862
   <211> 48
   <212> PRT
   <213> Homo sapiens
<400> 862
   Cys Asp Cys His Pro Val Gly Ala Ala Gly Lys Thr Cys Asn Gln Thr
   1               5                   10                  15
   Thr Gly Gln Cys Pro Cys Lys Asp Gly Val Thr Gly Leu Thr Cys Asn
               20                  25                  30
   Arg Cys Ala Pro Gly Phe Gln Gln Ser Arg Ser Pro Val Ala Pro Cys
           35                  40                  45
<210> 863
   <211> 70
   <212> PRT
   <213> Homo sapiens
<400> 863
   Cys Phe Cys Asn Gly His Ala Asp Gln Cys Ile Pro Val His Gly Phe
   1               5                   10                  15
   Arg Pro Val Lys Ala Pro Gly Thr Phe His Met Val His Gly Lys Cys
               20                  25                  30
   Met Cys Lys His Asn Thr Ala Gly Ser His Cys Gln His Cys Ala Pro
           35                  40                  45
   Leu Tyr Asn Asp Arg Pro Trp Glu Ala Ala Asp Gly Lys Thr Gly Ala
       50                  55                  60
   Pro Asn Glu Cys Arg Thr
   65                  70
<210> 864
   <211> 63
   <212> PRT
   <213> Homo sapiens
<400> 864
   Cys Lys Cys Asn Gly His Ala Asp Thr Cys His Phe Asp Val Asn Val
   1               5                   10                  15
   Trp Glu Ala Ser Gly Asn Arg Ser Gly Gly Val Cys Asp Asp Cys Gln
               20                  25                  30
   His Asn Thr Glu Gly Gln Tyr Cys Gln Arg Cys Lys Pro Gly Phe Tyr
           35                  40                  45
   Arg Asp Leu Arg Arg Pro Phe Ser Ala Pro Asp Ala Cys Lys Pro
       50                  55                  60
<210> 865
   <211> 54
   <212> PRT
   <213> Homo sapiens
<400> 865
   Cys Ser Cys His Pro Val Gly Ser Ala Val Leu Pro Ala Asn Ser Val
   1               5                   10                  15
   Thr Phe Cys Asp Pro Ser Asn Gly Asp Cys Pro Cys Lys Pro Gly Val
               20                  25                  30
   Ala Gly Arg Arg Cys Asp Arg Cys Met Val Gly Tyr Trp Gly Phe Gly
           35                  40                  45
   Asp Tyr Gly Cys Arg Pro
       50
<210> 866
   <211> 55
   <212> PRT
   <213> Homo sapiens
<400> 866
   Cys Gln Cys His Gly His Ala Ala Arg Cys Ala Ala Arg Ala Arg Pro
   1               5                   10                  15
   Pro Arg Cys His Cys Arg His His Thr Thr Gly Pro Gly Cys Glu Ser
               20                  25                  30
   Cys Arg Pro Ser His Arg Asp Trp Pro Trp Arg Pro Ala Thr Pro Arg
           35                  40                  45
   His Pro His Pro Cys Leu Pro
       50                  55
<210> 867
   <211> 63
   <212> PRT
   <213> Homo sapiens
<400> 867
   Cys Ser Cys Asn Gln His Ala Arg Arg Cys Arg Phe Asn Ser Glu Leu
   1               5                   10                  15
   Phe Arg Leu Ser Gly Gly Arg Ser Gly Gly Val Cys Glu Arg Cys Arg
               20                  25                  30
   His His Thr Ala Gly Arg His Cys His Tyr Cys Gln Pro Gly Phe Trp
           35                  40                  45
   Arg Asp Pro Ser Gln Pro Ile Phe Ser Arg Arg Ala Cys Arg Ala
       50                  55                  60
<210> 868
   <211> 50
   <212> PRT
   <213> Homo sapiens
<400> 868
   Cys Gln Cys His Pro Ile Gly Ala Thr Gly Gly Thr Cys Asn Gln Thr
   1               5                   10                  15
   Ser Gly Gln Cys Thr Cys Lys Leu Gly Val Thr Gly Leu Thr Cys Asn
               20                  25                  30
   Arg Cys Gly Pro Gly Tyr Gln Gln Ser Arg Ser Pro Arg Met Pro Cys
           35                  40                  45
   Gln Arg
       50
<210> 869
   <211> 60
   <212> PRT
   <213> Homo sapiens
<400> 869
   Cys Lys Cys Asn Leu His Ala Thr Val Cys Val Tyr Asp Asn Ser Lys
   1               5                   10                  15
   Leu Thr Cys Glu Cys Glu His Asn Thr Thr Gly Pro Asp Cys Gly Lys
               20                  25                  30
   Cys Lys Lys Asn Tyr Gln Gly Arg Pro Trp Ser Pro Gly Ser Tyr Leu
           35                  40                  45
   Pro Ile Pro Lys Gly Thr Ala Asn Thr Cys Ile Pro
       50                  55                  60
<210> 870
   <211> 56
   <212> PRT
   <213> Homo sapiens
<400> 870
   Cys Glu Cys Phe Gly His Ser Asn Arg Cys Ser Tyr Ile Asp Leu Leu
   1               5                   10                  15
   Asn Thr Val Ile Cys Val Ser Cys Lys His Asn Thr Arg Gly Gln His
               20                  25                  30
   Cys Glu Leu Cys Arg Leu Gly Tyr Phe Arg Asn Ala Ser Ala Gln Leu
           35                  40                  45
   Asp Asp Glu Asn Val Cys Ile Glu
       50                  55
<210> 871
   <211> 50
   <212> PRT
   <213> Homo sapiens
<400> 871
   Cys Tyr Cys Asn Pro Leu Gly Ser Ile His Asp Arg Cys Asn Gly Ser
   1               5                   10                  15
   Gly Phe Cys Glu Cys Lys Thr Gly Thr Thr Gly Pro Lys Cys Asp Glu
               20                  25                  30
   Cys Leu Pro Gly Asn Ser Trp His Tyr Gly Cys Gln Pro Asn Val Cys
           35                  40                  45
   Asp Asn
       50
<210> 872
   <211> 60
   <212> PRT
   <213> Homo sapiens
<400> 872
   Cys Lys Cys Asn Leu His Ala Asn Leu Cys Ser Met Arg Glu Gly Ser
   1               5                   10                  15
   Leu Gln Cys Glu Cys Glu His Asn Thr Thr Gly Pro Asp Cys Gly Lys
               20                  25                  30
   Cys Lys Lys Asn Phe Arg Thr Arg Ser Trp Arg Ala Gly Ser Tyr Leu
           35                  40                  45
   Pro Leu Pro His Gly Ser Pro Asn Ala Cys Ala Thr
       50                  55                  60
<210> 873
   <211> 56
   <212> PRT
   <213> Homo sapiens
<400> 873
   Cys Glu Cys Tyr Gly His Ser Asn Arg Cys Ser Tyr Ile Asp Phe Leu
   1               5                   10                  15
   Asn Val Val Thr Cys Val Ser Cys Lys His Asn Thr Arg Gly Gln His
               20                  25                  30
   Cys Gln His Cys Arg Leu Gly Tyr Tyr Arg Asn Gly Ser Ala Glu Leu
           35                  40                  45
   Asp Asp Glu Asn Val Cys Ile Glu
       50                  55
<210> 874
   <211> 45
   <212> PRT
   <213> Homo sapiens
<400> 874
   Cys Asn Cys Asn Gln Ile Gly Ser Val His Asp Arg Cys Asn Glu Thr
   1               5                   10                  15
   Gly Phe Cys Glu Cys Arg Glu Gly Ala Ala Gly Pro Lys Cys Asp Asp
               20                  25                  30
   Cys Leu Pro Thr His Tyr Trp Arg Gln Gly Cys Tyr Pro
           35                  40                  45
<210> 875
   <211> 44
   <212> PRT
   <213> Homo sapiens
<400> 875
   Gly Gly Ser Pro Cys Glu Ala Gly Glu Glu Gly Glu Gly Gly Val Cys
   1               5                   10                  15
   Leu Asn Gly Gly Val Cys Ser Val Val Asp Asp Gln Ala Val Cys Asp
               20                  25                  30
   Cys Ser Arg Thr Gly Phe Arg Gly Lys Asp Cys Ser
           35                  40
<210> 876
   <211> 38
   <212> PRT
   <213> Homo sapiens
<400> 876
   Thr Ala Lys Pro Cys Leu Ser Asn Pro Cys Lys Asn Asn Gly Met Cys
   1               5                   10                  15
   Arg Asp Gly Trp Asn Arg Tyr Val Cys Asp Cys Ser Gly Thr Gly Tyr
               20                  25                  30
   Leu Gly Arg Ser Cys Glu
           35
<210> 877
   <211> 38
   <212> PRT
   <213> Homo sapiens
<400> 877
   Pro Ser Thr Thr Cys Gln Glu Asp Ser Cys Ser Asn Gln Gly Val Cys
   1               5                   10                  15
   Leu Gln Gln Trp Asp Gly Phe Ser Cys Asp Cys Ser Met Thr Ser Phe
               20                  25                  30
   Ser Gly Pro Leu Cys Asn
           35
<210> 878
   <211> 41
   <212> PRT
   <213> Homo sapiens
<400> 878
   Ala Asp Pro Leu Cys Ala Pro Ala Arg Asn Pro Cys Ala Asn Gly Gly
   1               5                   10                  15
   Leu Cys Thr Val Leu Ala Pro Gly Glu Val Gly Cys Asp Cys Ser His
               20                  25                  30
   Thr Gly Phe Gly Gly Lys Phe Cys Ser
           35                  40
<210> 879
   <211> 38
   <212> PRT
   <213> Homo sapiens
<400> 879
   Thr Leu Lys Gln Cys Ala Ser Ala Pro Cys Arg Asn Gly Gly Val Cys
   1               5                   10                  15
   Arg Glu Gly Trp Asn Arg Phe Ile Cys Asp Cys Ile Gly Thr Gly Phe
               20                  25                  30
   Leu Gly Arg Val Cys Glu
           35
<210> 880
   <211> 38
   <212> PRT
   <213> Homo sapiens
<400> 880
   Pro Ser Thr Thr Cys Thr Glu Glu Ser Cys Ala Asn Gln Gly Val Cys
   1 5 10 15
   Leu Gln Gln Trp Asp Gly Phe Thr Cys Asp Cys Thr Met Thr Ser Tyr
   20 25 30
   Gly Gly Pro Val Cys Asn
   35
<210> 881
   <211> 38
   <212> PRT
   <213> Homo sapiens
<400> 881
   Ala Glu Gly Pro Cys Gly Glu Arg Pro Cys Glu Asn Gly Gly Ile Cys
   1 5 10 15
   Phe Leu Leu Asp Gly His Pro Thr Cys Asp Cys Ser Thr Thr Gly Tyr
   20 25 30
   Gly Gly Lys Leu Cys Ser
   35
<210> 882
   <211> 38
   <212> PRT
   <213> Homo sapiens
<400> 882
   Ser Ala Lys Gln Cys Asp Ser Tyr Pro Cys Lys Asn Asn Ala Val Cys
   1 5 10 15
   Lys Asp Gly Trp Asn Arg Phe Ile Cys Asp Cys Thr Gly Thr Gly Tyr
   20 25 30
   Trp Gly Arg Thr Cys Glu
   35
<210> 883
   <211> 38
   <212> PRT
   <213> Homo sapiens
<400> 883
   Pro Ser Thr Thr Cys Gln Glu Asp Ser Cys Ala Asn Gln Gly Val Cys
   1 5 10 15
   Met Gln Gln Trp Glu Gly Phe Thr Cys Asp Cys Ser Met Thr Ser Tyr
   20 25 30
   Ser Gly Asn Gln Cys Asn
   35
<210> 884
   <211> 37
   <212> PRT
   <213> Homo sapiens
<400> 884
   His Ser Asp Pro Cys Glu Asn Asn Pro Cys Leu His Gly Gly Thr Cys
   1 5 10 15
   Asn Ala Asn Gly Thr Met Tyr Gly Cys Ser Cys Asp Gln Gly Phe Ala
   20 25 30
   Gly Glu Asn Cys Glu
   35
<210> 885
   <211> 37
   <212> PRT
   <213> Homo sapiens
<400> 885
   Asp Ile Asp Asp Cys Leu Cys Ser Pro Cys Glu Asn Gly Gly Thr Cys
   1 5 10 15
   Ile Asp Glu Val Asn Gly Phe Val Cys Leu Cys Leu Pro Ser Tyr Gly
   20 25 30
   Gly Ser Phe Cys Glu
   35
<210> 886
   <211> 39
   <212> PRT
   <213> Homo sapiens
<400> 886
   Arg Gly Pro Arg Cys Ser Gln Pro Gly Glu Thr Cys Leu Asn Gly Gly
   1 5 10 15
   Lys Cys Glu Ala Ala Asn Gly Thr Glu Ala Cys Val Cys Gly Gly Ala
   20 25 30
   Phe Val Gly Pro Arg Cys Gln
   35
<210> 887
   <211> 39
   <212> PRT
   <213> Homo sapiens
<400> 887
   Leu Asn Asp Ala Cys Ile Ser Asn Pro Cys Asn Glu Gly Ser Asn Cys
   1 5 10 15
   Asp Thr Asn Pro Val Asn Gly Lys Ala Ile Cys Thr Cys Pro Ser Gly
   20 25 30
   Tyr Thr Gly Pro Ala Cys Ser
   35
<210> 888
   <211> 39
   <212> PRT
   <213> Homo sapiens
<400> 888
   Asp Val Asp Glu Cys Ser Leu Gly Ala Asn Pro Cys Glu His Ala Gly
   1               5                   10                  15
   Lys Cys Ile Asn Thr Leu Gly Ser Phe Glu Cys Gln Cys Leu Gln Gly
               20                  25                  30
   Tyr Thr Gly Pro Arg Cys Glu
           35
<210> 889
   <211> 37
   <212> PRT
   <213> Homo sapiens
<400> 889
   Asp Val Asn Glu Cys Val Ser Asn Pro Cys Gln Asn Asp Ala Thr Cys
   1               5                   10                  15
   Leu Asp Gln Ile Gly Glu Phe Gln Cys Ile Cys Met Pro Gly Tyr Glu
               20                  25                  30
   Gly Val His Cys Glu
           35
<210> 890
   <211> 37
   <212> PRT
   <213> Homo sapiens
<400> 890
   Asn Thr Asp Glu Cys Ala Ser Ser Pro Cys Leu His Asn Gly Arg Cys
   1               5                   10                  15
   Leu Asp Lys Ile Asn Glu Phe Gln Cys Glu Cys Pro Thr Gly Phe Thr
               20                  25                  30
   Gly His Leu Cys Gln
           35
<210> 891
   <211> 37
   <212> PRT
   <213> Homo sapiens
<400> 891
   Asp Val Asp Glu Cys Ala Ser Thr Pro Cys Lys Asn Gly Ala Lys Cys
   1               5                   10                  15
   Leu Asp Gly Pro Asn Thr Tyr Thr Cys Val Cys Thr Glu Gly Tyr Thr
               20                  25                  30
   Gly Thr His Cys Glu
           35
<210> 892
   <211> 36
   <212> PRT
   <213> Homo sapiens
<400> 892
   Asp Ile Asp Glu Cys Asp Pro Asp Pro Cys His Tyr Gly Ser Cys Lys
   1               5                   10                  15
   Asp Gly Val Ala Thr Phe Thr Cys Leu Cys Arg Pro Gly Tyr Thr Gly
               20                  25                  30
   His His Cys Glu
           35
<210> 893
   <211> 37
   <212> PRT
   <213> Homo sapiens
<400> 893
   Asn Ile Asn Glu Cys Ser Ser Gln Pro Cys Arg His Gly Gly Thr Cys
   1               5                   10                  15
   Gln Asp Arg Asp Asn Ala Tyr Leu Cys Phe Cys Leu Lys Gly Thr Thr
               20                  25                  30
   Gly Pro Asn Cys Glu
           35
<210> 894
   <211> 36
   <212> PRT
   <213> Homo sapiens
<400> 894
   Asn Leu Asp Asp Cys Ala Ser Ser Pro Cys Asp Ser Gly Thr Cys Leu
   1               5                   10                  15
   Asp Lys Ile Asp Gly Tyr Glu Cys Ala Cys Glu Pro Gly Tyr Thr Gly
               20                  25                  30
   Ser Met Cys Asn
           35
<210> 895
   <211> 37
   <212> PRT
   <213> Homo sapiens
<400> 895
   Asn Ile Asp Glu Cys Ala Gly Asn Pro Cys His Asn Gly Gly Thr Cys
   1               5                   10                  15
   Glu Asp Gly Ile Asn Gly Phe Thr Cys Arg Cys Pro Glu Gly Tyr His
               20                  25                  30
   Asp Pro Thr Cys Leu
           35
<210> 896
   <211> 36
   <212> PRT
   <213> Homo sapiens
<400> 896
   Glu Val Asn Glu Cys Asn Ser Asn Pro Cys Val His Gly Ala Cys Arg
   1               5                   10                  15
   Asp Ser Leu Asn Gly Tyr Lys Cys Asp Cys Asp Pro Gly Trp Ser Gly
               20                  25                  30
   Thr Asn Cys Asp
           35
<210> 897
   <211> 41
   <212> PRT
   <213> Homo sapiens
<400> 897
   Asp Pro Asn Pro Cys Leu Ser Thr Pro Cys Lys Asn Ala Gly Thr Cys
   1               5                   10                  15
   His Val Val Asp Arg Arg Gly Val Ala Asp Tyr Ala Cys Ser Cys Ala
               20                  25                  30
   Leu Gly Phe Ser Gly Pro Leu Cys Leu
           35                  40
<210> 898
   <211> 37
   <212> PRT
   <213> Homo sapiens
<400> 898
   Asn Asn Asn Glu Cys Glu Ser Asn Pro Cys Val Asn Gly Gly Thr Cys
   1               5                   10                  15
   Lys Asp Met Thr Ser Gly Tyr Val Cys Thr Cys Arg Glu Gly Phe Ser
               20                  25                  30
   Gly Pro Asn Cys Gln
           35
<210> 899
   <211> 37
   <212> PRT
   <213> Homo sapiens
<400> 899
   Asn Ile Asn Glu Cys Ala Ser Asn Pro Cys Leu Asn Gln Gly Thr Cys
   1               5                   10                  15
   Ile Asp Asp Val Ala Gly Tyr Lys Cys Asn Cys Leu Leu Pro Tyr Thr
               20                  25                  30
   Gly Ala Thr Cys Glu
           35
<210> 900
   <211> 39
   <212> PRT
   <213> Homo sapiens
<400> 900
   Val Leu Ala Pro Cys Ala Pro Ser Pro Cys Arg Asn Gly Gly Glu Cys
   1 5 10 15
   Arg Gln Ser Glu Asp Tyr Glu Ser Phe Ser Cys Val Cys Pro Thr Gly
   20 25 30
   Trp Gln Gly Gln Thr Cys Glu
   35
<210> 901
   <211> 37
   <212> PRT
   <213> Homo sapiens
<400> 901
   Asp Ile Asn Glu Cys Val Leu Ser Pro Cys Arg His Gly Ala Ser Cys
   1 5 10 15
   Gln Asn Thr His Gly Gly Tyr Arg Cys His Cys Gln Ala Gly Tyr Ser
   20 25 30
   Gly Arg Asn Cys Glu
   35
<210> 902
   <211> 37
   <212> PRT
   <213> Homo sapiens
<400> 902
   Asp Ile Asp Asp Cys Arg Pro Asn Pro Cys His Asn Gly Gly Ser Cys
   1 5 10 15
   Thr Asp Gly Ile Asn Thr Ala Phe Cys Asp Cys Leu Pro Gly Phe Arg
   20 25 30
   Gly Thr Phe Cys Glu
   35
<210> 903
   <211> 37
   <212> PRT
   <213> Homo sapiens
<400> 903
   Asp Ile Asn Glu Cys Ala Ser Asp Pro Cys Arg Asn Gly Ala Asn Cys
   1 5 10 15
   Thr Asp Cys Val Asp Ser Tyr Thr Cys Thr Cys Pro Ala Gly Phe Ser
   20 25 30
   Gly Ile His Cys Glu
   35
<210> 904
   <211> 37
   <212> PRT
   <213> Homo sapiens
<400> 904
   Asn Thr Pro Asp Cys Thr Glu Ser Ser Cys Phe Asn Gly Gly Thr Cys
   1 5 10 15
   Val Asp Gly Ile Asn Ser Phe Thr Cys Leu Cys Pro Pro Gly Phe Thr
   20 25 30
   Gly Ser Tyr Cys Gln
   35
<210> 905
   <211> 37
   <212> PRT
   <213> Homo sapiens
<400> 905
   Asp Val Asn Glu Cys Asp Ser Gln Pro Cys Leu His Gly Gly Thr Cys
   1 5 10 15
   Gln Asp Gly Cys Gly Ser Tyr Arg Cys Thr Cys Pro Gln Gly Tyr Thr
   20 25 30
   Gly Pro Asn Cys Gln
   35
<210> 906
   <211> 37
   <212> PRT
   <213> Homo sapiens
<400> 906
   Leu Val His Trp Cys Asp Ser Ser Pro Cys Lys Asn Gly Gly Lys Cys
   1 5 10 15
   Trp Gln Thr His Thr Gln Tyr Arg Cys Glu Cys Pro Ser Gly Trp Thr
   20 25 30
   Gly Leu Tyr Cys Asp
   35
<210> 907
   <211> 47
   <212> PRT
   <213> Homo sapiens
<400> 907
   Pro Ser Val Ser Cys Glu Val Ala Ala Gln Arg Gln Gly Val Asp Val
   1 5 10 15
   Ala Arg Leu Cys Gln His Gly Gly Leu Cys Val Asp Ala Gly Asn Thr
   20 25 30
   His His Cys Arg Cys Gln Ala Gly Tyr Thr Gly Ser Tyr Cys Glu
   35 40 45
<210> 908
   <211> 38
   <212> PRT
   <213> Homo sapiens
<400> 908
   Leu Asp Asn Ala Cys Leu Thr Asn Pro Cys Arg Asn Gly Gly Thr Cys
   1 5 10 15
   Asp Leu Leu Thr Leu Thr Glu Tyr Lys Cys Arg Cys Pro Pro Gly Trp
   20 25 30
   Ser Gly Lys Ser Cys Gln
   35
<210> 909
   <211> 37
   <212> PRT
   <213> Homo sapiens
<400> 909
   Leu Val Asp Glu Cys Ser Pro Ser Pro Cys Gln Asn Gly Ala Thr Cys
   1 5 10 15
   Thr Asp Tyr Leu Gly Gly Tyr Ser Cys Lys Cys Val Ala Gly Tyr His
   20 25 30
   Gly Val Asn Cys Ser
   35
<210> 910
   <211> 37
   <212> PRT
   <213> Homo sapiens
<400> 910
   Glu Ile Asp Glu Cys Leu Ser His Pro Cys Gln Asn Gly Gly Thr Cys
   1 5 10 15
   Leu Asp Leu Pro Asn Thr Tyr Lys Cys Ser Cys Pro Arg Gly Thr Gln
   20 25 30
   Gly Val His Cys Glu
   35
<210> 911
   <211> 45
   <212> PRT
   <213> Homo sapiens
<400> 911
   Asn Val Asp Asp Cys Asn Pro Pro Val Asp Pro Val Ser Arg Ser Pro
   1 5 10 15
   Lys Cys Phe Asn Asn Gly Thr Cys Val Asp Gln Val Gly Gly Tyr Ser
   20 25 30
   Cys Thr Cys Pro Pro Gly Phe Val Gly Glu Arg Cys Glu
   35 40 45
<210> 912
   <211> 39
   <212> PRT
   <213> Homo sapiens
<400> 912
   Asp Val Asn Glu Cys Leu Ser Asn Pro Cys Asp Ala Arg Gly Thr Gln
   1               5                   10                  15
   Asn Cys Val Gln Arg Val Asn Asp Phe His Cys Glu Cys Arg Ala Gly
               20                  25                  30
   His Thr Gly Arg Arg Cys Glu
           35
<210> 913
   <211> 40
   <212> PRT
   <213> Homo sapiens
<400> 913
   Val Ile Asn Gly Cys Lys Gly Lys Pro Cys Lys Asn Gly Gly Thr Cys
   1               5                   10                  15
   Ala Val Ala Ser Asn Thr Ala Arg Gly Phe Ile Cys Lys Cys Pro Ala
               20                  25                  30
   Gly Phe Glu Gly Ala Thr Cys Glu
           35                  40
<210> 914
   <211> 37
   <212> PRT
   <213> Homo sapiens
<400> 914
   Asp Ala Arg Thr Cys Gly Ser Leu Arg Cys Leu Asn Gly Gly Thr Cys
   1               5                   10                  15
   Ile Ser Gly Pro Arg Ser Pro Thr Cys Leu Cys Leu Gly Pro Phe Thr
               20                  25                  30
   Gly Pro Glu Cys Gln
           35
<210> 915
   <211> 40
   <212> PRT
   <213> Homo sapiens
<400> 915
   Ala Ser Ser Pro Cys Leu Gly Gly Asn Pro Cys Tyr Asn Gln Gly Thr
   1               5                   10                  15
   Cys Glu Pro Thr Ser Glu Ser Pro Phe Tyr Arg Cys Leu Cys Pro Ala
               20                  25                  30
   Lys Phe Asn Gly Leu Leu Cys His
           35                  40
<210> 916
   <211> 37
   <212> PRT
   <213> Homo sapiens
<400> 916
   Gln Ala Asp Pro Cys Ala Ser Asn Pro Cys Ala Asn Gly Gly Gln Cys
   1               5                   10                  15
   Leu Pro Phe Glu Ala Ser Tyr Ile Cys His Cys Pro Pro Ser Phe His
               20                  25                  30
   Gly Pro Thr Cys Arg
           35
<210> 917
   <211> 39
   <212> PRT
   <213> Homo sapiens
<400> 917
   Asp Val Asn Glu Cys Gly Gln Lys Pro Gly Leu Cys Arg His Gly Gly
   1               5                   10                  15
   Thr Cys His Asn Glu Val Gly Ser Tyr Arg Cys Val Cys Arg Ala Thr
               20                  25                  30
   His Thr Gly Pro Asn Cys Glu
           35
<210> 918
   <211> 38
   <212> PRT
   <213> Homo sapiens
<400> 918
   Pro Tyr Val Pro Cys Ser Pro Ser Pro Cys Gln Asn Gly Gly Thr Cys
   1               5                   10                  15
   Arg Pro Thr Gly Asp Val Thr His Glu Cys Ala Cys Leu Pro Gly Phe
               20                  25                  30
   Thr Gly Gln Asn Cys Glu
           35
<210> 919
   <211> 37
   <212> PRT
   <213> Homo sapiens
<400> 919
   Asn Ile Asp Asp Cys Pro Gly Asn Asn Cys Lys Asn Gly Gly Ala Cys
   1               5                   10                  15
   Val Asp Gly Val Asn Thr Tyr Asn Cys Arg Cys Pro Pro Glu Trp Thr
               20                  25                  30
   Gly Gln Tyr Cys Thr
           35
<210> 920
   <211> 39
   <212> PRT
   <213> Homo sapiens
<400> 920
   Asp Val Asp Glu Cys Gln Leu Met Pro Asn Ala Cys Gln Asn Gly Gly
   1               5                   10                  15
   Thr Cys His Asn Thr His Gly Gly Tyr Asn Cys Val Cys Val Asn Gly
               20                  25                  30
   Trp Thr Gly Glu Asp Cys Ser
           35
<210> 921
   <211> 37
   <212> PRT
   <213> Homo sapiens
<400> 921
   Asn Ile Asp Asp Cys Ala Ser Ala Ala Cys Phe His Gly Ala Thr Cys
   1               5                   10                  15
   His Asp Arg Val Ala Ser Phe Tyr Cys Glu Cys Pro His Gly Arg Thr
               20                  25                  30
   Gly Leu Leu Cys His
           35
<210> 922
   <211> 38
   <212> PRT
   <213> Homo sapiens
<400> 922
   Leu Gln Cys Arg Asp Gly Tyr Glu Pro Cys Val Asn Glu Gly Met Cys
   1               5                   10                  15
   Val Thr Tyr His Asn Gly Thr Gly Tyr Cys Lys Cys Pro Glu Gly Phe
               20                  25                  30
   Leu Gly Glu Tyr Cys Gln
           35
<210> 923
   <211> 39
   <212> PRT
   <213> Homo sapiens
<400> 923
   Leu Asp Asp Ala Cys Ile Ser Asn Pro Cys His Lys Gly Ala Leu Cys
   1               5                   10                  15
   Asp Thr Asn Pro Leu Asn Gly Gln Tyr Ile Cys Thr Cys Pro Gln Gly
               20                  25                  30
   Tyr Lys Gly Ala Asp Cys Thr
           35
<210> 924
   <211> 40
   <212> PRT
   <213> Homo sapiens
<400> 924
   Asp Val Asp Glu Cys Ala Met Ala Asn Ser Asn Pro Cys Glu His Ala
   1               5                   10                  15
   Gly Lys Cys Val Asn Thr Asp Gly Ala Phe His Cys Glu Cys Leu Lys
               20                  25                  30
   Gly Tyr Ala Gly Pro Arg Cys Glu
           35                  40
<210> 925
   <211> 37
   <212> PRT
   <213> Homo sapiens
<400> 925
   Asp Ile Asn Glu Cys His Ser Asp Pro Cys Gln Asn Asp Ala Thr Cys
   1               5                   10                  15
   Leu Asp Lys Ile Gly Gly Phe Thr Cys Leu Cys Met Pro Gly Phe Lys
               20                  25                  30
   Gly Val His Cys Glu
           35
<210> 926
   <211> 37
   <212> PRT
   <213> Homo sapiens
<400> 926
   Glu Ile Asn Glu Cys Gln Ser Asn Pro Cys Val Asn Asn Gly Gln Cys
   1               5                   10                  15
   Val Asp Lys Val Asn Arg Phe Gln Cys Leu Cys Pro Pro Gly Phe Thr
               20                  25                  30
   Gly Pro Val Cys Gln
           35
<210> 927
   <211> 37
   <212> PRT
   <213> Homo sapiens
<400> 927
   Asp Ile Asp Asp Cys Ser Ser Thr Pro Cys Leu Asn Gly Ala Lys Cys
   1               5                   10                  15
   Ile Asp His Pro Asn Gly Tyr Glu Cys Gln Cys Ala Thr Gly Phe Thr
               20                  25                  30
   Gly Val Leu Cys Glu
           35
<210> 928
   <211> 36
   <212> PRT
   <213> Homo sapiens
<400> 928
   Asn Ile Asp Asn Cys Asp Pro Asp Pro Cys His His Gly Gln Cys Gln
   1               5                   10                  15
   Asp Gly Ile Asp Ser Tyr Thr Cys Ile Cys Asn Pro Gly Tyr Met Gly
               20                  25                  30
   Ala Ile Cys Ser
           35
<210> 929
   <211> 37
   <212> PRT
   <213> Homo sapiens
<400> 929
   Gln Ile Asp Glu Cys Tyr Ser Ser Pro Cys Leu Asn Asp Gly Arg Cys
   1               5                   10                  15
   Ile Asp Leu Val Asn Gly Tyr Gln Cys Asn Cys Gln Pro Gly Thr Ser
               20                  25                  30
   Gly Val Asn Cys Glu
           35
<210> 930
   <211> 36
   <212> PRT
   <213> Homo sapiens
<400> 930
   Asn Phe Asp Asp Cys Ala Ser Asn Pro Cys Ile His Gly Ile Cys Met
   1               5                   10                  15
   Asp Gly Ile Asn Arg Tyr Ser Cys Val Cys Ser Pro Gly Phe Thr Gly
               20                  25                  30
   Gln Arg Cys Asn
           35
<210> 931
   <211> 37
   <212> PRT
   <213> Homo sapiens
<400> 931
   Asp Ile Asp Glu Cys Ala Ser Asn Pro Cys Arg Lys Gly Ala Thr Cys
   1               5                   10                  15
   Ile Asn Gly Val Asn Gly Phe Arg Cys Ile Cys Pro Glu Gly Pro His
               20                  25                  30
   His Pro Ser Cys Tyr
           35
<210> 932
   <211> 36
   <212> PRT
   <213> Homo sapiens
<400> 932
   Gln Val Asn Glu Cys Leu Ser Asn Pro Cys Ile His Gly Asn Cys Thr
   1               5                   10                  15
   Gly Gly Leu Ser Gly Tyr Lys Cys Leu Cys Asp Ala Gly Trp Val Gly
               20                  25                  30
   Ile Asn Cys Glu
           35
<210> 933
   <211> 39
   <212> PRT
   <213> Homo sapiens
<400> 933
   His Arg Asp Pro Cys Glu Lys Asn Arg Cys Gln Asn Gly Gly Thr Cys
   1               5                   10                  15
   Val Ala Gln Ala Met Leu Gly Lys Ala Thr Cys Arg Cys Ala Ser Gly
               20                  25                  30
   Phe Thr Gly Glu Asp Cys Gln
           35
<210> 934
   <211> 37
   <212> PRT
   <213> Homo sapiens
<400> 934
   Asp Lys Asn Glu Cys Leu Ser Asn Pro Cys Gln Asn Gly Gly Thr Cys
   1               5                   10                  15
   Asp Asn Leu Val Asn Gly Tyr Arg Cys Thr Cys Lys Lys Gly Phe Lys
               20                  25                  30
   Gly Tyr Asn Cys Gln
           35
<210> 935
   <211> 37
   <212> PRT
   <213> Homo sapiens
<400> 935
   Asn Ile Asp Glu Cys Ala Ser Asn Pro Cys Leu Asn Gln Gly Thr Cys
   1               5                   10                  15
   Phe Asp Asp Ile Ser Gly Tyr Thr Cys His Cys Val Leu Pro Tyr Thr
               20                  25                  30
   Gly Lys Asn Cys Gln
           35
<210> 936
   <211> 39
   <212> PRT
   <213> Homo sapiens
<400> 936
   Val Leu Ala Pro Cys Ser Pro Asn Pro Cys Glu Asn Ala Ala Val Cys
   1               5                   10                  15
   Lys Glu Ser Pro Asn Phe Glu Ser Tyr Thr Cys Leu Cys Ala Pro Gly
               20                  25                  30
   Trp Gln Gly Gln Arg Cys Thr
           35
<210> 937
   <211> 37
   <212> PRT
   <213> Homo sapiens
<400> 937
   Asp Ile Asp Glu Cys Ile Ser Lys Pro Cys Met Asn His Gly Leu Cys
   1               5                   10                  15
   His Asn Thr Gln Gly Ser Tyr Met Cys Glu Cys Pro Pro Gly Phe Ser
               20                  25                  30
   Gly Met Asp Cys Glu
           35
<210> 938
   <211> 37
   <212> PRT
   <213> Homo sapiens
<400> 938
   Asp Ile Asp Asp Cys Leu Ala Asn Pro Cys Gln Asn Gly Gly Ser Cys
   1               5                   10                  15
   Met Asp Gly Val Asn Thr Phe Ser Cys Leu Cys Leu Pro Gly Phe Thr
               20                  25                  30
   Gly Asp Lys Cys Gln
           35
<210> 939
   <211> 37
   <212> PRT
   <213> Homo sapiens
<400> 939
   Asp Met Asn Glu Cys Leu Ser Glu Pro Cys Lys Asn Gly Gly Thr Cys
   1               5                   10                  15
   Ser Asp Tyr Val Asn Ser Tyr Thr Cys Lys Cys Gln Ala Gly Phe Asp
               20                  25                  30
   Gly Val His Cys Glu
           35
<210> 940
   <211> 37
   <212> PRT
   <213> Homo sapiens
<400> 940
   Asn Ile Asn Glu Cys Thr Glu Ser Ser Cys Phe Asn Gly Gly Thr Cys
   1               5                   10                  15
   Val Asp Gly Ile Asn Ser Phe Ser Cys Leu Cys Pro Val Gly Phe Thr
               20                  25                  30
   Gly Ser Phe Cys Leu
           35
<210> 941
   <211> 37
   <212> PRT
   <213> Homo sapiens
<400> 941
   Glu Ile Asn Glu Cys Ser Ser His Pro Cys Leu Asn Glu Gly Thr Cys
   1               5                   10                  15
   Val Asp Gly Leu Gly Thr Tyr Arg Cys Ser Cys Pro Leu Gly Tyr Thr
               20                  25                  30
   Gly Lys Asn Cys Gln
           35
<210> 942
   <211> 37
   <212> PRT
   <213> Homo sapiens
<400> 942
   Leu Val Asn Leu Cys Ser Arg Ser Pro Cys Lys Asn Lys Gly Thr Cys
   1               5                   10                  15
   Val Gln Lys Lys Ala Glu Ser Gln Cys Leu Cys Pro Ser Gly Trp Ala
               20                  25                  30
   Gly Ala Tyr Cys Asp
           35
<210> 943
   <211> 47
   <212> PRT
   <213> Homo sapiens
<400> 943
   Pro Asn Val Ser Cys Asp Ile Ala Ala Ser Arg Arg Gly Val Leu Val
   1               5                   10                  15
   Glu His Leu Cys Gln His Ser Gly Val Cys Ile Asn Ala Gly Asn Thr
               20                  25                  30
   His Tyr Cys Gln Cys Pro Leu Gly Tyr Thr Gly Ser Tyr Cys Glu
           35                  40                  45
<210> 944
   <211> 39
   <212> PRT
   <213> Homo sapiens
<400> 944
   Thr Ser His Pro Cys Phe Val Ser Arg Pro Cys Leu Asn Gly Gly Thr
   1               5                   10                  15
   Cys His Met Leu Ser Arg Asp Thr Tyr Glu Cys Thr Cys Gln Val Gly
               20                  25                  30
   Phe Thr Gly Lys Glu Cys Gln
           35
<210> 945
   <211> 37
   <212> PRT
   <213> Homo sapiens
<400> 945
   Gln Leu Asp Glu Cys Ala Ser Asn Pro Cys Gln His Gly Ala Thr Cys
   1               5                   10                  15
   Ser Asp Phe Ile Gly Gly Tyr Arg Cys Glu Cys Val Pro Gly Tyr Gln
               20                  25                  30
   Gly Val Asn Cys Glu
           35
<210> 946
   <211> 37
   <212> PRT
   <213> Homo sapiens
<400> 946
   Glu Val Asp Glu Cys Gln Asn Gln Pro Cys Gln Asn Gly Gly Thr Cys
   1               5                   10                  15
   Ile Asp Leu Val Asn His Phe Lys Cys Ser Cys Pro Pro Gly Thr Arg
               20                  25                  30
   Gly Leu Leu Cys Glu
           35
<210> 947
   <211> 38
   <212> PRT
   <213> Homo sapiens
<400> 947
   Asn Ile Asp Asp Cys Ala Arg Gly Pro His Cys Leu Asn Gly Gly Gln
   1               5                   10                  15
   Cys Met Asp Arg Ile Gly Gly Tyr Ser Cys Arg Cys Leu Pro Gly Phe
               20                  25                  30
   Ala Gly Glu Arg Cys Glu
           35
<210> 948
   <211> 39
   <212> PRT
   <213> Homo sapiens
<400> 948
   Asp Ile Asn Glu Cys Leu Ser Asn Pro Cys Ser Ser Glu Gly Ser Leu
   1               5                   10                  15
   Asp Cys Ile Gln Leu Thr Asn Asp Tyr Leu Cys Val Cys Arg Ser Ala
               20                  25                  30
   Phe Thr Gly Arg His Cys Glu
           35
<210> 949
   <211> 40
   <212> PRT
   <213> Homo sapiens
<400> 949
   Phe Val Asp Val Cys Pro Gln Met Pro Cys Leu Asn Gly Gly Thr Cys
   1               5                   10                  15
   Ala Val Ala Ser Asn Met Pro Asp Gly Phe Ile Cys Arg Cys Pro Pro
               20                  25                  30
   Gly Phe Ser Gly Ala Arg Cys Gln
           35                  40
<210> 950
   <211> 39
   <212> PRT
   <213> Homo sapiens
<400> 950
   Cys Glu Ser Gly Cys Ala Ser Ser Pro Cys Gln His Gly Gly Ser Cys
   1               5                   10                  15
   His Pro Gln Arg Gln Pro Pro Tyr Tyr Ser Cys Gln Cys Ala Pro Pro
               20                  25                  30
   Phe Ser Gly Ser Arg Cys Glu
           35
<210> 951
   <211> 37
   <212> PRT
   <213> Homo sapiens
<400> 951
   Trp Thr Asp Ala Cys Leu Ser His Pro Cys Ala Asn Gly Ser Thr Cys
   1               5                   10                  15
   Thr Thr Val Ala Asn Gln Phe Ser Cys Lys Cys Leu Thr Gly Phe Thr
               20                  25                  30
   Gly Gln Lys Cys Glu
           35
<210> 952
   <211> 38
   <212> PRT
   <213> Homo sapiens
<400> 952
   Asp Val Asn Glu Cys Asp Ile Pro Gly His Cys Gln His Gly Gly Thr
   1               5                   10                  15
   Cys Leu Asn Leu Pro Gly Ser Tyr Gln Cys Gln Cys Pro Gln Gly Phe
               20                  25                  30
   Thr Gly Gln Tyr Cys Asp
           35
<210> 953
   <211> 38
   <212> PRT
   <213> Homo sapiens
<400> 953
   Leu Tyr Val Pro Cys Ala Pro Ser Pro Cys Val Asn Gly Gly Thr Cys
   1               5                   10                  15
   Arg Gln Thr Gly Asp Phe Thr Phe Glu Cys Asn Cys Leu Pro Gly Phe
               20                  25                  30
   Glu Gly Ser Thr Cys Glu
           35
<210> 954
   <211> 37
   <212> PRT
   <213> Homo sapiens
<400> 954
   Asn Ile Asp Asp Cys Pro Asn His Arg Cys Gln Asn Gly Gly Val Cys
   1               5                   10                  15
   Val Asp Gly Val Asn Thr Tyr Asn Cys Arg Cys Pro Pro Gln Trp Thr
               20                  25                  30
   Gly Gln Phe Cys Thr
           35
<210> 955
   <211> 39
   <212> PRT
   <213> Homo sapiens
<400> 955
   Asp Val Asp Glu Cys Leu Leu Gln Pro Asn Ala Cys Gln Asn Gly Gly
   1               5                   10                  15
   Thr Cys Ala Asn Arg Asn Gly Gly Tyr Gly Cys Val Cys Val Asn Gly
               20                  25                  30
   Trp Ser Gly Asp Asp Cys Ser
           35
<210> 956
   <211> 37
   <212> PRT
   <213> Homo sapiens
<400> 956
   Asn Ile Asp Asp Cys Ala Phe Ala Ser Cys Thr Pro Gly Ser Thr Cys
   1 5 10 15
   Ile Asp Arg Val Ala Ser Phe Ser Cys Met Cys Pro Glu Gly Lys Ala
   20 25 30
   Gly Leu Leu Cys His
   35
<210> 957
   <211> 38
   <212> PRT
   <213> Homo sapiens
<400> 957
   Ala Pro Pro Cys Leu Asp Gly Ser Pro Cys Ala Asn Gly Gly Arg Cys
   1 5 10 15
   Thr Gln Leu Pro Ser Arg Glu Ala Ala Cys Leu Cys Pro Pro Gly Trp
   20 25 30
   Val Gly Glu Arg Cys Gln
   35
<210> 958
   <211> 39
   <212> PRT
   <213> Homo sapiens
<400> 958
   Asp Val Asp Glu Cys Ser Ile Gly Ala Asn Pro Cys Glu His Leu Gly
   1 5 10 15
   Arg Cys Val Asn Thr Gln Gly Ser Phe Leu Cys Gln Cys Gly Arg Gly
   20 25 30
   Tyr Thr Gly Pro Arg Cys Glu
   35
<210> 959
   <211> 37
   <212> PRT
   <213> Homo sapiens
<400> 959
   Asp Val Asn Glu Cys Leu Ser Gly Pro Cys Arg Asn Gln Ala Thr Cys
   1 5 10 15
   Leu Asp Arg Ile Gly Gln Phe Thr Cys Ile Cys Met Ala Gly Phe Thr
   20 25 30
   Gly Thr Tyr Cys Glu
   35
<210> 960
   <211> 37
   <212> PRT
   <213> Homo sapiens
<400> 960
   Asp Ile Asp Glu Cys Gln Ser Ser Pro Cys Val Asn Gly Gly Val Cys
   1               5                   10                  15
   Lys Asp Arg Val Asn Gly Phe Ser Cys Thr Cys Pro Ser Gly Phe Ser
               20                  25                  30
   Gly Ser Thr Cys Gln
           35
<210> 961
   <211> 37
   <212> PRT
   <213> Homo sapiens
<400> 961
   Asp Val Asp Glu Cys Ala Ser Thr Pro Cys Arg Asn Gly Ala Lys Cys
   1               5                   10                  15
   Val Asp Gln Pro Asp Gly Tyr Glu Cys Arg Cys Ala Glu Gly Phe Glu
               20                  25                  30
   Gly Thr Leu Cys Asp
           35
<210> 962
   <211> 36
   <212> PRT
   <213> Homo sapiens
<400> 962
   Asn Val Asp Asp Cys Ser Pro Asp Pro Cys His His Gly Arg Cys Val
   1               5                   10                  15
   Asp Gly Ile Ala Ser Phe Ser Cys Ala Cys Ala Pro Gly Tyr Thr Gly
               20                  25                  30
   Thr Arg Cys Glu
           35
<210> 963
   <211> 37
   <212> PRT
   <213> Homo sapiens
<400> 963
   Gln Val Asp Glu Cys Arg Ser Gln Pro Cys Arg His Gly Gly Lys Cys
   1               5                   10                  15
   Leu Asp Leu Val Asp Lys Tyr Leu Cys Arg Cys Pro Ser Gly Thr Thr
               20                  25                  30
   Gly Val Asn Cys Glu
           35
<210> 964
   <211> 36
   <212> PRT
   <213> Homo sapiens
<400> 964
   Asn Ile Asp Asp Cys Ala Ser Asn Pro Cys Thr Phe Gly Val Cys Arg
   1               5                   10                  15
   Asp Gly Ile Asn Arg Tyr Asp Cys Val Cys Gln Pro Gly Phe Thr Gly
               20                  25                  30
   Pro Leu Cys Asn
           35
<210> 965
   <211> 37
   <212> PRT
   <213> Homo sapiens
<400> 965
   Glu Ile Asn Glu Cys Ala Ser Ser Pro Cys Gly Glu Gly Gly Ser Cys
   1               5                   10                  15
   Val Asp Gly Glu Asn Gly Phe Arg Cys Leu Cys Pro Pro Gly Ser Leu
               20                  25                  30
   Pro Pro Leu Cys Leu
           35
<210> 966
   <211> 36
   <212> PRT
   <213> Homo sapiens
<400> 966
   Pro Ser His Pro Cys Ala His Glu Pro Cys Ser His Gly Ile Cys Tyr
   1               5                   10                  15
   Asp Ala Pro Gly Gly Phe Arg Cys Val Cys Glu Pro Gly Trp Ser Gly
               20                  25                  30
   Pro Arg Cys Ser
           35
<210> 967
   <211> 37
   <212> PRT
   <213> Homo sapiens
<400> 967
   Ala Arg Asp Ala Cys Glu Ser Gln Pro Cys Arg Ala Gly Gly Thr Cys
   1               5                   10                  15
   Ser Ser Asp Gly Met Gly Phe His Cys Thr Cys Pro Pro Gly Val Gln
               20                  25                  30
   Gly Arg Gln Cys Glu
           35
<210> 968
   <211> 41
   <212> PRT
   <213> Homo sapiens
<400> 968
   Leu Glu Asp Pro Cys His Ser Gly Pro Cys Ala Gly Arg Gly Val Cys
   1               5                   10                  15
   Gln Ser Ser Val Val Ala Gly Thr Ala Arg Phe Ser Cys Arg Cys Pro
               20                  25                  30
   Arg Gly Phe Arg Gly Pro Asp Cys Ser
           35                  40
<210> 969
   <211> 38
   <212> PRT
   <213> Homo sapiens
<400> 969
   Leu Leu Ser Pro Cys Thr Pro Asn Pro Cys Glu His Gly Gly Arg Cys
   1               5                   10                  15
   Glu Ser Ala Pro Gly Gln Leu Pro Val Cys Ser Cys Pro Gln Gly Trp
               20                  25                  30
   Gln Gly Pro Arg Cys Gln
           35
<210> 970
   <211> 38
   <212> PRT
   <213> Homo sapiens
<400> 970
   Asp Val Asp Glu Cys Ala Gly Pro Ala Pro Cys Gly Pro His Gly Ile
   1               5                   10                  15
   Cys Thr Asn Leu Ala Gly Ser Phe Ser Cys Thr Cys His Gly Gly Tyr
               20                  25                  30
   Thr Gly Pro Ser Cys Asp
           35
<210> 971
   <211> 37
   <212> PRT
   <213> Homo sapiens
<400> 971
   Asp Ile Asn Asp Cys Asp Pro Asn Pro Cys Leu Asn Gly Gly Ser Cys
   1               5                   10                  15
   Gln Asp Gly Val Gly Ser Phe Ser Cys Ser Cys Leu Pro Gly Phe Ala
               20                  25                  30
   Gly Pro Arg Cys Ala
           35
<210> 972
   <211> 36
   <212> PRT
   <213> Homo sapiens
<400> 972
   Asp Val Asp Glu Cys Leu Ser Asn Pro Cys Gly Pro Gly Thr Cys Thr
   1               5                   10                  15
   Asp His Val Ala Ser Phe Thr Cys Thr Cys Pro Pro Gly Tyr Gly Gly
               20                  25                  30
   Phe His Cys Glu
           35
<210> 973
   <211> 37
   <212> PRT
   <213> Homo sapiens
<400> 973
   Asp Leu Pro Asp Cys Ser Pro Ser Ser Cys Phe Asn Gly Gly Thr Cys
   1               5                   10                  15
   Val Asp Gly Val Asn Ser Phe Ser Cys Leu Cys Arg Pro Gly Tyr Thr
               20                  25                  30
   Gly Ala His Cys Gln
           35
<210> 974
   <211> 37
   <212> PRT
   <213> Homo sapiens
<400> 974
   Glu Ala Asp Pro Cys Leu Ser Arg Pro Cys Leu His Gly Gly Val Cys
   1               5                   10                  15
   Ser Ala Ala His Pro Gly Phe Arg Cys Thr Cys Leu Glu Ser Phe Thr
               20                  25                  30
   Gly Pro Gln Cys Gln
           35
<210> 975
   <211> 35
   <212> PRT
   <213> Homo sapiens
<400> 975
   Leu Val Asp Trp Cys Ser Arg Gln Pro Cys Gln Asn Gly Gly Arg Cys
   1               5                   10                  15
   Val Gln Thr Gly Ala Tyr Cys Leu Cys Pro Pro Gly Trp Ser Gly Arg
               20                  25                  30
   Leu Cys Asp
           35
<210> 976
   <211> 36
   <212> PRT
   <213> Homo sapiens
<400> 976
   Ile Gly Val Arg Leu Glu Gln Leu Cys Gln Ala Gly Gly Gln Cys Val
   1               5                   10                  15
   Asp Glu Asp Ser Ser His Tyr Cys Val Cys Pro Glu Gly Arg Thr Gly
               20                  25                  30
   Ser His Cys Glu
           35
<210> 977
   <211> 37
   <212> PRT
   <213> Homo sapiens
<400> 977
   Glu Val Asp Pro Cys Leu Ala Gln Pro Cys Gln His Gly Gly Thr Cys
   1               5                   10                  15
   Arg Gly Tyr Met Gly Gly Tyr Met Cys Glu Cys Leu Pro Gly Tyr Asn
               20                  25                  30
   Gly Asp Asn Cys Glu
           35
<210> 978
   <211> 37
   <212> PRT
   <213> Homo sapiens
<400> 978
   Asp Val Asp Glu Cys Ala Ser Gln Pro Cys Gln His Gly Gly Ser Cys
   1               5                   10                  15
   Ile Asp Leu Val Ala Arg Tyr Leu Cys Ser Cys Pro Pro Gly Thr Leu
               20                  25                  30
   Gly Val Leu Cys Glu
           35
<210> 979
   <211> 38
   <212> PRT
   <213> Homo sapiens
<400> 979
   Leu Pro Asp Pro Cys Leu Ser Ser Pro Cys Ala His Gly Ala Arg Cys
   1               5                   10                  15
   Ser Val Gly Pro Asp Gly Arg Phe Leu Cys Ser Cys Pro Pro Gly Tyr
               20                  25                  30
   Gln Gly Arg Ser Cys Arg
           35
<210> 980
   <211> 44
   <212> PRT
   <213> Homo sapiens
<400> 980
   Asn Glu Asp Asp Cys Gly Pro Gly Pro Pro Leu Asp Ser Gly Pro Arg
   1               5                   10                  15
   Cys Leu His Asn Gly Thr Cys Val Asp Leu Val Gly Gly Phe Arg Cys
               20                  25                  30
   Thr Cys Pro Pro Gly Tyr Thr Gly Leu Arg Cys Glu
           35                  40
<210> 981
   <211> 40
   <212> PRT
   <213> Homo sapiens
<400> 981
   Asp Ile Asn Glu Cys Arg Ser Gly Ala Cys His Ala Ala His Thr Arg
   1               5                   10                  15
   Asp Cys Leu Gln Asp Pro Gly Gly Gly Phe Arg Cys Leu Cys His Ala
               20                  25                  30
   Gly Phe Ser Gly Pro Arg Cys Gln
           35                  40
<210> 982
   <211> 42
   <212> PRT
   <213> Homo sapiens
<400> 982
   Val Leu Ser Pro Cys Glu Ser Gln Pro Cys Gln His Gly Gly Gln Cys
   1               5                   10                  15
   Arg Pro Ser Pro Gly Pro Gly Gly Gly Leu Thr Phe Thr Cys His Cys
               20                  25                  30
   Ala Gln Pro Phe Trp Gly Pro Arg Cys Glu
           35                  40
<210> 983
   <211> 37
   <212> PRT
   <213> Homo sapiens
<400> 983
   Val Ala Arg Ser Cys Arg Glu Leu Gln Cys Pro Val Gly Val Pro Cys
   1               5                   10                  15
   Gln Gln Thr Pro Arg Gly Pro Arg Cys Ala Cys Pro Pro Gly Leu Ser
               20                  25                  30
   Gly Pro Ser Cys Arg
           35
<210> 984
   <211> 39
   <212> PRT
   <213> Homo sapiens
<400> 984
   Ser Asn Ala Ser Cys Ala Ala Ala Pro Cys Leu His Gly Gly Ser Cys
   1               5                   10                  15
   Arg Pro Ala Pro Leu Ala Pro Phe Phe Arg Cys Ala Cys Ala Gln Gly
               20                  25                  30
   Trp Thr Gly Pro Arg Cys Glu
           35
<210> 985
   <211> 38
   <212> PRT
   <213> Homo sapiens
<400> 985
   Asp Val Asp Glu Cys Arg Val Gly Glu Pro Cys Arg His Gly Gly Thr
   1               5                   10                  15
   Cys Leu Asn Thr Pro Gly Ser Phe Arg Cys Gln Cys Pro Ala Gly Tyr
               20                  25                  30
   Thr Gly Pro Leu Cys Glu
           35
<210> 986
   <211> 38
   <212> PRT
   <213> Homo sapiens
<400> 986
   Pro Ala Val Pro Cys Ala Pro Ser Pro Cys Arg Asn Gly Gly Thr Cys
   1               5                   10                  15
   Arg Gln Ser Gly Asp Leu Thr Tyr Asp Cys Ala Cys Leu Pro Gly Phe
               20                  25                  30
   Glu Gly Gln Asn Cys Glu
           35
<210> 987
   <211> 37
   <212> PRT
   <213> Homo sapiens
<400> 987
   Asn Val Asp Asp Cys Pro Gly His Arg Cys Leu Asn Gly Gly Thr Cys
   1               5                   10                  15
   Val Asp Gly Val Asn Thr Tyr Asn Cys Gln Cys Pro Pro Glu Trp Thr
               20                  25                  30
   Gly Gln Phe Cys Thr
           35
<210> 988
   <211> 39
   <212> PRT
   <213> Homo sapiens
<400> 988
   Asp Val Asp Glu Cys Gln Leu Gln Pro Asn Ala Cys His Asn Gly Gly
   1               5                   10                  15
   Thr Cys Phe Asn Thr Leu Gly Gly His Ser Cys Val Cys Val Asn Gly
               20                  25                  30
   Trp Thr Gly Glu Ser Cys Ser
           35
<210> 989
   <211> 37
   <212> PRT
   <213> Homo sapiens
<400> 989
   Asn Ile Asp Asp Cys Ala Thr Ala Val Cys Phe His Gly Ala Thr Cys
   1               5                   10                  15
   His Asp Arg Val Ala Ser Phe Tyr Cys Ala Cys Pro Met Gly Lys Thr
               20                  25                  30
   Gly Leu Leu Cys His
           35
<210> 990
   <211> 39
   <212> PRT
   <213> Homo sapiens
<400> 990
   Leu Asp Asp Ala Cys Val Ser Asn Pro Cys His Glu Asp Ala Ile Cys
   1               5                   10                  15
   Asp Thr Asn Pro Val Asn Gly Arg Ala Ile Cys Thr Cys Pro Pro Gly
               20                  25                  30
   Phe Thr Gly Gly Ala Cys Asp
           35
<210> 991
   <211> 40
   <212> PRT
   <213> Homo sapiens
<400> 991
   Arg Gly Leu Leu Cys Gly Ser Phe Pro Glu Pro Cys Ala Asn Gly Gly
   1               5                   10                  15
   Thr Cys Leu Ser Leu Ser Leu Gly Gln Gly Thr Cys Gln Cys Ala Pro
               20                  25                  30
   Gly Phe Leu Gly Glu Thr Cys Gln
           35                  40
<210> 992
   <211> 39
   <212> PRT
   <213> Homo sapiens
<400> 992
   Leu Glu Asp Met Cys Leu Ser Gln Pro Cys His Gly Asp Ala Gln Cys
   1 5 10 15
   Ser Thr Asn Pro Leu Thr Gly Ser Thr Leu Cys Leu Cys Gln Pro Gly
   20 25 30
   Tyr Ser Gly Pro Thr Cys His
   35
<210> 993
   <211> 42
   <212> PRT
   <213> Homo sapiens
<400> 993
   Asp Leu Asp Glu Cys Leu Met Ala Gln Gln Gly Pro Ser Pro Cys Glu
   1 5 10 15
   His Gly Gly Ser Cys Leu Asn Thr Pro Gly Ser Phe Asn Cys Leu Cys
   20 25 30
   Pro Pro Gly Tyr Thr Gly Ser Arg Cys Glu
   35 40
<210> 994
   <211> 37
   <212> PRT
   <213> Homo sapiens
<400> 994
   Asp His Asn Glu Cys Leu Ser Gln Pro Cys His Pro Gly Ser Thr Cys
   1 5 10 15
   Leu Asp Leu Leu Ala Thr Phe His Cys Leu Cys Pro Pro Gly Leu Glu
   20 25 30
   Gly Gln Leu Cys Glu
   35
<210> 995
   <211> 37
   <212> PRT
   <213> Homo sapiens
<400> 995
   Glu Thr Asn Glu Cys Ala Ser Ala Pro Cys Leu Asn His Ala Asp Cys
   1 5 10 15
   His Asp Leu Leu Asn Gly Phe Gln Cys Ile Cys Leu Pro Gly Phe Ser
   20 25 30
   Gly Thr Arg Cys Glu
   35
<210> 996
   <211> 37
   <212> PRT
   <213> Homo sapiens
<400> 996
   Asp Ile Asp Glu Cys Arg Ser Ser Pro Cys Ala Asn Gly Gly Gln Cys
   1               5                   10                  15
   Gln Asp Gln Pro Gly Ala Phe His Cys Lys Cys Leu Pro Gly Phe Glu
               20                  25                  30
   Gly Pro Arg Cys Gln
           35
<210> 997
   <211> 37
   <212> PRT
   <213> Homo sapiens
<400> 997
   Glu Val Asp Glu Cys Leu Ser Asp Pro Cys Pro Val Gly Ala Ser Cys
   1               5                   10                  15
   Leu Asp Leu Pro Gly Ala Phe Phe Cys Leu Cys Pro Ser Gly Phe Thr
               20                  25                  30
   Gly Gln Leu Cys Glu
           35
<210> 998
   <211> 34
   <212> PRT
   <213> Homo sapiens
<400> 998
   Val Pro Leu Cys Ala Pro Asn Leu Cys Gln Pro Lys Gln Ile Cys Lys
   1               5                   10                  15
   Asp Gln Lys Asp Lys Ala Asn Cys Leu Cys Pro Asp Gly Ser Pro Gly
               20                  25                  30
   Cys Ala
<210> 999
   <211> 29
   <212> PRT
   <213> Homo sapiens
<400> 999
   Pro Glu Asp Asn Cys Thr Cys His His Gly His Cys Gln Arg Ser Ser
   1               5                   10                  15
   Cys Val Cys Asp Val Gly Trp Thr Gly Pro Glu Cys Glu
               20                  25
<210> 1000
   <211> 37
   <212> PRT
   <213> Homo sapiens
<400> 1000
   Glu Leu Gly Gly Cys Ile Ser Ala Pro Cys Ala His Gly Gly Thr Cys
   1               5                   10                  15
   Tyr Pro Gln Pro Ser Gly Tyr Asn Cys Thr Cys Pro Thr Gly Tyr Thr
               20                  25                  30
   Gly Pro Thr Cys Ser
   35
<210> 1001
   <211> 37
   <212> PRT
   <213> Homo sapiens
<400> 1001
   Glu Met Thr Ala Cys His Ser Gly Pro Cys Leu Asn Gly Gly Ser Cys
   1               5                   10                  15
   Asn Pro Ser Pro Gly Gly Tyr Tyr Cys Thr Cys Pro Pro Ser His Thr
               20                  25                  30
   Gly Pro Gln Cys Gln
           35
<210> 1002
   <211> 52
   <212> PRT
   <213> Homo sapiens
<400> 1002
   Phe Pro Asp Pro Cys Gln Asn Ala Gln Leu Cys Gln Asn Gly Gly Ser
   1               5                   10                  15
   Cys Gln Ala Leu Leu Pro Ala Pro Leu Gly Leu Pro Ser Ser Pro Ser
               20                  25                  30
   Pro Leu Thr Pro Ser Phe Leu Cys Thr Cys Leu Pro Gly Phe Thr Gly
           35                  40                  45
   Glu Arg Cys Gln
       50
<210> 1003
   <211> 37
   <212> PRT
   <213> Homo sapiens
<400> 1003
   Ser Thr Asp Tyr Cys Val Ser Ala Pro Cys Phe Asn Gly Gly Thr Cys
   1               5                   10                  15
   Val Asn Arg Pro Gly Thr Phe Ser Cys Leu Cys Ala Met Gly Phe Gln
               20                  25                  30
   Gly Pro Arg Cys Glu
           35
<210> 1004
   <211> 37
   <212> PRT
   <213> Homo sapiens
<400> 1004
   Leu Arg Pro Ser Cys Ala Asp Ser Pro Cys Arg Asn Arg Ala Thr Cys
   1               5                   10                  15
   Gln Asp Ser Pro Gln Gly Pro Arg Cys Leu Cys Pro Thr Gly Tyr Thr
               20                  25                  30
   Gly Gly Ser Cys Gln
           35
<210> 1005
   <211> 37
   <212> PRT
   <213> Homo sapiens
<400> 1005
   Leu Met Asp Leu Cys Ala Gln Lys Pro Cys Pro Arg Asn Ser His Cys
   1               5                   10                  15
   Leu Gln Thr Gly Pro Ser Phe His Cys Leu Cys Leu Gln Gly Trp Thr
               20                  25                  30
   Gly Pro Leu Cys Asn
           35
<210> 1006
   <211> 47
   <212> PRT
   <213> Homo sapiens
<400> 1006
   Pro Leu Ser Ser Cys Gln Lys Ala Ala Leu Ser Gln Gly Ile Asp Val
   1               5                   10                  15
   Ser Ser Leu Cys His Asn Gly Gly Leu Cys Val Asp Ser Gly Pro Ser
               20                  25                  30
   Tyr Phe Cys His Cys Pro Pro Gly Phe Gln Gly Ser Leu Cys Gln
           35                  40                  45
<210> 1007
   <211> 37
   <212> PRT
   <213> Homo sapiens
<400> 1007
   His Val Asn Pro Cys Glu Ser Arg Pro Cys Gln Asn Gly Ala Thr Cys
   1               5                   10                  15
   Met Ala Gln Pro Ser Gly Tyr Leu Cys Gln Cys Ala Pro Gly Tyr Asp
               20                  25                  30
   Gly Gln Asn Cys Ser
           35
<210> 1008
   <211> 37
   <212> PRT
   <213> Homo sapiens
<400> 1008
   Glu Leu Asp Ala Cys Gln Ser Gln Pro Cys His Asn His Gly Thr Cys
   1               5                   10                  15
   Thr Pro Lys Pro Gly Gly Phe His Cys Ala Cys Pro Pro Gly Phe Val
               20                  25                  30
   Gly Leu Arg Cys Glu
           35
<210> 1009
   <211> 39
   <212> PRT
   <213> Homo sapiens
<400> 1009
   Asp Val Asp Glu Cys Leu Asp Gln Pro Cys His Pro Thr Gly Thr Ala
   1               5                   10                  15
   Ala Cys His Ser Leu Ala Asn Ala Phe Tyr Cys Gln Cys Leu Pro Gly
               20                  25                  30
   His Thr Gly Gln Trp Cys Glu
           35
<210> 1010
   <211> 40
   <212> PRT
   <213> Homo sapiens
<400> 1010
   Glu Ile Asp Pro Cys His Ser Gln Pro Cys Phe His Gly Gly Thr Cys
   1               5                   10                  15
   Glu Ala Thr Ala Gly Ser Pro Leu Gly Phe Ile Cys His Cys Pro Lys
               20                  25                  30
   Gly Phe Glu Gly Pro Thr Cys Ser
           35                  40
<210> 1011
   <211> 40
   <212> PRT
   <213> Homo sapiens
<400> 1011
   Arg Ala Pro Ser Cys Gly Phe His His Cys His His Gly Gly Leu Cys
   1               5                   10                  15
   Leu Pro Ser Pro Lys Pro Gly Phe Pro Pro Arg Cys Ala Cys Leu Ser
               20                  25                  30
   Gly Tyr Gly Gly Pro Asp Cys Leu
           35                  40
<210> 1012
   <211> 42
   <212> PRT
   <213> Homo sapiens
<400> 1012
   Ala Pro Lys Gly Cys Gly Pro Pro Ser Pro Cys Leu Tyr Asn Gly Ser
   1               5                   10                  15
   Cys Ser Glu Thr Thr Gly Leu Gly Gly Pro Gly Phe Arg Cys Ser Cys
               20                  25                  30
   Pro His Ser Ser Pro Gly Pro Arg Cys Gln
           35                  40
<210> 1013
   <211> 38
   <212> PRT
   <213> Homo sapiens
<400> 1013
   Leu Glu Asp Pro Cys Pro Pro Ser Phe Cys Ser Lys Arg Gly Arg Cys
   1               5                   10                  15
   His Ile Gln Ala Ser Gly Arg Pro Gln Cys Ser Cys Met Pro Gly Trp
               20                  25                  30
   Thr Gly Glu Gln Cys Gln
           35
<210> 1014
   <211> 37
   <212> PRT
   <213> Homo sapiens
<400> 1014
   Leu Arg Asp Phe Cys Ser Ala Asn Pro Cys Val Asn Gly Gly Val Cys
   1               5                   10                  15
   Leu Ala Thr Tyr Pro Gln Ile Gln Cys His Cys Pro Pro Gly Phe Glu
               20                  25                  30
   Gly His Ala Cys Glu
           35
<210> 1015
   <211> 39
   <212> PRT
   <213> Homo sapiens
<400> 1015
   Asp Val Asn Glu Cys Phe Gln Asp Pro Gly Pro Cys Pro Lys Gly Thr
   1               5                   10                  15
   Ser Cys His Asn Thr Leu Gly Ser Phe Gln Cys Leu Cys Pro Val Gly
               20                  25                  30
   Gln Glu Gly Pro Arg Cys Glu
           35
<210> 1016
   <211> 41
   <212> PRT
   <213> Homo sapiens
<400> 1016
   Arg Ala Gly Pro Cys Pro Pro Arg Gly Cys Ser Asn Gly Gly Thr Cys
   1               5                   10                  15
   Gln Leu Met Pro Glu Lys Asp Ser Thr Phe His Leu Cys Leu Cys Pro
               20                  25                  30
   Pro Gly Phe Ile Gly Pro Asp Cys Glu
           35                  40
<210> 1017
   <211> 37
   <212> PRT
   <213> Homo sapiens
<400> 1017
   Asn Pro Asp Asn Cys Val Ser His Gln Cys Gln Asn Gly Gly Thr Cys
   1               5                   10                  15
   Gln Asp Gly Leu Asp Thr Tyr Thr Cys Leu Cys Pro Glu Thr Trp Thr
               20                  25                  30
   Gly Trp Asp Cys Ser
           35
<210> 1018
   <211> 40
   <212> PRT
   <213> Homo sapiens
<400> 1018
   Asp Val Asp Glu Cys Glu Thr Gln Gly Pro Pro His Cys Arg Asn Gly
   1               5                   10                  15
   Gly Thr Cys Gln Asn Ser Ala Gly Ser Phe His Cys Val Cys Val Ser
               20                  25                  30
   Gly Trp Gly Gly Thr Ser Cys Glu
           35                  40
<210> 1019
   <211> 37
   <212> PRT
   <213> Homo sapiens
<400> 1019
   Asn Leu Asp Asp Cys Ile Ala Ala Thr Cys Ala Pro Gly Ser Thr Cys
   1               5                   10                  15
   Ile Asp Arg Val Gly Ser Phe Ser Cys Leu Cys Pro Pro Gly Arg Thr
               20                  25                  30
   Gly Leu Leu Cys His
           35
<210> 1020
   <211> 41
   <212> PRT
   <213> Homo sapiens
<400> 1020
   Ser Arg Gln Thr Cys Ala Asn Asn Arg His Gln Cys Ser Val His Ala
   1 5 10 15
   Glu Cys Arg Asp Tyr Ala Thr Gly Phe Cys Cys Ser Cys Val Ala Gly
   20 25 30
   Tyr Thr Gly Asn Gly Arg Gln Cys Val
   35 40
<210> 1021
   <211> 42
   <212> PRT
   <213> Homo sapiens
<400> 1021
   Leu Gln Asn Pro Cys Tyr Ile Gly Thr His Gly Cys Asp Thr Asn Ala
   1 5 10 15
   Ala Cys Arg Pro Gly Pro Arg Thr Gln Phe Thr Cys Glu Cys Ser Ile
   20 25 30
   Gly Phe Arg Gly Asp Gly Arg Thr Cys Tyr
   35 40
<210> 1022
   <211> 42
   <212> PRT
   <213> Homo sapiens
<400> 1022
   Asp Ile Asp Glu Cys Ser Glu Gln Pro Ser Val Cys Gly Ser His Thr
   1 5 10 15
   Ile Cys Asn Asn His Pro Gly Thr Phe Arg Cys Glu Cys Val Glu Gly
   20 25 30
   Tyr Gln Phe Ser Asp Glu Gly Thr Cys Val
   35 40
<210> 1023
   <211> 44
   <212> PRT
   <213> Homo sapiens
<400> 1023
   Pro Ile Asn Tyr Cys Glu Thr Gly Leu His Asn Cys Asp Ile Pro Gln
   1 5 10 15
   Arg Ala Gln Cys Ile Tyr Thr Gly Gly Ser Ser Tyr Thr Cys Ser Cys
   20 25 30
   Leu Pro Gly Phe Ser Gly Asp Gly Gln Ala Cys Gln
   35 40
<210> 1024
   <211> 39
   <212> PRT
   <213> Homo sapiens
<400> 1024
   Asp Val Asp Glu Cys Gln Pro Ser Arg Cys His Pro Asp Ala Phe Cys
   1               5                   10                  15
   Tyr Asn Thr Pro Gly Ser Phe Thr Cys Gln Cys Lys Pro Gly Tyr Gln
               20                  25                  30
   Gly Asp Gly Phe Arg Cys Val
           35
<210> 1025
   <211> 37
   <212> PRT
   <213> Homo sapiens
<400> 1025
   Gly His Asn Tyr Cys Ser Val Asn Asn Gly Gly Cys Thr His Leu Cys
   1               5                   10                  15
   Leu Ala Thr Pro Gly Ser Arg Thr Cys Arg Cys Pro Asp Asn Thr Leu
               20                  25                  30
   Gly Val Asp Cys Ile
           35
<210> 1026
   <211> 41
   <212> PRT
   <213> Homo sapiens
<400> 1026
   Asn Lys Glu Thr Cys Glu His Asn His Arg Gln Cys Ser Arg His Ala
   1               5                   10                  15
   Phe Cys Thr Asp Tyr Ala Thr Gly Phe Cys Cys His Cys Gln Ser Lys
               20                  25                  30
   Phe Tyr Gly Asn Gly Lys His Cys Leu
           35                  40
<210> 1027
   <211> 42
   <212> PRT
   <213> Homo sapiens
<400> 1027
   Pro Gly Asn Pro Cys Tyr Asp Gly Ser His Met Cys Asp Thr Thr Ala
   1               5                   10                  15
   Arg Cys His Pro Gly Thr Gly Val Asp Tyr Thr Cys Glu Cys Ala Ser
               20                  25                  30
   Gly Tyr Gln Gly Asp Gly Arg Asn Cys Val
           35                  40
<210> 1028
   <211> 43
   <212> PRT
   <213> Homo sapiens
<400> 1028
   Asp Glu Asn Glu Cys Ala Thr Gly Phe His Arg Cys Gly Pro Asn Ser
   1               5                   10                  15
   Val Cys Ile Asn Leu Pro Gly Ser Tyr Arg Cys Glu Cys Arg Ser Gly
               20                  25                  30
   Tyr Glu Phe Ala Asp Asp Arg His Thr Cys Ile
           35                  40
<210> 1029
   <211> 44
   <212> PRT
   <213> Homo sapiens
<400> 1029
   Pro Ala Asn Pro Cys Glu Asp Gly Ser His Thr Cys Ala Pro Ala Gly
   1               5                   10                  15
   Gln Ala Arg Cys Val His His Gly Gly Ser Thr Phe Ser Cys Ala Cys
               20                  25                  30
   Leu Pro Gly Tyr Ala Gly Asp Gly His Gln Cys Thr
           35                  40
<210> 1030
   <211> 39
   <212> PRT
   <213> Homo sapiens
<400> 1030
   Asp Val Asp Glu Cys Ser Glu Asn Arg Cys His Pro Ala Ala Thr Cys
   1               5                   10                  15
   Tyr Asn Thr Pro Gly Ser Phe Ser Cys Arg Cys Gln Pro Gly Tyr Tyr
               20                  25                  30
   Gly Asp Gly Phe Gln Cys Ile
           35
<210> 1031
   <211> 24
   <212> PRT
   <213> Homo sapiens
<400> 1031
   Met Cys Val Thr Tyr His Asn Gly Thr Gly Tyr Cys Lys Cys Pro Glu
   1               5                   10                  15
   Gly Phe Leu Gly Glu Tyr Cys Gln
               20
<210> 1032
   <211> 38
   <212> PRT
   <213> Homo sapiens
<400> 1032
   Asp Val Asn Glu Cys Asp Ile Pro Gly His Cys Gln His Gly Gly Thr
   1               5                   10                  15
   Cys Leu Asn Leu Pro Gly Ser Tyr Gln Cys Gln Cys Leu Gln Gly Phe
               20                  25                  30
   Thr Gly Gln Tyr Cys Asp
           35
<210> 1033
   <211> 38
   <212> PRT
   <213> Homo sapiens
<400> 1033
   Leu Tyr Val Pro Cys Ala Pro Ser Pro Cys Val Asn Gly Gly Thr Cys
   1               5                   10                  15
   Arg Gln Thr Gly Asp Phe Thr Phe Glu Cys Asn Cys Leu Pro Glu Thr
               20                  25                  30
   Val Arg Arg Gly Thr Glu
           35
<210> 1034
   <211> 41
   <212> PRT
   <213> Homo sapiens
<400> 1034
   Asp Glu Asn Glu Cys Glu Gln Asn Asn Gly Gly Cys Ser Glu Ile Cys
   1               5                   10                  15
   Val Asn Leu Lys Asn Ser Tyr Arg Cys Glu Cys Gly Val Gly Arg Val
               20                  25                  30
   Leu Arg Ser Asp Gly Lys Thr Cys Glu
           35                  40
<210> 1035
   <211> 38
   <212> PRT
   <213> Homo sapiens
<400> 1035
   His Arg Ala Lys Cys Ala Pro Ser Tyr Leu Phe Ser Cys Phe Asn Gly
   1               5                   10                  15
   Gly Glu Cys Val His Pro Ala Phe Cys Asp Cys Arg Arg Phe Asn Ala
               20                  25                  30
   Thr Gly Pro Arg Cys Gln
           35
<210> 1036
   <211> 45
   <212> PRT
   <213> Homo sapiens
<400> 1036
   Val Glu Asp Lys Cys Glu Lys Ala Cys Arg Pro Glu Glu Glu Cys Leu
   1               5                   10                  15
   Ala Leu Asn Ser Thr Trp Gly Cys Phe Cys Arg Gln Asp Leu Asn Ser
               20                  25                  30
   Ser Asp Val His Ser Leu Gln Pro Gln Leu Asp Cys Gly
           35                  40                  45
<210> 1037
   <211> 33
   <212> PRT
   <213> Homo sapiens
<400> 1037
   Asp Cys Ser Arg Pro Cys Pro Pro Gly Arg Gln Lys Ser Pro Arg Asp
   1               5                   10                  15
   Pro Cys Gln Cys Val Cys His Gly Ser Ala Val Thr Thr Gln Asp Cys
               20                  25                  30
   Cys
<210> 1038
   <211> 39
   <212> PRT
   <213> Homo sapiens
<400> 1038
   Phe Asp Met Pro Cys Asp Glu Thr Leu Cys Ser Ala Asp Ser Phe Cys
   1               5                   10                  15
   Val Asn Asp Tyr Thr Trp Gly Gly Ser Arg Cys Gln Cys Thr Leu Gly
               20                  25                  30
   Lys Gly Gly Glu Ser Cys Ser
           35
<210> 1039
   <211> 38
   <212> PRT
   <213> Homo sapiens
<400> 1039
   Ser Ser Gly Ile Cys Asp Glu Ala Ser Cys Ile His Gly Gly Thr Cys
   1               5                   10                  15
   Thr Ala Ile Lys Ala Asp Ser Tyr Ile Cys Leu Cys Pro Leu Gly Phe
               20                  25                  30
   Lys Gly Arg His Cys Glu
           35
<210> 1040
   <211> 37
   <212> PRT
   <213> Homo sapiens
<400> 1040
   Ala Ala His Pro Cys Val Arg Ala Pro Cys Ala His Gly Gly Ser Cys
   1               5                   10                  15
   Arg Pro Arg Lys Glu Gly Tyr Asp Cys Asp Cys Pro Leu Gly Phe Glu
               20                  25                  30
   Gly Leu His Cys Gln
           35
<210> 1041
   <211> 31
   <212> PRT
   <213> Homo sapiens
<400> 1041
   Val Pro Leu Cys Ala Gln Glu Cys Val His Gly Arg Cys Val Ala Pro
   1               5                   10                  15
   Asn Gln Cys Gln Cys Val Pro Gly Trp Arg Gly Asp Asp Cys Ser
               20                  25                  30
<210> 1042
   <211> 36
   <212> PRT
   <213> Homo sapiens
<400> 1042
   Ser Gly Phe Phe Cys Pro Ser Thr His Ser Cys Gln Asn Gly Gly Val
   1               5                   10                  15
   Phe Gln Thr Pro Gln Gly Ser Cys Ser Cys Pro Pro Gly Trp Met Gly
               20                  25                  30
   Thr Ile Cys Ser
           35
<210> 1043
   <211> 36
   <212> PRT
   <213> Homo sapiens
<400> 1043
   His Gly Pro Asn Cys Ser Gln Glu Cys Arg Cys His Asn Gly Gly Leu
   1               5                   10                  15
   Cys Asp Arg Phe Thr Gly Gln Cys Arg Cys Ala Pro Gly Tyr Thr Gly
               20                  25                  30
   Asp Arg Cys Arg
           35
<210> 1044
   <211> 36
   <212> PRT
   <213> Homo sapiens
<400> 1044
   Phe Gly Gln Asp Cys Ala Glu Thr Cys Asp Cys Ala Pro Asp Ala Arg
   1               5                   10                  15
   Cys Phe Pro Ala Asn Gly Ala Cys Leu Cys Glu His Gly Phe Thr Gly
   20 25 30
   Asp Arg Cys Thr
   35
<210> 1045
   <211> 36
   <212> PRT
   <213> Homo sapiens
<400> 1045
   His Gly Pro Gly Cys Gln Glu His Cys Leu Cys Leu His Gly Gly Val
   1 5 10 15
   Cys Gln Ala Thr Ser Gly Leu Cys Gln Cys Ala Pro Gly Tyr Thr Gly
   20 25 30
   Pro His Cys Ala
   35
<210> 1046
   <211> 36
   <212> PRT
   <213> Homo sapiens
<400> 1046
   Trp Gly Phe Ser Cys Asn Ala Ser Cys Gln Cys Ala His Glu Ala Val
   1 5 10 15
   Cys Ser Pro Gln Thr Gly Ala Cys Thr Cys Thr Pro Gly Trp His Gly
   20 25 30
   Ala His Cys Gln
   35
<210> 1047
   <211> 31
   <212> PRT
   <213> Homo sapiens
<400> 1047
   Ser Asn Thr Cys Thr Cys Lys Asn Gly Gly Thr Cys Leu Pro Glu Asn
   1 5 10 15
   Gly Asn Cys Val Cys Ala Pro Gly Phe Arg Gly Pro Ser Cys Gln
   20 25 30
<210> 1048
   <211> 35
   <212> PRT
   <213> Homo sapiens
<400> 1048
   Tyr Gly Lys Arg Cys Val Pro Cys Lys Cys Ala Asn His Ser Phe Cys
   1 5 10 15
   His Pro Ser Asn Gly Thr Cys Tyr Cys Leu Ala Gly Trp Thr Gly Pro
   20 25 30
   Asp Cys Ser
   35
<210> 1049
   <211> 36
   <212> PRT
   <213> Homo sapiens
<400> 1049
   Trp Gly Glu Asn Cys Ala Gln Thr Cys Gln Cys His His Gly Gly Thr
   1               5                   10                  15
   Cys His Pro Gln Asp Gly Ser Cys Ile Cys Pro Leu Gly Trp Thr Gly
               20                  25                  30
   His His Cys Leu
           35
<210> 1050
   <211> 41
   <212> PRT
   <213> Homo sapiens
<400> 1050
   His Phe Ser Arg Cys Pro Lys Gln Tyr Lys His Tyr Cys Ile Lys Gly
   1               5                   10                  15
   Arg Cys Arg Phe Val Val Ala Glu Gln Thr Pro Ser Cys Val Cys Asp
               20                  25                  30
   Glu Gly Tyr Ile Gly Ala Arg Cys Glu
           35                  40
<210> 1051
   <211> 42
   <212> PRT
   <213> Homo sapiens
<400> 1051
   Glu Gln Lys Leu Cys Lys Leu Arg Lys Gly Asn Cys Ser Ser Thr Val
   1               5                   10                  15
   Cys Gly Gln Asp Leu Gln Ser His Leu Cys Met Cys Ala Glu Gly Tyr
               20                  25                  30
   Ala Leu Ser Arg Asp Arg Lys Tyr Cys Glu
           35                  40
<210> 1052
   <211> 41
   <212> PRT
   <213> Homo sapiens
<400> 1052
   Asp Val Asn Glu Cys Ala Phe Trp Asn His Gly Cys Thr Leu Gly Cys
   1               5                   10                  15
   Lys Asn Thr Pro Gly Ser Tyr Tyr Cys Thr Cys Pro Val Gly Phe Val
               20                  25                  30
   Leu Leu Pro Asp Gly Lys Arg Cys His
           35                  40
<210> 1053
   <211> 41
   <212> PRT
   <213> Homo sapiens
<400> 1053
   Gln Leu Val Ser Cys Pro Arg Asn Val Ser Glu Cys Ser His Asp Cys
   1               5                   10                  15
   Val Leu Thr Ser Glu Gly Pro Leu Cys Phe Cys Pro Glu Gly Ser Val
               20                  25                  30
   Leu Glu Arg Asp Gly Lys Thr Cys Ser
           35                  40
<210> 1054
   <211> 43
   <212> PRT
   <213> Homo sapiens
<400> 1054
   Thr Cys Ser Gly Cys Ser Ser Pro Asp Asn Gly Gly Cys Ser Gln Leu
   1               5                   10                  15
   Cys Val Pro Leu Ser Pro Val Ser Trp Glu Cys Asp Cys Phe Pro Gly
               20                  25                  30
   Tyr Asp Leu Gln Leu Asp Glu Lys Ser Cys Ala
           35                  40
<210> 1055
   <211> 41
   <212> PRT
   <213> Homo sapiens
<400> 1055
   Gly Ala Asp Pro Cys Leu Tyr Gln Asn Gly Gly Cys Glu His Ile Cys
   1               5                   10                  15
   Lys Lys Arg Leu Gly Thr Ala Trp Cys Ser Cys Arg Glu Gly Phe Met
               20                  25                  30
   Lys Ala Ser Asp Gly Lys Thr Cys Leu
           35                  40
<210> 1056
   <211> 39
   <212> PRT
   <213> Homo sapiens
<400> 1056
   Asp Gln Asp Asp Cys Ala Pro Val Gly Cys Ser Met Tyr Ala Arg Cys
   1               5                   10                  15
   Ile Ser Glu Gly Glu Asp Ala Thr Cys Gln Cys Leu Lys Gly Phe Ala
               20                  25                  30
   Gly Asp Gly Lys Leu Cys Ser
           35
<210> 1057
   <211> 42
   <212> PRT
   <213> Homo sapiens
<400> 1057
   Asp Ile Asp Glu Cys Glu Met Gly Val Pro Val Cys Pro Pro Ala Ser
   1               5                   10                  15
   Ser Lys Cys Ile Asn Thr Glu Gly Gly Tyr Val Cys Arg Cys Ser Glu
               20                  25                  30
   Gly Tyr Gln Gly Asp Gly Ile His Cys Leu
           35                  40
<210> 1058
   <211> 41
   <212> PRT
   <213> Homo sapiens
<400> 1058
   Asp Ile Asp Glu Cys Gln Leu Gly Glu His Ser Cys Gly Glu Asn Ala
   1               5                   10                  15
   Ser Cys Thr Asn Thr Glu Gly Gly Tyr Thr Cys Met Cys Ala Gly Arg
               20                  25                  30
   Leu Ser Glu Pro Gly Leu Ile Cys Pro
           35                  40
<210> 1059
   <211> 42
   <212> PRT
   <213> Homo sapiens
<400> 1059
   Ser Asp Ser Glu Cys Pro Leu Ser His Asp Gly Tyr Cys Leu His Asp
   1               5                   10                  15
   Gly Val Cys Met Tyr Ile Glu Ala Leu Asp Lys Tyr Ala Cys Asn Cys
               20                  25                  30
   Val Val Gly Tyr Ile Gly Glu Arg Cys Gln
           35                  40
<210> 1060
   <211> 41
   <212> PRT
   <213> Homo sapiens
<400> 1060
   Ser Ile Thr Lys Cys Ser Ser Asp Met Asn Gly Tyr Cys Leu His Gly
   1               5                   10                  15
   Gln Cys Ile Tyr Leu Val Asp Met Ser Gln Asn Tyr Cys Arg Cys Glu
               20                  25                  30
   Val Gly Tyr Thr Gly Val Arg Cys Glu
           35                  40
<210> 1061
   <211> 41
   <212> PRT
   <213> Homo sapiens
<400> 1061
   Lys Arg Asp Pro Cys Leu Arg Lys Tyr Lys Asp Phe Cys Ile His Gly
   1               5                   10                  15
   Glu Cys Lys Tyr Val Lys Glu Leu Arg Ala Pro Ser Cys Ile Cys His
               20                  25                  30
   Pro Gly Tyr His Gly Glu Arg Cys His
           35                  40
<210> 1062
   <211> 39
   <212> PRT
   <213> Homo sapiens
<400> 1062
   Leu Gln Gly Asn Cys Ser Arg Leu Gly Cys Gln His His Cys Val Pro
   1               5                   10                  15
   Thr Leu Asp Gly Pro Thr Cys Tyr Cys Asn Ser Ser Phe Gln Leu Gln
               20                  25                  30
   Ala Asp Gly Lys Thr Cys Lys
           35
<210> 1063
   <211> 41
   <212> PRT
   <213> Homo sapiens
<400> 1063
   Glu Leu Ser Pro Cys Arg Ile Asn Asn Gly Gly Cys Gln Asp Leu Cys
   1               5                   10                  15
   Leu Leu Thr His Gln Gly His Val Asn Cys Ser Cys Arg Gly Gly Arg
               20                  25                  30
   Ile Leu Gln Asp Asp Leu Thr Cys Arg
           35                  40
<210> 1064
   <211> 41
   <212> PRT
   <213> Homo sapiens
<400> 1064
   Ile Asn Glu Cys Leu Ser Arg Lys Leu Ser Gly Cys Ser Gln Asp Cys
   1               5                   10                  15
   Glu Asp Leu Lys Ile Gly Phe Lys Cys Arg Cys Arg Pro Gly Phe Arg
               20                  25                  30
   Leu Lys Asp Asp Gly Arg Thr Cys Ala
           35                  40
<210> 1065
   <211> 41
   <212> PRT
   <213> Homo sapiens
<400> 1065
   Asp Val Asp Glu Cys Ser Thr Thr Phe Pro Cys Ser Gln Arg Cys Ile
   1               5                   10                  15
   Asn Thr His Gly Ser Tyr Lys Cys Leu Cys Val Glu Gly Tyr Ala Pro
               20                  25                  30
   Arg Gly Gly Asp Pro His Ser Cys Lys
           35                  40
<210> 1066
   <211> 42
   <212> PRT
   <213> Homo sapiens
<400> 1066
   Pro Asn His Pro Cys Lys Val Asn Asn Gly Gly Cys Ser Asn Leu Cys
   1               5                   10                  15
   Leu Leu Ser Pro Gly Gly Gly His Lys Cys Ala Cys Pro Thr Asn Phe
               20                  25                  30
   Tyr Leu Gly Ser Asp Gly Arg Thr Cys Val
           35                  40
<210> 1067
   <211> 43
   <212> PRT
   <213> Homo sapiens
<400> 1067
   Lys Leu Thr Ser Cys Ala Thr Asn Ala Ser Ile Cys Gly Asp Glu Ala
   1               5                   10                  15
   Arg Cys Val Arg Thr Glu Lys Ala Ala Tyr Cys Ala Cys Arg Ser Gly
               20                  25                  30
   Phe His Thr Val Pro Gly Gln Pro Gly Cys Gln
           35                  40
<210> 1068
   <211> 38
   <212> PRT
   <213> Homo sapiens
<400> 1068
   Asp Ile Asn Glu Cys Leu Arg Phe Gly Thr Cys Ser Gln Leu Cys Asn
   1               5                   10                  15
   Asn Thr Lys Gly Gly His Leu Cys Ser Cys Ala Arg Asn Phe Met Lys
               20                  25                  30
   Thr His Asn Thr Cys Lys
           35
<210> 1069
   <211> 37
   <212> PRT
   <213> Homo sapiens
<400> 1069
   Val Thr Asn Pro Cys Asp Arg Lys Lys Cys Glu Trp Leu Cys Leu Leu
   1               5                   10                  15
   Ser Pro Ser Gly Pro Val Cys Thr Cys Pro Asn Gly Lys Arg Leu Asp
               20                  25                  30
   Asn Gly Thr Cys Val
           35
<210> 1070
   <211> 37
   <212> PRT
   <213> Homo sapiens
<400> 1070
   Arg Pro Gly Thr Cys Asn Leu Gln Cys Phe Asn Gly Gly Ser Cys Phe
   1               5                   10                  15
   Leu Asn Ala Arg Arg Gln Pro Lys Cys Arg Cys Gln Pro Arg Tyr Thr
               20                  25                  30
   Gly Asp Lys Cys Glu
           35
<210> 1071
   <211> 37
   <212> PRT
   <213> Homo sapiens
<400> 1071
   Glu Leu Asp Gln Cys Trp Glu His Cys Arg Asn Gly Gly Thr Cys Ala
   1               5                   10                  15
   Ala Ser Pro Ser Gly Met Pro Thr Cys Arg Cys Pro Thr Gly Phe Thr
               20                  25                  30
   Gly Pro Lys Cys Thr
           35
<210> 1072
   <211> 37
   <212> PRT
   <213> Homo sapiens
<400> 1072
   Thr Gln Gln Val Cys Ala Gly Tyr Cys Ala Asn Asn Ser Thr Cys Thr
   1               5                   10                  15
   Val Asn Gln Gly Asn Gln Pro Gln Cys Arg Cys Leu Pro Gly Phe Leu
               20                  25                  30
   Gly Asp Arg Cys Gln
           35
<210> 1073
   <211> 40
   <212> PRT
   <213> Homo sapiens
<400> 1073
   Asp Phe Asp Glu Cys Ser Val Tyr Gly Thr Cys Ser Gln Leu Cys Thr
   1               5                   10                  15
   Asn Thr Asp Gly Ser Phe Ile Cys Gly Cys Val Glu Gly Tyr Leu Leu
               20                  25                  30
   Gln Pro Asp Asn Arg Ser Cys Lys
                       35                  40
<210> 1074
   <211> 37
   <212> PRT
   <213> Homo sapiens
<400> 1074
   Gln Tyr Arg Gln Cys Ser Gly Tyr Cys Glu Asn Phe Gly Thr Cys Gln
   1               5                   10                  15
   Met Ala Ala Asp Gly Ser Arg Gln Cys Arg Cys Thr Ala Tyr Phe Glu
               20                  25                  30
   Gly Ser Arg Cys Glu
           35
<210> 1075
   <211> 36
   <212> PRT
   <213> Homo sapiens
<400> 1075
   Glu Val Asn Lys Cys Ser Arg Cys Leu Glu Gly Ala Cys Val Val Asn
   1               5                   10                  15
   Lys Gln Ser Gly Asp Val Thr Cys Asn Cys Thr Asp Gly Arg Val Ala
               20                  25                  30
   Pro Ser Cys Leu
           35
<210> 1076
   <211> 37
   <212> PRT
   <213> Homo sapiens
<400> 1076
   Ser Cys Leu Thr Cys Val Gly His Cys Ser Asn Gly Gly Ser Cys Thr
   1               5                   10                  15
   Met Asn Ser Lys Met Met Pro Glu Cys Gln Cys Pro Pro His Met Thr
               20                  25                  30
   Gly Pro Arg Cys Glu
           35
<210> 1077
   <211> 47
   <212> PRT
   <213> Homo sapiens
<400> 1077
   Arg Ser His Ala Cys Glu Asn Asp Gln Tyr Gly Lys Pro Gly Gly Cys
   1               5                   10                  15
   Ser Asp Ile Cys Leu Leu Ala Asn Ser His Lys Ala Arg Thr Cys Arg
               20                  25                  30
   Cys Arg Ser Gly Phe Ser Leu Gly Ser Asp Gly Lys Ser Cys Lys
           35                  40                  45
<210> 1078
   <211> 41
   <212> PRT
   <213> Homo sapiens
<400> 1078
   Gly Thr Asn Lys Cys Arg Val Asn Asn Gly Gly Cys Ser Ser Leu Cys
   1               5                   10                  15
   Leu Ala Thr Pro Gly Ser Arg Gln Cys Ala Cys Ala Glu Asp Gln Val
                    20                  25                  30
   Leu Asp Ala Asp Gly Val Thr Cys Leu
           35                  40
<210> 1079
   <211> 40
   <212> PRT
   <213> Homo sapiens
<400> 1079
   Asp Gln Cys Ser Leu Asn Asn Gly Gly Cys Ser His Asn Cys Ser Val
   1               5                   10                  15
   Ala Pro Gly Glu Gly Ile Val Cys Ser Cys Pro Leu Gly Met Glu Leu
               20                  25                  30
   Gly Pro Asp Asn His Thr Cys Gln
           35                  40
<210> 1080
   <211> 40
   <212> PRT
   <213> Homo sapiens
<400> 1080
   Ile Gln Ser Tyr Cys Ala Lys His Leu Lys Cys Ser Gln Lys Cys Asp
   1               5                   10                  15
   Gln Asn Lys Phe Ser Val Lys Cys Ser Cys Tyr Glu Gly Trp Val Leu
               20                  25                  30
   Glu Pro Asp Gly Glu Ser Cys Arg
           35                  40
<210> 1081
   <211> 44
   <212> PRT
   <213> Homo sapiens
<400> 1081
   Ala Pro Asn Pro Cys Glu Ala Asn Gly Gly Gln Gly Pro Cys Ser His
   1               5                   10                  15
   Leu Cys Leu Ile Asn Tyr Asn Arg Thr Val Ser Cys Ala Cys Pro His
               20                  25                  30
   Leu Met Lys Leu His Lys Asp Asn Thr Thr Cys Tyr
           35                  40
<210> 1082
   <211> 42
   <212> PRT
   <213> Homo sapiens
<400> 1082
   Gly Thr Asn Pro Cys Ser Val Asn Asn Gly Asp Cys Ser Gln Leu Cys
   1               5                   10                  15
   Leu Pro Thr Ser Glu Thr Thr Arg Ser Cys Met Cys Thr Ala Gly Tyr
               20                  25                  30
   Ser Leu Arg Ser Gly Gln Gln Ala Cys Glu
           35                  40
<210> 1083
   <211> 41
   <212> PRT
   <213> Homo sapiens
<400> 1083
   Gly Thr Asn Val Cys Ala Val Ala Asn Gly Gly Cys Gln Gln Leu Cys
   1               5                   10                  15
   Leu Tyr Arg Gly Arg Gly Gln Arg Ala Cys Ala Cys Ala His Gly Met
               20                  25                  30
   Leu Ala Glu Asp Gly Ala Ser Cys Arg
           35                  40
<210> 1084
   <211> 45
   <212> PRT
   <213> Homo sapiens
<400> 1084
   His Leu Val Lys Cys Ala Glu Lys Glu Lys Thr Phe Cys Val Asn Gly
   1               5                   10                  15
   Gly Glu Cys Phe Met Val Lys Asp Leu Ser Asn Pro Ser Arg Tyr Leu
               20                  25                  30
   Cys Lys Cys Gln Pro Gly Phe Thr Gly Ala Arg Cys Thr
           35                  40                  45
<210> 1085
   <211> 42
   <212> PRT
   <213> Homo sapiens
<400> 1085
   His Ala Arg Lys Cys Asn Glu Thr Ala Lys Ser Tyr Cys Val Asn Gly
   1               5                   10                  15
   Gly Val Cys Tyr Tyr Ile Glu Gly Ile Asn Gln Leu Ser Cys Lys Cys
               20                  25                  30
   Pro Asn Gly Phe Phe Gly Gln Arg Cys Leu
           35                  40
<210> 1086
   <211> 44
   <212> PRT
   <213> Homo sapiens
<400> 1086
   His Phe Lys Pro Cys Arg Asp Lys Asp Leu Ala Tyr Cys Leu Asn Asp
   1               5                   10                  15
   Gly Glu Cys Phe Val Ile Glu Thr Leu Thr Gly Ser His Lys His Cys
               20                  25                  30
   Arg Cys Lys Glu Gly Tyr Gln Gly Val Arg Cys Asp
           35                  40
<210> 1087
   <211> 42
   <212> PRT
   <213> Homo sapiens
<400> 1087
   His Glu Glu Pro Cys Gly Pro Ser His Lys Ser Phe Cys Leu Asn Gly
   1               5                   10                  15
   Gly Leu Cys Tyr Val Ile Pro Thr Ile Pro Ser Pro Phe Cys Arg Cys
               20                  25                  30
   Val Glu Asn Tyr Thr Gly Ala Arg Cys Glu
           35                  40
<210> 1088
   <211> 39
   <212> PRT
   <213> Homo sapiens
<400> 1088
   Pro Val Asn Pro Cys Cys Tyr Tyr Pro Cys Gln His Gln Gly Ile Cys
   1               5                   10                  15
   Val Arg Phe Gly Leu Asp Arg Tyr Gln Cys Asp Cys Thr Arg Thr Gly
               20                  25                  30
   Tyr Ser Gly Pro Asn Cys Thr
           35
<210> 1089
   <211> 38
   <212> PRT
   <213> Homo sapiens
<400> 1089
   Ala Asn Pro Cys Cys Ser His Pro Cys Gln Asn Arg Gly Val Cys Met
   1               5                   10                  15
   Ser Val Gly Phe Asp Gln Tyr Lys Cys Asp Cys Thr Arg Thr Gly Phe
               20                  25                  30
   Tyr Gly Glu Asn Cys Ser
           35
<210> 1090
   <211> 39
   <212> PRT
   <213> Homo sapiens
<400> 1090
   Asn Asn Thr Arg Cys Leu Ser Asn Ser Cys Gln Asn Asn Ser Thr Cys
   1               5                   10                  15
   Lys Asp Phe Ser Lys Asp Asn Asp Cys Ser Cys Ser Asp Thr Ala Asn
               20                  25                  30
   Asn Leu Asp Lys Asp Cys Asp
           35
<210> 1091
   <211> 43
   <212> PRT
   <213> Homo sapiens
<400> 1091
   Asp Val Asn Glu Cys Ser Ser Asn Pro Cys Gln Asn Gly Gly Thr Cys
   1               5                   10                  15
   Glu Asn Leu Pro Gly Asn Tyr Thr Cys His Cys Pro Phe Asp Asn Leu
               20                  25                  30
   Ser Arg Thr Phe Tyr Gly Gly Arg Asp Cys Ser
           35                  40
<210> 1092
   <211> 41
   <212> PRT
   <213> Homo sapiens
<400> 1092
   Ile Leu Leu Gly Cys Thr His Gln Gln Cys Leu Asn Asn Gly Thr Cys
   1               5                   10                  15
   Ile Pro His Phe Gln Asp Gly Gln His Gly Phe Ser Cys Leu Cys Pro
               20                  25                  30
   Ser Gly Tyr Thr Gly Ser Leu Cys Glu
           35                  40
<210> 1093
   <211> 37
   <212> PRT
   <213> Homo sapiens
<400> 1093
   Arg Lys Asp Trp Cys Glu Ser Gln Pro Cys Gln Ser Arg Gly Arg Cys
   1               5                   10                  15
   Ile Asn Leu Trp Leu Ser Tyr Gln Cys Asp Cys His Arg Pro Tyr Glu
               20                  25                  30
   Gly Pro Asn Cys Leu
           35
<210> 1094
   <211> 37
   <212> PRT
   <213> Homo sapiens
<400> 1094
   Gly Asp Asn Ser Cys Lys Ser Asn Pro Cys His Asn Gly Gly Val Cys
   1               5                   10                  15
   His Ser Arg Trp Asp Asp Phe Ser Cys Ser Cys Pro Ala Leu Thr Ser
               20                  25                  30
   Gly Lys Ala Cys Glu
           35
<210> 1095
   <211> 37
   <212> PRT
   <213> Homo sapiens
<400> 1095
   Glu Val Gln Trp Cys Gly Phe Ser Pro Cys Pro His Gly Ala Gln Cys
   1               5                   10                  15
   Gln Pro Val Leu Gln Gly Phe Glu Cys Ile Ala Asn Ala Val Phe Asn
               20                  25                  30
   Gly Gln Ser Gly Gln
           35
<210> 1096
   <211> 37
   <212> PRT
   <213> Homo sapiens
<400> 1096
   Gln Leu Asn Val Cys Asn Ser Asn Pro Cys Leu His Gly Gly Asn Cys
   1               5                   10                  15
   Glu Asp Ile Tyr Ser Ser Tyr His Cys Ser Cys Pro Leu Gly Trp Ser
               20                  25                  30
   Gly Lys His Cys Glu
           35
<210> 1097
   <211> 36
   <212> PRT
   <213> Homo sapiens
<400> 1097
   Asn Ile Asp Glu Cys Phe Ser Asn Pro Cys Ile His Gly Asn Cys Ser
   1               5                   10                  15
   Asp Arg Val Ala Ala Tyr His Cys Thr Cys Glu Pro Gly Tyr Thr Gly
               20                  25                  30
   Val Asn Cys Glu
           35
<210> 1098
   <211> 37
   <212> PRT
   <213> Homo sapiens
<400> 1098
   Asp Ile Asp Asn Cys Gln Ser His Gln Cys Ala Asn Gly Ala Thr Cys
   1               5                   10                  15
   Ile Ser His Thr Asn Gly Tyr Ser Cys Leu Cys Phe Gly Asn Phe Thr
               20                  25                  30
   Gly Lys Phe Cys Arg
           35
<210> 1099
   <211> 41
   <212> PRT
   <213> Homo sapiens
<400> 1099
   Pro Ser Thr Val Cys Gly Asn Glu Lys Thr Asn Leu Thr Cys Tyr Asn
   1               5                   10                  15
   Gly Gly Asn Cys Thr Glu Phe Gln Thr Glu Leu Lys Cys Met Cys Arg
               20                  25                  30
   Pro Gly Phe Thr Gly Glu Trp Cys Glu
           35                  40
<210> 1100
   <211> 37
   <212> PRT
   <213> Homo sapiens
<400> 1100
   Asp Ile Asp Glu Cys Ala Ser Asp Pro Cys Val Asn Gly Gly Leu Cys
   1               5                   10                  15
   Gln Asp Leu Leu Asn Lys Phe Gln Cys Leu Cys Asp Val Ala Phe Ala
               20                  25                  30
   Gly Glu Arg Cys Glu
           35
<210> 1101
   <211> 39
   <212> PRT
   <213> Homo sapiens
<400> 1101
   Met Lys Asp Pro Cys Phe Ser Asn Pro Cys Gln Gly Ser Ala Thr Cys
   1 5 10 15
   Val Asn Thr Pro Gly Glu Arg Ser Phe Leu Cys Lys Cys Pro Pro Gly
   20 25 30
   Tyr Ser Gly Thr Ile Cys Glu
   35
<210> 1102
   <211> 37
   <212> PRT
   <213> Homo sapiens
<400> 1102
   Thr Ile Gly Ser Cys Gly Lys Asn Ser Cys Gln His Gly Gly Ile Cys
   1 5 10 15
   His Gln Asp Pro Ile Tyr Pro Val Cys Ile Cys Pro Ala Gly Tyr Ala
   20 25 30
   Gly Arg Phe Cys Glu
   35
<210> 1103
   <211> 37
   <212> PRT
   <213> Homo sapiens
<400> 1103
   Asp His Asp Glu Cys Ala Ser Ser Pro Cys Gln Asn Gly Ala Val Cys
   1 5 10 15
   Gln Asp Gly Ile Asp Gly Tyr Ser Cys Phe Cys Val Pro Gly Tyr Gln
   20 25 30
   Gly Arg His Cys Asp
   35
<210> 1104
   <211> 37
   <212> PRT
   <213> Homo sapiens
<400> 1104
   Glu Val Asp Glu Cys Ala Ser Asp Pro Cys Lys Asn Glu Ala Thr Cys
   1 5 10 15
   Leu Asn Glu Ile Gly Arg Tyr Thr Cys Ile Cys Pro His Asn Tyr Ser
   20 25 30
   Gly Val Asn Cys Glu
   35
<210> 1105
   <211> 37
   <212> PRT
   <213> Homo sapiens
<400> 1105
   Glu Ile Asp Glu Cys Trp Ser Gln Pro Cys Leu Asn Gly Ala Thr Cys
   1               5                   10                  15
   Gln Asp Ala Leu Gly Ala Tyr Phe Cys Asp Cys Ala Pro Gly Phe Leu
               20                  25                  30
   Gly Asp His Cys Glu
           35
<210> 1106
   <211> 38
   <212> PRT
   <213> Homo sapiens
<400> 1106
   Asn Thr Asp Glu Cys Ala Ser Gln Pro Cys Leu His Gly Gly Leu Cys
   1               5                   10                  15
   Val Asp Gly Glu Asn Arg Tyr Ser Cys Asn Cys Thr Gly Ser Gly Phe
               20                  25                  30
   Thr Gly Thr His Cys Glu
           35
<210> 1107
   <211> 37
   <212> PRT
   <213> Homo sapiens
<400> 1107
   Leu Met Pro Leu Cys Trp Ser Lys Pro Cys His Asn Asn Ala Thr Cys
   1               5                   10                  15
   Glu Asp Ser Val Asp Asn Tyr Thr Cys His Cys Trp Pro Gly Tyr Thr
               20                  25                  30
   Gly Ala Gln Cys Glu
           35
<210> 1108
   <211> 57
   <212> PRT
   <213> Homo sapiens
<400> 1108
   Asp Leu Asn Glu Cys Asn Ser Asn Pro Cys Gln Ser Asn Gly Glu Cys
   1               5                   10                  15
   Val Glu Leu Ser Ser Glu Lys Gln Tyr Gly Arg Ile Thr Gly Leu Pro
               20                  25                  30
   Ser Ser Phe Ser Tyr His Glu Ala Ser Gly Tyr Val Cys Ile Cys Gln
           35                  40                  45
   Pro Gly Phe Thr Gly Ile His Cys Glu
       50                  55
<210> 1109
   <211> 40
   <212> PRT
   <213> Homo sapiens
<400> 1109
   Glu Pro Arg Gly Cys Ala Thr Gln Pro Cys His His Gly Ala Leu Cys
   1               5                   10                  15
   Val Pro Gln Gly Pro Asp Pro Thr Gly Phe Arg Cys Tyr Cys Val Pro
               20                  25                  30
   Gly Phe Gln Gly Pro Arg Cys Glu
           35                  40
<210> 1110
   <211> 37
   <212> PRT
   <213> Homo sapiens
<400> 1110
   Arg Arg Glu Gln Cys Arg Pro Leu Pro Cys Val His Gly Gly Ser Cys
   1               5                   10                  15
   Val Asp Leu Trp Thr His Phe Arg Cys Asp Cys Ala Arg Pro His Arg
               20                  25                  30
   Gly Pro Thr Cys Ala
           35
<210> 1111
   <211> 37
   <212> PRT
   <213> Homo sapiens
<400> 1111
   Ser Glu Asp Met Cys Ser Pro Asp Pro Cys Phe Asn Gly Gly Thr Cys
   1               5                   10                  15
   Leu Val Thr Trp Asn Asp Phe His Cys Thr Cys Pro Ala Asn Phe Thr
               20                  25                  30
   Gly Pro Thr Cys Ala
           35
<210> 1112
   <211> 37
   <212> PRT
   <213> Homo sapiens
<400> 1112
   Gly Ala Pro Val Cys Ala Pro Ser Pro Cys Leu His Asp Gly Ala Cys
   1               5                   10                  15
   Arg Asp Leu Phe Asp Ala Phe Ala Cys Ala Cys Gly Pro Gly Trp Glu
               20                  25                  30
   Gly Pro Arg Cys Glu
           35
<210> 1113
   <211> 37
   <212> PRT
   <213> Homo sapiens
<400> 1113
   His Val Asp Pro Cys His Ser Ala Pro Cys Ala Arg Gly Arg Cys His
   1               5                   10                  15
   Thr His Pro Asp Gly Arg Phe Glu Cys Arg Cys Pro Pro Gly Phe Gly
               20                  25                  30
   Gly Pro Arg Cys Arg
           35
<210> 1114
   <211> 38
   <212> PRT
   <213> Homo sapiens
<400> 1114
   Pro Ser Lys Glu Cys Ser Leu Asn Val Thr Cys Leu Asp Gly Ser Pro
   1               5                   10                  15
   Cys Glu Gly Gly Ser Pro Ala Ala Asn Cys Ser Cys Leu Glu Gly Leu
               20                  25                  30
   Ala Gly Gln Arg Cys Gln
           35
<210> 1115
   <211> 37
   <212> PRT
   <213> Homo sapiens
<400> 1115
   Pro Thr Leu Pro Cys Glu Ala Asn Pro Cys Leu Asn Gly Gly Thr Cys
   1               5                   10                  15
   Arg Ala Ala Gly Gly Val Ser Glu Cys Ile Cys Asn Ala Arg Phe Ser
               20                  25                  30
   Gly Gln Phe Cys Glu
           35
<210> 1116
   <211> 37
   <212> PRT
   <213> Homo sapiens
<400> 1116
   Asp Ile Asp Glu Cys Ala Ser Arg Pro Cys His His Gly Ala Thr Cys
   1               5                   10                  15
   Arg Asn Leu Ala Asp Arg Tyr Glu Cys His Cys Pro Leu Gly Tyr Ala
               20                  25                  30
   Gly Val Thr Cys Glu
           35
<210> 1117
   <211> 37
   <212> PRT
   <213> Homo sapiens
<400> 1117
   Glu Val Asp Glu Cys Ala Ser Ala Pro Cys Leu His Gly Gly Ser Cys
   1               5                   10                  15
   Leu Asp Gly Val Gly Ser Phe Arg Cys Val Cys Ala Pro Gly Tyr Gly
               20                  25                  30
   Gly Thr Arg Cys Gln
           35
<210> 1118
   <211> 38
   <212> PRT
   <213> Homo sapiens
<400> 1118
   Asp Leu Asp Glu Cys Gln Ser Gln Pro Cys Ala His Gly Gly Thr Cys
   1               5                   10                  15
   His Asp Leu Val Asn Gly Phe Arg Cys Asp Cys Ala Gly Thr Gly Tyr
               20                  25                  30
   Glu Gly Thr His Cys Glu
           35
<210> 1119
   <211> 37
   <212> PRT
   <213> Homo sapiens
<400> 1119
   Glu Val Leu Glu Cys Ala Ser Ala Pro Cys Glu His Asn Ala Ser Cys
   1               5                   10                  15
   Leu Glu Gly Leu Gly Ser Phe Arg Cys Leu Cys Trp Pro Gly Tyr Ser
               20                  25                  30
   Gly Glu Leu Cys Glu
           35
<210> 1120
   <211> 58
   <212> PRT
   <213> Homo sapiens
<400> 1120
   Asp Glu Asp Glu Cys Ala Ser Ser Pro Cys Gln His Gly Gly Arg Cys
   1               5                   10                  15
   Leu Gln Arg Ser Asp Pro Ala Leu Tyr Gly Gly Val Gln Ala Ala Phe
               20                  25                  30
   Pro Gly Ala Phe Ser Phe Arg His Ala Ala Gly Phe Leu Cys His Cys
           35                  40                  45
   Pro Pro Gly Phe Glu Gly Ala Asp Cys Gly
       50                  55
<210> 1121
   <211> 37
   <212> PRT
   <213> Homo sapiens
<400> 1121
   Glu Val Asp Glu Cys Ala Ser Arg Pro Cys Leu Asn Gly Gly His Cys
   1 5 10 15
   Gln Asp Leu Pro Asn Gly Phe Gln Cys His Cys Pro Asp Gly Tyr Ala
   20 25 30
   Gly Pro Thr Cys Glu
   35
<210> 1122
   <211> 37
   <212> PRT
   <213> Homo sapiens
<400> 1122
   Asp Val Asp Glu Cys Leu Ser Asp Pro Cys Leu His Gly Gly Thr Cys
   1 5 10 15
   Ser Asp Thr Val Ala Gly Tyr Ile Cys Arg Cys Pro Glu Thr Trp Gly
   20 25 30
   Gly Arg Asp Cys Ser
   35
<210> 1123
   <211> 41
   <212> PRT
   <213> Homo sapiens
<400> 1123
   Gln Leu Thr Gly Cys Gln Gly His Thr Cys Pro Leu Ala Ala Thr Cys
   1 5 10 15
   Ile Pro Ile Phe Glu Ser Gly Val His Ser Tyr Val Cys His Cys Pro
   20 25 30
   Pro Gly Thr His Gly Pro Phe Cys Gly
   35 40
<210> 1124
   <211> 32
   <212> PRT
   <213> Homo sapiens
<400> 1124
   Ala Glu Cys Phe Pro Ala Cys Asn Pro Gln Asn Gly Phe Cys Glu Asp
   1 5 10 15
   Asp Asn Val Cys Arg Cys Gln Pro Gly Trp Gln Gly Pro Leu Cys Asp
   20 25 30
<210> 1125
   <211> 34
   <212> PRT
   <213> Homo sapiens
<400> 1125
   Leu Cys Asp Gln Cys Val Thr Ser Pro Gly Cys Leu His Gly Leu Cys
   1               5                   10                  15
   Gly Glu Pro Gly Gln Cys Ile Cys Thr Asp Gly Trp Asp Gly Glu Leu
               20                  25                  30
   Cys Asp
<210> 1126
   <211> 38
   <212> PRT
   <213> Homo sapiens
<400> 1126
   Asp Val Arg Ala Cys Ser Ser Ala Pro Cys Ala Asn Asn Arg Thr Cys
   1               5                   10                  15
   Val Ser Leu Asp Asp Gly Leu Tyr Glu Cys Ser Cys Ala Pro Gly Tyr
               20                  25                  30
   Ser Gly Lys Asp Cys Gln
           35
<210> 1127
   <211> 42
   <212> PRT
   <213> Homo sapiens
<400> 1127
   Lys Asp Gly Pro Cys Val Ile Asn Gly Ser Pro Cys Gln His Gly Gly
   1               5                   10                  15
   Thr Cys Val Asp Asp Glu Gly Arg Ala Ser His Ala Ser Cys Leu Cys
               20                  25                  30
   Pro Pro Gly Phe Ser Gly Asn Phe Cys Glu
           35                  40
<210> 1128
   <211> 37
   <212> PRT
   <213> Homo sapiens
<400> 1128
   Val Ala Asn Ser Cys Thr Pro Asn Pro Cys Glu Asn Asp Gly Val Cys
   1               5                   10                  15
   Thr Asp Ile Gly Gly Asp Phe Arg Cys Arg Cys Pro Ala Gly Phe Ile
               20                  25                  30
   Asp Lys Thr Cys Ser
           35
<210> 1129
   <211> 38
   <212> PRT
   <213> Homo sapiens
<400> 1129
   Pro Val Thr Asn Cys Ala Ser Ser Pro Cys Gln Asn Gly Gly Thr Cys
   1               5                   10                  15
   Leu Gln His Thr Gln Val Ser Tyr Glu Cys Leu Cys Lys Pro Glu Phe
               20                  25                  30
   Thr Gly Leu Thr Cys Val
           35
<210> 1130
   <211> 32
   <212> PRT
   <213> Homo sapiens
<400> 1130
   Asp Asp Cys Ser Ser His Cys Asp Leu Ala His Gly Cys Cys Ala Pro
   1               5                   10                  15
   Asp Gly Ser Cys Arg Cys Asp Pro Gly Trp Glu Gly Leu His Cys Glu
               20                  25                  30
<210> 1131
   <211> 28
   <212> PRT
   <213> Homo sapiens
<400> 1131
   Arg Met Pro Gly Cys Gln His Gly Thr Cys His Gln Pro Trp Gln Cys
   1               5                   10                  15
   Ile Cys His Ser Gly Trp Ala Gly Lys Phe Cys Asp
               20                  25
<210> 1132
   <211> 39
   <212> PRT
   <213> Homo sapiens
<400> 1132
   Asp Glu His Ile Cys Thr Thr Gln Ser Pro Cys Gln Asn Gly Gly Gln
   1               5                   10                  15
   Cys Met Tyr Asp Gly Gly Gly Glu Tyr His Cys Val Cys Leu Pro Gly
               20                  25                  30
   Phe His Gly Arg Asp Cys Glu
           35
<210> 1133
   <211> 42
   <212> PRT
   <213> Homo sapiens
<400> 1133
   Lys Ala Gly Pro Cys Glu Gln Ala Gly Ser Pro Cys Arg Asn Gly Gly
   1               5                   10                  15
   Gln Cys Gln Asp Asp Gln Gly Phe Ala Leu Asn Phe Thr Cys Arg Cys
               20                  25                  30
   Leu Val Gly Phe Val Gly Ala Arg Cys Glu
           35                  40
<210> 1134
   <211> 37
   <212> PRT
   <213> Homo sapiens
<400> 1134
   Asn Val Asp Asp Cys Leu Met Arg Pro Cys Ala Asn Gly Ala Thr Cys
   1               5                   10                  15
   Leu Asp Gly Ile Asn Arg Phe Ser Cys Leu Cys Pro Glu Gly Phe Ala
               20                  25                  30
   Gly Arg Phe Cys Thr
           35
<210> 1135
   <211> 37
   <212> PRT
   <213> Homo sapiens
<400> 1135
   Asn Leu Asp Asp Cys Ala Ser Arg Pro Cys Gln Arg Gly Ala Arg Cys
   1               5                   10                  15
   Arg Asp Arg Val His Asp Phe Asp Cys Leu Cys Pro Ser Gly Tyr Gly
               20                  25                  30
   Gly Lys Thr Cys Glu
           35
<210> 1136
   <211> 43
   <212> PRT
   <213> Homo sapiens
<400> 1136
   Lys Gln Gln Phe Cys Gln Glu Ser Leu Ser Ser Glu Phe Cys Ser Gly
   1               5                   10                  15
   His Gly Lys Cys Leu Ser Glu Ala Trp Ser Lys Thr Tyr Ser Cys His
               20                  25                  30
   Cys Gln Pro Pro Phe Ser Gly Lys Tyr Cys Gln
           35                  40
<210> 1137
   <211> 37
   <212> PRT
   <213> Homo sapiens
<400> 1137
   Glu Ser Asn Glu Cys Lys Met Asn Pro Cys Lys Asn Asn Ser Thr Cys
   1               5                   10                  15
   Thr Asp Leu Tyr Lys Ser Tyr Arg Cys Glu Cys Thr Ser Gly Trp Thr
               20                  25                  30
   Gly Gln Asn Cys Ser
           35
<210> 1138
   <211> 39
   <212> PRT
   <213> Homo sapiens
<400> 1138
   Glu Ile Asn Glu Cys Asp Ser Asp Pro Cys Met Asn Gly Gly Leu Cys
   1               5                   10                  15
   His Glu Ser Thr Ile Pro Gly Gln Phe Val Cys Leu Cys Pro Pro Leu
               20                  25                  30
   Tyr Thr Gly Gln Phe Cys His
           35
<210> 1139
   <211> 40
   <212> PRT
   <213> Homo sapiens
<400> 1139
   Arg Tyr Asn Leu Cys Asp Leu Leu His Asn Pro Cys Arg Asn Asn Ser
   1               5                   10                  15
   Thr Cys Leu Ala Leu Val Asp Ala Asn Gln His Cys Ile Cys Arg Glu
               20                  25                  30
   Glu Phe Glu Gly Lys Asn Cys Glu
           35                  40
<210> 1140
   <211> 37
   <212> PRT
   <213> Homo sapiens
<400> 1140
   Asp Val Lys Asp Cys Leu Phe Leu Ser Cys Gln Asp Tyr Gly Asp Cys
   1               5                   10                  15
   Glu Asp Met Val Asn Asn Phe Arg Cys Ile Cys Arg Pro Gly Phe Ser
               20                  25                  30
   Gly Ser Leu Cys Glu
           35
<210> 1141
   <211> 37
   <212> PRT
   <213> Homo sapiens
<400> 1141
   Glu Ile Asn Glu Cys Ser Ser Glu Pro Cys Lys Asn Asn Gly Thr Cys
   1               5                   10                  15
   Val Asp Leu Thr Asn Arg Phe Phe Cys Asn Cys Glu Pro Glu Tyr His
               20                  25                  30
   Gly Pro Phe Cys Glu
       35
<210> 1142
   <211> 37
   <212> PRT
   <213> Homo sapiens
<400> 1142
   Asp Val Asn Lys Cys Lys Ile Ser Pro Cys Leu Asp Glu Glu Asn Cys
   1               5                   10                  15
   Val Tyr Arg Thr Asp Gly Tyr Asn Cys Leu Cys Ala Pro Gly Tyr Thr
               20                  25                  30
   Gly Ile Asn Cys Glu
           35
<210> 1143
   <211> 37
   <212> PRT
   <213> Homo sapiens
<400> 1143
   Asn Leu Asp Glu Cys Leu Ser Glu Pro Cys Leu His Asp Gly Val Cys
   1               5                   10                  15
   Ile Asp Gly Ile Asn His Tyr Thr Cys Asp Cys Lys Ser Gly Phe Phe
               20                  25                  30
   Gly Thr His Cys Glu
           35
<210> 1144
   <211> 36
   <212> PRT
   <213> Homo sapiens
<400> 1144
   Asn Ala Asn Asp Cys Leu Ser Asn Pro Cys Leu His Gly Arg Cys Thr
   1               5                   10                  15
   Glu Leu Ile Asn Glu Tyr Pro Cys Ser Cys Asp Ala Asp Gly Thr Ser
               20                  25                  30
   Thr Gln Cys Lys
           35
<210> 1145
   <211> 37
   <212> PRT
   <213> Homo sapiens
<400> 1145
   Lys Ile Asn Asp Cys Thr Ser Ile Pro Cys Met Asn Glu Gly Phe Cys
   1               5                   10                  15
   Gln Lys Ser Ala His Gly Phe Thr Cys Ile Cys Pro Arg Gly Tyr Thr
               20                  25                  30
   Gly Ala Tyr Cys Glu
           35
<210> 1146
   <211> 43
   <212> PRT
   <213> Homo sapiens
<400> 1146
   Ser Ile Asp Asn Cys Ala Glu Pro Glu Leu Asn Ser Val Ile Cys Leu
   1               5                   10                  15
   Asn Gly Gly Ile Cys Val Asp Gly Pro Gly His Thr Phe Asp Cys Arg
               20                  25                  30
   Cys Leu Pro Gly Phe Ser Gly Gln Phe Cys Glu
           35                  40
<210> 1147
   <211> 42
   <212> PRT
   <213> Homo sapiens
<400> 1147
   Glu Leu Asp Ala Cys Ser Phe Lys Pro Cys Lys Asn Asn Gly Ser Cys
   1               5                   10                  15
   Ile Asn Lys Arg Glu Asn Trp Asp Glu Gln Ala Tyr Glu Cys Val Cys
               20                  25                  30
   His Pro Pro Phe Thr Gly Lys Asn Cys Ser
           35                  40
<210> 1148
   <211> 37
   <212> PRT
   <213> Homo sapiens
<400> 1148
   Asn Ile Asn Glu Cys Ser Ser Ser Pro Cys Leu His Gly Ala Asp Cys
   1               5                   10                  15
   Glu Asp His Ile Asn Gly Tyr Val Cys Lys Cys Gln Pro Gly Trp Ser
               20                  25                  30
   Gly His His Cys Glu
           35
<210> 1149
   <211> 42
   <212> PRT
   <213> Homo sapiens
<400> 1149
   Ala Pro Ser Val Cys Gln Gln Asp Val Cys His Asn Gly Gly Thr Cys
   1               5                   10                  15
   His Ala Ile Phe Leu Ser Ser Gly Ile Val Ser Phe Gln Cys Asp Cys
               20                  25                  30
   Pro Leu His Phe Thr Gly Arg Phe Cys Glu
           35                  40
<210> 1150
   <211> 34
   <212> PRT
   <213> Homo sapiens
<400> 1150
   His Val Pro Trp Cys Ala His His Leu Cys Arg Asn Asn Gly Thr Cys
   1 5 10 15
   Ile Ser Asp Asn Glu Asn Leu Phe Cys Glu Cys Pro Arg Leu Tyr Ser
   20 25 30
   Gly Lys
<210> 1151
   <211> 38
   <212> PRT
   <213> Homo sapiens
<400> 1151
   Gln Phe Ala Ser Cys Glu Asn Asn Pro Cys Gly Asn Gly Ala Thr Cys
   1 5 10 15
   Val Pro Lys Ser Gly Thr Asp Ile Val Cys Leu Cys Pro Tyr Gly Arg
   20 25 30
   Ser Gly Pro Leu Cys Thr
   35
<210> 1152
   <211> 37
   <212> PRT
   <213> Homo sapiens
<400> 1152
   His Ala Ser Pro Cys Ser Leu Met Lys Cys Gly Asn Gly Gly Thr Cys
   1 5 10 15
   Ile Glu Ser Gly Thr Ser Val Tyr Cys Asn Cys Thr Thr Gly Trp Lys
   20 25 30
   Gly Ser Phe Cys Thr
   35
<210> 1153
   <211> 42
   <212> PRT
   <213> Homo sapiens
<400> 1153
   Thr Val Ser Thr Cys Asp Pro Glu His Asp Pro Pro His His Cys Ser
   1 5 10 15
   Arg Gly Ala Thr Cys Ile Ser Leu Pro His Gly Tyr Thr Cys Phe Cys
   20 25 30
   Pro Leu Gly Thr Thr Gly Ile Tyr Cys Glu
   35 40
<210> 1154
   <211> 37
   <212> PRT
   <213> Homo sapiens
<400> 1154
   Asp Gly Thr Ala Cys Gly Tyr Asn Thr Cys Arg Asn Gly Gly Glu Cys
   1               5                   10                  15
   Thr Val Asn Gly Thr Thr Phe Ser Cys Arg Cys Leu Pro Asp Trp Ala
               20                  25                  30
   Gly Asn Thr Cys Asn
           35
<210> 1155
   <211> 38
   <212> PRT
   <213> Homo sapiens
<400> 1155
   Gln Ser Val Ser Cys Leu Asn Asn Leu Cys Leu His Gln Ser Leu Cys
   1               5                   10                  15
   Ile Pro Asp Gln Ser Phe Ser Tyr Ser Cys Leu Cys Thr Leu Gly Trp
               20                  25                  30
   Val Gly Arg Tyr Cys Glu
           35
<210> 1156
   <211> 37
   <212> PRT
   <213> Homo sapiens
<400> 1156
   Ile Ile Gly Gln Cys Gln Pro His Val Cys Phe His Gly Asn Cys Ser
   1               5                   10                  15
   Asn Ile Thr Ser Asn Ser Phe Ile Cys Glu Cys Asp Glu Gln Phe Ser
               20                  25                  30
   Gly Pro Phe Cys Glu
           35
<210> 1157
   <211> 37
   <212> PRT
   <213> Homo sapiens
<400> 1157
   Asp Val Ser Glu Phe Ser Leu Val Pro Cys Gln Asn Gly Thr Asp Cys
   1               5                   10                  15
   Ile Lys Ile Ser Asn Asp Val Met Cys Ile Cys Ser Pro Ile Phe Thr
               20                  25                  30
   Asp Leu Leu Cys Lys
           35
<210> 1158
   <211> 37
   <212> PRT
   <213> Homo sapiens
<400> 1158
   Ile Gln Thr Ser Cys Glu Ser Phe Pro Leu Arg Asn Asn Ala Thr Cys
   1               5                   10                  15
   Lys Lys Cys Glu Lys Asp Tyr Pro Cys Ser Cys Ile Ser Gly Phe Thr
               20                  25                  30
   Glu Lys Asn Cys Glu
           35
<210> 1159
   <211> 36
   <212> PRT
   <213> Homo sapiens
<400> 1159
   Thr Thr Asp Asp Gln Glu Asn Glu Cys Gln His Glu Ala Val Cys Lys
   1               5                   10                  15
   Asp Glu Ile Asn Arg Pro Arg Cys Ser Cys Ser Leu Ser Tyr Ile Gly
               20                  25                  30
   Arg Leu Cys Val
           35
<210> 1160
   <211> 37
   <212> PRT
   <213> Homo sapiens
<400> 1160
   Asp Thr Glu Asp Cys Lys Ser Ala Ser Cys Lys Asn Gly Thr Thr Ser
   1               5                   10                  15
   Thr His Leu Arg Gly Tyr Phe Phe Arg Lys Cys Val Pro Gly Phe Lys
               20                  25                  30
   Gly Thr Gln Cys Glu
           35
<210> 1161
   <211> 40
   <212> PRT
   <213> Homo sapiens
<400> 1161
   Asp Ile Asp Glu Cys Ala Ser His Pro Cys Lys Asn Gly Ala Thr Cys
   1               5                   10                  15
   Ile Asp Gln Pro Gly Asn Tyr Phe Cys Gln Cys Val Pro Pro Phe Lys
               20                  25                  30
   Val Val Asp Gly Phe Ser Cys Leu
           35                  40
<210> 1162
   <211> 37
   <212> PRT
   <213> Homo sapiens
<400> 1162
   Asp Ile Asp Asp Cys Ile Leu Asn Ala Cys Glu His Asn Ser Thr Cys
   1               5                   10                  15
   Lys Asp Leu His Leu Ser Tyr Gln Cys Val Cys Leu Ser Asp Trp Glu
               20                  25                  30
   Gly Asn Phe Cys Glu
           35
<210> 1163
   <211> 39
   <212> PRT
   <213> Homo sapiens
<400> 1163
   Ser Val Asn Ser Cys Ala Val Asn Pro Cys Leu His Asn Gly Glu Cys
   1               5                   10                  15
   Val Ala Asp Asn Thr Ser Arg Gly Tyr His Cys Arg Cys Pro Pro Ser
               20                  25                  30
   Trp Gln Gly Asp Asp Cys Ser
           35
<210> 1164
   <211> 39
   <212> PRT
   <213> Homo sapiens
<400> 1164
   Asp Val Asn Glu Cys Leu Ser Asn Pro Cys Pro Ser Thr Ala Met Cys
   1               5                   10                  15
   Asn Asn Thr Gln Gly Ser Phe Ile Cys Lys Cys Pro Val Gly Tyr Gln
               20                  25                  30
   Leu Glu Lys Gly Ile Cys Asn
           35
<210> 1165
   <211> 34
   <212> PRT
   <213> Homo sapiens
<400> 1165
   Asn Arg Ala Ile Cys Arg Gln Gly Cys Ser Pro Lys His Gly Ser Cys
   1               5                   10                  15
   Lys Leu Pro Gly Asp Cys Arg Cys Gln Tyr Gly Trp Gln Gly Leu Tyr
               20                  25                  30
   Cys Asp
<210> 1166
   <211> 42
   <212> PRT
   <213> Homo sapiens
<400> 1166
   Asp Thr Pro Glu Gly Val Arg Tyr Ile Ser Ser Asn Val Cys Gly Pro
   1               5                   10                  15
   His Gly Lys Cys Lys Ser Gln Ser Gly Gly Lys Phe Thr Cys Asp Cys
               20                  25                  30
   Asn Lys Gly Phe Thr Gly Thr Tyr Cys His
           35                  40
<210> 1167
   <211> 37
   <212> PRT
   <213> Homo sapiens
<400> 1167
   Asn Ile Asn Asp Cys Glu Ser Asn Pro Cys Arg Asn Gly Gly Thr Cys
   1               5                   10                  15
   Ile Asp Gly Val Asn Ser Tyr Lys Cys Ile Cys Ser Asp Gly Trp Glu
               20                  25                  30
   Gly Ala Tyr Cys Glu
           35
<210> 1168
   <211> 37
   <212> PRT
   <213> Homo sapiens
<400> 1168
   Asn Ile Asn Asp Cys Ser Gln Asn Pro Cys His Asn Gly Gly Thr Cys
   1               5                   10                  15
   Arg Asp Leu Val Asn Asp Phe Tyr Cys Asp Cys Lys Asn Gly Trp Lys
               20                  25                  30
   Gly Lys Thr Cys His
           35
<210> 1169
   <211> 37
   <212> PRT
   <213> Homo sapiens
<400> 1169
   Arg Asp Ser Gln Cys Asp Glu Ala Thr Cys Asn Asn Gly Gly Thr Cys
   1               5                   10                  15
   Tyr Asp Glu Gly Asp Ala Phe Lys Cys Met Cys Pro Gly Gly Trp Glu
               20                  25                  30
   Gly Thr Thr Cys Asn
           35
<210> 1170
   <211> 37
   <212> PRT
   <213> Homo sapiens
<400> 1170
   Arg Asn Ser Ser Cys Leu Pro Asn Pro Cys His Asn Gly Gly Thr Cys
   1               5                   10                  15
   Val Val Asn Gly Glu Ser Phe Thr Cys Val Cys Lys Glu Gly Trp Glu
               20                  25                  30
   Gly Pro Ile Cys Ala
           35
<210> 1171
   <211> 37
   <212> PRT
   <213> Homo sapiens
<400> 1171
   Asn Thr Asn Asp Cys Ser Pro His Pro Cys Tyr Asn Ser Gly Thr Cys
   1               5                   10                  15
   Val Asp Gly Asp Asn Trp Tyr Arg Cys Glu Cys Ala Pro Gly Phe Ala
               20                  25                  30
   Gly Pro Asp Cys Arg
           35
<210> 1172
   <211> 37
   <212> PRT
   <213> Homo sapiens
<400> 1172
   Asn Ile Asn Glu Cys Gln Ser Ser Pro Cys Ala Phe Gly Ala Thr Cys
   1               5                   10                  15
   Val Asp Glu Ile Asn Gly Tyr Arg Cys Val Cys Pro Pro Gly His Ser
               20                  25                  30
   Gly Ala Lys Cys Gln
           35
<210> 1173
   <211> 31
   <212> PRT
   <213> Homo sapiens
<400> 1173
   Lys Cys Ile Pro His Pro Gly Cys Val His Gly Ile Cys Asn Glu Pro
   1               5                   10                  15
   Trp Gln Cys Leu Cys Glu Thr Asn Trp Gly Gly Gln Leu Cys Asp
               20                  25                  30
<210> 1174
   <211> 39
   <212> PRT
   <213> Homo sapiens
<400> 1174
   Asp Leu Asn Tyr Cys Gly Thr His Gln Pro Cys Leu Asn Gly Gly Thr
   1               5                   10                  15
   Cys Ser Asn Thr Gly Pro Asp Lys Tyr Gln Cys Ser Cys Pro Glu Gly
               20                  25                  30
   Tyr Ser Gly Pro Asn Cys Glu
           35
<210> 1175
   <211> 37
   <212> PRT
   <213> Homo sapiens
<400> 1175
   Ala Glu His Ala Cys Leu Ser Asp Pro Cys His Asn Arg Gly Ser Cys
   1               5                   10                  15
   Lys Glu Thr Ser Leu Gly Phe Glu Cys Glu Cys Ser Pro Gly Trp Thr
               20                  25                  30
   Gly Pro Thr Cys Ser
           35
<210> 1176
   <211> 37
   <212> PRT
   <213> Homo sapiens
<400> 1176
   Asn Ile Asp Asp Cys Ser Pro Asn Asn Cys Ser His Gly Gly Thr Cys
   1               5                   10                  15
   Gln Asp Leu Val Asn Gly Phe Lys Cys Val Cys Pro Pro Gln Trp Thr
               20                  25                  30
   Gly Lys Thr Cys Gln
           35
<210> 1177
   <211> 37
   <212> PRT
   <213> Homo sapiens
<400> 1177
   Asp Ala Asn Glu Cys Glu Ala Lys Pro Cys Val Asn Ala Lys Ser Cys
   1               5                   10                  15
   Lys Asn Leu Ile Ala Ser Tyr Tyr Cys Asp Cys Leu Pro Gly Trp Met
               20                  25                  30
   Gly Gln Asn Cys Asp
           35
<210> 1178
   <211> 36
   <212> PRT
   <213> Homo sapiens
<400> 1178
   Asn Ile Asn Asp Cys Leu Gly Gln Cys Gln Asn Asp Ala Ser Cys Arg
   1 5 10 15
   Asp Leu Val Asn Gly Tyr Arg Cys Ile Cys Pro Pro Gly Tyr Ala Gly
   20 25 30
   Asp His Cys Glu
   35
<210> 1179
   <211> 37
   <212> PRT
   <213> Homo sapiens
<400> 1179
   Asp Ile Asp Glu Cys Ala Ser Asn Pro Cys Leu Asn Gly Gly His Cys
   1 5 10 15
   Gln Asn Glu Ile Asn Arg Phe Gln Cys Leu Cys Pro Thr Gly Phe Ser
   20 25 30
   Gly Asn Leu Cys Gln
   35
<210> 1180
   <211> 37
   <212> PRT
   <213> Homo sapiens
<400> 1180
   Asp Ile Asp Tyr Cys Glu Pro Asn Pro Cys Gln Asn Gly Ala Gln Cys
   1 5 10 15
   Tyr Asn Arg Ala Ser Asp Tyr Phe Cys Lys Cys Pro Glu Asp Tyr Glu
   20 25 30
   Gly Lys Asn Cys Ser
   35
<210> 1181
   <211> 34
   <212> PRT
   <213> Homo sapiens
<400> 1181
   Lys Glu Ala Val Cys Lys Gln Gly Cys Asn Leu Leu His Gly Gly Cys
   1 5 10 15
   Thr Val Pro Gly Glu Cys Arg Cys Ser Tyr Gly Trp Gln Gly Arg Phe
   20 25 30
   Cys Asp
<210> 1182
   <211> 61
   <212> PRT
   <213> Homo sapiens
<400> 1182
   Pro Arg Glu Pro Cys Pro Gly Gly Ala Cys Arg Val Ile Asp Gly Cys
   1               5                   10                  15
   Gly Ser Asp Ala Gly Pro Gly Met Pro Gly Thr Ala Ala Ser Gly Val
               20                  25                  30
   Cys Gly Pro His Gly Arg Cys Val Ser Gln Pro Gly Gly Asn Phe Ser
           35                  40                  45
   Cys Ile Cys Asp Ser Gly Phe Thr Gly Thr Tyr Cys His
       50                  55                  60
<210> 1183
   <211> 37
   <212> PRT
   <213> Homo sapiens
<400> 1183
   Asn Ile Asp Asp Cys Leu Gly Gln Pro Cys Arg Asn Gly Gly Thr Cys
   1               5                   10                  15
   Ile Asp Glu Val Asp Ala Phe Arg Cys Phe Cys Pro Ser Gly Trp Glu
               20                  25                  30
   Gly Glu Leu Cys Asp
           35
<210> 1184
   <211> 37
   <212> PRT
   <213> Homo sapiens
<400> 1184
   Asn Pro Asn Asp Cys Leu Pro Asp Pro Cys His Ser Arg Gly Arg Cys
   1               5                   10                  15
   Tyr Asp Leu Val Asn Asp Phe Tyr Cys Ala Cys Asp Asp Gly Trp Lys
               20                  25                  30
   Gly Lys Thr Cys His
           35
<210> 1185
   <211> 37
   <212> PRT
   <213> Homo sapiens
<400> 1185
   Arg Glu Phe Gln Cys Asp Ala Tyr Thr Cys Ser Asn Gly Gly Thr Cys
   1               5                   10                  15
   Tyr Asp Ser Gly Asp Thr Phe Arg Cys Ala Cys Pro Pro Gly Trp Lys
               20                  25                  30
   Gly Ser Thr Cys Ala
           35
<210> 1186
   <211> 37
   <212> PRT
   <213> Homo sapiens
<400> 1186
   Lys Asn Ser Ser Cys Leu Pro Asn Pro Cys Val Asn Gly Gly Thr Cys
   1               5                   10                  15
   Val Gly Ser Gly Ala Ser Phe Ser Cys Ile Cys Arg Asp Gly Trp Glu
               20                  25                  30
   Gly Arg Thr Cys Thr
           35
<210> 1187
   <211> 37
   <212> PRT
   <213> Homo sapiens
<400> 1187
   Asn Thr Asn Asp Cys Asn Pro Leu Pro Cys Tyr Asn Gly Gly Ile Cys
   1               5                   10                  15
   Val Asp Gly Val Asn Trp Phe Arg Cys Glu Cys Ala Pro Gly Phe Ala
               20                  25                  30
   Gly Pro Asp Cys Arg
           35
<210> 1188
   <211> 37
   <212> PRT
   <213> Homo sapiens
<400> 1188
   Asn Ile Asp Glu Cys Gln Ser Ser Pro Cys Ala Tyr Gly Ala Thr Cys
   1               5                   10                  15
   Val Asp Glu Ile Asn Gly Tyr Arg Cys Ser Cys Pro Pro Gly Arg Ala
               20                  25                  30
   Gly Pro Arg Cys Gln
           35
<210> 1189
   <211> 31
   <212> PRT
   <213> Homo sapiens
<400> 1189
   Glu Cys Val Pro Tyr Pro Gly Cys Val His Gly Ser Cys Val Glu Pro
   1               5                   10                  15
   Trp Gln Cys Asn Cys Glu Thr Asn Trp Gly Gly Leu Leu Cys Asp
               20                  25                  30
<210> 1190
   <211> 39
   <212> PRT
   <213> Homo sapiens
<400> 1190
   Asp Leu Asn Tyr Cys Gly Ser His His Pro Cys Thr Asn Gly Gly Thr
   1               5                   10                  15
   Cys Ile Asn Ala Glu Pro Asp Gln Tyr Arg Cys Thr Cys Pro Asp Gly
               20                  25                  30
   Tyr Ser Gly Arg Asn Cys Glu
           35
<210> 1191
   <211> 37
   <212> PRT
   <213> Homo sapiens
<400> 1191
   Ala Glu His Ala Cys Thr Ser Asn Pro Cys Ala Asn Gly Gly Ser Cys
   1               5                   10                  15
   His Glu Val Pro Ser Gly Phe Glu Cys His Cys Pro Ser Gly Trp Ser
               20                  25                  30
   Gly Pro Thr Cys Ala
           35
<210> 1192
   <211> 37
   <212> PRT
   <213> Homo sapiens
<400> 1192
   Asp Ile Asp Glu Cys Ala Ser Asn Pro Cys Ala Ala Gly Gly Thr Cys
   1               5                   10                  15
   Val Asp Gln Val Asp Gly Phe Glu Cys Ile Cys Pro Glu Gln Trp Val
               20                  25                  30
   Gly Ala Thr Cys Gln
           35
<210> 1193
   <211> 37
   <212> PRT
   <213> Homo sapiens
<400> 1193
   Asp Ala Asn Glu Cys Glu Gly Lys Pro Cys Leu Asn Ala Phe Ser Cys
   1               5                   10                  15
   Lys Asn Leu Ile Gly Gly Tyr Tyr Cys Asp Cys Ile Pro Gly Trp Lys
               20                  25                  30
   Gly Ile Asn Cys His
           35
<210> 1194
   <211> 36
   <212> PRT
   <213> Homo sapiens
<400> 1194
   Asn Val Asn Asp Cys Arg Gly Gln Cys Gln His Gly Gly Thr Cys Lys
   1               5                   10                  15
   Asp Leu Val Asn Gly Tyr Gln Cys Val Cys Pro Arg Gly Phe Gly Gly
               20                  25                  30
   Arg His Cys Glu
           35
<210> 1195
   <211> 37
   <212> PRT
   <213> Homo sapiens
<400> 1195
   Glu Arg Asp Glu Cys Ala Ser Ser Pro Cys His Ser Gly Gly Leu Cys
   1               5                   10                  15
   Glu Asp Leu Ala Asp Gly Phe His Cys His Cys Pro Gln Gly Phe Ser
               20                  25                  30
   Gly Pro Leu Cys Glu
           35
<210> 1196
   <211> 37
   <212> PRT
   <213> Homo sapiens
<400> 1196
   Asp Val Asp Leu Cys Glu Pro Ser Pro Cys Arg Asn Gly Ala Arg Cys
   1               5                   10                  15
   Tyr Asn Leu Glu Gly Asp Tyr Tyr Cys Ala Cys Pro Asp Asp Phe Gly
               20                  25                  30
   Gly Lys Asn Cys Ser
           35
<210> 1197
   <211> 42
   <212> PRT
   <213> Homo sapiens
<400> 1197
   Asp Ile Asp Glu Cys Ala Ala Lys Met His Tyr Cys His Ala Asn Thr
   1               5                   10                  15
   Val Cys Val Asn Leu Pro Gly Leu Tyr Arg Cys Asp Cys Val Pro Gly
               20                  25                  30
   Tyr Ile Arg Val Asp Asp Phe Ser Cys Thr
           35                  40
<210> 1198
   <211> 41
   <212> PRT
   <213> Homo sapiens
<400> 1198
   Glu His Asp Glu Cys Gly Ser Gly Gln His Asn Cys Asp Glu Asn Ala
   1               5                   10                  15
   Ile Cys Thr Asn Thr Val Gln Gly His Ser Cys Thr Cys Lys Pro Gly
               20                  25                  30
   Tyr Val Gly Asn Gly Thr Ile Cys Arg
           35                  40
<210> 1199
   <211> 31
   <212> PRT
   <213> Homo sapiens
<400> 1199
   Ala Phe Cys Glu Glu Gly Cys Arg Tyr Gly Gly Thr Cys Val Ala Pro
   1               5                   10                  15
   Asn Lys Cys Val Cys Pro Ser Gly Phe Thr Gly Ser His Cys Glu
               20                  25                  30
<210> 1200
   <211> 39
   <212> PRT
   <213> Homo sapiens
<400> 1200
   Asp Ile Asp Glu Cys Ser Glu Gly Ile Ile Glu Cys His Asn His Ser
   1               5                   10                  15
   Arg Cys Val Asn Leu Pro Gly Trp Tyr His Cys Glu Cys Arg Ser Gly
               20                  25                  30
   Phe His Asp Asp Gly Thr Tyr
           35
<210> 1201
   <211> 36
   <212> PRT
   <213> Homo sapiens
<400> 1201
   Asp Ile Asp Glu Cys Ala Leu Arg Thr His Thr Cys Trp Asn Asp Ser
   1               5                   10                  15
   Ala Cys Ile Asn Leu Ala Gly Gly Phe Asp Cys Leu Cys Pro Ser Gly
               20                  25                  30
   Pro Ser Cys Ser
           35
<210> 1202
   <211> 43
   <212> PRT
   <213> Homo sapiens
<400> 1202
   Gly Tyr Asp Phe Cys Ser Glu Arg His Asn Cys Met Glu Asn Ser Ile
   1 5 10 15
   Cys Arg Asn Leu Asn Asp Arg Ala Val Cys Ser Cys Arg Asp Gly Phe
   20 25 30
   Arg Ala Leu Arg Glu Asp Asn Ala Tyr Cys Glu
   35 40
<210> 1203
   <211> 42
   <212> PRT
   <213> Homo sapiens
<400> 1203
   Asp Ile Asp Glu Cys Ala Glu Gly Arg His Tyr Cys Arg Glu Asn Thr
   1 5 10 15
   Met Cys Val Asn Thr Pro Gly Ser Phe Met Cys Ile Cys Lys Thr Gly
   20 25 30
   Tyr Ile Arg Ile Asp Asp Tyr Ser Cys Thr
   35 40
<210> 1204
   <211> 36
   <212> PRT
   <213> Homo sapiens
<400> 1204
   Asp Ile Asp Glu Cys Gly Thr Gly Arg His Ser Cys Ala Asn Asp Thr
   1 5 10 15
   Ile Cys Phe Asn Leu Asp Gly Gly Tyr Asp Cys Arg Cys Pro His Gly
   20 25 30
   Lys Asn Cys Thr
   35
<210> 1205
   <211> 41
   <212> PRT
   <213> Homo sapiens
<400> 1205
   Glu His Asp Glu Cys Ile Thr Asn Gln His Asn Cys Asp Glu Asn Ala
   1 5 10 15
   Leu Cys Phe Asn Thr Val Gly Gly His Asn Cys Val Cys Lys Pro Gly
   20 25 30
   Tyr Thr Gly Asn Gly Thr Thr Cys Lys
   35 40
<210> 1206
   <211> 31
   <212> PRT
   <213> Homo sapiens
<400> 1206
   Ala Phe Cys Lys Asp Gly Cys Arg Asn Gly Gly Ala Cys Ile Ala Ala
   1               5                   10                  15
   Asn Val Cys Ala Cys Pro Gln Gly Phe Thr Gly Pro Ser Cys Glu
               20                  25                  30
<210> 1207
   <211> 47
   <212> PRT
   <213> Homo sapiens
<400> 1207
   Asp Ile Asp Glu Cys Ser Asp Gly Phe Val Gln Cys Asp Ser Arg Ala
   1               5                   10                  15
   Asn Cys Ile Asn Leu Pro Gly Trp Tyr His Cys Glu Cys Arg Asp Gly
               20                  25                  30
   Tyr His Asp Asn Gly Met Phe Ser Pro Ser Gly Glu Ser Cys Glu
           35                  40                  45
<210> 1208
   <211> 38
   <212> PRT
   <213> Homo sapiens
<400> 1208
   Val His His Gly Cys Glu Asp Asp Pro Cys Pro Glu Gly Ser Glu Cys
   1               5                   10                  15
   Val Ser Asp Pro Trp Glu Glu Lys His Thr Cys Val Cys Pro Ser Gly
               20                  25                  30
   Arg Phe Gly Gln Cys Pro
           35
<210> 1209
   <211> 38
   <212> PRT
   <213> Homo sapiens
<400> 1209
   Ala Thr Glu Asp Cys Ala Ser Asn Pro Cys Gln Asn Gly Gly Val Cys
   1               5                   10                  15
   Asn Pro Ser Pro Ala Gly Gly Tyr Tyr Cys Lys Cys Ser Ala Leu Tyr
               20                  25                  30
   Ile Gly Thr His Cys Glu
           35
<210> 1210
   <211> 37
   <212> PRT
   <213> Homo sapiens
<400> 1210
   Ser Val Asn Pro Cys Ser Ser Lys Pro Cys Leu Tyr Gly Gly Thr Cys
   1               5                   10                  15
   Val Val Asp Asn Gly Gly Phe Val Cys Gln Cys Arg Gly Leu Tyr Thr
               20                  25                  30
   Gly Gln Arg Cys Gln
           35
<210> 1211
   <211> 37
   <212> PRT
   <213> Homo sapiens
<400> 1211
   Leu Ser Pro Tyr Cys Lys Asp Glu Pro Cys Lys Asn Gly Gly Thr Cys
   1               5                   10                  15
   Phe Asp Ser Leu Asp Gly Ala Val Cys Gln Cys Asp Ser Gly Phe Arg
               20                  25                  30
   Gly Glu Arg Cys Gln
           35
<210> 1212
   <211> 37
   <212> PRT
   <213> Homo sapiens
<400> 1212
   Asp Ile Asp Glu Cys Ser Gly Asn Pro Cys Leu His Gly Ala Leu Cys
   1               5                   10                  15
   Glu Asn Thr His Gly Ser Tyr His Cys Asn Cys Ser His Glu Tyr Arg
               20                  25                  30
   Gly Arg His Cys Glu
           35
<210> 1213
   <211> 38
   <212> PRT
   <213> Homo sapiens
<400> 1213
   His Ser Asp Tyr Cys Ser Gln Asn Thr Cys Leu Asn Gly Gly Lys Cys
   1               5                   10                  15
   Ser Trp Thr His Gly Ala Gly Tyr Val Cys Lys Cys Pro Pro Gln Phe
               20                  25                  30
   Ser Gly Lys His Cys Glu
           35
<210> 1214
   <211> 37
   <212> PRT
   <213> Homo sapiens
<400> 1214
   Gly Arg Glu Asn Cys Thr Phe Ala Pro Cys Leu Glu Gly Gly Thr Cys
   1               5                   10                  15
   Ile Leu Ser Pro Lys Gly Ala Ser Cys Asn Cys Pro His Pro Tyr Thr
               20                  25                  30
   Gly Asp Arg Cys Glu
           35
<210> 1215
   <211> 39
   <212> PRT
   <213> Homo sapiens
<400> 1215
   Ser Asn Asp Pro Cys Val Glu Lys Pro Cys Pro Gly Asp Met Gln Cys
   1               5                   10                  15
   Val Ser Tyr Glu Ala Ser Arg Arg Pro Phe Leu Cys Gln Cys Pro Pro
               20                  25                  30
   Gly Lys Leu Gly Glu Cys Ser
           35
<210> 1216
   <211> 38
   <212> PRT
   <213> Homo sapiens
<400> 1216
   Tyr Pro Asp Ala Cys Lys Arg Ser Pro Cys Gln His Gly Gly Ser Cys
   1               5                   10                  15
   Thr Gly Leu Pro Ser Gly Gly Tyr Gln Cys Thr Cys Leu Ser Gln Phe
               20                  25                  30
   Thr Gly Arg Asn Cys Glu
           35
<210> 1217
   <211> 37
   <212> PRT
   <213> Homo sapiens
<400> 1217
   Glu Ile Thr Ala Cys Phe Pro Asn Pro Cys Arg Asn Gly Gly Ser Cys
   1               5                   10                  15
   Asp Pro Ile Gly Asn Thr Phe Ile Cys Asn Cys Lys Ala Gly Leu Thr
               20                  25                  30
   Gly Val Thr Cys Glu
           35
<210> 1218
   <211> 37
   <212> PRT
   <213> Homo sapiens
<400> 1218
   Asp Ile Asn Glu Cys Glu Arg Glu Glu Cys Glu Asn Gly Gly Ser Cys
   1 5 10 15
   Val Asn Val Phe Gly Ser Phe Leu Cys Asn Cys Thr Pro Gly Tyr Val
   20 25 30
   Gly Gln Tyr Cys Gly
   35
<210> 1219
   <211> 59
   <212> PRT
   <213> Homo sapiens
<400> 1219
   Asp His Asp Ser Cys Val His Gly Pro Cys Gln Asn Gly Gly Ser Cys
   1 5 10 15
   Leu Arg Arg Leu Ala Val Ser Ser Val Leu Lys Ser Arg Glu Ser Leu
   20 25 30
   Pro Val Ile Ile Val Ala Asn Glu Pro Leu Gln Pro Phe Leu Cys Lys
   35 40 45
   Cys Leu Pro Gly Tyr Ala Gly Ser Trp Cys Glu
   50 55
<210> 1220
   <211> 37
   <212> PRT
   <213> Homo sapiens
<400> 1220
   Asp Ile Asp Glu Cys Leu Pro Ser Pro Cys His Ser Gly Gly Thr Cys
   1 5 10 15
   His Asn Leu Val Gly Gly Phe Ser Cys Ser Cys Pro Asp Gly Phe Thr
   20 25 30
   Gly Arg Ala Cys Glu
   35
<210> 1221
   <211> 37
   <212> PRT
   <213> Homo sapiens
<400> 1221
   Asp Ile Asn Glu Cys Leu Gln Ser Pro Cys Lys Asn Gly Ala Ile Cys
   1 5 10 15
   Gln Asn Phe Pro Gly Ser Phe Asn Cys Val Cys Lys Thr Gly Tyr Thr
   20 25 30
   Gly Lys Met Cys Glu
   35
<210> 1222
   <211> 37
   <212> PRT
   <213> Homo sapiens
<400> 1222
   Ser Val Asn Tyr Cys Glu Cys Asn Pro Cys Phe Asn Gly Gly Ser Cys
   1               5                   10                  15
   Gln Ser Gly Val Asp Ser Tyr Tyr Cys His Cys Pro Phe Gly Val Phe
               20                  25                  30
   Gly Lys His Cys Glu
           35
<210> 1223
   <211> 37
   <212> PRT
   <213> Homo sapiens
<400> 1223
   Leu Glu Gly Ala Cys Thr Arg Ser Pro Cys Gln His Gly Gly Thr Cys
   1               5                   10                  15
   Met Asp Tyr Trp Ser Trp Gln Gln Cys His Cys Lys Glu Gly Leu Thr
               20                  25                  30
   Gly Lys Tyr Cys Glu
           35
<210> 1224
   <211> 38
   <212> PRT
   <213> Homo sapiens
<400> 1224
   Pro Pro Gly Asp Cys Ala Ser His Pro Cys Gln Asn Gly Gly Ser Cys
   1               5                   10                  15
   Glu Pro Gly Leu His Ser Gly Phe Thr Cys Ser Cys Pro Asp Ser His
               20                  25                  30
   Thr Gly Arg Thr Cys Glu
           35
<210> 1225
   <211> 41
   <212> PRT
   <213> Homo sapiens
<400> 1225
   His Phe Asn Asp Cys Pro Asp Ser His Thr Gln Phe Cys Phe His Gly
   1               5                   10                  15
   Thr Cys Arg Phe Leu Val Gln Glu Asp Lys Pro Ala Cys Val Cys His
               20                  25                  30
   Ser Gly Tyr Val Gly Ala Arg Cys Glu
           35                  40
<210> 1226
   <211> 37
   <212> PRT
   <213> Homo sapiens
<400> 1226
   Tyr Thr Ala Ser Cys Gln Asp Met Ser Cys Ser Lys Gln Gly Glu Cys
   1               5                   10                  15
   Leu Glu Thr Ile Gly Asn Tyr Thr Cys Ser Cys Tyr Pro Gly Phe Tyr
               20                  25                  30
   Gly Pro Glu Cys Glu
           35
<210> 1227
   <211> 35
   <212> PRT
   <213> Homo sapiens
<400> 1227
   Asn Leu Lys Leu Cys Asp Ala Ser Asn His Cys Asp Arg His Gly Val
   1               5                   10                  15
   Cys Asn Asn Phe Asn His Cys His Cys Glu Lys Gly Tyr Asn Pro Pro
               20                  25                  30
   Tyr Cys Gln
           35
<210> 1228
   <211> 39
   <212> PRT
   <213> Homo sapiens
<400> 1228
   Cys Pro Pro Cys Pro Lys Tyr Ala Ser Cys His Asn Ser Thr His Cys
   1               5                   10                  15
   Thr Cys Glu Asp Gly Phe Arg Ala Arg Ser Gly Arg Thr Tyr Phe His
               20                  25                  30
   Asp Ser Ser Glu Lys Cys Glu
           35
<210> 1229
   <211> 51
   <212> PRT
   <213> Homo sapiens
<400> 1229
   Asp Ile Asn Glu Cys Glu Thr Gly Leu Ala Lys Cys Lys Tyr Lys Ala
   1               5                   10                  15
   Tyr Cys Arg Asn Lys Val Gly Gly Tyr Ile Cys Ser Cys Leu Val Lys
               20                  25                  30
   Tyr Thr Leu Phe Asn Phe Leu Ala Gly Ile Ile Asp Tyr Asp His Pro
           35                  40                  45
   Asp Cys Tyr
       50
<210> 1230
   <211> 30
   <212> PRT
   <213> Homo sapiens
<400> 1230
   Leu Asn Arg Thr Cys Cys Leu Asn Gly Gly Thr Cys Met Leu Glu Ser
   1               5                   10                  15
   Phe Cys Ala Cys Pro Pro Ser Phe Tyr Gly Arg Asn Cys Glu
               20                  25                  30
<210> 1231
   <211> 32
   <212> PRT
   <213> Homo sapiens
<400> 1231
   Ile Gly Pro Ser Cys Leu Lys Phe Cys Ser Gly Arg Gly Gln Cys Thr
   1               5                   10                  15
   Arg His Gly Cys Lys Cys Asp Pro Gly Phe Ser Gly Pro Ala Cys Glu
               20                  25                  30
<210> 1232
   <211> 32
   <212> PRT
   <213> Homo sapiens
<400> 1232
   Ile Gly Gln Gln Cys Pro Asn Met Cys Ser Gly His Gly Ser Cys Asp
   1               5                   10                  15
   His Gly Ile Cys Arg Cys Asp Gln Gly Tyr Gln Gly Thr Glu Cys His
               20                  25                  30
<210> 1233
   <211> 34
   <212> PRT
   <213> Homo sapiens
<400> 1233
   Ile Ser Glu Pro Cys Pro Ser Tyr Cys Ser Gly His Gly Asp Cys Ile
   1               5                   10                  15
   Ser Gly Val Cys Phe Cys Asp Leu Gly Tyr Thr Ala Ala Gln Gly Thr
               20                  25                  30
   Cys Val
<210> 1234
   <211> 32
   <212> PRT
   <213> Homo sapiens
<400> 1234
   Leu Ala Ser Gly Cys Pro Trp Met Cys Ser Gly Arg Gly Ile Cys Asp
   1               5                   10                  15
   Ala Gly Arg Cys Val Cys Asp Arg Gly Phe Gly Gly Pro Tyr Cys Val
               20                  25                  30
<210> 1235
   <211> 33
   <212> PRT
   <213> Homo sapiens
<400> 1235
   Ile Gly Pro Gln Cys Glu Glu Met Cys Asn Gly Gln Gly Ser Cys Ile
   1               5                   10                  15
   Asn Gly Thr Lys Cys Ile Cys Asp Pro Gly Tyr Ser Gly Pro Thr Cys
               20                  25                  30
   Lys
<210> 1236
   <211> 32
   <212> PRT
   <213> Homo sapiens
<400> 1236
   Ile Gly Asp Gly Cys Ile Asp Met Cys Ser Gly His Gly Arg Cys Ile
   1               5                   10                  15
   Gln Gly Asn Cys Val Cys Asp Glu Gln Trp Gly Gly Leu Tyr Cys Asp
               20                  25                  30
<210> 1237
   <211> 32
   <212> PRT
   <213> Homo sapiens
<400> 1237
   Leu Gly Pro Gly Cys Leu Asp Asn Cys Arg Gly His Gly Asp Cys Leu
   1               5                   10                  15
   Arg Glu Gln Cys Ile Cys Asp Pro Gly Tyr Ser Gly Pro Asn Cys Tyr
               20                  25                  30
<210> 1238
   <211> 33
   <212> PRT
   <213> Homo sapiens
<400> 1238
   Ile Gly Glu Ala Cys Pro Lys Leu Cys Ser Gly His Gly Tyr Cys Thr
   1               5                   10                  15
   Thr Gly Ala Ile Cys Ile Cys Asp Glu Ser Phe Gln Gly Asp Asp Cys
               20                  25                  30
   Ser
<210> 1239
   <211> 35
   <212> PRT
   <213> Homo sapiens
<400> 1239
   Thr Ile Pro Ile Cys Glu Gly Pro Asp Ala Cys Gln Lys Asp Glu Val
   1 5 10 15
   Cys Val Lys Pro Gly Leu Cys Arg Cys Lys Pro Gly Phe Phe Gly Ala
   20 25 30
   His Cys Ser
   35
<210> 1240
   <211> 36
   <212> PRT
   <213> Homo sapiens
<400> 1240
   Trp Gly Pro Asp Cys Arg Glu Ser Cys Pro Cys His Pro His Gly Gln
   1 5 10 15
   Cys Glu Pro Ala Thr Gly Ala Cys Gln Cys Gln Ala Asp Arg Trp Gly
   20 25 30
   Ala Arg Cys Glu
   35
<210> 1241
   <211> 37
   <212> PRT
   <213> Homo sapiens
<400> 1241
   Trp Ser Ser Thr Cys Arg Arg Pro Cys Gln Cys Asn Thr Ala Ala Ala
   1 5 10 15
   Arg Cys Glu Gln Ala Thr Gly Ala Cys Val Cys Lys Pro Gly Trp Trp
   20 25 30
   Gly Arg Arg Cys Ser
   35
<210> 1242
   <211> 35
   <212> PRT
   <213> Homo sapiens
<400> 1242
   Trp Gly Pro Glu Cys Gln Gln Gln Cys Glu Cys Val Arg Gly Arg Cys
   1 5 10 15
   Ser Ala Ala Ser Gly Glu Cys Thr Cys Pro Pro Gly Phe Arg Gly Ala
   20 25 30
   Arg Cys Glu
   35
<210> 1243
   <211> 38
   <212> PRT
   <213> Homo sapiens
<400> 1243
   Phe Gly Glu Ser Cys Glu Gln Gln Cys Pro His Cys Arg His Gly Glu
   1               5                   10                  15
   Ala Cys Glu Pro Asp Thr Gly His Cys Gln Arg Cys Asp Pro Gly Trp
               20                  25                  30
   Leu Gly Pro Arg Cys Glu
           35
<210> 1244
   <211> 32
   <212> PRT
   <213> Homo sapiens
<400> 1244
   Cys Gly Ser Thr Cys Pro Thr Cys Val Gln Gly Ser Cys Asp Thr Val
   1               5                   10                  15
   Thr Gly Asp Cys Val Cys Ser Ala Gly Tyr Trp Gly Pro Ser Cys Asn
              20                   25                  30
<210> 1245
   <211> 40
   <212> PRT
   <213> Homo sapiens
<400> 1245
   Gln Gly Asp Glu Cys Gly Ile Ala Val Cys Glu Gly Asn Ser Thr Cys
   1               5                   10                  15
   Ser Glu Asn Glu Val Cys Val Arg Pro Gly Glu Cys Arg Cys Arg His
               20                  25                  30
   Gly Tyr Phe Gly Ala Asn Cys Asp
           35                  40
<210> 1246
   <211> 32
   <212> PRT
   <213> Homo sapiens
<400> 1246
   Cys Lys Glu Leu Cys Ser Cys His Pro His Gly Gln Cys Glu Asp Val
   1               5                   10                  15
   Thr Gly Gln Cys Thr Cys His Ala Arg Arg Trp Gly Ala Arg Cys Glu
               20                  25                  30
<210> 1247
   <211> 35
   <212> PRT
   <213> Homo sapiens
<400> 1247
   Trp Gly Ala Arg Cys Glu His Ala Cys Gln Cys Gln His Gly Thr Cys
   1               5                   10                  15
   His Pro Arg Ser Gly Ala Cys Arg Cys Glu Pro Gly Trp Trp Gly Ala
               20                  25                  30
   Gln Cys Ala
           35
<210> 1248
   <211> 30
   <212> PRT
   <213> Homo sapiens
<400> 1248
   Ser Ala Cys Tyr Cys Ser Ala Thr Ser Arg Cys Asp Pro Gln Thr Gly
   1               5                   10                  15
   Ala Cys Leu Cys His Ala Gly Trp Trp Gly Arg Ser Cys Asn
               20                  25                  30
<210> 1249
   <211> 29
   <212> PRT
   <213> Homo sapiens
<400> 1249
   Asn Gln Cys Ala Cys Asn Ser Ser Pro Cys Glu Gln Gln Ser Gly Arg
   1               5                   10                  15
   Cys Gln Cys Arg Glu Arg Thr Phe Gly Ala Arg Cys Asp
               20                  25
<210> 1250
   <211> 35
   <212> PRT
   <213> Homo sapiens
<400> 1250
   Phe Gly Ala Arg Cys Asp Arg Tyr Cys Gln Cys Phe Arg Gly Arg Cys
   1               5                   10                  15
   His Pro Val Asp Gly Thr Cys Ala Cys Glu Pro Gly Tyr Arg Gly Lys
               20                  25                  30
   Tyr Cys Arg
           35
<210> 1251
   <211> 32
   <212> PRT
   <213> Homo sapiens
<400> 1251
   Cys Ala Phe Val Cys Ala Asp Cys Gly Ser Gly His Cys Asp Phe Gln
   1               5                   10                  15
   Ser Gly Arg Cys Leu Cys Ser Pro Gly Val His Gly Pro His Cys Asn
               20                  25                  30
<210> 1252
   <211> 40
   <212> PRT
   <213> Homo sapiens
<400> 1252
   Cys Pro Leu Thr Cys Asp Asp Ile Ser Gly Gln Val Thr Cys Pro Pro
   1               5                   10                  15
   Asp Trp Pro Cys Gly Ser Pro Gly Cys Trp Cys Pro Glu Gly Gln Val
               20                  25                  30
   Leu Gly Ser Glu Gly Trp Cys Val
           35                  40
<210> 1253
   <211> 38
   <212> PRT
   <213> Homo sapiens
<400> 1253
   Trp Pro Arg Gln Cys Pro Cys Leu Val Asp Gly Ala Arg Tyr Trp Pro
   1               5                   10                  15
   Gly Gln Arg Ile Lys Ala Asp Cys Gln Leu Cys Ile Cys Gln Asp Gly
               20                  25                  30
   Arg Pro Arg Arg Cys Arg
           35
<210> 1254
   <211> 41
   <212> PRT
   <213> Homo sapiens
<400> 1254
   Asp Val Asp Glu Cys Ser Glu Gly Thr Asp Asp Cys His Ile Asp Ala
   1               5                   10                  15
   Ile Cys Gln Asn Thr Pro Lys Ser Tyr Lys Cys Leu Cys Lys Pro Gly
               20                  25                  30
   Tyr Lys Gly Glu Gly Lys Gln Cys Glu
           35                  40
<210> 1255
   <211> 43
   <212> PRT
   <213> Homo sapiens
<400> 1255
   Asp Ile Asp Glu Cys Glu Asn Asp Tyr Tyr Asn Gly Gly Cys Val His
   1               5                   10                  15
   Glu Cys Ile Asn Ile Pro Gly Asn Tyr Arg Cys Thr Cys Phe Asp Gly
               20                  25                  30
   Phe Met Leu Ala His Asp Gly His Asn Cys Leu
           35                  40
<210> 1256
   <211> 37
   <212> PRT
   <213> Homo sapiens
<400> 1256
   Asp Val Asp Glu Cys Gln Asp Asn Asn Gly Gly Cys Gln Gln Ile Cys
   1 5 10 15
   Val Asn Ala Met Gly Ser Tyr Glu Cys Gln Cys His Ser Gly Phe Phe
   20 25 30
   Leu Ser Asp Asn Gln
   35
<210> 1257
   <211> 37
   <212> PRT
   <213> Homo sapiens
<400> 1257
   Cys Met Asn Lys Asp His Gly Cys Ala His Ile Cys Arg Glu Thr Pro
   1 5 10 15
   Lys Gly Gly Val Ala Cys Asp Cys Arg Pro Gly Phe Asp Leu Ala Gln
   20 25 30
   Asn Gln Lys Asp Cys
   35
<210> 1258
   <211> 39
   <212> PRT
   <213> Homo sapiens
<400> 1258
   Gly Arg Thr Cys Ile Glu Thr Cys Ala Val Asn Asn Gly Gly Cys Asp
   1 5 10 15
   Arg Thr Cys Lys Asp Thr Ala Thr Gly Val Arg Cys Ser Cys Pro Val
   20 25 30
   Gly Phe Thr Leu Gln Pro Asp
   35
<210> 1259
   <211> 36
   <212> PRT
   <213> Homo sapiens
<400> 1259
   Cys Ala Val Asn Asn Gly Gly Cys Asp Arg Thr Cys Lys Asp Thr Ala
   1 5 10 15
   Thr Gly Val Arg Cys Ser Cys Pro Val Gly Phe Thr Leu Gln Pro Asp
   20 25 30
   Gly Lys Thr Cys
   35
<210> 1260
   <211> 41
   <212> PRT
   <213> Homo sapiens
<400> 1260
   Asp Ile Asn Glu Cys Leu Val Asn Asn Gly Gly Cys Asp His Phe Cys
   1               5                   10                  15
   Arg Asn Thr Val Gly Ser Phe Glu Cys Gly Cys Arg Lys Gly Tyr Lys
               20                  25                  30
   Leu Leu Thr Asp Glu Arg Thr Cys Gln
           35                  40
<210> 1261
   <211> 39
   <212> PRT
   <213> Homo sapiens
<400> 1261
   Asp Ile Asp Glu Cys Ser Phe Glu Arg Thr Cys Asp His Ile Cys Ile
   1               5                   10                  15
   Asn Ser Pro Gly Ser Phe Gln Cys Leu Cys His Arg Gly Tyr Ile Leu
               20                  25                  30
   Tyr Gly Thr Thr His Cys Gly
           35
<210> 1262
   <211> 41
   <212> PRT
   <213> Homo sapiens
<400> 1262
   Asp Val Asp Glu Cys Ser Met Ser Asn Gly Ser Cys Asp Gln Gly Cys
   1               5                   10                  15
   Val Asn Thr Lys Gly Ser Tyr Glu Cys Val Cys Pro Pro Gly Arg Arg
               20                  25                  30
   Leu His Trp Asn Gly Lys Asp Cys Val
           35                  40
<210> 1263
   <211> 41
   <212> PRT
   <213> Homo sapiens
<400> 1263
   Asp Val Asp Glu Cys Ala Gln Gly Leu Asp Asp Cys His Ala Asp Ala
   1               5                   10                  15
   Leu Cys Gln Asn Thr Pro Thr Ser Tyr Lys Cys Ser Cys Lys Pro Gly
               20                  25                  30
   Tyr Gln Gly Glu Gly Arg Gln Cys Glu
           35                  40
<210> 1264
   <211> 42
   <212> PRT
   <213> Homo sapiens
<400> 1264
   Asp Ile Asp Glu Cys Gly Asn Glu Leu Asn Gly Gly Cys Val His Asp
   1 5 10 15
   Cys Leu Asn Ile Pro Gly Asn Tyr Arg Cys Thr Cys Phe Asp Gly Phe
   20 25 30
   Met Leu Ala His Asp Gly His Asn Cys Leu
   35 40
<210> 1265
   <211> 41
   <212> PRT
   <213> Homo sapiens
<400> 1265
   Asp Val Asp Glu Cys Leu Glu Asn Asn Gly Gly Cys Gln His Thr Cys
   1 5 10 15
   Val Asn Val Met Gly Ser Tyr Glu Cys Cys Cys Lys Glu Gly Phe Phe
   20 25 30
   Leu Ser Asp Asn Gln His Thr Cys Ile
   35 40
<210> 1266
   <211> 37
   <212> PRT
   <213> Homo sapiens
<400> 1266
   Cys Met Asn Lys Asp His Gly Cys Ser His Ile Cys Lys Glu Ala Pro
   1 5 10 15
   Arg Gly Ser Val Ala Cys Glu Cys Arg Pro Gly Phe Glu Leu Ala Lys
   20 25 30
   Asn Gln Arg Asp Cys
   35
<210> 1267
   <211> 36
   <212> PRT
   <213> Homo sapiens
<400> 1267
   Cys Asn His Gly Asn Gly Gly Cys Gln His Ser Cys Asp Asp Thr Ala
   1 5 10 15
   Asp Gly Pro Glu Cys Ser Cys His Pro Gln Tyr Lys Met His Thr Asp
   20 25 30
   Gly Arg Ser Cys
   35
<210> 1268
   <211> 36
   <212> PRT
   <213> Homo sapiens
<400> 1268
   Cys Ala Val Asn Asn Gly Gly Cys Asp Arg Thr Cys Lys Asp Thr Ser
   1 5 10 15
   Thr Gly Val His Cys Ser Cys Pro Val Gly Phe Thr Leu Gln Leu Asp
   20 25 30
   Gly Lys Thr Cys
   35
<210> 1269
   <211> 41
   <212> PRT
   <213> Homo sapiens
<400> 1269
   Asp Ile Asp Glu Cys Gln Thr Arg Asn Gly Gly Cys Asp His Phe Cys
   1 5 10 15
   Lys Asn Ile Val Gly Ser Phe Asp Cys Gly Cys Lys Lys Gly Phe Lys
   20 25 30
   Leu Leu Thr Asp Glu Lys Ser Cys Gln
   35 40
<210> 1270
   <211> 39
   <212> PRT
   <213> Homo sapiens
<400> 1270
   Asp Val Asp Glu Cys Ser Leu Asp Arg Thr Cys Asp His Ser Cys Ile
   1 5 10 15
   Asn His Pro Gly Thr Phe Ala Cys Ala Cys Asn Arg Gly Tyr Thr Leu
   20 25 30
   Tyr Gly Phe Thr His Cys Gly
   35
<210> 1271
   <211> 41
   <212> PRT
   <213> Homo sapiens
<400> 1271
   Asp Thr Asn Glu Cys Ser Ile Asn Asn Gly Gly Cys Gln Gln Val Cys
   1 5 10 15
   Val Asn Thr Val Gly Ser Tyr Glu Cys Gln Cys His Pro Gly Tyr Lys
   20 25 30
   Leu His Trp Asn Lys Lys Asp Cys Val
   35 40
<210> 1272
   <211> 41
   <212> PRT
   <213> Homo sapiens
<400> 1272
   Asp Val Asp Glu Cys Val Glu Gly Thr Asp Asn Cys His Ile Asp Ala
   1               5                   10                  15
   Ile Cys Gln Asn Thr Pro Arg Ser Tyr Lys Cys Ile Cys Lys Ser Gly
               20                  25                  30
   Tyr Thr Gly Asp Gly Lys His Cys Lys
           35                  40
<210> 1273
   <211> 42
   <212> PRT
   <213> Homo sapiens
<400> 1273
   Asp Val Asp Glu Cys Glu Arg Glu Asp Asn Ala Gly Cys Val His Asp
   1               5                   10                  15
   Cys Val Asn Ile Pro Gly Asn Tyr Arg Cys Thr Cys Tyr Asp Gly Phe
               20                  25                  30
   His Leu Ala His Asp Gly His Asn Cys Leu
           35                  40
<210> 1274
   <211> 37
   <212> PRT
   <213> Homo sapiens
<400> 1274
   Asp Val Asp Glu Cys Ala Glu Gly Asn Gly Gly Cys Gln Gln Ser Cys
   1               5                   10                  15
   Val Asn Met Met Gly Ser Tyr Glu Cys His Cys Arg Glu Gly Phe Phe
               20                  25                  30
   Leu Ser Asp Asn Gln
           35
<210> 1275
   <211> 42
   <212> PRT
   <213> Homo sapiens
<400> 1275
   Glu Gly Met Asn Cys Met Asn Lys Asn His Gly Cys Ala His Ile Cys
   1               5                   10                  15
   Arg Glu Thr Pro Lys Gly Gly Ile Ala Cys Glu Cys Arg Pro Gly Phe
               20                  25                  30
   Glu Leu Thr Lys Asn Gln Arg Asp Cys Lys
           35                  40
<210> 1276
   <211> 39
   <212> PRT
   <213> Homo sapiens
<400> 1276
   Leu Thr Cys Asn Tyr Gly Asn Gly Gly Cys Gln His Thr Cys Asp Asp
   1               5                   10                  15
   Thr Glu Gln Gly Pro Arg Cys Gly Cys His Ile Lys Phe Val Leu His
               20                  25                  30
   Thr Asp Gly Lys Thr Cys Ile
           35
<210> 1277
   <211> 39
   <212> PRT
   <213> Homo sapiens
<400> 1277
   Glu Thr Cys Ala Val Asn Asn Gly Gly Cys Asp Ser Lys Cys His Asp
   1               5                   10                  15
   Ala Ala Thr Gly Val His Cys Thr Cys Pro Val Gly Phe Met Leu Gln
               20                  25                  30
   Pro Asp Arg Lys Thr Cys Lys
           35
<210> 1278
   <211> 41
   <212> PRT
   <213> Homo sapiens
<400> 1278
   Asp Ile Asp Glu Cys Arg Leu Asn Asn Gly Gly Cys Asp His Ile Cys
   1               5                   10                  15
   Arg Asn Thr Val Gly Ser Phe Glu Cys Ser Cys Lys Lys Gly Tyr Lys
               20                  25                  30
   Leu Leu Ile Asn Glu Arg Asn Cys Gln
           35                  40
<210> 1279
   <211> 39
   <212> PRT
   <213> Homo sapiens
<400> 1279
   Asp Ile Asp Glu Cys Ser Phe Asp Arg Thr Cys Asp His Ile Cys Val
   1               5                   10                  15
   Asn Thr Pro Gly Ser Phe Gln Cys Leu Cys His Arg Gly Tyr Leu Leu
               20                  25                  30
   Tyr Gly Ile Thr His Cys Gly
           35
<210> 1280
   <211> 42
   <212> PRT
   <213> Homo sapiens
<400> 1280
   Asp Val Asp Glu Cys Ser Ile Asn Arg Gly Gly Cys Arg Phe Gly Cys
   1 5 10 15
   Ile Asn Thr Pro Gly Ser Tyr Gln Cys Thr Cys Pro Ala Gly Gln Gly
   20 25 30
   Arg Leu His Trp Asn Gly Lys Asp Cys Thr
   35 40
<210> 1281
   <211> 36
   <212> PRT
   <213> Homo sapiens
<400> 1281
   Asp Leu Cys Leu Ser Ser Pro Cys Gln Asn Gln Gly Thr Cys His Asn
   1 5 10 15
   Asp Pro Leu Glu Val Tyr Arg Cys Ala Cys Pro Ser Gly Tyr Lys Gly
   20 25 30
   Arg Asp Cys Glu
   35
<210> 1282
   <211> 40
   <212> PRT
   <213> Homo sapiens
<400> 1282
   Ser Leu Asp Ser Cys Ser Ser Gly Pro Cys Glu Asn Gly Gly Thr Cys
   1 5 10 15
   His Ala Gln Glu Gly Glu Asp Ala Pro Phe Thr Cys Ser Cys Pro Thr
   20 25 30
   Gly Phe Glu Gly Pro Thr Cys Gly
   35 40
<210> 1283
   <211> 37
   <212> PRT
   <213> Homo sapiens
<400> 1283
   Asn Thr Asp Asp Cys Val Asp His Ala Cys Ala Asn Gly Gly Val Cys
   1 5 10 15
   Val Asp Gly Val Gly Asn Tyr Thr Cys Gln Cys Pro Leu Gln Tyr Glu
   20 25 30
   Gly Lys Ala Cys Glu
   35
<210> 1284
   <211> 39
   <212> PRT
   <213> Homo sapiens
<400> 1284
   Leu Val Asp Leu Cys Ser Pro Asp Leu Asn Pro Cys Gln His Glu Ala
   1               5                   10                  15
   Gln Cys Val Gly Thr Pro Asp Gly Pro Arg Cys Glu Cys Met Pro Gly
               20                  25                  30
   Tyr Ala Gly Asp Asn Cys Ser
           35
<210> 1285
   <211> 37
   <212> PRT
   <213> Homo sapiens
<400> 1285
   Asn Gln Asp Asp Cys Arg Asp His Arg Cys Gln Asn Gly Ala Gln Cys
   1               5                   10                  15
   Met Asp Glu Val Asn Ser Tyr Ser Cys Leu Cys Ala Glu Gly Tyr Ser
               20                  25                  30
   Gly Gln Leu Cys Glu
           35
<210> 1286
   <211> 37
   <212> PRT
   <213> Homo sapiens
<400> 1286
   Pro Lys Ser Pro Cys Glu Gly Thr Glu Cys Gln Asn Gly Ala Asn Cys
   1               5                   10                  15
   Val Asp Gln Gly Asn Arg Pro Val Cys Gln Cys Leu Pro Gly Phe Gly
               20                  25                  30
   Gly Pro Glu Cys Glu
           35
<210> 1287
   <211> 35
   <212> PRT
   <213> Homo sapiens
<400> 1287
   Glu Pro Cys Arg Lys Leu Tyr Cys Leu His Gly Ile Cys Gln Pro Asn
   1               5                   10                  15
   Ala Thr Pro Gly Pro Met Cys His Cys Glu Ala Gly Trp Val Gly Leu
               20                  25                  30
   His Cys Asp
           35
<210> 1288
   <211> 37
   <212> PRT
   <213> Homo sapiens
<400> 1288
   Ala Asp Gly Pro Cys His Gly His Lys Cys Val His Gly Gln Cys Val
   1 5 10 15
   Pro Leu Asp Ala Leu Ser Tyr Ser Cys Gln Cys Gln Asp Gly Tyr Ser
   20 25 30
   Gly Ala Leu Cys Asn
   35
<210> 1289
   <211> 37
   <212> PRT
   <213> Homo sapiens
<400> 1289
   Leu Ala Glu Pro Cys Arg Gly Leu Gln Cys Leu His Gly His Cys Gln
   1 5 10 15
   Ala Ser Gly Thr Lys Gly Ala His Cys Val Cys Asp Pro Gly Phe Ser
   20 25 30
   Gly Glu Leu Cys Glu
   35
<210> 1290
   <211> 38
   <212> PRT
   <213> Homo sapiens
<400> 1290
   Lys Cys Asn Pro Cys Leu Ser Asn Pro Cys Lys Asn Asp Gly Thr Cys
   1 5 10 15
   Asn Ser Asp Pro Val Asp Phe Tyr Arg Cys Thr Cys Pro Tyr Gly Phe
   20 25 30
   Lys Gly Gln Asp Cys Asp
   35
<210> 1291
   <211> 40
   <212> PRT
   <213> Homo sapiens
<400> 1291
   Pro Ile His Ala Cys Ile Ser Asn Pro Cys Lys His Gly Gly Thr Cys
   1 5 10 15
   His Leu Lys Glu Gly Glu Glu Asp Gly Phe Trp Cys Ile Cys Ala Asp
   20 25 30
   Gly Phe Glu Gly Glu Asn Cys Glu
   35 40
<210> 1292
   <211> 37
   <212> PRT
   <213> Homo sapiens
<400> 1292
   Asn Val Asp Asp Cys Glu Asp Asn Asp Cys Glu Asn Asn Ser Thr Cys
   1               5                   10                  15
   Val Asp Gly Ile Asn Asn Tyr Thr Cys Leu Cys Pro Pro Glu Tyr Thr
               20                  25                  30
   Gly Glu Leu Cys Glu
           35
<210> 1293
   <211> 39
   <212> PRT
   <213> Homo sapiens
<400> 1293
   Lys Leu Asp Phe Cys Ala Gln Asp Leu Asn Pro Cys Gln His Asp Ser
   1               5                   10                  15
   Lys Cys Ile Leu Thr Pro Lys Gly Phe Lys Cys Asp Cys Thr Pro Gly
               20                  25                  30
   Tyr Val Gly Glu His Cys Asp
   35
<210> 1294
   <211> 37
   <212> PRT
   <213> Homo sapiens
<400> 1294
   Asp Phe Asp Asp Cys Gln Asp Asn Lys Cys Lys Asn Gly Ala His Cys
   1               5                   10                  15
   Thr Asp Ala Val Asn Gly Tyr Thr Cys Ile Cys Pro Glu Gly Tyr Ser
               20                  25                  30
   Gly Leu Phe Cys Glu
           35
<210> 1295
   <211> 37
   <212> PRT
   <213> Homo sapiens
<400> 1295
   Arg Thr Ser Pro Cys Asp Asn Phe Asp Cys Gln Asn Gly Ala Gln Cys
   1               5                   10                  15
   Ile Val Arg Ile Asn Glu Pro Ile Cys Gln Cys Leu Pro Gly Tyr Gln
               20                  25                  30
   Gly Glu Lys Cys Glu
           35
<210> 1296
   <211> 37
   <212> PRT
   <213> Homo sapiens
<400> 1296
   Gly Cys Glu Pro Cys His Lys Lys Val Cys Ala His Gly Thr Cys Gln
   1 5 10 15
   Pro Ser Ser Gln Ala Gly Phe Thr Cys Glu Cys Gln Glu Gly Trp Met
   20 25 30
   Gly Pro Leu Cys Asp
   35
<210> 1297
   <211> 36
   <212> PRT
   <213> Homo sapiens
<400> 1297
   Asn Ala Cys Leu Ser Ser Pro Cys Lys Asn Asn Gly Thr Cys Thr Gln
   1 5 10 15
   Asp Pro Val Glu Leu Tyr Arg Cys Ala Cys Pro Tyr Ser Tyr Lys Gly
   20 25 30
   Lys Asp Cys Thr
   35
<210> 1298
   <211> 40
   <212> PRT
   <213> Homo sapiens
<400> 1298
   Pro Ile Asn Thr Cys Ile Gln Asn Pro Cys Gln His Gly Gly Thr Cys
   1 5 10 15
   His Leu Ser Asp Ser His Lys Asp Gly Phe Ser Cys Ser Cys Pro Leu
   20 25 30
   Gly Phe Glu Gly Gln Arg Cys Glu
   35 40
<210> 1299
   <211> 37
   <212> PRT
   <213> Homo sapiens
<400> 1299
   Asn Pro Asp Asp Cys Glu Asp Asn Asp Cys Glu Asn Asn Ala Thr Cys
   1 5 10 15
   Val Asp Gly Ile Asn Asn Tyr Val Cys Ile Cys Pro Pro Asn Tyr Thr
   20 25 30
   Gly Glu Leu Cys Asp
   35
<210> 1300
   <211> 39
   <212> PRT
   <213> Homo sapiens
<400> 1300
   Val Ile Asp His Cys Val Pro Glu Leu Asn Leu Cys Gln His Glu Ala
   1               5                   10                  15
   Lys Cys Ile Pro Leu Asp Lys Gly Phe Ser Cys Glu Cys Val Pro Gly
               20                  25                  30
   Tyr Ser Gly Lys Leu Cys Glu
           35
<210> 1301
   <211> 37
   <212> PRT
   <213> Homo sapiens
<400> 1301
   Asp Asn Asp Asp Cys Val Ala His Lys Cys Arg His Gly Ala Gln Cys
   1               5                   10                  15
   Val Asp Thr Ile Asn Gly Tyr Thr Cys Thr Cys Pro Gln Gly Phe Ser
               20                  25                  30
   Gly Pro Phe Cys Glu
           35
<210> 1302
   <211> 37
   <212> PRT
   <213> Homo sapiens
<400> 1302
   Gln Thr Ser Pro Cys Asp Gln Tyr Glu Cys Gln Asn Gly Ala Gln Cys
   1               5                   10                  15
   Ile Val Val Gln Gln Glu Pro Thr Cys Arg Cys Pro Pro Gly Phe Ala
               20                  25                  30
   Gly Pro Arg Cys Glu
           35
<210> 1303
   <211> 26
   <212> PRT
   <213> Homo sapiens
<400> 1303
   His Gly Leu Cys Arg Ser Val Glu Lys Asp Ser Val Val Cys Glu Cys
   1               5                   10                  15
   Arg Pro Gly Trp Thr Gly Pro Leu Cys Asp
               20                  25
<210> 1304
   <211> 36
   <212> PRT
   <213> Homo sapiens
<400> 1304
   Ala Arg Asp Pro Cys Leu Gly His Arg Cys His His Gly Lys Cys Val
   1               5                   10                  15
   Ala Thr Gly Thr Ser Tyr Met Cys Lys Cys Ala Glu Gly Tyr Gly Gly
               20                  25                  30
   Asp Leu Cys Asp
           35
<210> 1305
   <211> 37
   <212> PRT
   <213> Homo sapiens
<400> 1305
   Ser Ala Asn Ala Cys Ser Ala Phe Lys Cys His His Gly Gln Cys His
   1               5                   10                  15
   Ile Ser Asp Gln Gly Glu Pro Tyr Cys Leu Cys Gln Pro Gly Phe Ser
               20                  25                  30
   Gly Glu His Cys Gln
           35
<210> 1306
   <211> 47
   <212> PRT
   <213> Homo sapiens
<400> 1306
   Cys Ser Leu Leu Cys Leu Pro Lys Ala Asn Asn Ser Arg Ser Cys Arg
   1               5                   10                  15
   Cys Pro Glu Asp Val Ser Ser Ser Val Leu Pro Ser Gly Asp Leu Met
               20                  25                  30
   Cys Asp Cys Pro Gln Gly Tyr Gln Leu Lys Asn Asn Thr Cys Val
           35                  40                  45
<210> 1307
   <211> 39
   <212> PRT
   <213> Homo sapiens
<400> 1307
   His Glu Cys Pro Gly Gly Ala Glu Thr Pro Cys Asn Gly His Gly Thr
   1               5                   10                  15
   Cys Leu Asp Gly Met Asp Arg Asn Gly Thr Cys Val Cys Gln Glu Asn
               20                  25                  30
   Phe Arg Gly Ser Ala Cys Gln
           35
<210> 1308
   <211> 39
   <212> PRT
   <213> Homo sapiens
<400> 1308
   Thr Ser Pro Gln Cys Pro Arg Lys Cys Asp Pro Asn Ala Asn Cys Val
   1               5                   10                  15
   Gln Asp Ser Ala Gly Ala Ser Thr Cys Ala Cys Ala Ala Gly Tyr Ser
               20                  25                  30
   Gly Asn Gly Ile Phe Cys Ser
           35
<210> 1309
   <211> 42
   <212> PRT
   <213> Homo sapiens
<400> 1309
   Glu Val Asp Pro Cys Ala His Gly His Gly Gly Cys Ser Pro His Ala
   1               5                   10                  15
   Asn Cys Thr Lys Val Ala Pro Gly Gln Arg Thr Cys Thr Cys Gln Asp
               20                  25                  30
   Gly Tyr Met Gly Asp Gly Glu Leu Cys Gln
           35                  40
<210> 1310
   <211> 43
   <212> PRT
   <213> Homo sapiens
<400> 1310
   Glu Ile Asn Ser Cys Leu Ile His His Gly Gly Cys His Ile His Ala
   1               5                   10                  15
   Glu Cys Ile Pro Thr Gly Pro Gln Gln Val Ser Cys Ser Cys Arg Glu
               20                  25                  30
   Gly Tyr Ser Gly Asp Gly Ile Arg Thr Cys Glu
           35                  40
<210> 1311
   <211> 43
   <212> PRT
   <213> Homo sapiens
<400> 1311
   Leu Leu Asp Pro Cys Ser Lys Asn Asn Gly Gly Cys Ser Pro Tyr Ala
   1               5                   10                  15
   Thr Cys Lys Ser Thr Gly Asp Gly Gln Arg Thr Cys Thr Cys Asp Thr
               20                  25                  30
   Ala His Thr Val Gly Asp Gly Leu Thr Cys Arg
           35                  40
<210> 1312
   <211> 35
   <212> PRT
   <213> Homo sapiens
<400> 1312
   Leu Gln Pro Val Cys Thr Pro Pro Cys Ala Pro Glu Ala Val Cys Arg
   1               5                   10                  15
   Ala Gly Asn Ser Cys Glu Cys Ser Leu Gly Tyr Glu Gly Asp Gly Arg
               20                  25                  30
   Val Cys Thr
           35
<210> 1313
   <211> 41
   <212> PRT
   <213> Homo sapiens
<400> 1313
   Val Ala Asp Leu Cys Gln Asp Gly His Gly Gly Cys Ser Glu His Ala
   1               5                   10                  15
   Asn Cys Ser Gln Val Gly Thr Met Val Thr Cys Thr Cys Leu Pro Asp
               20                  25                  30
   Tyr Glu Gly Asp Gly Trp Ser Cys Arg
           35                  40
<210> 1314
   <211> 43
   <212> PRT
   <213> Homo sapiens
<400> 1314
   Ala Arg Asn Pro Cys Thr Asp Gly His Arg Gly Gly Cys Ser Glu His
   1               5                   10                  15
   Ala Asn Cys Leu Ser Thr Gly Leu Asn Thr Arg Arg Cys Glu Cys His
               20                  25                  30
   Ala Gly Tyr Val Gly Asp Gly Leu Gln Cys Leu
           35                  40
<210> 1315
   <211> 38
   <212> PRT
   <213> Homo sapiens
<400> 1315
   Phe Gly Pro Asp Cys Gln Ser Val Cys Ser Cys Val His Gly Val Cys
   1               5                   10                  15
   Asn His Gly Pro Arg Gly Asp Gly Ser Cys Leu Cys Phe Ala Gly Tyr
               20                  25                  30
   Thr Gly Pro His Cys Asp
           35
<210> 1316
   <211> 35
   <212> PRT
   <213> Homo sapiens
<400> 1316
   Glu Leu Pro Val Cys Gln Glu Leu Arg Cys Pro Gln Asn Thr Gln Cys
   1               5                   10                  15
   Ser Ala Glu Ala Pro Ser Cys Arg Cys Leu Pro Gly Tyr Thr Gln Gln
               20                  25                  30
   Gly Ser Glu
           35
<210> 1317
   <211> 40
   <212> PRT
   <213> Homo sapiens
<400> 1317
   Ala Pro Asn Pro Cys Trp Pro Ser Pro Cys Ser Leu Leu Ala Gln Cys
   1               5                   10                  15
   Ser Val Ser Pro Lys Gly Gln Ala Gln Cys His Cys Pro Glu Asn Tyr
               20                  25                  30
   His Gly Asp Gly Met Val Cys Leu
           35                  40
<210> 1318
   <211> 41
   <212> PRT
   <213> Homo sapiens
<400> 1318
   Asp Cys Thr Gln Cys Pro Gly Gly Phe Ser Asn Pro Cys Tyr Gly Lys
   1               5                   10                  15
   Gly Asn Cys Ser Asp Gly Ile Gln Gly Asn Gly Ala Cys Leu Cys Phe
               20                  25                  30
   Pro Asp Tyr Lys Gly Ile Ala Cys His
           35                  40
<210> 1319
   <211> 41
   <212> PRT
   <213> Homo sapiens
<400> 1319
   Ser Met Gly Asp Cys Gly Pro Thr Gly Leu Ala Gln His Cys His Leu
   1               5                   10                  15
   His Ala Arg Cys Val Ser Gln Glu Gly Val Ala Arg Cys Arg Cys Leu
               20                  25                  30
   Asp Gly Phe Glu Gly Asp Gly Phe Ser
           35                  40
<210> 1320
   <211> 43
   <212> PRT
   <213> Homo sapiens
<400> 1320
   Pro Ser Asn Pro Cys Ser His Pro Asp Arg Gly Gly Cys Ser Glu Asn
   1               5                   10                  15
   Ala Glu Cys Val Pro Gly Ser Leu Gly Thr His His Cys Thr Cys His
               20                  25                  30
   Lys Gly Trp Ser Gly Asp Gly Arg Val Cys Val
           35                  40
<210> 1321
   <211> 43
   <212> PRT
   <213> Homo sapiens
<400> 1321
   Ala Ile Asp Glu Cys Glu Leu Asp Met Arg Gly Gly Cys His Thr Asp
   1               5                   10                  15
   Ala Leu Cys Ser Tyr Val Gly Pro Gly Gln Ser Arg Cys Thr Cys Lys
               20                  25                  30
   Leu Gly Phe Ala Gly Asp Gly Tyr Gln Cys Ser
           35                  40
<210> 1322
   <211> 40
   <212> PRT
   <213> Homo sapiens
<400> 1322
   Pro Ile Asp Pro Cys Arg Ala Gly Asn Gly Gly Cys His Gly Leu Ala
   1               5                   10                  15
   Thr Cys Arg Ala Val Gly Gly Gly Gln Arg Val Cys Thr Cys Pro Pro
               20                  25                  30
   Gly Phe Gly Gly Asp Gly Phe Ser
           35                  40
<210> 1323
   <211> 41
   <212> PRT
   <213> Homo sapiens
<400> 1323
   Asp Cys Ile Glu Cys Pro Gly Gly Ala Gly Ser Pro Cys Asn Gly Arg
   1               5                   10                  15
   Gly Ser Cys Ala Glu Gly Met Glu Gly Asn Gly Thr Cys Ser Cys Gln
               20                  25                  30
   Glu Gly Phe Gly Gly Thr Ala Cys Glu
           35                  40
<210> 1324
   <211> 40
   <212> PRT
   <213> Homo sapiens
<400> 1324
   Pro Ile Thr Ser Cys Leu Glu Gln Thr Gly Lys Cys His Pro Leu Ala
   1               5                   10                  15
   Ser Cys Gln Ser Thr Ser Ser Gly Val Trp Ser Cys Val Cys Gln Glu
               20                  25                  30
   Gly Tyr Glu Gly Asp Gly Phe Leu
           35                  40
<210> 1325
   <211> 39
   <212> PRT
   <213> Homo sapiens
<400> 1325
   Thr Glu Asp Asn Cys Asn Gly Thr Cys His Thr Ser Ala Asn Cys Leu
   1               5                   10                  15
   Thr Asn Ser Asp Gly Thr Ala Ser Cys Lys Cys Ala Ala Gly Phe Gln
               20                  25                  30
   Gly Asn Gly Thr Ile Cys Thr
           35
<210> 1326
   <211> 42
   <212> PRT
   <213> Homo sapiens
<400> 1326
   Ala Ile Asn Ala Cys Glu Ile Ser Asn Gly Gly Cys Ser Ala Lys Ala
   1               5                   10                  15
   Asp Cys Lys Arg Thr Thr Pro Gly Arg Arg Val Cys Thr Cys Lys Ala
               20                  25                  30
   Gly Tyr Thr Gly Asp Gly Ile Val Cys Leu
           35                  40
<210> 1327
   <211> 42
   <212> PRT
   <213> Homo sapiens
<400> 1327
   Glu Ile Asn Pro Cys Leu Glu Asn His Gly Gly Cys Asp Lys Asn Ala
   1               5                   10                  15
   Glu Cys Thr Gln Thr Gly Pro Asn Gln Ala Ala Cys Asn Cys Leu Pro
               20                  25                  30
   Ala Tyr Thr Gly Asp Gly Lys Val Cys Thr
           35                  40
<210> 1328
   <211> 40
   <212> PRT
   <213> Homo sapiens
<400> 1328
   Leu Ile Asn Val Cys Leu Thr Lys Asn Gly Gly Cys Ser Glu Phe Ala
   1               5                   10                  15
   Ile Cys Asn His Thr Gly Gln Val Glu Arg Thr Cys Thr Cys Lys Pro
               20                  25                  30
   Asn Tyr Ile Gly Asp Gly Phe Thr
           35                  40
<210> 1329
   <211> 35
   <212> PRT
   <213> Homo sapiens
<400> 1329
   Leu Pro Ala Val Cys Thr Pro Pro Cys Ser Ala His Ala Thr Cys Lys
   1               5                   10                  15
   Glu Asn Asn Thr Cys Glu Cys Asn Leu Asp Tyr Glu Gly Asp Gly Ile
               20                  25                  30
   Thr Cys Thr
           35
<210> 1330
   <211> 41
   <212> PRT
   <213> Homo sapiens
<400> 1330
   Val Val Asp Phe Cys Lys Gln Asp Asn Gly Gly Cys Ala Lys Val Ala
   1               5                   10                  15
   Arg Cys Ser Gln Lys Gly Thr Lys Val Ser Cys Ser Cys Gln Lys Gly
               20                  25                  30
   Tyr Lys Gly Asp Gly His Ser Cys Thr
           35                  40
<210> 1331
   <211> 43
   <212> PRT
   <213> Homo sapiens
<400> 1331
   Glu Ile Asp Pro Cys Ala Asp Gly Leu Asn Gly Gly Cys His Glu His
   1               5                   10                  15
   Ala Thr Cys Lys Met Thr Gly Pro Gly Lys His Lys Cys Glu Cys Lys
               20                  25                  30
   Ser His Tyr Val Gly Asp Gly Leu Asn Cys Glu
           35                  40
<210> 1332
   <211> 38
   <212> PRT
   <213> Homo sapiens
<400> 1332
   Phe Gly Pro Ser Cys Ser Ser Val Cys Asn Cys Val His Gly Val Cys
   1               5                   10                  15
   Asn Ser Gly Leu Asp Gly Asp Gly Thr Cys Glu Cys Tyr Ser Ala Tyr
               20                  25                  30
   Thr Gly Pro Lys Cys Asp
           35
<210> 1333
   <211> 42
   <212> PRT
   <213> Homo sapiens
<400> 1333
   Pro Ile Pro Glu Cys Ala Ala Leu Leu Cys Pro Glu Asn Ser Arg Cys
   1               5                   10                  15
   Ser Pro Ser Thr Glu Asp Glu Asn Lys Leu Glu Cys Lys Cys Leu Pro
               20                  25                  30
   Asn Tyr Arg Gly Asp Gly Lys Tyr Cys Asp
           35                  40
<210> 1334
   <211> 40
   <212> PRT
   <213> Homo sapiens
<400> 1334
   Pro Ile Asn Pro Cys Leu Arg Lys Ile Cys His Pro His Ala His Cys
   1               5                   10                  15
   Thr Tyr Leu Gly Pro Asn Arg His Ser Cys Thr Cys Gln Glu Gly Tyr
               20                  25                  30
   Arg Gly Asp Gly Gln Val Cys Leu
           35                  40
<210> 1335
   <211> 41
   <212> PRT
   <213> Homo sapiens
<400> 1335
   Met Thr Asp Ile Cys Lys Ser Asp Asn Pro Cys His Arg Asn Ala Asn
   1               5                   10                  15
   Cys Thr Thr Val Ala Pro Gly Arg Thr Glu Cys Ile Cys Gln Lys Gly
               20                  25                  30
   Tyr Val Gly Asp Gly Leu Thr Cys Tyr
           35                  40
<210> 1336
   <211> 41
   <212> PRT
   <213> Homo sapiens
<400> 1336
   Asp Cys Asn Gln Cys Pro Gly Gly Phe Ser Asn Pro Cys Ser Gly Asn
   1               5                   10                  15
   Gly Gln Cys Ala Asp Ser Leu Gly Gly Asn Gly Thr Cys Ile Cys Glu
               20                  25                  30
   Glu Gly Phe Gln Gly Ser Gln Cys Gln
           35                  40
<210> 1337
   <211> 41
   <212> PRT
   <213> Homo sapiens
<400> 1337
   Gln Thr Ser Ala Cys Gly Pro Tyr Val Gln Phe Cys His Ile His Ala
   1               5                   10                  15
   Thr Cys Glu Tyr Ser Asn Gly Thr Ala Ser Cys Ile Cys Lys Ala Gly
               20                  25                  30
   Tyr Glu Gly Asp Gly Thr Leu Cys Ser
           35                  40
<210> 1338
   <211> 43
   <212> PRT
   <213> Homo sapiens
<400> 1338
   Glu Met Asp Pro Cys Thr Gly Leu Thr Pro Gly Gly Cys Ser Arg Asn
   1               5                   10                  15
   Ala Glu Cys Ile Lys Thr Gly Thr Gly Thr His Thr Cys Val Cys Gln
               20                  25                  30
   Gln Gly Trp Thr Gly Asn Gly Arg Asp Cys Ser
           35                  40
<210> 1339
   <211> 43
   <212> PRT
   <213> Homo sapiens
<400> 1339
   Glu Ile Asn Asn Cys Leu Leu Pro Ser Ala Gly Gly Cys His Asp Asn
   1               5                   10                  15
   Ala Ser Cys Leu Tyr Val Gly Pro Gly Gln Asn Glu Cys Glu Cys Lys
               20                  25                  30
   Lys Gly Phe Arg Gly Asn Gly Ile Asp Cys Glu
           35                  40
<210> 1340
   <211> 38
   <212> PRT
   <213> Homo sapiens
<400> 1340
   Gly Leu Asp Val Cys Ala Thr Cys His Glu His Ala Thr Cys Gln Gln
   1               5                   10                  15
   Arg Glu Gly Lys Lys Ile Cys Ile Cys Asn Tyr Gly Phe Val Gly Asn
               20                  25                  30
   Gly Arg Thr Gln Cys Val
           35
<210> 1341
   <211> 40
   <212> PRT
   <213> Homo sapiens
<400> 1341
   Asp Lys Asn Glu Cys Gln Phe Gly Ala Thr Leu Val Cys Gly Asn His
   1               5                   10                  15
   Thr Ser Cys His Asn Thr Pro Gly Gly Phe Tyr Cys Ile Cys Leu Glu
               20                  25                  30
   Gly Tyr Arg Ala Thr Asn Asn Asn
           35                  40
<210> 1342
   <211> 38
   <212> PRT
   <213> Homo sapiens
<400> 1342
   Asp Ile Asp Glu Cys Glu Val Ser Gly Leu Cys Arg His Gly Gly Arg
   1               5                   10                  15
   Cys Val Asn Thr His Gly Ser Phe Glu Cys Tyr Cys Met Asp Gly Tyr
               20                  25                  30
   Leu Pro Arg Asn Gly Pro
           35
<210> 1343
   <211> 37
   <212> PRT
   <213> Homo sapiens
<400> 1343
   Val Phe His Glu Cys Phe Phe Asn Pro Cys His Asn Ser Gly Thr Cys
   1               5                   10                  15
   Gln Gln Leu Gly Arg Gly Tyr Val Cys Leu Cys Pro Leu Gly Tyr Thr
               20                  25                  30
   Gly Leu Lys Cys Glu
           35
<210> 1344
   <211> 37
   <212> PRT
   <213> Homo sapiens
<400> 1344
   Asp Ile Asp Glu Cys Ser Pro Leu Pro Cys Leu Asn Asn Gly Val Cys
   1               5                   10                  15
   Lys Asp Leu Val Gly Glu Phe Ile Cys Glu Cys Pro Ser Gly Tyr Thr
               20                  25                  30
   Gly Gln Arg Cys Glu
           35
<210> 1345
   <211> 37
   <212> PRT
   <213> Homo sapiens
<400> 1345
   Asn Ile Asn Glu Cys Ser Ser Ser Pro Cys Leu Asn Lys Gly Ile Cys
   1               5                   10                  15
   Val Asp Gly Val Ala Gly Tyr Arg Cys Thr Cys Val Lys Gly Phe Val
               20                  25                  30
   Gly Leu His Cys Glu
           35
<210> 1346
   <211> 37
   <212> PRT
   <213> Homo sapiens
<400> 1346
   Glu Val Asn Glu Cys Gln Ser Asn Pro Cys Leu Asn Asn Ala Val Cys
   1               5                   10                  15
   Glu Asp Gln Val Gly Gly Phe Leu Cys Lys Cys Pro Pro Gly Phe Leu
               20                  25                  30
   Gly Thr Arg Cys Gly
           35
<210> 1347
   <211> 37
   <212> PRT
   <213> Homo sapiens
<400> 1347
   Asn Val Asp Glu Cys Leu Ser Gln Pro Cys Lys Asn Gly Ala Thr Cys
   1               5                   10                  15
   Lys Asp Gly Ala Asn Ser Phe Arg Cys Leu Cys Ala Ala Gly Phe Thr
               20                  25                  30
   Gly Ser His Cys Glu
           35
<210> 1348
   <211> 37
   <212> PRT
   <213> Homo sapiens
<400> 1348
   Asn Ile Asn Glu Cys Gln Ser Asn Pro Cys Arg Asn Gln Ala Thr Cys
   1               5                   10                  15
   Val Asp Glu Leu Asn Ser Tyr Ser Cys Lys Cys Gln Pro Gly Phe Ser
               20                  25                  30
   Gly Lys Arg Cys Glu
           35
<210> 1349
   <211> 40
   <212> PRT
   <213> Homo sapiens
<400> 1349
   Asp Val Asp Glu Cys Ala Val Gly Ser Asp Cys Ser Glu His Ala Ser
   1               5                   10                  15
   Cys Leu Asn Val Asp Gly Ser Tyr Ile Cys Ser Cys Val Pro Pro Tyr
               20                  25                  30
   Thr Gly Asp Gly Lys Asn Cys Ala
           35                  40
<210> 1350
   <211> 33
   <212> PRT
   <213> Homo sapiens
<400> 1350
   Glu Glu Pro Ile Cys Ile Leu Pro Cys Leu Asn Gly Gly Arg Cys Val
   1               5                   10                  15
   Ala Pro Tyr Gln Cys Asp Cys Pro Pro Gly Trp Thr Gly Ser Arg Cys
               20                  25                  30
   His
<210> 1351
   <211> 32
   <212> PRT
   <213> Homo sapiens
<400> 1351
   Thr Ala Val Cys Gln Ser Pro Cys Leu Asn Gly Gly Lys Cys Val Arg
   1               5                   10                  15
   Pro Asn Arg Cys His Cys Leu Ser Ser Trp Thr Gly His Asn Cys Ser
               20                  25                  30
<210> 1352
   <211> 32
   <212> PRT
   <213> Homo sapiens
<400> 1352
   Ser Glu Pro Glu Cys Pro Gly Asn Cys His Leu Arg Gly Arg Cys Ile
   1               5                   10                  15
   Asp Gly Gln Cys Ile Cys Asp Asp Gly Phe Thr Gly Glu Asp Cys Ser
               20                  25                  30
<210> 1353
   <211> 32
   <212> PRT
   <213> Homo sapiens
<400> 1353
   Ser Asp Met Ser Cys Pro Asn Asp Cys His Gln His Gly Arg Cys Val
   1               5                   10                  15
   Asn Gly Met Cys Val Cys Asp Asp Gly Tyr Thr Gly Glu Asp Cys Arg
               20                  25                  30
<210> 1354
   <211> 32
   <212> PRT
   <213> Homo sapiens
<400> 1354
   Arg Asp Arg Gln Cys Pro Arg Asp Cys Ser Asn Arg Gly Leu Cys Val
   1               5                   10                  15
   Asp Gly Gln Cys Val Cys Glu Asp Gly Phe Thr Gly Pro Asp Cys Ala
               20                  25                  30
<210> 1355
   <211> 32
   <212> PRT
   <213> Homo sapiens
<400> 1355
   Ala Glu Leu Ser Cys Pro Asn Asp Cys His Gly Gln Gly Arg Cys Val
   1               5                   10                  15
   Asn Gly Gln Cys Val Cys His Glu Gly Phe Met Gly Lys Asp Cys Lys
               20                  25                  30
<210> 1356
   <211> 32
   <212> PRT
   <213> Homo sapiens
<400> 1356
   Lys Glu Gln Arg Cys Pro Ser Asp Cys His Gly Gln Gly Arg Cys Val
   1               5                   10                  15
   Asp Gly Gln Cys Ile Cys His Glu Gly Phe Thr Gly Leu Asp Cys Gly
               20                  25                  30
<210> 1357
   <211> 32
   <212> PRT
   <213> Homo sapiens
<400> 1357
   Gly Gln His Ser Cys Pro Ser Asp Cys Asn Asn Leu Gly Gln Cys Val
   1 5 10 15
   Ser Gly Arg Cys Ile Cys Asn Glu Gly Tyr Ser Gly Glu Asp Cys Ser
   20 25 30
<210> 1358
   <211> 32
   <212> PRT
   <213> Homo sapiens
<400> 1358
   Ser Gln Leu Ala Cys Pro Ser Asp Cys Asn Asp Gln Gly Lys Cys Val
   1 5 10 15
   Asn Gly Val Cys Ile Cys Phe Glu Gly Tyr Ala Gly Ala Asp Cys Ser
   20 25 30
<210> 1359
   <211> 33
   <212> PRT
   <213> Homo sapiens
<400> 1359
   Ser Arg Glu Ile Cys Pro Val Pro Cys Ser Glu Glu His Gly Thr Cys
   1 5 10 15
   Val Asp Gly Leu Cys Val Cys His Asp Gly Phe Ala Gly Asp Asp Cys
   20 25 30
   Asn
<210> 1360
   <211> 32
   <212> PRT
   <213> Homo sapiens
<400> 1360
   Asn Lys Pro Leu Cys Leu Asn Asn Cys Tyr Asn Arg Gly Arg Cys Val
   1 5 10 15
   Glu Asn Glu Cys Val Cys Asp Glu Gly Phe Thr Gly Glu Asp Cys Ser
   20 25 30
<210> 1361
   <211> 32
   <212> PRT
   <213> Homo sapiens
<400> 1361
   Ser Glu Leu Ile Cys Pro Asn Asp Cys Phe Asp Arg Gly Arg Cys Ile
   1 5 10 15
   Asn Gly Thr Cys Tyr Cys Glu Glu Gly Phe Thr Gly Glu Asp Cys Gly
   20 25 30
<210> 1362
   <211> 32
   <212> PRT
   <213> Homo sapiens
<400> 1362
   Gly Lys Pro Thr Cys Pro His Ala Cys His Thr Gln Gly Arg Cys Glu
   1               5                   10                  15
   Glu Gly Gln Cys Val Cys Asp Glu Gly Phe Ala Gly Val Asp Cys Ser
               20                  25                  30
<210> 1363
   <211> 32
   <212> PRT
   <213> Homo sapiens
<400> 1363
   Ser Glu Lys Arg Cys Pro Ala Asp Cys His Asn Arg Gly Arg Cys Val
   1               5                   10                  15
   Asp Gly Arg Cys Glu Cys Asp Asp Gly Phe Thr Gly Ala Asp Cys Gly
               20                  25                  30
<210> 1364
   <211> 32
   <212> PRT
   <213> Homo sapiens
<400> 1364
   Gly Glu Leu Lys Cys Pro Asn Gly Cys Ser Gly His Gly Arg Cys Val
   1               5                   10                  15
   Asn Gly Gln Cys Val Cys Asp Glu Gly Tyr Thr Gly Glu Asp Cys Ser
               20                  25                  30
<210> 1365
   <211> 32
   <212> PRT
   <213> Homo sapiens
<400> 1365
   Ser Gln Leu Arg Cys Pro Asn Asp Cys His Ser Arg Gly Arg Cys Val
   1               5                   10                  15
   Glu Gly Lys Cys Val Cys Glu Gln Gly Phe Lys Gly Tyr Asp Cys Ser
               20                  25                  30
<210> 1366
   <211> 32
   <212> PRT
   <213> Homo sapiens
<400> 1366
   Glu Arg Leu Ala Cys Pro Gly Ala Cys Ser Gly His Gly Arg Cys Val
   1               5                   10                  15
   Asp Gly Arg Cys Leu Cys His Glu Pro Tyr Val Gly Ala Asp Cys Gly
               20                  25                  30
<210> 1367
   <211> 31
   <212> PRT
   <213> Homo sapiens
<400> 1367
   Tyr Pro Ala Cys Pro Glu Asn Cys Ser Gly His Gly Glu Cys Val Arg
   1 5 10 15
   Gly Val Cys Gln Cys His Glu Asp Phe Met Ser Glu Asp Cys Ser
   20 25 30
<210> 1368
   <211> 31
   <212> PRT
   <213> Homo sapiens
<400> 1368
   Glu Lys Arg Cys Pro Gly Asp Cys Ser Gly His Gly Phe Cys Asp Thr
   1 5 10 15
   Gly Glu Cys Tyr Cys Glu Glu Gly Phe Thr Gly Leu Asp Cys Ala
   20 25 30
<210> 1369
   <211> 31
   <212> PRT
   <213> Homo sapiens
<400> 1369
   Ala Ser Gly Ser Cys Pro Asp Asp Cys Asn Asp Gln Gly Arg Cys Val
   1 5 10 15
   Arg Gly Arg Cys Val Cys Phe Pro Gly Tyr Thr Gly Pro Ser Cys
   20 25 30
<210> 1370
   <211> 31
   <212> PRT
   <213> Homo sapiens
<400> 1370
   Gly Val Arg Ser Cys Pro Gly Asp Cys Arg Gly Arg Gly Arg Cys Glu
   1 5 10 15
   Ser Gly Arg Cys Met Cys Trp Pro Gly Tyr Thr Gly Arg Asp Cys
   20 25 30
<210> 1371
   <211> 31
   <212> PRT
   <213> Homo sapiens
<400> 1371
   Gly Thr Arg Ala Cys Pro Gly Asp Cys Arg Gly Arg Gly Arg Cys Val
   1 5 10 15
   Asp Gly Arg Cys Val Cys Asn Pro Gly Phe Thr Gly Glu Asp Cys
   20 25 30
<210> 1372
   <211> 31
   <212> PRT
   <213> Homo sapiens
<400> 1372
   Gly Ser Arg Arg Cys Pro Gly Asp Cys Arg Gly His Gly Leu Cys Glu
   1 5 10 15
   Asp Gly Val Cys Val Cys Asp Ala Gly Tyr Ser Gly Glu Asp Cys
   20 25 30
<210> 1373
   <211> 31
   <212> PRT
   <213> Homo sapiens
<400> 1373
   Ser Thr Arg Ser Cys Pro Gly Gly Cys Arg Gly Arg Gly Gln Cys Leu
   1 5 10 15
   Asp Gly Arg Cys Val Cys Glu Asp Gly Tyr Ser Gly Glu Asp Cys
   20 25 30
<210> 1374
   <211> 31
   <212> PRT
   <213> Homo sapiens
<400> 1374
   Gly Val Arg Gln Cys Pro Asn Asp Cys Ser Gln His Gly Val Cys Gln
   1 5 10 15
   Asp Gly Val Cys Ile Cys Trp Glu Gly Tyr Val Ser Glu Asp Cys
   20 25 30
<210> 1375
   <211> 31
   <212> PRT
   <213> Homo sapiens
<400> 1375
   Ser Ile Arg Thr Cys Pro Ser Asn Cys His Gly Arg Gly Arg Cys Glu
   1 5 10 15
   Glu Gly Arg Cys Leu Cys Asp Pro Gly Tyr Thr Gly Pro Thr Cys
   20 25 30
<210> 1376
   <211> 32
   <212> PRT
   <213> Homo sapiens
<400> 1376
   Ala Thr Arg Met Cys Pro Ala Asp Cys Arg Gly Arg Gly Arg Cys Val
   1               5                   10                  15
   Gln Gly Val Cys Leu Cys His Val Gly Tyr Gly Gly Glu Asp Cys Gly
               20                  25                  30
<210> 1377
   <211> 31
   <212> PRT
   <213> Homo sapiens
   400> 1377
<210> 1378
   <211> 32
   <212> PRT
   <213> Homo sapiens
<400> 1378
   Ala Ile Gln Thr Cys Pro Gly Asp Cys Arg Gly Arg Gly Glu Cys His
   1               5                   10                  15
   Asp Gly Ser Cys Val Cys Lys Asp Gly Tyr Ala Gly Glu Asp Cys Gly
               20                  25                  30
<210> 1379
   <211> 31
   <212> PRT
   <213> Homo sapiens
<400> 1379
   Gly Trp Pro Ser Cys Pro Gly Asp Cys Gln Gly Arg Gly Arg Cys Val
   1               5                   10                  15
   Gln Gly Val Cys Val Cys Arg Ala Gly Phe Ser Gly Pro Asp Cys
               20                  25                  30
<210> 1380
   <211> 31
   <212> PRT
   <213> Homo sapiens
<400> 1380
   Ser Gln Arg Ser Cys Pro Arg Gly Cys Ser Gln Arg Gly Arg Cys Glu
   1               5                   10                  15
   Gly Gly Arg Cys Val Cys Asp Pro Gly Tyr Thr Gly Asp Asp Cys
               20                  25                  30
<210> 1381
   <211> 31
   <212> PRT
   <213> Homo sapiens
<400> 1381
   Gly Met Arg Ser Cys Pro Arg Gly Cys Ser Gln Arg Gly Arg Cys Glu
   1               5                   10                  15
   Asn Gly Arg Cys Val Cys Asn Pro Gly Tyr Thr Gly Glu Asp Cys
               20                  25                  30
<210> 1382
   <211> 31
   <212> PRT
   <213> Homo sapiens
<400> 1382
   Gly Val Arg Ser Cys Pro Arg Gly Cys Ser Gln Arg Gly Arg Cys Lys
   1               5                   10                  15
   Asp Gly Arg Cys Val Cys Asp Pro Gly Tyr Thr Gly Glu Asp Cys
               20                  25                  30
<210> 1383
   <211> 31
   <212> PRT
   <213> Homo sapiens
<400> 1383
   Gly Thr Arg Ser Cys Pro Trp Asp Cys Gly Glu Gly Gly Arg Cys Val
   1               5                   10                  15
   Asp Gly Arg Cys Val Cys Trp Pro Gly Tyr Thr Gly Glu Asp Cys
               20                  25                  30
<210> 1384
   <211> 31
   <212> PRT
   <213> Homo sapiens
<400> 1384
   Ser Thr Arg Thr Cys Pro Arg Asp Cys Arg Gly Arg Gly Arg Cys Glu
   1               5                   10                  15
   Asp Gly Glu Cys Ile Cys Asp Thr Gly Tyr Ser Gly Asp Asp Cys
               20                  25                  30
<210> 1385
   <211> 31
   <212> PRT
   <213> Homo sapiens
<400> 1385
   Gly Val Arg Ser Cys Pro Gly Asp Cys Asn Gln Arg Gly Arg Cys Glu
   1 5 10 15
   Asp Gly Arg Cys Val Cys Trp Pro Gly Tyr Thr Gly Thr Asp Cys
   20 25 30
<210> 1386
   <211> 31
   <212> PRT
   <213> Homo sapiens
<400> 1386
   Gly Ser Arg Ala Cys Pro Arg Asp Cys Arg Gly Arg Gly Arg Cys Glu
   1 5 10 15
   Asn Gly Val Cys Val Cys Asn Ala Gly Tyr Ser Gly Glu Asp Cys
   20 25 30
<210> 1387
   <211> 32
   <212> PRT
   <213> Homo sapiens
<400> 1387
   Ile Met Asp Asp Cys Ser Thr Asn Cys Asn Gly Asn Gly Glu Cys Ile
   1 5 10 15
   Ser Gly His Cys His Cys Phe Pro Gly Phe Leu Gly Pro Asp Cys Ala
   20 25 30
<210> 1388
   <211> 32
   <212> PRT
   <213> Homo sapiens
<400> 1388
   Arg Asp Ser Cys Pro Val Leu Cys Gly Gly Asn Gly Glu Tyr Glu Lys
   1 5 10 15
   Gly His Cys Val Cys Arg His Gly Trp Lys Gly Pro Glu Cys Asp Val
   20 25 30
<210> 1389
   <211> 33
   <212> PRT
   <213> Homo sapiens
<400> 1389
   Pro Glu Glu Gln Cys Ile Asp Pro Thr Cys Phe Gly His Gly Thr Cys
   1 5 10 15
   Ile Met Gly Val Cys Ile Cys Val Pro Gly Tyr Lys Gly Glu Ile Cys
   20 25 30
   Glu
<210> 1390
   <211> 33
   <212> PRT
   <213> Homo sapiens
<400> 1390
   Glu Glu Asp Cys Leu Asp Pro Met Cys Ser Asn His Gly Ile Cys Val
   1               5                   10                  15
   Lys Gly Glu Cys His Cys Ser Thr Gly Trp Gly Gly Val Asn Cys Glu
               20                  25                  30
   Thr
<210> 1391
   <211> 34
   <212> PRT
   <213> Homo sapiens
<400> 1391
   Pro Leu Pro Val Cys Gln Glu Gln Cys Ser Gly His Gly Thr Phe Leu
   1               5                   10                  15
   Leu Asp Ala Gly Val Cys Ser Cys Asp Pro Lys Trp Thr Gly Ser Asp
               20                  25                  30
   Cys Ser
<210> 1392
   <211> 30
   <212> PRT
   <213> Homo sapiens
<400> 1392
   Thr Glu Leu Cys Thr Met Glu Cys Gly Ser His Gly Val Cys Ser Arg
   1               5                   10                  15
   Gly Ile Cys Gln Cys Glu Glu Gly Trp Val Gly Pro Thr Cys
               20                  25                  30
<210> 1393
   <211> 32
   <212> PRT
   <213> Homo sapiens
<400> 1393
   Glu Glu Arg Ser Cys His Ser His Cys Thr Glu His Gly Gln Cys Lys
   1               5                   10                  15
   Asp Gly Lys Cys Glu Cys Ser Pro Gly Trp Glu Gly Asp His Cys Thr
               20                  25                  30
<210> 1394
   <211> 36
   <212> PRT
   <213> Homo sapiens
<400> 1394
   Val Arg Asp Gly Cys Pro Gly Leu Cys Phe Gly Asn Gly Arg Cys Thr
   1               5                   10                  15
   Leu Asp Gln Asn Gly Trp His Cys Val Cys Gln Val Gly Trp Ser Gly
               20                  25                  30
   Thr Gly Cys Asn
           35
<210> 1395
   <211> 29
   <212> PRT
   <213> Homo sapiens
<400> 1395
   Asp Cys Pro Arg Asn Cys His Gly Asn Gly Glu Cys Val Ser Gly Val
   1               5                   10                  15
   Cys His Cys Phe Pro Gly Phe Leu Gly Ala Asp Cys Ala
               20                  25
<210> 1396
   <211> 37
   <212> PRT
   <213> Homo sapiens
<400> 1396
   Leu Gly Ala Asp Cys Ala Lys Ala Ala Cys Pro Val Leu Cys Ser Gly
   1               5                   10                  15
   Asn Gly Gln Tyr Ser Lys Gly Thr Cys Gln Cys Tyr Ser Gly Trp Lys
               20                  25                  30
   Gly Ala Glu Cys Asp
           35
<210> 1397
   <211> 33
   <212> PRT
   <213> Homo sapiens
<400> 1397
   Pro Met Asn Gln Cys Ile Asp Pro Ser Cys Gly Gly His Gly Ser Cys
   1               5                   10                  15
   Ile Asp Gly Asn Cys Val Cys Ser Ala Gly Tyr Lys Gly Glu His Cys
               20                  25                  30
   Glu
<210> 1398
   <211> 33
   <212> PRT
   <213> Homo sapiens
<400> 1398
   Glu Val Asp Cys Leu Asp Pro Thr Cys Ser Ser His Gly Val Cys Val
   1               5                   10                  15
   Asn Gly Glu Cys Leu Cys Ser Pro Gly Trp Gly Gly Leu Asn Cys Glu
               20                  25                  30
   Leu
<210> 1399
   <211> 34
   <212> PRT
   <213> Homo sapiens
<400> 1399
   Ala Arg Val Gln Cys Pro Asp Gln Cys Ser Gly His Gly Thr Tyr Leu
   1               5                   10                  15
   Pro Asp Thr Gly Leu Cys Ser Cys Asp Pro Asn Trp Met Gly Pro Asp
               20                  25                  30
   Cys Ser
<210> 1400
   <211> 29
   <212> PRT
   <213> Homo sapiens
<400> 1400
   Val Cys Ser Val Asp Cys Gly Thr His Gly Val Cys Ile Gly Gly Ala
   1               5                   10                  15
   Cys Arg Cys Glu Glu Gly Trp Thr Gly Ala Ala Cys Asp
               20                  25
<210> 1401
   <211> 29
   <212> PRT
   <213> Homo sapiens
<400> 1401
   Val Cys His Pro Arg Cys Ile Glu His Gly Thr Cys Lys Asp Gly Lys
   1               5                   10                  15
   Cys Glu Cys Arg Glu Gly Trp Asn Gly Glu His Cys Thr
               20                  25
<210> 1402
   <211> 34
   <212> PRT
   <213> Homo sapiens
<400> 1402
   Asp Gly Cys Pro Asp Leu Cys Asn Gly Asn Gly Arg Cys Thr Leu Gly
   1               5                   10                  15
   Gln Asn Ser Trp Gln Cys Val Cys Gln Thr Gly Trp Arg Gly Pro Gly
               20                  25                  30
   Cys Asn
<210> 1403
   <211> 32
   <212> PRT
   <213> Homo sapiens
<400> 1403
   Ser Val Val Glu Cys Pro Arg Asn Cys His Gly Asn Gly Glu Cys Val
   1               5                   10                  15
   Ser Gly Thr Cys His Cys Phe Pro Gly Phe Leu Gly Pro Asp Cys Ser
               20                  25                  30
<210> 1404
   <211> 31
   <212> PRT
   <213> Homo sapiens
<400> 1404
   Arg Ala Ala Cys Pro Val Leu Cys Ser Gly Asn Gly Gln Tyr Ser Lys
   1               5                   10                  15
   Gly Arg Cys Leu Cys Phe Ser Gly Trp Lys Gly Thr Glu Cys Asp
               20                  25                  30
<210> 1405
   <211> 33
   <212> PRT
   <213> Homo sapiens
<400> 1405
   Pro Thr Thr Gln Cys Ile Asp Pro Gln Cys Gly Gly Arg Gly Ile Cys
   1               5                   10                  15
   Ile Met Gly Ser Cys Ala Cys Asn Ser Gly Tyr Lys Gly Glu Ser Cys
               20                  25                  30
   Glu
<210> 1406
   <211> 32
   <212> PRT
   <213> Homo sapiens
<400> 1406
   Glu Ala Asp Cys Ile Asp Pro Gly Cys Ser Asn His Gly Val Cys Ile
   1               5                   10                  15
   His Gly Glu Cys His Cys Ser Pro Gly Trp Gly Gly Ser Asn Cys Glu
               20                  25                  30
<210> 1407
   <211> 34
   <212> PRT
   <213> Homo sapiens
<400> 1407
   Leu Lys Thr Met Cys Pro Asp Gln Cys Ser Gly His Gly Thr Tyr Leu
   1               5                   10                  15
   Gln Glu Ser Gly Ser Cys Thr Cys Asp Pro Asn Trp Thr Gly Pro Asp
               20                  25                  30
   Cys Ser
<210> 1408
   <211> 32
   <212> PRT
   <213> Homo sapiens
<400> 1408
   Asn Glu Ile Cys Ser Val Asp Cys Gly Ser His Gly Val Cys Met Gly
   1               5                   10                  15
   Gly Thr Cys Arg Cys Glu Glu Gly Trp Thr Gly Pro Ala Cys Asn Gln
               20                  25                  30
<210> 1409
   <211> 30
   <212> PRT
   <213> Homo sapiens
<400> 1409
   Arg Ala Cys His Pro Arg Cys Ala Glu His Gly Thr Cys Lys Asp Gly
   1               5                   10                  15
   Lys Cys Glu Cys Ser Gln Gly Trp Asn Gly Glu His Cys Thr
               20                  25                  30
<210> 1410
   <211> 35
   <212> PRT
   <213> Homo sapiens
<400> 1410
   Ile Glu Gly Cys Pro Gly Leu Cys Asn Ser Asn Gly Arg Cys Thr Leu
   1               5                   10                  15
   Asp Gln Asn Gly Trp His Cys Val Cys Gln Pro Gly Trp Arg Gly Ala
               20                  25                  30
   Gly Cys Asp
           35
<210> 1411
   <211> 32
   <212> PRT
   <213> Homo sapiens
<400> 1411
   Ser Val Asp Asn Cys Pro Ser Asn Cys Tyr Gly Asn Gly Asp Cys Ile
   1               5                   10                  15
   Ser Gly Thr Cys His Cys Phe Leu Gly Phe Leu Gly Pro Asp Cys Gly
               20                  25                  30
<210> 1412
   <211> 31
   <212> PRT
   <213> Homo sapiens
<400> 1412
   Arg Ala Ser Cys Pro Val Leu Cys Ser Gly Asn Gly Gln Tyr Met Lys
   1               5                   10                  15
   Gly Arg Cys Leu Cys His Ser Gly Trp Lys Gly Ala Glu Cys Asp
               20                  25                  30
<210> 1413
   <211> 33
   <212> PRT
   <213> Homo sapiens
<400> 1413
   Pro Thr Asn Gln Cys Ile Asp Val Ala Cys Ser Asn His Gly Thr Cys
   1               5                   10                  15
   Ile Thr Gly Thr Cys Ile Cys Asn Pro Gly Tyr Lys Gly Glu Ser Cys
               20                  25                  30
   Glu
<210> 1414
   <211> 32
   <212> PRT
   <213> Homo sapiens
<400> 1414
   Glu Val Asp Cys Met Asp Pro Thr Cys Ser Gly Arg Gly Val Cys Val
   1               5                   10                  15
   Arg Gly Glu Cys His Cys Ser Val Gly Trp Gly Gly Thr Asn Cys Glu
               20                  25                  30
<210> 1415
   <211> 34
   <212> PRT
   <213> Homo sapiens
<400> 1415
   Pro Arg Ala Thr Cys Leu Asp Gln Cys Ser Gly His Gly Thr Phe Leu
   1               5                   10                  15
   Pro Asp Thr Gly Leu Cys Ser Cys Asp Pro Ser Trp Thr Gly His Asp
               20                  25                  30
   Cys Ser
<210> 1416
   <211> 32
   <212> PRT
   <213> Homo sapiens
<400> 1416
   Ile Glu Ile Cys Ala Ala Asp Cys Gly Gly His Gly Val Cys Val Gly
   1               5                   10                  15
   Gly Thr Cys Arg Cys Glu Asp Gly Trp Met Gly Ala Ala Cys Asp Gln
               20                  25                  30
<210> 1417
   <211> 30
   <212> PRT
   <213> Homo sapiens
<400> 1417
   Arg Ala Cys His Pro Arg Cys Ala Glu His Gly Thr Cys Arg Asp Gly
   1               5                   10                  15
   Lys Cys Glu Cys Ser Pro Gly Trp Asn Gly Glu His Cys Thr
               20                  25                  30
<210> 1418
   <211> 44
   <212> PRT
   <213> Homo sapiens
<400> 1418
   Ile Ala His Tyr Leu Asp Arg Val Val Lys Glu Gly Cys Pro Gly Leu
   1               5                   10                  15
   Cys Asn Gly Asn Gly Arg Cys Thr Leu Asp Leu Asn Gly Trp His Cys
               20                  25                  30
   Val Cys Gln Leu Gly Trp Arg Gly Ala Gly Cys Asp
           35                  40
<210> 1419
   <211> 30
   <212> PRT
   <213> Homo sapiens
<400> 1419
   Leu Asn Arg Thr Cys Cys Leu Asn Gly Gly Thr Cys Met Leu Gly Ser
   1               5                   10                  15
   Phe Cys Ala Cys Pro Pro Ser Phe Tyr Gly Arg Asn Cys Glu
               20                  25                  30
<210> 1420
   <211> 41
   <212> PRT
   <213> Homo sapiens
<400> 1420
   Gly Ala Trp Asp Cys Ser Val Glu Asn Gly Gly Cys Glu His Ala Cys
   1               5                   10                  15
   Asn Ala Ile Pro Gly Ala Pro Arg Cys Gln Cys Pro Ala Gly Ala Ala
               20                  25                  30
   Leu Gln Ala Asp Gly Arg Ser Cys Thr
           35                  40
<210> 1421
   <211> 41
   <212> PRT
   <213> Homo sapiens
<400> 1421
   Ala Thr Gln Ser Cys Asn Asp Leu Cys Glu His Phe Cys Val Pro Asn
   1               5                   10                  15
   Pro Asp Gln Pro Gly Ser Tyr Ser Cys Met Cys Glu Thr Gly Tyr Arg
               20                  25                  30
   Leu Ala Ala Asp Gln His Arg Cys Glu
           35                  40
<210> 1422
   <211> 39
   <212> PRT
   <213> Homo sapiens
<400> 1422
   Asp Val Asp Asp Cys Ile Leu Glu Pro Ser Pro Cys Pro Gln Arg Cys
   1               5                   10                  15
   Val Asn Thr Gln Gly Gly Phe Glu Cys His Cys Tyr Pro Asn Tyr Asp
               20                  25                  30
   Leu Val Asp Gly Glu Cys Val
           35
<210> 1423
   <211> 41
   <212> PRT
   <213> Homo sapiens
<400> 1423
   Pro Val Asp Pro Cys Phe Arg Ala Asn Cys Glu Tyr Gln Cys Gln Pro
   1               5                   10                  15
   Leu Asn Gln Thr Ser Tyr Leu Cys Val Cys Ala Glu Gly Phe Ala Pro
               20                  25                  30
   Ile Pro His Glu Pro His Arg Cys Gln
           35                  40
<210> 1424
   <211> 37
   <212> PRT
   <213> Homo sapiens
<400> 1424
   Cys Gln Met Phe Cys Asn Gln Thr Ala Cys Pro Ala Asp Cys Asp Pro
   1               5                   10                  15
   Asn Thr Gln Ala Ser Cys Glu Cys Pro Glu Gly Tyr Ile Leu Asp Asp
               20                  25                  30
   Gly Phe Ile Cys Thr
           35
<210> 1425
   <211> 41
   <212> PRT
   <213> Homo sapiens
<400> 1425
   Asp Ile Asp Glu Cys Glu Asn Gly Gly Phe Cys Ser Gly Val Cys His
   1               5                   10                  15
   Asn Leu Pro Gly Thr Phe Glu Cys Ile Cys Gly Pro Asp Ser Ala Leu
               20                  25                  30
   Ala Arg His Ile Gly Thr Asp Cys Asp
           35                  40
<210> 1426
   <211> 41
   <212> PRT
   <213> Homo sapiens
<400> 1426
   Pro Ile Asp Gly Cys Leu Ser Asn Pro Cys Phe Ala Gly Val Lys Cys
   1               5                   10                  15
   Thr Ser Tyr Pro Asp Gly Ser Trp Lys Cys Gly Ala Cys Pro Pro Gly
               20                  25                  30
   Tyr Ser Gly Asn Gly Ile Gln Cys Thr
           35                  40
<210> 1427
   <211> 45
   <212> PRT
   <213> Homo sapiens
<400> 1427
   Pro Arg Asn Pro Cys Thr Asp Gly Thr His Asp Cys Asn Lys Asn Ala
   1               5                   10                  15
   Lys Cys Asn Tyr Leu Gly His Tyr Ser Asp Pro Met Tyr Arg Cys Glu
               20                  25                  30
   Cys Lys Pro Gly Tyr Ala Gly Asn Gly Ile Ile Cys Gly
           35                  40                  45
<210> 1428
   <211> 41
   <212> PRT
   <213> Homo sapiens
<400> 1428
   Pro Val Asp Gly Cys Leu Ser Asn Pro Cys Phe Pro Gly Ala Gln Cys
   1               5                   10                  15
   Ser Ser Phe Pro Asp Gly Ser Trp Ser Cys Gly Ser Cys Pro Val Gly
               20                  25                  30
   Phe Leu Gly Asn Gly Thr His Cys Glu
           35                  40
<210> 1429
   <211> 45
   <212> PRT
   <213> Homo sapiens
<400> 1429
   Pro Glu Asn Pro Cys Lys Asp Lys Thr His Asn Cys His Lys His Ala
   1               5                   10                  15
   Glu Cys Ile Tyr Leu Gly His Phe Ser Asp Pro Met Tyr Lys Cys Glu
               20                  25                  30
   Cys Gln Thr Gly Tyr Ala Gly Asp Gly Leu Ile Cys Gly
           35                  40                  45
<210> 1430
   <211> 39
   <212> PRT
   <213> Homo sapiens
<400> 1430
   Asp Ile Asn Glu Cys Ala His Ala Asp Pro Cys Phe Pro Gly Ser Ser
   1               5                   10                  15
   Cys Ile Asn Thr Met Pro Gly Phe His Cys Glu Ala Cys Pro Arg Gly
               20                  25                  30
   Tyr Lys Gly Thr Gln Val Ser
           35
<210> 1431
   <211> 41
   <212> PRT
   <213> Homo sapiens
<400> 1431
   Asp Ile Asp Glu Cys Asn Asp Gly Asn Asn Gly Gly Cys Asp Pro Asn
   1               5                   10                  15
   Ser Ile Cys Thr Asn Thr Val Gly Ser Phe Lys Cys Gly Pro Cys Arg
               20                  25                  30
   Leu Gly Phe Leu Gly Asn Gln Ser Gln
           35                  40
<210> 1432
   <211> 43
   <212> PRT
   <213> Homo sapiens
<400> 1432
   Pro Ala Arg Thr Cys His Ser Pro Ala His Ser Pro Cys His Ile His
   1               5                   10                  15
   Ala His Cys Leu Phe Glu Arg Asn Gly Ala Val Ser Cys Gln Cys Asn
               20                  25                  30
   Val Gly Trp Ala Gly Asn Gly Asn Val Cys Gly
           35                  40
<210> 1433
   <211> 40
   <212> PRT
   <213> Homo sapiens
<400> 1433
   Pro Pro Arg Arg Cys Asp Ser Asn Pro Cys Phe Arg Gly Val Gln Cys
   1               5                   10                  15
   Thr Asp Ser Arg Asp Gly Phe Gln Cys Gly Pro Cys Pro Glu Gly Tyr
               20                  25                  30
   Thr Gly Asn Gly Ile Thr Cys Ile
           35                  40
<210> 1434
   <211> 38
   <212> PRT
   <213> Homo sapiens
<400> 1434
   Asp Val Asp Glu Cys Lys Tyr His Pro Cys Tyr Pro Gly Val His Cys
   1               5                   10                  15
   Ile Asn Leu Ser Pro Gly Phe Arg Cys Asp Ala Cys Pro Val Gly Phe
               20                  25                  30
   Thr Gly Pro Met Val Gln
           35
<210> 1435
   <211> 41
   <212> PRT
   <213> Homo sapiens
<400> 1435
   Asp Ile Asp Glu Cys Arg Asn Gly Ala Cys Val Pro Asn Ser Ile Cys
   1               5                   10                  15
   Val Asn Thr Leu Gly Ser Tyr Arg Cys Gly Pro Cys Lys Pro Gly Tyr
               20                  25                  30
   Thr Gly Asp Gln Ile Arg Gly Cys Lys
           35                  40
<210> 1436
   <211> 43
   <212> PRT
   <213> Homo sapiens
<400> 1436
   Ala Glu Arg Asn Cys Arg Asn Pro Glu Leu Asn Pro Cys Ser Val Asn
   1               5                   10                  15
   Ala Gln Cys Ile Glu Glu Arg Gln Gly Asp Val Thr Cys Val Cys Gly
               20                  25                  30
   Val Gly Trp Ala Gly Asp Gly Tyr Ile Cys Gly
           35                  40
<210> 1437
   <211> 44
   <212> PRT
   <213> Homo sapiens
<400> 1437
   Asp Val Asn Glu Cys Ala Asp Gly Ala His Pro Pro Cys His Ala Ser
   1               5                   10                  15
   Ala Arg Cys Arg Asn Thr Lys Gly Gly Phe Gln Cys Leu Cys Ala Asp
               20                  25                  30
   Pro Tyr Glu Leu Gly Asp Asp Gly Arg Thr Cys Val
           35                  40
<210> 1438
   <211> 39
   <212> PRT
   <213> Homo sapiens
<400> 1438
   Pro Val Lys Ser Cys Ser Glu Pro Arg Cys Phe Asn Gly Gly Thr Cys
   1               5                   10                  15
   Gln Gln Ala Leu Tyr Phe Ser Asp Phe Val Cys Gln Cys Pro Glu Gly
               20                  25                  30
   Phe Ala Gly Lys Cys Cys Glu
           35
<210> 1439
   <211> 42
   <212> PRT
   <213> Homo sapiens
<400> 1439
   Glu Glu Asp Glu Cys Ala Lys Pro Asp Arg Gly Gly Cys Glu Gln Arg
   1               5                   10                  15
   Cys Leu Asn Thr Leu Gly Ser Tyr Gln Cys Ala Cys Glu Pro Gly Tyr
               20                  25                  30
   Glu Leu Gly Pro Asp Arg Arg Ser Cys Glu
           35                  40
<210> 1440
   <211> 41
   <212> PRT
   <213> Homo sapiens
<400> 1440
   Asp Lys Asp Glu Cys Ser Lys Asp Asn Gly Gly Cys Gln His Glu Cys
   1               5                   10                  15
   Val Asn Thr Met Gly Ser Tyr Met Cys Gln Cys Arg Asn Gly Phe Val
               20                  25                  30
   Leu His Asp Asn Lys His Asp Cys Lys
           35                  40
<210> 1441
   <211> 42
   <212> PRT
   <213> Homo sapiens
<400> 1441
   Glu Val Asp Glu Cys Ser Trp Pro Asp His Gly Gly Cys Glu His Arg
   1               5                   10                  15
   Cys Val Asn Thr Leu Gly Ser Tyr Lys Cys Ala Cys Asp Pro Gly Tyr
               20                  25                  30
   Glu Leu Ala Ala Asp Lys Lys Met Cys Glu
           35                  40
<210> 1442
   <211> 41
   <212> PRT
   <213> Homo sapiens
<400> 1442
   Asp Lys Asp Glu Cys Ala Lys Asp Asn Gly Gly Cys Gln His Glu Cys
   1               5                   10                  15
   Val Asn Thr Phe Gly Ser Tyr Leu Cys Arg Cys Arg Asn Gly Tyr Trp
                   20                  25                  30
   Leu His Glu Asn Gly His Asp Cys Lys
           35                  40
<210> 1443
   <211> 41
   <212> PRT
   <213> Homo sapiens
<400> 1443
   Asn His Met Pro Cys Pro Glu Asn Leu Asn Gly Tyr Cys Ile His Gly
   1               5                   10                  15
   Lys Cys Glu Phe Ile Tyr Ser Thr Gln Lys Ala Ser Cys Arg Cys Glu
               20                  25                  30
   Ser Gly Tyr Thr Gly Gln His Cys Glu
           35                  40
<210> 1444
   <211> 41
   <212> PRT
   <213> Homo sapiens
<400> 1444
   His His Ile Pro Cys Pro Glu His Tyr Asn Gly Phe Cys Met His Gly
   1               5                   10                  15
   Lys Cys Glu His Ser Ile Asn Met Gln Glu Pro Ser Cys Arg Cys Asp
               20                  25                  30
   Ala Gly Tyr Thr Gly Gln His Cys Glu
           35                  40
<210> 1445
   <211> 37
   <212> PRT
   <213> Homo sapiens
<400> 1445
   Pro Cys Leu Asn Asp Cys Gly Pro Tyr Gly Gln Cys Leu Leu Leu Arg
   1               5                   10                  15
   Arg His Ser Tyr Leu Tyr Ala Ser Cys Ser Cys Lys Ala Gly Trp Arg
               20                  25                  30
   Gly Trp Ser Cys Thr
           35
<210> 1446
   <211> 40
   <212> PRT
   <213> Homo sapiens
<400> 1446
   Phe Leu Ser Pro Cys Val Asp Asp Cys Gly Pro Tyr Gly Gln Cys Lys
   1               5                   10                  15
   Leu Leu Arg Thr His Asn Tyr Leu Tyr Ala Ala Cys Glu Cys Lys Ala
               20                  25                  30
   Gly Trp Arg Gly Trp Gly Cys Thr
           35                  40
<210> 1447
   <211> 43
   <212> PRT
   <213> Homo sapiens
<400> 1447
   Gly Cys Gly Ala Gly Arg Trp Gly Pro Gly Cys Thr Lys Glu Cys Pro
   1               5                   10                  15
   Gly Cys Leu His Gly Gly Val Cys His Asp His Asp Gly Glu Cys Val
               20                  25                  30
   Cys Pro Pro Gly Phe Thr Gly Thr Arg Cys Glu
           35                  40
<210> 1448
   <211> 46
   <212> PRT
   <213> Homo sapiens
<400> 1448
   Ala Cys Arg Glu Gly Arg Phe Gly Gln Ser Cys Gln Glu Gln Cys Pro
   1               5                   10                  15
   Gly Ile Ser Gly Cys Arg Gly Leu Thr Phe Cys Leu Pro Asp Pro Tyr
               20                  25                  30
   Gly Cys Ser Cys Gly Ser Gly Trp Arg Gly Ser Gln Cys Gln
           35                  40                  45
<210> 1449
   <211> 41
   <212> PRT
   <213> Homo sapiens
<400> 1449
   Ala Cys Ala Pro Gly His Phe Gly Ala Asp Cys Arg Leu Gln Cys Gln
   1               5                   10                  15
   Cys Gln Asn Gly Gly Thr Cys Asp Arg Phe Ser Gly Cys Val Cys Pro
               20                  25                  30
   Ser Gly Trp His Gly Val His Cys Glu
           35                  40
<210> 1450
   <211> 37
   <212> PRT
   <213> Homo sapiens
<400> 1450
   Val Pro Ser Asn Cys Asp Cys Leu Asn Gly Gly Thr Cys Val Ser Asn
   1               5                   10                  15
   Lys Tyr Phe Ser Asn Ile His Trp Cys Asn Cys Pro Lys Lys Phe Gly
               20                  25                  30
   Gly Gln His Cys Glu
           35
<210> 1451
   <211> 37
   <212> PRT
   <213> Homo sapiens
<400> 1451
   Glu Ala Arg Trp Cys Ser Glu Cys His Ser Asn Ala Thr Cys Thr Glu
   1               5                   10                  15
   Asp Glu Ala Val Thr Thr Cys Thr Cys Gln Glu Gly Phe Thr Gly Asp
               20                  25                  30
   Gly Leu Thr Cys Val
           35
<210> 1452
   <211> 43
   <212> PRT
   <213> Homo sapiens
<400> 1452
   Asp Leu Asp Glu Cys Ala Ile Pro Gly Ala His Asn Cys Ser Ala Asn
   1               5                   10                  15
   Ser Ser Cys Val Asn Thr Pro Gly Ser Phe Ser Cys Val Cys Pro Glu
               20                  25                  30
   Gly Phe Arg Leu Ser Pro Gly Leu Gly Cys Thr
           35                  40
<210> 1453
   <211> 42
   <212> PRT
   <213> Homo sapiens
<400> 1453
   Asp Val Asp Glu Cys Ala Glu Pro Gly Leu Ser His Cys His Ala Leu
   1               5                   10                  15
   Ala Thr Cys Val Asn Val Val Gly Ser Tyr Leu Cys Val Cys Pro Ala
               20                  25                  30
   Gly Tyr Arg Gly Asp Gly Trp His Cys Glu
           35                  40
<210> 1454
   <211> 50
   <212> PRT
   <213> Homo sapiens
<400> 1454
   Asp Val Asn Glu Cys Phe Tyr Glu Glu Leu Asn Ala Cys Ser Gly Arg
   1               5                   10                  15
   Glu Leu Cys Ala Asn Leu Glu Gly Ser Tyr Trp Cys Val Cys His Gln
               20                  25                  30
   Glu Ala Pro Ala Thr Ser Pro Arg Lys Leu Asn Leu Glu Trp Glu Asp
           35                  40                  45
   Cys Pro
       50
<210> 1455
   <211> 46
   <212> PRT
   <213> Homo sapiens
<400> 1455
   Asp Trp Asp Glu Cys Val Asp Ser Ala Glu His Asp Cys Ser Pro Ala
   1               5                   10                  15
   Ala Trp Cys Ile Asn Leu Glu Gly Ser Tyr Thr Cys Gln Cys Arg Thr
               20                  25                  30
   Thr Arg Asp Ala Thr Pro Ser Arg Ala Gly Arg Ala Cys Glu
           35                  40                  45
<210> 1456
   <211> 42
   <212> PRT
   <213> Homo sapiens
<400> 1456
   Asp Tyr Asp Glu Cys Glu Arg Lys Glu Asp Asp Cys Val Pro Gly Thr
   1               5                   10                  15
   Ser Cys Arg Asn Thr Leu Gly Ser Phe Thr Cys Ser Cys Glu Gly Gly
               20                  25                  30
   Ala Pro Asp Phe Pro Val Glu Tyr Ser Glu
           35                  40
<210> 1457
   <211> 57
   <212> PRT
   <213> Homo sapiens
<400> 1457
   Cys Gln Cys His Gly His Ala Asp Asn Cys Asp Thr Thr Ser Gln Pro
   1               5                   10                  15
   Tyr Arg Cys Leu Cys Ser Gln Glu Ser Phe Thr Glu Gly Leu His Cys
               20                  25                  30
   Asp Arg Cys Leu Pro Leu Tyr Asn Asp Lys Pro Phe Arg Gln Gly Asp
           35                  40                  45
   Gln Val Tyr Ala Phe Asn Cys Lys Pro
       50                  55
<210> 1458
   <211> 51
   <212> PRT
   <213> Homo sapiens
<400> 1458
   Cys Asn Cys His Leu Ser Gly Ala Leu Asn Glu Thr Cys His Leu Val
   1               5                   10                  15
   Thr Gly Gln Cys Phe Cys Lys Gln Phe Val Thr Gly Ser Lys Cys Asp
               20                  25                  30
   Ala Cys Val Pro Ser Ala Ser His Leu Asp Val Asn Asn Leu Leu Gly
           35                  40                  45
   Cys Ser Lys
       50
<210> 1459
   <211> 66
   <212> PRT
   <213> Homo sapiens
<400> 1459
   Cys Gln Cys Asn Ser His Ser Lys Ser Cys His Tyr Asn Ile Ser Val
   1               5                   10                  15
   Asp Pro Phe Pro Phe Glu His Phe Arg Gly Gly Gly Gly Val Cys Asp
               20                  25                  30
   Asp Cys Glu His Asn Thr Thr Gly Arg Asn Cys Glu Leu Cys Lys Asp
           35                  40                  45
   Tyr Phe Phe Arg Gln Val Gly Ala Asp Pro Ser Ala Ile Asp Val Cys
       50                  55                  60
   Lys Pro
   65
<210> 1460
   <211> 53
   <212> PRT
   <213> Homo sapiens
<400> 1460
   Cys Asp Cys Asp Thr Val Gly Thr Arg Asn Gly Ser Ile Leu Cys Asp
   1               5                   10                  15
   Gln Ile Gly Gly Gln Cys Asn Cys Lys Arg His Val Ser Gly Arg Gln
               20                  25                  30
   Cys Asn Gln Cys Gln Asn Gly Phe Tyr Asn Leu Gln Glu Leu Asp Pro
           35                  40                  45
   Asp Gly Cys Ser Pro
       50
<210> 1461
   <211> 53
   <212> PRT
   <213> Homo sapiens
<400> 1461
   Cys Asn Cys Asn Thr Ser Gly Thr Val Asp Gly Asp Ile Thr Cys His
   1               5                   10                  15
   Gln Asn Ser Gly Gln Cys Lys Cys Lys Ala Asn Val Ile Gly Leu Arg
               20                  25                  30
   Cys Asp His Cys Asn Phe Gly Phe Lys Phe Leu Arg Ser Phe Asn Asp
           35                  40                  45
   Val Gly Cys Glu Pro
       50
<210> 1462
   <211> 48
   <212> PRT
   <213> Homo sapiens
<400> 1462
   Cys Gln Cys Asn Leu His Gly Ser Val Asn Lys Phe Cys Asn Pro His
   1               5                   10                  15
   Ser Gly Gln Cys Glu Cys Lys Lys Glu Ala Lys Gly Leu Gln Cys Asp
               20                  25                  30
   Thr Cys Arg Glu Asn Phe Tyr Gly Leu Asp Val Thr Asn Cys Lys Ala
           35                  40                  45
<210> 1463
   <211> 52
   <212> PRT
   <213> Homo sapiens
<400> 1463
   Cys Asp Cys Asp Thr Ala Gly Ser Leu Pro Gly Thr Val Cys Asn Ala
   1               5                   10                  15
   Lys Thr Gly Gln Cys Ile Cys Lys Pro Asn Val Glu Gly Arg Gln Cys
               20                  25                  30
   Asn Lys Cys Leu Glu Gly Asn Phe Tyr Leu Arg Gln Asn Asn Ser Phe
           35                  40                  45
   Leu Cys Leu Pro
       50
<210> 1464
   <211> 53
   <212> PRT
   <213> Homo sapiens
<400> 1464
   Cys Asn Cys Asp Lys Thr Gly Thr Ile Asn Gly Ser Leu Leu Cys Asn
   1 5 10 15
   Lys Ser Thr Gly Gln Cys Pro Cys Lys Leu Gly Val Thr Gly Leu Arg
   20 25 30
   Cys Asn Gln Cys Glu Pro His Arg Tyr Asn Leu Thr Ile Asp Asn Phe
   35 40 45
   Gln His Cys Gln Met
   50
<210> 1465
   <211> 51
   <212> PRT
   <213> Homo sapiens
<400> 1465
   Cys Glu Cys Asp Ser Leu Gly Thr Leu Pro Gly Thr Ile Cys Asp Pro
   1 5 10 15
   Ile Ser Gly Gln Cys Leu Cys Val Pro Asn Arg Gln Gly Arg Arg Cys
   20 25 30
   Asn Gln Cys Gln Pro Gly Phe Tyr Ile Ser Pro Gly Asn Ala Thr Gly
   35 40 45
   Cys Leu Pro
   50
<210> 1466
   <211> 51
   <212> PRT
   <213> Homo sapiens
<400> 1466
   Cys Ser Cys His Thr Thr Gly Ala Val Asn His Ile Cys Asn Ser Leu
   1 5 10 15
   Thr Gly Gln Cys Val Cys Gln Asp Ala Ser Ile Ala Gly Gln Arg Cys
   20 25 30
   Asp Gln Cys Lys Asp His Tyr Phe Gly Phe Asp Pro Gln Thr Gly Arg
   35 40 45
   Cys Gln Pro
   50
<210> 1467
   <211> 38
   <212> PRT
   <213> Homo sapiens
<400> 1467
   Gln Pro Gln Asp Cys Pro Pro Ser Thr Cys Leu Asn Gly Gly Thr Cys
   1 5 10 15
   His Leu Gly Thr Arg His His Leu Ala Cys Leu Cys Pro Glu Gly Phe
   20 25 30
   Thr Gly Leu Tyr Cys Glu
   35
<210> 1468
   <211> 37
   <212> PRT
   <213> Homo sapiens
<400> 1468
   Gly Pro Asp Arg Cys Lys Met Asn Pro Cys Leu Asn Gly Gly Thr Cys
   1               5                   10                  15
   Tyr Pro Thr Glu Thr Ser Tyr Val Cys Thr Cys Val Pro Gly Tyr Ser
               20                  25                  30
   Gly Asp Gln Cys Glu
           35
<210> 1469
   <211> 40
   <212> PRT
   <213> Homo sapiens
<400> 1469
   His Ile Asn Glu Cys Leu Val Asn Asn Gly Gly Cys Ser His Ile Cys
   1               5                   10                  15
   Lys Asp Leu Val Ile Gly Tyr Glu Cys Asp Cys Ala Ala Gly Phe Glu
               20                  25                  30
   Leu Ile Asp Arg Lys Thr Cys Gly
           35                  40
<210> 1470
   <211> 40
   <212> PRT
   <213> Homo sapiens
<400> 1470
   Asp Ile Asp Glu Cys Gln Asn Pro Gly Ile Cys Ser Gln Ile Cys Ile
   1               5                   10                  15
   Asn Leu Lys Gly Gly Tyr Lys Cys Glu Cys Ser Arg Gly Tyr Gln Met
               20                  25                  30
   Asp Leu Ala Thr Gly Val Cys Lys
           35                  40
<210> 1471
   <211> 49
   <212> PRT
   <213> Homo sapiens
<400> 1471
   Gly Lys Asn Trp Cys Glu Glu Asp Met Glu Asn Gly Gly Cys Glu Tyr
   1               5                   10                  15
   Leu Cys Leu Pro Ala Pro Gln Ile Asn Asp His Ser Pro Lys Tyr Thr
               20                  25                  30
   Cys Ser Cys Pro Ser Gly Tyr Asn Val Glu Glu Asn Gly Arg Asp Cys
           35                  40                  45
   Gln
<210> 1472
   <211> 36
   <212> PRT
   <213> Homo sapiens
<400> 1472
   Leu Glu His Pro Cys Ala Ser Leu Cys Cys Gly His Gly Thr Cys Ile
   1               5                   10                  15
   Asp Gly Ile Gly Ser Phe Ser Cys Asp Cys Arg Ser Gly Trp Glu Gly
               20                  25                  30
   Arg Phe Cys Gln
           35
<210> 1473
   <211> 41
   <212> PRT
   <213> Homo sapiens
<400> 1473
   Ser Phe Leu Asn Cys Ser Leu Asp Asn Gly Gly Cys Thr His Tyr Cys
   1               5                   10                  15
   Leu Glu Glu Val Gly Trp Arg Arg Cys Ser Cys Ala Pro Gly Tyr Lys
               20                  25                  30
   Leu Gly Asp Asp Leu Leu Gln Cys His
           35                  40
<210> 1474
   <211> 39
   <212> PRT
   <213> Homo sapiens
<400> 1474
   Ile Pro Asp Gln Cys Ser Pro Leu Pro Cys Asn Glu Asp Gly Tyr Met
   1               5                   10                  15
   Ser Cys Lys Asp Gly Lys Ala Ser Phe Thr Cys Thr Cys Lys Pro Gly
                   20                  25                  30
   Trp Gln Gly Glu Lys Cys Glu
           35
<210> 1475
   <211> 44
   <212> PRT
   <213> Homo sapiens
<400> 1475
   Asp Ile Asn Glu Cys Lys Asp Pro Ser Asn Ile Asn Gly Gly Cys Ser
   1               5                   10                  15
   Gln Ile Cys Asp Asn Thr Pro Gly Ser Tyr His Cys Ser Cys Lys Asn
               20                  25                  30
   Gly Phe Val Met Leu Ser Asn Lys Lys Asp Cys Lys
           35                  40
<210> 1476
   <211> 42
   <212> PRT
   <213> Homo sapiens
<400> 1476
   Asp Val Asp Glu Cys Ser Leu Lys Pro Ser Ile Cys Gly Thr Ala Val
   1               5                   10                  15
   Cys Lys Asn Ile Pro Gly Asp Phe Glu Cys Glu Cys Pro Glu Gly Tyr
               20                  25                  30
   Arg Tyr Asn Leu Lys Ser Lys Ser Cys Glu
           35                  40
<210> 1477
   <211> 41
   <212> PRT
   <213> Homo sapiens
<400> 1477
   Asp Ile Asp Glu Cys Ser Glu Asn Met Cys Ala Gln Leu Cys Val Asn
   1               5                   10                  15
   Tyr Pro Gly Gly Tyr Thr Cys Tyr Cys Asp Gly Lys Lys Gly Phe Lys
               20                  25                  30
   Leu Ala Gln Asp Gln Lys Ser Cys Glu
           35                  40
<210> 1478
   <211> 37
   <212> PRT
   <213> Homo sapiens
<400> 1478
   Gly Gly Ser Pro Cys Ile Ser Gln Pro Cys Leu His Asn Gly Ser Cys
   1               5                   10                  15
   Gln Asp Ser Ile Trp Gly Tyr Thr Cys Thr Cys Ser Pro Gly Tyr Glu
               20                  25                  30
   Gly Ser Asn Cys Glu
           35
<210> 1479
   <211> 42
   <212> PRT
   <213> Homo sapiens
<400> 1479
   Ala Lys Asn Glu Cys His Pro Glu Arg Thr Asp Gly Cys Gln His Phe
   1               5                   10                  15
   Cys Leu Pro Gly Gln Glu Ser Tyr Thr Cys Ser Cys Ala Gln Gly Tyr
               20                  25                  30
   Arg Leu Gly Glu Asp His Lys Gln Cys Val
           35                  40
<210> 1480
   <211> 38
   <212> PRT
   <213> Homo sapiens
<400> 1480
   Pro Gly Pro Cys Asp Ser Gln Pro Cys Gln Asn Gly Gly Thr Cys Val
   1               5                   10                  15
   Pro Glu Gly Leu Asp Gly Tyr Gln Cys Leu Cys Pro Leu Ala Phe Gly
               20                  25                  30
   Gly Glu Ala Asn Cys Ala
           35
<210> 1481
   <211> 37
   <212> PRT
   <213> Homo sapiens
<400> 1481
   Pro Val Asn Leu Cys Lys Pro Ser Pro Cys Met Asn Glu Gly Ser Cys
   1               5                   10                  15
   Val Leu Gln Asn Gly Ser Tyr Arg Cys Lys Cys Arg Asp Gly Trp Glu
               20                  25                  30
   Gly Pro His Cys Glu
           35
<210> 1482
   <211> 29
   <212> PRT
   <213> Homo sapiens
<400> 1482
   Leu Cys Ser Phe Gly Cys Gly Ser Gly Ile Cys Ile Ala Pro Asn Val
   1               5                   10                  15
   Cys Ser Cys Gln Asp Gly Glu Gln Gly Ala Thr Cys Pro
               20                  25
<210> 1483
   <211> 39
   <212> PRT
   <213> Homo sapiens
<400> 1483
   Asp Ile Asp Glu Cys Val Thr Ser Ser Cys Glu Gly His Cys Val Asn
   1               5                   10                  15
   Thr Glu Gly Gly Phe Val Cys Glu Cys Gly Pro Gly Met Gln Leu Ser
               20                  25                  30
   Ala Asp Arg His Ser Cys Gln
           35
<210> 1484
   <211> 39
   <212> PRT
   <213> Homo sapiens
<400> 1484
   Asp Thr Asp Glu Cys Leu Gly Thr Pro Cys Gln Gln Arg Cys Lys Asn
   1               5                   10                  15
   Ser Ile Gly Ser Tyr Lys Cys Ser Cys Arg Thr Gly Phe His Leu His
               20                  25                  30
   Gly Asn Arg His Ser Cys Val
           35
<210> 1485
   <211> 43
   <212> PRT
   <213> Homo sapiens
<400> 1485
   Asp Val Asn Glu Cys Arg Arg Pro Leu Glu Arg Arg Val Cys His His
   1               5                   10                  15
   Ser Cys His Asn Thr Val Gly Ser Phe Leu Cys Thr Cys Arg Pro Gly
               20                  25                  30
   Phe Arg Leu Arg Ala Asp Arg Val Ser Cys Glu
           35                  40
<210> 1486
   <211> 40
   <212> PRT
   <213> Homo sapiens
<400> 1486
   Thr Val Lys Ser Cys Asp Cys Leu Asn Gly Gly Ser Cys Val Ser Asp
   1               5                   10                  15
   Arg Asn Phe Ser Pro Gly Ser Gly Val Tyr Leu Cys Val Cys Leu Pro
               20                  25                  30
   Gly Phe His Gly Ser Leu Cys Glu
           35                  40
<210> 1487
   <211> 33
   <212> PRT
   <213> Homo sapiens
<400> 1487
   Ala Phe Thr Ile Cys Lys Tyr Pro Cys Gly Lys Ser Arg Glu Cys Val
   1               5                   10                  15
   Ala Pro Asn Ile Cys Lys Cys Lys Pro Gly Tyr Ile Gly Ser Asn Cys
               20                  25                  30
   Gln
<210> 1488
   <211> 33
   <212> PRT
   <213> Homo sapiens
<400> 1488
   Asp Glu Glu His Cys Asn Pro Pro Cys Gln His Gly Gly Thr Cys Leu
   1               5                   10                  15
   Ala Gly Asn Leu Cys Thr Cys Pro Tyr Gly Phe Val Gly Pro Arg Cys
               20                  25                  30
   Glu
<210> 1489
   <211> 33
   <212> PRT
   <213> Homo sapiens
<400> 1489
   Ser Thr Ala Leu Cys Asp Pro Val Cys Leu Asn Gly Gly Ser Cys Asn
   1               5                   10                  15
   Lys Pro Asn Thr Cys Leu Cys Pro Asn Gly Phe Phe Gly Glu His Cys
               20                  25                  30
   Gln
<210> 1490
   <211> 32
   <212> PRT
   <213> Homo sapiens
<400> 1490
   Asn Ala Phe Cys His Pro Pro Cys Lys Asn Gly Gly His Cys Met Arg
   1               5                   10                  15
   Asn Asn Val Cys Val Cys Arg Glu Gly Tyr Thr Gly Arg Arg Phe Gln
               20                  25                  30
<210> 1491
   <211> 33
   <212> PRT
   <213> Homo sapiens
<400> 1491
   Asn Thr Pro Ile Cys Leu Gln Lys Cys Lys Asn Gly Gly Glu Cys Ile
   1               5                   10                  15
   Ala Pro Ser Ile Cys His Cys Pro Ser Ser Trp Glu Gly Val Arg Cys
               20                  25                  30
   Gln
<210> 1492
   <211> 32
   <212> PRT
   <213> Homo sapiens
<400> 1492
   Ile Pro Ile Cys Asn Pro Lys Cys Leu Tyr Gly Gly Arg Cys Ile Phe
   1               5                   10                  15
   Pro Asn Val Cys Ser Cys Arg Thr Glu Tyr Ser Gly Val Lys Cys Glu
               20                  25                  30
<210> 1493
   <211> 33
   <212> PRT
   <213> Homo sapiens
<400> 1493
   Gln Gln Ala Glu Cys Pro Gly Gly Cys Arg Asn Gly Gly Phe Cys Asn
   1               5                   10                  15
   Glu Arg Arg Ile Cys Glu Cys Pro Asp Gly Phe His Gly Pro His Cys
               20                  25                  30
   Glu
<210> 1494
   <211> 32
   <212> PRT
   <213> Homo sapiens
<400> 1494
   Lys Ala Leu Cys Thr Pro Arg Cys Met Asn Gly Gly Leu Cys Val Thr
   1               5                   10                  15
   Pro Gly Phe Cys Ile Cys Pro Pro Gly Phe Tyr Gly Val Asn Cys Asp
               20                  25                  30
<210> 1495
   <211> 29
   <212> PRT
   <213> Homo sapiens
<400> 1495
   Lys Ala Asn Cys Ser Thr Thr Cys Phe Asn Gly Gly Thr Cys Phe Tyr
   1               5                   10                  15
   Pro Gly Lys Cys Ile Cys Pro Pro Gly Leu Glu Gly Glu
               20                  25
<210> 1496
   <211> 33
   <212> PRT
   <213> Homo sapiens
<400> 1496
   Glu Ile Ser Lys Cys Pro Gln Pro Cys Arg Asn Gly Gly Lys Cys Ile
   1               5                   10                  15
   Gly Lys Ser Lys Cys Lys Cys Ser Lys Gly Tyr Gln Gly Asp Leu Cys
               20                  25                  30
   Ser
<210> 1497
   <211> 32
   <212> PRT
   <213> Homo sapiens
<400> 1497
   Lys Pro Val Cys Glu Pro Gly Cys Gly Ala His Gly Thr Cys His Glu
   1               5                   10                  15
   Pro Asn Lys Cys Gln Cys Gln Glu Gly Trp His Gly Arg His Cys Asn
               20                  25                  30
<210> 1498
   <211> 37
   <212> PRT
   <213> Homo sapiens
<400> 1498
   Pro Glu Ser Pro Cys Leu Gln Asn Pro Cys Gln Asn Asp Gly Gln Cys
   1               5                   10                  15
   Arg Glu Gln Gly Ala Thr Phe Thr Cys Glu Cys Glu Val Gly Tyr Gly
               20                  25                  30
   Gly Gly Leu Cys Met
           35